# EUROPEAN PATENT SPECIFICATION

(11) **EP 2 167 531 B1**
(45) Date of publication and mention of the grant of the patent: **21.03.2012**
(21) Application number: 08789058.8
(22) Date of filing: 23.05.2008
(51) Int. Cl.: C07K 14/315, C12N 9/10, C07K 16/12, A61K 39/09, G01N 33/569

(54) **STREPTOCOCCUS PNEUMONIAE PILUS ANTIGENS**
STREPTOCOCCUS PNEUMONIAE PILUS-ANTIGENE
ANTIGÈNES DE PILUS DE STREPTOCOCCUS PNEUMONIAE

(30) Priority: 25.05.2007 US 940167 P
(43) Date of publication of application: 31.03.2010
(73) Proprietor: Novartis AG, 4056 Basel (CH)
(72) Inventor: DONATI, Claudio, I-53100 Siena (IT); MASIGNANI, Vega, I-53100 Siena (IT); MORA, Marirosa, I-53100 Siena (IT); MOSCHIONI, Monica, I-53100 Siena (IT)
(74) Representative: Bullett, Rachel Margaret
(86) International application number: PCT/IB2008/002108
(87) International publication number: WO 2008/146164

(56) References cited:
- BAROCCHI M A ET AL: "A pneumococcal pilus influences virulence and host inflammatory responses" PROCEEDINGS OF THE NATIONAL ACADEMY OF SCIENCES OF USA, NATIONAL ACADEMY OF SCIENCE, WASHINGTON, DC.; US, vol. 103, no. 8, 21 February 2006 (2006-02-21), pages 2857-2862, XP002466451 ISSN: 0027-8424
- BAROCCHI MICHELE ANNE ET AL: "Vaccines in the era of genomics: The pneumococcal challenge" VACCINE, vol. 25, no. 16, Sp. Iss. SI, April 2007 (2007-04), pages 2963-2973, XP002509767 ISSN: 0264-410X

## Description

### FIELD

This invention relates to polypeptides, including pili proteins, from *Streptococcus pneumoniae* (*S. pneumoniae*), including fragments and variants thereof, and methods of their use in the treatment of and immunization against S. *pneumoniae* infections.

### BACKGROUND

The Gram-positive bacterium *Streptococcus pneumoniae* (also known as Spn or pneumococcus) is a major cause of morbidity and mortality world-wide and represents one of the four major infectious disease killers, together with HIV, malaria, and tuberculosis (Bruyn, G. A. W. & van Furth, R. (1991) Eur. J. Clin. Microbiol. Infect. Dis. 10, 897-910; Ryan, M. W. & Antonelli, P. J. (2000) Laryngoscope 110, 961-964; Cutts, F. T., Zaman, S. M., Enwere, G., Jaffar, S., Levine, O. S., Okoko, C. Oluwalana, A., Vaughan, S., Obaro, A., Leach, A., et al. (2005) Lancet 365, 1139-1146; Swiatlo, E., Champlin, F. R., Holman, S. C., Wilson, W. W.&Watt, J. M. (2002) Infect. Immun. 70, 412-415; Sandgren, A., Albiger, B., Orihuela, C., Tuomanen, E., Noumark, S. & Henriques-Nonnark, B. (2005) J. Infect. Dis. 192, 791-800). It is a main cause of respiratory tract infections such as otitis media, sinusitis, and community acquired pneumonia, but also an important pathogen in invasive diseases such as septicemia and meningitis. Even though pneumococcus is a devastating pathogen, it also harmlessly colonizes healthy children attending day-care centers to a high extent (Henriques Normark, B., Christensson, B., Sandgren, A., Noreen, B., Sylvan, S., Burman, L. G. & Olsson-Liljequist, B. (2003) Microb. Drug Resist. 9, 337-344; Nunes, S., Sá-Leão, R., Carriço, J., Alves, C. R., Mato, R., Avô, A. B., Saldanha, J., Almeida, J. S., Sanches, I. S. & de Lencastre, H. (2005) J. Clin. Microbiol. 43, 1285-1293). A major virulence factor in pneumococcal disease is the polysaccharide capsule, by which pneumococci are grouped into at least ninety different serotypes (Henrichsen, J. (1995) J. Clin. Microbiol. 33, 2759-2762). Other genetic factors, such as CbpA (choline-binding protein A) and pneumolysin, have been described to be of importance for virulence (Lau, G. W., Haataja, S., Lonetto, M., Kensit, S. E., Marra, A., Bryant, A. P., McDevitt, D., Morrison, D. A. & Holden, D. W. (2001) Mol. Microbiol. 40, 555-571; Rosenow, C., Ryan, P., Weiser, J. N., Johnson, S., Fontan, P., Ortqvist, A. & Masure, H. R. (1997) Mol. Microbiol. 25, 819-829; Tuomanen, E. (1999) Currant Opin. Biol. 2, 35-39).

Infection by *S. pneumonie* leads to invasive disease triggered by initial colonization of the nasopharynx, but the mechanisms of adhesion are not well understood. *In vitro* adhesion of encapsulated pneumococci is much lower than for nonencapsulated nonvirulent derivatives (Swiatlo, E., Champlin, F. R., Holman, S. C., Wilson, W. W.&Watt, J. M. (2002) Infect. Immun. 70, 412-415), even though capsule expression is essential for successful colonization of the upper airways. These observations suggest that *in vivo,* pneumococci are adhesive despite the production of a thick capsule (Sandgren, A., Albiger, B., Orihuela, C., Tuomanen, E., Normark, S. & Henriques-Normark, B. (2005) J. Infect. Dis. 192, 791-800).

In other Gram-positive bacteria, such as *Corymebacterium diphtheriae* (Ton-That, H., Marraffini, L. A. & Schneewind, O. (2004) Mol. Microbiol. 53, 251-261; Ton-That, H. & Schneewind, O. (2003) Mol. Microbiol. 50, 1429-1438), *Actinomyces* spp. (Kelstrup, J., Theilade, J. & Fejerskov, O. (1979) Scand. J. Dent. Res. 87, 415-423), and recently group A streptococci (GAS) and group B streptococci (GBS) (Mora, M., Bensi, G., Capo, S., Falugi, F., Zingaretti, C., Manetti, A. G. O., Maggi, T., Taddei, A. R., Grandi, G. & Telford, J. L. (2005) Proc. Natl. Acad. Sci. USA 102, 15641-15646; Lauer, P., Rinaudo, C. D., Soriani, M., Margarit, I., Mainone, D., Rosini, R., Taddei, A. R., Mora, M., Rappuoli, R., Grandi, G. & Telford, J. L. (2005) Science 309, 105), pili-like surface structures have been identified by electron microscopy and characterized genetically as well as biochemically (Ton-That, H., Marraffini, L. A. & Schneewind, O. (2004) Mol. Microbiol. 53, 251-261; Ton-That, H. & Schneewind, O. (2003) Mol. Microbiol. 50, 1429-1438; Mora, M., Bensi, G., Capo, S., Falugi, F., Zingaretti, C., Manetti, A. G. O., Maggi, T., Taddei, A. R., Grandi, G. & Telford, J. L. (2005) Proc. Natl. Acad. Sci. USA 102, 15641-15646; Lauer, P., Rinaudo, C. D., Soriani, M., Margarit, I., Mainone, D., Rosini, R., Taddei, A. R., Mora, M., Rappuoli, R., Grandi, G. & Telford, J. L. (2005) Science 309, 105). In *Actinomyces* spp. type 1 fimbrial genes mediate adhesion to dental and mucosal surfaces (Li, T., Khah, M. K., Slavnic, S., Johansson, I. & Stromberg, N. (2001) Infect Immun. 69, 7224-7233). However, there is a need for functional data on the physiological role and function in infectious disease of pili and other antigens in pathogenic *Streptococcus* spp.

Gram-positive pili are extended polymers formed by a transpeptidase reaction involving covalent cross-linking of subunit proteins containing specific amino acid motifs, which are assembled by specific sortases. Sortases are also responsible for covalent attachment of the pilus to the peptidoglycan cell wall.

### SUMMARY

The present disclosure describes polypeptides from *Streptococcus pneumoniae.* In some aspects the polypeptides described herein include pili peptides from *S. pneumoniae.* In other aspects, other polypeptides from *S. preumoniae* are described. The *Streptococcus pneumoniae* polypeptides described herein are useful in methods of treatment for and immunization against *S. pneumoniae* infections.

In some aspects, the disclosure features pili polypeptides from a second pili island identified in *Streptococcus pneumoniae* INV104B (pilus II island (FN-V104B)). In other aspects, the disclosure features pili polypeptides identified in *S. pneumoniae* 23F, INV200, and OXC141. The pili are thought to play a role in the pathogenesis of *S. pneumoniae.*

In some aspects, the disclosure features isolated pilus encoded by the *Streptococcus pneumoniae* pilus II island (INV104B). In some embodiments, the pilus includes a sortase. In some embodiments, the pilus includes an LPXTG cell wall anchored protein, *e.g.,* a polypeptide having the amino acid sequence of SEQ ID NO:2, 4 and/or 6, or a processed form thereof.

In some embodiments, the pili are separated from cells by enzymatic digestion (*e.g.,* with one or more enzymes such as peptidoglycan hydrolases (*e.g.,* mutanolysin, lysostaphin, and lysozyme)). In some embodiments, the pili are separated from cells by mechanical shearing (*e.g.,* by ultrasonication). In some embodiments, the pili are substantially free of bacterial cells. In some embodiments, the disclosure features methods of producing the pilus (*e.g., S. pneumoniae* pili), wherein the methods include subjecting a bacterial cell that produces the pilus to enzymatic digestion or mechanical shearing and isolating the pilus from the cell.

In other aspects, the disclosure features immunogenic compositions including more or more of the isolated pili (*e.g., S. pneumoniae* pili).

In other aspects, the disclosure features an isolated *Streptococcus pneumoniae* sortase, wherein the sortase is one of SEQ ID NO:282, SEQ ID NO: 1386, SEQ ID NO:676, or SEQ ID NO:1123.

In other aspects, the disclosure features an isolated *Streptococcus pneumoniae* LPXTG cell wall anchored protein, wherein the LPXTG cell wall anchored protein is one of SEQ ID NO:2, SEQ ID NO:4, SEQ ID NO:6, SEQ ID NO:7, SEQ ID NO:8, or SEQ ID NO:9.

In further aspects, the disclosure features methods of isolating pili encoded by the *Streptococcus pneumoniae* pilus II island (INV 104B), wherein the methods include subjecting bacterial cells that produce pili encoded by the *Streptococcus pneumoniae* pilus II island (INV104B) to enzymatic digestion (*e.g.,* mutanolysin) or mechanical shearing (*e.g.,* ultrasonication) and isolating the pili from the cells. In some embodiments, isolating includes a density gradient centrifugation. In some embodiments, isolating includes reduction of polydispersity, such as separating components by size, *e.g.,* using gel filtration chromatography.

In other aspects, the disclosure features antibodies that bind specifically to a pilus encoded by the *Streptococcus pneumoniae* pilus II island (INV 104B). In some embodiments, the antibody is a monoclonal antibody, a polyclonal antibody, a chimeric antibody, a human antibody, a humanized antibody, a single-chain antibody, or a Fab fragment.

In other aspects, the disclosure features an immunogenic composition comprising a purified *Streptococcus pneumoniae* pilus II island (INV104B) polypeptide in oligomeric form. In some embodiments, the polypeptide is a hyperoligomer. In other embodiments, the polypeptide is a fragment of a LPXTG cell wall anchored protein encoded by the *Streptococcus pneumoniae* pilus II island (INV104B).

In further aspects, the disclosure features methods of inducing an immune response against *Streptococcus pueumoniae.* In some embodiments, the methods comprise administering an effective amount of pili encoded by the *Streptococcus pneumoniae* pilus II island (INV104B) to a subject.

In other aspects, the disclosure features methods of detecting a *Streptococcus pneumoniae* infection in a subject. In some embodiments, the methods comprise assaying a sample from the subject for the presence of an antibody to pili encoded by the *Streptococcus pneumoniae* pilus II island (INV104B).

In other aspects, the disclosure features methods of detecting a *Streptococcus pneumoniae* infection in a subject. In some embodiments, the methods comprise contacting a sample with an antibody and detecting binding of the antibody to a component of the sample. In some embodiments, the antibody binds to a pili component. In other embodiments, the antibody binds to a pili complex.

In other aspects, the disclosure features methods of treating a subject having a *Streptococcus pneumoniae* infection. In some embodiments, the methods comprise administering to the subject an effective amount of an agent that binds specifically to pili encoded by the *Stieptococcus pneumoniae* pilus II island (INV104B). In some embodiments, the agent is an antibody. In some embodiments, the antibody blocks attachment of *Streptococcus pneumoniae* to cells. In some embodiments, the antibody specifically binds to one or more LPXTG cell wall anchored proteins encoded by the *Streptococcus pneumoniae* pilus II island (INV104B).

In other aspects, the disclosure features methods of determining the course of treatment for a subject having a *Streptococcus pneumoniae* infection. In some embodiments, the method comprises assaying a sample from the subject for the presence of an antibody to pili encoded by the *Streptococcus pneumoniae* pilus II island (INV 104B), and administering to the subject an anti-inflammatory agent if the presence of the antibody is detected. In other embodiments, the method comprises assaying a sample from the subject for the presence of an antibody to pili encoded by the *Streptococcus pneumoniae* pilus II island (INV104B), and administering to the subject an antibiotic agent if the presence of the antibody is not detected.

In other aspects, the disclosure features isolated pilus or pilus-like multimers that comprise an amino acid sequence of a pilus protein encoded by the *Streptococcus pneumoniae* pilus II island (INV104B) that has up to 30 amino acid substitutions, insertions, or deletions. In some embodiments, the amino acid sequence has up to 20 amino acid substitutions, insertions, or deletions. In other embodiments, the amino acid sequence has up to 10 amino acid substitutions, insertions, or deletions. In still other embodiments, amino acid sequence has up to 5 amino acid substitutions, insertions, or deletions.

In further aspects, the disclosure features polypeptides that have the amino acid sequence of one or more LPXTG cell wall anchored proteins encoded by the *Streptococcus pneumoniae* pilus II island (INV 104B). In other aspects, the disclosure features immunogenic fragments of one or more LPXTG cell wall anchored proteins encoded by the *Streptococcus pneumoniae* pilus II island (INV104B). In other aspects, the disclosure features polynucleotides that encode polypeptides that have the amino acid sequence of one or more LPXTG cell wall anchored proteins encoded by the *Streptococcus pneumoniae* pilus II island (INV104B).

In other aspects, the disclosure features purified polypeptides with the amino acid sequence of SEQ ID NO:2, 4, 6, 7, 8, and 9. In other aspects, the disclosure features purified polypeptides having ten consecutive residues of SEQ ID NO:2, 4, 6, 7, 8, and 9. In still other aspects, the disclosure features purified polypeptides with amino acid sequences at least 85% identical to SEQ ID NO:2, 4, 6, 7, 8, and 9.

In additional aspects, the disclosure features purified polypeptides with at least 85% sequence identity to a sequence selected from the group consisting of SEQ ID NO:29 through SEQ ID NO: 1742, or immunogenic fragments thereof. In other aspects, the disclosure features purified polypeptides with an amino acid sequence selected from the group consisting of SEQ ID NO:29 through SEQ ID NO: 1742, or immunogenic fragments thereof.

In some embodiments, the disclosure features purified OCX141 polypeptides with at least 85% sequence identity to a sequence selected from the group SEQ ID NO: 53, SEQ ID NO: 65, SEQ ID NO: 70, SEQ ID NO: 99, SEQ ID NO: 104, SEQ ID NO: 117, SEQ ID NO: 135, SEQ ID NO: 177, SEQ ID NO: 178, SEQ ID NO: 198, SEQ ID NO: 235, SEQ ID NO: 236, SEQ ID NO: 237, SEQ ID NO: 242, SEQ ID NO: 247, SEQ ID NO: 248, SEQ ID NO: 250, SEQ ID NO: 251, SEQ ID NO: 252, SEQ ID NO: 253, SEQ ID NO: 433, SEQ ID NO: 439, SEQ ID NO: 444, SEQ ID NO: 538, SEQ ID NO: 539, SEQ ID NO: 540, SEQ ID NO: 541, SEQ ID NO: 542, SEQ ID NO: 543, SEQ ID NO: 544, SEQ ID NO: 545, SEQ ID NO: 581, or SEQ ID NO: 593, and immunogenic fragments thereof.

In other embodiments, the disclosure features purified INV200 polypeptides with at least 85% sequence identity to a sequence selected from the group SEQ ID NO: 626, SEQ ID NO: 628, SEQ ID NO: 629, SEQ ID NO: 630, SEQ ID NO: 631, SEQ ID NO: 632, SEQ ID NO: 639, SEQ ID NO: 645, SEQ ID NO: 747, SEQ ID NO: 751, SEQ ID NO: 752, SEQ ID NO: 783, SEQ ID NO: 786, SEQ ID NO: 787, SEQ ID NO: 810, SEQ ID NO: 812, SEQ ID NO: 813, SEQ ID NO: 824, SEQ ID NO: 831, SEQ ID NO: 842, SEQ ID NO: 847, SEQ ID NO: 875, SEQ ID NO: 876, SEQ ID NO: 879, SEQ ID NO: 880, SEQ ID NO: 882, SEQ ID NO: 913, SEQ ID NO: 914, SEQ ID NO: 925, SEQ ID NO: 926, SEQ ID NO: 947, SEQ ID NO: 948, SEQ ID NO: 968, SEQ ID NO: 987, SEQ ID NO: 988, SEQ ID NO: 990, SEQ ID NO: 992, SEQ ID NO: 1003, SEQ ID NO: 1007, SEQ ID NO: 1008, SEQ ID NO: 1036, SEQ ID NO: 1082, SEQ ID NO: 1120, or SEQ ID NO: 1123, and immunogenic fragments thereof.

In further embodiments, the disclosure features purified 23F polypeptides with at least 85% sequence identity to a sequence selected from the group SEQ ID NO: 1297, SEQ ID NO: 1309, SEQ ID NO: 1311, SEQ ID NO: 1343, SEQ ID NO: 1362, SEQ ID NO: 1364, SEQ ID NO: 1434, SEQ ID NO: 1451, SEQ ID NO: 1455, SEQ ID NO: 1466, SEQ ID NO: 14678, SEQ ID NO: 1470, SEQ ID NO: 1474, SEQ ID NO: 1484, SEQ ID NO: 1485, SEQ ID NO: 1456, SEQ ID NO: 1487, or SEQ ID NO: 1491, and immunogenic fragments thereof.

In other aspects, the disclosure features immunogenic fragments of an LPXTG cell wall anchored protein encoded by the *Streptococcus pneumoniae* pilus II island (INV 104B).

In further aspects, the disclosure features isolated nucleic acids with the polynucleotide sequence of SEQ ID NO:1, 3, and 5. In other aspects, the disclosure features isolated nucleic acids that hybridize under stringent conditions to a hybridization probe, wherein the probe has the polynucleotide sequence of SEQ ID NO:1, 3, and 5 or the complement of SEQ ID NO:1, 3, and 5. In still other aspects, the disclosure features an isolated nucleic acid having a sequence that encodes an amino acid sequence that is at least 85% identical to SEQ ID NO:2, 4, 6, 7, 8, and 9.

In other aspects, the disclosure features isolated nucleic acids having a sequence that encodes an amino acid sequence that is at least 85% identical to a sequence selected from the group consisting of SEQ ID NO: 29 though SEQ ID NO: 1742. In other aspects, the disclosure features isolated nucleic acids having a sequence that encodes an amino acid sequence that is selected from the group consisting of SEQ ID NO: 29 though SEQ ID NO: 1742.

In other aspects, the disclosure features methods of inducing an immune response against *Streptococcus pneumoniae.* In some embodiments, the methods include administering an effective amount of an immunogenic fragment of a LPXTG cell wall anchored protein encoded by the *Streptococcus pneumoniae* pilus II island (INV104B) to a subject. In some embodiments, the subject is a human.

In other aspects, the disclosure features an antibody to a pilus protein encoded by the *Streptococcus pneumoniae* pilus II island (INV104B) in a cell. In some embodiments, the methods include expressing a nucleic acid encoding the antibody to the pilus protein encoded by the *Streptococccrs pneumoniae* pilus II island (INV104B) in the cell. In some embodiments, the pilus protein is a LPXTG cell wall anchored protein.

In still other aspects, the disclosure features methods of purifying *Streptococcus pneumoniae* from a sample comprising *Streptococcus pneumoniae.* These methods include: providing an affinity matrix comprising an antibody bound to a solid support; contacting the sample with the affinity matrix to form an affinity matrix- *Streptococcus pneumoniae* complex; separating the affinity matrix-*Streptococcus pneumoniae* complex from the remainder of the sample; and releasing *Streptococcus pneumoniae* from the affinity matrix.

In further aspects, the disclosure features methods of delivering a cytotoxic agent or a diagnostic agent to *Streptococcus pneumoniae.* These methods include: providing the cytotoxic agent or the diagnostic agent conjugated to an antibody or fragment thereof; and exposing the *Streptococcus pneumoniae* to the antibody-agent or fragment-agent conjugate.

In other aspects, the disclosure features methods of identifying a binding modulator for pili encoded by the *Streptococcars pneumoniae* pilus II island (INV104B). These methods include contacting an animal cell susceptible to *Streptococcus pneumoniae* pili binding with a candidate compound and a bacterial cell having pili encoded by the *Streptococcus pneumoniae* pilus II island (INV104B), and determining whether binding of the bacterial cell to the animal cell is inhibited. In some embodiments, inhibition of the binding activity is indicative of an inhibitor of binding by pili encoded by the *Streptococcus pneumoniae* pilus II island (INV104B).

In other aspects, the disclosure features methods of identifying binding modulators to the activities of pili encoded by the *Streptococcus pneumoniae* pilus II island (INV 104B). These methods include contacting an cell susceptible to *Stieptococcus pneumoniae* pili binding with a candidate compound and a pili encoded by the *Streptococcus pneumoniae* pilus II island (INV104B), and determining whether binding of the pili to the cell is inhibited. In some embodiments, inhibition of binding activity is indicative of an inhibitor of binding by pili encoded by the *Streptococcus pneumoniae* pilus II island (INV104B).

In further aspects, the disclosure features methods of identifying a binding modulator for pili encoded by the *Streptococcus pneumoniae* pilus II island (INV1048). These methods include contacting an cell susceptible to *Streptococcus pneumoniae* pili binding with a candidate compound and a pilus protein or cell binding fragment thereof encoded by the *Streptococcus pneumoniae* pilus II island (INV104B), and determining whether binding of the pilus protein or cell binding fragment thereof to the cell is inhibited. In some embodiments, inhibition of binding activity is indicative of an inhibitor of binding by pili encoded by the *Streptococcus pneumoniae* pilus II island (INV104B).

In still other aspects, the disclosure features methods of isolating pili encoded by the *Streptococcus pneumoniae* pilus II island (INV104B). These methods include subjecting *Streptococcus pneumoniae* cells that produce pili encoded by the *Streptococcus pneumoniae* pilus II island (INV104B) to ultrasonication or digestion with a lytic enzyme; separating non-cellular components by density gradient centrifugation; and isolating pili encoded by the *Streptococcus pneumoniae* pilus II island (INV104B). In some embodiments, the lytic enzyme is mutanolysin. In other embodiments, the *Streptococcus pneumoniae* cells that produce pili encoded by the *Streptococcus pneumoniae* pilus II island (INV104B) are *Streptococcus pneumoniae* TIGR4 cells

Unless otherwise defined, all technical and scientific terms used herein have the same meaning as commonly understood by one of ordinary skill in the art. Although methods and materials similar or equivalent to those described herein can be used in the practice or testing of the present invention, suitable methods and materials are described below. In case of conflict, the present specification, including definitions, will control .

The details of one or more embodiments of the invention are set forth in the accompanying figures and the description below. Other features, objects, and advantages of the invention will be apparent from the description and figures, and from the additional embodiments below.

### BRIEF DESCRIPTION OF THE FIGURES

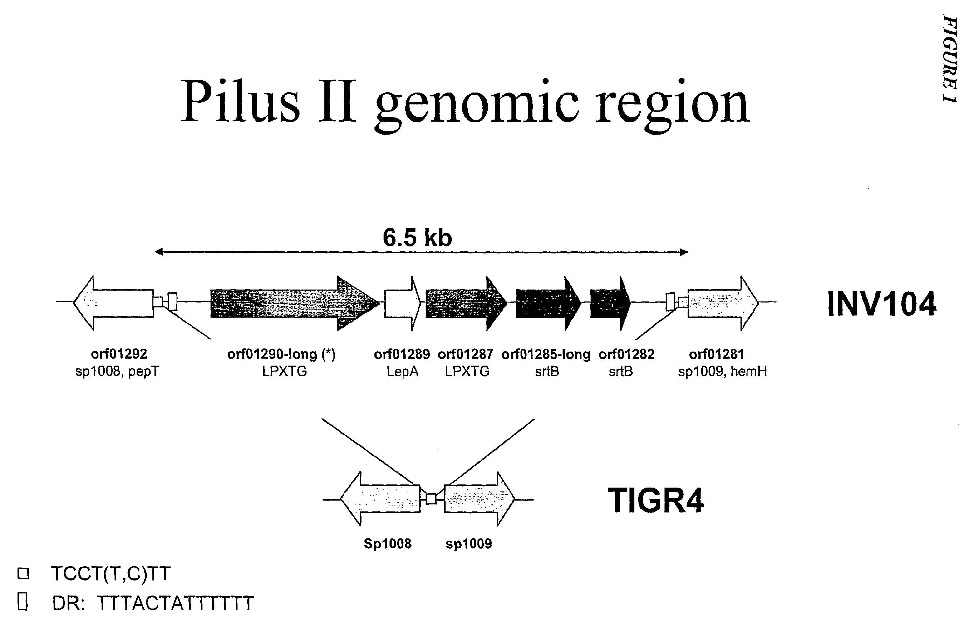

Fig 1. is a diagram showing the *S. pneumoniae* pilus II island (INV104B).

### DESCRIPTION

The Applicants have identified new polypeptide sequences from *Streptococcus pneumoniae* (also known as pneumococcus), including new pili polypeptides and other polypeptides useful as antigens. New pili polypeptide sequences were identified in the unfinished genome sequence for *S. pneunzoniae* isolate INV104, as sequenced by The Institute for Genomic Research (*see worldwide web site* "tigr.org"). These pili polypeptide sequences are encoded by a pathogenicity island, referred to herein as the pilus II island (INV104B), which is present in some, but not all, clinical pneumococcal isolates. Pili are important for pneumococcal adherence to lung epithelial cells as well as for colonization. Additionally, the Sanger partial genomes for *S. pneumoniae* strains 23F, INV200, and OXC141 were analyzed (*see worldwide web site* "sanger.ac.uk/Projects/Microbes/") to identify genes encoding polypeptides absent from INV104, the results of which included additional pili polypeptides as well as a large number of other non-INV104 polypeptides. Accordingly, this disclosure features, inter alia, *S. pneumoniae* pili, pilus, and other polypeptide compositions and the use of the same in methods of treatment for, diagnosis of, and immunization against *S. pneumoniae* infections. As used herein the term *S. pneumoniae* Polypeptide(s) is meant to include *S. pneumoniae* pilus II island (INV 104B) pili polypeptides, pili polypeptides from 23F, INV200, and OXC141, and other polypeptides from *S. pneumoniae.* Also as used herein the term *S. pneumoniae* Pilus Polypeptide(s) is meant to include *S. pneumoniae* pilus II island (INV 104B) pili polypeptides and pili polypeptides from 23F, INV200, and OXC141.

### S. pneumoniae Pili Of The Pilus II Island Of INV 104B

Pneumococcal pili encoded by a 6.5kb insert between genes corresponding to sp 1008 and sp1009 of *S. pneumoniae* isolate INV104B (ST227, serotype 1) of strain TIGR4 (as illustrated by Fig. 1) are described herein. This region of *S. pneumoniae* isolate INV 104B is referred to herein as the *"S. pneumoniae* pilus II island (INV104B)." The *S. pneumoniae* pilus II island (INV104B) encodes three LPXTG cell wall anchored proteins (herein referred to as LPXTG-1, LPXTG-1A, and LPXTG-2), a LepA peptidase (orf01289; SEQ ID NO:673), and two predicted sortases (herein referred to as sort-1 (SEQ ID NO:676) and sort-2 (SEQ ID NO: 1123)). See Example 4 for these LepA and sortase sequences.

An exemplary nucleic acid sequence for LPXTG-1 (orf01290-long) is hereby provided:

ATGAACGTTCAATATGATTTTAAGAAGATTCAATATTTTACCAGTAGTTTAGTT
ATCTTTCTCGCTATTCTTTTTTTGTGTGCACCAATTAATTCTTTACGTGCAGAT
TCAATAACTGAACCTCAGACAACTCTGCACAAAACGATTACTCCGATATCAGGG
CAAAAAGACCAGTATGAGTTGTCACTGGATATCACATCTAAACTGGGAACGGAG
ACCCAGTCAGAACCCTTGGATGTAGTCTTGGTTGCCGATCTTTCAGGGAGTATG
GAAGAGCGAGATGTGTGGTCTTACTCTAGTAGACGATACATTAGTAGGATTGAA
GCACTAAAACATACACTGAAAGGTGTGAATGGTCGTCAGGGGCTCATTGATACA
ATTCTTTCTAATTCCCAAAACCGTCTGTCTATAGTTGGTTTTGCCGGAAAGATT
GATAATCAGTATAATGACCGTTATTATAATGAATATTATCTGAGTTATCAATAT
GGAACTTGGCCAAATTGAGCTGGTTGGTATTCAAATATCTCTTCATATGATGAT

GCTAAAACTTTAGTATCTTGGAGCACGGATTCTAATAGCTCAAAAAATATTGTT
AGTTCGTTAACAATTGCTGACTCTAGTCGTTCTTATGGTATGGACGCGGCATT
GGCACTGGGACAAATATAAATGCTGGGTTAACTGAAGCTCAAAGATTGTTGCAA
AGTGCAAGGGCTGGGGCAAAAAAAGTAGTTATTCTGCTGTCAGATGGCGAAGCT
AATATGTATTACGAGTCTAATAGTGGGAGAACAATATATAACTATTATTCTAAT
CCAAATGTGGGACGTATGATTGATACTCCATATTGGTTTACCTCTGGTTTAGAG
AGAGGAATGCTGAATATATCTAGTTTAATAGCTCCAAAAATAGATGGCTTTTAT
TCAATCAAATTCAGATATATAGGTTCAAACGATAGTATCACATCTCTTAAAGGA
TATATCAGTGGTTATAATTCTGGAATCCCCAACGAAATATTTTCTGCCAATAAT
GAAAATGACTTGCAACAAAAATTCAAAGAAATCACAGATAAAATTCTACCTCTA
GGCGTACACCATGTAACTATATCAGATGTCTTGTCCAAGTACGTGCAGCTGTTA
CCTGGTGATGCTTCACACCTTCGTGTCGTCAAAATCAAGGATGGTAACGAGCAA
GAACTGAATGACAATCAAGTTACGATTGAAACTAAGAAGAACGAACAGGATTA
GTGGAAGTAACAGCCAAGTTTAATCCGAGTTACACTTTGGAGGATGACGCCAAG
TACGTTCTCAAGTTTACTGTCACCTCTAGCCAAGAGGCATTTGATGCGATTGCG
GGTGATAAGACACTTACTAGTGATGATGCCGAAGAAGCCGATGCTACTAAACTC
TACTCCAACAAGGGGGCAAAAGTTGCCTATTCCTATGGTATTGGACCTCACGT
ACCAAAATAAAAGACTATTCTGAGAAGCCCACTTTCAAGCCGTCAGATCCATTG
ACGGTTCCTGTAGAGATTGAGTGGAAAGGTGTGGATGGAAAATCAAATCCATCA
GCAAATCGTCCACCTAGTGTCGAATTAAACTTAAACCAAAAGAAAGATGGAAGT
ATAAAGGATTCCTATCGAAAGGTCACTAGTCCAGTTCAAACGAATAGTTTTACT
GAAAATACTAGTTTTGCAAAGGTAGCTAAGGGATATGACTACGAACTGAAAGCA
CCAGACGCTCCGGGATACACAGTCGAAGTTCAAAAGACAGGTACGAAAGAGAAA
CCATCCTTCAAAGTTATTTACCGACAGCTTCCAAGTCTCACCGTAAAGAAAATC
CTAGAAGGTGAACAATCACCTAATAAATCTTTCACAATTAATGTTACCTTTTCA
GATAAGGATGGCAAGCCGATTAACGGCAAGTTTGGAATACAACAGTGACTAAC
GGGAAAGCACAGATTTCTCTCAAAAATAGTCAGGAAACTGCCCTCAGTTATCTG
CCTCGTGATACCCACTATAAGGTGGAAGAAGTAGAGAACTCTAGAACGGATAT
CATGTCACCTATGAAAAACAAGAGGGGACTTTGTCAGAGGATGTTCAAACAATC
GTCACCAACCACAGACTTCCGACACTTTCAGTCACAAAAAAAGTTACAGGTGCT
TTTGCTAATCTTCTGCAATCCTTTAAGATTACCATTAACGTAAAGGATGCGCAA
AATAAACCATTGAATGGATCGTATAGTGCAATAGTAAATAATCAAAAAACAACG
CTACAATTCACCAATGGTAAGGCGACAGTTGATCTAAAGAAAGATAAAACCATC
AAGATTCTCGACCTTCCTCTAAATGCTCGTTATAGTATCGAAGAAGAAGCAAGT
TCGTCTCGTGGGTATCAGGTGTCCTATGATAAAAAAGAAGGAACTCTTGATGCA
AATAAGTCTGCGACAGTCACGAATAATAAAAACAGCGTACCTGAAACGGAATT
GACTTCTTGAGTAGCACTCTCGTGCTTGGAGTCGTTCTTCCTCTAGGAGGGATC
TTCTTTATCATCTTACTTGGTCACCTTGTGGTGAATAGGAGGAA (SEQ ID
NO:1)

An exemplary amino acid sequence for LPXTG-1 is hereby provided:

MNVQYDFKKIQYFTSSLVIFLAILFLCAPINSLRADSITEPQTTLHKTITPISG
QKDQYELSLDITSKLGTETQSEPLDVVLVADLSGSMEERDVWSYSSRRYISRIE
ALKHTLKGVNGRQGLIDTILSNSQNRLSIVGFAGKIDNQYNDRYYNEYYLSYQY
GTWPN*AGWYSNISSYDDAKTLVSWSTDSNSSKNIVSSLTIADSSRSYGMDAGI
GTGTNINAGLTEAQRLLQSARAGAKKVVILLSDGEANMYYESNSGRTIYNYYSN
PNVGRMIDTPYWFTSGLERGMLNISSLIAPKIDGFYSIKFRYIGSNDSITSLKG
YISGYNSGIPNEIFSANNENDLQQKFKEITDKILPLGVHHVTISDVLSKYVQLL
PGDASHLRVVKIKDGNEQELNDNQVTIETKKNEQGLVEVTAKFNPSYTLEDDAK
YVLKFTVTSSQEAFDAIAGDKTLTSDDAEEADATKLYSNKGAKVAYSYGIGTSR
TKIKDYSEKPTFKPSDPLTVPVEIEWKGVDGKSNPSANRPPSVELNLNQKKDGS
IKDSYRKVTSPVQTNSFTENTSFAKVAKGYDYELKAPDAPGYTVEVQKTGTKEK
PSFKVIYRQLPSLTVKKILEGEQSPNKSFTINVTFSDKDGKPINGKFGNTTVTN

GKAQISLKNSQETALSYLPRDTHYKVEEVENSRTGYHVTYEKQEGTLSEDVQTI
VTNHRLPTLSVTKKVTGAFANLLQSFKITINVKDAQNKPLNGSYSAIVNNQKTT
LQFTNGKATVDLKKDKTIKILDLPLNARYSIEEEASSSRGYQVSYDKKEGTLDA
NKSATVTNNKNSVPETGIDFLSSTLVLGVVLPLGGIFFIILLGHLVVNRRK
(SEQ ID NOs:769 and 2)

LPXTG-1 contains a sortase substrate motif, VPXTG (SEQ ID NO: 16), shown in underscore in SEQ ID NO:2, above.

The orf01290-long sequence (SEQ ID NO:1) has an intermediary stop codon that is bolded and underscored in SEQ ID NO:1. An exemplary nucleic acid sequence halted at this stop codon (orf01290-short) would have the following transcribed gene sequence (LPXTG-1A):

ATGGACGCGGGCATTGGCACTGGGACAAATATAAATGCTGGGTTAACTGAAGCT
CAAAGATTGTTGCAAAGTGCAAGGCTGGGGCAAAAAAAGTAGTTATTCTGCTG
TCAGATGGCGAAGCTAATATGTATTACGAGTCTAATAGTGGGAGAACAATATAT
AACTATTATTCTAATCCAAATGTGGGACGTATGATTGATACTCCATATTGGTTT
ACCTCTGGTTTAGAGAGAGGAATGCTGAATATATCTAGTTTAATAGCTCCAAAA
ATAGATGGCTTTTATTCAATCAAATTCAGATATATAGGTTCAAACGATAGTATC
ACATCTCTTAAAGGATATATCAGTGGTTATAATTCTGGAATCCCCAACGAAATA
TTTTCTGCCAATAATGAAAATGACTTGCAACAAAAATTCAAAGAAATCACAGAT
AAAATTCTACCTCTAGGCGTACACCATGTAACTATATCAGATGTCTTGTCCAAG
TACGTGCAGCTGTTACCTGGTGATGCTTCACACCTTCGTGTCGTCAAAATCAAG
GATGGTAACGAGCAAGAACTGAATGACAATCAAGTTACGATTGAAACTAAGAAG
AACGAACAGGGATTAGTGGAAGTAACAGCCAAGTTTAATCCGAGTTACACTTTG
GAGGATGACGCCAAGTACGTTCTCAAGTTTACTGTCACCTCTAGCCAAGAGGCA
TTTGATGCGATTGCGGGTGATAAGACACTTACTAGTGATGATGCCGAAGAAGCC
GATGCTACTAAACTCTACTCCAACAAGGGGGCAAAAGTTGCCTATTCCTATGGT
ATTGGACCTCACGTACCAAAATAAAAGACTATTCTGAGAAGCCCACTTTCAAG
CCGTCAGATCCATTGACGGTTCCTGTAGAGATTGAGTGGAAAGGTGTGGATGGA
AAATCAAATCCATCAGCAAATCGTCCACCTAGTGTCGAATTAAACTTAAACCAA
AAGAAAGATGGAAGTATAAAGGATTCCTATCGAAAGGTCACTAGTCCAGTTCAA
ACGAATAGTTTTACTGAAAATACTAGTTTTGCAAAGGTAGCTAAGGATATGAC
TACGAACTGAAAGCACCAGACGCTCCGGGATACACAGTCGAAGTTCAAAAGACA
GGTACGAAAGAGAAACCATCCTTCAAAGTTATTTACCGACAGCTTCCAAGTCTC
ACCGTAAAGAAAATCCTAGAAGGTGAACAATCACCTAATAAATCTTTCACAATT
AATGTTACCTTTTCAGATAAGGATGGCAAGCCGATTAACGGCAAGTTTGGAAT
ACAACAGTGACTAACGGAAAGCACAGATTTCTCTCAAAAATAGTCAGGAAACT
GCCCTCAGTTATCTGCCTCGTGATACCCACTATAAGGTGGAAGAAGTAGAGAAC
TCTAGAACGGGATATCATGTCACCTATGAAAAACAAGAGGGGACTTTGTCAGAG
GATGTTCAAACAATCGTCACCAACCACAGACTTCCGACACTTTCAGTCACAAAA
AAAGTTACAGGTGCTTTTGCTAATCTTCTGCAATCCTTTAAGATTACCATTAAC
GTAAAGGATGCGCAAAATAAACCATTGAATGGATCGTATAGTGCAATAGTAAAT
AATCAAAAAACAACGCTACAATTCACCAATGGTAAGGCGACAGTTGATCTAAAG
AAAGATAAAACCATCAAGATTCTCGACCTTCCTCTAAATGCTCGTTATAGTATC
GAAGAAGAAGCAAGTTCGTCTCGTGGGTATCAGGTGTCCTATGATAAAAAGAA
GGAACTCTTGATGCAAATAAGTCTGCGACAGTCACGAATAATAAAAACAGCGTA
CCTGAAACGGGAATTGACTTCTTGAGTAGCACTCTCGTGCTTGGAGTCGTTCTT
CCTCTAGGAGGGATCTTCTTTATCATCTTACTTGGTCACCTTGTGGTGAATAGG
AGGAA (SEQ ID NO:3)

An exemplary amino acid sequence for LPXTG-1A (from orf01290-short) is hereby provided:

MDAGIGTGTNINAGLTEAQRLLQSARAGAKKVVILLSDGEANMYYESNSGRTIY
NYYSNPNVGRMIDTPYWFTSGLERGMLNISSLIAPKIDGFYSIKFRYIGSNDSI
TSLKGYISGYNSGIPNEIFSANNENDLQQKFKEITDKILPLGVHHVTISDVLSK
YVQLLPGDASHLRVVKIKDGNEQELNDNQVTIETKKNEQGLVEVTAKFNPSYTL
EDDAKYVLKFTVTSSQEAFDAIAGDKTLTSDDAEEADATKLYSNKGAKVAYSYG
IGTSRTKIKDYSEKPTFKPSDPLTVPVEIEWKGVDGKSNPSANRPPSVELNLNQ
KKDGSIKDSYRKVTSPVQTNSFTENTSFAKVAKGYDYELKAPDAPGYTVEVQKT
GTKEKPSFKVIYRQLPSLTVKKILEGEQSPNKSFTINVTFSDKDGKPINGKFGN
TTVTNGKAQISLKNSQETALSYLPRDTHYKVEEVENSRTGYHVTYEKQEGTLSE
DVQTIVTNHRLPTLSVTKKVTGAFANLLQSFKITINVKDAQNKPLNGSYSAIVN
NQKTTLQFTNGKATVDLKKDKTIKILDLPLNARYSIEEEASSSRGYQVSYDKKE
GTLDANKSATVTNNKNSVPETGIDFLSSTLVLGVVLPLGGIFFIILLGHLVVNR
RK (SEQ ID NO:4)

LPXTG-1A contains a sortase substrate motif, VPXTG (SEQ ID NO: 16), shown in underscore in SEQ ID NO:4, above.

An exemplary nucleic acid sequence for LPXTG-2 (orf01287) is hereby provided:

TTGATGATCATAATGAAAAAAGAAAATAAAAAAACAAAAGAAATAATCATGAAA
AAAACATTCTTTAAAAAGCTATTCACTGCAAGCATTGCAGCTATAACCGCTTTG
TCCGTATTCAGAGGTGTCCCGACTTTTGCGGATGATAATTCAGCAATAACCAAA
GCAAATGGTGAAAATAATGCTGTTGTGAAGATTAATAAAACGTTGAATATTGCA
GAGGGAATAACAACACCAACAGCGACATTTACATTTAAGTTTACAGAAAAAACA
GGACAATCTTCTAACGGTGCGCCATATCAAACCGGAGTTGCAATTCCAGATAGA
AATGTAGAATACAATAAAAATGATCACCCAACTGCTGATAAGATTCAAAAAGCA
ACAGAAGACATTTTTTCGGGAGTTGCTTATGGCCATGCTGGTGAATACGTTTAT
GATGTAGCGGAAGCAAAAACTGGATGGCAGGCGATTACCAAAAATGGTAAAACA
ATTGATGCCATGAGATACGACAAACGTACATATGAAATGCACGTTATTGTTAAG
AATAAAGTAAATGGTGGTGTCTATATTTCATCAGTATACTTTAAGGAAAATAAT
AAATCTAACGCCCCTAAAGTAGAACCAAGTGAACAAGGCGTTTATAATTTATTT
GATAACACATATACCAAAGACGCAAGTAAGGAGCCTAATCCTGATGATCCGAGT
CAAGTAGACCCCAATGCGAAAGCATTAACAATTACTAAAAAAGTTGATGGAGCT
TCAGGGGATAAAACAAGAGATTTCCAATTCCATATCAAGATTCAACTTCCAAGT
ACAAATAAAACAGCAGAAACCCCTGTTACGAATATTATAGTAAAACATGGATCT
AAGTCAGAGGTGTTGGCAGTAGTGACCCCAGCAGATACAGTTGAGTACAATTTT
ACTCTTAAAGATGGTGAAACATTTACAGTTGAACAACTACCAGCAGGTTCTAAA
TATACAGTAACTGAAACTGGAGTAGCAGGTTATACAGATTCATCAATTTATACT
ACAAATGGTGCAGAACAAACATCTCAAGGACAAAAAAATGTAGATTTTACATTA
ACAGATATCCTCATAGGTGAAAAGAAAAACGACAACAAAGTTACTAACAAAATC
GACGACGTTACTCCTACTGGTCTCTTGATTGATAACCTTCCATTCATTTTGATG
ATTGGTCTTGGTTTGGCTGGATTTGTTGTCTTGTCTAAAAAACGTAGAGAAGCC
TA(SEQ ID NO:5)

An exemplary amino acid sequence for LPXTG-2 is hereby provided:

MIIMKKENKKTKEIIMKKTFFKKLFTASIAAITALSVFRGVPTFADDNSAITKA
NGENNAVVKINKTLNIAEGITTPTATFTFKFTEKTGQSSNGAPYQTGVAIPDRN
VEYNKNDHPTADKIQKATEDIFSGVAYGHAGEYVYDVAEAKTGWQAITKNGKTI
DAMRYDKRTYEMHVIVKNKVNGGVYISSVYFKENNKSNAPKVEPSEQGVYNLFD
NTYTKDASKEPNPDDPSQVDPNAKALTITKKVDGASGDKTRDFQFHIKIQLPST
NKTAETPVTNIIVKHGSKSEVLAVVTPADTVEYNFTLKDGETFTVEQLPAGSKY
TVTETGVAGYTDSSIYTTNGAEQTSQGQKNVDFTLTDILIGEKKNDNKVTNKID
DVTPTGLLIDNLPFILMIGLGLAGFVVLSKKRREA (SEQ ID NO:6)

LPXTG-2 contains a sortase substrate motif, VTXTG (SEQ ID NO:21), shown in underscore in SEQ ID NO:6, above.

### Polypeptide Sequences Identified In 23F, INV200, And OXC141 S. pneumoniae Strains

The Sanger partial genomes for *S. pneumoniae* strains 23F, INV200, and OXC141 do not contain the *S. pneumoniae* pilus II island (INV104B) region. However, 23F, INV200, and OXC 141 encode sortases and LPXTG cell wall anchored proteins, which are disclosed herein. For example, 23F and OXC141 each encode at least one sortase (sort-23F (orf01917; SEQ ID NO:1386) and sort-OXC141 (orf01672; SEQ ID NO:282)) and INV200 encodes at least three cell wall anchored proteins (Anchor-1, Anchor-2, and Anchor-3) that are not encoded by *S. pneumoniae* strain INV 104B.

An exemplary amino acid sequence for Anchor-1 (INV200-orf00426) is hereby provided:

MRVSSDTNIYEYRALSPQQKAALEMIRADLYKFTVPYENLEYRFYKPDWVFGLG
YQALATVRWKIEPATITVTKKWENVKEGAKKPDVWIQLLKDGKPEGERKRIESD
KGQTTFEIPNKDEINKYSVKEVDKEGRDWKHKDFTAGQPVNKGNGHFEITNTKK
EKPKIKVTFKKIAGDTNKDLAGAHLVLKKIFDDGNGLLIKQWDTIGQPVDIDLD
AGSYTLTEEKAPDGYMLAAPVSFYVEEDGQIILPKGEDLEAQNDKTITMVDEKI
KEKPTKPSGKLATTVEVDGTKADAQKELELSVATDKVTKTVKDTVVYENLLAGE
TYKLTGQLMKITADKEEEVATKETTFVADASGNGTTSLEFEDVSLEAGVKYVVY
ETAESEKEIDFKEGKEKHKVEHKDKDDKAQTVVVTKEKPTKPSGKLATTVEVDG
TKADAQKELELSVATDKVTKTVKDTVVYENLLAGETYKLTGQLMKITADKEEEV
ATKETTFVADASGNGTTSLEFEDVSLEAGVKYVVYETAESEKEIDFKEGKEKHK
VEHKDKDDKAQTVVVSKIKPEPGAQEVHFSKVNVGGEEIAGAEIHIKQGDTVVA
SWVSEAGKTHTLKLKPGHYIFHEAVAPGGYLAVTDIHFSVDETGQVTVTDVNGN
TAVAEGNKLTVTDQTKPVTPPSPEEPGAQEVHFSKVNVGGEEIAGAEIHIKQGD
TVVASWVSEAGKTHTLKLKPGHYIFHEAVAPGGYLAVTDIHFSVDETGQVTVTD
VNGNTAVAEGNKLTVTDQTKPVTPPSPEEPGAQEVHFSKVNVGGEEIAGAEIHI
KQGDTVVASWVSEAGKTHTLKLKPGHYIFHEAVAPGGYLAVTDIHFSVDETGQV
TVTDVNGNTAVAEGNKLTVTDQSADKDKQDKLPNTGETTGTYLSILGMITAVFA
SLLYRSKKK (SEQ ID NO:7)

Anchor-1 contains a sortase substrate motif, LPNTG (SEQ ID NO:10), shown in underscore in SEQ ID NO:7, above.

An exemplary amino acid sequence for Anchor-2 (INV200-orf00441) is hereby provided:

MNKGLFEKRCKYSIRKFSLGVASVMIGAAFFGTSPVLADSVQSGSTANLPADLA
TALATAKENDGRDFEAPKVGEDQGSPEVTDGPKTEEELLALEKEKPAEEKPKED
KPAAAKPETPKTVTPEWQTVEKKEQKGTVTIREEKGVRYNQLSSTAQNDNAGKP
ALFEKKGLTVVANGNATVDLTFKDDSEKGKSRFGVFLKFKDTNNNVFVGYDKDG
WFWEYKSPTTSTWYRGSRVAAPETGSTNRLSITLKSDGQLNASNNDVNLFDTVT
LPAAVNDHLKNEKKILLKAGSYGNDRTVVSVKTDNQEGVKADDTPAQKETGPVV
DDSKVTYDTIQSKVLKAVIDQAFPRVKEYSLNGHTLPGQVQQFNQVFINNHRIT
PEVTYKKINETTAEYLMKIRDDAHLINAEMTVRLQVVDNQLHFDVTKIVNHNQV
TPGQKIDDERKLLSSISFLGNALVSVSSDQTGAKFDGATMSNNTHVSGDDHIDV
TNPMKDLAKGYMYGFVSTDKLAAGVWSNSQNSYGGGSNDWTRLTAYKETVGNAN

YVGIHSSEWQWEKAYKGIVFPEYTKELPSAKVVITEDANADKKVDWQDGAIAYR
SIMNNPQGWEKVKDITAYRIAMNFGSQAQNPFLMTLDGIKKINLHTDGLGQGVL
LKGYGSEGHDSGHLNYADIGKRIGGVEDFKTLIEKAKKYGAHLGIHVNASETYP
ESKYFNEKILRKNPDGSYSYGWNWLDQGINIDAAYDLAHGRLARWEDLKKKLGD
GLDFIYVDVWGNGQSGDNGAWATHVLAKEINKQGWRFAIEWGHGGEYDSTFHHW
AADLTYGGYTNKGINSAITRFIRNHQKDAWVGDYRSYGGAANYPLLGGYSMKDF
EGWQGRSDYNGYVTNLFAHDVMTKYFQHFTVSKWENGTPVTMTDNGSTYKWTPE
MRVELVDADNNKVVVTRKSNDVNSPQYRERTVTLNGRVIQDGSAYLTPWNWDAN
GKKLSTDKEKMYYFNTQAGATTWTLPSDWAKSKVYLYKLTDQGKTEEQELTVKD
GKITLDLLANQPYVLYRSKQTNPEMSWSEGMHIYDQGFNSGTLKHWTISGDASK
AEIVKSQGANDMLRIQGNKEKVSLTQKLTGLKPNTKYAVYVGVDNRSNAKASIT
VNTGEKEVTTYTNKSLALNYVKAYAHNTRRDNATVDDTSYFQNMYAFFTTGADV
SNVTLTLSREAGDQATYFDEIRTFENNSSMYGDKHDTGKGTFKQDFENVAQGIF
PFVVGGVEGVEDNRTHLSEKHNPYTQRGWNGKKVDDVIEGNWSLKTNGLVSRRN
LVYQTIPQNFRFEAGKTYRVTFEYEAGSDNTYAFVVGKGEFQSGRRGTQASNLE
MHELPNTWTDSKKAKKATFLVTGAETGDTWVGIYSTGNASNTRGDSGGNANFRG
YNDFMMDNLQIEEITLTGKMLTENALKNYLPTVAMTNYTKESMDALKEAVFNLS
QADDDISVEEARAEIAKIEALKNALVQKKTALVADDFASLTAPAQAQEGLANAF
DGNVSSLWHTSWNGGDVGKPATMVLKEPTEITGLRYVPRGSGSNGNLRDVKLVV
TDESGKEHTFTATDWPDNNKPKDIDFGKTIKAKKIVLTGTKTYGDGGDKYQSAA
ELIFTRPQVAETPLDLSGYEAALAKAQKLTDKDNQEEVASVQASMKYATDNHLL
TERMVEYFADYLNQLKDSATKPDAPTVEKPEFKLSSLVSEQGKTPDYKQEIARP
ETPEQILPATGESQSDTSLFLASVSLALSALFVVKTKKD (SEQ ID NO:8)

Anchor-2 contains a sortase substrate motif, LPATG (SEQ ID NO: 1 0), shown in underscore in SEQ ID NO:8, above.

An exemplary amino acid sequence for Anchor-3 (INV200-orf03448) is hereby provided:

MRVSSDTNIYEYRALSPQQKAALEMIRADLYKFTVPYENLEYRFYKPDWVFGLG
YQALATVRWKIEPATITVTKKWENVKEGAKKPDVWIQLLKDGKPEGERKRIESD
KGQTTFEIPNKDEINKYSVKEVDKEGRDWKHKDFTAGQPVNKGNGHFEITNTKK
EKPKIKVTFKKIAGDTNKDLAGAHLVLKKIFDDGNGLLIKQWDTIGQPVDIDLD
AGSYTLTEEKAPDGYMLAAPVSFYVEEDGQIILPKGEDLEAQNDKTITMVDEKI
KEKPTKPSGKLATTVEVDGTKADAQKELELSVATDKVTKTVKDTVVYENLLAGE
TYKLTGQLMKITADKEEEVATKETTFVADASGNGTTSLEFEDVSLEAGVKYVVY
ETAESEKEIDFKEGKEKHKVEHKDKDDKAQTVVVTKEKPTKPSGKLATTVEVDG
TKADAQKELELSVATDKVTKTVKDTVVYENLLAGETYKLTGQLMKITADKEEEV
ATKETTFVADASGNGTTSLEFEDVSLEAGVKYVVYETAESEKEIDFKEGKEKHK
VEHKDKDDKAQTVVVTKEKPTKPSGKLATTVEVDGTKADAQKELELSVATDKVT
KTVKDTVVYENLLAGETYKLTGQLMKITADKEEEVATKETTFVADASGNGTTSL
EFEDVSLEAGVKYVVYETAESEKEIDFKEGKEKHKVEHKDKDDKAQTVVVTKEK
PTKPSGKLATTVEVDGTKADAQKELELSVATDKVTKTVKDTVVYENLLAGETYK
LTGQLMKITADKEEEVATKETTFVADASGNGTTSLEFEDVSLEAGVKYVVYETA
ESEKEIDFKEGKEKHKVEHKDKDDKAQTVVVSKIKPEPGAQEVHFSKVNVGGEE
IAGAEIHIKQGDTVVASWVSEAGKTHTLKLKPGHYIFHEAVAPGGYLAVTDIHF
SVDETGQVTVTDVNGNTAVAEGNKLTVTDQTKPVTPPSPEEPGAQEVHFSKVNV
GGEEIAGAEIHIKQGDTVVASWVSEAGKTHTLKLKPGHYIFHEAVAPGGYLAVT
DIHFSVDETGQVTVTDVNGNTAVAEGNKLTVTDQTKPVTPPSPEEPGAQEVHFS
KVNVGGEEIAGAEIHIKQGDTVVASWVSEAGKTHTLKLKPGHYIFHEAVAPGGY
LAVTDIHFSVDETGQVTVTDVNGNTAVAEGNKLTVTDQTKPVTPPSPEEPGAQE
VHFSKVNVGGEEIAGAEIHIKQGDTVVASWVSEAGKTHTLKLKPGHYIFHEAVA

PGGYLAVTDIHFSVDETGQVTVTDVNGNTAVAEGNKLTVTDQSADKDKQDKLPN
TGETTGTYLSILGMITAVFASLLYRSKKK (SEQ ID NO:9)

Anchor-3 contains a sortase substrate motif, LPNTG (SEQ ID NO:10), shown in underscore in SEQ ID NO: 9, above.

23F, INV200, and OXC141 also encode additional polypeptide sequences that are not encoded by *S. pneumoniae* strain INV104B. These additional polypeptide sequences are disclosed herein in Example 2 as specific examples of sequences that can be used in the methods described herein and as antigens in immunogenic compositions for the production of antibodies and/or the stimulation of an immune response in a subject.

### Other Bacterial Pili Polypeptides And Polypeptides From S. pneumoniae

The methods and compositions described herein can be used with pili polypeptides or other polypeptides from any Gram-positive bacterium including, for example, *S. pneumoniae.* Known and putative pili proteins have been identified in GAS (*e.g., Streptococcus pyogenes*) (Mora et al., 2005, Proc. Natl. Acad. Sci. USA, 102:15641-6), GBS (*e.g., Streptococcus agalactiae*) (Lauer et al., 2005, Science, 309:105; WO 2006/078318), *Actinomycetes naeslundii* (Yeung et al., 1998, Infect. Immun., 66:14S2-91), *Corynebacterium diphtheriae* (Ton-That et al., 2003, Mol. Microbiol., 50:1429-38; Ton-That and Schneewind, 2004, Trends. Microbiol., 12:228-34), *Clostridium perfringens,* and *Enterococcus faecalis.* Examples of Gram-positive bacteria include, without limitation, firmicutes such as those of genera *Streptococccus* (*e.g., S. pneumoniae, S. agalactiae, S. pyogenes, S. suis, S. zooepidemicus, S. viridans, S. mutans, S. gordonii, S. equi.*), *Bacillus* (*e.g., B. anthracis, B. cereus, B. subtilis*), *Listeria* (*e.g., L. inmocua, L. monocytogenes*), *Staphylococcus* (*e.g., S. aureus, S. epidermidis, S. caprae, S. saprophyticus, S. lugdumensis, S. schleiferi*), *Enterococcus* (*e.g., E. faecalis, E. faecium*), *Lactobacillus, Lactococcus (e.g., L. lactis), Leuconostoc (e.g., L. mesenteroides), Pectinatus, Pediococcus, Acetobacterium, Clostridium* (*e.g., C. botulinum, C. difficile, C. perfringens, C tetani*), *Ruminococcus (e.g., R. albus*), *Heliobacterium, Heliospirillum,* and *Sporomusa;* and actinobacteria such as those of genera *Actinomycetes (e.g., A. naeslumdii*), *Corynebacterium* (*e.g., C. diphtheriae, C. efficient*), *Arthrobacter, Bifidobacterium* (*e.g., B. longum*), *Frankia, Micrococcus, Micromonospora, Mycobacterium (e.g., M. tuberculosis, M. leprae, M, bovis, M. africamum, M. microti), Nocardia* (*e.g., N. asteroides*), *Propionibacteriun,* and *Streptomyces* (*e.g., S. somaliensis, S. avermitilis, S. coelicolor*).

### Isolated Pili Polypeptides And Other Polypeptides From S. pneumoniae

Isolated *S. pneumoniae* Polypeptides, can be used in the methods described herein and as antigens in immunogenic compositions for the production of antibodies and/or the stimulation of an immune response in a subject. Examples of useful *S. pneumoniae* LPXTG cell wall anchor pili polypeptides include LPXTG-1, LPXTG-1A, LPXTG-2, Anchor-1, Anchor-2, and Anchor-3 (*i.e*., SEQ ID NOs:2, 4, 6, 7, S, and 9). Examples of useful *S*. *pneumoniae* sortase polypeptides include sort-1, sort-2, sort-23F, and sort-OXC141 (*i.e*., SEQ ID NOs:676, 1123, 1386, and 282). Variants of *S*. *pneumoniae* Polypeptides can also be used in the methods described herein and as antigens in immunogenic compositions for the production of antibodies and/or the stimulation of an immune response in a subject. For example, *S*. *pneumoniae* Polypeptides containing at least 80% sequence identity, *e.g.*, at least 85%, at least 90%, at least 95%, at least 98%, or at least 99%, with a *S*. *pneumoniae* Polypeptide sequence are also useful in the new methods. Furthermore, a *S. pneumoniae* Polypeptide sequence with up to 50, *e.g.,* 1, 3, 5, 10, 15, 20, 25, 30, or 40 amino acid insertions, deletions, or substitutions, *e.g*., conservative amino acid substitutions will be useful in the compositions and methods described herein.

The determination of percent identity between two amino acid sequences can be accomplished using the BLAST 2.0 program, which is available to the public at ncbi.nlm.nih.gov/BLAST. Sequence comparison is performed using an ungapped alignment and using the default parameters (BLOSUM 62 matrix, gap existence cost of 11, per residue gap cost of 1, and a lambda ratio of 0.85). The mathematical algorithm used in BLAST programs is described in Altschul et al., 1997, Nucleic Acids Research, 25:3359-3402.

As used herein, "conservative amino acid substitution" means a substitution of an amino acid in a polypeptide within an amino acid family. Families of amino acids are recognized in the art and are based on physical and chemical properties of the amino acid side chains. Families include the following: amino acids with basic side chains (*e.g*. lysine, arginine, and histidine); amino acids with acidic side chains (*e.g*., aspartic acid and glutamic acid); amino acids with uncharged polar side chains (*e.g*. glycine, asparagine, glutamine, serine, threonine, tyrosine, and cysteine); amino acids with nonpolar side chains (*e.g*. alanine, valine, leucine, isoleucine, proline, phenylalanine, methionine, and tryptophan); amino acids with branched side chains (*e.g*., threonine, valine, and isoleucine); and amino acids with aromatic side chains (*e.g.,* tyrosine, phenylalanine, tryptophan, and histidine). An amino acid can belong to more than one family.

Fragments of *S*. *pneumoniae* Polypeptides, *e.g*., immunogenic fragments, are also useful in the methods and compositions described herein. Typically, the fragments are at least 8, 10, 15, 20, 50, 100, 200, or 500 contiguous amino acid residues of a *S. pneumoniae* Polypeptide. Examples of *S*. *pneumoniae* LPXTG polypeptides with useful fragments include LPXTG-1, LPXTG-1A, LPXTG-2, Anchor-1, Anchor-2, or Anchor-3 (*e.g*., SEQ ID NO:2, 4, 6, 7, S, or 9). Non-limiting examples of *S*. *pneumoniae* sortase polypeptides with useful fragments include sort-1, sort-2, sort-23F, and sort-OXC141 (*i.e*., SEQ ID NOs:676, 1123, 1386, and 282). In some embodiments the fragments retain at least one biological activity of the full-length protein, such as covalent attachment to a peptidoglycan cell wall or the ability to cross-link to another fragment or protein through an LPXTG motif.

In some embodiments the immunogenic compositions described herein comprise one or more *S*. *pneumoniae* Pilus Polypeptides that may be formulated or purified in an oligomeric (pilus) form. In some embodiments, the oligomeric form is a hyperoligomer. In some embodiments the immunogenic compositions described herein comprise one or more *S*. *pneumoniae* Pilus Polypeptides that have been isolated in an oligomeric (pilus) form. The oligomer or hyperoligomer pilus structures comprising *S*. *pneumoniae* Pilus Polypeptides may be purified or otherwise formulated for use in immunogenic compositions.

One or more of the *S*. *pneumoniae* Polypeptides from *S*. *pneumoniae* open reading frame polynucleotide sequences may be replaced by a polynucleotide sequence coding for a fragment of the replaced ORF. In some embodiments, one or more of the *S*. *pneumoniae* Polypeptides from *S*. *pneumoniae* open reading frames may be replaced by a sequence having sequence homology to the replaced ORF.

One or more of the *S*. *pneumoniae* Pilus Polypeptide sequences typically include an LPXTG motif (such as LPXTG (SEQ ID NO:10)) or other sortase substrate motif. The LPXTG sortase substrate motif of a *S*. *pneumoniae* pilus protein may be generally represented by the formula X₁X₂X₃X₄G (SEQ ID NO:1746), wherein X₁ is an L, a V, an E, a Y, an I, or a Q; wherein X₂ is a P if X₁ is an L; wherein X₂ is a V if X₁ is a E or a Q; wherein X₂ is a V or a P if X₁ is a V; wherein X₃ is any amino acid residue; wherein X₄ is a T if X₁ is a V, E, or Q; and wherein X₄ is a T, S, or A if X₁ is an L. Non-limiting examples of LPXTG motifs include YPXTG (SEQ ID NO:11), IPXTG (SEQ ID NO:12), LPXSG (SEQ ID NO:13), VVXTG (SEQ ID NO:14), EVXTG (SEQ ID NO:15), VPXTG (SEQ ID NO:16), QVXTG (SEQ ID NO:17), LPXAG (SEQ ID NO:18), QVPTG (SEQ ID NO:19), FPXTG (SEQ ID NO:20) and VTXTG (SEQ ID NO:21).

The *S*. *pneumoniae* Pilus Polypeptides described herein can effect the ability of the *S*. *pneumoniae* bacteria to adhere to and invade epithelial cells. These pilus polypeptides may also affect the ability of *S*. *pneumoniae* to translocate through an epithelial cell layer. In some embodiments, one or more *S*. *pneumoniae* Pilus Polypeptides from *S. pneumoniae* are capable of binding to or otherwise associating with an epithelial cell surface or synthetic model thereof. In some embodiments one or more *S*. *pneumoniae* Pilus Polypeptides bind to or associate with one or more of fibrinogen, fibronectin, or collagen.

The *S. pneumoniae* sortase proteins are predicted to be involved in the secretion and anchoring of the LPXTG containing surface proteins. The *S*. *pneumoniae* pilus II island (INV104B) sortase proteins disclosed herein are encoded by genes (sort-1 and sort-2) found in the same 6.5kb insert between genes corresponding to sp1008 and sp1009 of TIGR4 as the LPXTG-1 and LPXTG-2 genes discussed above. 23F and OXC141 also encode sortases: sort-23F (SEQ ID NO:1386) and sort-OXC141 (SEQ ID NO:282). Sortase proteins and variants of sortase proteins useful in the methods described herein can be obtained from Gram-positive bacteria.

The *S. pneumoniae* Pilus Polypeptides can be covalently attached to the bacterial cell wall by membrane-associated transpeptidases, such as a sortase. The sortase may function to cleave the surface protein, preferably between the threonine and glycine residues of an LPXTG motif. The sortase may then assist in the formation of an amide link between the threonine carboxyl group and a cell wall precursor such as lipid II. The precursor can then be incorporated into the peptidoglycan via the transglycoslylation and transpeptidation reactions of bacterial wall synthesis. *See* Comfort et al., Infection & Immunity (2004) 72(5): 2710 - 2722.

In some embodiments, the compositions described herein comprise oligomeric, pilus-like structures comprising *S*. *pneumoniae* Pilus Polypeptides such as LPXTG-1, LPXTG-1A, LPXTG-2, Anchor-1, Anchor-2, or Anchor-3 (*e.g*., SEQ ID NO:2, 4, 6, 7, 8, or 9). The oligomeric, pilus-like structure may comprise numerous units of pilus poplypeptides. In some embodiments, the oligomeric, pilus-like structures comprise two or more pilus proteins. In some embodiments, the oligomeric, pilus-like structure comprises a hyper-oligomeric pilus-like structure comprising at least two (*e.g.,* 2, 3, 4, 5, 6, 7, 8, 9, 10, 11, 12, 13, 14, 15, 20, 25, 30, 35, 40, 45, 50, 60, 70, 80, 90, 100, 120, 140, 150, 200 or more) oligomeric subunits, wherein each subunit comprises a pilus protein or a fragment thereof. The oligomeric subunits may be covalently associated via a conserved lysine within a pilin motif. The oligomeric subunits may be covalently associated via an LPXTG motif, in some embodiments, via a threonine or serine amino acid residue.

*S. pneumoniae* Pilus Polypeptides or fragments thereof can be incorporated into the oligomeric, pilus-like structures described herein and will, in some embodiments, include a pilin motif. Oligomeric, pilus-like structures may be used alone or in combination. In some embodiments, the compositions described herein comprise a *S*. *pneumoniae* pilus II island (INV104B) pilus in oligomeric form. Further, in some embodiments the *S*. *pneumoniae* pilus II island (INV104B) pilus can be organized in a hyperoligomeric form.

### Methods of Purification of Pili

Pili encoded by *Streptococcus pneumoniae* can be purified from cells, such as bacterial cells, that express *Streptococcus pneumoniae* pili or pili-like structures by separating the pili from the cells, *e.g*., by mechanical shearing or enzymatic digestion, and isolating the separated pili.

Suitable bacterial cells for purification of pili include piliated Gram-positive bacterial strains that express *Streptococcus pneumoniae* Pili Polypeptides, non-piliated Gram-positive bacteria that have been transformed with one or more Gram-positive pilus proteins, such as *S*. *pneumoniae* LPXTG-1, LPXTG-1A, LPXTG-2, Anchor-1, Anchor-2, and Anchor-3 (*e.g.,* SEQ ID NOs:2, 4, 6, 7, 8, and 9), and Gram-negative or other cells transformed with one or more Gram-positive pilus proteins, such as *S*. *pneumoniae* LPXTG-1, LPXTG-1A, LPXTG-2, Anchor-1, Anchor-2, and Anchor-3 (*e.g*., SEQ ID NOs:2, 4, 6, 7, 8, and 9). Typically, a cell used for purification of pili will produce only the type or types of pili desired, *e.g*., endogenous or heterologous pili. For the production of heterologous pili, the cell can be altered, *e.g*., by mutation or recombinant DNA methods, to not produce endogenous pili. Typically, a pili-producing Gram-positive bacterial cell useful for purification will express one or more compatible sortases such that the pili are expressed on the cell surface. Examples of *S*. *pneumoniae* pilus II island (INV104B), 23F, INV200, and OXC141 LPXTG cell wall anchor polypeptides that could be purified include LPXTG-1, LPXTG-1A, LPXTG-2, Anchor-1, Anchor-2, and Anchor-3 (*e.g*., SEQ ID NOs:2, 4, 6, 7, 8, and 9). Examples of *S*. *pneumoniae* pilus II island (INV104B), 23F, INV200, and OXC141 sortases that could be purified include sort-1, sort-2, sort-23F, and sort-OXC141 (e.g., SEQ ID NOs:676, 1123, 1386, and 282).

Separation of pili from Gram-positive bacterial cells is typically accomplished by mechanical shearing, enzymatic digestion, decreasing or inhibiting sortase activity, or treatment with a compound that interferes with cell wall integrity. Mechanical shearing can physically remove the pili from the cells, whereas other methods can eliminate the point of attachment of the pili (*e.g*., by degradation of cell wall or pilus components). Following separation of the pili from the cells, the pili and cells can be separated, *e.g*., by centrifugation.

Non-limiting examples of mechanical shearing methods include ultrasonication, glass bead shearing, and mixing. Methods of sonication are discussed, for example, in Yamaguchi et al., 2004, Current Microbiol., 49:59-65. Methods of glass bead shearing are discussed, for example, in Levesque et al., 2001, J. Bacteriol., 183:2724-32. General methods of mechanical shearing are discussed, for example, in Wolfgang et al., 1998, Mol. Microbiol., 29:321-30; Trachtenberg et al., 2005, J. Mol. Biol., 346:665-676; Parge et al., 1990, J. Biol. Chem., 265:2278-35; Isaacson et al., 1981, J. Bacteriol., 146:784-9; Korhonen et al., 1980, Infect. Immun., 27:569-75; Hahn et al., 2002, J. Mol. Biol., 323:845-57; St. Geme et al., 1996, Proc. Natl. Acad. Sci. USA, 93:11913-18; Weber et al., 2005, J. Bacteriol., 187:2458-68; and Mu et al., 2002, J. Bacteriol., 184:4868-74.

Non-limiting examples of enzymes suitable for enzymatic digestion include cell-wall degrading enzymes such as mutanolysin, lysostaphin, and lysozymes. Methods of enzymatic digestion are discussed, for example, in Bender et al., 2003, J. Bacteriol., 185:6057-66; Ton-That et al., 2004, Mol. Microbiol., 53:251-61; and Ton-That et al., 2003, Mol. Microbiol., 50:1429-38. For downstream administration to subjects, one can use multiple enzymes to remove cell-wall components that may cause an undesired host reaction.

Non-limiting examples of methods of inhibiting or decreasing sortase activity include decreasing, for example, SrtA activity by introduction of a loss-of-function allele of SrtA, deleting the endogenous SrtA gene, expression of a nucleic acid that decreases SrtA expression (*e.g*., an antisense or miRNA), and treating the cells with a compound that inhibits SrtA activity (*see, e.g.,* Marrafini et al., Microbiol. Mol. Biol. Rev., 70:192-221, 2006).

Exemplary sortase A inhibitors include methane-thiosulfonates (e.g., MTSET and (2-sulfonatoethyl) methane-thiosulfonate) (Ton-That and Schneewind, J. Biol. Chem., 274:24316-24320, 1999), *p*-hydroxymercuribenzoic acid, glucosylsterol β-sitosterol-3-O-glucopyranol (Kim et al., Biosci. Biotechnol. Biochem., 67:2477-79, 2003), berberine chloride (Kim et al., Biosci. Biotechnol. Biochem., 65:421-24, 2004), peptidyl-diazomethane (LPAT-CHN2) (Scott et al., Biochem. J., 366:953-58, 2002), peptidyl-chloromethane (LPAT-CH2Cl), peptidyl-vinyl sulfone [LPAT-SO₂(Ph)] (Conolly et al., J. Biol. Chem., 278:34061-65, 2003), vinyl sulfones (e.g., di-, ethyl-, methyl-, and phenyl vinyl sulfones) (Frankel et al., J. Am. Chem. Soc., 126:3404-3405, 2004), LPXTG motif peptides with the threonine residue replaced by a phosphinate group (e.g., LPEΨ{PO₂H-CH₂}G) (Kruger et al., Bioorg. Med. Chem., 12:3723-29,2004), substituted (Z)-diaryl-acrylonitriles (Oh et al., J. Med. Chem., 47:2418-21, 2004), and extracts of various medicinal plants (Kim et al., Biosci. Biotechnol. Biochem., 66:2751-54, 2002).

Non-limiting examples of compounds that interfere with cell wall integrity include glycine and antibiotics such as penicillins (e.g., methicillin, amoxicillin, ampicillin), cephalosporins (e.g., cefalexin, cefproxil, cefepime), glycopeptides (e.g., vancomycin, teicoplanin, ramoplanin), and cycloserine.

Separated pili can be separated from other components by density, for example by using density gradient centrifugation. For example, the pili can be separated by centrifugation on a sucrose gradient.

Typically, a sample containing Gram-positive pili will contain pili oligomers of different molecular weights due to differing numbers of pilus protein subunits present in the pili. To reduce polydispersity, a sample containing Gram-positive pili can be separated by size. For example, a gel filtration or size exclusion column can be used. An ultrafiltration membrane can also be used to reduce polydispersity.

Gram-positive pili can also be isolated using affinity methods such as affinity chromatography. For example, a protein that binds specifically to a Gram-positive pilus, *e.g*., an antibody that binds specifically to a pilus component or an antibody that binds preferentially to pili, can be immobilized on a solid substrate (*e.g*., a chromatography substrate), then a sample containing Gram-positive pili can be exposed to the immobilized binding protein. Such affinity isolation methods can also be used to isolate, purify, or enrich preparations of cells that express Gram-positive pili.

Gram-positive pili can also be isolated using any other protein purification method known in the art, *e.g*., precipitations, column chromatography methods, and sample concentrations. Additional methods are described, *e.g*., in Ruffolo et al., 1997, Infect. Immun., 65:339-43. Methods of protein purification are described in detail in, *e.g.,* Scopes, R.K., Protein Purification: Principles and Practice, 3rd. ed., 1994, Springer, NY.

The presence of Gram-positive pili in fractions during purification can be followed by electrophoresis (*e.g*., polyacrylamide electrophoresis), measuring binding of an agent that specifically binds to the gram positive pili (*e.g*., an antibody against a pilus protein or an antibody that binds preferentially to pili), and/or measuring an activity of the pili such as protein or cell binding.

### Antibodies

The *S*. *pneumoniae* Polypeptides described herein may also be used to prepare antibodies specific to those *S*. *pneumoniae* Polypeptides. In some embodiments the antibodies are specific to an oligomeric or hyper-oligomeric form of a *S*. *pneumoniae* Pilus Polypeptide. The compositions described herein also include combinations of antibodies specific to *S*. *pneumoniae* pilus polypeptides and other polypeptides from *S*. *pneumoniae* selected to provide protection against an increased range of serotypes and strain isolates. For example, such a combination may comprise a first and second antibody, wherein the first antibody is specific to a first *S*. *pneumoniae* Polypeptide and the second antibody is specific to a second *S*. *pneumoniae* Polypeptide or non-*S*. *pneumoniae* Polypeptide.

The specific *S*. *pneumoniae* Polypeptide antibodies described herein include one or more biological moieties that, through chemical or physical means, can bind to or associate with an epitope of a *S*. *pneumoniae* Polypeptide. The antibodies described herein include antibodies which specifically bind to *a S. pneumoniae* Polypeptide. The compositions described herein include antibodies obtained from both polyclonal and monoclonal preparations, as well as the following: hybrid (chimeric) antibody molecules (*see, e.g.,* Winter et al. (1991) Nature 349: 293-299; and U.S. Patent No. 4,816,567; F(ab')2 and F(ab) fragments; Fv molecules (non-covalent heterodimers, *see, e.g.,* Inbar et al. (1972) Proc Natl Acad Sci USA 69:2659-2662; and Ehrlich et al. (1980) Biochem 19:4091-4096); single-chain Fv molecules (sFv) *(see, e.g.,* Huston et al. (1988) Proc Natl Acad Sci USA 85:5897-5883); dimeric and trimeric antibody fragment constructs; minibodies *(see, e.g.,* Pack et al. (1992) Biochem 31:1579-1584; Cumber et al. (1992) J Immunology 149B: 120-126); humanized antibody molecules *(see, e.g.,* Riechmann et al. (1988) Nature 332:323-327; Verhoeyan et al. (1988) Science 239:1534-1536; and U.K. Patent Publication No. GB 2,276,169, published 21 September 1994); and, any functional fragments obtained from such molecules, wherein such fragments retain immunological binding properties of the parent antibody molecule. The compositions described herein further include antibodies obtained through non-conventional processes, such as phage display.

The antibodies described herein can be polyclonal, monoclonal, recombinant, *e.g*., chimeric or humanized, fully human, non-human, *e.g*., murine, or single chain antibodies. Methods of making such antibodies are known. In some cases, the antibodies have effector function and can fix complement. The antibodies can also be coupled to toxins, reporter groups, or imaging agents.

In some embodiments, the specific *S*. *pneumoniae* antibodies described herein are monoclonal antibodies. These monoclonal antibodies include an antibody composition having a homogeneous antibody population. The monoclonal antibodies described herein may be obtained from murine hybridomas, as well as human monoclonal antibodies obtained using human rather than murine hybridomas. *See, e.g.,* Cote, et al. Monoclonal Antibodies and Cancer Therapy, Alan R. Liss, 1955, p 77.

Chimeric, humanized, *e.g*., completely human, antibodies are desirable for applications that include repeated administration, *e.g*., therapeutic treatment (and some diagnostic applications) of a human subject.

The antibodies described herein can also be used in the prophylactic or therapeutic treatment of *S*. *pneumoniae* infection. The antibodies may block the attachment or some other activity of *S*. *pneumoniae* on host cells. Additionally, the antibodies can be used to deliver a toxin or therapeutic agent such as an antibiotic to *S*. *pneumoniae* cells.

The antibodies described herein may be used in diagnostic applications, for example, to detect the presence or absence of *S*. *pneumoniae* Polypeptides in a biological sample. Anti-pili, pilus polypeptide or other polypeptide from *S*. *pneumoniae* antibodies can be used diagnostically to monitor protein levels in tissue as part of a clinical testing procedure, *e.g*., to determine the efficacy of a given treatment regimen. Detection can be facilitated by coupling (*i.e*., physically linking) the antibody to a detectable substance (*i.e*., antibody labeling). Examples of detectable substances include various enzymes, prosthetic groups, fluorescent materials, contrast agents, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; examples of contrast agents include electron dense materials useful for electron microscopy, such as gold particles, or magnetically active materials useful for magnetic resonance imaging, such as supermagnetic iron particles; an example of a luminescent material includes luminol; examples of bioluminescent materials include luciferase, luciferin, and aequorin, and examples of suitable radioactive material include ¹²⁵I, ¹³¹I, ³⁵S or ³H. Such diagnostic antibodies can be used in methods to detect the presence of piliated *S*. *pneumoniae* containing *Streptococcus pneumoniae* pilus II island (INV 104B) pili in an infected patient, *e.g*., by testing a sample from the patient. The course of treatment can then be selected based on the presence or absence of *Streptococcus pneumoniae* Polypeptides. For example, a patient infected with non-piliated *S*. *pneumoniae* could be treated with an antibiotic, whereas a patient infected with piliated *S*. *pneumoniae* containing *S*. *pneumoniae* Pili Polypeptides could also be treated with a pili-binding compound, such as an antibody, and/or an anti-inflammatory agent (*e.g*., IL-6 or an anti-TNF agent such as an anti-TNF antibody).

### Screening Assays

In some aspects, the methods described herein (also referred to herein as "screening assays") can be used to identify modulators, *i.e*., candidate compounds or agents identified from one or more test compounds (*e.g*., antibodies, proteins, peptides, peptidomimetics, peptoids, small inorganic molecules, small non-nucleic acid organic molecules, nucleic acids (*e.g.,* antisense nucleic acids, siRNA, oligonucleotides, or synthetic oligonucleotides), or other drugs) that inhibit an activity, *e.g*., a binding activity of *S*. *pneumoniae* Pili Polypeptides. Compounds thus identified can be used to modulate the binding activity of *S*. *pneumoniae* containing *S*. *pneumoniae* Pili Polypeptides or the attachment of such *S*. *pneumoniae* in a therapeutic protocol, to elaborate the biological function *S*. *pneumoniae.*

In some embodiments, assays are provided for screening test compounds to identify those that can bind to *S*. *pneumoniae* Pili Polypeptides or a portion thereof. Compounds that
bind to *S*. *pneumoniae* Pili Polypeptides can be tested for their ability to modulate an activity associated with *S*. *pneumoniae* pili such as attachment, infection, or an inflammatory response.

The test compounds used in the methods described herein can be obtained using any of the numerous approaches in combinatorial library methods known in the art, including:
biological libraries; peptoid libraries (libraries of molecules having the functionalities of peptides, but with a novel, non-peptide backbone, which are resistant to enzymatic degradation, but which, nevertheless, remain bioactive; *see, e.g.,* Zuckermann et al., 1994, J. Med. Chem., 37:2678-2685); spatially addressable parallel solid phase or solution phase libraries; synthetic library methods requiring deconvolution; the "one-bead one-compound" library method; and synthetic library methods using affinity chromatography selection. The biological library and peptoid library approaches are limited to peptide libraries, while the other four approaches are applicable to peptide, non-peptide oligomer, or small molecule libraries of compounds (Lam, 1997, Anticancer Drug Des., 12:145).

Examples of methods for the synthesis of molecular libraries can be found in the art, for example in: DeWitt et al. (1993, Proc. Natl. Acad. Sci. USA, 90:6909; Erb et al., 1994, Proc. Natl. Acad. Sci. USA, 91:11422; Zuckermann et al., 1994, J. Med. Chem., 37:2678; Cho et al., 1993, Science, 261:1303; Carrell et al., 1994, Angew. Chem. Int. Ed. Engl., 33:2059; Carell et al., 1994, Angew. Chem. Int. Ed. Engl., 33:2061; and in Gallop et al., 1994, J. Med. Chem., 37:1233).

Libraries of compounds may be presented in solution (*e.g*., Houghten, 1992, Biotechniques, 13:412-421), or on beads (Lam, 1991, Nature, 354:82-84), chips (Fodor, 1993, Nature, 364:555-556), bacteria (Ladner, U.S. Patent No. 5,223,409), spores (Ladner, U.S. Patent No. 5,223,409), plasmids (Cull et al., 1992, Proc. Natl. Acad. Sci. USA, 89:1865-1869), or on phage (Scott and Smith, 1990, Science, 249:386-390; Devlin, 1990, Science, 249:404-406; Cwirla et al., 1990, Proc. Natl. Acad. Sci. USA, 87:6378-6382; Felici, 1991, J. Mol. Biol., 222:301-310; and Ladner *supra*).

In some embodiments, the assay is a cell-based assay in which a cell, *e.g*., a bacterial cell, that expresses a *S*. *pneumoniae* Polypeptides or biologically active portion thereof is contacted with a test compound, and the ability of the test compound to modulate *S*. *pneumoniae* activity is determined, for example, by monitoring cell binding. The cell, for example, can be of mammalian origin, *e.g*., murine, rat, or human origin. The cell can be an epithelial cell, *e.g.,* a A549 lung epithelial cell.

The ability of the test compound to modulate *S. pneumoniae* Pili Polypeptides from binding to a ligand or substrate, *e.g*., a cell or a protein such as fibrinogen, fibronectin, or collagen can be evaluated, for example, by coupling the compound, *e*.*g*., the substrate, with a radioisotope or enzymatic label such that binding of the compound, *e.g*., the substrate, to *S*. *pneumoniae* pilus II island (INV104B) pili or pilus protein can be determined by detecting the labeled compound, *e.g.,* substrate, in a complex. Alternatively, *S*. *pneumoniae* Polypeptides can be coupled with a radioisotope or enzymatic label to monitor the ability of a test compound to modulate *S*. *pneumoniae* Polypeptide binding to a substrate in a complex. For example, compounds (*e.g., S. pneumoniae* Polypeptide binding partners) can be labeled with ¹²⁵I, ³⁵S, ^{14c}, or ³H, either directly or indirectly, and the radioisotope detected by direct counting of radioemission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound to interact with a *S*. *pneumoniae* Polypeptide with or without the labeling of any of the interactants can be evaluated. For example, a microphysiometer can be used to detect the interaction of a compound with a *S*. *pneumoniae* Polypeptide without labeling either the compound or the *S*. *pneumoniae* Polypeptide (McConnell et al., 1992, Science 257:1906-1912). As used herein, a "microphysiometer" (*e.g*., Cytosensor^{®}) is an analytical instrument that measures the rate at which a cell acidifies its environment using a light-addressable potentiometric sensor (LAPS). Changes in this acidification rate can be used as an indicator of the interaction between a compound and a *S. pneumoniae* Polypeptide.

In some embodiments, a cell-free assay is provided in which *a S. pneumoniae* Polypeptide or biologically active portion thereof is contacted with a test compound and the ability of the test compound to bind to the *S*. *pneumoniae* Polypeptide or biologically active portion thereof is evaluated. In general, biologically active portions of *a S. pneumoniae* Polypeptide to be used in the new assays include fragments that participate in interactions with other *S*. *pneumoniae* Polypeptide molecules.

Cell-free assays involve preparing a reaction mixture of the target gene protein and the test compound under conditions and for a time sufficient to allow the two components to interact and bind, thus forming a complex that can be removed and/or detected.

An interaction between two molecules can be detected using fluorescence energy transfer (FET) (*see, e.g*., Lakowicz et al., U.S. Patent No. 5,631,169 and Stavrianopoulos et al., U.S. Patent No. 4,868,103). A fluorophore label on the first 'donor' molecule is selected such that its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor.' Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g*., using a fluorimeter).

In some embodiments, determinng the ability of a *S*. *pneumoniae* Polypeptide to bind to a target molecule (*e.g.*, a fibrinogen, fibronectin, or collagen polypeptide or fragment thereof) can be accomplished using real-time Biomolecular Interaction Analysis (BIA) (*e.g*., Sjolander et al., 1991, Anal. Chem., 63:2338-2345 and Szabo et al., 1995, Curr. Opin. Struct. Biol., 5:699-705). "Surface plasmon resonance" or "BIA" detects biospecific interactions in real time, without labeling any of the interactants (*e.g*., BIAcore). Changes in the mass at the binding surface (indicative of a binding event) result in alterations of the refractive index of light near the surface (the optical phenomenon of surface plasmon resonance (SPR)), resulting in a detectable signal that can be used as an indication of real-time reactions between biological molecules.

In some embodiments, the target gene product or the test substance is anchored onto a solid phase. The target gene product/test compound complexes anchored on the solid phase can be detected at the end of the reaction. The target gene product can be anchored onto a solid surface, and the test compound, which is not anchored, can be labeled, either directly or indirectly, with a detectable labelsdiscussed herein.

Multiple target gene products can be anchored onto a solid phase using protein microarray technology, which is also known by other names including: protein chip technology and solid-phase protein array technology. Protein microarray technology is well known to those of ordinary skill in the art and is based on, but not limited to, obtaining an array of identified peptides or proteins on a fixed substrate, binding target molecules or biological constituents to the peptides, and evaluating such binding. *See, e.g.,* G. MacBeath and S. L. Schreiber, "Printing Proteins as Microarrays for High-Throughput Function Determination," Science 289(5485): 1760-1763, 2000. Microarray substrates include but are not limited to glass, silica, aluminosilicates, borosilicates, metal oxides such as alumina and nickel oxide, various clays, nitrocellulose, or nylon. The microarray substrates may be coated with a compound to enhance synthesis of a probe (*e.g*., a peptide) on the substrate. Coupling agents or groups on the substrate can be used to covalently link the first amino acid to the substrate. A variety of coupling agents or groups are known to those of skill in the art. Peptide probes can be synthesized directly on the substrate in a predetermined grid. Alternatively, peptide probes can be spotted on the substrate, and in such cases the substrate may be coated with a compound to enhance binding of the probe to the substrate. In these embodiments, presynthesized probes are applied to the substrate in a precise, predetermined volume and grid pattern, preferably utilizing a computer-controlled robot to apply probe to the substrate in a contact-printing manner or in a non-contact manner such as ink jet or piezo-electric delivery. Probes may be covalently linked to the substrate. In some embodiments, one or more control peptide or protein molecules are attached to the substrate. Control peptide or protein molecules allow determination of factors such as peptide or protein quality and binding characteristics, reagent quality and effectiveness, hybridization success, and analysis thresholds and success.

In some embodiments it is desirable to immobilize a *S*. *pneumoniae* Polypeptide, an anti-pilus or pilus protein antibody, or a *S*. *pneumoniae* Polypeptide binding protein to facilitate separation of complexed from uncomplexed forms of one or both of the proteins, as well as to accommodate automation of the assay. Binding of a test compound to a *S. pneumoniae* Polypeptide, or interaction of a *S. pneumoniae* Polypeptide with a target molecule in the presence or absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, glutathione-S-transferase/ pilus II island (INV104B) pilus protein fusion proteins or glutathione-S-transferase/target fusion proteins can be adsorbed onto glutathione Sepharose^{™} beads (Sigma Chemical, St. Louis, MO) or glutathione derivatized microtiter plates, which are then combined with the test compound or the test compound and either the non-adsorbed target protein or *S*. *pneumoniae* pilus II island (INV104B) pili or pilus protein, and the mixture incubated under conditions conducive to complex formation (*e.g*., at physiological conditions for salt and pH). Following incubation, the beads or microtiter plate wells are washed to remove unbound components, the matrix immobilized in the case of beads, complex determined either directly or indirectly, for example, as described above. Alternatively, the complexes can be dissociated from the matrix, and the level of *S*. *pneumoniae* Polypeptide binding or activity determined using standard techniques.

Other techniques for immobilizing either a *S*. *pneumoniae* Polypeptide or a binding target on matrices include using conjugation of biotin and streptavidin. Biotinylated *S*. *pneumoniae* Polypeptides or target molecules can be prepared from biotin-NHS (N-hydroxysuccinimide) using techniques known in the art (*e.g*., biotinylation kits from Pierce Chemicals, Rockford, IL), and immobilized in the wells of streptavidin-coated 96 well plates (Pierce Chemical).

To conduct the assay, the non-immobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g*., using a labeled antibody specific for the immobilized component (the antibody, in turn, can be directly labeled or indirectly labeled with, *e.g.,* a labeled anti-Ig antibody).

In some embodiments, the assay is performed utilizing antibodies that bind specifically to *S*. *pneumoniae* Polypeptides or binding targets, but do not interfere with binding of the *S*. *pneumoniae* Polypeptide to its target. Such antibodies can be derivatized to the wells of the plate, and unbound target or *S*. *pneumoniae* Polypeptide trapped in the wells by antibody conjugation. Methods for detecting such complexes, in addition to those described above for the GST-immobilized complexes, include immunodetection of complexes using antibodies reactive with the *S*. *pneumoniae* Polypeptide or target molecule, as well as enzyme-linked assays which rely on detecting an enzymatic activity associated with the *S*. *pneumoniae* Polypeptide or target molecule.

In some embodiments, cell-free assays can be conducted in a liquid phase. In such an assay, the reaction products are separated from unreacted components, by any of a number of standard techniques, including but not limited to: differential centrifugation (for example, Rivas et al., 1993, Trends Biochem. Sci., 18:284-287); chromatography (gel filtration chromatography, ion-exchange chromatography); electrophoresis (*e.g*., Ausubel et al., eds., 1999, Current Protocols in Molecular Biology, J. Wiley: New York.); and immunoprecipitation (for example, Ausubel et al., eds., 1999, Current Protocols in Molecular Biology, J. Wiley: New York). Such resins and chromatographic techniques are known to those skilled in the art (*e.g*., Heegaard, 1998, J. Mol. Recognit., 11:141-148 and Hage et al., 1997, J. Chromatogr. B. Biomed. Sci. Appl., 699:499-525). Further, fluorescence energy transfer may also be conveniently utilized, as described herein, to detect binding without further purification of the complex from solution.

In some embodiments, the assay includes contacting a *S*. *pneumoniae* Pili Polypeptide, or biologically active portion thereof with a known cell or compound (*e.g*., a protein) that binds to *S. pneumoniae* Pili Polypeptide, to form an assay mixture, contacting the assay mixture with a test compound, and determining the ability of the test compound to affect binding of the *S. pneumoniae* Pili Polypeptide to the cell or compound.

A general assay for binding of bacterial cells that express *S. pneumoniae* pilus II island (INV104B) pili involves incubating bacterial cells that express *S. pneumoniae* pilus II island (INV104B) pili with A549 lung epithelial cells, washing to remove nonadherent bacterial cells, and detecting adherent bacterial cells. Bacterial adherence can be measured by any means in the art, *e.g.,* detecting binding of an antibody to the adherent bacterial cells or lysing the epithelial cells and counting the number of associated bacterial cells. HEP2 cells, CHO cells, or HeLa cells can also be used in assays of binding of bacterial cells that express *S. pneumoniae* pilus II island (INV104B) pili.

### Immunogenic Compositions

Immunogenic compositions described herein that include *S. pneumoniae* Polypeptides may further comprise one or more antigenic agents. Exemplary antigens include those listed below. Additionally, the compositions described herein may be used to treat or prevent infections caused by any of the below-listed microbes. Antigens for use in the immunogenic compositions include, but are not limited to, one or more of the following set forth below, or antigens derived from one or more of the following set forth below:

### Bacterial Antigens

*N. meningitidis:* a protein antigen from *N. meningitides* serogroup A, C, W135, Y, and/or B (Bruyn, G. A. W. & van Furth, R. (1991) Eur. J. Clin. Microbiol. Infect. Dis. 10, 897-910; Ryan, M. W. & Antonelli, P. J. (2000) Laryngoscope 110, 961-964; Cutts, F. T., Zaman, S. M., Enwere, G., Jaffar, S., Levine, O. S., Okoko, C. Oluwalana, A., Vaughan, S., Obaro, A., Leach, A., et al. (2005) Lancet 365, 1139-1146; Swiatlo, E., Champlin, F. R., Holman, S. C., Wilson, W. W.&Watt, J. M. (2002) Infect. Immun. 70, 412-415; Sandgren, A., Albiger, B., Orihuela, C., Tuomanen, E., Normark, S. & Henriques-Normark, B. (2005) J. Infect. Dis. 192, 791-800; Henriques Normark, B., Christensson, B., Sandgren, A., Noreen, B., Sylvan, S., Burman, L. G. & Olsson-Liljequist, B. (2003) Microb. Drug Resist. 9, 337-344; Nunes, S., Sá-Leão, R., Carriço, J., Alves, C. R., Mato, R., Avô, A. B., Saldanha, J., Almeida, J. S., Sanches, I. S. & de Lencastre, H. (2005) J. Clin. Microbiol. 43, 1285-1293); an outer-membrane vesicle (OMV) preparation from *N. meningitides* serogroup B. (Henrichsen, J. (1995) J. Clin. Microbiol. 33, 2759-2762; Lau, G. W., Haataja, S., Lonetto, M., Kensit, S. E., Marra, A., Bryant, A. P., McDevitt, D., Morrison, D. A. & Holden, D. W. (2001) Mol. Microbiol. 40, 555-571; Rosenow, C., Ryan, P., Weiser, J. N., Johnson, S., Fontan, P., Ortqvist, A. & Masure, H. R. (1997) Mol. Microbiol. 25, 819-829; Tuomanen, E. (1999) Current Opin. Biol. 2, 35-39); a saccharide antigen, including LPS, from *N. meningitides* serogroup A, B, C W135 and/or Y, such as the oligosaccharide from serogroup C *(see* PCT/U99/09346; PCT IB98/01665; and PCT IB99/00103);

*Streptococcus pneumoniae:* a saccharide or protein antigen, particularly a saccharide from *Streptococcus pneumoniae* or a protein or antigenic peptide of PhtD (BVH-11-2, SP1003, spr0907) (Adamou et al., Infect. Immun., 69:949-53, 2001; Hamel et al., Infect. Immun., 72:2659-70, 2004); PhtE (BVH-3, SP1004, spr0908) (Adamou et al., Infect. Immun., 69:949-53, 2001; Hamel et al., Infect. Immun., 72:2659-70, 2004); PhtB (PhpA, BVH-11, SP1174, spr1060) (Adamou et al., Infect. Immun., 69:949-53, 2001; Zhang et al., Infect. Immun., 69:3827-36, 2001; Hamel et al., Infect. Immun., 72:2659-70, 2004); PhtA (BVH-11-3, SP1175, spr1061) (Adamou et al., Infect. Immun., 69:949-53, 2001; Wizemann et al., Infect. Immun., 69:1593-98, 2001; Zhang et al., Infect. Immun., 69:3827-36, 2001; Hamel et al., Infect. Immun., 72:2659-70, 2004); NanA (SP1693, spr1536) (Tong et al., Infect. Immun., 73:7775-78, 2005); SP1872 (spr1687) (Brown et al., Infect. Immun., 69:6702-06,2001); PspC (CbpA, SP2190, spr1995) (Ogunniyi et al., Infect. Immun., 69:5997-6003, 2001); PspA (SP0177, spr0121, spr1274) (Briles et al., Vaccine, 19:S87-S95, 2001); SP0498 (spr0440); LytB (SP0965, spr0867) (Wizemann et al., Infect. Immun., 69:1593-98, 2001); AliB (SP1527, spr1382); PpmA (SP0981, spr0884) (Overweg et al., Infect. Immun., 68:4180-4188, 2000); LytC (SP1573, spr1431) (Wizemann et al., Infect. Immun., 69:1593-98, 2001); PsaA (Briles et al., Vaccine, 19:S87-S95, 2001); PdB (Ogunniyi et al., Infect. Immun., 69:5997-6003, 2001); RPhp (Zhang et al., Infect. Immun., 69:3827-36, 2001); PiuA (Jomaa et al., Vaccine, 24:5133-39, 2006); PiaA (Jomaa et al., Vaccine, 24:5133-39, 2006); 6PGD (Daniely et al., Clin. Exp. Immunol., 144:254-263, 2006); or PppA (Green et al., Infect. Immun., 73:981-89, 2005);

*Streptococcus agalactiae:* such as, Group B streptococcus antigens;

*Streptococcus pyogenes:* such as, Group A streptococcus antigens;

*Enterococcus faecalis or Enterococcus faecium* such as, a trisaccharide repeat or other *Enterococcus* derived antigens provided in U.S. Patent No. 6,756,361;

*Helicobacter pylori:* including: Cag, Vac, Nap, HopX, HopY and/or urease antigen;

*Bordetella pertussis:* such as pertussis holotoxin (PT) and filamentous hemagglutinin (FHA) from *B. pertussiss,* optionally also combination with pertactin and/or agglutinogens 2 and 3 antigen;

*Staphylococcus aureus:* including *S. aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin);

*Staphylococcus epidermis:* particularly, *S. epidermidis* slime-associated antigen (SAA);

*Staphylococcus saprophyticus:* (causing urinary tract infections) particularly the 160 kDa hemagglutinin of *S. saprophyticus* antigen;

*Pseudomonas aeruginosa:* particularly, endotoxin A, Wzz protein, *P. aeruginosa* LPS, more particularly LPS isolated from PAO1 (O5 serotype), and/or Outer Membrane Proteins, including Outer Membrane Proteins F (OprF) *(*Infect Immun. 2001 May; 69(5): 3510-3515);

*Bacillus anthracis* (anthrax): such as *B. anthracis* antigens (optionally detoxified) from A-components (lethal factor (LF) and edema factor (EF)), both of which can share a common B-component known as protective antigen (PA);

*Moraxella catarrhalis:* (respiratory) including outer membrane protein antigens (HMW-OMP), C-antigen, and/or LPS;

*Yersinia pestis* (plague): such as F1 capsular *antigen* (Infect Immun. 2003 Jan; 71(1)): 374-383, LPS (Infect Immmun. 1999 Oct; 67(10): 5395), Yersinia pestis V antigen (Infect Immun. 1997 Nov; 65(11): 4476-4482);

*Yernisia enterocolitica* (gastrointestinal pathogen): particularly LPS *(*Infect Immun. 2002 August; 70(8): 4414);

*Yersinia pseudotuberculosis:* gastrointestinal pathogen antigens;

*Mycobacterium tuberculosis:* such as lipoproteins, LPS, BCG antigens, a fusion protein of antigen 85B (Ag85B) and/or ESAT-6 optionally formulated in cationic lipid vesicles (Infect Immun. 2004 October; 72(10): 6148), Mycobacterium tuberculosis (Mtb) isocitrate dehydrogenase associated antigens (Proc. Natl Acad Sci U.S.A. 2004 Aug 24; 101 (34): 12652), and/or MPT51 antigens (Infect Immun. 2004 July; 72(7): 3829);

*Legiollella pneumophila* (Legionnaires' Disease): L. pneumophila antigensoptionally derived from cell lines with disrupted *asd* genes (Infect Immun. 1998 May; 66(5): 1898);

*Richettsia:* including outer membrane proteins, including the outer membrane protein A and/or B (OmpB) (Biochim Biophys Acta. 2004 Nov 1;1702(2):145), LPS, and surface protein antigen (SPA) (J Autoimmun. 1989 Jun;2 Suppl:81);

*E. coli:* including antigens from enterotoxigenic *E. coli* (ETEC), enteroaggregative *E. coli* (EAggEC), diffusely adhering *E. coli* (DAEC), enteropathogenic *E. coli* (EPEC), and/or enterohemorrhagic *E. coli* (EHEC);

*Vibrio cholerae:* including proteinase antigens, LPS, particularly lipopolysaccharides of Vibrio cholerae II, O1 Inaba O-specific polysaccharides, V. cholera 0139, antigens of IEM108 vaccine (Infect Immun. 2003 Oct;71(10):5498-504), and/or Zonula occludens toxin (Zot);

*Salmonella typhi* (typhoid fever): including capsular polysaccharides preferably conjugates (Vi, *i.e.* vax-TyVi);

*Salmonella typhimurium* (gastroenteritis): antigens derived therefrom are contemplated for microbial and cancer therapies, including angiogenesis inhibition and modulation of flk;

*Listeria monocytogenes* (systemic infections in immunocompromised or elderly people, infections of fetus): antigens derived from L. monocytogenes are preferably used as carriers/vectors for intracytoplasmic delivery of the conjugates/associated compositions described herein;

*Porphyromonas gingivalis:* such as, *P. gingivalis* outer membrane protein (OMP);

*Tetanus:* such as tetanus toxoid (TT) antigens, *e.g.,* used as a carrier protein in conjunction/conjugated with the compositions described herein;

*Diphtheria:* such as a diphtheria toxoid or a diphtheria toxoid mutant, *e.g.,* CRM₁₉₇, additionally antigens capable of modulating, inhibiting or associated with ADP ribosylation are contemplated for combination/co-administration/conjugation with the compositions described herein, the diphtheria toxoids can be used as carrier proteins;

*Borrelia burgdorferi* (Lyme disease): such as antigens associated with P39 and P 13 (an integral membrane protein, Infect Immun. 2001 May; 69(5): 3323-3334.), VlsE Antigenic Variation Protein (J Clin Microbiol. 1999 Dec; 37(12): 3997);

*Haemophilus influenzae B:* such as a saccharide antigen therefrom;

*Klebsiella:* such as an OMP, including OMP A, or a polysaccharide optionally conjugated to tetanus toxoid;

*Neiserria gonorrhoeae:* including, a Por (or porin) protein, such as PorB (*see* Zhu et al., Vaccine (2004) 22:660 - 669), a transferring binding protein, such as TbpA and TbpB *(see* Price et al., Infection and Immunity (2004) 71(1):277 - 283), a opacity protein (such as Opa), a reduction-modifiable protein (Rmp), and outer membrane vesicle (OMV) preparations (*see* Plante et al., J Infectious Disease (2000) 182:848 - 855), also *see, e.g.,* WO99/24578, WO99/36544, WO99/57280, WO02/079243);

*Chlamydia pneumoniae:* particularly *C. pneumoniae* protein antigens;

*Chlamydia trachomatis:* including antigens derived from serotypes A, B, Ba and C are (agents of trachoma, a cause of blindness), serotypes L₁, L₂ & L₃ (associated with Lymphogranuloma venereum), and serotypes, D-K;

*Treponema pallidum* (Syphilis): particularly a TmpA antigen; and

*Haemophilus ducreyi* (causing chancroid): including outer membrane protein (DsrA).

Where not specifically referenced, further bacterial antigens as described herein may be capsular antigens, polysaccharide antigens or protein antigens of any of the above. Further bacterial antigens may also include an outer membrane vesicle (OMV) preparation. Additionally, antigens include live, attenuated, split (for enveloped viruses), and/or purified versions of any of the aforementioned bacteria. The bacterial or microbial derived antigens described herein may be gram-negative or gram-positive and aerobic or anaerobic.

Additionally, any of the above bacterial-derived saccharides (polysaccharides, LPS, LOS or oligosaccharides) can be conjugated to another agent or antigen, such as a carrier protein (for example CRM₁₉₇). Such conjugation maybe direct conjugation effected by reductive amination of carbonyl moieties on the saccharide to amino groups on the protein, as provided in U.S. Patent No. 5,360,897 and Can J Biochem Cell Biol, 1984 May;62(5):270-5. Alternatively, the saccharides can be conjugated through a linker, such as, with succinamide or other linkages provided in Bioconjugate Techniques, 1996 and CRC, Chemistry of Protein Conjugation and Cross-Linking, 1993.

### Viral Antigens

*Influenza:* including whole viral particles (attenuated), split, or subunit comprising hemagglutinin (HA) and/or neuraminidase (NA) surface proteins, the influenza antigens may be derived from chicken embryos or propagated on cell culture, and/or the influenza antigens are derived from influenza type A, B, and/or C, among others;

*Respiratory syncytial virus (RSV):* including the F protein of the A2 strain of RSV (J Gen Virol. 2004 Nov; 85(Pt 11):3229) and/or G glycoprotein;

*Parainfluenza virus (PIV):* including PIV type 1, 2, and 3, preferably containing hemagglutinin, neuraminidase and/or fusion glycoproteins;

*Poliovirus:* including antigens from a family of picornaviridae, preferably poliovirus antigens such as OPV or, preferably IPV;

*Measles:* including split measles virus (MV) antigen optionally combined with the Protollin and or antigens present in MMR vaccine;

*Mumps:* including antigens present in MMR vaccine;

*Rubella:* including antigens present in MMR vaccine as well as other antigens from Togaviridae, including dengue virus;

*Rabies:* such as lyophilized inactivated virus (RabAvert™);

*Flaviviridae viruses:* such as (and antigens derived therefrom) yellow fever virus, Japanese encephalitis virus, dengue virus (types 1, 2, 3, or 4), tick borne encephalitis virus, and West Nile virus;

*Caliciviridae;* antigens therefrom;

*HIV:* including HIV-1 or HIV-2 strain antigens, such as gag (p24gag and p55gag), env (gp160 and gp41), pol, tat, nef, rev vpu, miniproteins, (preferably p55 gag and gp140v delete) and antigens from the isolates HIV_{IIIb}, HIV_{SF2}, HIV_{LAV}, HIV_{LAI}, HIV_{MN}, HIV-1_{CM235}, HIV-1_{US4}, HIV-2; simian immunodeficiency virus (SIV) among others;

*Rotavirus:* including VP4, VP5, VP6, VP7, VP8 proteins (*Protein Expr Purif.* 2004 Dec;38(2):205) and/or NSP4;

*Pestivirus:* such as antigens from classical porcine fever virus, bovine viral diarrhea virus, and/or border disease virus;

*Parvovirus:* such as parvovirus B 19;

*Coronavirus:* including SARS virus antigens, particularly spike protein or proteases therefrom, as well as antigens included in WO 04/92360;

*Hepatitis A virus:* such as inactivated virus;

*Hepatitis B virus:* such as the surface and/or core antigens (sAg), as well as the presurface sequences, pre-S1 and pre-S2 (formerly called pre-S), as well as combinations of the above, such as sAg/pre-S1, sAg/pre-S2, sAg/pre-S1/pre-S2, and pre-S1/pre-S2, (*see, e.g.,* AHBV Vaccines - Human Vaccines and Vaccination, pp. 159-176; and U.S. Patent Nos. 4,722,840, 5,098,704, 5,324,513; Beames et al., J. Virol. (1995) 69:6833-6838, Birnbaum et al., J. Virol. (1990) 64:3319-3330; and Zhou et al., J. Virol. (1991) 65:5457-5464);

*Hepatitis C virus:* such as E1, E2, E1/E2 *(see,* Houghton et al., *Hepatology* (1991) 14:381), NS345 polyprotein, NS 345-core polyprotein, core, and/or peptides from the nonstructural regions (International Publication Nos. WO 89/04669; WO 90/11089; and WO 90/14436);

*Delta hepatitis virus (HDV):* antigens derived therefrom, particularly δ-antigen from HDV (*see, e.g.,* U.S. Patent No. 5,378,814);

*Hepatitis E virus (HEV);* antigens derived therefrom;

*Hepatitis G virus (HGV);* antigens derived therefrom;

*Varcicella zoster virus:* antigens derived from varicella zoster virus (VZV) (J. Gen. Virol. (1986) 67:1759);

*Epstein-Barr virus:* antigens derived from EBV (Baer et al., Nature (1984) 310:207);

*Cytomegalovirus:* CMV antigens, including gB and gH (Cytomegaloviruses (J.K. McDougall, ed., Springer-Verlag 1990) pp. 125-169);

*Herpes simplex virus:* including antigens from HSV-1 or HSV-2 strains and glycoproteins gB, gD and gH (McGeoch et al., J. Gen. Virol. (1988) 69:1531 and U.S. Patent No. 5,171,568);

*Human Herpes Virus:* antigens derived from other human herpesviruses such as HHV6 and HHV7; and

*HPV:* including antigens associated with or derived fi-om human papillomavirus (HPV), for example, one or more of E1 - E7, L1, L2, and fusions thereof, particularly the compositions described herein may include a virus-like particle (VLP) comprising the L1 major capsid protein, more particular still, the HPV antigens are protective against one or more of HPV serotypes 6, 11, 16 and/or 18.

Further provided are antigens, compositions, methods, and microbes included in Vaccines, 4th Edition (Plotkin and Orenstein ed. 2004); Medical Microbiology 4th Edition (Murray et al. ed. 2002); Virology, 3rd Edition (W.K. Joklik ed. 1988); Fundamental Virology, 2nd Edition (B.N. Fields and D.M. Knipe, eds. 1991), which are contemplated in conjunction with the compositions described herein.

Additionally, antigens include live, attenuated, split, and/or purified versions of any of the aforementioned viruses.

### Fungal Antigens

Fungal antigens for use herein that are associated with vaccines include those described in: U.S. Pat. Nos. 4,229,434 and 4,368,191 for prophylaxis and treatment of trichopytosis caused by *Trichophyton mentagrophytes;* U.S. Pat. Nos. 5,277,904 and 5,284,652 for a broad spectrum dermatophyte vaccine for the prophylaxis of dermatophyte infection in animals, such as guinea pigs, cats, rabbits, horses and lambs, these antigens comprises a suspension of killed *T. equinum, T. mentagrophytes* (var. granulare), *M. canis* and/or *M. gypseum* in an effective amount optionally combined with an adjuvant; U.S. Pat. Nos. 5,453,273 and 6,132,733 for a ringworm vaccine comprising an effective amount of a homogenized, formaldehyde-killed fungi, *i.e., Microsporum canis* culture in a carrier; and U.S. Pat. No. 5,948,413 involving extracellular and intracellular proteins for pythiosis. Additional antigens identified within antifungal vaccines include *Ringvac bovis* LTF-130 and Bioveta.

Further, fungal antigens for use herein may be derived from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme.*

Fungal pathogens for use as antigens or in derivation of antigens in conjunction with the compositions described herein comprise *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichumi clavatum, Histoplasma capsulatum, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum. Sporothrix schenckii, Trichosporon beige*/*ii, Penicillium marneffei,* Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, and Saksenaea spp.

Other fungi from which antigens are derived include Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Processes for producing fungal antigens are well known in the art *(see* U.S. Patent No. 6,333,164). In some methods a solubilized fraction extracted and separated from an insoluble fraction obtainable from fungal cells of which cell wall has been substantially removed or at least partially removed, characterized in that the process comprises: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

### STD Antigens

In some embodiments, microbes (bacteria, viruses and/or fungi) against which the present compositions and methods can be implement include those that cause sexually transmitted diseases (STDs) and/or those that display on their surface an antigen that can be the target or antigen composition as described herein. In some embodiment, compositions are combined with antigens derived from a viral or bacterial STD. Antigens derived from bacteria or viruses can be administered in conjunction with the compositions described herein to provide protection against at least one of the following STDs, among others: chlamydia, genital herpes, hepatitis (particularly HCV), genital warts, gonorrhea, syphilis and/or chancroid (*see*, WO00/15255).

In some embodiments, the compositions described herein are co-administered with one or more antigens for the prevention or treatment of an STD.

Antigens derived from the following viruses associated with STDs, which are described in greater detail above, are preferred for co-administration with the compositions described herein: hepatitis (particularly HCV), HPV, HIV, or HSV.

Additionally, antigens derived from the following bacteria associated with STDs, which are described in greater detail above, are preferred for co-administration with the compositions described herein: *Neiserria gonorrhoeae, Chlamydia pneumoniae, Chlamydia trachomatis, Treponema pallidum,* or *Haemophilus ducreyi.*

### Respiratory Antigens

The *S*. *pneumoniae* antigen may be a respiratory antigen and could further be used in an immunogenic composition for methods of preventing and/or treating infection by a respiratory pathogen, including a virus, bacteria, or fungi such as respiratory syncytial virus (RSV), PIV, SARS virus, influenza, *Bacillus anthracis,* particularly by reducing or preventing infection and/or one or more symptoms of respiratory virus infection. A composition comprising an antigen described herein, such as one derived from a respiratory virus, bacteria or fungus is administered in conjunction with the compositions described herein to an individual which is at risk of being exposed to that particular respiratory microbe, has been exposed to a respiratory microbe or is infected with a respiratory virus, bacteria or fungus. The composition(s) described herein is/are co-administered at the same time or in the same formulation with one or more antigens of the respiratory pathogen. Administration of the composition results in reduced incidence and/or severity of one or more symptoms of respiratory infection.

### Pediatric/Geriatric Antigens

In some embodiments, the compositions described herein are used in conjunction with one or more antigens for treatment of a pediatric population, as in a pediatric antigen. In some embodiments, the age of the subjects in the pediatric population is less than about 3 years old, or less than about 2 years, or less than about 1 years old. In some embodiments the pediatric antigen (in conjunction with the composition described herein) is administered multiple times over at least 1, 2, or 3 years.

In some embodiments, the compositions described herein are used in conjunction with one or more antigens for treatment of a geriatric population, as in a geriatric antigen. In some embodiments, the age of the subjects in the geriatric population is greater than about 50 years old, greater than about 55 years old, greater than about 60 years old, greater than about 65 years old, greater than about 70 years old, greater than about 75 years old, greater than about 80 years old, or greater than about 85 years old. In some embodiments the geriatric antigen (in conjunction with the composition described herein) is administered multiple times over at least 1, 2, or 3 years.

### Other Antigens

Other antigens for use in conjunction with the compositions of the present include hospital acquired (nosocomial) associated antigens.

In some embodiments, parasitic antigens are contemplated in conjunction with the compositions described herein. Examples of parasitic antigens include those derived from organisms causing malaria and/or Lyme disease.

In some embodiments, the antigens in conjunction with the compositions described herein are associated with or effective against a mosquito born illness. In some embodiments, the antigens in conjunction with the compositions described herein are associated with or effective against encephalitis. In some embodiments the antigens in conjunction with the compositions described herein are associated with or effective against an infection of the nervous system.

In some embodiments, the antigens in conjunction.with the compositions described herein are antigens transmissible through blood or body fluids.

### Antigen Formulations

In some aspects, methods of producing microparticles having adsorbed antigens are provided. The methods comprise: (a) providing an emulsion by dispersing a mixture comprising (i) water, (ii) a detergent, (iii) an organic solvent, and (iv) a biodegradable polymer selected from the group consisting of a poly(α-hydroxy acid), a polyhydroxy butyric acid, a polycaprolactone, a polyorthoester, a polyanhydride, and a polycyanoacrylate. The polymer is typically present in the mixture at a concentration of about 1% to about 30% relative to the organic solvent, while the detergent is typically present in the mixture at a weight-to-weight detergent-to-polymer ratio of from about 0.00001:1 1 to about 0.1:1 (more typically about 0.0001:1 1 to about 0.1:1, about 0.001:1 to about 0.1:1, or about 0.005:1 to about 0.1:1); (b) removing the organic solvent from the emulsion; and (c) adsorbing an antigen on the surface of the microparticles. In some embodiments, the biodegradable polymer is present at a concentration of about 3% to about 10% relative to the organic solvent.

In some embodiments, microparticles for use herein are formed from materials that are sterilizable, non-toxic and biodegradable. Such materials include, without limitation, poly(α-hydroxy acid), polyhydroxybutyric acid, polycaprolactone, polyorthoester, polyanhydride, PACA, and polycyanoacrylate. In some embodiments, microparticles for use with the methods described herein are derived from a poly(α-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered macromolecule. These parameters are discussed more fully below.

Further antigens may also include an outer membrane vesicle (OMV) preparation.

Antigens can also be adsorbed to peptidoglycans of various gram-positive bacteria to make gram-positive enhancer matrix (GEM) particles, as described in Bosma et al., Appl. Env. Microbiol., 72:880-889, 2006. This method relies on the non-covalent binding of the LysM motif (Buist et al., J. Bact., 177:1554-63, 1995; Bateman and Bycroft, J. Mol. Biol., 299:1113-19, 2000) to the cell wall peptidoglycan of acid-treated cells. Briefly, a polypeptide antigen linked to one or more LysM motifs (e.g., non-covalently or covalently (e.g., as a fusion protein or by conjugation) is added to acid-treated gram-positive bacteria. The antigen peptides bind with high affinity and can be used in immunogenic compositions. Exemplary acids used in these methods include trichloroacetic acid (*e.g.*, at 0.1%-10%), acetic acid (*e.g.*, at 5.6 M), HCl (*e.g.*, at 0.01 M), lactic acid (*e.g.*, at 0.72 M), and formic acid (*e.g.*, at 0.56 M).

Additional formulation methods and antigens (especially tumor antigens) are provided in U.S. Patent No. 6,884,435.

### Antigen References

The following references include antigens useful in conjunction with the compositions described herein:

International patent application WO99/24578

International patent application WO99/36544.

International patent application WO99/57280.

International patent application WO00/22430.

Tettelin et al. (2000) Science 287:1809-1815.

International patent application WO96/29412.

Pizza et al. (2000) Science 287:1816-1820.

PCT WO 01/52885.

Bjune et al. (1991) Lancet 338(8775).

Fuskasawa et al. (1999) Vaccine 17:2951-2958.

Rosenqist et al. (1998) Dev. Biol. Strand 92:323-333.

Costantino et al. (1992) Vaccine 10:691-698.

Costantino et al. (1999) Vaccine 17:1251-1263.

Watson (2000) Pediatr Infect Dis J 19:331-332.

Rubin (20000) Pediatr Clin North Am 47:269-285,v.

Jedrzejas (2001) Microbiol Mol Biol Rev 65:187-207.

International patent application filed on 3^{rd} July 2001 claiming priority from GB0016363.4; WO 02/02606; PCT IB/01/00166.

Kalman et al. (1999) Nature Genetics 21:385-389.

Read et al. (2000) Nucleic Acids Res 28:1397-406.

Shirai et al. (2000) J. Infect. Dis 181 (Suppl 3):S524-S527.

International patent application WO99/27105.

International patent application WO00/27994.

International patent application WO00/37494.

International patent application WO99/28475.

Bell (2000) Pediatr Infect Dis J 19:1187-1188.

Iwarson (1995) APMIS 103:321-326.

Gerlich et al. (1990) Vaccine 8 Suppl:S63-68 & 79-80.

Hsu et al. (1999) Clin Liver Dis 3:901-915.

Gastofsson et al. (1996) N. Engl. J. Med. 334-:349-355.

Rappuoli et al. (1991) TIBTECH 9:232-238.

Vaccines (1988) eds. Plotkin & Mortimer. ISBN 0-7216-1946-0.

Del Guidice et al. (1998) Molecular Aspects of Medicine 19:1-70.

International patent application WO93/018150.

International patent application WO99/53310.

International patent application WO98/04702.

Ross et al. (2001) Vaccine -19:135-142.

Sutter et al. (2000) Pediatr Clin North Am 47:287-308.

Zimmerman & Spann (1999) Am Fan Physician 59:113-118, 125-126.

Dreensen (1997) Vaccine 15 Suppl"S2-6.

MMWR Morb Mortal Wkly rep 1998 Jan 16:47(1):12, 9.

McMichael (2000) Vaccine19 Suppl 1:S101-107.

Schuchat (1999) Lancet 353(9146):51-6.

GB patent applications 0026333.5, 0028727.6 & 0105640.7.

Dale (1999) Infect Disclin North Am 13:227-43, viii.

Ferretti et al. (2001) PNAS USA 98: 4658-4663.

Kuroda et al. (2001) Lancet 357(9264):1225-1240; *see also* pages 1218-1219.

Ramsay et al. (2001) Lancet 357(9251):195-196.

Lindberg (1999) Vaccine 17 Suppl 2:S28-36.

Buttery & Moxon (2000) J R Coil Physicians Long 34:163-168.

Ahmad & Chapnick (1999) Infect Dis Clin North Am 13:113-133, vii.

Goldblatt (1998) J. Med. Microbiol. 47:663-567.

European patent 0 477 508.

U.S. Patent No. 5,306,492.

International patent application WO98/42721.

Conjugate Vaccines (eds. Cruse et al.) ISBN 3805549326, particularly vol. 10:48-114.

Hermanson (1996) Bioconjugate Techniques ISBN: 012323368 & 012342335X.

European patent application 0372501.

European patent application 0375881.

European patent application 0427347.

International patent application WO93/17712.

International patent application WO98/58668.

European patent application 0471177.

International patent application WO00/56360.

International patent application WO00/67161.

### Fusion Proteins

The *S. pneumoniae* polypeptides used with the compositions described herein may be present in the compositions as individual separate polypeptides, but in some embodiments at least two *(i.e.* 2, 3, 4, 5, 6, 7, 8, 9, 10, 11, 12, 13, 14, 15, 16, 17, or 18) of the antigens can be expressed as a single polypeptide chain (a "hybrid" or "fusion" polypeptide). Such fusion polypeptides offer two principal advantages: first, a polypeptide that may be unstable or poorly expressed on its own can be assisted by adding a suitable fusion partner that overcomes the problem; second, commercial manufacture is simplified as only one expression and purification need be employed in order to produce two polypeptides which are both antigenically useful.

The fusion polypeptide may comprise one or more polypeptide sequences encoded by the *S. pneumoniae* Polypeptides as identified herein. Accordingly, the compositions described herein may include a fusion peptide comprising a first amino acid sequence and a second amino acid sequence, wherein said first and second amino acid sequences are selected from a protein encoded by the *S. pneumoniae* pilus II island (INV104B) or a fragment thereof. In some embodiments, the first and second amino acid sequences in the fusion polypeptide comprise different epitopes.

In some embodiments, hybrids (or fusions) consisting of amino acid sequences from two, three, four, five, six, seven, eight, nine, or ten antigens encoded by the *S. pneumoniae* Polypeptide sequences discussed herein are preferred. In some embodiments, hybrids consisting of amino acid sequences from two, three, four, or five antigens encoded by the *S. pneumoniae* Polypeptide sequences discussed herein are preferred.

Different hybrid polypeptides may be mixed together in a single formulation. Within such combinations, a sequence encoded by the *S. pneumoniae* Polypeptide sequences discussed herein may be present in more than one hybrid polypeptide and/or as a non-hybrid polypeptide. In some embodiments, however, that an antigen is present either as a hybrid or as a non-hybrid, but not as both.

Hybrid polypeptides can be represented by the formula NH₂-A- {-X-L-}ₙ-B-COOH, wherein: X is an amino acid sequence of a *S. pnemoniae* Polypeptide as described herein or a fragment thereof; L is an optional linker amino acid sequence; A is an optional N-terminal amino acid sequence; B is an optional C-terminal amino acid sequence; and *n* is 2, 3, 4, 5, 6, 7, 8, 9, 10, 11, 12, 13, 14 or 15.

If a -X- moiety has a leader peptide sequence in its wild-type form, this may be included or omitted in the hybrid protein. In some embodiments, the leader peptides will be deleted except for that of the -X- moiety located at the N-terminus of the hybrid protein *i.e.* the leader peptide of X₁ will be retained, but the leader peptides of X₂ ... Xₙ will be omitted. This is equivalent to deleting all leader peptides and using the leader peptide of X₁ as moiety -A-.

For each *n* instances of {-X-L-}, linker amino acid sequence -L- may be present or absent. For instance, when *n*=2 the hybrid may be NH₂-X₁-L₁-X₂-L₂-COOH; NH₂-X₁-X₂-COOH; NH₂-X₁-L₁-X₂-COOH; NH₂-X₁-X₂-L₂-COOH; *etc*. Linker amino acid sequence(s) -L-will typically be short *(e.g.* 20 or fewer amino acids *i.e.* 19, 18, 17, 16, 15, 14, 13, 12, 11, 10, 9, 8, 7, 6, 5, 4, 3, 2, 1). Examples comprise short peptide sequences that facilitate cloning, poly-glycine linkers (*i.e.* comprising Gly*ₙ* where *n* = 2, 3, 4, 5, 6, 7, 8, 9, 10 or more). Other suitable linker amino acid sequences will be apparent to those skilled in the art. A useful linker is GSGGGG, with the Gly-Ser dipeptide being formed from a *Bam*HI restriction site, thus aiding cloning and manipulation, and the (Gly)₄ tetrapeptide being a typical poly-glycine linker.

In the hybrid polypeptide formula provided above, -A- is an optional N-terminal amino acid sequence. This will typically be short *(e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification. Other suitable N-terminal amino acid sequences will be apparent to those skilled in the art. If X₁ lacks its own N-terminus methionine, -A- is preferably an oligopeptide (*e.g.* with 1, 2, 3, 4, 5, 6, 7 or 8 amino acids) which provides a N-terminus methionine.

In the hybrid polypeptide formula provided above, -B- is an optional C-terminal amino acid sequence. This will typically be short *(e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification, or sequences which enhance protein stability. Other suitable C-terminal amino acid sequences will be apparent to those skilled in the art.

In the hybrid polypeptide formula provided above, most preferably, *n* is 2 or 3.

### Nucleic Acids

This description provides nucleic acids encoding the *S. pneumoniae* Polypeptide sequences and/or the hybrid fusion polypeptides. This description also provides nucleic acids encoding *S. pneumoniae* Polypeptide antigen, and/or the hybrid fusion polypeptides described herein. Furthermore, the description provides nucleic acids which can hybridize to these nucleic acids, preferably under "high stringency" conditions (*e.g.* 65 °C in a 0.1x SSC, 0.5% SDS solution).

The polypeptides described herein can be prepared by various means *(e.g.* recombinant expression, purification from cell culture, chemical synthesis, etc.) and in various forms (*e.g.* native, fusions, non-glycosylated, lipidated, *etc.*). They are preferably prepared in substantially pure form (*i.e.* substantially free from other host cell proteins).

The nucleic acids described herein can be prepared in many ways (*e.g.* by chemical synthesis, from genomic or cDNA libraries, from the organism itself, *etc.)* and can take various forms (*e.g.* single stranded, double stranded, vectors, probes, *etc*.). They are preferably prepared in substantially pure form (*i.e.* substantially free from other host cell nucleic acids).

The terms "nucleic acid" and "nucleic acids," as used herein, include DNA and RNA, and also their analogs, such as those containing modified backbones (*e.g.*, phosphorothioates, *etc*.), and also peptide nucleic acids (PNA), *etc.* The compositions described herein include nucleic acids comprising sequences complementary to those described above (*e.g.*, for antisense or probing purposes).

This description also discloses a process for producing a polypeptide, comprising the step of culturing a host cell transformed with nucleic acid as described herein under conditions which induce polypeptide expression.

This description also discloses a process for producing a polypeptide, comprising the step of synthesizing at least part of the polypeptide by chemical means.

This description also discloses a process for producing nucleic acid, comprising the step of amplifying nucleic acid using a primer-based amplification method (*e.g.* PCR).

This description also discloses a process for producing nucleic acid, comprising the step of synthesizing at least part of the nucleic acid by chemical means.

### Purification and Recombinant Expression

The *S. pneumoniae* Polypeptide sequences described herein may be isolated from native *S. pneumonia*, or they may be recombinantly produced, for instance in a heterologous host. For example, the *S. pneumoniae* pilus II island (INV104B) antigens described herein may be isolated from *S. pneumoniae* containing the *S. pneumoniae* pilus II island (INV104B), or they may be recombinantly produced, for instance, in a heterologous host.

The heterologous host may be prokaryotic (*e.g.*, a bacterium) or eukaryotic. It is preferably *E. coli,* but other suitable hosts include *Bacillus subtilis, Vibrio cholerae, Salmonella typhi, Salmonella typhimurium, Neisseria lactamica, Neisseria cinerea, Mycobacteria (e.g. M. tuberculosis), S. gordonii, L. lactic,* yeasts, etc.

Recombinant production of polypeptides may be facilitated by adding a tag protein to the *S. pneumoniae* Polypeptide sequences as described herein to be expressed as a fusion protein comprising the tag protein and the polypeptide. For example, recombinant production of polypeptides is facilitated by adding a tag protein to the *S. pneumoniae* pilus II island (INV104B) pilus antigen to be expressed as a fusion protein comprising the tag protein and the S. *pneumoniae* pilus II island (INV104B) pilus antigen. Such tag proteins can facilitate purification, detection and stability of the expressed protein. Tag proteins suitable for use with the compositions described herein include a polyarginine tag (Arg-tag), FLAG-tag, Strep-tag, c-myc-tag, S-tag, calmodulin-binding peptide, cellulose-binding domain, SBP-tag" chitin-binding domain, glutathione S-transferase-tag (GST), maltose-binding protein, transcription termination anti-termination factor (NusA), *E. coli* thioredoxin (TrxA) and protein disulfide isomerase I (DsbA). Preferred tag proteins include GST. A full discussion on the use of tag proteins can be found at Terpe et al., "Overview of tag protein fusions: from molecular and biochemical fundamentals to commercial systems," Appl. Microbiol. Biotechnol. (2003) 60:523-533.

After purification, the tag proteins may optionally be removed from the expressed fusion protein, *i.e.*, by specifically tailored enzymatic treatments known in the art. Commonly used proteases include enterokinase, tobacco etch virus (TEV), thrombin, and factor Xₐ.

### Immunogenic compositions and medicaments

The compositions described herein are preferably immunogenic compositions, and are more preferably vaccine compositions. In some embodiments, the pH of the composition is between 6 and 8, preferably about 7. The pH may be maintained by the use of a buffer. The composition may be sterile and/or pyrogen-free. The composition may be isotonic with respect to humans.

Vaccines as described herein may either be prophylactic (*i.e.* to prevent infection) or therapeutic (*i.e.* to treat infection), but will typically be prophylactic. Accordingly, methods are described for the therapeutic or prophylactic treatment of a *S. pneumoniae* infection in an animal susceptible to such *S. pneumoniae* infection comprising administering to said animal a therapeutic or prophylactic amount of the immunogenic composition described herein. For example, methods are described for the therapeutic or prophylactic treatment of a *S. pneumoniae* infection in an animal susceptible to streptococcal infection comprising administering to said animal a therapeutic or prophylactic amount of the immunogenic compositions described herein.

The compositions described herein can also be used as a medicament. In some embodiments, the medicament is preferably able to raise an immune response in a mammal (*i.e.* it is an immunogenic composition) and is, in some embodiments, a vaccine. The compositions described herein can also be used in the manufacture of a medicament for raising an immune response in a mammal. In some embodiments, the medicament is a vaccine.

The compositions described herein can also be use in kits containing one or more containers of the compositions. Such compositions can be in liquid form or can be lyophilized, as can individual antigens. Suitable containers for the compositions include, for example, bottles, vials, syringes, and test tubes. Containers can be formed from a variety of materials, including glass or plastic. A container may have a sterile access port (for example, the container may be an intravenous solution bag or a vial having a stopper pierceable by a hypodermic injection needle). Such compositions may comprise a first component comprising one or more *S. pneumoniae* Pili Polypeptides. Preferably, the *S. pneumoniae* Pili Polypeptides are in an oligomeric or hyperoligomeric form.

The kit can further contain a second container comprising a pharmaceutically-acceptable buffer, such as phosphate-buffered saline, Ringer's solution, or dextrose solution. It can also contain other materials useful to the end-user, including other buffers, diluents, filters, needles, and syringes. The kit can also contain a second or third container with another active agent, for example an antibiotic.

The kit can also contain a package insert containing written instructions for methods of inducing immunity against *S. pneumoniae* or for treating *S. pneumoniae* infections. The package insert can be an unapproved draft package insert or can be a package insert approved by the Food and Drug Administration (FDA) or other regulatory body.

A delivery device pre-filled with the immunogenic compositions described herein can also be used.

A method for inducing an immune response in a mammal can include the step of administering an effective amount of a composition as described herein. In some embodiments, the immune response is protective and preferably involves antibodies and/or cell-mediated immunity. This immune response will, in some embodiments, induce long lasting (*e.g.*, neutralizing) antibodies and a cell mediated immunity that can quickly respond upon exposure to one or more *S. pneumoniae* antigens. In some embodiments, the method raise a booster response.

Methods of neutralizing *S. pneumoniae* infection in a mammal can include administering to the mammal an effective amount of the immunogenic compositions as described herein, a vaccine as described herein, or antibodies which recognize an immunogenic composition as described herein.

In some embodiments, the mammal is a human. Where the vaccine is for prophylactic use, the human can be a male or a female (either of child bearing age or a teenager). Alternatively, the human may be elderly (*e.g.*, over the age of 50, 55, 60, 65, 70, 75, 80, or 85) and may have an underlying disease such as diabetes or cancer. In some embodiments, where the vaccine is for therapeutic use, the human can be a pregnant female or an elderly adult.

In some embodiments, the uses and methods described herein are for the prevention and/or treatment of a disease caused by *S. pneumoniae.* The compositions may also be effective against other streptococcal bacteria. The compositions may also be effective against other Gram positive bacteria.

Some methods of checking efficacy of therapeutic treatment involves monitoring *S. pneumoniae* bacterial infection after administration of a composition as described herein. A non-limiting means of checking efficacy of prophylactic treatment involves monitoring immune responses against the *S. pneumoniae* antigens in the compositions described herein after administration of the composition.

A non-limiting means of assessing the immunogenicity of the component proteins of the immunogenic compositions described herien is to express the proteins recombinantly and to screen patient sera or mucosal secretions by immunoblot. A positive reaction between the protein and the patient serum indicates that the patient has previously mounted an immune response to the protein in question-that is, the protein is an immunogen. This method may also be used to identify immunodominant proteins and/or epitopes.

Another means of checking efficacy of therapeutic treatment involves monitoring *S. pneumoniae* infection after administration of the compositions described herein. One way of checking efficacy of prophylactic treatment involves monitoring immune responses both systemically (such as monitoring the level of IgG1 and IgG2a production) and mucosally (such as monitoring the level of IgA production) against the *S. pneumoniae* antigens in the compositions described herein after administration of the composition. Typically, *S. pneumoniae* serum specific antibody responses are determined post-immunization, but pre-challenge, whereas mucosal *S. pneumoniae* specific antibody body responses are determined post-immunization and post-challenge.

The vaccine compositions described herein can, in some embodiments, be evaluated in *in vitro* and *in vivo* animal models prior to host, *e.g.,* human, administration.

The efficacy of immunogenic compositions described herein can also be determined *in vivo* by challenging animal models of *S. pneumoniae* infection, *e.g.*, guinea pigs or mice, with the immunogenic compositions. The immunogenic compositions may or may not be derived from the same serotypes as the challenge serotypes. In some embodiments, the immunogenic compositions are derivable from the same serotypes as the challenge serotypes. In some embodiments, the immunogenic composition and/or the challenge serotypes are derivable from the group of *S. pneumoniae* serotypes.

*In vivo* efficacy models include but are not limited to: (i) a murine infection model using human *S. pneumoniae* serotypes; (ii) a murine disease model which is a murine model using a mouse-adapted *S. pneumoniae* strain, such as those strains which are particularly virulent in mice and (iii) a primate model using human *S. pneumoniae* isolates.

The immune response may be one or both of a TH1 immune response and a TH2 response. The immune response may be an improved or an enhanced or an altered immune response. The immune response may be one or both of a systemic and a mucosal immune response. In some embodiments the immune response is an enhanced system and/or mucosal response. An enhanced systemic and/or mucosal immunity is reflected in an enhanced TH1 and/or TH2 immune response. In some embodiments, the enhanced immune response includes an increase in the production of IgG1 and/or IgG2a and/or IgA. In some embodiments, the mucosal immune response is a TH2 immune response. In some embodiments, the mucosal immune response includes an increase in the production of IgA.

Activated TH2 cells enhance antibody production and are therefore of value in responding to extracellular infections. Activated TH2 cells may secrete one or more of IL-4, IL-5, IL-6, and IL-10. A TH2 immune response may result in the production of IgG1, IgE, IgA and memory B cells for future protection.

A TH2 immune response may include one or more of an increase in one or more of the cytokines associated with a TH2 immune response (such as IL-4, IL-5, IL-6 and IL-10), or an increase in the production of IgG1, IgE, IgA and memory B cells. In some embodiments, the enhanced TH2 immune response will include an increase in IgG1 production.

A TH1 immune response may include one or more of an increase in CTLs, an increase in one or more of the cytokines associated with a TH1 immune response (such as IL-2, IFNγ, and TNFβ), an increase in activated macrophages, an increase in NK activity, or an increase in the production of IgG2a. In some embodiments, the enhanced TH1 immune response will include an increase in IgG2a production.

The immunogenic compositions described herein, in particular, immunogenic compositions comprising one or more *S. pneumoniae* Polypeptide antigen, may be used either alone or in combination with other antigens optionally with an immunoregulatory agent capable of eliciting a Th1 and/or Th2 response.

The compositions described herein will generally be administered directly to a patient. Certain routes may be favored for certain compositions, as resulting in the generation of a more effective immune response, in some embodiments, a CMI response, or as being less likely to induce side effects, or as being easier for administration. Direct delivery may be accomplished by parenteral injection (*e.g.* subcutaneously, intraperitoneally, intradermally, intravenously, intramuscularly, or to the interstitial space of a tissue), or by rectal, oral (*e.g.* tablet, spray), vaginal, topical, transdermal (*see, e.g.,* WO 99/27961) or transcutaneous (*see, e.g.,* WO 02/074244 and WO 02/064162), intranasal *(see, e.g.,* WO03/028760), ocular, aural, pulmonary or other mucosal administration.

The compositions described herein may be used to elicit systemic and/or mucosal immunity.

In some embodiments, the immunogenic composition comprises one or more *S. pneumoniae* Polypeptide antigen(s) which elicits a neutralizing antibody response and one or more *S. pneumoniae* Polypeptide antigen(s) which elicits a cell mediated immune response. In this way, the neutralizing antibody response prevents or inhibits an initial *S. pneumoniae* infection while the cell-mediated immune response capable of eliciting an enhanced Th1 cellular response prevents further spreading of the *S. pneumoniae* infection. The immunogenic composition may include one or more *S. pneumoniae* Polypeptide antigens; one or more *S. pneumoniae* pilus or other *S. pneumoniae* antigens; and one or more non-pilus S. *pneumoniae* antigens, *e.g.*, cytoplasmic antigens. In some embodiments, the immunogenic composition comprises one or more *S. pneumoniae* surface antigens or the like and one or other antigens, such as a cytoplasmic antigen capable of eliciting a Th1 cellular response.

Dosage treatment can be a single dose schedule or a multiple dose schedule. Multiple doses may be used in a primary immunization schedule and/or in a booster immunization schedule. In a multiple dose schedule the various doses may be given by the same or different routes *e.g.* a parenteral prime and mucosal boost, a mucosal prime and parenteral boost, *etc.*

The compositions described herein may be prepared in various forms. For example, the compositions may be prepared as injectables, either as liquid solutions or suspensions. Solid forms suitable for solution in, or suspension in, liquid vehicles prior to injection can also be prepared (*e.g.* a lyophilized composition). The composition may be prepared for topical administration *e.g.* as an ointment, cream or powder. The composition may be prepared for oral administration *e.g.* as a tablet or capsule, as a spray, or as a syrup (optionally flavored). The composition may be prepared for pulmonary administration *e.g.* as an inhaler, using a fine powder or a spray. The composition may be prepared as a suppository or pessary. The composition may be prepared for nasal, aural or ocular administration *e.g.* as drops. The composition may be in kit form, designed such that a combined composition is reconstituted just prior to administration to a patient. Such kits may comprise one or more antigens in liquid form and one or more lyophilized antigens.

Immunogenic compositions used as vaccines comprise an immunologically effective amount of antigen(s), as well as any other components, such as antibiotics, as needed. By 'immunologically effective amount', it is meant that the administration of that amount to an individual, either in a single dose or as part of a series, is effective for treatment or prevention, or increases a measurable immune response or prevents or reduces a clinical symptom. This amount varies depending upon the health and physical condition of the individual to be treated, age, the taxonomic group of individual to be treated (*e.g.* non-human primate, primate, *etc.),* the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

### Further Components of the Composition

The compositions described herein can, in addition to the components mentioned above, comprise one or more 'pharmaceutically acceptable carriers', which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. A thorough discussion of pharmaceutically acceptable excipients is available in Gennaro (2000) Remington: The Science and Practice of Pharmacy. 20th ed., ISBN: 0683306472.

### Adjuvants

Vaccines as described herein may be administered in conjunction with other immunoregulatory agents. In some embodiments, compositions further comprise one or more adjuvants. Adjuvants for use with the vaccines described herein include, but are not limited to, one or more of the following set forth below:

### Mineral Containing Compositions

Mineral containing compositions suitable for use as adjuvants with the compositions disclosed herein include mineral salts, such as aluminum salts and calcium salts. Including mineral salts such as hydroxides (*e.g.* oxyhydroxides), phosphates (*e.g.* hydroxyphosphates, orthophosphates), sulfates, *etc.* (*see, e.g.*, chapters 8 & 9 of Vaccine Design... (1995) eds. Powell & Newman. ISBN: 030644867X. Plenum.), or mixtures of different mineral compounds (*e.g.* a mixture of a phosphate and a hydroxide adjuvant, optionally with an excess of the phosphate), with the compounds taking any suitable form *(e.g.* gel, crystalline, amorphous, *etc.),* and with absorption to the salt(s) being preferred. The mineral containing compositions may also be formulated as a particle of metal salt (WO 00/23105).

Aluminum salts may be included in vaccines described herein such that the dose of Al³⁺ is between 0.2 and 1.0 mg per dose.

### Oil-Emulsions

Oil-emulsion compositions suitable for use as adjuvants with the compositions described herein include squalene-water emulsions, such as MF59 (5% Squalene, 0.5% Tween S0, and 0.5% Span 85, formulated into submicron particles using a microfluidizer). *See* WO90/14837. *See also,* Podda, "The adjuvanted influenza vaccines with novel adjuvants: experience with the MF59-adjuvanted vaccine", Vaccine (2001) 19: 2673-2680; Frey et al., "Comparison of the safety, tolerability, and immunogenicity of a MF59-adjuvanted influenza vaccine and a non-adjuvanted influenza vaccine in non-elderly adults", Vaccine (3003) 31:4334-4237. MF59 is used as the adjuvant in the FLUAD™ influenza virus trivalent subunit vaccine.

In some embodiments adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80™ (polyoxyelthylenesorbitan monooleate), and/or 0.25-1.0% Span 85™ (sorbitan trioleate), and, optionally, N-acetylmuramyl-L-alanyl-D-isogluatminyl-L-alanine-2-(1'-2'-dipalmitoyl-*sn*-glycero-3-huydroxyphosphophoryloxy)-ethylamine (MTP-PE), for example, the submicron oil-in-water emulsion known as "MF59" (International Publication No. WO 90/14837; U.S. Patent Nos. 6,299,884 and 6,451,325 ; and Ott et al., "MF59 -- Design and Evaluation of a Safe and Potent Adjuvant for Human Vaccines" in Vaccine Design: The Subunit and Adjuvant Approach (Powell, M.F. and Newman, M.J. eds.) Plenum Press, New York, 1995, pp. 277-296). MF59 contains 4-5% w/v Squalene (*e.g*. 4.3%), 0.25-0.5% w/v Tween 80™, and 0.5% w/v Span 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 µg/dose, more preferably 0-250 µg/dose and most preferably, 0-100 µg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 µg MTP-PE per dose, and so on. MF69, another submicron oil-in-water emulsion for use herein, contains 4.3% w/v squalene, 0.25% w/v Tween 80™, and 0.75% w/v Span 85™ and optionally MTP-PE. Yet another submicron oil-in-water emulsion is MF75, also known as SAF, containing 10% squalene, 0.4% Tween 80™, 5% pluronic-blocked polymer L121, and thr-MDP, also microfluidized into a submicron emulsion. MF75-MTP denotes an M75 formulation that includes MTP, such as from 100-400 µg MTP-PE per dose.

Submicron oil-in-water emulsions, methods of making the same and immunostimulating agents, such as muramyl peptides, for use in the compositions, are described in detail in International Publication No. WO 90/14837 and U.S. Patent Nos. 6,399,584 and 6,451,325.

Complete Freund's adjuvant (CFA) and incomplete Freund's adjuvant (IFA) may also be used as adjuvants with the compositions disclosed herein.

### Saponin Formulations

Saponin formulations, may also be used as adjuvants with the compositions disclosed herein. Saponins are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using High Performance Thin Layer Chromatography (HP-TLC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in U.S. Patent No. 5,057,540. Saponin formulations may also comprise a sterol, such as cholesterol (*see* WO96/33739).

Combinations of saponins and cholesterols can be used to form unique particles called Immunostimulating Complexs (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP0109942, WO 96/11711 and WO 96/33739. Optionally, the ISCOMS may be devoid of additional detergent. *See* WO 00/07621.

A review of the development of saponin based adjuvants can be found at Ban, et al., "ISCOMs and other saponin based adjuvants", Advanced Drug Delivery Reviews (1998) 32:247-271. *See also* Sjolander, et al., "Uptake and adjuvant activity of orally delivered saponin and ISCOM vaccines", Advanced Drug Delivery Reviews (1998) 32:321-338.

### Virosomes and Virus Like Particles (VLPs)

Virosomes and Virus Like Particles (VLPs) can also be used as adjuvants with the compositions disclosed herein. These structures generally contain one or more proteins from a virus optionally combined or formulated with a phospholipid. They are generally non-pathogenic and non-replicating, and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO 03/024480, WO 03/024451, and Niikura et al., "Chimeric Recombinant Hepatitis E Virus-Like Particles as an Oral Vaccine Vehicle Presenting Foreign Epitopes", Virology (2002) 293:273-250; Lenz et al., "Papillomarivurs-Like Particles Induce Acute Activation of Dendritic Cells", Journal of Immunology (2001) 5246-5355; Pinto, et al., "Cellular Immune Responses to Human Papillomavirus (HPV)-16 L1 Healthy Volunteers Immunized with Recombinant HPV-16 L1 Virus-Like Particles", Journal of Infectious Diseases (2003) 188:327-338; and Gerber et al., "Human Papillomavirus Virus-Like Particles Are Efficient Oral Immunogens when Coadministered with Escherichia coli Heat-Labile Enterotoxin Mutant R192G or CpG", Journal of Virology (2001) 75(10):4752-4760. Virosomes are discussed further in, for example, Gluck et al., "New Technology Platforms in the Development of Vaccines for the Future", Vaccine (2002) 20:B10-B16. Immunopotentiating reconstituted influenza virosomes (IRIV) are used as the subunit antigen delivery system in the intranasal trivalent INFLEXAL™ product {Mischler & Metcalfe (2002) Vaccine 20 Suppl 5:B17-23} and the INFLUVAC PLUS™ product.

### Bacterial or Microbial Derivatives

Adjuvants suitable for use with the compositions described herein include bacterial or microbial derivatives such as:

### (a) Non-toxic derivatives of enterobacterial lipopolysaccharide (LPS)

Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (*see* EP 0 689 454). Other non-toxic LPS derivatives include monophosphoryl lipid A mimics, such as aminoalkyl glucosaminide phosphate derivatives *e.g.* RC-529. *See* Johnson et al. (1999) Bioorg Med Chem Lett 9:2273-2278.

### (b) Lipid A Derivatives

Lipid A derivatives include derivatives of lipid A from *Escherichia coli* such as OM-174. OM-174 is described for example in Meraldi et al., "OM-174, a New Adjuvant with a Potential for Human Use, Induces a Protective Response with Administered with the Synthetic C-Terminal Fragment 242-310 from the circumsporozoite protein of Plasmodium berghei", Vaccine (2003) 21:2485-2491; and Pajak, et al., "The Adjuvant OM-174 induces both the migration and maturation of murine dendritic cells in vivo", Vaccine (2003) 21:836-842.

### (c) Immunostimulatory oligonucleotides

Immunostimulatory oligonucleotides suitable for use as adjuvants with the compositions described herein include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.

The CpG's can include nucleotide modifications/analogs such as phosphorothioate modifications and can be double-stranded or single-stranded. Optionally, the guanosine may be replaced with an analog such as 2'-deoxy-7-deazaguanosine. *See* Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393-2400; WO02/26757 and WO99/62923 for examples of possible analog substitutions. The adjuvant effect of CpG oligonucleotides is further discussed in Krieg, "CpG motifs: the active ingredient in bacterial extracts?", Nature Medicine (2003) 9(7): 831-835; McCluskie, et al., "Parenteral and mucosal prime-boost immunization strategies in mice with hepatitis B surface antigen and CpG DNA", FEMS Immunology and Medical Microbiology (2002) 32:179-185; WO98/40100; U.S. Patent No. 6,207,646; U.S. Patent No. 6,239,116 and U.S. Patent No. 6,429,199.

The CpG sequence may be directed to TLR9, such as the motif GTCGTT (SEQ ID NO:22) or TTCGTT (SEQ ID NO:23). *See* Kandimalla, et al., "Toll-like receptor 9: modulation of recognition and cytokine induction by novel synthetic CpG DNAs," Biochemical Society Transactions (2003) 31 (part 3): 654-658. The CpG sequence may be specific for inducing a Th1 immune response, such as a CpG-A ODN, or it may be more specific for inducing a B cell response, such a CpG-B ODN. CpG-A and CpG-B ODNs are discussed in Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8):4061-4068; Krieg, "From A to Z on CpG", TRENDS in Immunology (2002) 23(2): 64-65 and WO01/95935. Preferably, the CpG is a CpG-A ODN.

In some embodiments, the CpG oligonucleotide is constructed so that the 5' end is accessible for receptor recognition. Optionally, two CpG oligonucleotide sequences may be attached at their 3' ends to form "immunomers". *See, e.g.,* Kandimalla, et al., "Secondary structures in CpG oligonucleotides affect immunostimulatory activity", BBRC (2003) 306:948-953; Kandimalla, et al., "Toll-like receptor 9: modulation of recognition and cytokine induction by novel synthetic GpG DNAs", Biochemical Society Transactions (2003) 31(part 3):664-658; Bhagat et al., "CpG penta- and hexadeoxyribonucleotides as potent immunomodulatory agents" BBRC (2003) 300:853-861 and WO 03/035836.

### (d) ADP-ribosylating toxins and detoxified derivatives thereof.

Bacterial ADP-ribosylating toxins and detoxified derivatives thereof may be used as adjuvants with the compositions described herein. In some embodiments, the protein is derived from *E. coli* (*i.e.,* E. coli heat labile enterotoxin "LT), cholera ("CT"), or pertussis ("PT"). The use of detoxified ADP-ribosylating toxins as mucosal adjuvants is described in WO95/17311 and as parenteral adjuvants in WO98/42375. In some embodiments, the adjuvant is a detoxified LT mutant such as LT-K63, LT-R72, and LTR192G. The use of ADP-ribosylating toxins and detoxified derivatives thereof, particularly LT-K63 and LT-R72, as adjuvants can be found in the following references Beignon, et al., "The LTR72 Mutant of Heat-Labile Enterotoxin of Escherichia coli Enhances the Ability of Peptide Antigens to Elicit CD4+ T Cells and Secrete Gamma Interferon after Coapplication onto Bare Skin," Infection and Immunity (2002) 70(6):3012-3019; Pizza, et al., "Mucosal vaccines: non toxic derivatives of LT and CT as mucosal adjuvants," Vaccine (2001) 19:2534-2541; Pizza, et al., "LTK63 and LTR72, two mucosal adjuvants ready for clinical trials," Int. J. Med. Microbiol (2000) 290(4-5):455-461; Scharton-Kersten et al., "Transcutaneous Immunization with Bacterial ADP-Ribosylating Exotoxins, Subunits and Unrelated Adjuvants," Infection and Immunity (2000) 68(9):5306-5313; Ryan et al., "Mutants of Escherichia coli Heat-Labile Toxin Act as Effective Mucosal Adjuvants for Nasal Delivery of an Acellular Pertussis Vaccine: Differential Effects of the Nontoxic AB Complex and Enzyme Activity on Th1 and Th2 Cells," Infection and Immunity (1999) 67(12):6270-6280; Partidos et al., "Heat-labile enterotoxin of Escherichia coli and its site-directed mutant LTK63 enhance the proliferative and cytotoxic T-cell responses to intranasally co-immunized synthetic peptides," Immunol. Lett. (1999) 67(3):209-216; Peppoloni et al., "Mutants of the Escherichia coli heat-labile enterotoxin as safe and strong adjuvants for intranasal delivery of vaccines," Vaccines (2003) 2(2):285-293; and Pine et al., (2002) "Intranasal immunization with influenza vaccine and a detoxified mutant of heat labile enterotoxin from Escherichia coli (LTK63)," J. Control Release (2002) 85(1-3):263-370. Numerical reference for amino acid substitutions is preferably based on the alignments of the A and B subunits of ADP-ribosylating toxins set forth in Domenighini et al., Mol. Microbiol (1995) 15(6):1165-1167 .

### Bioadhesives and Mucoadhesives

Bioadhesives and mucoadhesives may also be used as adjuvants with the compositions described herein. Suitable bioadhesives include esterified hyaluronic acid microspheres (Singh et al. (2001) J. Cont. Rele. 70:267-276) or mucoadhesives such as crosslinked derivatives of poly(acrylic acid), polyvinyl alcohol, polyvinyl pyrollidone, polysaccharides and carboxymethylcellulose. Chitosan and derivatives thereof may also be used as adjuvants with the compositions described herein. *See, e.g.,* WO 99/27960.

### Microparticles

Microparticles may also be used as adjuvants with the compositions described herein. Microparticles (*i.e*. a particle of ∼100nm to ∼150µm in diameter, more preferably ∼200nm to ∼30µm in diameter, and most preferably ∼500nm to ∼10µm in diameter) formed from materials that are biodegradable and non-toxic (*e.g*. a poly(α-hydroxy acid), a polyhydroxybutyric acid, a polyorthoester, a polyanhydride, a polycaprolactone, etc.), with poly(lactide co glycolide) are preferred, optionally treated to have a negatively-charged surface (*e.g*. with SDS) or a positively-charged surface (*e.g*. with a cationic detergent, such as CTAB).

### Liposomes

Examples of liposome formulations suitable for use as adjuvants are described in U.S. Patent No. 6,090,406, U.S. Patent No. 5,916,588, and EP 0 626 169.

### Polyoyethylene ether and Polyoxyethylene Ester Formulations

Adjuvants suitable for use with the compositions described herein include polyoxyethylene ethers and polyoxyethylene esters. *See* WO99/52549. Such formulations further include polyoxyethylene sorbitan ester surfactants in combination with an octoxynol (WO01/21207) as well as polyoxyethylene alkyl ethers or ester surfactants in combination with at least one additional non-ionic surfactant such as an octoxynol (WO 01/21152).

In some embodiments polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### Polyphosphazene (PCPP)

PCPP formulations are described, for example, in Andrianov et al., "Preparation of hydrogel microspheres by coacervation of aqueous polyphophazene solutions", Biomaterials (1998) 19(1-3):109-115 and Payne et al., "Protein Release from Polyphosphazene Matrices", Adv. Drug. Delivery Review (1998) 31(3):185-196.

### Muramyl peptides

Examples of muramyl peptides suitable for use as adjuvants with the compositions described herein include N-acetyl-muramyl-L-threonyl-D-isoglutamine (thr-MDP), N-acetyl-normuramyl-1-alanyl-d-isoglutamine (nor-MDP), and N-acetylmuramyl-1-alanyl-d-isoglutaminyl-1-alanine-2-(1'-2'-dipalmitoyl-sn-glycero-3-hydroxyphosphoryloxy)-ethylamine MTP-PE).

### Imidazoquinolone Compounds.

Examples of imidazoquinolone compounds suitable for use adjuvants with the compositions described herein include Imiquamod and its homologues, described further in Stanley, "Imiquimod and the imidazoquinolones: mechanism of action and therapeutic potential," Clin Exp Dermatol (2002) 27(7):571-577 and Jones, "Resiquimod 3M," Curr Opin Investig Drugs (2003) 4(2):214-218.

The compositions described herein may also include combinations of the adjuvants identified above. For example, the following adjuvant compositions may be used with the compositions described herein:

(1) a saponin and an oil-in-water emulsion (WO 99/11241);

(2) a saponin (*e.g*.., QS21) + a non-toxic LPS derivative (*e.g.* 3dMPL) (*see* WO 94/00153);

(3) a saponin (*e.g*.., QS21) + a non-toxic LPS derivative (*e.g*. 3dMPL) + a cholesterol;

(4) a saponin (*e.g*. QS21) + 3dMPL + IL-12 (optionally + a sterol) (WO 98/57659);

(5) combinations of 3dMPL with, for example, QS21 and/or oil-in-water emulsions (*See* European patent applications 0835318, 0735898 and 0761231);

(6) SAF, containing 10% Squalane, 0.4% Tween 80, 5% pluronic-block polymer L121, and thr-MDP, either microfluidized into a submicron emulsion or vortexed to generate a larger particle size emulsion;

(7) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™);

(8) one or more mineral salts (such as an aluminum salt) + a non-toxic derivative of LPS (such as 3dPML).

(9) one or more mineral salts (such as an aluminum salt) + an immunostimulatory oligonucleotide (such as a nucleotide sequence including a CpG motif). Combination No. (9) is a preferred adjuvant combination.

### Human Immunomodulators

Human immunomodulators suitable for use as adjuvants with the compositions described herein include cytokines, such as interleukins (*e.g*. IL-1, IL-2, IL-4, IL-5, IL-6, IL-7, IL-12, *etc*.), interferons (*e.g*. interferon-γ), macrophage colony stimulating factor, and tumor necrosis factor.

Aluminum salts and MF59 are preferred adjuvants for use with injectable influenza vaccines. Bacterial toxins and bioadhesives are preferred adjuvants for use with mucosally-delivered vaccines, such as nasal vaccines.

The immunogenic compositions described herein may be administered in combination with an antibiotic treatment regime. In some embodiments, the antibiotic is administered prior to administration of the antigen as described herein or the composition comprising the one or more of the antigens as described herein.

In some embodiments, the antibiotic is administered subsequent to the administration of the one or more antigens as described herein or the composition comprising the one or more antigens as described herein. Examples of antibiotics suitable for use in the treatment streptococcal infections include but are not limited to penicillin or a derivative thereof or clindamycin or the like.

### EXAMPLES

### Materials and Methods

### S. pneumoniae Chromosomal DNA Extraction

The *S. pneumoniae* strains listed in Table 1 were grown in 200 mL liquid culture (THYE medium) until the OD600 reached between 0.25 to 0.5. Next, the samples were centrifuged at 6000 RPM for 15-20 minutes (some pellets were stored at this point at -20°C). The pellets were then resuspended with 2.7 mL (for a 3mL final volume) of 50 mM EDTA at pH 8.0 and transferred to a 15 mL Falcon™ tube (BD Biosciences; Bedford, MA). 0.55 mL of freshly prepared Lysozyme (12 mg/mL Sigma L-6876 in 50 mM EDTA pH 8.0 (Sigma-Aldrich Co.; St. Louis, MO)) and 50 µL of 5000 U/mL Mutanolysin (Sigma M-9901) in water were then sequentially added to the Falcon tube. These samples were incubated for one hour at 37°C.

After incubation, 3.6 mL of Nuclei Lysis Solution (Wizard^{®} Genomic DNA Purification Kit (Promega Corp.; Madison, WI)) was added and the samples were mixed by inversion six-times. The samples were then incubated for 5 minutes at 80°C, then cooled to room temperature. Next 18 µL of RNase solution (Wizard® Genomic DNA Purification Kit) was added and the samples were mixed by inversion ten-times. The samples were then incubated for thirty minutes at 37°C.

Each sample was then divided into six 1.2 mL Eppendorf tubes. To each Eppendorf tube was added 0.2 mL of Protein Precipitation Solution (Wizard® Genomic DNA Purification Kit) and mixed by inversion ten-times. Immediately after the tenth inversion, the samples were centrifuged at room temperature in an Eppendorf centrifuge for three minutes at full speed. The supernatants from the Eppendorf tubes were then pooled into a 15 mL Falcon tube (about 8 mL was typically recovered). Next 0.6 vol. of isopropanol was added and the samples were mixed by inversion ten-times.

Precipitated DNA was recovered by inserting a Pasteur pipet that had been heat sealed and bent in a flame into the Falcon tube and hooking the pellet. The precipitated DNA pellet was then washed with Ethanol 70%. Finally, the precipitated DNA pellet was dissolved into 3 mL ofTE (10 mM Tris HCl, 1 mM EDTA, pH 8.0).

### PCR Assays

The details of PCR are well known to those of skill in the art. (*See* Mark A. Valasek, M.A., & Repa, J.J. (2005) Advan. Physiol. Educ. 29, 151-159; Ausubel, F.M., Brent, R., Kingston, R.E., Moore, D.D., Seidman, J.G., Smith, J.A. & Struhl, K, Current Protocols in Molecular Biology, Hoboken, NJ:Wiley, 2005.). The specific parameters used herein are as follows (50 µL final volume of amplification):

PCR Amplification of Genomic DNA

### Reagents

5 µL Buffer (10x concentrated)
0.5 µL BSA (0.5 mg/ml)
1 µL dNTP (10 mM concentrated)
1.5 µL oligo @ 10 pmol/µL concentration (15 pmoles)
1.5 µL oligo @ 10 pmol/µL concentration (15 pmoles)
0.25 µL *Tag* polymerase (F. Hoffmann-La Roche Ltd; Basel, Switzerland)
50 ng genomic DNA
water to 50 µL

### Cycle parameters:

94°C for 3 minutes (one cycle)
94°C for 30 seconds, 52°C for 30 seconds, 72°C for 1 minute and 20 seconds (6 cycles)
94°C for 30 seconds, 58°C for 30 seconds, 72°C for 1 minute and 20 seconds (30 cycles)
72°C for 8 minutes (one cycle)

### PCR Amplification of Bacterial (Colony) DNA

### Reagents

5 µL Buffer (10x concentrated)
0.5 µL BSA (0.5 mg/ml)
1 µL dNTP (10 mM concentrated)
1.5 µL oligo @ 10 pmol/µL concentration (15 pmoles)
1.5 µL oligo @ 10 pmol/µL concentration (15 pmoles)
0.25 µL *Taq* polymerase (F. Hoffmann-La Roche Ltd; Basel, Switzerland)
40.25 µL water
bacteria

### Cycle parameters

94°C for 8 minutes (one cycle)
94°C for 30 seconds, 52°C for 30 seconds, 72°C for 1 minute and 20 seconds (6 cycles)
94°C for 30 seconds, 58°C for 30 seconds, 72°C for 1 minute and 20 seconds (30 cycles)
72°C for 8 minutes (one cycle)

### DNA Probe Preparation and Hybridization

DNA samples were initially prepared (for steps 1 and 2) in parallel with separate containers for Cy3 and Cy5 labeling.

### (1) Pre-anneal primer to DNA

One µg genomic DNA, 2.6 µL random nonamers (GE Healthcare; Buckinghamshire, UK (formerly Amersham Pharmacia)), and 1.5 µL spikes (control DNAs) were combined and water was added to a final volume of 29.5 µL. The mixture was heated to 70°C for five minutes, then cooled to room temperature for five minutes. Finally, the mixture was centrifuged briefly.

### (2) Reaction

4 mL NEB2 buffer, 2 mL Nucleotide Mix (2mM dATP, dGTP, dTTP and 1mM dCTP) (individually obtained from GE Healthcare; Buckinghamshire, UK), and 2 mL dCTP Cy3 (or Cy5) (GE Healthcare; Buckinghamshire, UK (formerly Amersham Pharmacia) were added to the mixture from part (1). The combination was mixed gently and 2.5 mL DNA Polymerase I, Large (Klenow) Fragment (New England Biolabs, Inc.; Ipswich, MA) was added. The combination was gently mixed further then incubated at 37°C for 2.5 hours. Finally, the mixture was centrifuged briefly.

The two labeling reaction solutions were then combined and unincorporated nucleotides were removed by running the solution through a Qiaquick PCR clean-up spin column (Qiagen, Inc.; Valencia, CA). The samples were eluted from the column with two 30µL portions of EB buffer. Volume was reduced to 7.5 µL in a speed vacuum.

### (3) Pre-hybridization

Aluminum slides were incubated for two hours at 42°C in a 5X SSC, 50% formamide, 0.2% SDS solution. Then the slides were dipped in water three-times in one dish, and two more times in a separate dish. These prepared slides were blown dry with nitrogen.

### (4) Hybridization

The DNA samples from step 2 were heat denatured for two minutes at 95°C. Then the tube was spun quickly. A hybridization mix containing 1 vol. of version 11 buffer and 2 vol. 100% formamide (deionized) was prepared and 22.5 µL of this mix was added to each sample. Then the samples were mixed, spun briefly and incubated for one hour at 42°C. Finally, the slides prepared at step 3 were loaded with sample and incubated overnight at 42°C.

### (5) Post-hybridization washes

The slides prepared at step 4 were submerged in a 1X SSC/0.2% SDS solution at 55°C (after cover slips were removed). The slides were then removed and placed in a cradle in a second tray containing the same solution. The slides were incubated in the second tray at room temperature for five minutes on an orbiting shaker. Next, the cradle and slides were transferred to a third tray containing a 0.1X SSC/0.2% SDS solution at 55°C. The cradle was dipped five times in the solution in the third tray and then incubated at room temperature for ten minutes. The slides were then transferred to a fourth tray containing the same solution as the third tray where the slides were again dipped five times and incubated at room temperature for five minutes. Next the cradle and slides were transferred to a fifth tray containing a 0.1X SSC solution at 55°C, where the cradle and slides were dipped five times. The cradle and slides were then transferred to a sixth tray containing the same solution as the fifth tray where the slides were again dipped five times. Finally, the cradle and slides were transferred to a seventh tray containing water, where the cradle and slides were dipped twice quickly and then dried in the cradle with nitrogen gas.

### Microarray Data Analysis Protocols for Comparative Genomic Hybridization of Genomic DNA of S. Pneumoniae Strains

### Slide Acquisition

Two color images of hybridized slides were acquired with the microarray scanner ScanArray (PerkinElmer Life and Analytical Sciences; Shelton, CT) at 10 µm of resolution. Acquisition parameters (photomultiplier gain and laser power) were adjusted to maintain a comparable fluorescence level between the two fluorophores channels (measuring the intensity level of specific control spots and the mean intensity of all the spots).

### Image Analysis

Images were analyzed with the software Genepix 5.x (Molecular Devices Corp.; Sunnyvale, CA). Automatic and manually curated flagging procedures for no good quality spots were applied. From the acquired images, the Genepix software extracted the intensity levels and computed default statistical parameters for each feature spotted on the microarray slide.

### Data Management and Quality Check

Data management was performed by a local installation of BASE (BioArray Software Environment, rel 1.2.10, Lund University). The data analysis consisted of two phases:

(a) Background correction and normalization-In this procedure, the local background intensity was subtracted to each spot intensity. The intensities were then normalized to the mean or median intensity of all the spots. The background correction and the normalization procedure (plug-in rel. 1.4) were applied in BASE with the following parameters:

| | |
|---|---|
| Experimental Normalization: | Experimental Median |
| Experimental Background: | Background local |
| Threshold to correct (bkg stdev): | 1 |
| New values (bkg stdev): | 1 |
| Symmetric correction (CH1=CH2): | Yes |

(b) Data merging-Intensity values of repeated spots of a single slide or of repeated slides were merged computing the mean intensities of the separated two color channels. Spots that were flagged for low quality level or for background correction were filtered out. The algorithm also evaluated the probability of having different intensity levels between the averages of the two colors intensities (applying a T-test, plug-in rel. 1.7). The resulting dataset was stored in the BASE application and could be exported for other analyses.

### Clustering Analysis

The ratio of the two color intensity levels was transformed in log2 scale.

The computed ratios were organized in a table dataset containing a column for each slide or group of merged slides (experiments), and a row for each spot or group merged spots (genes). The table was uploaded in the software TMeV (rel, 3.1, TIGR) for the clustering analysis.

Three main clustering algorithms were applied:

(a) Hierarchical clustering-This algorithm was applied to discover groups of genes showing similar presence/absence patterns among different experiments. In particular the algorithm was applied with the following settings of parameters:

| | |
|---|---|
| Tree selection: | Gene Tree and /or Sample Tree |
| Distance Metric Selection: | Euclidean distance, Pearson uncentered |

| | |
|---|---|
| Linkage method selection: Average linkage clustering | |

A similarity tree (based on the unweighted pair group method with arithmetic mean algorithm, UPGMA) and a similarity matrix (based on the distance metric selected) were built to represent graphically and numerically the distances between experiments and between genes.

(b) Pavlidis template matching-This algorithm was applied to discover genes showing presence/absence patterns similar to a template pattern. Pavlidis template matching was applied to discover genes showing patterns correlated or anti-correlated to a reference gene among different experiments. The algorithm was applied using the threshold parameters Absolute R (selects paterns that are either correlated or anti-correlated with the template), a threshold p-value of 0.05, Pearson default distance metrics, and hierarchical clustering of the elements in the resulting matched and unmatched groups.

(c) Significance analysis of microamays-This algorithm was applied to discover significant genes showing presence/absence patterns that able to divide the experiments in separated groups (defined *a priori*). The algorithm was applied to discover genes that were specific of a given group of experiments. The algorithm was applied with the following parameters:

| | |
|---|---|
| Statistical test: | Two-class unpaired |
| Number of permutations: | 100 |
| S0: | Tusher et al. method |
| Q-values: | No |
| Imputation engine: | K-nearest neighbour imputer |

Hierarchical clustering of the elements in the resulting matched and unmatched groups

### Example 1: Determination of the presence of the pilus II island (INV104B) in genomic DNA

The determination of the presence of the pilus II island (INV104B) in the *S. Pneumoniae* strains listed in Table 1 was performed on purified genomic DNA or directly on bacteria (colony PCR). The four oligos used for amplification are listed in Table 2.

**Table 2: PCR Oligos**

| **Designation** | **Direction** | **Sequence** |
|---|---|---|
| 1008for | 5' → 3' | GCTGGATCGAGTTTGAAACCAGAA (SEQ ID NO:24) |
| 1009 rev | 3' → 5' | TAAGGATCACCAAAGTCCAAGGCA (SEQ ID NO:25) |
| Int-rev | 3' → 5' | TTTCAGTGTATGTTTTAGTGCTTCA (SEQ ID NO:26) |
| Int-for | 5' → 3' | ATGGCTTCAGGGGCTATGTTCGGTG (SEQ ID NO:27) |

Three diagnostic PCRs were performed for each strain listed in Table 1 with the following PCR Oligo combinations: 1008for-1009rev; 1008for-intrev; and intfor-1009rev. Clones positive for the pilus II island (INV104B) were successfully amplified by 1008for-intrev and intfor-1009rev. Strains negative for the pilus II island (INV104B) were successfully amplified by 1008for-1009rev.

The determination of the presence of the pilus island, i.e., the rlrA islet (as opposed to the pilus II island (INV104B)), in the *S. Pneumoniae* strains listed in Table 1 was performed. Similarly to the pilus II island (INV104B), specific primers were designed and used to amplify defined regions of the rlrA islet. Examples of methods for determining the presence of the rlrA islet are set forth in WO 2007/116322.

Table 1 indicates that a great number of *S. pneumoniae* strains contain one or both pilus and pilus II island (INV104B).

### Example 2: Sequences from 23F. INV200, and OXC141

### 1. Sequence dowmload and assembly

The preliminary sequences of four strains of *S.pneumoniae* were downloaded from the Sanger Web site (*see* worldwide web site "sanger.ac.uk/Projects/Microbes/"). The Sanger sequences are composed by a variable number of non-overlapping contigs. Details for the downloaded sequences are listed in Table 3:

**Table 3: Downloaded Sequences**

| **Strain** | **Serotype** | **ST** | **# of Contigs** | **Total bp** |
|---|---|---|---|---|
| 23F | 23F | 81 | 21 | 2225211 |
| INV104B | 1 | 227 | 68 | 1986609 |
| INV200 | 14 | 9 | 167 | 2022487 |
| OXC141 | 3 | 180 | 120 | 1962139 |

To identify the likely ordering of the contigs, the sequences were aligned against the TIGR4 complete sequence using MUMmer3.19, to form a single pseudo-molecule. To separate two subsequent contigs the following sequence was inserted:
NNNNNCATTCCATTCATTAATTAATTAATGAATGAATGNNNNN (SEQ ID NO:28) SEQ ID NO:28 was designed to (i) generate a stop codon in all six reading frames so that no gene is predicted across junctions and (ii) provide a start site in all frames, pointing toward contigs to predict incomplete genes at their extremities. Contigs that did not align against the TIGR4 genome were put at the end of the pseudochromosome.

### 2. Gene prediction

The genes were predicted using the glimmer3.02 suite. The Hidden Markov Model was trained using the predicted genes of TIGR4 as a training set.

### 3. Protein set comparison

All the proteins of each genome were compared against all the proteins of the other genomes using Fasta. A protein was considered to be conserved if its aminoacidic sequence could be aligned with a percent identity of at least 90% on at least 50% of its length.

The amino acid sequences of OYC141, INV200, and 23F that did not meet this criteria are set forth below. These sequences may provide polypeptides and/or proteins that can be used in compositions or methods of treatment for, diagnosis of, and immunization against *S. pneumoniae* infections.

### 4. Start Codons

The N-terminal residues in the amino acid sequences listed herein are given as the amino acid encoded by the first codon in the corresponding nucleotide sequence. In those sequences in which the first codon is not ATG, it will be understood that the first codon will be translated as methionine when the codon is a start codon, but will be translated as the indicated non-Met amino acid when the sequence is at the C-terminus of a fusion partner. The listed sequences specifically disclose and encompass each of the amino acid sequences listed having a N-terminus methionine residue (*e.g.* a formyl methionine residue) in place of any indicated non-Met residue.

### 5. Sequences Identified from. OXC141

>orf00007
   MEMSFIAQDFDKLNIITVLESRTQAIIRNPMNTRLSSDTESSFNKIVRN (SEQ ID NO: 29)
>orf00009
   MELAETSIVKKNHQIPCIINQKIAQKLIKKTSMTDIDHQLSISTSTVIRKINDFHFEHOFSRLPEIMS (SEX ID NO: 30)
>orf00013
   LFKIGRVYYRQLQEDLLTCCNKYPKLFFFIIISLNSTQSGGVF (SEQ ID NO: 31)
>orf00015 
MKYNKTKYPNIYYYETAKGKRYYVRRSFFFRGKKREKSKSGFTTLPQARAALVELEQQIQ
EQELGINTNLTLDQYWDIYSEKRLSTGRWNDTSYYLNDNLYNNHIKAKFGSILLKNLDRN
EYELFIAEKLQNHTRYTVQTLNSSFMALLNDAVKNGNLLSNRLKGVFIGQSDIPAANKKV
TLKEFKTWIAKAEEIMPKQFYALTYLTIFGLRRGEVFGLRPMDITQNDSGRAILHLRDSR
SNQTLKGKGGLKTKDSERYVCLDDIGTDLIYYLIAEASKIKRKLGIIKEQHKDYITINEK
GGLINPNQLNRNFNLVNEATGLHVTPHMMRHFFTTQSIIAGVPLEQLSQALGHTKVYMTD
RYNQVEDELAEATTDLFLSHIR (SEQ ID NO: 32)
>orf00016 
MSYSYVALDVETANDFRGSVCSIGLVKFKDGNIVDTFYTLINPEEEFDDFNIFIHGITPE
DVLDSPTFPEVRKSIVDFIGLDIVVAHFAQFDMGALKDVYQKYELDFDNIEYICSYRLAK
VALPGQLNYKLKRLAKNLNIELDHHNALSDARASGLILEYLLSTNSFSDLTAFLKEYRYN
KTGLLGQYGFKRKKGYQYKENLIYQPTEEEKAAMNPDHYFYGLYFCFTGKLERMTRKEAN
KAAALVGGIPEKGVTKHTNILVVGEQDWRVVGTDGLSSKMKKAQTLLEKGQDIEIMTEND
FIRLLEE (SEQ ID NO: 33)
>orf00017
   MSLLSLHQCKVFLFYHDFISHGFKIVVGHEFEVIKLCGVIQAF (SEQ ID NO: 34)
>orf00019 
MKNKKPNAERLQEIADYFNVSTDYLLGRTDNPAIAGDSKEYTWQGKTLNVEEMASNVMMF
GGRELTDEKKKIIQSIIEGYLKEAGD (SEQ ID NO: 35)
>orf00020 
MSKLTKEDVLQVSQEIINDAIPVIKDMLDEVFKEYPIDMEIRKAILNSVLVAHKLSTETT
VSLLTELVNAQEN (SEQ ID NO: 36)
>or00021 
MSKELKIIKAKIKTRLIELDMTQAELAKQVSVASSVISELLKYGKGSESVKEKVADVLGI
ENPWENS (SEQ ID NO: 37)
>orf00022 
MDYQILIQPAISVILAIISGLWSYIASKANNKAEIEKQAKEHSHIVEKLEKEFHYQIDTL
KQQHTLELEKVKQAHELRLQELEKVSQIDTETDKAMKMNDLIYKTFTGEVDLDKALKLAD
 KANNHKQKLNKKFIQKTSKKS (SEQ ID NO: 38)
>orf00023 
MNEIFNFHGQEVRTLTIDDEPWFVGKDVADILGYSKARNAITLHVDEEDALKQGIPTSGG
TQDMLIINESGLYSLILSSKLPQAKEFKRWVTSEVLPAIRKQGGFIREDLDEDAFIALFT
GQKKLREQQATMLEDIDYLKSEQPIHPSYAQSLLKKRKARVVACLGGIDSPAYADKVFAQ
SVFRQAEIDFKDHFNISRYDLLPKKHADAALAYWMTWEPSTNTKMKIMKLNSFDDV (SEQ ID NO:
39)
>orf00025 
MFEPPILDQLMGVGALLLGFAGACRHIKLQEQRKEEERREEQEFASMIIQGYNHAYERGR
EAERQEIRKNIRRPFKGFTYDNEPPQGLRPEPLALPEPKQSAIRLL (SEQ ID NO: 40)
>orf00026 
MEELIESLDNLIMIVKELEGRESTSRHFITIWENDYKNLLLVKEYLTDYEKLAKDYRYVT
LKNKLLKIEKMELEGRHIYEDMRMKYRANRRKWGARYV (SEQ ID NO: 41)
>orf00027 
VLGMSEIKWIKITTDIFDDEKICLIDALPDPDAILVIWFKILTLAGKHNSNGLLMMTDKV
HYTDEMLATIFRRPLNTVRMAIGVFEQFGMIEIIDGIISLPNWEKHQNVDGMEKIKEQTR
NRVAKYRKKQKNLALGNVTGNVTVTDGNALEEDKDKNKNRLDKDKNKKRITTTSSGSEEN
ILELFQSEFRRLLSGFEIEEINHLLNENDVDLVKEALKTAINSGKPNIKYIGGILRNWQM
NNVTTVEQVRQSEKKNKDKKEEQEAKDEWGY (SEQ ID NO: 42)
>orf00029 
MRRSASMVDNVFEEIALSYRRNTEQQEEFCEKHNIPLKIKILRTESVVCRMCESERIHEEN
QERVNELANAENERERKYYLEKFSLYDEVLKNATLDNFETPTEKEAEKLAFAKRICREWS
EGARNNIVLQGEAGTGKSHLAFAMVKVLSEYTKEIAIFINVTDLLMKIKADFSQEEFLVN
KIASAKFLVLDDLGMEKDSEWSFTILYNILNKRSNTIITTNLISADIQKRYGRPFMSRLM
KGVDKDHLMVFNDLTNKRKQYF (SEQ ID NO: 43)
>orf00030 
MLELYFVYNGHCKFFLGRFDNVDDLIEQMEDHQWAFSAITHPRFQKHIGQRTTRFDYGSK
DCYYLATFSGGE (SEQ ID NO: 44)
>orf00031 
MVGVTYQEIHLFVEFLKEQYGQGRPDYIEALNDLDGLVEVSYREAIERFLEDEVR (SEQ ID NO:
45)
>orf00032 
MMEELKKKVNAVYNWTVEDGKPQPPQDLPQAVKDRVDYFWEMAEDGMTFMGAMECIFAD
EKPTDYELGATKGWLPKSKEFDDWIGYSPSMAQVVIAVYLIYGGN (SEQ ID NO: 46)
>orf00033
   MEETKMNKQELIKKLEERRTIIGNFQGYAVSYFWIYWIVEK (SEQ ID NO: 47)
>orf00035 
MEFVSPIKDNDDIQAMKDYLREWNEMYYMLFITGLNTGLRVGDILTLKVKDVQGWHIKLR
ERKTGKQITRRMTKELKKEMRRYVEGKPFHHFLFKSRQGQNKAITRERAYQIIHEAAEEL
GIDNVGTHTMRKTFGYKYYNKTKDVGTLQKMFNHSSPAITLRYIGIEQAELDDALRNFVI
(SEQ ID NO: 48)
>orf00036 
MYNKPVRPSLKSKKWEKFRDRIMRKHDYLCQESLRYGISVQAEMVHHIFPVSEYPELEFV
EWNCLPLTNKKHNTFHDRVNDRVINQGLYWQKKRKKEFLNFFKNEK (SEQ ID NO: 49)
>orf00037 
LAKPITAKSIKSKVVKQMKDLGTYRKEFEMIIDIFAGMLYQYQKLAQDYANLGYPVTDTY
VNKAGAENERKVPILTAMEILRKDILSYSNQLMMNPKSLGEVVEQEGDSVLTEVLKFKNE
IKKKRVSGNG (SEQ ID NO: 50)
>orf00040 MGNLDKAKEYAQHVLTHREEHCEENILAAERFFRDLENPAFEMDEDMVDFVIHFIENVIV 
HQQGDDMFAVSIRNKPLLLQPWQHFVVVNLFGFYYKGTNERRFKEALIMLARKNGKTSFT
AAIALAYQILDTDSGSKCYIVANSVKQAMEAFGFLRFNVERWNDKNIRIKDNNQEHSITA
NFGDEGSFFIQALANDESRLDSLNGNVIILDEAHTMRNSKKHGLMKKTMSAYRNSMLFVI
STAGDIPTGFLANRLKYCQKVLKQLVTDDSFFIFICKANQSADGDVVNYLDENILKMANP
SWGVTVSLKALKEEAEQAMNDPQTRNEFFNKTLNIFTNSMNAYFNPDEFIASDSCYDWSL
EELARLPIRWYGGADLSRLHDLTAAALYGVYHDGEKDVDICITHAFFPRINAQKKANDDG
IPLFGWQSDGWLTMSNTPTVLYDDIVKWFIKMREKGFKIAAVGMDRKFGREFLTKMKQAR
FKMIDQPQLFYLKSEGFRRIEFKVKNKEFYYLHSDAYEYCVSNVRAIEKVDDAVQYEKLD
GDGGTARIDLFDASVFACIQALANLGKGGDVMRFFD (SEQ ID NO: 51)
>orf00043 
MNEIVLSEHEINLLINKGRVKVILNGEVVTIRQRHMKNLMAETVKWEKQVIDVSQNIVRN
KHFDSLFQNTFR (SEQ ID NO: 52)
>orf00045 
MGIFEKFWKRNKPSKPINMLSHSDLGLSNLMDSYVPLARNPDVVTAVNKIADLVSNMTIH
LMENTDKGDIRIRDGLARKIDINPCKHMTRKSWIFKIVRDLLLYGDGNSVLHVEYEPVTD
YISNLRPFPMREVSFQTDKDSYVISFRGEEYSPDEVVHFVINPDPDILYIGTGFRVTLTD
VVQSLNMATKTKKSFMNGKNIPSLIVKVDSSSAELDSEQGRERIAEKYLSTSRVGAPWIV
PEALLDIQQVKPLSLTDIALNESVELDKRTVAGLLGVPAFILGVGEFNKTEYNNFVNTTV
MSIATTITQTLTRDLLLSSNRYFKLNPRSLFSYNITELSAVAQQMANSAAMRRNEWRDWL
GMAPDPEMEELIVLENFLPQEKLGDQNKLKGGEEENAKAK (SEQ ID NO: 53)
>orf00047 
MQKRNSYRATQFQTREEESGDLVLSGYFIKFDEETELWRGYHEVIKRAGVEKAVTDADIR
ALFNHDDSLVLGRTGNGTLTLGVDDVGLFGDIIINKDDPQAVGAYARVKRGDVIGCSFGF
IPVKIETEEREDGSYLDTVLELEIFEVSPCTFPAYPQTEIAARQKDFESQKRANREALDK
RKKEIKEKFKL (SEQ ID NO: 54)
>orf00048 
MNKALIFGARMRAKATKVVELEETIEELNKRSVVELEKLDRAKNDEEVLAVEKTVDGLQR
EIEEKEAEKVQLENEIDELDKQIKEQNRKAPTPGKMEERGGKTLGQREAFNHYLRTKEAR
ADGFKSAEGEAIIPVELMTPKEAKQDKTDLTSLVNIVNVKNASGKWSVVKLTDQAMNTVE
ELEENPELAKPTFTKVNYEIKTRRGHLPVSQEFIDDADYDVMGLVAKQTKNQERITKNKE
IAKVLKTATSKSAAGLDGLKDILNVELKTYYNATIVCTQSMFAALDKIKDKDGRYMLQTD
ITSPTGYKFAGRVIVVYPDDIIGESKGDLKAFIGDVGEFATLFDRAQTTVKWQDDKIYGQ
YLATANRFDVVKVDGDAGFYVTYTDAVL (SEQ ID NO: 55)
>orf00050
   LMNVEDKDMSKDDTLTTEVEKEETKVVDGKPEEGDEE (SEQ ID NO: 56)
>orf00051 
MDNVQLLELLKLKLGIATKLRDKPLEKIIEAVKTELEDNLGVLLDLDSSEDQMFVVDFAA
FRYEGGVDMPRHLQWRLHNLQIASKKKVKNVES (SEQ ID NO: 57)
>orf00052 
MWNHEIKLISKKITGKDKLLQPISEDVEVTLLCRKKNVTRSEFYQANQAGLKPSLVVEIR
NFEYENQEFAKFEGKQYRILKTYPIDSEILELTLTEVLK (SEQ ID NO: 58)
>orf00054 
MSNDLADLIAKELAAYSDEVTEEVDKIAEQVTDETVDELKETSPKRYGKYRRSWKKKKLA
NGSFVVFNAVASLTHILENGHLSRNGGRVAGIVHIKPAEEKAIQNFEKRIKEIGK (SEQ ID NO:
59)
>orf00055 
MKLSDFAAILEQANLPVTYRAFKIGNAPDLPYLVYYESSPVINSADNTVNHQIKSVTVEL
AFESKDEDLEERLEELWANHKLFFEVQEETFIETERLYVKSYTVYLY (SEQ ID NO: 60)
>orf00057 
MTQENKVTFGLENVHIAPIKTLAADGVITYGDVFRFPGAMELILDTKGETTPIKADNKNY
HFMNSNEGYEGKLKIPHIIDEFATKILGEIKDPQTGVMTEKADASLTEFAIMFQFEGDKN
KTRYVMYYCFASRPSLGSKTKNGTSTNERELSFKASPRPLDTVVKRSITSADDKDAYDNW
FKKVYEPTAVAA (SEQ ID NO: 61)
>orf00058 
MRKIVLVGDQEYELGTNGYTPIAYKQQFGKDYFQDLFSMLKNQSFMNELNKLETDKELTA
TNIDISMLSDFDMTFFNRLFWTFAKSANPQIKPYEQFFMEMEIFPIQEVGPVLMEMLNAS
MTTKKHQMNQNQLAKKSSQ (SEQ ID NO: 62)
>orf00060 
MAGNIKGIKIEIDGDTQPLQKALKAINKESVNTTNELKQIDKALKFDTGNVILLTQKQEV
LQKQIGITRDKLETLRQAQSKVDEEFKKGNIGSEQYRAFQREVEVTQNVLKGYEGKLASL
TQALEGNGDAAKNNQAQLKELQNEQKLLASESEKVVSSFKLQESQMGANASEADKLALAE
KKIGAQSEIVTRQIENLEKQLSLTKEQYGENSAEANKMEAELNQAKTAYANLNQELGKLG
STAKSNQTQLKELQNEQSQLASEMSKVTSSFKLQESALGSNASEAERNALAQKKIGAQSE
IVSKQISNLEQQLEITKKEFGENSTQANKMESELNQAKTAFNHLNDEMKGTKSAADSTQE
SLSEISRNLRAELLQQFSEKLSAISEKLVEVGKEALEAAAQMQASNAQFTTVFGDMETQA
REALNAIGQEMDIVPERLQGSFTQMASFAKTSGLDTAEALDLTSRATRAAADGAAFYDKS
IESVTESLQSFLKGNFANDAALGISATETTRNAAANKLYGKSFKDLSEAQKQLTLLQMVE
DGNKLSGALGQAARESDGLENVMGNLKQAGTNALSAIGQPLLEMMIPVFQTLATIVKGVA
ELFSSLPAPVKDFVVILGTVVTAVGVIAPIFLSLQALAEFLKISIGEMIIAALPIIGTAI
AIAAAVAAIVAIVKYLWETNEGFRDAVTTVWNAILEVINAVVSEISNFVMSIFGTVVTWW
TENQELIRTSAETVWNAIYTVISTILDILGPLLQAGWDNIQLIITTTWEIIKIVVETAIN
VVLGVIQAVMQIITGDWSGAWETIKGVFSTVWQAIQSIVQTIFSAIQSYISNILNGISGT
VSNIWNSIKDTVSNVLNAISSTVSSVWEGIKSTISSAINGARDAVSSAIEAIKGLFNFNI
SWPHIPLPHFYVSGSANPLDWLSQGVPSIGIEWYAKGGIMTKPTIFGMNGNNIMVGGEAG
NEAVLPLNDKTLGAIGRGIAQTMGGTSPTINITITGNTVREEADISRIADEVAQRIADEL
QRKTQLRGGFT (SEQ ID NO: 63)
>orf00064 
MIKHNELVIDGVRTSSFPFKVIVHDSPSIALGESKTALLEHGGISGAIVQTNKHRELVKK
TYTIYLVKPTEEQMNQFMSLFIREKFWLESERVKTTRLWCYKVNVSDLEEVQPGLYMTKA
TFTCHPTKHFKVTDTQRLTRSGTLNVQGSALAFPKITIVGQSASETSFTIAGQVIRLERL
TESLVMVNNPDNPSFKTTTGKPVKWSGDFITVDPAKVKNIGVVLGPGIQSLEIETVWGWA
(SEQ ID NO: 64)
>orf00068 
LLYLLNEDVRTVRWNGESLHEATSAIVKETMNGDFTLTVKYPISDSGIYQLIQEDMLIKA
PTPVLGAQLFRIKKPVEHNDHLEITAYHISDDVMQRSITQMSVTSQSCGMALSRMVQNTK
TALGDFSFNSDIQDRRTFNTTETETLYSVLLDGKHSIVGTWEGELVRDNFAMTVKKSRGE
NRGVVITTHKNLKDYQRTKNSQNVVTRIHAKSTFKPEGAEKETTIRVTVDSPLINSYPYI
NEKEYENNNAKTVEELQKWAQSKFSNEGIDKVSDAIKIEAYELDGQVVHMGDTVNLKSWK
HNVDAFKKAIAYEFDALKEEYISLTFDDKAGIGGSRASGGLSSAADAILGVTESAQEIAL
EKALQNADLDFDHKAGLLRQEISDDIELAKARAEEVKRELSDTINQRFNSFDNGPLKETK
RKAEEALRNAGASSSLAQESKRIGLDSVARLEAFKSQTTSAQTALSGDLDALKRTIANDI
RPKQAQAEAEIAKQVEALSRTKNELAGASTLLAQEAKRIELDSVARLEAFKSQTTSAQTA
LSGDLDALKRTIANDIRPKQAQAEAEIAKQVEALSRTKNELAGASTLLAQEAKRIELDSV
ARLEAFKSQTTSAQTALSGDLDALKRTIVNDIRPKQAQAEAEIAKQVEALSRTKNELAGV
KSAQATYKETTTRRLSELTNLANGKASKSELTQTAEELSSKIASVQASGRNLFLNSLFKQ
DIPKTGIWTTSTYTATIDSESKYLGHKALKIIGLNPSGRDGGNPKVTYPALGQFGKVIPG
STTNQDVTISFYAKANKNGIMLRSRLGNIGYKTGNVTLSTEIKRYAVHIPKGWTNESKRT
TNEWLFNFNQEGTVWIWMPKFEISDVDTSYSEAPEDIEGQISTVESTFKQRANSLEAGVS
RLTEGLRTKADISSLNVTAENIRQSVKSLETDTQNKLNQKLSQAEFEVRAGSIRQEILNA
TKDKADKTLVVSEAGKLREEFSKMKVGGRNLWIKSKTVGAVIEKLPENHVTGQKECYRLE
NNSTLTFNLEPDFSSRLYQKVTFSAWIKYENVVQGRNFWNVFNCFKHYLFRKNSETGVQS
GPDYDTLGRYKGSADWKYITFTYDYSEKTNFDQLKTLLRFNLEGATSGTAWVTGIKVEIG
SVATDWSPAPEDADGLITEAKATFERTAQGLRTDLSAITEGLRTKVDISALNVTAENIRQ
SVKSLETDTQNKLNQKLSQAEFEVRAGSIRQEILNATKDKADKTLVVSEAGKLREEFSKM
KVGGRNLWIKSKTVGAVIEKLPENHVTGQKECYRLENNSTLTFNLEPDFSSRLYQKVTFS
AWIKYENVVQGRNFWNVFNCFKHYLFRKNSETGVQSGPDYDTLGRYKGSADWKYITFTYD
YSEKTNFDQLKTSLRFNLEGATSGTAWVTGIKVEIGSVATDWSPAPEDADGLITEAKATF
ERTAQGLRTDLSAIQEYVNKDGQRQEALQRYTREESTRQATAVRELVNRDFVGKATYQED
VKGINQRIEAVKTSANKDIASQIASYRQSVDGKFTDISSQITTYKQDVGGQISGLSNRLT
SSEQGTTTQISNLSNRINSNKQGTDNQISNLKTQVATNKDNAERQMGRISDQVSANKANA
DSQFANVTNQLARKVETTDFQRVKETSKLYERILGNTENGIADKVARMALTNQLFQVEVA
KNASNGQNLLKGTKDFSGGWKNKGANWKKHAEKYKGVDVLFKNNSWNGVGQEIDAKIGEV
 
YTFSLWMKSDWKNDTVNFYVNRNGSVEKGWGVPSETSVAITSEWKRYSFAFKITVDGFIF
PRVERLNQNTNLYIAGLKLEKGSYATPYTEAPEDTDEAIRSVQSQLTGSWAVQNINSAGD
IISGINLGANGHNRFVGKLTHITGETLIDRAVIKSAMVDKLKTANFEAGSVTTTILDAEA
VTADKVRFDAAFIRKMTANDAFIDQLTSKRIFSTKVESVISSSTFLEAYQGRIGGFTIGR
FAQGRGRWISGINQFSVGMGNGEGGSYNGENTAFWANWGHSWNSPGPNAWYVTTSGNMYC
RNGADFHGKVDFSNSSRANFYGNTTFSRSPVFSNGIELGSKDVLGDGWNPKGGRNAVVWW
NQVGSGSVKYWMEQKSDRRLKENITDTAVKALDKINRLRMVAFDFIENKKHEEIGLIAQE
AETIVPRIVSRDPENPDGYLHIDYTALVPYLIKAIQELNQKIEKMEKTIA (SEQ ID NO: 65)
>orf00069 
MNTEQLNQALQMTIREMSTTSTNSMITSNILSIQLNEQREENQRLQARVDELEALLDEQT
KPADKGE (SEQ ID NO: 66)
>orf00071 
MAETIQNTDNLLDLTKITEPFDLASALRYMKENGEFIRCKNVSDDFYMYRDVQKRPVIVN
GRRQFKDIETVWAFNQWGGTITTINVAVLLNHEFYIMKFDAEGNPDWTNPTVEPKE (SEQ ID NO:
67)
>orf00072
   MQIEFFNFLRSVVQTEDGLVLYALALIVSMEIIDFVTGH (SEQ ID NO: 68)
>orf00073 
MILIPASVLLPEKTGFVFLHSIYLGYIAFTFQSLIENYRKLKGNVTLFQPIVKVFQRLLE
KDDDTKKGE (SEQ ID NO: 69)
>orf00074 
MQQITEIITNGAISILVILAGIAVKAVKEYLVKKGGEKTIKIVEILAKNAVNAVEQVAAE
TGYKGDEKLAQARAKVRAELTKYNISMTDKDLDTFVESAVKQMNDAWKGR (SEQ ID NO: 70)
>orf00075
   MAFNQFNRCVTLSIPTAPNIPTSVVHRTYLHDTAVSDNM (SEQ ID NO: 71)
>orf00079
   MKNREEEWQGIIAKNAILLIIAPFYFLIIVKNGVLLKIKTVTEITAF (SEQ ID NO: 72)
>orf00085 
VEEVEVAEVKNARVSLTGEKTKPMKLAEVTSINVNRTKTEMEEFTRVLGGGVVPGSLVLI
GGDPGIGKSTLLLQVSTQLSQVGTVLYVSGEESAQQIKLRAERLGDIDSEFYLYAETNMQ
SVRAEVERIQPDFLIIDSIQTIMSPEISGVQGSVSQVREVTAELMQLAKTNNIAIFIVGH
VTKEGTLAGPRMLEHMVDTVLYFEGERHHTFRILRAVKNRFGSTNEIGIFEMQSGGLVEV
LNPSQVFLEERLDGATGSSIVVTMEGTRPILAEVQALVTPTMFGNAKRTTTGLDFNRASL
IMAVLEKRAGLLLQNQDAYLKSAGGVKLDEPAIDLAVAVAIASIYKDKPTNPQECFVGEL
GLTGEIRRVNRIEQRINEAAKLGFTKIYVPKNSLTGITLPKEIQVIGVTTIQEVLKKVFA
(SEQ ID NO: 73)
>orf00088 
MGVSIFLALFYMIPALYFLFHIGKKWELPKKVLILSLLGAICSFTSLLLFGIYNHRRKSS
KV (SEQ ID NO: 74)
>orf00094 
VFVALVSITFSLTNFFKILINLTAQVSPQVIDEKILMMDLNLNNYLSTVIQLRQDVYTGI
KILH (SEQ ID NO: 75)
>orf00095 
VNIASLQNGHIFCWQVQHIANKLTSDFWIAEYFLSNQIISWADARMSENPPIPPSIVS (SEQ ID
NO: 76)
>orf00103 
MKIKEQTRKLAAGCSKHCFKVVNGTDEVSSKHCFEVVDGTDEVSSKHCFEVVDRTDEVSN
HIYGKATLTWFEEIFEEYKSLHNKTHITKVV (SEQ ID NO: 77)
>orf00109 MNDDDSRCIHIERDGKTIEFGYLNISSTDRNTSHADGLVGIFNSNFSGVRVRGIAVFLNG PDNLDTTLVGNFQTIWNFRIICIHSQNTCNKG (SEQ ID NO: 78)
>orf00110
   LEFNFCRSIIKNGRDNLPNTNSTSGMATRWANHNWSDDIKDRLKTK (SEQ ID NO: 79)
>orf00114
   MRYDFGKVYKEIRESKGLTQEEVCGNVISRTSLSKIESGKATPKYENMEFLLRQINMSF (SEQ ID NO: 80)
>orf00116 
MNYNLKYLLSGIFVLVFIGFLVWMRYFNQRKEEEHSDVSFEARLDSEVKTLYKQLGLGEE
PHYFLAYRYLHPWFDLAILPPTVEIFLTKIALVMVTPDELLIRNLGNGLTFTSEHHRDLR
QGLIRIPKSEMKEFEIRNWKKFFVFGDFLTIKTSQHSYYLQVRDDGLQKGSLSTKHFSDL
KSQDFLGLLTDKRTF (SEQ ID NO: 81)
>orf00124
   LSLLDLRGSLCLRIYLHEPLITTVSQDFTSLSDISHF (SEQ ID NO: 82)
>orf00125
   MDFKSFIIGLVVGIFGPYMDDLIRKKFLKSSEKKTEKSVKK (SEQ ID NO: 83)
>orf00175
   MGRFILFENLFKPGQLHLAVDMVTDFVSSIHNLKTVF (SEQ ID NO: 84)
>orf00177 
METSISMADFYGKYQNENLELIDVREAHEFQAGHAPGAKNLPLSTLEQGYKELKPDHEYY
VICQGGVRSASTCQFLSSQGLTVTNVEGGMNVWPGQVE (SEQ ID NO: 85)
>orf00179
   LGGKSCLLEDRLCDIAAQTTVAADDVGLFFVQFISFLLDTLSVFDTIVQN (SEQ ID NO: 86)
>orf00183
   MKIKDQTRKLAAGCSKHCFEVVDRTDEVSSKHCFEVADRTDEVSNIYTARRR (SEQ ID NO: 87)
>orf00184 
MKLLSIAISSYNAAAYLHYCVESLVIGGEQVGILIINDGSQDQTQEIAECLASKYPNIVR
AIYQENKGHGGVVNRGLAEASGRYFKVVDSDDWWILVPT (SEQ ID NO: 88)
>orf00185 
LKILETLQELESKGQEMDVFVTNFVYEKEGQSRKKSMSYESVLPVRQIFGWDQVGNFSKG
QYIMMHSLIYRTDLLRASQF (SEQ ID NO: 89)
>orf00186 
MYYLPVDFYRYLIGREDQSVNEQVMIKCIDQQLKVNRLLVDQLDLSQVSHPKMREYLLNH
IEITTVISSTLLNRSETAEHLAKKRQLWTYIQQENPEVFQAIRKTMLSRLTKHSVLPDRK
LSNVVYQITKSVYGFN (SEQ ID NO: 90)
>orf00194 
MSLQIKLKKLAKELSKLLKDSNLETVDKDVLENSQEELQKAVLFLADEKGSEHTAAELID
NLKEVIAKLKANA (SEQ ID NO: 91)
>orf00202
   MKIKEQTRKLAAGCSKHCFEVVDKTDEVSYIYLRQGEADAV (SEQ ID NO: 92)
>orf00213 
MSKEKVILAYSGGLDTSVAITWLKKDYDVVSVCMDVGEGKDLDFIHDKALKVGAVESYVI
DVKDEFATDYVLVALQSHAYYEQKYPLVSALSRPLISKKLVEIAHQTGATTIAHGCTGKG
NDQVEYQIAVAKKANEAKK (SEQ ID NO: 93)
>or00215
   VLDSLVFMGFSMKLIHDLDTHTTHSTAKMLYNVKAIKNDFSIRE (SEQ ID NO: 94)
>or00218 MGQLHFITKLLDIKDTNTQIIDVVNRDSHKEIIAKLDYEAPSCPECRSQMKKYYFQKPSK 
IPYLETTGMPTRILLRKRRFKCYHCSKMMVAETSIVKKNHQIPRIINQKIAQKLIEKISM
TDIAHQLSISTSTVIRKLNDFHFECNFRNLPKIMSWDVETVRGVTVSIGRWR (SEQ ID NO: 95)
>orf00220 
MRYDFGKVYKEIRESKGLTQEEVCGGVLSRTSLSKIESGKTTPKYENMEFLLRQINMSFE
EFEYICHLYQPSQRTEIMQTYLNMTSIIGSNSLVHFFETCQDYLKTHHDLPIEEIRDMLE
VVIYIRQHGAGELSDHAEQVVKKLWRKIEKQDTWYESDLKILNTILFSFPIEYLHLITGK
ILQRLEVYKNYQHLYDLRIAILLNLSTLYLYNQDKNMCKQICYTLLEDAKNKKSYDRLAI
CYVRIGICTDNAKLIQKGFSLLELTEETSMLSHLKKEVETHYQPKKL (SEQ ID NO: 96)
>orf00221 
MNSKELSISMLKKYPCTMQHDQSDCAAAVVSTVLLSYKKELSIMKIREIIGTDMYGTTVS
GIVSGLNKLNFTVKAVRVALEDLTPKLTFPAILQVKNDLGQNHFVVLHSIKRNSKFYVAD
PASGIRKMSSDELGEIYQGITLFMVPNSDFERGKLKGKGLLDLFGRLIFNQKGLISTVIL
ASFVLSIIGILSSLFSKVIMDEVIPYALKNSLYMFLIVFGIVSFLQTLLSAFRQHVLLFL
SRKIDIPVLMGYYDHIIHLPYSFFGSRRVGDVLTRFQDAMTIKNVFTSVSISLVMDITLS
VISAVVLWTINQSLFLILVFMVIVNIILIYCFKKPYKKINHEQMEANGLLNSQLIESIRN
IDTIKSQHDEEQRLNKIEEKFVHTLEIGYKEGVLQNIQSTISSMTSTMGGLLFMGVGALF
IIDGKMTIGDLLVFQTLSQYFTEPIQNLVGLQLTFQEVQVAVSRLQELMEVDREDIALDY
SIRDFTLCDDIEFKDVTFAYGSRPPVIKDFNLRIKQGEKIAFVGESGAGKSTLVRLLLRF
INPSEGKIRIGENDLSDLDYGKLRKKISYIPQTIELFTGTIIDNLKIGNPSVTYEDMVRV
CRIVGIHDTIQRLQNRYGSFVEEGGQNFSGGEKQRLAIARALLSKADLYIFDEATSNLDS
FSEQIIQDLIFNKIMDKTTIVVAHRLSTILRCDKICFLENGTIVEYGTHEELMAKNGKYA
RMVGLQSVQVNQQIQSQAVLDTEEVTYG (SEQ ID NO: 97)
>orf00222 
MAKLEVKDNKKLVLKSVICKKLHDTKVEDVDQEINKFHQHLQLLKAQIFGPLIVKSCGTT
IHDDGLITTDFEFYIQAHNAQQYSNIYDVQDSISVPYCLYVRFEDSPEYLQYAYSKLDLY
VYENDIQTDGIVYTVYVNSSPEKMVVDIFRPIVSL (SEQ ID NO: 98)
>orf00223 
MKLYNKSELRYSRIFFDKRPPAFAFILIISTAIILSGALVGAAYIPKNYIVKANGNSVIT
GTEFLSAIGSGKVVTLHKSEGDMVNAGDVIISLSSGQEGLQASSLNKQLEKLRAKEAIFQ
KFEQSLNEKYNHLSNSGEEQEYYGKVEYYLSQLNSENYNNGTQYSKIQDEYTKLNKITAE
RNQLDADLQTLQNELIQLQQQGDSSSLSDTTSDDDKAKLETKISEITTKIEALKTNITSK
NSEIDSQQSNIKDMNRTYNDPTSQAYNIYAQLISELGTARSNNNKSITELEANLGVATGQ
DKAHSILASNEGTLHYLVPLKQGMSIQQGQTIAEVSGKEKGYYVEAFVLASDISRVSKGA
KVDVAITGVNSQKYGTLKGQVRQIDSGTISQETKEGNISLYKVMIELETLTLKHGSETVI
LQKDMPVEVRIVYDKETYLDWILEMLSFKQ (SEQ ID NO: 99)
>orf00224 
MELVLPNNYVVIDEEEMMYLDGGAIYIPRWAITGAITGAAYAALAAAGGGLQLVLASYG
LRSALVAGIVKGLGVLGIHIGNAFANTVIRSIASAGIGAGADWIFTNIIDGWDGRRDNQL
RIG (SEQ ID NO: 100)
>orf00226 
MKDDQKYLLAGLYSLLVAIFYFPLIESKGIFVSILMAVLLLYLIYFIATVIHIVIIKFIR
KKSFKYLVLYPFTYDGSWRFQPINLLYFPEMVRDVIPINLVQEYCQGQPYGLLKKMLKRI
RLSREISLLLATIIVYFFTHRILPLSVFTFIFSYILLFAQSYLGGNTVWIGNRRLIIDDE
FEKILLSKSYIKEISSARYSEYLTCEYKNLTPIILLAIFENLLDSYLIQNQSKVDLDIFY
KVLPLLYKEKYTMGFNYFVSLNYLLYKVGFLGIIYDNEALRDLSKQYLNKNISELQDGSF
EDGIQDAVASKQIVVINEFIACLNSRCVPSQYDRFFYKDRPYIFSRKSPIKG (SEQ ID NO:
101)
>orf00227 
MKNKRYFFDTILIILLLISTIFCVSPVFIKLDILGTPSHAILTFVLAIPLFYILSQCLHT
LLLLVSSIFCKLRPIYFYFIFVIIIGARKYYRILFHQLMGFSPGVAVFYKESQTTKNLFK
FYYFLYFTTLISYYFFFTFVYDKPLLLPLIPFSIIIALVQKLYRIENQQLFLLKSKVLTI
LESKKDCEFNLQDYHEIWKLQSKSELPCVALSYISLIKPYLSESVREQIDLLEVKRFKKI
NHPISLYGMLDVIKLNLYLRHYNEKNKYESMLKKILEVRPDFVLIEQNIDDSLNSSQPLS
LSLAISEIQLLLEVYMGIKHVSIRR (SEQ ID NO: 102)
>orf00228 
MIRKPIIFLLMLPIWGLWIELHLLVSNLQLNLEIPFDFVVSTSLTFFVLILSKIVLDILY
ALKDLYKKEALITIFPFIFIRRKKVNVRFSPYFSFHRKSLSPDDLRSRIIWSFILEIAII
LVFILKIPFAIIMLTTIFFWTIMDINHLVFNKTEFLFNQNKWQKEDSFESDLTKTLKDKI
QKSELSYSDLMSLQLYDAMNQSTFLTDSELFEDILKKIEDSHNTLLCTGLVELLLYEMSI
SNNNNWQEKVDKIRIQLIRINQLDFFYYTSWLRQNFDFCMNREYHKMKSRKLLLSNKKIV
(SEQ ID NO: 103)
>orf00329 
MELVLPNNYVVIDEEEMMYLDGGAYLSKRACQGICVALAMSPGTFIALTGAAVLTKKLIN
YIKVGGLGGWLIGAAAGVLAGAAGRIAYCIGYGALNRGCDISGNPYPWDGFISATVR (SEQ ID
NO: 104)
>orf00230 
LGWIHICDSKMSNVDKIRKIHIIVCWVYIFLSFRAIINDTEYFLLIFLAFIYSIVSLPLY
SVKNKIVSICLVINSILLMSFPILINKFFPESFSTYIVLISVFITELIIFHLIGKDFDTK
LTNEYKKISQFRSKVSQSPWIKYLEISSFILTIFPSILYGTVDNHVLTLIFLIKICVDTT
IKFLFIRLFDTSTLMKRRIFFLFALDVIAYLFLGYLLVIQKAGYLFSVLLLFSNFSVPFI
KAKEYELFKNSK (SEQ ID NO: 105)
>0ref00232 
MNKKKMILTSLASVAILGAGFVASSPTFVRAEEAPQVVEKSSLEKKYEEAKAKYDAAKKD
YDEAKKKAAEAQKKYEEDQKKTEEKAKKEKEAAKEVDDASLAVQKAHVEYRKVLDSRNSY
RNPSDYAKKLAEADKKITEETTKLTNAQTKFQSIRTTIVVPGQSELAETKKKAEEAKAEE
KVAKRKYDYATLKVALAKKEVEAKELEIEKLQDEISTLEQEVATAQHQVDNLKKLLAGVD
PDDTEAIEAKLKKGEAELNAKQAELAKKQTELEKLLDSLDPEGKTQDELDKEAAEAELNK
KVESLQNKVADLEKEISNLEILLGGADSEDDTAALQNKLATKKAELAKKQTELEKLLDSL
DPEGKTQDELDKEAAEAELDKKVESLQNKVADLEKEISNLEILLGGADSEDDTAALQNKL
ATKKAELEKTQKELDAALNELGPDGDEEETPAPAPQPEQPAPAPKPEQPAPAPKPEQPAP
APKPEQPAKPEKPAEEPTQPEKPATPKTGWKQENGMWYFYNTDGSMATGWLQNNGSWYYL
NANGSMATGWVKDGDTWYYLEASGAMKASQWFKVSDKWYYVNSNGAMATGWLQYNGSWYY
LNSNGAMATGWAKVNGSWYYLNANGSMATGWVKDGDTWYYLEASGAMKASQWFKVSDKWY
YVNSNGAMATGWLQYNGSWYYLNSNGAMATGWAKVNGSWYYLNANGSMATGWVKDGDTWY
YLEASGAMKASQWFKVSDKWYYVNSNGAMATGWLQYNGSWYYLNSNGAMATGWAKVNGSW
YYLNANGSMATGWVKDGDTWYYLEASGAMKASQWFKVSDKWYYVNGLGALAVNTTVDGYR
VNANGEWV (SEQ ID NO: 106)
>orf00233
   MKIRRRYTHIIRIICILTISFKKQFLSSSLSSLTKRVIMNTAQATFNREAHTTFNRE (SEQ ID NO: 107)
>orf00252
   LKKRMNRWQFLLNQSKEMVGILLLKVKEQELIEFVVNL (SEQ ID NO: 108)
>orf00253
   LIKVIKRKAFGFRNFNNFKKRILMTLNIKKESTNFVLSRL (SEQ ID NO: 109)
>orf00257 
MTYNEKRLTNSLERGHMEQLKNTTDLLGLEDKNIKILSVLKYQTHLVVQAKLDSPAPPCP
HCQGKMIKYDFQKASKIPLLDCQGLPTVLHLKKRRFQCKNCLKVVVSQTSIVKKNCQISN
MVRQKIAQLLLEKQSMTEIAHRLAVSTSTVIRKLREFKFETDWTKLPKVMSWDEYSFKKS
KMSFIAQDFESKSILAILDGRTHAVIRNHFQRYQREVRELVEVITMDMYSPYYRLAKQLF
PKAKIVLDRFHIVQHLSRAMNRVRIQIMNQFDRKSLEYRALKRFWNPRFFVSRLGLNQST
GLIYYTRIASSSVRNDSISPRFECT (SEQ ID NO: 110)
>orf00258 
MGYSLKKSRTYCEQDPEKVNRFLKELNHLSYLTPIYIYETGVETYFYLEYDRALSRQLVS
LEEDIII (SEQ ID NO: 111)
>orf00265 
LREGCSIYDNLYPSRIVVGDETVEGRKIAELFLSISTHSTANIKNVMLVSPTEAEAIKLF
SNTFLALRVAFFNELDFFAERRSLNAEVVIKGVCLDPRIGNFYNNPSFEFGGYCLPKDTK
QLKKEFIEINAPVIEAIDISNTNRKQFIVKQILERKPKIVGIYKLGMKYNSDNYKESAIL
SIINELLIVGIKILVYEPNLNVSIDNVIFEKNFELFTKQSDLIVANRWDRGLEAYKDKVY
 TRGIWIRD (SEQ ID NO: 112)
>orf00268 
MLNLQFAETMELTEAELETVYGGEFGNNAVIPAGAWGGLGTSWSITNFWKKYFNHDSSTV
NRRHY (SEQ ID NO: 113)
>orf00272 
MKIKEQTRKLAAGCSKHRFEVADRTDEVSSKHCFEVVDRTDEVSNHTYGKVKLTWFEESF
EEYK (SEQ ID NO: 114)
>orf00338
   LNTSYSFGKKDQFALEHCFCIKLSIFARAVTLFVSCIN (SEQ ID NO: 115)
>orf00359 
MLIGEGYRTFPVLIYTQFISEVGGNSAFAIMAIIIALAIFLIQKHIANRYSFSMNLLHPI
EPKKTTKGKMAAIYATVYGIIFISVLPQIYLIYTSFLKTSGMVFVKGYSPNSYKVAFNRM
GSAIFNTIRIPLIALVLVVLFTTFISYLAVRKRNLFTNLIDSLSMVPYIVPGTVLGIAFI
SSFNTGLFGSGFLMITGTAFILIMSLSVRRLPYTIRSSVASLQQIAPSIEEAAESLGSSR
LNTFAKITTPMMLSGIISGAILSWVTMISKLSTSILLYNVKTRTMTVAIYTEVLRGNYGV
AAALSTILTVLTVGSLLLFMKISKSNSITL (SEQ ID NO: 116)
>orf00360 
LIIIASMSTPFVGAYSWILLLGRNEVITKFLTNALYLPAIDIYGFKGIILVFTLQLFPLV
FLYVAGTMNSIDNSLLEAAESMGSFGFKPIVTVVLPLLVPTLLAAPCLYL (SEQ ID NO: 117)
>orf00362 
LLSTTEFIGLSIRILSNLHESKILVGLLNQFFFWNLLLHKTKSNVVSDSQMWENSVVLEN
QPDIAFAGFHIIDFCIIEVKFSIFDTVETCNHTKKGRFPTS (SEQ ID NO: 118)
>orf00380
   VTAPTSITPLLVNTHERKSSQSLTSCLVYVVKTVLTSQHLIYSKLKLK (SEQ ID NO: 119)
>orf00382 
MITIKKQEIVKLEDVLHLYQAVGWTNYTHQPEMLEQALSHSLVIYLALDGDAVVGLIRLV
GDGFSSVLVQDLIVLPIYQRQGIGSALMKEALEDYKDAYQVQLVTEQTERTLGFYRSMGF
EILSTYNCIGMTWMNRKK (SEQ ID NO: 120)
>orf00441
   MGAFVLFLFQLTINRYKKKSFYWYKEVIESNGETLDN (SEQ ID NO: 121)
>orf00465 
VAIDKIAGITSEKDSRAHQIFRISPTCSRCFCNDELVKWVARTIFLQFTKRCCLRSGNIT
RSNSVTLDIGSTVFRRNVAGQHFQAPFSSSISANCFTSQFAHHRTNIDNLSMPFLYHRRN
NCL (SEQ ID NO: 122)
>orf00466 
LFDLLDHGLDTVLVCHVTDISMGFDANFTISFNPFIDQILIDIVKDNSSAGFSVGFGNSK
SNSIRSAGDESNFSF (SEQ ID NO: 123)
>orf00478 
MKSLARLLNIHVFISIFLFFALISGAVSHTVLLLLLLFLPALNKGLEKIQSKRIPVLNAA
LFFLLISFPQLLTNPVQWKFSIFLVVTIISSLAYFYNFYQVVKEVDQKQLI (SEQ ID NO: 124)
>or00480 
LEAAGEIETEFQGWIVLVVFNHIDSLSRDTDILGEFELGNTQFLAKFFHTIHLVSFLIYV
VYI (SEQ ID NO: 125)
>orf00485
   MVDRTDEVSSKHGFEVVDKEKLMMFEEVFEECKKILVS (SEQ ID NO: 126)
>orf00492 
MEGVNHVDIIKVSCGSFISQVNWMMKGKIPNREGFKFSVARLDAIDLVVVHIGHTRCQFS
RTGSRSGYDNQVATDFDVVVFAHAFWGNDVIHIRRISFDWIMKIRINSVFLKLVAEGICS
 GLTSVLCNDNGTNKNP (SEQ ID NO: 127)
>orf00493
   MFNVASINGNHNLNLLFQFLQELDFWRFITRKDTSSVEIF (SEQ ID NO: 128)
>orf00495
   LFFHFLPLDSIIIKNWKLGNYGAKKEIKKIKQTLAQNFKKCYHIL (SEQ ID NO: 129)
>orf00498
   MQLTSVTAPTGTDNENIQKLLADIKSEYRFDGRPEFVLLGCLQESDCR (SEQ ID NO: 130)
>orf00501 
MIDIHSHIVFDVDDGPKSIEDSKALLREAYNQGVRMIVSTSHRRKGMFETPEEKIVTNFI
KVREIAKEVADDLVIAYGAEIYYTLDALEKLEKKKFLPLMIVVML (SEQ ID NO: 131)
>or00502 
MHTSYREIHTRLSNILMLGITPVIAHIERYDALENNEKRVRELIDMGCYTQIDSYHVSKP
KFFGEKYKFMKKRARYFLERDLVHVVASDMHNLDSRPPYMQQAYDIIAKKYRAKKAKELF
VDNPRKIIMDQLI (SEQ ID NO: 132)
>orf00503 
MKEQNTLEIDVLQLFRALWKRKLVILLVAIITSSVAFAYSTFVIKPEFTSTTRIYVVNRN
QGEKSGLTNQDLQAGSYLVKDYREIILSQDVLEEVISDLKLDLTPKGLANKIKVTVPVDT
RIVSVSVNDRVPEEASRIANSLREVAAQKIISITRVSDVATLEEARPAISPSSPNIKRNT
LIGFLAGGIGTSVIVLLLELLDTHVKRPEDIEDTLQMTLLGVVPNLGKLK (SEQ ID NO: 133
>orf00505 
MAKGHHIKLKVKDKIANVLTCNTISINSITSHSNSTQFMPFGMVLTQPLNIRRHPVFSNF
NLSSFYILTERTIQ (SEQ ID NO: 134)
>orf00506 
MKIAIAGSGYVGLSLAVLLAQHHEVKVIDVIKDKVESINNRKSPIKDEAIEKYLVEKELN
LEASLDPAHVYKDVEYAIIATPTNYDVDLNQFDTSSVEAAIKTCMEYNDTCTIVIKSTIP
EGYTKEVREKFNTDRIIFSPEFLRESKALYDNLYPSRIVVGTDLDDSELTKRAWQFADLL
KGGAIKEEVPILVVAFNEAEVAKLFSNTYLATRVAYFNEIDTYSEVKGLNPKTIIDIVCY
DPRIGSYYNNPSFGYGGYCLPKDTKQLKASFRDVPENLITAVVQSNKTRKDYIAGAILAK
QPSVVGIYRLIMKSDSDNFRSSAVKGVMERLDNYGKEIVIYEPTIECDTFMGYRVIKSLD
EFKNISDIVVANRMHDDLRDIQEKLYTRDLFGRE (SEQ ID NO: 135)
>orf00507 
MYTFILMLLDFFQNHDFHFFMLFFVFILIRWAVIYFHAVRYKSYSCSVSDEKLFSSVIIP
VVDEPLNLFESVLNRISRHKPSEIIVVINGPKNERLVKLCHDFNEKLENNMTPIQCYYTP
VPGKRNAIRVGLEHVDSQSDITVLVDSDTVWTPRTLSELLKPFVCDKKIGGVTTRQKILD
PERNLVTMFANLLEEIRAEGTMKAMSVTGKVGCLPGRTIAFRTEILRECIHEFMNETFMG
FHKEVSDDRSLTNLTLKKGYKTVMQDTSVVYTDAPTSWKKFIRQQLRWAEGSQYNNLKMT
PWMIRNAPLMFFIYFTDMILPMLLISFGVNIFLLKILNITTIVYTASWWEIILYVLLGMI
FSFGGRNFKAMSRMKWYYVFLIPVFIIVLSIIMCPIRLLGLMRCSDDLGWGTRNLTE (SEQ ID
NO: 136)
>orf00508
   LIFEKEKCFLMLRKNLKYQIMTRAGTILAILFFIILGIIVEVLF (SEQ ID NO: 137)
>orf00509 
MKKVKKAVIPAAGLGTRFLPATKALAKEMLPIVDRPTIHFVIEEALRSGIEDILVVTGKS
KRSIEDYFDSTFELEYSLRKQGKMELLKSVNESTDIKVHFVRQSSPRGLGDAVLQAKSFV
GDDPFVVMLGDDLMDITDSTAVPLTRQLMDDYNATQASTIAVMPVRYEDVSSYGVISPRL
ESSNGLYSVDAFVEKPKPEEAPSNLAIIGRYLLTPEIFSILETQKPGAGNEIQLTDAIDT
LNKTQSVFAREFVGKRYDVGDKFNFMKTSIDYALQHPQIKESLKNYVIALGKQLEKLDDC
SSSGHL (SEQ ID NO: 138)
>orf00510 
MNCIESYQKWLNVPDLPAYLKDELLSMDDKTKEDAFYTNLEFGTAGMRGYIGAGTNRINI
YVVRQATEGLAKLVESKGETAKKAGVAIAYDSRHFSPEFAFESAQVLAAHGIKSYVFESL
 
RPTPELSFAVRHLGAFAGIMVTASHNPAPFNGYKVYGSDGGQMLPADADALTDYIRAIDN
PFAVALADLEEAKSTGLIEVIGETLDSAYLEEVKSVNINQDLIDQYGRDMKIVYTPLHGT
GEMLARRALAQAGFESVQVVEAQAKPAPDFSTVASPNPESQAAFALAEELGRQVDADVLV
ATDPDADRLGVEIRQADGSYWNLSGNQIGALIAKYILEAHKQAGTLPKNAALAKSIVSTE
LVTKIAESYGATMFNVLTGFKFIAEKIQEFEEKHNHTYMFGFEEG (SEQ ID NO: 139)
>orf00520 
MNKGLFEKRCKYSIRKFSLGVASVMIGAAFFGTSPVLADSVQSGSTANLPADLATALATA
KENDGRDFEAPKVGEDQGSPEVTDGPKTEEELLALEKEKPAEEKPKEDKPAAAKPETPKT
VTPEWQTVEKKEQQGTVTIREEKGVRYNQLSSTAQNDNAGKPALFEKKGLTVDANGNATV
DLTFKEDSEKGKSRFGVFLKFKDTNNNVFVGYDKDGWFWEYKSPTTSTWYRGSRVAAPET
GSTNRLSITLKSDGQLNASNNDVNLFDTVTLPAAVNDHLKNEKKILLKAGSYDDERTVVS
VKTDNQERVKTEDTPAQKETGPVVDDSKVTYDTIQSKVLKAVIDQAFPRVKEYTLNGHTL
PGQVQQFNQVFINNHRITPEVTYKKINETTAEYLMKLRDDAHLINAEMTVRLQVVDNQLH
FDVTKIVNHNQVTPGQKIDDERKLLSSISFLGNALVSVSSDQTGAKFDGATMSNNTHVSG
DDHIDVTNPMKDLAKGYMYGFVSTDKLAAGVWSNSQNSYGGGSNDWTRLTAYKETVGNAN
YVGIHSSEWQWEKAYKGIVFPEYTKELPSAKVVITEDANADKKVDWQDGAIAYRSIMNNP
QGWEKVKDITAYRIAMNFGSQAQNPFLMTLDGIKKINLHTDGLGQGVLLKGYGSEGHDSG
HLNYADIGKRIGGVEDFKTLIEKAKKYGAHLGIHVNASETYPESKYFNEKILRKNPDGSY
SYGWNWLDQGINIDAAYDLAHGRLARWEDLKKKLGDGLDFIYVDVWGNGQSGDNGAWATH
VLAKEINKQGWRFAIEWGHGGEYDSTFHHWAADLTYGGYTNKGINSAITRFIRNHQKDAW
VGDYRSYGGAANYPLLGGYSMKDFEGWQGRSDYNGYVTNLFAHDVMTKYFQHFTVSKWEN
GTPVTMTDNGSTYKWTPEMRVELVDADNNKVVVTRKSNDVNSPQYRERTVTLNGRVIQDG
SAYLTPWNWDANGKKLSTEKEKMYYFNTQAGATTWTLPSDWAKSKVYLYKLTDQGKTEEQ
ELTVKDGKITLDLLANQPYVLYRSKQTNPEMSWSEGMHIYDQGFNSGTLKHWTISGDASK
AEIVKSQGANDMLRIQGNKEKVSLTQKLTGLKPNTKYAVYVGVDNRSNAKASITVNTGEK
EVTTYTNKSLALNYVKAYAHNTRRDNATVNDTSYFQNMYAFFTTGSDVSNVTLTLSREAG
DQATYFDEIRTFENNSSMYGDKHDTGKGTFKQDFENVAQGIFPFVVGGVEGVEDNRTHLS
EKHYPYTQRGWNGKKVDDVIEGNWSLKTNGLVSRRNLVYQTIPQNFRFEAGKTYRVTFEY
EVGSDNTYAFVVGKGEFQSGRRGTQASNLEMHELPNTWTDSKKAKKATFLVTGAETGDTW
VGIYSTGNASNTRGDSGGNANFRGYNDFMMDNLQIEEITLTGKILTENALKNYLPTVAMT
NYTKESMDALKEAVFNLSQADDDISVEEARAEIAKIEALKNALVQKKTSLVADDFASLTA
PAQAQEGLANAFDGNLSSLWHTSWGGGDVGKPATMVLKEPTEITGLRYVPRGSGSNGNLR
DVKLVVTDESGKEHTFTTTDWPDNNKSKDIDFGKTIKAKKIVLTGTKTYGDGGDKYQSAA
ELIFTRPQVAETPLDLSGYEAALAKAQKLTDKDNQEEVASVQASMKYATDNHLLTERMVE
YFADYLNQLKDSATKPDAPTVEKPEFKLSSLASDQGKTPDYKQEIDRPETPEQILPATGE
SQSDTALFLAGVSLALSALFVVKTKKD (SEQ ID NO: 140)
>orf00523 
LQIAQESSQDTDGINPPVVEEAMVFDRNDCLNQICGNIISLGIDAAFRTQVSNELIFIVV
DFTRSCCN (SEQ ID NO: 141)
>orf00525
   MLNLMWMKIFHRNRTFLFCFLDFKVDVISIINARIVRR (SEQ ID NO: 142)
>orf00526
   MYNSQALRQIVVVGSIDHLFKRHSSICEIFGLRKRCLSFLW (SEQ ID NO: 143)
>orf00537 
MKLLKKTMQAGLTVIFFGLLATNTVFADNSEGWQFVQENGRTYYKKGDLKETYWRVIDGK
YYYFDSLSGEMVVGWQYIPFPSKGSTIGPYPNGMRLEGFPNSEWYYFDKNGVLQEFVGWK
TLEIKTKDSVGRKYGEKREDSEDKEEKRYYTNYYFNQNHSLETGWLYDQSNWYYLAKTEI
NGENYLGGERRAGWINDDLTWYYLDPTTGIMQTGWQYLGNKWYYLRSSGAMATGWYQEGT
TWYYLDQPNGDMKTGWQNLGNKWYYLRSSGAMATGWYQEGTTWYYLDQPNGDMKTGWQNL
GNKWYYLRSSGAMATGWYQDGSTWYYLNAGNGDMKTGWFQVNGNWYYAYSSGALAVNTTV
DGYSVNYNGEWVR (SEQ ID NO: 144)
>orf00541
   MTTGWFQVNGRWYYAYSSGALAVNTTVDGYFVNYNGEWVQ (SEQ ID NO: 145)
>orf00551 
MSLADLLEELEAAKDSKKARSMEAYMRHQFSFLGIAVPERNKLYKNIFQKRKKQRLSIGI
LQTLAGKRILENTNMWLLTI (SEQ ID NO: 146)
>orf00552
   MEKILLHNLNQTEFFINKAIGWTLRDYSKTNPTWVTCFIEKNKERMAELSIKEASKYL (SEQ ID NO: 147)
>orf00555 
VLEILKEYLLLEEGDYIFTNNGSPLMITCFNYFLKNSFRKSEIKKDDFVLTAHVFRYSHI
SLLAELEVPITAIMDRVDYTNETKILSVYTHVTEKMKSNITEKLDKLVLEND (SEQ ID NO:
148)
>orf00580
   LVIFKNCSHCTSNCKSRTVQGMKEFHLAICFITVTDLSTTGLEIFXXXFHSLIN (SEQ ID NO: 149)
>orf00581 
MNXXARGFSFVCKDFEVAAHFASSDIGSNLIDMAPRNLDKLFDIDIWVKFQSLISDKEFP
NFPSDREVGSACRTEEYCFVK (SEQ ID NO: 150)
>orf00587
   LQKPLFQILQKLVKISQAFIHDSNFFLAHAINNFXXHSFIN (SEQ ID NO: 151)
>orf00590
   VGFSLLLLLIFCLFCLLFNVSNQVTNCFQVTNCFQVIFCFDLDIKSIFDF (SEQ ID NO: 152)
>orf00614
   VNIDSSEFYISHITDGIFDSFLDSNRYLRNFYSVLKVEIDICCEFFVHVFKINATAE (SEQ ID NO: 153)
>orf00615
   VNTLYLCSSNSNDFFKYTWGDNDFAKLFFNSHRMTSF (SEQ ID NO: 154)
>orf00625
   LFSPRPGGDNGKGTTFLNFFLNIHKFSFFDSLFFLFQ (SEQ ID NO: 155)
>orf00627 
VKEEKKAIVLGADNAYMDKVETTLKSLCVHHYNLKFYVFNDDLPREWFQLMEKRLETLNS
EIVNV (SEQ ID NO: 156)
 >orf00643 
LAQISILHFDFLSIDKHSHTVFNTLRKSLQTTLALSATSKQCFEQLAASFLVCSLIFIEY
KV (SEQ ID NO: 157)
>orf00652 
MSLITHRRFISSKVTRQKFVDNQIDLKYYIWRYSHALCGIDVAKNKHDVTALNVSGKTVL
KPLTFSNNKAGFELLDLSLRQLNQDYLIALEDTGHYAFNLLNFLHEQGYKVYTYNPLLIK
KFAKSLLLRKTKTDKKDAHGIALKLLSDPNREQFQHDNRQVELKILARHIHRLKKKQSDW
KVQYTRCLDIIFPELDKIVGKHSEYTYQLLTCYPNPQKRLEAGFDKLIEIKRLTASKIQD
ILSVAPRSIGTTSPAREFEIIENIKHYKRLIDKAETCVNDLMAEFNSVITTVTGIGNRLG
AVILAEIQNIHAFDNPAQLQAFAGLDSSIYQSGQIDLAGRMVKRGSPHLR (SEQ ID NO: 158)
>orf00654
   MDTKSSCLITTGRNDSPSTCLPRVASNNDRFSSEFRIIPDFHCSKKGIHVNMDDFS (SEQ ID NO: 159)
>orf00657 
MDKMKFVFQALNKELIQENLTLTIICVGGYVLEYHGLRATQDVDAFYDQNQKINEIIARV
GKQFNLNTHEELWLNNHVANMNKQPPLSLCESLYSFENLTVLVVPIEYVLGMKMMSIREQ
DLKDIGAIIKYKNFHSPFDTFKYLKDMGFDTIDLSVLLEGFSYAYGMDWLEKFFKENQDK
LREFY (SEQ ID NO: 160)
>orf00663 
MIPLYRTDNDITKFFTKIRNGHLAKTAGGLDDKFHEANASTSKAFDRQGVGEVNDIRDSA
GSQELRINDKRKTENILFLEIRVRIFRVPHPNDSFFSSHFLG (SEQ ID NO: 161)
>orf00664 
VLSQGDKDITILDAGLLKNGKIGPVTKDTNDIKATDNMIENSFVLLNQQNIMLFCNQGAT
EGKTNFSPSDKDNFHNKTYFFMM (SEQ ID NO: 162)
>orf00669 
MKIKEQTRKLAAGCSKQCFEIVDRTDEVSSKHGFEVVDETDEVSSKHGFEIVDETDEVSN
HTYGKAKLTWFEEIFEEYKMMGKAGQLVFFDVYRLVRQVS (SEQ ID NO: 163)
>orf00698
   MEKFNFKNNIGQENKLLQIEIYKFTNFCKLQNYTSVNIFSKDIFEAIVN (SEQ ID NO: 164)
>orf00701 
VRIKSIYWNFGQNKPEKSFRYIDTSSIDRKKNIINYKNLQYLSPEQAPSRARKLVSQNSV
LFSTVRPYLKNIAVVRELKEYLIASTAFIVLDTLLNETYLKYYLLSDNFINRVNNKSTGT
SYPAINDYNFNLLLIPLPPLSEQQRIVEAIESALEKVDEYAESYNRLEQLDKEFPDKLKK
SILQYAMQGKLVEQDPNDESVEVLLEKIRAEKQKLFEEGKIKKKDLDISIVSQGDDNSYY
GNIPMNWVVIKIKDIFSINTGLSYKKGDLSINKGVRIIRGGNIKPLEFSLLDNDYYIDTQ
FISSEQVYLKHNQLITPVSTSLEHIGKFARIDKDYDGVVAGGFIFQLTPFESSEIISKFL
LFNLSSPLFYKQLKAITKLSGQALYNIPKTTLSELLIPLAPFEEQELITQKVEKLFEKVN
QLWK (SEQ ID NO: 165)
>orf00720
   MIRKVNHNIFKHRSVVIFTLTNSYFCKFFINDISISFHSHQHFCWIIIQIK (SEQ ID NO: 166)
>orf00724
   LDNIHIVLDSLNAVSGIQDFICDGLAIFCNQITSGCSSCK (SEQ ID NO: 167)
>orf00735 
MNIAWILLYALVINGLEIVIFFKVDGIGLTFDRIFKAFLLKFLLGIIFTTFQFLAVSKYL
SYFIEPLFGIGLSFLLLRGLPKKILIFYGLFPMILVELFYRGVSYFVLPFLGQGIVDGDG
NPIFLLIMIFVCFIVLVFLKWLDYDFTRLRREFLDTGFQKSLTKINWAMGAYYLVMQSLS
YLEYEQGIQSTTVRHLILVFYLLFFMGGIKKLDTYLKEKLQEELNQEQTLRYRDMERYSR
HIEELYKEIRSFRHDYTNLLTSLRLGIEEEDMEQIKEIYDSVLRDSSQKLQDNKYDLGRL
VNIRDRALKSLLAGKFIKAREKNIVFNVEVPEEIQVEGMSLLDFLTIVSILCDNAIEASA
EASQPHVSIAFLKNGAQETFIIENSIKEEGIDISEIFSFGASSKGEERGVGLYTVMKIVE
SHPNTNLNTTCQNQVFRQVLTVIHAE (SEQ ID NO: 168)
>orf00737 
MISQEDILKACEVAEIRQDIERMPMGYQTQLSDGAGLSGGQKQRIALARALLTKSPVLIL
DEATSGLDVLTEKKVIDNLMSLTDKTILFVAHRLSIAERTNRVIVLDQGKIIEVGIHQEL
MQAQGFYHHLFNK (SEQ ID NO: 169)
>orf00738 
MSSKISIGQLITFNTLLSYFTTPMENIINLQTKLQSAKVANNRLNEVYLVESEFQVQENP
VHSHFLMGDIEFDDLSYKYGFGRDTLTDINLTIKQGDKVSLVGVSGSGKTTLAKMIVNFF
EPYKGHISINHQDIKNIDKKSLAPSY (SEQ ID NO: 170)
>orf00740 
MKSTLGIISVGLVITYILQQVMSFSRDYLLTVLSQRLSIDVILSYIRHIFELPMSFFATR
RTGEIISRFTDANSIIDALASTILSLFLDVSILILVEGVLLAQNPNLFLLSLISIPIYMF
IIFSFMKPFEKMNHDVMQSNSMVSSAIIEDINGIETIKSLTSEENRYQNIDSEFVDYLEK
SFKLSKYSILQTSLKQGNKISSEYPYPMVWRSISHVE (SEQ ID NO: 171)
>orf00742 
MTSYKRTFVPQIDARDCGVAALASIAKFYGSDFSLAHLRELAKTNKEGTTALGIVKAADE
MGFETRPVQADKTLFDMSDVPYPFIVHVNKEGKLQHYYVVYQTKKDYLIIGDPDPSVKIT
KMSKERFFYEWTGVAIFLATKPSYQPHKDKKNGLLSKLPSSDFQTKISHCLHCSLKLIGH
YYQYRWFLLSPRNLG (SEQ ID NO: 172)
>orf00767
   MGLIKTLAKIYGNYFLTVQGVKVMKTIKKDDHVVVGLGKLFIADKLMDTARWLIKPEDKK (SEQ ID NO: 173)
>orf00768
   MKFFWGLLAIIFIKPIIGIVKFFWMIISFAVQLLFYKIVFKILDWLFKLI (SEQ ID NO: 174)
>orf00776
   MHSQTFQFLLMTDKTSLLHRKHRSFIRNIHSKFLILFDLLCGILSRNDSNHNPIS (SEQ ID NO: 175)
>orf00779 
MARTELPDKIETERLVLRVRTVADAEDIFDYASLPEIAYPAGFPPVKTLEDEIYYLEHIL
PERNQKENLPAGYGIVVKGTDKIVGSVDFNHRHEDDVLEIGYTLHPDYWGRGYVPEAARA
LIDLAFKDLGLHKIELTCFGYNLQSKRVAEKLGFTLETRIRDRKDVQGNRCDSLIYGLLK
SEWEE (SEQ ID NO: 176)
>orf00781 
MSDVKEEVSSLSEKQLRQIDVEYAELNDSDIIERLAYLEINNNEKRIVISDIEPTKEIMS
VSDQIFEIQKNFQKIKNMFELFISDVSDFLSIKNKLESKELEIEEADVNRFMIHLLSSGK
LFVDFNENQIKQKYSKDSEEFDCIHGFASYQYDINFTYRFCHSLRNYSQHTDLPINEVKA
VSPDDETVIIDFYIDLDYLLNSNFKWKKLKGELIKLNQETSKIDAIALVKEYFNALTELY
GNYNKLFLKLNHNTLVDIKSKLESLKLKHSRYYISKISKYDLKYNPGNYTMSPLAAFAEI
EEIYIELSKIGLVKIVNKSN (SEQ ID NO: 177)
>orf00785 
MSKHPHYELLNLIGYGLAKFDKLFIKEFQCSSKSEFYRYVVSLGIAETTGVVKNRMDLFD
PYFDNNRKGWWQKAEVYRFRKDLIDMMFGNEDVHSYAEIVKMLLASEGKKTGITIVEKPI
VRTKFKRLQETGMEAENYFILHFDKEEKFQGGQLTDARLYGDGYDFQVDVQEYSYLAEVK
GIRKSKGRVRLTAKEFEKVKEFQSDFILSLVTNLDDIPKLVLIDNPLKHFEFKKNIIKNE
IIEYRSVEDLY (SEQ ID NO: 178)
>orf00787
   LSTCWNGKFCHICVALFHCFRAFKLALNEILCLLTNVSFIFVSVAF (SEQ ID NO: 179)
>orf00788
   LLRKQEREYLRAENAILKKLRELRLKEEKEKEERQKLFKN (SEQ ID NO: 180)
>orf00809
   LKHLFCHFNPLWIDEIIRLAYKDQDTKDVKSKVKIGN (SEQ ID NO: 181)
>orf00856
   MKEIAFDAFYQLYQNDQLSLVDVREVDEFAALHLEGAHNLPLSQLADSYD (SEQ ID NO: 182)
>orf00859
   MVSNHKIACFQLFDKVGIFSLLDELNSLANKAHINLLNCF (SEQ ID NO: 183)
>orf00871
   MDFFFMNEVKEQVLFRDNHSEHIFWIEGVSDFMIKVNTALW (SEQ ID NO: 184)
>orf00878
   MKIKEQTRKLAAGCSKHCFEVVDRTDEVSNHTHGKATLTWFEEIF (SEQ ID NO: 185)
>orf00885 
MKIKEQTRKLAAGCSKHSFEVVDKTDEVSSKHGFEVVDETDEVSSKHGFEVVDETDEVSN
HIYGKATLTKFELDFRRV (SEQ ID NO: 186)
>orf00890 
MEELVTLDCLFIDGTKIEANANKYSFVWKKTTEKFSAKLQEQIQVYFQEEITPLLIKYAM
FDKKQKRGYKQSAKNLANWHYNDKEDSYIHPDGWCYRFHHIKYQKTQTDFQQEIKVYYAD
EPESAPQKGLYMNERYQNLKAKECQALLSPQDRQIFAQRKIDVEPVFGQIKACLGYKRCN
LRGKRQVRIDMGLVLMANNLLKHSEMK (SEQ ID NO: 187)
>orf00892 
MHIHYNTNQTTLPLEISSFLPQDHLVFTIEKVVNTLEDCHFHAFYHAFDRPSYHLKMLVS
TLLFAYSQGIFSGRKIEKWKS (SEQ ID NO: 188)
>orf00894 
LRLWVIFVIMKVMKSYNTLNDYYRKLFGEKTFKVPIDAGFDCPNRDGTVAHGGSTFCTVS
GSGDAIVAPDAPIREQFYKEIDFMHRKWPDVQKYLVYFQNFTNTHEKVEVIRERYEQAIN
EFGVVGINIGTRPDCLPDETIEYLAELSECMHVTVELGLQTTYEATSDLINRVHSYEL
(SEQ ID NO: 767)
>orf00896 
VETVKRLRKYPKIEIVSHLINGLPGETHEMMVENVRRCVTDNDIQGIKLHLLHLMTNTRM
QRDYHEGRLQLMSQDEYVRVICDQLEIIPKHIVIHRITGDAPRDMLIGPMWSLKKWEVLN
SIEMEMRRRGSVQGCKAVKQEFENEKTT (SEQ ID NO: 189)
>orf00908
   VQVCVFTNFCFFHCFSSLANCRLFNLRGICLPCISYQ (SEQ ID NO: 190)
>orf00915 
VFKKDRFSIRKIKGVVGSVFLGSLLMAPSVVDAATYHYVNKEIISQEAKDLIQTGKPDRN
EVVYGLVYQKDQLPQTGTEASVLTAFGLLTVGSLLLIYKRKKIASVFLVGAMGLVVLPSA
GAVDPVATLALASREGVVEMDGYRYVGYLSGDILKTLGLDTVLEETSAKPGEVTVVEVET
PQSTTNQEQARTENQVVETEEAPKEEAPKTEESPKEEPKSEVKPTDDTLPKVEEGKEDSA
EPSPVEEVGGEVESKPEEKVAVKPESQPSDKPAEESKVEPPVEQAKVPEQPVQPTQAEQP
STPKESSQQENPKEDRGAEETPKQEDEQPAEAPEIKVEEPVESKEETVNQPVEQPKVETP
AVEKQTEPTEEPKVEVTSIPQTTRYEEDLTKEHGTREVVKEGKNGSRTVTTPYILNATDG
TTTEGTSTTDEAEMEKEVVRVGTKPKEKLAPVLSLTSVTDNAMLRSARLTYHLENTDSVD
VKKIHAEIKNGDKVVKTIDLSKERLSDAVDGLELYKDYKIVTSMTYDRGNGEETSTLEET
PLRLDLKKVELKNIGSTNLVKVNEDGTEVASDFLTSKPVDVQNYYLKVTSRDNKVFRLTV
EKIEEVTEEGQPLYKVTAKAPNLIQHTDATKMQDEYVYYIEKTRATDGDIYYNFNDLVNA
MNKNKTGTFKLGADLNATGVPTPAKSYVTGDFRGTLTSVDGEHYTIHNTSRPLFNNIIGG
TIKDINLGNVNIHMPWANNVASLANIIKGGTTIENVKVTGNVLGKDWVSGFIDKIDSGGT
LRNVAFIGNVTSVGTGGSFLTGIVGENWKGLVEQAYVDANIRGKKAKAAGIAYWSQNGGD
NYAVGRYGAIKKSVVKGSIDVEKPIEVGGAVGSLNYLGYIEDTVAMMKVKNGEIFYGSHD
IDTDPYYTGERVNRNFIVDGVSEGKSSYKYSKQQNRIKSVSQEEADKKIKELAITADKYA
ITEPIVNKLNALTTRDNEYRTTQDYKADRELAYRNIEKLQPFYNKEWIVDQGNKVPSNSK
LLTTEVLSVTGMKDGQFVTDLSEIDKIMIHYADGTKEEMNVTAVADSKVKQVREYDVTDL
GVVYTPNMVDKNRDQLIADVKAKLSSVELISPEVRALMDKRGKAEENTEGRQNGYIRDLF
LEESFAEVKAGLGKLVKALVENEDYQLNSDEAAMRALIKKVEDNKAKIMMGLAYLNQYYS
FKYAELSIKDIMMFKPDFYGKNVNVLDFLIKIGSSERNVKGDRTLEAYRETIGGTIGINE
LNGFLHYNMKLFTNHIDINDWFKKAIEKNAYVVEQPSTNPAFANKKYRLYEGINNGQHGR
MILPLLNLKNAHLFMISTYNTISFSSFEKYGKDTDEKREKFKSEINKRAKEQVNYLDFWS
RLATDNVRDKLLKSQNVVPTPVWDNHNSPNGWASRHGHIDGKPDYAPIREFFGRINKYHG
YKYGYGAYAYIFAAPQPMDAVYFVMTDLISDFGTSAFTHETTHINDRMAYYGGHWHREGT
DLEAFAQGMLQTPSVSNPNGEYGALGLNMAYERQNDGNQWYNPNPNKLKSRAEIDHYMKN
YNEALMMLDYLEAESVLPKLKGNNDRWFKKMDKQMRKDGQPHQFDKIRDLNNEEKKIQLA
SIEDLVDNNFMTKHGAPGNGTYNPSDFSSAYVNMNMMTGVYGGNSSDGAPGAASFKHNTF
RMWGYFGYENGFIGYASNKYKAEANKAGQTLSDKYIINKVSGGTFNTLEAWKKEWFKQIK
TKAQKGFTAIEIDGKTIDSYEKLKDLFDKAVEEDLKGTGTDKTVKLKEKVYKQLLKNTDG
FSGDLFTAPQA (SEQ ID NO: 191)
>orf00933 
VGDRIFIAFLQKLGLLDNLTGIREKLHPITGQGDSLGIADKDLNAHFIFQISHCIGETWL
SDKELLGCLIHGASFDDFDNIM (SEQ ID NO: 192)
>orf00941
   VSRNDGHSDKGEAPAGKTSYANIWTKWGEQVAFYCDYD (SEQ ID NO: 193)
>orf00955 
MKLFKPLLTVLALAFALIFITACSSGGNAGPSSGKTTAKARTIDENKKSGELRIAVFGDK
KPFGYVDNDGSYQGYDIELGNQLAQDLGVKVKYISVDAANRAEYLISNKVDITLANFTVT
DERKKQVDFALPYMKVSLGVVSPKTGLITDVKQLEGKTLIVTKGTTAETYFEKNHPEIKL
QKYDQYSDSYQALLDGRGDAFSTDNTEVLAWALENKGFEVGITSLGDPDTIAAAVQKGNQ
ELLDFINKDIEKLGKENFFHKAYEKTLHPTYGDAAKADDLVVEGGKVD (SEQ ID NO: 194)
>orf00988 
MDTPDENGYVADDYRITYLEAHIKAMRDAIYQDGVDLLGYTTWGCIDPVSAGTGEMNKRY
GFIYVDRDNVGNGALKRSKKKSFYWYKDVIDSNGASIG (SEQ ID NO: 195)
>orf01015 
MTEPDFWNDNIAAQKTSQELNELKNTYNTFHKMEELQDEVEILLDFLAEDESVHDELVAQ
LAELDKIMTSYEMTLLLSEPYDHNNAILEIHPGSGGTEAQDWGDMLLRMYTRYGNAKGFK
VEVLDYQAGDEAGIKSVTLSFEGPNAYGLLKSEMGVHRLVRISPFDSAKRRHTSFTSVEV
MPELDDTIEVEIREDDIKMDTFRSGGAGGQNVNKVSTGVRLTHIPTGIVVQSTVDRTQYG
NRDRAMKMLQAKLYQMEQEKKAAEVDSLKGEKKEITWGSQIRSYVFTPYTMVKDHRTSFE
VAQVDKVMDGDLDGFIDAYLKWRIS (SEQ ID NO: 196)
>orf01045 
MQVIKRNGEIAEFNPDKIYQAILKAAQTVYVLTDDLRQNLAQVTKKVVLDLQEAKVERAT
ISMIQSMVEHRLLGAGYITIAEHYISYRLQRDLERSGYGDHIAVHLHFEQIR (SEQ ID NO:
197)
>orf01068 
MELFKTWKKNMVLYGLKSQIGTVYRNNDRTTSFYDVGNFLYLAGELDSRFWEDFVRKYGL
DYKIIISENTNWQDFLHRKVGLNSFTRYSFKDKANFQVEFLNNLVTHLEEGYNIVPIDNH
IYNCFSTEEWSQDLQGDFESYQDFVLKGGFGFVILKNNELIAGISSGLVYRKAVEVEVAT
RPNEQGNGFAKKLGAAMILESLNRDMFPLWDAHNEASKKVAEFLGYELSEPYEAFELEEI
LI (SEQ ID NO: 198)
>orf01090
   LGSDRKALHKPVYLFWCESFDILFCTWSSQFSVLKAFI (SEQ ID NO: 199)
>orf01110 
MKVINQTLLEKVIIERSRSSHKGDYGRLLLLGGTYPYGGAIIMAALAAVKSGAGLVTVGT
DRENIPALHSHLPEPMAFSLQDQQLLKEQLEKAEVILLGPGLRDDASGENLVKQVFVNLS
QNQILIVDGGALTILARTSLSFPSSQLILTPHQKEWEKLSGITIEKQKEDATASVLTSFP
QGTILVEKGPATRIWEVGQSDYYQLQVGGPYQATGGMGDTLAGMIAGFVGQFRQASLYER
VAVATHLHSAIAQELSQENYVVLPTEISRYLPKIMKIICQQDRVSKDKLV (SEQ ID NO: 200)
>orf01114
   VLASNRKFIFFFFRIGILILKNIKSFNQFLALFHKIPSHDGSRKSLSWSDGKSLKXXFIH (SEQ ID NO: 201)
>orf01121 
VLDSKEELKESENDAPKLETPLREEPRLAPQTLLEASEVLENKREESKVGITEPAQDSPI
LAPVEETKEEAVTEKPTNTRSLTAEDLVKISKGELHLENDLIDESFYGEKALDLEGDDYQ
DGIKNKDGKDYLGYNSHPLLADSDGDGLADGEDDNKKEWYVTDRDSLLFMELAYRDDDYI
EKILDHKNLFPSLYLDRQEHKLMHNELAPFWKMKKAYYTDSGLDAFLFETKSDLPYLKDG
TVHMLAIRGTRVNDAKDLSADFVLLGGNKLAQADDIRKVVGELAKDISITKLYMTGHSLG
GYLAQIAAVEDYQKYPDFYNHVLRKVTTFSAPKVITSRTVWDAKNGF (SEQ ID NO: 202)
>orf01148
   MGRNPKTRPEERTELERLQSENEYLRAENAILKKLRELRLKEEKEKEERQKLFKN (SEQ ID NO: 203)
>orf01150
   LSTCWNGKFCHICVALFHCFRAFKLALNEILCLLTNVSFIFVSVAF (SEQ ID NO: 204)
>orf01165 
VVLSTSAILVACGKTDKEADAPTTFSYVYAVDPASLGYSIATRTSRTDVIGNVIDGLMEN
DKYGNVAPSQKDYDLNSTGWAPSYQDPASYLNIMDPKSGSAMKHLGITKGKDKDVVAKPG
LDKYKKLLEDAVSETTDLEKRYEKYAKAQAWSTDSSLLMPTASSGGSPVVSNVVPFSKPY
SQVGIKGEPYIFKGMKLQKDIVTTKEYNEVFKKWQKEKLESNSKYQKELEKYIK (SEQ ID NO:
205)
>orf01169
   LNFDFFIFLAHFIPLFTFSILQENPKTSKKKLYIRLL (SEQ ID NO: 206)
>orf01176
   MGFSMKLIHDLDTHTTHSTAKMLYNVKAIKNDFSIRE (SEQ ID NO: 207)
>orf01191 
LIRIIRNIYRSGEGNTSVFQSFIDQINSNQFCYGSNFDRLRCILLIENFTSICLNSNRMF
SGNGKILSNSSRSTP (SEQ ID NO: 208)
>orf01202 
MIYFDNSATTKPYPEALETYMQVASKILGNPSSLHRLGDQATRILDASRHQIADLIGKKT
DEIFLTSGGTEGDNRVLQGVAFEKAQFGKHIIVSPLPHSAVLE (SEQ ID NO: 209)
>or01223 
MKIKEQTRKLAAGCSKHCFEVVDKTDEVSSKHGFEVVDETDEVSNHTYGKATLTRIEEIF
EEYKSS (SEQ ID NO: 210)
>orf01233
   LVYAPFSFNILLDYITFDFKILLFSVFLAINRFHNDFIQFLL (SEQ ID NO: 211)
>orf01236
   MSGYSGLSFFEVALAEFLDIVSAVYLEDADGIIVNLWGILDK (SEQ ID NO: 212)
>orf01246 
MEGVAKGRIGRKKNNGIDNRCCHKKRNGRVTWNLFFQKTIDDGDDSTFTRREKHTDEGPK
KDSPPTISREKMINLVRCDINFNQP (SEQ ID NO: 213)
>orf01249 
VDKTDEVSSKHCFEVVDKTDEVSSKHCFEVVDKTDEVSSKHCFEVVDRTDEVSSKHCFEV
VDRTDEVSNRTTVRRS (SEQ ID NO: 214)
>orf01251
   MDYGLYHPCPIVTPSQSSSIVANPASKLISASEELIPDAIAVDFVEVNCY (SEQ ID NO: 215)
>orf01254
   MKKLFQEKFSKKPSHKEIERVQLGCAMMQATFHLMGY (SEQ ID NO: 216)
>orf01261 
VVIGVASATTNIWIIFLSGFAAILAGAFSMAGGEYVSVSTPKDTEEAAVSREKLLLDQDR
GLAKKSLYAAYIQNGECKTSAQLLTNKIFLKNPLKALVEEKYGIEYEEFTNPWHAAISSF
VAFFLRSLPPMLSVTIFPSEYRIPATVLIVGVALLLTGYTSARLGKDPTRTAMIRNLAIG
LLTMGVTFLLEQLFSI (SEQ ID NO: 217)
>orf01269
   MLYVGIDVAKNKHDVTALNVPGKLFLNHSLFQIIKLVLNS (SEQ ID NO: 218)
>orf01296 
MDFEYFYNREAERFNFLKVPEILVDREEFRGLSAEAIILYSILLKQTGMSFKNNWIDKEG
RVFIYFTVEEIMKRRNISKPTAIKTLDELDVKKGIGLIERVRLGLGKPNIIYVKDFMSIF
QVKENDLQKSKNLTSEVKDFNLRSKENELQEVKNLDSNYIENNKSKYSKREYSFGENGLG
TFQNVFLAAEDISDLQIIMNSQLENYIRLPAKLES (SEQ ID NO: 219)
>orf01304 
LLHIRVCKTFDRIPYCMLALFLSKSIGLTILLHKVKTVIFIDDQSNDKTCKICIHISFFR
IKLSQQCQLSFSVYF (SEQ ID NO: 220)
>orf01309
   MTQEDALIVISHIKVLSIVPNRCLKPLDKTFSLYNWIFLSQKYILLQANFLKISRVQLQ (SEQ ID NO: 221)
>orf01313 
VTTHDEPVYEKHGVLHYAVANIPGAVARTSTIALTNVTLPYIEALAGKGFAQAISEDEGL
RQGVTTYQGYLTSLPVAQGLNRDYTDINDLV (SEQ ID NO: 222)
>orf01314 VFFIDGFIVRCHTVSCFDNATLVNSNVNDTEPGRICLTISSVTNSGAFAPGMRMAPITTS ASLILASMLNELDIRV (SEQ ID NO: 223)
>orf01316 
MLIGIPKEIKNNENRVALTPAGVHSLVSRGHRVLIETNAGLGSGFTDADYQKQGAEIVAT
AGEAWAAELVVKVKEPLSSEYGYLRDDLLLFTYLHMAAAPELADAMLAAKTTGIAYETVR
DNQGQLPLLVPMSEVAGRMAV (SEQ ID NO: 224)
>orf01334
   LIRILGNSFISIDIKAHEQAEEYGHSFEREMGFLAVHGLFTY (SEQ ID NO: 225)
>orf01383
   VILFFIRFRVSWLTYFIMSLIFIRFTILVCLIFTNMVASVTKAIILMADVRLDVRHST (SEQ ID NO: 226)
>orf01387
   MIAMRSYITLICNLNNNLFCLNSFFLTNLVWSQIFSLLSVFITVYI (SEQ ID NO: 227)
>orf01388
   MFLFLAIDFIFYSYIFCMSLIFKVNIILSIYLNNISSLNLTDDILIFRLIVGGFH (SEQ ID NO: 228)
>orf01390 
VIPRGDHNHYIKVQTKGYEAALKNKIPSLQSNYQPGTFDEKAVLAKVDQLLADSRSIYKD
KPIEQRQIELALGQFTESLKKIKVS (SEQ ID NO: 229)
>orf01419 
MARLEPAKIAKIVLGILLYIIDLIKSSFVLPIPKAAKKSLILISFVPSFNDKNIVIRRPR
QITKIMPRFICFLFRIFACIS (SEQ ID NO: 230)
>orf01436 
MELSAIYHRPESEYAYLYKDKKLHIRIRTKKGDIESINLHYGDPFIFMEEFYQDTKEMVK
ITSGTLFDHWQVEVSVDFARIQYLFELRDTEGQNILYGDKGCVENSLENLHAIGNGFKLP
YLHEIDACKVPDWVSNTVWYQIFPERFANGNALLNPEGTLDWDSSVTPKSDDFFGGDLQG
IIDHMDYLQDLGITGLYLCPIFESTSNHKYNTTDYFEIDRHFGDKETFRELVDQAHHRGM
KVMLDAVFNHIGSQSLQWKNVVKNGEQSAYKDWFHIQQFPVTTEKLVNKRDLPYHVFGFE
DYMPKLNTANPEVKNYLLKVATYWIEEFNIDAWRLDVANEIDHQFWKDFRKAVLAKNPDL
YILGEVWHTSQPWLNGDEFHAVMNYPLSDSIKDYFLRGIKKTDQFIDEINGEFMYYKQQI
SEVMFNLLDSHDTERILWTANEDVQLVKSALAFLFLQKGTPCIYYGTELALTGGPDPDCR
RCMPWERVSSDNDMLNFMKRLIKIRKYASVIISHGKYSLQEIKSDLVALEWKYEGRILKA
IFNQSTEDYLLEKEAVALASNCQELENQLVISPDGFVIF (SEQ ID NO: 231)
>or01442 
MGQEIKLIRKQFRSTRQEEKQIKEMMREQKVDSFSEFLRQNLLKKNYQDRIFESWFSLWQ
SQKFEQISRDVYEVLVVARENHQVTQEHVSILLTCVQELIAEVNQVQPLSREFREKYMG
(SEQ ID NO: 232)
>orf01443 
MVYRYRTNLKKVFLTDPELHQLNERIAKSNCQNFSVYARKVLLNPNMSFVTINTDTYDQL
VFELRRIGNNINQIARAINQSHLISQDQLQELSKGVGELIKEVDKEFQVEVKRLKEFHGS
Y (SEQ ID NO: 233)
>orf01444 
MVVTKHFATHGKKYRRRLIKYILNLDKTDNLKLVSDFGMSNYLDFPSHAEMVEMYNVNFT
NNDKLYESRNDRQEKHQQTIHAHHIIQSFSPEDNLTPEEINRIGYETMMELTGGRFKFIV
ATHTDKDHVHNHILINAIDRNSDKKLIWNYALERNLRMISDRISKMTGAKIIEKRYSYRD
YKKYKESSHKFELKQRLYFLLQQSKSFDDFLEKAKQLHVQIDFSQKHSRFLMTDRTMIKP
IRGRQLSKRDLYDEEFFRTHFAKQEIESRLEFLLNRVNSLEDLITKAKELNLTIDLKQKN
VTFILEENNQKISLGHQKISDKKLYDVKFFQDYFKNKEVVASEGLENLQEQYHAFQEERD
KDKVSTEEIEEAFKTFKEK (SEQ ID NO: 234)
>orf01446 
VELAENQIEKLVDKGVYIKVSFGVKQSGLIFIPNYQLDIMEEENHKKYKVYIRETSSYFV
YNKENMDNNCFIKGRTLIRQLSNDSQKLPYRRPTLKSLQEKISEINLMIELSNTNKQYQE
 
IKDELVLEIAEIDMKLEETQEKIATLNKMAEVLINLKSEDHETRKLARYDFSKLNLTEST
SLENVNEEIRVLQENLDYYLYEFEKRAIRLEIFVSTLNMEKDVFVIDKF (SEQ ID NO: 235)
>orf01447 
MAIIKKIVVLYGGLSEEREVSENSAKEISKSLLTLGYDVISIDLSQDCSYEIGEIEKSER
GLNKQKVEIGSGIIDVCRKVDIVFLATHGGIGENGKLQAIFDVEKIDYTGNSFLSTAISM
DKKLSKIVASSVGIKCASNLMVDRIQANDFPIVIKPIRSGSSKGIKIFQNKKQFDIYYKE
HPELGSVFVEKYIKGREFSVGILGETVLPVIEIKVKNGFYDYNNKYTVGAAEEVVPAKIS
EKLTHTLQKSAYKIHKALGFNVYSRTDFIVDEKGDVYFIESNSLPGMTKTSLLPQEAKAA
GIDFPNLCERIIELSREIRSQ (SEQ ID NO: 236)
>orf01449 
MKIINGICRYIFDSKGFATIEVEIFLDSGDTGIGAAPRGSTTGHYDIQYNEYYPRGNNFS
PIPDGNIEFFNENILPRIINREVEDIEDITELDKHLFDIPEIENYGNVAIACSYAVWEAF
SKNKKSPLWKLFFEPGSASKGKVKHLVNIIDGKPDSLLAGFEFLLVSEKEITFQSLLEIS
NIKNELMIKFKNQGFYTSISNQGALIINTDDFYIILDSLLETLKLYKNRYDIGLDLAMTD
RYDSSLGIYKVPWCVSQQQTVTEIMDTYCDWGVKYPLVYLEDPFSDEDLDSWRKFQLIKP
LKLQVFGDDFYATNLERISQFKDCADGIVIKPNQVGSVSKTLEVMEYAEKSGISMAFSQR
TAETENNIISHLAMSVTSSYLKAGGLDRLDRIAKYNEVLRNG (SEQ ID NO: 237)
>orf01450 
MDNGKISTDGALVNNKNIEVNISSASIRYGISVFEAPKMFLLGSDIYIFRFQDYFNRLKT
SCKFLNIELPLDYNKLLYDIKKFIEFVPWQESYALRINVFCPYESELLGEVECALTLSYL
DIGIRSKSGVSSKVIKRSNLIRTSNNNLYMIKSPSHYINARKELYSDIEFDDILYVNEKE
NICELSRSNIFLIKDRTVYTPDLGSGILDGITRKLIIDLCQEHNILIEIKELNYSKLSDF
DSAFCVGTTNGITPIRNIDKNIHFDTNNLLLAEIVNYYKEVFSKKGVEKYKEWFVKL (SEQ ID
NO: 238)
>orf01451 
MIKDVNYFNERAQIIRRTINDMIFGAESGHFGASLSSVDFINVIYENYVFPENAEFILSK
GHAAPALYAKLIESGVLDKDFIYGFREYRSLLTGHPNHRIPTLKFGLGSLGQGPSIGVGM
AWVNKRKKSDKKIFVMLGDGELNEGQVWEAFYTCRNLNLQNLVFIIDRNFLQLDGKCEDV
ANFPNLAQKISSFLGTNPIEVNGNSYDEILNVLDNIDYSQTNVIISNTTKGKGIEFMEGK
TEFHSYNILSSEKEVLYKRAMECLGGDKMA (SEQ ID NO: 239)
>orf01452 
MRKTFIDELINKNIEEQNIVVLTGDVGRSTYASKFKEIFPENYLNLGICEANIVGISAGI
AGTLEFVPFVMLFSKHLILRALEQINDSILMNKKKVILVGGYSGYSASKEGETHQLLNDI
SILSSFPDISIYCPYDQSSIQTAINESINNDYSSYIRINKNKILNDVVSRYISQGNKSII
ISMGYLGSLLSKKYMEDSDIREFADFILVSKIKPIDWEYWENFLRKYETVYIIEENTLTG
GLGEQFKNYFFGLGINIISFGIKPHFGETGDYETLLTNEGLSPDKIFEKIRRINHVQSSE
KSIWR (SEQ ID NO: 240)
>orf01453 
MYNLVKRVFGDDNFVKSDGIYLYTDDNKEIIDSCSNSMNVNLGYGVLEIEKVIHEQIRKI
NFMHTGKGTTYESLMLANRLHDIIKPYGNYKVYFATSGSDCIEAALRISVLYQQKNKRNQ
ESNTRFATFEGSYHGSTLGALSVSGHRKFQKLYNGYIGNCLTIPTRWNLEWEMDFTDITA
FVLDSMITNPIGSEMIDRDYLNYLVQICKESGVITIIDEIATSIGRLGCFFGFEDSGISP
DIVCISKGLGVGYANIGAVIVKSNIIDSINSADILGHTYNASPIDCKIAMTVLDYIEEHG
IFEHVNNLSNYIEYRLKVLKNKLPCISKVSGKGFMWSIHFRNNVNASKVFKQCYNNGLLL
LYLEYEEYNHMTFCPPLITSKNQIDSMLDILEKSITEVLHGY (SEQ ID NO: 241)
>orf01455 
MDIKKLGFIIITNETFAFNLVEEVVEKLSILDVNRAIIKLLPKVDETTLIKHYREHLINW
NGKEGKSQVPSLGWPAVRNRFSSSVVLILLEGNKQSLSDEILQIKGNTYPERCRQNQIRI
KGFNPTYNLMHSSDSAEEALKEAELYFSKKELESFFCGEIGITFEEILNFILELDSIQKG
GIRSNSDLENKVNKMCYWKQRIFDLFSSKTKEESRKISEELNFIKSNRSPEDVILYLENE
SILWSKLERDLYLSHLIIEEISGE (SEQ ID NO: 242)
>orf01456 
MVKIYEETLRDGIQSSELSMLNTEDKKKVIKNFDNAGIEGCCIGFINSSKKENDEINELF
QFIITNKLKIVPAVLCRLLIEDFKEIKNIVQCNLFNQLKVYTYIAVSPIRMKVENWSEEI
IIEKIIDFLNYCQMENAKITIAFEDASRADKSFLKKLIEIINNYPLVKSVVIADTVGTCN
 
YNSTRDLVYFFRKNLFLSKTIEWHGHNDLGLAVSNALSAIESGADIVHTCTLGIGERCGN
TSTEQLIINLIVSECGRRATYKLRYLYDVAKILENKSQFPISPKTPCFGMDSFFTCAGTH
ASSLYKSYLKNDNNFSIIFSPYDSNIFGREVTVGINSQSGRSSIEYISRKYGLELSQENI
TSILGLVKQNDLFFREEEFVDYVKQKRVERLDYK (SEQ ID NO: 243)
>orf01457 
MLNKKEWKDLTISKSENDSIISCLYNWGFELPKSKDYTFSLTWYGYNNVSYKYCEKECLT
LGELSLLFPQLNDLFWENQKDNIEGRRNFYKNCKNLLGKFNIYSVRDILEISDRDLQFAL
TNRVGSKEFENSIVSLMYINKLSDDNKAENTLEKKLYFSINNEDSIIPYYENTLGMTAGN
YYFTASHRTFFSSTFPDFFKIDVPYKISNSVRNLTRNELRIRASEIIKSELANSENPILK
IFDDYAYVRIGKRNMVLRNKFFNGFIAPLFAFFQQRIFDNGKSFFTHFYDLRKNTSQNIP
DYLLLNILFPLIDCFEFFLNVSIKYKRLIFPYDFHMQNIMISIYDNQIGFIFQDFDMSKE
LTVTSFYEQLDFFFEKFVVTNILESINELKLDEKKYIMREMKKIINTKIIIKAESYFGFP
SPSEVIFPDFMKQYLRENQNVKERTIKYRSYLR (SEQ ID NO: 244)
>orf01458 
LFIDILKKKMKIIEAHDALSAYIIDQTKYSADKGDIFEYDGIWISSLCDSIIRGKPDME
VVNITDRLQTIDDILCVTEKHIIVDIDSGGTLAQTASLIEKITLRKIAGIVIEDKIGQKY
NSLFGEKNTQLQDSTESFERKIRVAIKSRKNSKTAIIARIESLVLGKSLEDLLYRGNCYI
KSGAEALVIHALNSRLDDLYQALKYFKRYFPEIPLIVIPTDFPYVCAEELFESGADLIIY
ANHLLRSIIKPMEYIAKSILIDGYSNGVENKLLPISEILNYIPLLEEIDEK (SEQ ID NO: 245)
>orf01459 
MKSKDLISSLKLNGVTRFFGVPDTFLYPITSQLANDELIIVPNEGNAVSMAFGYSITTSR
MPVIFLQNSGLGNILDPVQSLVGQAVYNHPMLYIIGFRGGTDDAPQHSECGKVTKKLLEI
SQFDIYEKDYFTNALDTDIRRIIDNIKKKNKSAAILVDKEFFSDIVNIKNYQISDYNDIL
NKICAVLEKEKDSIVLTSTGYITRHMENVKDTYKKNFVHIPMPGSLGQTISFGAGICMGT
SINNKKKKIYIIDGDGSLFMHMGSLALFDYYNLDVTYILLNNYKHLSVGGENTLASSCNF
FKLAESMNFDNIYSSDNLEVVDFEEKLNQQGKNFIEIICSNEVTYSLLERMDFNFEEIKQ
FNMEN (SEQ ID NO: 246)
>orf01460 
MPDRYVEYSQNDYDNIMKLDTLFISQLDSKYKVEYHKLPSLISGEVLKRCGYFSTMPNQL
SKVDIIDVSQLESLGNNRELEEIKYSSSENYFLTPAACLHFYPMLEHKEVKECIYSSLVD
VFRYENGNFTMGTRQWEFTVREFLAIGNPKFVEEFLEDLKEKFLTIALRFDSTAKIQNAC
DNFYPTNLNKVKQKFQKYNNLKFELIVHISEKEVSVASFNYHNNHFSKEFNFDSNDTIVT
GCVGLGIDRWISLIKESEKDYKL (SEQ ID NO: 247)
>or01461 
MRKQIISLFHAYLNENTQDWVEIDENFLLYQHLDMFYYYLCKKNNIEPPLMEKFEVRKKV
IESRNRQYIKVARDLNAIFEKQSIQVAFLKGIQTSEKYYEEPWIRYYSDLDILVAREMIP
GVEKLFYQLGYVFGHLKDNGEIHHATREEILYQKLFTHEIYNLVKKENDNVFINVDINFL
FSWKGLSDSEIEFNDIKNDIIYDSKIKINTLDKVMNFIHICCHLYNEARYFALNRSFLGG
DPREIQLSRVFEIALILKDLKQEEFNSVVYSSRKLNCDNKVFASIGVTKQLLELNLSILD
NYTEELKVKDEFNSYIDKDQKVKYWPISIEDRVFDLKLKVKTCDKIFIN (SEQ ID NO: 248)
>orf01462 
LKYDSEIKNVIKDYIDFEFDINEIDNNVALTDYGVDSLKYISIILALEDYFKIEIPDNYL
VFSKSNTIKKLNSIIEELL (SEQ ID NO: 249)
>orf01463 
MKYINSSLKPVDNYYSLEEVTCFDRLLGIFLNSINNSYCDLFYMINNFYRCYKVDDSKNE
YDFEREMHILRNFFCIESEKINFSDEYVLTEFIVKNINKYGSVFVPINLKEIYYSAYYKE
QDWPHLFLINGYDKEKELFYVIDATQIYSDILTEQNFCITFEILERAYKSYFHQTILNKE
KEYIFVVTTVVNELTETEIFHETFKYLFNQSSISSRELEIVYKILTNRDFTLLANLKNIT
KKKKLFFTIFFEKLRVYELISNKELLYLSRTVETILEEWTIFINRCIKNILKNDTKLVNY
DFFVLEEKKIFDFFSKQASRYKERLFEIISSNSNRYNEVFECIQNSGKIITITEDNSRKF
RFSFIGDKIYNSWFNDDSPKVRINQDLNYLIVKINVIHKEKDSKFVAGLYCFIDDNLYYF
GLDSNYFINLDLMGKTPEIFRKRLETSEVYLKISRQEDSCKFSYSVDGITYFYATSLDIR
SCHFSCGIGCKTYSKPTPLCIDFEDLLIG (SEQ ID NO: 250)
>orf01464 
MAMIEVSCLRKDFVKIIKEPGLKGAIRSFIHPEKQIFEAVKDLSFEVPKGQILGFIGANG
[?]5
 
AGKSTTIKMLTGILKPTSGFCRINGKIPQENRQDYVKDIGVVFGQRTQLWWDLALQETYS
VLKEIYDVPDAVFQKRMDFLNDVLDLKEFIKDPVRTLSLGQRMRADIAASLLHNPKVLFL
DEPTIGLDVSVKDNIRRAITQINQEEETTILLTTHDLSDIEQLCDRIFMIDKGQEIFDGT
VNQLKETFGKMKTLTFELRPGQNHVVSQFVGISDIHVTRKELLLNIQYDSSRYQTADIIQ
KTLSDFAIRDLKMTDVNIEDIIRRFYRKEL (SEQ ID NO: 251)
>orf01466 
MVKLWKRYKLFISAGMQELITYRVNFFLYRIGDVMGAFVAFYLWRAVFSSSHQSLIRGFS
LSDMTFYIIMSFVTNLLTKSDSSFMIGEEVKDGSIIMRLLRPVHFAASYLFTEIGFRWLV
FVSVGLPFLIIIIGLKLLSGQPILQISLMTLTYLLSLILAFLINFFFNICFGFSAFVFKN
LWGSNLLKNSLVAFMSGSLIPLSFFPKIIADILYFLPFSSLIYTPVMIIVGKYNISQMIQ
AVLLQLFWLLVMIALSQIIWKRVQSHITIQGG (SEQ ID NO: 252)
>orf01467 
LLVGISLLSATVTSLTWTWTKVFIFLISIPFATLIYTSLKIATASIAFWTKQSGAVIYIF
YMFNDFAKYPVVIYNSFLRWLISFIIPFAFTAYYPASYFLKDKDGLFNIGGLILISLIFF
TISLKLWNKDLDAYESSGS (SEQ ID NO: 253)
 >orf01468
   MDEVFKFYCTNIIRVIFIKWLFCLKSMNHTNLTLVTTTFKQFNSTSLGNHHAMV (SEQ ID NO: 254)
>orf01469
   MQAFDGSTEFIHNGKGEKDSSKYQINGAGRCEVPVYVPKTDWDWIIYPQGIYD (SEQ ID NO: 255)
>orf01471
   MRVKRDYPNYKIYITENGLGYKDEFVDNTVYDDGRIDYDYVKKHLEVI (SEQ ID NO: 256)
>orf01472
   MEWIINIIRGIRYIGREAGVTFVEPSQTSIKSFDITIL (SEQ ID NO: 257)
>orf01473 
VRHYKDYISQRSDWELAGIYADEGISGTQVGKRQDFQRLINDCVNGEIDYIVTKAIARFA
RNTLDTLKYVRMLNDMQIGVYFEEENIDILTMDGELLLTILSSVAQQEVENTSAHVKKGL
KMKMERGELVGFQGCLGYDYDVETKQISINKKESKIVRYIFERYLEGIGGKVIARELDEL
GYKSPRGLEHWNDTTVLGIIKNEKYKGDILIGKTFTVDPISKRRLSNFGEEDKYYIKDNH
EPIISK (SEQ ID NO: 258)
>orf01474 
MYAFSSMLECGFCGSILSRRSCHCRSDYRKVVWHCVTSIKKGKKFCKHSKGLEKLAIEGA
FMEAYRQLYHSNENLMTDLLETIESELNDNSLNKELKRITNKLRTLLKKEENLVNLRLEG
KISDTIYDEKYNEISSEKEFLAEEKVNIETTLKSEIDVKKRLTEFKHLLSSQKMLTEFDR
AVFESIVEKIIVGGVNSNGEIDPAMLTIIFKTGETQNKDGKQFKSKRKNAKLETDKLCPQ
NSDEDKKLYSQGTDNTRGVCSVAGSILVSQ (SEQ ID NO: 259)
>orf01475 
MGGNPPIKKYSIVDKIVLSTKIKRIIIFTVFRENWEPYMKKYTEVFQSQFPNLNIDYLLL
DTEQIDLDSYLDADLIIIGGGNTEKYIATYVNQEFKNYIDHMLNKGAKVIGFSAGALLLG
EKVYVSPNDNSDHQIKIKDGLGLFSQFLISVYYDSWNDKANKDRAEELVNVPIIPLNDHS
CLVLDKLGNIIEKID (SEQ ID NO: 260)
>orf01476 
MDFSSKIAINTGWSDDKKYCVTDQNQQKYFLRVSDKEKLDSKKFEFDMMGKVASLGVPMC
KPISIELCDDEVHSLHEWIDGRDAIDSILTYSENQQYTYGVEAGKILRKIHTIPATEVCE
DWEIFFNLKIDDKISNEMIW (SEQ ID NO: 261)
>orf01514
   LCSALKNSYDIELIKVLSNKAHLYLPIETVTPQTVSTS (SEQ ID NO: 262)
>orf01537
   LVKNPFIEIERIERTWLTAHLYRKFDKYFHKTRPPIKVFEHLIGGLFIMKTFT (SEQ ID NO: 263)
>orf01538
   MIKIYFTKFSENHNPFCKIFEIIFTSLIFQSILNKNKKNPLHQGETNVV (SEQ ID NO: 264)
>orf01545 
MSQVKGLCVLDVDGTLILEEVIDLLGREAGHEAEISQITSRAMRGELVFESSLRKRVSLL
EGLPILVFDNVFNSIHLSLNVPEFISILQKNGILVGLVSGGFTPIVGEISKIPWYCLFHC
QPA (SEQ ID NO: 265)
>orf01546
   HLKSAELGIAFCSKEHLKKEIPHHVDKRDFLEVLPLIDCLE (SEQ ID NO: 266)
>orf01551
   MFGNWFFKAFVCSLERLAQDRTMNWFSCIGNKNTVAFVPILIGCFA (SEQ ID NO: 267)
>orf01565 
MEKYFGEKQERFSFRKLSVGLVSATISSLFFMSVLASSSVDAQETAGVHYKYVADSELSS
EEKKQLVYDIPTYVENDDETYYLVYKLNSQNQLAELPNTGSKNERQALVAGASLAALGIL
IFAVSKKKVKNKTVLHLVLVAGIGNGVLVSVHALENHLLLNYNTDYELTSGEKLPLPKEI
SGYTYIGYIKEGKTTSDFEVSNQEKSAATPTKQQKVDYNVTPNFVDHPSTVQAIQEQTPV
SSTKPTEVQVVEKPFSTKLINPRKEEKQSSDSQEQLAEHKNLETKKEEKISPKEKTGVNT
LNPQDEVLSGQLNKPELLYREETIETKIDFQEEIQENPDLAEGTVRVKQEGKLGKKVEIV
RIFSVNKEEVSREIVSTSTTAPSPRIVEKGTKKTQVIKEQPETGVEHKDVQSGAIVEPAI
QPELPEAVVSDKGVPEVQPALSEAVVTDKGETEVQPESSDTVVSDKGEPKQVAPLPEYKG
NIEQVKPETPVEKTKEQGPEKTEEVPVKPTEETPVNPNEGTTEGTSIQGAENPVQPAEES
TTNSEKVSPDTSSENTGEVSNKPSDSKPPVEESNQPENSGNTTSENGQTEPEPSNGNSTE
NVSTKSNTSNSNGNEEIKQENELDPDKKVEDPEKTLELRNVSDLELYSLSNGTYKQHISL
EQVPSNPNSYFVKVKSSSFKDVYLPVASISEERKNDKILYKITAKVEKLQQEIESRYKDN
FTFYLAKKGTEETTNFTSFSNLVKAINQNLSGTYHLGASLNANEVELSTDDKSYIKGTFT
GQLIGEKDGKHYAIYNLKKPLFESLRGATIEKLSLKNVSISGKDDIGSLANEAQNNTKIK
QVHVDGVLAGERGIGGLLAKADQSSITESSFKGRIVNTYETTASYNIGGLVGHLTGSKAS
LTKSKATVVISSNTNSSDQTVGGIAGLVDKDAHIQNSYSEGDINNSQRFGKVAGIAGNLW
DRESNSENHAGRLTNVLSDVNVTNGNAISGYHYNGMKITDAFSNKANKVFNVTLEKDEVV
SKESFEERGTMLDASQIASKKAEINLLTPPIVKPLSTSGKKDSDFSKIAHYQANRALVYK
NIEKLLPFYNKATIVKYGNLVKENSILYQKELLSAVMMKDDQVITDIISNKQTANKLLLH
YKDHSSEKFDLRYQADFAKLAEYSLGDTGLLYTPNQFLYDQDSIINQVLPELQQVAYDSE
AIRKTLGISPEVKQTELYMEDQFTKTKQDLANSLKKLLSADAGLAGDNPVTRGYLVDKIK
NNKEALLLGLTYLERWYNFSYGQVNVKNLVMYHLDFFGKGNTSPLDTLIELGKSGFNNLL
AKNNVDTYAISLASHHGTTDLFSTLENYRKVFLPDKTNNDWFKSQTKAYIVEEKSNIEEV
KTKQGLVGTKYSIGVYDRITSDSWKYRNMVLPLLTLPERSVFVISTISSLGFGAYDRYRN
KEHQANGDLNSFVEKSAHETAERQRDHYDYWYRILDEKGREKLYRNILLYDAYKFGTDHT
EGKATEVANFDNPNPAMKHFFGPVGNKVGHNGHGAYATGDAVYYMGYRMLDKDGAITYTH
EMTHDSDQDIYLGGYGRRSGLGPEFFAKGLLQAPDQPSDATITINSILKHKTSDSTEGQR
LQVLDPTTRFNDAADLQNYVHNMFDVVYMLEYLEGQSIVKQLDAYQKMTALRKIENKYVK
DPADGNDVYATNVVKNLTEDEAKKLTSFDSLIDNNILSAREYKAGTYERNGYFTIKLFAP
IFSALSSEKGTPGDLMGRRIAYELLAAKGFKDGMVPYISNQYEEDAKQQGQTINLYGKER
GLVTDELVLKKVFDGKYKTWAEFKTAMYQERVDQFGNLKQVTFKDPTKRWPSYGTKTINN
VDELQKLMDEAVLQDATGTRWSNYNPEIDSAVHKLKRAIFKAYLAQTNDFRSSIFENKK
(SEQ ID NO: 268)
>orf01580 
VLGGRANSVTSCTTNSHWNLTFTTKHVTCFSSLVDDIVHGNNREVHEGHIDDWTKSCHGC
SCCSSRDGSFRNRTVTDTFWTKFFKHSNRSTEVSSEDTDVFSHQEHIFIATHFLRHSKDN
GVTEGHCFCFHFISFSLVCVNIFKG (SEQ ID NO: 269)
>orf01581 
MDMFYIGHFLDIRRDTVTVVNAIENDWQVPDRSHVHCFVENTFIGRTISKEADNDFTGIL
HLLTEGCTDSDPHTTTYDTIGTKVPSIKVSDMHRSTFPFTGSSVFTKDFSHHSVEVNPFS
NSLPVSTVV (SEQ ID NO: 270)
>or01602 
MKINKKYLVGSAAALILSVCSYELGLYQARTVKENNRVSYIDGKQATQKTENLTPDEVSK
REGINAEQIVIKITDQGYVTSHGDHYHYYNGKVPYDAIFSEELLMKDPNYKLKDEDIVNE
 
VKGGYVIKVDGKYYVYLKDAAHADNVRTKEEINRQKQEHSQHREGGTSTNDGAVAFARSQ
GRYTTDDGYIFNASDIIEDTGDAYIVPHGDHYHYIPKNELSASELAAAEAFLSGRENLSN
LRTYRRQNSDNTPRTNWVPSVSNPGTTNTNTSNNSNTNSQASQSNDIDSLLKQLYKLPLS
QRHVESDGLIFDPAQITSRTARGVAVPHGNHYHFIPYEQMSELEKRIARIIPLRYRSNHW
VPDSRPEQPSPQSTPEPSPSLQPAPNPQPAPSNPIDEKLVKEAVRKVGDGYVFEENGVSR
YIPAKDLSAETAAGIDSKLAKQESLSHKLGAKKTDLPSSDREFYNKAYDLLARIHQDLLD
NKGRQVDFEALDNLLERLKDVSSDKVKLVDDILAFLAPIRHPERLGKPNAQITYTDDEIQ
VAKLAGKYTTEDGYIFDPRDITSDEGDAYVTPHMTHSHWIKKDSLSEAERAAAQAYAKEK
GLTPPSTDHQDSGNTEAKGAEAIYNRVKAAKKVPLDRMPYNLQYTVEVKNGSLIIPHYDH
YHNIKFEWFDEGLYEAPKGYTLEDLLATVKYYVEHPNERPHSDNGFGNASDHVQRNKNGQ
ADTNQTEKPQTEKPEEETPREEKPQSEKPESPKPTEEPEESPEESEEPQVETEKVEEKLR
EAEDLLGKIQDPIIKSNAKETLTGLKNNLLFGTQDNNTIMAEAEKLLALLKESK (SEQ ID NO:
271)
>orf01625 
MDESFDDIAHEQFTSNLTTKTDNVGVQLFFSIKGCCHITNQGRANTWNFIYSVVDTNTST
TDTYSKISLAASYSFPYFFTKDWVVSPCMVICTKVNDFISF (SEQ ID NO: 272)
>orf01629 
MLDFQDRSPWLEGQKEIDLSYDLFSTDAVTLDELQSRTIALRSLKHDKGLKVHFAEFPNL
IIWSTLNKGPFITFEPWSGLSTFLEEGDHLEDKKNVCLLEANQVEELGFEIEVL (SEQ ID NO:
273)
>orf01634
   MKIKEQTRKLAAGCSKHSFEVVDETDEVSNHTYGKATLTWFEEIFEEYKN (SEQ ID NO: 279)
>orf01643 
LIESQVFSSLQVCCLNLCHLKFQHFDTCLVFLLVFLDFQNLLAHFPIGIKTRLIGFFQVP
KSGITKFIQHLDMQLGTH (SEQ ID NO: 275)
>orf01644
   MVMLTMNIYKMLPNSSQNRQINHLTIYTADTTTILQDFPTDDNFIT (SEQ ID NO: 276)
>orf01645 MTNNICRRTSSQHHIHGINDNRLPCTRFTSQDSHPLFKIEGNSLNNGKVFYRNFK (SEQ ID NO: 277)
>orf01656 
MPHTRDNWQTRFKNSSYHNFFVKGPEILNRTTSTTNNEQIQIVPLISTRNISSNFLRSPF
TLNLGRIKKDVNTWES PADGRDNI SNNGSTTAGYYTNSLRKLGQSLLEAFLKQAFFCQFF
LKLFKLNRKRPNPIRLSFFNDDGVATTWFIDLYTPNHIDLHSFFQVKP (SEQ ID NO: 278)
>orf01660 
LAIIRNRTCSLKLINGHLTFWTLHFLTSTRILIELATINLNCRIHRGNLGNRPSQASNRF
INKLFIQGRQNRGFCDHFPTSILSRRGIAQSNFPLIDLTLVLHKLDHACRLANRNRQNTH
HIRIQGSTMTNFLGSQNLTQFKNRIMRGHSCFFF (SEQ ID NO: 279)
>orf01667 
MFALRKPGNIYTRITNPTTAALEGGVEALATASGMTAVTYTILAIAHAGDHVVAASTIYG
GTFNLLKEPLPRYGITTTFVDIDNLEEVEAAIKDNTKLVLIETLGNPLINIPDLEKLAEI
AHKHQIPLVSDNTFATPYLINVFSHGVDIAIHSATKFIGGHGTTIGGIIVDSGSFDWTAS
GKFPQFVDEGPSCHNLSYTRDVGAAAFIIAVRVQLLRDTGAALSPFNAFLLLQRLETLSL
RVERHVQNAETIVDFLVNHPKVEKVNYPKLADSPYHALAEKYLPKGVGSIFTFHVKGGEA
EARKVIDNLEIFSDLANVADAKSLVVHPATTTHGQLSDKDLEAAGVTPNQIRLSIGLENV
EDLIEDLRLALEKI (SEQ ID NO: 280)
>orf01668
   MTRDFKFETLQLHAVQVVAPATKSRAVPIYQTTFFVFDDT (SEQ ID NO: 281)
>orf01672 
MSQKKNNKKKNKRKNLLTNILAGFLILLSLALIFNTQIRNIFIVWNTNKYQVSQVSKEKL
EENQDTEGNFDFDSVKAISSEAVLTSQWDAQKLPVIGGIAIPELEMNLPIFKGLDNVNLF
YGAGTMKREQVMGEGNYSLASHHIFGVDNANKMLFSPLDNAKNGMKIYLTDKNKVYTYEI
 
REVKRVTPDRVDEVDDRDGVNEITLVTCEDLAATERIIVKGDLKETKDYSQTSNEILTAF
NQPYKQFY (SEQ ID NO: 282)
>orf01679 
MRWNIGCHPNRDTSCSINQKVWKTRWQDQGFPFIGIIVINEINCIFVDITKHFQSNLAHT
CLGITLSGSTISIHGTKIPMTIYKHVTVAPPLSHTDHGFINRGIPVWVIFTHDIPCNTSR
FFMGFVWGHTQFIHSVENATVNRF (SEQ ID NO: 283)
>orf01683 
LKKKWFFADYYDTTIILLALISVILVLLGFAEMIDLDNPPYSIIDLVIWGVFVIDYSWRF
FITKRKWRFILENIFDLLAILPLNAIFTVFRLGRIFRLVKLTKLLKLTRLLRIIGLTGKL
ERKISRFLRTNGLIYILYVNIFIILVGSSILSVVEEKSFSDSLWWALVTVTTVGYGDIVP
VSLLGKWLAVLLMLVGIGTIGMLTSALTNFFVKDNPDEQIKLDKLKDELSS (SEQ ID NO: 284)
>orf01693 
VVDFKQTRQDPHDITIYSWLRQVKSNTGNGSCCVRSNPFQAGNSFIGIWKLATKVSHNLL
GCSLHIANSRIITQALPSFQ (SEQ ID NO: 285)
>orf01698 
LINSQLIPLVQVVVNQGRKGIVGSCNSMHISSKVEVDVFHWQNLCIPTTSSTTLDPHDWT
KRRFADSNHGFLANLVQGIRKTNGKRRLSFTCRCWVDGSNQDQFTDWIALNCTNFIKAEF
SLVLSVQLQIVVRNTKFLYNINNWLQLNTLCDFNICFHSKFL (SEQ ID NO: 286)
>orf01711 
MLFIIGHLNFPTAGSFIDSTLHRLGNRVCIHDDMAFTVTSSTSNSLDESTFVAKETFLVS
IENSYEAHFRNVNSFTEQVNSDQDIKDTQAQVTDNLRPFQGLDIRVHVLDLDTHFLEVVG
QILCHFLGQSCDKGTLIFFNAGIDFTQEVINLSHSRTDFHLWIQESRWTNDLLNHCLGLF
IFIVTRCR (SEQ ID NO: 287)
>orf01712 
MNVTLKLLPTERTIVQSRRQTETIINQHFFTRTVSIVHALDLPYGHMTLVNHNQEIIWEE
VEKRIRRLSFAPSIHVARIIFNPIGIAHLTQHFDIILCPLFQTLGFKQFTFLFKDS (SEQ ID NO:
288)
>orf01713 
MIHFSQHLTCQSLNFTNTVNFVSKKFYSKGMFISGSWENLYHIPTNAKSSALEINIIAFK
LNIDQVIQEFITRNL (SEQ ID NO: 289)
>orf01714 
VAKLVNLVIDRTILLNIGIARRDIGLWLVIIIVGYEILNCIFREKFLKLPIELTSQSFIV
GNNQSWFIDFRNDLTHSIRLPCSSRPHQNLSFFSPLNVIHQLLDSLGLIS (SEQ ID NO: 290)
>orf01734 
MNITQTDFLAVNLVFAISTTIDMAFHPDFLTCILDKSVMIIQSHNYRSIIERFATFGSSK
DDIRHLAPTETLDTRLPQSPTQTFCNIGLSRSIGSNNCRHTLVKNDLGLISKRLEPLNFD
FL (SEQ ID NO: 291)
>orf01736 
MGFIVCNHLKLACFNLRNHDLIDKFLDLGHILIQKKGTKKGFKGITKNGVTIATTRFFFP
FTQLDKLVKLAITRKTS (SEQ ID NO: 292)
>orf01748 
LFTCFSKLDNKTASTTYISHKFFTAIPVCFEFFKGFWFPRKDTTKKNIFIPMFLVECFNF
WVELR (SEQ ID NO: 293)
>orf01753 
VVRELTGEIYFGDHILEERKARDINDYSYEEVERIIRKAFEIARNRRKIVTSIDKQNVLA
TSKLWRKVAEEVAQDFSDVTLEHQLVDSAAMLMITNPAKFDVIVTENLFGDILSDESSVL
SGTLGVMPSASHSENGPSLYEPIHGSAPDIAGQGIANPISMILSVVMMLRDSFGRYEDTE
RIKRAVETSLAAGILTRDIGGQASTKEMMEAIIARL (SEQ ID NO: 294)
>orf01754 MAKKIVALVGDGIGPEIMEAGLEVLEALAEKTGFDYEIDRRPFGGADIDAAGPPLPDETL KASREADAILLVAIGSPQYDGVAVRPEQGLMALRKNSIFTLIFVL (SEQ ID NO: 295)
>orf01758 
LANIESHCNFFQSSIFSSLPNTIDSLFNTSCTILDSSKAICHCHSEVIMTVRRIDDLTIR
LDILNQVFEDGTTFLWCGKSYIF (SEQ ID NO: 296)
>orf01768 
MPRNRFSFTVRVTREKNFISFFSFFFQVIDKRAFSSDIDILRFIIIFNIDGHTGFLQITD
MPDTG (SEQ ID NO: 297)
>orf01772
   MKIKAQTRKLATGCSKHCFEVVDKTDEVSSKYCFEVADGS (SEQ ID NO: 298)
>orf01781 
VHAHTDKLCNGCNRIFNSIISHHTIFRERNKLSHKAIKSTRQEMGPCHVVFIEFFITLHR
RLIGNHDNFLTNLVGSGRVRNDGST (SEQ ID NO: 299)
>orf01782
   VNHCHWKLFIQNLGITFSLIVTLIRMTDSHVVGTDKDMILLVNSLFLIFDIDKLRLS (SEQ ID NO: 300)
>orf01784 
VGNNDILWSKRTISINGFNDFLNTCIAVSTTLCNDDTFLIKRKIFIYKIFCMRNPVSMNT
NYNFFNTWLQDKFFNCMNQNRSIT (SEQ ID NO: 301)
>orf01794 
LVAPVASSTRFFKNNDSLTSWNNGFIIITINTIISYQRISKGQDLSIIRLVCNGFLVAGH
PCIKDDFACYINICSEGLAFKNCAIF (SEQ ID NO: 302)
>orf01796
   VVCYFYITIDWSWVHEDCCFFQTIVTFLSQAMLGMVVFF (SEQ ID NO: 303)
>orf01797
   MAFVLHTEKHHDINLINDFINGYKLSIVCKLLTSPFLRSREKEFSSQAFQNLHIGFGNA (SEQ ID NO: 304)
>orf01798 
VVQVTCNSNFKTLKVAKFLINGHQIKQALARVLARTISTIDDGSRNRWTSNQFSIVVDLW
MANHTDIHS (SEQ ID NO: 305)
>orf01799
   MCPCRILKEEIGNNRMVFIGNLGSIFKLNSSLDQFHYLINSEVFHGHHMVQCLLIF (SEQ ID b10: 306)
>or01824 
VQFHLIIFQNLFCSLDIVIDSLTTNTKLLSYLSKTVIISVVKLYIIHLLICQKRRIKFKE
RIHTIGFFDSKLTIXXXFHSLIN (SEQ ID NO: 307)
>orf01826
   MHFHIIKLVNHFQLLIKLNRISHPNLHIKSSFLSLVLLFYQKEQDFAIMVI (SEQ ID NO: 308)
>orf01840
   MTGKKGFLFLNCHICMVTTTTCFLKERVESELLIFFYISPNRCLITV (SEQ ID NO: 309)
>orf01843 
LNRSILDNITLKHEVTSQKIEEVCKAVQIYDEIMAMPMKFNTIISEMGSNISGGQRQRIA
LARALINNPSIVILDEATSALDTINEERITKYIKSQGCTQIIVAHRLSTIKDADIIVVMK
GGKIVESGNHKYLMDLGGEYYSLYTKRK (SEQ ID NO: 310)
>orf01845
   LFKGGVTISRTPLSSEDTVMIDATEVKINRPKKKTISE (SEQ ID NO: 311)
>orf01848
   MAGKKDFLFLNCHICMVTTTTCFLKERVESELLIFFYILLNRCLITV (SEQ ID NO: 312)
>orf01850 
MYQDEAGFGRISKLGSCWSPIGVGPHVHSHYIREFHYCYGAVDAHTGESFFLIAGGCNTE
WMNSFLEELSQAYPDDYLLLVMDNAIWHKSSTLKIPTNIGFTFIPPYTPEMNPLNKCGKR
FVNVDLRIRPFELWKMS (SEQ ID NO: 313)
>orf01852 
LLQSPYAIDTINLKKDFLEKPIDIEKFKAFLEKEEIPLAIAWQGDSLHFYTKDRSILDNH
LDHLLEKMVNDPEKLSDFSMDKSLDDTIDEAKSQITFK (SEQ ID NO: 314)
>orf01853 
MKVVNLYDLKQMGNKGGCTIQLIHHFPFGMGLGHLKKDYIEFKRVGIFDGKAVEVTLREP
YSRDILQVVKSIKQRQKLIAYRYKEGKLLFVKKEV (SEQ ID NO: 315)
>orf01876 
MKPSGQEFPHPIFYSLFCFYTDTLGINSQVMQFSFERLLFQFCLNCWHLVMRSQNHNCRP
STRKVGCIGPIFFGHLLNHRKFSYQVLTIALMEEISLDCLPSGHHVSCQQGSNRYIGDRT
CSNSFLIRQFFRQDTTAVAST (SEQ ID NO: 316)
>orf01878 
LQVWYNLQSDFEQEITLIMWNPFANLVFNQPLISFFADLNLKILGYSYTDRVKTWPDIGT
GCRYNNLHLILLAP (SEQ ID NO: 317)
>orf01909 
LRQNRCYNCFHDHSCSWKSSRITSLHGCLVRFVGFDIHTHKRFIKSRNGFHDPTNNDGLP
ISHTTFKTT (SEQ ID NO: 318)
>orf01910 
LAAFTITSLKAKTKFHPFKGIDRDNSLSQSCIQFSIPLDIGTKTNWNASDDCLHNPTDGI
TITFDLVNIVLDFLFSFLVDNRNFRLGSSLLNFSDCQIFRNIDFLATKDHDMVGNLHIQL
SQETFGYCTNCHPHGGFTS (SEQ ID NO: 319)
>orf01911 
MTWARMSNFPLAFKAVFNVLRRHDVQPFLVVLIDDIHSNRRPCRLPVANARSKDNLVILN
LHTTTTTVATLTASKVLIDILSCQWKSSWNSLNNSC (SEQ ID NO: 320)
>orf01913 
MHDLAITGSRFDGMANSVAKIEVKTNTIVQLIFNHHLALHLTRMFNQGLCMFQNTLNRTI
QSRQESPQFWILNQAILDNFTHPFNQLSFSEGFKNKWINQNPIWLGKGPHHIFSKWCVNT
CLSTDRRINLSCQTSRNLNKVNTPHIGRGYKSSQVPNNATTKSNDSIATSQTLLD (SEQ ID NO:
321)
>orf01915
   MVICHNDYLLRLPEFSQPLTSLGHTTFFNLNIIRMMRNIDSDFHRRVSLSLLVFFC (SEQ ID NO: 322)
>orf01920
   LIEGHLVFADKPAQALVLLRKVGSPKKVSFLTLHLYFLILKIDILKITGF (SEQ ID NO: 323)
>orf01951
   MAVTKSQVFSRQGFDFSILGQDLTRLQDVSHLATIGTRIHKDSTANASWNTTSKLKAS (SEQ ID NO: 324)
>orf01952 
MTEGNASCFNQVSPSFCFNGIAINRNVIELVTQDDKSTNPTITNDDIACIAKNHPRDIFL
VGKFHNASQLKTISWKDQIISLSTYFCITIAMQGFLKTDINSF (SEQ ID NO: 325)
>orf01965
   MRLRDLRRVDFPDPDGPIKAVISLGWKDRETLFKAFFLL (SEQ ID NO: 326)
>orf01968
   LKNHSNVFTHFINVDFWTVDINSTIENLPSYFSNINGIIHAIETA (SEQ ID NO: 327)
>orf01969
   LHINPLNGFIFTIVNMDILSRKGYFFFRKGKDMLLIPVIC (SEQ ID NO: 328)
>orf01981 
MTIHIQVVKTNMVILADRLFQGFILRSTDKFFIKIRLVRSHNLRFNSMDFSTVAVHENKG
RHHMDELLPRFIINSKATVAKKSIVAQGFRFDGNFFRKTRQTNHLNIIFCDNPDQIIVFQ
NGLITNSQFNRLHP (SEQ ID NO: 329)
>orf01988
   LGHSKAEEHETICSHPFDDHTTETIPNQVKGRDMTSSETLPFPSKNQNQGKAKQNP (SEQ ID NO: 330)
>orf01991 
LLLSCRKVIVCFIFSSTWNKNFFNLAFSWNFDNCIRGFFSINSNLFGNMTSLWINIVGPC
RGYIAILSVNCNRIFTTVFCFIFFKTNSRT (SEQ ID NO: 331)
>or01992 
MVKRRIRRGTREPEKVVVPEQSSIPSYPVSVTSNQGTDVAVEPAKAVAPTTGWKQENGMW
YFYNTDGSMATGWVQVNGSWYYLNSNGSMKVNQWFQVGGKWYYVNTSGELAVNTSIDGYR
VNDNGEWVR (SEQ ID NO: 332)
>orf02006
   MRFIVGRFTSFSLGIEFSPTSKLDDLLFKIAFLMILATWIKARKTKGAT (SEQ ID NO: 333)
>orf02010
   MANDNKSHYLIYRVLGISFEEGENIDLYQNKGRFLYKYAGSFLEEAAVLSFNEKFGTENT (SEQ ID NO: 334)
>or02019 
LVNCKPLEAYRQLEEAELVGCWVHVRRKFFEATPKQADKSSLGAKGLAYCDQLFALERDW
ETLSADERLQKRQEELQPLMEDFFAWCRRQSVLSGSKLGRAIEYSLKYEETFKTILKDGH
LVLSNNLAERAIKSLVMGRSKRVQWTLLA (SEQ ID NO: 335)
>orf02022 
MFPVETEEITYKRKKSKGKCQALLAQFDSEEVHHQVEESICSDCQGDLKEIGATLQRQEL
VFIPAQLKRIDHIQHAYKCQACSDKNPSDKIVKAPIPKAPL (SEQ ID NO: 336)
>orf02023 
LKIIQQQSATIDSLTNELALLREQVAYLTQKLYGKSSEKSVCPSGQLNLFEEESPSEEDG
DVPS (SEQ ID NO: 337)
>orf02024
   LT1PVKDFKAVFTSTTKEKDLTGERIQFKVGFNQISQ (SEQ ID NO: 338)
>orf02037 
LVCQTIKYWHKFHLHIGRCKLLIGLIPVLNFFIRADIDCLLVLLSLIDRQNGKQFNLCQW
IIASNGLNDSFEIIESLIHRNILSDIICPNQKKNFIYCSTI (SEQ ID NO: 339)
>orf02042
   MIFSCSDSCFSIILLDGDIHENTTFSPLSILFISHRFNSLIGNEVPH (SEQ ID NO: 340)
>orf02043
   MPSKDNIRSPIDHLVIKSFLFFSWFQSILNTHLRHDNGDICFLLCPFNFSLHLIFV (SEQ ID NO: 341)
>0ref02045
   MVSSKSPIAKSACLVHLLEPRSHILKIFMKVIGTVFFFS (SEQ ID NO: 342)
>orf02047 
VVEQIPVGHNSGSFFLFLLLRLLLSPLLRNSISFLTSQGIPWKLSNNKTKPIDKPTASKS
IATNPLLLHLR (SEQ ID NO: 343)
>orf02054
   MSCTKIGVPFKKLTKKACKGYKILTRLSSQWQKEAPNQSRSAAIXXHSIH (SEQ ID NO: 344)
>orf02075 
MNQIQIQIIQAFFYRFFQSLTSLLIGLLTIPKLGSYEHFFTWNSAIFDCLTNTFFILIN
RRRINMTITSLQGF (SEQ ID NO: 345)
>orf02080
   MLLLISLTQLIIFLFFERFNLLLKTFLLVDLKSNKSA (SEQ ID NO: 346)
>orf02095
   LIFIEYKIADKTITIIGLSRVNVGRRIALLIRYFIAK (SEQ ID NO: 347)
>orf02098 
VIPRYVTKHQGWDHNPHTITNSDDDPATLVTFRTFKFNVGNCTIPKNDQNGSSQKFSGIL
QCPCEIHLLDSP (SEQ ID NO: 348)
>orf02100 
VVPKTATSTETKTITRIIHYVDKVTNQNVKEDVVQPVTLSRTKTENKVTGVVTYGEWTTG
NWDEVISGKIDKYKDPDIPTVESQEVTSDSSDKEITVRYDRLSTPDKPTPEKPEIPSPQE
PGTPGEPTPEKPIPQPNPEHPSVPTPNPELPNQETPTPDKPTPEPGTPKTETPVNPDPEV
PTYETGKREELPNTGTEANATLASAGIMTLLAGLGLGFFKKKEDEK (SEQ ID NO: 349)
>orf02105
   MAYSTDFKQRALDSIKEGHSHVEAAKFFGVGVRTLFTWEKKDVNKDT (SEQ ID NO: 350)
>orf02143
   MPILQAKIDSFRPLFOGLTSRFKVGAGLFLKRFSFSRVKQILLALPQFSMMNPIVNG (SEQ ID NO: 351)
>orf02158
   MAVQANWSFDITHDSSFFFSNQKRGLNFSQMCFKDRRRNGFFDRKIFKFKFNNPIQIF (SEQ ID NO: 352)
>orf02160 
LFLQIKGIKPNHTIALASYIEQSFFFINKTVHFKIGKQLIRTLQTNPFVIQLNCHLFQGC
KKKCSQALSLMVRLDD (SEQ ID NO: 353)
>orf02168
   MEIVLVSFSISFQHFIIAYCLDFSSAGFRNSQNFSNFC (SEQ ID NO: 354)
>orf02180 
LNFNHVYFGYDENRPVLKDITCSIFKGQKIAFVGPSGSGKSTIVRLLERFYKPLSGDILM
EQSSIYDFNLKEWRSKIAWVSQNNAVLSGSIRDNLCLGLNRLVTDDELMKVLDLVSLGDE
IRSMKEGLDTEVGERGRFLSGGQSQRLQIARTYLKDAEILIFDEATANLDADSEYAIISS
LYSVLKEKTVVIIAHSLSTVKDVDCIFFLEEGKITGSGTHKELLENHERYARFVQEQMIE
(SEQ ID NO: 355)
>orf02181 
LIYAEKSFFDKSQSGELTSAIVNDMSVIREFLITTFPNIILSLVMVLGSIVVLFSLDWNL
SLLLFITLPCMMFIILPLSNISEKYSRRLQKEIGFLTGQLTEKIQEHELIKTNQAEKSVQ
NVLDNCIERVQNNSLKSDRVTSFETPFALLFIFATIAVMLTYGSYRVSAGYISVGTLVSF
LIYLFQLLNPISNIANFVTVYSRSKGS (SEQ ID NO: 356)
>orf02183 
MKLKLLRVDTKVIMGSFFLVLSSLLALLLPILKGLIDGSSIENIGSKVFQSFLIFIGQA
LFSSIGYYLFSQSGEKKIAKIRKKVI (SEQ ID NO: 357)
>orf02196 
LIRYLDQYEDVILREIKAQFPDVAVDKLMEEYIKAGLILRENKRYYLNFPTLESLDSLEL
DQEIFVREASPVYQALLEQSFETELRNQINAAILVEKTDFARIKMTLSNYFYKVKQQYPL
TEKQQELYDILGDVNPEYALKYMTAFLLKFLKKDHLMQKCRDIFVDSLVVLGYIVQNEDR
KYELAIDFDKERLTFYLA (SEQ ID NO: 358)
>orf02197
   MIGLKEVCRFLTDNTSLSTSMINHPIQINGNMAIVTCGSLDGLSHV (SEQ ID NO: 359)
>orf02206
   MLINDLTFFIFDISPIQSYKKVRLEITNLWNNTKNATSRGDSC (SEQ ID NO: 360)
>orf02208
   MRERVRLSGSLFTSLKTREHIKSTMELFHKYVFFLIQEIKIKMINFLKIGDLPTL (SEQ ID NO: 361)
>or02219 
MIDHFEIKVKDLQISEGFYRSFLAPLDYKLTFKTSSLISFLSPNSPHPGGDFWLTQGTQD
PVHFAFLAENKEEVQACYEAGLEAGGRDNGVPGYRSEHPIYYAAFMIDLDGNNIEVVCHK
E (SEQ ID NO: 362)
>orf02221
   VIVFLSRNKDGNAFCHLDLISIANPVWGWDDDFITWIDHSHKEGIERIFGSRSDCHLI (SEQ ID NO: 363)
>orf02224
   LSNQFYFSLQTKPILKVKQFLLFQSQMTRVSEILQFSNKL (SEQ ID NO: 364)
>orf02227 
LFRLGQLISLNVAVHKPIKKFQGWIVLSSLPFQSLDILKFFRRFLSRYLVETLQLTGRIE
SQGIKHGLTFWF (SEQ ID NO: 365)
>orf02229
   MEDKEMGFYLMVASMLLGLLALKIGFSQFKEKKDKFLSILTSLAGTALVLVAVWLGWPK (SEQ ID NO: 366)
>orf02250
   MLDSDIGCSRKNLLGLFWIRRRRNIHIVDRAMEKGISNRAPNKISLKACFFNFF (SEQ ID NO: 367)
>orf02278 
LLHPFTRNITCDRHILTLLGNLVNFIHIDNATLCTFDVKVSNLQEFEEDIFHVLTHITSL
RQSCRIRNSKRYIQALSQGLGKESFP (SEQ ID NO: 368)
 >orf02279 
VEIDAFVVVINRHCQGTLGTILTNYIVVQDMEEFNWFWHLRQVCQDFLNQFFSNDFLSQL
HAFITNKSIVASNYFLYFFLVFATK (SEQ ID NO: 369)
>orf02285
   MNXXGKGEEGEDLVVGVFKWLYSERLRFDTERVGGGGKGK (SEQ ID NO: 370)
>orf02291 
MHKNFVVVVTNFFTAVQFIQFNKEGTTCHNTTKFFNHLDSCLNSSTCRQKVIYNKNTLTW
LNGIRVHSQGIDTVLFFIVSRNNFAWQFTWLTNRRKTNSQLKGNWTTHDKSTSFRSHDHV
DFLVSSILNDFTNSVAISISISHQRTNITEGNAFLWIIFNCCNVIF (SEQ ID NO: 371)
>orf02299 
MLNRQVCFCFVNHISPLNVVIWENLSLEELLYAICICFITHKIAKQTSLTIDNAGIAMNN
IR (SEQ ID NO: 372)
>orf02304
   LDSRFFCTDFFKGRQAKGCSFSCTSLSLTDNILAFKGQRNSLFLDRTSFYKTSFFNFC (SEQ ID NO: 373)
>orf02315
   LLRKQEREYLRAENAILKKLRELRLKEEKEKEERQKLFKN (SEQ ID NO: 374)
>or02320
   MRFLADQDRIQHHRYSWALFDKVQGLLSHTDSREKTNLNSPKFHITQAI (SEQ ID NO: 375)
>orf02321 MLKNGIISWKDFKSFFCQGCQTSHCYKPMQVVQGIGSQISRQSTTTKNIISRKC (SEQ ID NO: 376)
>orf02324 
VTAHRIFGTSSIHSKLIGLAMLGITAMKIICHKLNRNHINIFRRLGIQGKTEFLLIHLIR
QVKMNDLSQGMNPTICPTSTVNSNDLPFI (SEQ ID NO: 377)
>orf02328
   MLARSKNCFMKSLSIFLLIFYFFDSYQISKKRRSLIGL (SEQ ID NO: 378)
>orf02339
   LEVCIHHHHQISCRILQACIKGCFFAKISRERNIMDCRILLPIGL (SEQ ID NO: 379)
>orf02345
   LTGNVICHPKLPDKISVKCLYSSKIQFKPRQRRLAMGMVTDFVSSIHNLKAAL (SEQ ID NO: 380)
>orf02356
   MGRKPKKRPEERTELERLQAENEYLRAENAILKKLRELRLKEEKEKEERQKLFKN (SEQ ID NO: 381)
>orf02360 
MDEIKNFRQWGSKTPGHPEVTHTSGVDATSGPLGQGISTAVGFAQAERFLAAKYNKDGFP
IFDHYTYVIAGDGDFMEGVSAEAASYAGHQALDKLIVLYDSNDICLDGETKDTFSENVRA
RYDAYGWHTVLVEDGTDLAAISTAIETAKFSGKPSLIEVKTVIGYGSPNKSGTNAVHGAP
LGAEETGATRKFLGWDYDPFEVPEEVYSDFKTNVADRGQEAYDAWASLVSDYKVAYPEVA
SEIDAIVAGKSPVTITEKDFPVYENGFSQATRNSSQDAINTAAVLPTFLGGSADLAHSNM
TYIKADGLQDKYNPLNRNIQFGVREFVMGTILNGMALHGGLRVYGGTFFVFSDYVKAAIR
LSAIQELPVTYVFTHDSIAVGEDGPTHEPVEHLAGLRSMPNLTVIRPADARETQATWHHA
LTSTTTPTVIVLTRQNLVVEEGTDFGKVAKGAYVVYDTPGFDTIIIATGSEVNLAIKAAK
ELVLQGGKVRVVSMPSTELFDAQDATYKEDILPSKTRRRVAIEMAATQSWYKYVGLDGAV
IGIDIFGASAPAQTVIDNYGFTVENIVAQVKSL (SEQ ID NO: 382)
>orf02362
   LNFLNEPRRQGNIGNKMAIHNIDMIRCYFIIQKSNLLFEFVQIHGHQRW (SEQ ID NO: 383)
>orf02367
   VIYSYDYPRLLHSRTLAMGNSNIIPNTGLSFCLSLIKTFDKLVSIRHITRLNQ (SEQ ID NO: 384)
>orf02371 
VSRKQEQMETLLLLLRDSKDYISAKVLGEKLNCSDKTVYRLVKGINKDCPVEAFILSEKG
RGFKLNPRSSLVDVDGNFTEAFDPEVRREKLLERLLLTAPKPHSIYDLGEEFYVSESVVL
KDRQILQESLAIYGLDLKMRQRKLFIDGDEAQIRSAILNLLPMFNQLDLEQITQNKVQPL
DGELAHFCLGLLITLERELGVNIPYPYNINIFSHLYIFISRNRRSTSIHVVAPSKPTIVD
EKIYSVCQKIIQEIEQYFRMKVDAVEIDYLYQYVVSSRLQKPFSSGKLPFSQRVLDVTHY
YFSRMCMDNREIETTDPDFVDLASHISPLLRRLDNRVQIKNSLLSQILLTYPNLVKELTT
ISKEVSLVFGFASLSLDEIGFLVLYFARFQEKRARPLKTVVMCTSGVGTSELLRARLEKQ
FSELDIIDVVAYHQLDELINLYPDLDFIVTTVALQEPASVPFVLVSAFLTEGDKQRLQAK
IQEINYE (SEQ ID NO: 385)
>orf02390
   MMSMVDPIDQTFIVNLKIRKSQVFSQLQFSCHIVVYPSEVHIYQALVIKLQNHILGPQVL P (SEQ ID NO: 386)
>orf02391 
LANRTRIDNQLPTSPVTKQLLVNMSINSNITGRMSHQAVKLLLFASMNQLSPPVLIRQMM
ANSHRQIPKLTMNLKRLIVEHFNFF (SEQ ID NO: 387)
>orf02395
   MDSIEFFHDKTFLFYLTSFYSKRMGVTTMIKIAEGRFS (SEQ ID NO: 388)
>orf02406 
LTRFEEIFEEYKNPQDTFFYPLVYKENTYKKTAISIFALLMLGVCCLFLFSQQSYKKLVQ
YYANDQNLPSRITYSEYSDK (SEQ ID NO: 389)
>or f02414 
MIAEFIDGLQKFHFLQNALITAIVVGIVAGAVGCFIILRGMSLMGDAISHAVLPGVALSF
ILGLDFFIGAIVFGLLAAIIITYIKGNSIIKSDTAIGITFSSFLALGIILIGVAKSSTDL
FHILFGNILAVQDTDMFITMGVGAAILLLIWIFFKQLLITSFDELLAKAMGMPVNFYHYL
LMVLLTLVSVTAMQSVGTILIVAMLITPAATAYLYANSLKSMIFLSSTFGATASVLGLFI
GYSFNVAAGSSIVLTAASFFLISFFIAPKQRYLKLKNKHLLK (SEQ ID NO: 390)
>orf02424 
LNQEIIWKTRKSFTFKSRSLTDIRSRRKCLTNIQLSLIVRHLRQSRTLLAELNVNIPKRQ
IGPILTRFWPN (SEQ ID NO: 391)
>or f02437 
MQGEMRFSLVQFLTTLIKFCIFPFLLPNWLFGRKACTHWEFSFPKIKGVFEFHGISFINN
NKLKTTTSKKDENRGTTFIRKKI (SEQ ID NO: 392)
>or f02438
   MYEEPEVAPVHPTGPTPATETVDSIPGFEAPQESVTIL (SEQ ID NO: 393)
>orf02468
   MRLSWHFMRFKKLPLLINQTILDVGSTDINCNVICHKYLLLD (SEQ ID NO: 394)
>or f02470 
MGNNGQFTFGYRHDFFQNQLAIFNALVDTFTRRTIDIKTLNTFINEVLNQGTRTFWAYFS
LIIITCVEGWNDTFVFFQI (SEQ ID NO: 395)
>orf02497
   LSTRNKYCKNLIIFESTFNILDIVKKDLKLNSKLEKDLKY (SEQ ID NO: 396)
>orf02499 
MNRSVQERKCRYSIRKLSVGAVSMIVGAVVFGTSPVLAQEGASEQPLANETQLSGESSTL
TDTEKSQPSSETELSGNKQEQERKDKQEEKIPRDYYARDLENVETVIEKEDVETNASNGQ
RVDLSSELDKLKKLENATVHMEFKPDAKAPAFYNLFSVSSATKKDEYFTMAVYNNTATLE
GRGSDGKQFYNNYNDAPLKVKPGQWNSVTFTVEKPTAELPKGRVRLYVNGVLSRTSLRSG
NFIKDMPDVTHVQIGATKRANNTVWGSNLQIRNLTVYNRALTPEEVQKRSQLFKRSDLEK
KLPEGAALTEKTDIFESGRNGKPNKDGIKSYRIPALLKTDKGTLIAGADERRLHSSDWGD
IGMVIRRSEDNGKTWGDRVTITNLRDNPKASDPSIGSPVNIDMVLVQDPETKRIFSIYDM
FPEGKGIFGMSSQKEEAYKKIDGKTYQILYREGEKGAYTIRENGTVYTPDGKATDYRVVV
DPVKPAYSDKGDLYKGNQLLGNIYFTTNKTSPFRIAKDSYLWMSYSDDDGKTWSAPQDIT
PMVKADWMKFLGVGPGTGIVLRNGPHKGRILIPVYTTNNVSHLNGSQSSRIIYSDDHGKT
WHAGEAVNDNRQVDGQKIHSSTMNNRRAQNTESTVVQLNNGDVKLFMRGLTGDLQVATSK
DGGVTWEKDIKRYPQVKDVYVQMSAIHTMHEGKEYIILSNAGGPKRENGMVHLARVEENG
ELTWLKHNPIQKGEFAYNSLQELGNGEYGILYEHTEKGQNAYTLSFRKFNWDFLSKDLIS
PTEAKVKRTREMGKGVIGLEFDSEVLVNKAPTLQLANGKTARFMTQYDTKTLLFTVDSED
MGQKVTGLAEGAIESMHNLPVSVAGTKLSNGMNGSEAAVHEVPEYTGPLGTSGEEPAPTV
EKPEYTGPLGTSGEEPAPTVEKPEYTGPLGTAGEEAAPTVEKPEFTGGVNGTEPAVHEIA
EYKGSDSLVTLTTKEDYTYKAPLAQQALPETGNKESDLLASLGLTAFFLGLFTLGKKREQ
(SEQ ID NO: 397)
>or 02501
   VDRTDEVSSKHCFEVVDRTDEVSSKHRFEVADRTDEVSNIYTARQS (SEQ ID NO: 398)
>or 02512
   MSCNCAFYRSQFFDVNSVSNYHSHQKELRFPNS1LFTYFVKVT (SEQ ID NO: 399)
>orf02535 
VGLIKLTSYVFVCISNSFLTRHDKNDNICFFHGNFCLVLDLFHERSIDIINSSCINHAKR
TIEPLTRCINTVTCHSFDIFYNGDSLTSDPIK (SEQ ID NO: 400)
>orf02537 
LSSKSCIDRTNQETFHTLGLEGVGMKSGSLFCSVQISDKEKENSRLANGFLRYQFIQGIF
LLLTSYHNHRVGLEILPR (SEQ ID NO: 401)
>orf02554 
MKSKEQTRKLAVGCSKYSFEVADKTDEVSSKHCFEVVDRTDEVSNYIYGKAKLTWFEEIF
EEY (SEQ ID NO: 402)
>orf02556
   LSNSFFLIKFSSSKISGKKRIVSDNIFIRNKFICHFKKE (SEQ ID NO: 403)
>orf02564 
MDYSKVAAEVIEAVGKDNLVAAAHCATRLRLVLKDEAKVNQAALDNNADVKGTFSTNGQY
QIIIGPGDVNFVYAEIIKKTGLKEVSTDDLKEIANKDKKFNPLMDLIKLLSDIFVPIIPA
LVAGGLLMALRNFLTSPDLFGPQSIEDMYPAIKGFSAMIQLMSAAPFMFLPVLVGISAAK
RFGANQFLGAAIGMIMTTPDLGGKEAFWDILGFHVTQTNYAYQVIPVLVAVWLLANLEKF
FHKKLPSAVDFTFTPLLSVMITGFLTFTVIGPVMLVVSDAITNAIVWLYNTTGAFGMGLF
GGTYSLIVMTGLHQSFPAIETQLLSAYNNNGTGFGDYIFVVASMANVAQGAATLAVYFLT
KNAKTKGLSSSAAVSAFLGITEPALFGVNLKYKFPFFCALAGSAIGAFVAGLTHVIAVSL
GAAGFIGFLSIKAGSIPMYIIAEIMSFVAAFAFTYFYGKTKAASVFADEAATATAETVTE
PTVEAPVVEETDTLQNETLVTPIVGDVVALADVNDPVFSSGAMGQGIAVKPSQGVVYAPA
DAEVSIAFPTGHAFGLKTRNGAEVLIHVGIDTVSMNGDGFEAKVAQGNKVKAGDVLGTFD
SNKIAAAGLDDTTMVIVTNTADYASVAPVATGSVAKGNAVIEVKI (SEQ ID NO: 404)
>orf02570 
MTESYTWVEADRATLSRYRHGQGHLTDQFFSFKVQRPAAKTLIASISTGKGMGPSFDGTP
VITSGNQNRINTIKNSFIMSSSSVRISLRKLTSQRNFLRNLSSLILLAAQVAKGDATACS
HQRIGRVVGQDSHETLSLTEFF (SEQ ID NO: 405)
>orf02571
   MNINNEKVWFAFYLLDMQITRPTSTFNDRRIGLIGKLQELRFLAGNLLLR (SEQ ID NO: 406)
>orf02572
   LIKGYLPNHLSLMOLCSKTTCTLDOFAGIAGRRNHRGFFCHIGNGVFLTVOKYLWNQ (SEQ ID NO: 407)
>orf02578
   LIKLTGRNFSDILIKCLVKCFTNLLSNQLMLLPSTLKL (SEQ ID NO: 408)
>orf02582 
MIQSENHCSASHSNRDYQSQHDNQGRTCQCFIIVPCHKKGSCSVGEITWNQRCQNGQDKD
HSRCLIKNT (SEQ ID NO: 409)
>orf02589
   MIARQLMVFFSTNQADTRITNMSIDSLIIbINSKDFQSSSHASVSFILTKLVNLLIFNF (SEQ ID NO: 410)
>orf02590
   MGEPFTHFIDCIDLGINPSYTQVCHRHFTSDIPCAMTSHPIS (SEQ ID NO: 411)
>orf02597 
LSSDSHFIGIKAFVILILGKSNSIVLRIVGLYQDLTCLFSTTCSTCHLSQELEGSLRRTE
IRQIQGRIRI (SEQ ID NO: 412)
>orf02598
   MAVHSLGIHMQGQRNIAVGTSIHRPTLPTHDKARITTAIEHENHLLFFNQTVLDSL (SEQ ID NO: 413)
>orf02599
   MVTGIAVLLISRFMLFINNHDTQIFQRSKDSRSGTNNNLGIATLHLAPFIILFTIG (SEQ ID NO: 414)
>orf02600
   VKNGYLVPKTCYKTLGHLRSQGNLRYQQNSCLALIQGTLDNLQVNLGLPTSCNPLK (SEQ ID NO: 415)
>or02601
   MVNLIPRLGLDLLLIDCLIFQTKQAFSSQTHHFSLLGKV (SEQ ID NO: 416)
>orf02602
   LGLQTKNNPLNQAITLTKRHMNPHPNFQHSLKFLRNPVTIGLVRLHQGHIYDNLS (SEQ ID NO: 417)
>orf02605 
LGNHFCTICSTTYQAFLQFIQIWWCQEDKDSIWNLFLDLKSTLNFNFKENIDSLVQGFID
IGQRSSIVVADIFCVFQHLSLTNQLFKFFTSTEEIVNTVHFSRTLCACRHRYRILKLVFR
TLKNLSSNRSFSNP (SEQ ID NO: 418)
>orf02618
   VLLPNVLIKFINRFCWHVIPIKGCDSTFWNNKLIAIFKGNLDRGIHTIFCLHTT (SEQ ID NO: 419)
>orf02628
   VGCSYICHELVTNHDHFLFVIVEFLHSTVNTKCEGLQGPVNVINPKFLNCSLNAFFGVI (SEQ ID NO: 420)
>orf02629
   LLHLWRSIRVVPSNEGIIQIDQNSLDSLRLQAGDCQIIDCFHSKIMYIIFNRHSGSFS (SEQ ID NO: 921)
>orf02631 
LFGSCRQINHTSLQIRQGKEVFLGSSLAQEVIDLIPTLVHLLNDRIVGIADDFQTGKEKL
INRKRVMALQITSHLFNDIGVLGITNGNQATMLDNKGHGKSLIVGXXHSIH  (SEQ ID NO:
422)
>orf02633 
VKGLLLATKLCRTNSHTDNLTRYSNRSICQNDLISHIQLTFKEDEKAIDDIRQKALGSHT
NRYPSNTSSSQQTRNWQT (SEQ ID NO: 423)
>orf02635 
LWGILGTLPNLSGIGLLGDLFVGGLKAVAPILVFALVANALSQHQKGQDSNMKTVVFLY
IL (SEQ ID NO: 424)
>orf02637 
MIGTFAAALVAVLASFIVPIEITLNSANTEIAPPDGIGQVLSNLLLKLVDNPVNALLTAN
YIRILSWAVIFGIAMREASKNSKELLKTIADVTSKIVEWIINLAPFGILGLVFKTISDKG
VGSLANYGILLVLLVTTMLFVAPVVNPLIAFFFMRRNPYPLVWNCLRVSGVTAFFTRSST
TNIPVNMKLCHDLGLNPDTYSVSIPLGSTINMAGVAITINLLTLVTVNTLGIPVDFATAF
VLSVVAAISACGASGGASGIAGGSLLLIPVAGSLFGISNDIAIQIVGVGFVIGVIQDSCE
TALNSSTDVLFTAVAEYAATRKKASLLMSCLLRFYSNLLGNSYVY (SEQ ID NO: 425)
>orf02639
   MIAHAIIILLFKNAGKLCFLVVFFFTVHTFGKNQLLLG (SEQ ID NO: 426)
>orf02645
   VFEVVDKTDEVSSKHCFEVADRTDEVSNHTYGKVKLTWFEEIFEEYHTKKPCSSR (SEQ ID NO: 427)
>orf02651 
MKNRIIDVFEVVNRILVITVENPDFEDLRVNQFVKIGDKKYRVRSGPMIHSTPPQSVLDR
DTFTIDYTDDELLDKEAVFTTH (SEQ ID NO: 428)
>orf02657 
MAYIEYKQRGKKRLWSFSIRERSKSLLHKSGFKTKREAKIEAEKVLHKLNTGSVLSSSMT
LSELYNEWLDLKILPSNRSVVTKKKYLMRKKVIERLFGNKPVSQIKPSEYQKIMNEYGET
 
VSRNFLGRLNSSIQASIQMAIADKVIIEDFTAYVELFSSKSGQKVEEKYLHTESDYQKVL
VYLKNKFDYQKSIVPYVIYFLFKTGMRFSELIALTWDEVDELNEQLKTYRRYNTAIHKFT
PPKNNTSIRLVPITSDMLSLLKTLKILQLKTNKELNIDNENNLIFQHFGYVYDVPDIATV
NKAIKVMLKELQIFPLITTKGARHTYGSYLWHNNIDLGVIAKILGHKDISMLIDVYGHTL
EEKISEEFTAVKSLL (SEQ ID NO: 429)
>orf02658
   MKDTISNKDLISMGYRPSTANAIIHQVRELLVSRGYTFYNRKRLMVVPKSVVKELLGMEL (SEQ ID NO: 430)
>orf02659 
MNKEVLNRAPSNSPITIHQMSNKSYSKFQEEVSLKYGFIGLKLDKLSLTAEVSEEFHSEI
LSGNFTLYDYFGVVEPNLNRNGELASYKGQFFNDEKENWYIEYTPVASIKMNKRPLKIEF
TPSKVSKKNFLIVFHRMFPYMFNIAISTFHLAYDFERDLSALRVNWPKVMYRPIYKGMKL
ETMDFGAPKGNYHLTAYNKLKERMDSGDMAEIEIYQQYDNLWRIEYKFYNEGNIKKELKN
GLPFLAKIPVYIENFKGLEFNNLGVNEKIYLFALRNKPELFAESDKRTVAKYKKLAESIS
EVNLNVFFQNALDFVEIFNQEPCLINFFEFMNSMLQGDIPKLTINQE (SEQ ID NO: 431)
>orf02660
   MHFDKSKFGAVFSAPGLYEVEVINNASFGQNAQYEVIQSRKLGTFAELIEMAKIK (SEQ ID NO: 932)
>orf02661

MFKIKQTASLSEHYFLNTSKLRSIRWFTIGFLSILSLYSCILFKGWFLQMFTLSVGLIVT
LYFERKIKGCFHQIEPLLIVRENLLFMLRRNSFLFTATKDGAILRSAKFNYQLNDVSIVI
QALKSGDEFTREMDDLDVLLSSVLGISLSYKEIYATHVEYVFVYRQPERLHITSLPLEED
NSLKIKIYDDFIIDLRKNFSMLISGASGAGKSFFTYYYLTRFISQTVNGRHAKIYVIDPK
LSDIYKLSKFSGLPVENYGTTNEDAFRIVRHYINEMNRRMEIYNKSDLFDSIGIDLGLPP
LLLVIEEYSSLVASMDSKAKKDFENMVAIVAQKARSLSMGVCIVMQQPRSDSLSTNIREQ
LVNAIFLGAPTRESSQMMFGTTDVPKVKKDKGVGLYSTDREPPKEFHSPMFDRDVFEVIL
PVWEWAAKDYMKDEDEDV (SEQ ID NO: 433)

>orf02662 
MKQKQPIVSRTKQHTFEELIQDQKLERLANLSPDLVGRYGFTASCASSFANLIKEAYGGK
NLNVVYASRMLALWNIACSCYHKADGYSLADALFSDKKICLDYFYYHNNTSDIITLDMIE
DVKKNYLQLVTTATSDNMSVIEFEMEKESDLYYFIKATLGSSFSRMHYSVLVKALAGALA
KNI (SEQ ID NO: 434)
>orf02664
   MGNKGTPPCLHPSNQDTTILIVQQCLRRIEVLAMINFLN (SEQ ID NO: 435)
>orf02665
   VFGSYYGVIASIFFKEFWITEISSNQLIWQVCSSYNWILGNLFKVNPVI (SEQ ID NO: 436)
>orf02666
   VLPSHQVLTFSMSPVHRPPNTIIWIELIKEMVFSTKIDKSIWIIDPTNLS (SEQ ID NO: 437)
>orf02689 
LIVSLKTKSRKAKDMAESIQGWLAQFLVNLFKSITFDCGKEFSKWKDISNHHDSESFFAN
LGCPRQRCLNEHSNRLLRCHDLPKQTDFNEVSQEF (SEQ ID NO: 438)
>orf02690 
VVEIIYFLIIIIASGLGSISGMGGGIIIKPLMDSFGYHSVSDIAFYSSFSVFIMAIISTT
KRFSQSKEIKWRLIFTVSFSSVLGGFLGHLIFQVLLSQLSVRLVSIVQMILLFVMLLVSF
VLTDFKKTYQFDKIGFYMICGLLLGLISSFLGIGGGPLNVSLLMVFFSISIKEATMYSLA
IIFFSQLSHLATIVVVTGLNQYHLAPVPVIFLASICGGVLGTVVSKVLPENWVRYCFKGM
LFFVMGMTLYNLFHIL (SEQ ID NO: 439)
>orf02691 
MMGTNSEEGFLDDFEGPQVAVSVKDFSIADTPVTNQEFAQFVKETGYKTLAERQEWSFVF
ILFVPEAEREGYPHPAGAPWWLQVSNACWKHPYGENSNLVGLEDHPVVHVALEDALAFCN
WSGMSLPTEAQWEYAARGGRQSEYPWGDTLLEGGYYHANTWQGRFPYENTALDGFIGTAP
VYEFLPNDFGLYQMIGNVWEWCRNPRYTLLASFNEDDYELPKYGIQDEEYAIRGGSFLCH
 CSYCNRYRVAARNGCISTSTSSHLGFRCLKE (SEQ ID NO: 440)
>orf02694 
MVQTKQPNIILIVVDQMRADALSLNSKDKLVSTPTLDMMASVGYNFENAYSPVPSCVPAR
AALLTGLDQDKSGRVGYQDEVPWNFTNTLPKVFKDMGYQTECIGKMHVFPSRQRLGFDHV
LLHDGYLHVDRKYDKTYGSQFDYASDYLAFLKGKVGYDVDLIDDGMDCNSWEARPWDKDE
KLHPTNWVVSESISFLQRRDPTVPFFLKMSFEKPHAPLNPPKYYFDMYMERLPQFLDLHI
GNWEVLEKQIPSIYALRGKLKEDDQRRMVAAYFGLITHIDHQISRFLTALKEFRHDKDTI
IWFVSDHGDQLGEHYLFRKGYPYQGSIHIPSFIYDPAGLIAGNRGTIKQLVKIQDIFPSL
VDLAGGTTTDELDGRSVKNLLFGQYEGWRTEFHGEHALGKDSSQYILTDQWKFIWFPVLN
HYQLFDMKKDPHEMNDLYPSEKYQPIVRQMKKKLVDFLRYREEGFVVDEELVPVELSKIT
PTLTKTGDSQS (SEQ ID NO: 441)
>orf02696 
MNTMLDKMQEKLSPIAMKVGNQKFLVALRDSFVGTMPVIMTGSIALLLNAFLVDLPQQFH
LESITKTFQWLVDINNLVFKGSIPIVSLLFIYCLGVNIAKIYKVDTVSAGLVSLASFVIS
IGSTVTKSFPLANVGDVKLDQILQGIDNLAFDGKNLMVTIGNVIPGNHINARGYFTAMMI
GFLASIIFCKVMKKNWVIKLPDSVPPAIAKPFTSIIPGFMAMYIVAILTYVFHLLSNDLL
IDWVYKVLQTPLLGLSQSFFAVILMIFLNKLFWFFGLHGGNVLAPIMEGLFGVAMLANLD
AFQKGEPIPYIWTSGSFGAFVWFGGLGLVLAILIFSRNSHYRKVAKLGLAPVLFNIGEPV
NYGLPVVLNPLLFIPFVLSPVFMATVAYWATSWGLVSPVTQNVTWVMPPILYGFFSTAFD
WRAIILSVVCLIISVLTYFPFVKMADKTELS (SEQ ID NO: 442)
>orf02697 
MDESNLESVMGLIMYGGEAKSNAMEAIQAAKKGDFSKANRRLADANAALLQAHKAQTEML
TREAQGEETSISLLMVHAQDHLMTSLTFVDLAKEVVEVYERFEKN (SEQ ID NO: 443)
>orf02698 
MAKVTIMLACAAGMSTSLLVTKMQKAAEDKGLDAEIFAVPAPEAEEIVATKEVNVLLLGP
QVRYLLGDFQEKLKDRQIPVAVIPMTDYGMMNGSKVLDLAESLLD (SEQ ID NO: 444)
>0ref02699 
MKRLISANPSEILQMNAEELKQSILASEGRVVLSENVVTRETFVGDITNSEIARAFGADM
ILLNCVDVFEPKIYALDSSGDDVIHRLHQLVACPIGVNLEPIDPSAKMLEETQEIVAGRV
ASVETLKRIEELGFDFVCLTGNPGTGVSNREIIKAVQTAKENFSGLIIAGKMHGAGVNEP
VAELSVAEQLLEAGADVILVPAVGTVPAFHDQELREVVDLVHSKGGLVLSAIGTSQETSD
TDTIKEIALRNKICGVDIQHIGDAGYGGLATVDNIYALSKAIRGVRHTVSRLARSVNR
(SEQ ID NO: 445)
>orf02700 
MEKLLQEKLLPVAARLGNNKALVSIRDGITLTIPLLLIGSLLMVIASFPIPGWEKYLGDI
GVADYLWKGVDSSFGLLGLVASFGIAYFMARQYKVDGIPAGIVSLSSFITVTPFITGEAG
AGMPTAFMASKGLFVAMILGLINGYIYQWFINHNIQIKMPDGVPPAVSKSFSAIIPGAVT
IVGWLIVYATLDKLSLPNLHEIAQVALGGPLGLLGNNVIGLLILIFLNSSFWFVGLHGGN
VVNAVMKPLWLANLDANKVAYQTGETLPNIFTSVFMDNFVFIGGGGATIGLVLALGYLAH
KKKASKQLKTLAPITVIPGLFNINEPAMFGVPIVLNILLLVPFILAPMFNLLVAWGAMAS
GLVPLTYTDPGWTMPPVISGLLATGSISGSLLQIVLIVLDVLLYLPFVIAIEKRFKLLED
(SEQ ID NO: 446)
>orf02701 
MTLSKKQLQLRAKILETVYTLGPISRIEIATKTGITPATTSSITNDLIKENILLELGEDE
HDTSVGRKKILLDIQAKRFYYIGCELSEKHFTFALGDNLGNILKEEKEIVTKQLIQEKGN
QLINQTLKQFLNNCSDYEIEAIGIALPGRYLDDYKITTNNPLWQHIDLEMIQSHFDKPLF
FSNNVNCMAIGKRLFSRQQNDTNFAYFHFARGMHCSYIYDGNIYGKGNLMIGEIGHTVVS
SEGEECSCGRKGCLQTFAGEAWLIKKSKILYHQSPYSLLPSLVKNADDIDIQVILTAYQL
GDTGIITLIHQALLYLSQTILNISMMIDSQKIYLHSPLLTNQHIIQKLYSEMNYKPKLLY
NRLPEVIIEPYNDFTAAHSAIALCLYHTILHS (SEQ ID NO: 447)
>orf02719 
MPFKENLICQHRNHHCSVFFISLGLLHNIHIEIDISQTRASFLDLSDYLQAVLMILQKFC
QAIGLAQRLDLLQLHLLHLTRLLL (SEQ ID NO: 448)
>orf02728
   MYLLLLVVKDHIALIDKEMHVWRPNCILRDLTNFFIKRNHIVTNKTNGSPTKREV (SEQ ID NO: 449)
>orf02729
   MVLALMNHFIKEIQGIPINRLTILIENSIFKLNLKNWIIG (SEQ ID NO: 450)
>of02731 
MKIKEQTRKLAAGCSKHCFEVVDRTDEVSSKHCFEVADRTDEVSSKHCFEVADRTDEVSN
HTYGKATLTRFEEIFEEYKGVPR (SEQ ID NO: 451)
>orf02743
   LVEQLTFNQWVTGSSPVRVIYAGLAELADAPDLGSGA (SEQ ID NO: 452)
>orf02745
   VCQRMDARTCKTTIIAVHIQVLTALQQTWIAVQLYQTK (SEQ ID NO: 453)
>orf02746
   LHLGKSILSLPVKGKDLEFLVHLFVINHWIGFPSRTSTFCRCKVLNGME (SEQ ID NO: 454)
>orf02747
   LEQTVIIANNPCELYVIDNHLSFLSDSLLKQVIVHLKRICLDIHHDRGCSHVRNDTT (SEQ ID NO: 455)
>orf02749 
LTDDGVLILVVDAGWRGNSCLQEQGCHHFRAILLCITWHFRSCTDKGHLTFKDIDQLRQF
VQTDTSDEISNLGNTAIVSRSHQTSFFIRIRHHGTKLPNLEPTVVLGHTLLLVNHWPLAI
QLDPNAQDEKDGRGQNQ (SEQ ID NO: 456)
>orf02750
   MLKMRKMGEVRTSKIKAKTQSKQRLKISEPFLLETSW (SEQ ID NO: 457)
>orf02765
   MDYNAVIPEFLVSNIEQSRSFYCGLLGFRIEYQRPEENFLFLLKSVN (SEQ ID NO: 458)
>orf02766 
MLEEGTKDQLAELTYPFGRGVNLSFGIKDVSKLYQKVMEANYPIYRPLTKRKFRVSDPYI
YPHKFAVLDPDGYFLRFSE (SEQ ID NO: 459)
>orf02771 
LACDKHGKGCFTDISALLIGDIHIHHTGCTTLMDTFSFNGQYIVEFSCLKVVDRGLECHP
IKSQRDNHQTTDLVT (SEQ ID NO: 460)
>orf02773
   MGIAIVVERRVHYFGRHHNVTISHFFNFVIFKGRYSVKMKVFHRFLLIFQTTL (SEQ ID NO: 461)
>orf02784 
MIACRHDICKSQKGLEHPFCIVRRLTRDFNQRPVCIVEANIFCLKITPQIIANMIVARTV
KSSKTGITLTTSMCKRDNHKITWFHRRNGFPSFFNNPNRFVSTIFVSNFRFWITVPP (SEQ ID
NO: 462)
>orf02799 
MNMNKDQIAILNGADNLNLTLWMTLKEICKEGCKSFFPVRNTCRMLDIGIPYRLGLSLSN
SSVLNGMDV (SEQ ID NO: 463)
>or02814 
MDGQLHHRAIFDWVHFENFINSWLGFLTINLKTTGQGAEIVLIGHTFNTRDINLVNLITR
VNHLVCKVPIIGQNQDTRCIPVQTTHRVNTFFDIGQEVDNRLATLVICYTGNDTAWFVKQ
IIDLFFVVDRLTFNFDLVA (SEQ ID NO: 464)
>orf02820 
MHKLRIFVNQLYRRFGIILGPFLVLGFQVLTQELELAIFFDLREEVLLQVIPQVCHFCYL
RKEFTTLNQHELTSHDHVLTRHFQTHGLQG (SEQ ID NO: 465)
>or02831
   MLHMNLFFQPFFTNLCKTLATGCCVKTVMENSSIATTIDFKIIE (SEQ ID NO: 466)
>orf02832
   LDNRAKEWIMSTAQiQQAIHLSNQGTQGFIDHLLGNTG (SEQ ID NO: 467)
>orf02843
   LSNFCFKTVTVHRYSVNTNVNQNFSTISCFQTKSVPCWKGN (SEQ ID NO: 468)
>orf02853
   MKIKEQTRKLAAGCSKHCFEVVDETDEVSNHTYGKATLTWFEEIFE (SEQ ID NO: 469)
>orf02858 
MSIVKSHSFSISLGIFNSFWNNIHTSECFYFLCKGKSNRSNSTISVNQMVFFINIQRFYC
FAIEDFCLLRI (SEQ ID NO: 470)
>orf02859
   LNTLLPPDNLCLFTIYLTGFSCICINSYCHNFWEIFNQLFYQLS (SEQ ID NO: 471)
>orf02865 
MIDKVIEQYKSHDNLDIRVELHKKYSKNKLGFNNWIFSNYQITDEVKVLELGCGTGELWK
SNSDSIDKMKQLIVTDFSKDMVKSTKSVIGNRNNVNYEIMDIQKISFENETFDIVIASML
LHHVNDIPKALSEVNRVLKTGGIFYCATFGENGVVNYLASLFKDEVNQDLENRTFTLQNG
KRYLSRYFNSVDTLLYDDELQVTSIDDLVKYIQSFKGISEIGSLEEEIIRKRLESEFNNG
MLIIPKEYGMFIARKES (SEQ ID NO: 472)
>orf02867 
MDLGFDYFGSALTISPHKNSQTINSIGIDVQKIYTPHYLPNDFKKNQGYKRSVEMCEEYD
IYRQCYCGCVYAAQAQNIDLV (SEQ ID NO: 473)
>orf02868
   LTKYADVTIYFANSNIHPKAEYHKRVYVTKKFVSOFNERTGNTVQYLEAPYEPN (SEQ ID NO: 474)
>0ref02876 
MYNKVILIGRLTSTPELHKTNNDKSVARATIAVNRRYKDQNGEREADFVNMVLWGRLAET
LASYATKGSLISVDGELRTRRFEKNGQMNYVTEVLVTGFQLLESRAQRAMRENNAGQDLA
DLVLEEEELPF (SEQ ID NO: 475)
>orf02880 
MQFTRTAHHTKTLFTTKFTWENEIPFWHHSSRKRDNGFQPHTRIGSSCNDLYSLITCDCN
LADVEVVTIWMGYHLNNFTDNKLRFLIINNFFCKTFRLQLLVQTSDLLICQKDLTALCNF
K (SEQ ID NO: 476)
>orf02885 
MSYSVDDVVSNAFKKRMILDSFFAFNCSGTMKVSTWVYDKGEWYYVSSSGAMIANDWVKD
NGK (SEQ ID NO: 477)
>orf02895
   LTSFCFKANMFNNCLRICRIAEGHILKLDLTFEVFISQLHLNRVLDRRMQI (SEQ ID NO: 478)
>orf02897 
LINPLSRDHSSGKNDEECSHEKEAHDNLHSIRHENNHVTKERQTRYRSSVVNHIGPNPVN
RHTQTT (SEQ ID NO: 479)
>orf02900 
LHLRTCFVRQTNKLSPLINRTRLQFHQTILHYTLNQITSNRLGNIEFLLDIFNQDQVLVF
LAIIQKMHNLTLRPTHKFNAATFGFLLHHQVNLMTKTLKD (SEQ ID NO: 480)
>orf02916 
MLEIWKYRPFVSEFWNDFKNNHDKQFVDPISLYLTLKDDDDPRIEEESEALENMILQYLG
EDDAS (SEQ ID NO: 481)
>orf02918
   LTLFFFELLICLLNSEFDLSKFIFVYFDEYFHEDSLKHNLHQFSFSF (SEQ ID NO: 482)
>orf02924
   MAFNQFNRCIGLSIPTAPNVPGTIINRSYLHDATVPNNVREKT (SEQ ID NO: 483)
>orf02940
   LRLQIELTWFEEIFEEYKFEIMKIRQTGGCFVSHLTERDGLRVT (SEQ ID NO: 484)
>orf02944
   MKKNRGIQKLAILVLLGVFMFSNTIPYQQFIQKNRQLEIRVQSQKKSNGLDVGKAD (SEQ ID NO: 485)
>orf02946 
MKKLFILISNLLASLFFVWVLTIWTDTYVSHYYPNVVVRDSSPETTFQHVATRLEKLAEE
TDSFIAIQHQDPNSEGTTVFSYTTFGDGKLPDGLQEKNLEDAQSSSVETNYFVFDGHLDI
HLLREELSQLGLTNMHLTIPSKLSTLMAIFSNGFQLISLLIFILTFVALTLISQISQLRS
SGIRLISGEKRWSIFLRPVGEDLKAIAVGFSLAGVLAILMQKILSLPTQSLMTIGEGLLS
YNLILLSISLFFAQLFAVGIKKIHLMQIIKGQVPVRGIISLILIGQLLAIIIVTLGIGSS
LKYSQAWQQHRIGQEIWSQERQLITLSISREGTSPGFDEQAQRKLRTWYQLMDLAVSEQK
AFLSRHQLIDRTLQNGMASSKNLITSTEWHDYNPNGNVLIVTPQYLERQNIPVDTTIEQK
MNHLNVGEFVLLLPEHLRSEEEHYKSVFEDDLTSRISSQDERQQMTATVGYLESGQDRFV
YNTTPISYQQFLKDPIIIVITPQSTGPQSILFWIDAVQNYVLFNQLSDAQELIQRQGIEN
WVSEMQTGYHNYITLLDNIQRERWVMLAGAVLGIATSILLFNTMNRLYFEEFRRAIFIKR
IAGLRFLEIHRTYLFAQLGVFLLGFVASVFLQVEIGVAFLVLLLFTGLSLLQLHVQMQKE
NKMSILVLKGG (SEQ ID NO: 486)
>orf02955 
VLKWCILRINHHISRKVDNFLEGTRAHIKGQAHTAWNPLEVPDVRYRSFQFDMSHTLTTN
FRTRYFNPTAVTNNSSVTNTFVLTTSTFPVFCRTKDHFIKESFTFWFQGTIIDCFRFFDF
SIRP (SEQ ID NO: 487)
>orf02962
   LVDPLVTSHDNLLSKGSIFIQTRVSLSYSIFIFFISCQPNNFRS (SEQ ID NO: 488)
>orf02966
   MPWKELCHKLAPKVFKVIRIYSRENKKSPSNWAFCSFET (SEQ ID NO: 489)
>orf02970
   VSVLFFCSYFSLSLEKGNFSSLISCKFMNQFLPFCWRQDSPWILTLAQDSITYH (SEQ ID NO: 490)
>orf02978
   VTDENTRKVRSLVAFFSIVIGYILSSFFISLYHLWQEALRGLL (SEQ ID NO: 491)
>orf02979 
MRLLFFFANRVIRSKENSSTCPSRSYNLLINTSNVSHITIAVNGTCTGNNTTITKIWVSY
LSIDS (SEQ ID NO: 492)
>orf02983
   VDSLFLSLGEEGNQEINLQESFSSTDCNPTLISPETTVAQGLCQDIIYRPFT (SEQ ID NO: 993)
>orf02985 
VNPKSLGSFFLQDSKGFKELVLGHAKLSLPRIVHNVCPQFKNASRIITTRDDFWNACYSL
QMFNIFKGIQVNGRTQFTCIGVFLVWRVVGREHNLRTQKVQFMAHQKLYITRAVHTTTFF
LENFQNSWSWSSLNCKIFLKALVPRKSLVDGSCLLTNPLLIIQVKGSRELGNNRF (SEQ ID NO:
494)
>orf02989
   MTTIRSLLLNFISISYSIFIQKIKKQTRKLVAGGSKHCF (SEQ ID NO: 495)
>orf03003
   MDALVLQKNQETIQQIAVKIRFLDGHDYYSLIDIDNRRTNQTVFPFVNFEDIAF (SEQ ID NO: 496)
>orf03004
   MAFFTEIPTRACLINLAITLHIVETCQGFNDLSLHLRVLAL (SEQ ID NO: 497)
>orf03008
   MLLPLPFNTSKIKQIAMHSDLNQKEMIGHIFHDEDIF (SEQ ID NO: 498)
>orf03013 
MKQTVKKLALVASIAATLGGSVAVASAAVQYPEGGVWTYGSGNGGAYSNYYHPSKYHSST
VVSRKTGSSDKGYAGAGGTSRAWIRTSWGEKVAFYYNV (SEQ ID NO: 499)
>orf03018
   MLNRRFIKTNNIHLCHTHLSSQGNFFCLTTCKFFYIQVCMCIKNHLF (SEQ ID NO: 500)
>orf03029 
MNTIERTRRLVKGCATHCFEVVDRTDEVSSKHCFEVADETDEVSSKHCFEVADETDEVSS
KHVFEVVDETDEVSSKHVFEVVDETDEVSSKHVFEVVDETDEVSNHTYGKATLTWFAEIF
EEY (SEQ ID NO: 501)
>orf03033 
MLERLKSIHYMFWASLIFMLFPILPVVIGELPAWHLLVDILFVVTYLGVLITKNQRLSWL
FWGLMLVYYVAGNTAFVAGNYIWFFFFLSNLLIYHFGVRSLKSLHVWTFLLAQVLVVGRLL
IFQRIEVEFLVYMLVILTFVDLMTLGSVRIRLVEDLKEAQVEQNTQINLLLAENERNRIG
QDLHDSLGHTFAMLSVKTDLALQLFQMQAYPQVEKELREIQQISKESMCEVRTIVENLKS
RTLTSELETVKKMLEIAGIEVETDNQLDTASLTQELDSMASMILLELVTNIIKHAKASKA
YLKLERTEKELILTVSDDGCGFAFLKGDELHTVRDRVFPFSGEVSVISQKHPTEVQVRLP
YKERN (SEQ ID NO: 502)
>orf03036 
MTVVKVEKLSKKIKDKEILRNISFEINDGECVALIGPNGAGKTTLLDCLLGDKLVTSGQV
SIQGLPVTSSKLDYTRAYLPQENVIVQKLKVKELIAFFQRIYPNPLSNQEIDQLLQFVKQ
QKEQLAEKLSGGQKRLFSFVLTLIGRPKIVFLDEPTASMDTSTRQRFWEIVQELKAQGVT
ILYSSHYIEEVEHTADRILLLNKGELIRDTTPLAMRSEEIEKHFILPIAYKEVVEQSNLV
ENWTLKQDSLQVVTREADAFWELLAQAGCRMQEIEVNNRSLLNTIFEETQKGDN (SEQ ID NO:
503)
>orf03039
   MGEEEMRNKMIIAMSLVVTGVMTYLMFSGLDEDFCHFPMKVFAGFGIMS (SEQ ID NO: 504)
>orf03040
   LVEQLTFNQWVTGSSPVRVIYAGLAELADAPDLGSGA (SEQ ID NO: 505)
>orf03063
   MPSLRSLKKTDGSCDELHHFDLPVNFFKNTVLGKQTCSGVIREVCQDCFNMLMR (SEQ ID NO: 506)
>orf03091
   LHTSFRSSVGHSHTWHQDIVRPILFSRFNDSIVILWQNCPTFNQGIYCYLDCFFPIVSL (SEQ ID NO: 507)
>orf03096 
MWSQTLGLIHPLTSLLELPFWMACLKGFGQFCKSLSGLLSFVAECQHLLSLCSRFIRITV
LQTSKV (SEQ ID NO: 508)
>orf03112 
MLEQARLKVEQQAIKNIQFLEQDLPKNPLEKEFDCLAVSRVLHHMPDLDAALSLFHQHLK
EDGKLIIADFTKTEANHHGFDLAELENKLIEHGFSSVHSQILYSAEDLFQGNHSEFFLIV
AQKSLA (SEQ ID NO: 509)
>orf03113
   MKHDFNHKAETFDFPKNIFLANLVCQAAEICQIDLLSDKEILDFGGGTGLLALPLTPSQAG (SEQ ID NO: 510)
>orf03117 
MVDLQSFFTRKYLNLNSVDAYLILPRLQGHLSYPQDFFLLQDFCFLLPIFLNLSQKEGRN
AGKDS (SEQ ID NO: 511)
>orf03133 
MRIRNSPFDHILQTIFEFEDRTCQVTCRFEACSSICNDNWEFSQHIISVFQSPSCHTVCD
KSDVFCSFLFDKNFASLWIYVVTITDQLCXXIPFIN (SEQ ID NO: 512)
>orf03142
   MHKTCLNIWKFLFYQIESLIHQMAADKSPCRIGNRGR (SEQ ID NO: 513)
>orf03144 
LNHRFNRQTTKVGRSTIWANGTVNRLIIFVIRSTCIVLINGHSFRCQTSSSTSLPNTKDK
VRLITIHLFFQYLSRFVKNCRHL (SEQ ID NO: 514)
>or03147
   MTLHQTFRFQNFEMPCQSSLINFQTLLHRHLVTRRMLQQKQ (SEQ ID NO: 515)
>orf03151 
MKKRMLLASTVALSFAPVLATQAEEVLWTARSVEQIQNDLTKTDNKTSYTVQYGDTLSTI
AEALGVDVTVLANLNKITNMDLIFPETVLTTTVNEAEEVTEVEIQTPQADSSEEVTTATA
DLTTNQVTVDDQTVQVADLSQPIAEAPKEVASSSEVTKTVIASEEVAPSTGTSVPEEQTA
ETTRPVEEATPQETTPAEKQETQASPQAALAVEATTTSSEAKEVASSNGATAAVSTYQSE
ETKVISTTYEAPAAPDYAGLAVAKSENAGLQPQTAAFKEEIANLFGITSFSGYRPGDSGD
HGKGLAIDFMVPERSELGDKIAEYAIQNMASRGISYIIWKQRFYAPFDSKYGPANTWNPM
PDRGSVTENHYDHVHVSMNG (SEQ ID NO: 516)
>orf03156
   MSNQITVHHSHEHLQKVFTHSWQCNIKNVFIFLKQSLLLMKRNSVGFPTEFTPSILKS (SEQ ID NO: 517)
>orf03171 
MAELNSVITTVTGIENRLGAVILAEIRNIHAFDNPAQLQAFAGLDSSIYQSGQIDLAGRM
VKRGSPHLR (SEQ ID NO: 518)
>orf03178 
MEXXXDIRKGRHAVVEKVMGAQTYIPNTIQMAEDTSIQLITGPNMSGKSTYMRQLAMTAV
MAQLGSYVPAESAHLPIFDAIFTRIGAADDLVSGQSTFMVEMMEANNAISHATKNSLILF
DELGRGTATYDGMALAQSIIEYIHEHIGAKTLFATHYHELTSLESSLQHLVNVHVATLEQ
DGQVTFLHKIEPGPADKSYGIHVAKIAGLPADLLARADKILTQLENQGTESPPPMRQTSA
VTEQISLFDRAEEHPILAELAKLDVYNMTPMQVMNVLVELKQKL (SEQ ID NO: 519)
>orf03191 
LTNLSSVDSEELFQFYRERGNAENFIKERKAGFFGDKTDSSTMIKNEIRMMMGCLAYNLY
LFLKQLAGDEVKALTIKRFRRLFLHIAGKYVSTARRHILKFSSLYAYSKQFQALFDTICQ
INLILPVPYRARGQGKTCLTE (SEQ ID NO: 520)
>orf03203 
LFDDRQAINICPPTNGSLRLTSLQVDQNPCPPSTNLNKILARSQFLNHIQQISLSLELLQ
ANLWNLV (SEQ ID NO: 521)
>orf03207
   LSVHFCSSHRCLLVRYNDTYSTKKGLKFETFLSVFRYDFLGM (SEQ ID NO: 522)
>orf03237 
MDFFNYLLWMICHNHGLHTLLLSKDCVCHTARDKDGNHRIKSVFPTKGQTCYQHDSSIYQ
ERNTTDILTRFLANSQADDIRPTTGDIVSKSKTNPQTHNNTPKKGIDNGILRQGCHRDKL
DKEGTHRYRDKGKDGELMANLIPS (SEQ ID NO: 523)
>orf03245 
MKIKEQTRKLAAGCSKHCFEVVDETDEVSSKHGFEVVDETDEVSSKHGFEVVDETDEVSN
HTYGKATLTWFEEIFEEH (SEQ ID NO: 524)
>orf03253 
MKIKEQTRKLAAGCSKHCFEVVDRTDEVSSKHGFEVVDETDEVSNHTYGKVKLTWFEEIL
EEY (SEQ ID NO: 525)
>orf03254 
LFFKDEKQALYTKPKTKSSSFRASKVSNQTIVATTRTDCQVIALNLCDKLENGVVVVVQT
THHIGIDDVIYSKIFQHLTHSIKMSLAFFIKKVQDRRRILYCHLVFFFLRVQDTKRIFLQ
ATLAILRQGLLERCQIVNQGLAVGCTALRISKSVEVQFDTLNTDFLQKMGCHNDCFHIGS
WIARTKTLNTNLVELAQAPCLWTLITEHRSHVVELAWLLHFWGEEFIFHIGTDNGRSSFW
TEGNMTVTLVIKIVHFLGYDIRCISDRATDNLVMLKNRRAHFCIVIALENLTGKALNVLP
LSRFSR (SEQ ID NO: 526)
>orf03260 
MEQIGKVFRQLRESRNISLRQATGGQFSPSMLSRFETGQSELSVEKFLFALENISASVEE
ILFLARGFQYDTDSELRKEITDVLEPKNIAPLEDLYRREYQKHAHSHNKQKHILNAIMIK
SYMKSMDERVELTAEEGKVLHDYLFSTEIWGIYELNLFSVSSAFLSVSLFTRYVREMVRK
SDFLMEMSGNRNFFHTILLNGFLASIECEEFTNAYYFKRVIEEHFYKENETYFRIVYLWA
EGLLDSKQGRVKEGQKKMEDAVRIFEMLGCNKSAEYYRNTTEC (SEQ ID NO: 527)
>or03261
   LIPYFLHFIIFFRKFIKNLPNCQNYEKIEDIYHVEGLL (SEQ ID NO: 528)
>orf03263
   MKIKGQTRKLAAGCSKHCFEVMORTDEVSSKHCFEVVORTDEVSNHTYGKATLT (SEQ ID NO: 529)
>orf03266 
VVPFSDTFKDRNQVDIFTIKISRCNSSTIGENSWDIHISNSNHRSRHVLVTATDSDEGIH
VVTTHSRLDGVRDDVTRC (SEQ ID NO: 530)
>orf03275
   MKKFSYPTRQTGEGVKYQSQMVRQWFLIRIFRLFSVA (SEQ ID NO: 531)
>orf03297
   VVLDHQNQLFEARFLEHTNPLTRIQLTRVKALRILLSSPPFLVIKGIRTEVDKSC (SEQ ID NO: 532)
>orf03305
   VQKLKKAIYKAHLKDSDDFRPETSTPNLFESCLKLCPCFLSS (SEQ ID NO: 533)
>orf03306 
MGALGYYEGFVPYVSNQYKNQAEEEDKPLSDKYIFEKILGKTYAAFKKDQINERVEKLGK
LKPITINYNGKSEVIDSKEKLQELMNKAVKDEVAQI (SEQ ID NO: 534)
 >orf03307 
MMGDGMKEFQFERKQRFSLRKYAIGACSVLLGTSLFFAGMGAQPVQDTETSSALISSHYL
DEQDLSEKLKSELQWFELENKLLNLWEH (SEQ ID NO: 535)
>orf03308
   MKIKEQTRKLATGCSKHRFEVVDKTOEVSSKHCFEVADRTDEVSNIYTARRR (SEQ ID NO: 536)
>orf03312 
VNITKTSIIKAHTTKEDGIDHTFTRFNIMSIFYSTRKIFLDKLNSTNRQFLGYIISTRCY
QSFNSVSQSIHTSSSSQAFRFGKHEFRVINRDKSKAILVNHYHLNLAFFISNHIVNSDFC
RSSCRCIDSHNWQAFFSRLMKPFIILWFSTICSHDRNTTSCILWRTPAKTDDKVTAMFLQ
SSYPICDIFTSRVWLYIAKDDIFDSFCIQWF (SEQ ID NO: 537)
>orf03318 MSQDEKLIREQICDVCHKMWQLGWVAANDGNVSVRLDEDTILATPTGISKSFITPEKLVK 
LNLKGEILEAEGDYCPSSEIKMHIRCYEEREDVRSVVHAHPPIATGFALAHIPLDTYSLI
ESAIVVGAIPITPFGVPSTMEVPEAITPYLPDHDVMLLENHGALTVGSDVITAYYRMETL
ELVAKTTFHGRMLLSTKGIEEQEIARPTLERLFSMRENYKVTGRHPGYRKYNGDGSMKET
EK (SEQ ID NO: 538)
>orf03320 
MESKKIAKQILIATAVLTSFLGSNLVYADVVQSNSNNRASTETARVTGNNLEKLITKDKE
IDKEMTYLSDMDWSSATHGDIDKTKTVQKDAPFTTGNKGEHTKISLLTSDDKVKYFDKGI
GTVADSPSVISYDISGQGFEKFETYIGIDQSANSSRSDHAVVDRIEIEIDGKVVYSSSVT
NPEGFRYNTQAQFISVTIPQNAKKISLKSFAGEHTWGDEVVFADAKLIKTVSTQTITPDL
LNKGINGGVYLSDLEWVDATHGDDDKSKTVQKDKPFTPGNNGSNNKIKLLIDGKEVEFNK
GLGTVASNPSSIKYDVSGANVTRFISYVGIDRSANHLNSDYADIQKFEVVADGKVIYSSD
SKYPKGIKYDTSAFLVDVEIPKDTQTIELKSYSGKHTWADELVLGGALFMANGKFKNPND
WSEVDKRREINNEHPLLMMPLYANGEEFNQGKYTFWGGDTLTGKWENIPDDLKPYTVIQL
HPDDLPKRDGAARDFYEHMLEEAAKYVNPKTGKNEPIPVILTVYTAGNMPYYTSAHWLST
SWIDKMYQKYPNLHGIFSTENYWIWANDIENKAADYLKVSAKNGGYFIWAEQNNGSAIEK
AFGKNGKIAFQKSVDKYWKNLIFMFKNTPAAEGNDSTTESYMKGLWLSNHTYQWGGLMDT
WKWYETGKWKLFASGNIGKSQGDRQWLTEPESMLGEEALGVYLNGGVVYNFEHPAYTYGV
NNKESLLFSEVIKEFFRYVIAHPAPSKEKVLEDTKVFIHGDYSNKGNGKFFVNVNTDREQ
TPLYMTGRYNVIPAIPGVLKTDKLKESVSSSRIQIKEITSPEFSSTQARKEYLNKLYPMN
YEGDIFAQKLDNRWFVYNYKVNENVKQTGKLKFNSLEMNVEFEPHTYGIFERISNGLKVN
LNNFRTNKDSLWSNAQDANQAKKLPQLTKKGAIKWIEEHYIKDTQFGEKRVTKIVLRGID
KLPTIHSLSGTNNSYDQPSLNFDQKNHMVTITINSNGNLEFELHF (SEQ ID NO: 539)
>orf03322 
MTIYINKDETVFHLAMKDSSYIFRILENGELQHLHFGKRIHVKENYNQLMAYKKRGFEVS
FSEEFEDIQQSMIQNEYSSYGKGDFRHPAFQVQGMNGSRITTLKYQGFELEKGKNRLNSL
PSTFDDIGQCAETLTIILTDSILDLTVRLNYTIFPEYNVLVRNTEFLNNSNNKLTLLKAM
SLQLDLPDSQYDFIQFSGAWLRERQLYRTSLRPGIQAIDSLRYSSSPQQNPFFMLSRRET
TEHSGEVYGFNFIYSGNFQNMIEVDHFDTARVTVGINPVEFRFLLNPAESFVTPEAIVIY
SDQGMNQMSQQLSDFYRHHLVNPNFSQASRPIILNSWETFYFDLGTEKILDLAKAAKDLG
IELFVLDDGWFGHRKDDKSSLGDWVTDRSRLPEGIGFLADEIHKIGLQFGLWFEPEMISI
DSDLYKNHADWTIHLLDREKSVGRNQYVLDLTRQEVVDYLFDSISKIIIKTNLDYIKWDM
NRHITDIYSIELDSEQQMEFGHRYILGLYQLLDRLITKFPSVLFESCSSGGGRFDLGLMY
YAPQAWTSDDTDPIERLKIQHGTSYGYSPSMMTAHVSISPNEQSGRQTSLDTRTNVAYFS
SFGYELDVTRLSVEEKEQVREQIQFYKKYRSLFQYGDFYRINSPFSCDSASWQVVSKDKC
QSILLYAQLNSKLNPGYTRVYFSGLDKDKCYSVSRFDEFFYGDELMNAGIKVSLSNLALC
VPEYLTKLFVIEEVVCKY (SEQ ID NO: 540)
>orf03323 
MKIENKNVRRNFFWGEGRFYTTDIVNKRAGVMIKNVSKEEFTITLENGIKLSSTHFSAIV
REEGDTRIQVSFVCPSIRLRLIFESRDDVLSKQLVLESSTEVIKSVEVESFEFETEDNIF
YPKRQDCIKEMANFSGYYVELGQPVYANSLFLGMEFPMSENKVDGRHYVSRYYLGTVVNQ
EKSLWSCIIGGACSYKKEEIQEAFFEYVEGIAQPSYFRKQYNSWYDHMTDITEEGILKSF
SEIRDGFENHGVHLDAYVVDDGWTNYQSVWEFNHKFPNGLRNIKYLVNGFGSSLGLWIGP
RGGYNGTEIIMSDWLEAHPELNIGSKNLISNDVNVADFNYLNQMKKKMLEYQKEFDISYW
KIDGWLLQPDKPDKSGPHGMYTMTAVYEFLIQLLIDLRKERGGKDCWLNLTSYVNPSPWF
LQWVNSLWIQISQDVGFTENAGNDINRMITYRDSQYQEFLEKREIQLPMWSLYNHEPIYA
VSANTWYMDHQMFASIPDFEAYLLFISTRGNAFWEFHYSFDMFDEERWKANARAVKWIEE
NYQTLKYSKKIGGSPEKFEIYGYKCHNQKTSTEILSLRNPAQIKQKIKIENLSIENFTRV
IGDFTIQEDEIELAPYSIVILKK (SEQ ID NO: 541)
>orf03324 
MKHTLETINSRIQWFREARFGMFIHWGLYSIPGKGEWIRSHQKLSIEDYEPYFRAFDPKE
YNPREWAKQAKAAGMKYMVLTAKHHDGFCLFDSKFTDYKATNTPAGRDLVKEFVDAVRAE
GLKVGLYFSLIDWHHPDFPKYADLNHPMRGNEVYRDEKINFDSYLEYLHNQVKEIVTGYG
QIDILWFDYSYEDMVGEKWGASKLIDMVRHYQPNVIVDNRLETSGEGFGSIVTDEITSYA
GDFVSPEQIVPHEGIRNFKGEPVPWELCLTMNNNWAYNPTDYLYKSSQTLIRKLVECVSK
NGNMILNVGPDALGRINDSSKKILDNFHRWMSRNGEAIYGCSGDENLPKPDWGYYTRNGN
TVYAHVFEQPIGPLALLGISKENVKRMSFLHDGSEVKISESWTTNAYKGICFAQFGEVPH
FTYPLPDLIDSVIKIELRE (SEQ ID NO: 54[?])
>orf03325 
MNTHINGISKKGKVLIYGYMLLTILISIFPIAWIFLSSLKADPMKNPGISLPTDFTLEGY
INVFTKLHVFTYFWNSFKVVSISVIISIVMISMSSYVIARMEFRGKKLVTSMLYSTLFIP
ATAMTFPVYRLVNELGIYNTPVALILVYSCSGIAMSFFIIKNYFEIIPKELEEAAEIDGA
TYAQTFWKVMLPIARPGILTAAVLAFINNWNEYYWASMLVIDKNELTVPALLGQFTTSFN
TNYNGLFSAIVVIVLPPIILFAFTSKYFIEALGGGAVKG (SEQ ID NO: 543)
>orf03326 
MAQKIMSLQNRKNQKRRFIFLFLLPTLICFFLFYFYSVVTIFLTSFAKWDYTNLNTPEFL
GFDKLFENYRYVFKEYPFFTEALINSVRWAVIGVIIQVPLAVSVAITLSKKLKGWKISRN
LYIVPSIISSAAMGLIFLQIYNPNYGVVNQIIHLFNPSFKDSVLLTPGLNIVAMTGAYIF
FAGASTIMILGQIFAIPEEVQEAAILDNITGWRKEWYITIPMIKGTIKTVSIMAATSGFL
LYNEVFFLTNGAAGTKSISFVIRELAVASSRTQYARANTIGVIQILGGMLIIVCINILFR
ERKRLKGEK (SEQ ID NO: 544)
>orf03327 
MNKKSLLKCAVIGLVATFGLAACGTSKDASGGSSSGKEVLEFYHGYHHSEDEWPVAKTMR
DLYDKFAEEHKDSGVEFKPTPVNGDLKDIMNNKVASGEFPDVIDLAGNAVSLAAIEQKLV
LDLKPYIDSNKLEKNVGLNYKQNQKDGKIYTVHEQLFTMGLWYNKDIFAKAGAKTPDQWN
TWDDFTQAMASIRKQDGVYAFGAGEPSIRLFNTVLGTTENGRKLLDKPLTKEGIESKEFA
DALKMVMKEIQANGSKNAGGDANAYSKDFQEGKSAVFFNGVWASGEMSKNPSLAPGIYPA
GVAISSSGGGITISSKMSEAKQKLALEFLKYMTSDDVQKVIFEKVGANPSNENVNVKELS
EKSSEATTKILGQAITQVKNAKAVVPTVSDVWGGDVHTAIINALTESAAENVDVDQKVKS
TQDVLKSLIG (SEQ ID NO: 545)
>orf03337
   MNIAIRIILNFFRVMGNHQNSLAMMMGAVVHEFVKFIFTSCIHPRCRLV (SEQ ID NO: 546)
>orf03338
   MLLIMSIQTTEPAFSRIATRLOKFIORTWKTSIKTGNLLRKIGYSQFLTLRICL (SEQ ID NO: 547)
>orf03339 
LQNSKTGLDERRLSRSIFPSQGNKFPTINTIIDMFKNRLLIIIEGQILYRNISHYLISPT
KAVKNR (SEQ ID NO: 548)
>orf03344
   LTSLIPRLMFQKTSQLVSIKILLEGCWIIAIFTEPLR (SEQ ID NO: 549)
>orf03352 
MSNSFVKLLVSQLFANLADIFFRVTIIANIYIISKSVIATSLVPILIGISSFVASLLVPL
VTKRLALNRVLSLSQFGKTILLAILVGMFTVMQSVAPLVTYLFVVAISILDGFAAPVSYA
IVPRYATDLGKANSALSMTGEAAQLIGWGLGGLLFATIGLLPTTFIILVLYIISSFLMLF
LPNAEVEVLESETNLEILLKGWKLVARNPRLRLFVSANLLEIFSNTIWVSSIILVFVTEL
LNKTESYWGYSNTAYSIGIIISGLIAFRLSEKFLAAKWESILFPLVAMAIVTLTILYFPN
AQMFLLFSALVGMLSQLKEVPESVFLQETVEENHLVNVYSVLEVISTLAFSVFVLLMSYI
TDFGYQPFV (SEQ ID NO: 550)
>orf03353 
MSKLLDKILSRENMLEAYNQVKSNKGSAGIDGMTIEEMDNYLRQNWRLTKELIKQRKYKP
QPVLRVEIPKPDGGIRQLGIPTVMDRMIQQAIVQVISPICEPHFSDTSYGFRPNRSCEKA
IMKFLEYLNDGYEWIVDIDLEKFFDTVPQDRLMSLVHNIIEDGDTESLIRKYLHSGIIIN
GQRHKTLVGTPQGGNLSPLLSNVMLNELDKELEKRGLRFVRYADDCVITVGSEAAAKRVM
YSASRFIEKRLGLKVNMTKAKITRPGELKYLGFGFWKSSDGWKSRPHQDSVRRFKLKLKK
LTQRKWSIDLTRRIEQLNLSIRGWINYFSLGNMKRIVASIDERLRTRLRVIIWKQWKKKS
RRLWGLLKLGVPKWIADKVSGWGDHYQLVAQKSVLKRAISKPVLEKRGLVSCLDYYLERH
ALKVS (SEQ ID NO: 551)
>orf03357 
MFLRCATFKLADSRLNIFTCFFFGEIRFNSRNQVVKAFITDGTVISTIIVRGTVPCNQWT
KTCPAAFDIINGDVGFWKAVVDNAK (SEQ ID NO: 552)
>orf03358
   MLQITCVVCISCTKVSLVFTWENKDHTTVTQTCVKVNWL (SEQ ID NO: 553)
>orf03359 
LRSLIRQITYFITPRTCCINNQTGLDFKYLVCQEITSYNTCNLATFVKEEAFCLHVVGNE
GTVLVGTFDVFNHETRIVVTEVKIHSTSYQAFLLQVWLAFQDLILAQNLVRSWCVAHTC
(SEQ ID NO: 554)
>orf03360
   LHFNQTSLKTASCRLQGYTSSCDSSTDNQEVQGAFLHFFN (SEQ ID NO: 555)
>orf03375
   LIHSKMFVKSIRLRKKRVNDKKILILGILYYKFLKSID (SEQ ID NO: 556)
>orf03379 
MFASKSERKVHYSIRKFSIGVASVAVASLVMGSVVHATENEGSTQAATFSNMANKSQTEQ
GEINIERDKAKTAVSEYKEKKVSEIYTKLERDRHKDTVDLVNKLQEIKNEYLNKIVESTS
KIEIQGLITTSRSKLDEAVSKYKKAPSSSSSSGSSTKPETPQPETSKPEVKPEPETPKPE
VKPEPETPKPEVKPEPETPKPEVKPEPETPKPEVKPEPETPKPEVKPEPETPKPEVKPEP
ETPKPEVKPEPETPKPEVKPEPETPKPEVKPEPETPKPEVKPEPETPKPEVKPEPETPKP
EVKPEPETPKPEVKPEPETPKPEVKPEPETPKPEVKPDNSKPQADDKKPSTPNNLSKDKQ
SSNQASTNENKKQGPATNKPKKSLPSTGSISNLALEIAGLLTLAGATILAKKRMK (SEQ ID NO:
557)
>orf03384
   MDREILKFFQDLLSILSHIIDMITLFCQKCCNSFSNHFLVICN (SEQ ID NO: 558)
>orf03386
   MFITLRRICLRACVVEKEQSYLKFLFFQKRPVSFLHVKSVLAGI (SEQ ID NO: 559)
>0ref03387
   MVKTTDRLEAIGFSFILFENLFKPCQLYLQPQNSVLSNLQLAA (SEQ ID NO: 560)
>orf03393
   MTRKLNPSYTNVASATTLTFNQVASTFRKACLDHVVNLTRNNLKGICQLTPLQLHDTRLI (SEQ ID NO: 561)
>orf03421 
VKAPIPKAPLAHSFGSASIIAHTIHQKFNLKVPNYRQEEDWTKMGLPITRKEISNWHIKT
SQYYLEPLYNLLRERLLTQPLLHADETSYRVLESDSQLTYYWTFLSGKAEKQGITLYHHV
LIDLFISYFNPL (SEQ ID NO: 562)
>orf03430
   LVSVFYSLLQVDNVDSVTFSKDVLSHLRIPATSLVTKVYTSLKKLFH (SEQ ID NO: 563)
>orf03440
   LIVWILIKNHTDLTTYIPIJIFLSQTLAIDIYNLSGFCFQ (SEQ ID NO: 564)
>orf03441
   MPYNRKPFSTFHVKRNILHIVVVLIFFIAKRKIFYINY (SEQ ID NO: 565)
>orf03445 
MFKKMSNSSRILFYISVNFCDKRIYRTKLYSDTPVNLFKFLFRQKSNCQSVGQTSSINHF
FYSWIVFFFKNNLCHSIPSIK (SEQ ID NO: 566)
>orf03458 
LADGSGKLAEGGTKLTSGLEDLQTGLASLGQGLGNASDQLKSVSTESKNAEILSNPLNLS
KTDNDQVPVNGIAIAPYMISVALFFAAISTNMIFAKLPSGRHPESRWAWLKS (SEQ ID NO:
567)
>orf03464
   MKNTVKLEQFVALKEKDLQKIKGGEMRLSKFFRDFILQRKK (SEQ ID NO: 568)
>orf03483
   LVEQLTFIJQWVTGSSPVFVIYAGLAELADAPDLGSGA (SEQ ID NO: 569)
>orf03487 
LWVNIDHIRHISCENACLQYFTSIWYIDDFDLDLRVFLLKITSDFFQGFGHFFFLVEILD
GHCVIRRFTIVGASAEPKQS (SEQ ID NO: 570)
>orf03496
   MKIKEQTRKLAAGCSKHCFEVVDETDEVSNHTYGKATLTWFEEIFE (SEQ ID NO: 571)
>orf03504 
LIDVLFINSFIGRICFYCYRRIHATCLFLQLFSIVILNVAHTLKHSIFIVITFISRCRNF
IIVRILLENQFSRNQGIDNRVGQSRY (SEQ ID NO: 572)
>orf03505
   MINVNQVSIEVICNTFKNWNFTSSIELTTFSKFSQSPTMT (SEQ ID NO: 573)
>orf03516
   MGFSMKLIHDLDMHTTHSTAKMLYNVKAIKNDFSIRE (SEQ ID NO: 574)
>orf03521 
MYQDLLRKIAEEKPNYNQEEIQWLLDHLGDPSPEIRDDLVFTSFARGIQEELFTQEQFHF
IAEGVSSDGGLDKEIDKIGLPTLERSFRALIYATLLSDDANQQSVFYQELNAGFRNDLSN
QGLHYLSKEKDTTGFSSQYGWVHSFAHGADLLTEVVCHPEFPKNRVHEVFDILGQLFKRI
SIRFTDDEDWRLARVIYEPILQGKLEQEQVASWIKTVDFPIEAREDFYKFSNFRSCLGKS
XXIHSLIN (SEQ ID NO: 575)
>orf03524
   MGFKVSHFKIPSSHLSINVLRTIENFTEIGQGLLHISP (SEQ ID NO: 576)
>orf03525 
VGFFDFGLTNSCRQVRQFTQTVQDFLVCYHQGIVKEGQGYAGICFKFHPSLGNIGKFVIA
IVRRLRHKSIVANMAHLNVDLFQFRKGLLEILKSVKIALVITAKLVDVFTSFLDCTQEIL
TVLV (SEQ ID NO: 577)
>orf03537 
LKKAQWGFSNQGPDGLFLVRPTSNRDEIPPRDLAHPQNQLFLSFSQVEKLAWHAPSPLSE
FALNISLPLSSPPYLWPFIQSPVLYCASHFSIHFNSFSSHLRLVITISPHLLSSTSLLLH
TLCAQHTIHSTDLHHLRTPPPSGLFPLALYTRLAAPTLTYHTLSNIQSLKQQLVXXFIH
(SEQ ID NO: 578)
>orf03548
   MANDNKSHYLIYRVLGISFEEGENIDLYQDIKGRFLYKYAGSFLEEAAVLSFNEKFGTENT (SEQ ID NO: 579)
>orf03553
   MVNAMHFSFSILIEGNSRKVGICLLNRTHTRFKLSQAIYL (SEQ ID NO: 580)
>orf03562 
MSQQLSDFYRHHLVNPNFSQASRPIILNSWETFYFDLGTEKILDLAKAAKDLGIELFVLD
DGWFGHRKDDKSSLGDWVTDRSRLPEGIGFLADEIHKIGLQFGLWFEPEMISIDSDLYKN
HADWTIHLLDREKSVGRNQYVLDLTRQEVVDYLFDSISKIIIKTNLDYIKWDMNRHITDI
YSIELDSEQQMEFGHRYILGLYQLLDRLITKFPSY (SEQ ID NO: 581)
>orf03572 
VKEEKKAIVLGADNAYMDKVETTLKSLCVHHYNLKFYVFNDDLPREWFQLMEKRLETLNS
EIVNV (SEQ ID NO: 582)
>orf03574 
MKIKEQTRKLAAGCSKHCFEVVDRTDEVSSKHRFEVVDRTDEVSSKHRFEVADRTDEVSS
KHRFEVADRTDEVSSKHRFEVADRTDEVSSKHRFEVADRTDEVSSKHRFEVADRTDEVSN
IYTARRR (SEQ ID NO: 583)
>orf03576
   MEXXXFKAGAKFFWAKLGLESLEAKEILRDGGWDDIVKNRCIKREQPRLIEEA (SEQ ID NO: 584)
>orf03588 
MILLQNKSFCYSISQKETSLCHANVVTLNLHEALLNQISDNFSIKSSKRLSKFFLKSRHG
NPGFLAESQEHFFFHLLLLTQVIFCNTSIAFLNRTEIMTNGTMIFIAYSIDITRCPCTNT
IVFSIVPVHEIMTTFKAGFGEIRNLIMLKTRSLQLCNDVLKHLRF (SEQ ID NO: 585)
>orf03612
   MDREILKFFQDLLSILSHNDMITLFCQKCGNSFSNHFLVICN (SEQ ID NO: 586)
>orf03614 
MEDHLLINAVDEFRSISFFQFFKHAFFHIFLVKTNILSPKTNSLIITKGSRTTCFFFACQ
IRSSWYLKNQTNIQ (SEQ ID NO: 587)
>orf03620 
LFIRKKHTWINLTPKQTIFSQMNAQFFINFTRRALKRTFITFTSPTWQFPHIGPGNACLI
IT (SEQ ID NO: 588)
>orf03637 
LNIAYTDNPAHIFGKIFHDNLLALLIIFDDVTCKACLRQDKVNSGLFLDSLFNSGCKGVG
FSLVRLVISI (SEQ ID NO: 589)
>orf03641 
MCELDILHDSLYQFCPELHLKRLNSLTLACHALLDCKTLTLTELGRNLPTKARTKHNIKR
IDRLLGNRHLHKERLAVYRWHASFICSGNTMPIVLVDWSDIREQKRLMVLRASVALHGRS
VTLYEKAFPLSEQCSKKAHDQFLADLASILPSNTTPLIVSDAGFKVPWYKSVEKLGWYWL
SRVRGKVQYADLGAENWKPISNLHDMSSSHSKTLGYKRLTKSNPISCQILLYKSRSKGRK
NQRSTRTHCHHPSPKIYSASAKEPWVLATNLPVEIRTPKQLVNIYSKRMQIEETFRDLKS
PAYGLGLRHSRTSSSERFDIMLLIALMLQLTCWLAGVHAQKQGWDKHFQANTVRNRNVLS
TVRLGMEVLRHSGYTITREDLLVAATLLAQNLFTHGYALGKL (SEQ ID NO: 590)
>0ref03644
   MXXTKSSCLITTGRNDSPSTCLPRVASNNDRFSSEFRIIPDFHCSKKGIHVNMDDFS (SEQ ID NO: 591)
>orf03650
   MHFHIIKLVNHFQLLIKLNRISHPNLHIKSSFLSLVLLFYQKEQDFAIMVI (SEQ ID NO: 592)
>orf03660 
MDRGESLSDCVCMAGYEPANSSRLSIEGTYENKLYKLISSKYHTTGNDIMVCVPCGYTKY
KETPGPHACTSCPGRTHAASTTNTNQDQCNRCPPGYYETNDPSYPCDVCSPNHICVGSDP
MDPALMLYSGKRIKCDKNSVTLVPFEENVHLASCLCDKGYMARTRTGIVKCEAVPKNTYK
DVVGNVGPTNCPPGSYTLKIGATDVSECVCKRGMFFDKDNKRCTVCPVGMYCLGGRLPNG
EHMLPMMCTDGNAVTKDGGATSPGECLCKPGFYLRQDGPGGCVECPENTYKSFISNENCS
PCPRIL (SEQ ID NO: 593)
>orf03667 
MXXAFCLPVFAHPETLVKVKDAEDQLGARVGYIELDLNSGKILESLRPEERFPMMSTFKV
LLCGAVLSRIDAGQEQLGRRIHYSQNDLVEYSPVTEKHLTDGMTVRELCSAAITMSDNTA
ANLLLATIGGPKELTAFLHNMGDHVTRLDRWEPELNEAIPNDERDTTMPVAMATTLRKLL
TGELLTLASRQQLIDWMEADKVAGPLLRSALPAGWFIADKSGAGERGSRGIIAALGPDGK
PSRIVVIYTTGSQATMDERNRQIAEIGASLIKHW (SEQ ID NO: 594)
>orf03668
   MARFIRSQTLTLLEKLNELDADEQADICESLHDHADELYRSCLARFGDDGENL (SEQ ID NO: 595)
>orf03669 
MTVRELCSAAITMSDNTAANLLLTTIGGPKELTAFLHNMGDHVTRLDRWEPELNEAIPND
ERDTTMPVAMATTLRKLLTGELLTLASRQQLIDWMEADKVAGSLLRSALPAGWFIADKSG
AGERGSRGIIAALGPDGKPSRIVVIYTTGSQATMDERNRQIAEIGASLIKHW (SEQ ID NO:
596)
>orf03673

MKIHKTVNPVAYENTYYLEGDKHLIVVDPGSHWEAIRQTIEKINKPICAILLTPAHYDHI
MSLDLVRETFGNPPVYIAESETQLAPKPLPNNPLGPPXHSFIN (SEQ ID NO: 597)

[0401] In some embodiments, preferred OXC141 antigens are selected from the polypeptides orf00045 (SEQ ID NO: 53), orf00068 (SEQ ID NO: 65), orf00074 (SEQ ID NO: 70), orf00223 (SEQ ID NO: 99), orf00229 (SEQ ID NO: 104), orf00360 (SEQ ID NO: 117), orf00506 (SEQ ID NO: 135), orf00781 (SEQ ID NO: 177), orf00785 (SEQ ID NO: 178), orf01068 (SEQ ID NO: 198), orf01446 (SEQ ID NO: 235), orf01447 (SEQ ID NO: 236), orf01449 (SEQ ID NO: 237), orf01455 (SEQ ID NO: 242), orf01460 (SEQ ID NO: 247), orf01461 (SEQ ID NO: 248), orf01463 (SEQ ID NO: 250), orf01464 (SEQ ID NO: 251), orf01466 (SEQ ID NO: 252), orf01467 (SEQ ID NO: 253), orf02661 (SEQ ID NO: 433), orf02690 (SEQ ID NO: 439), orf02698 (SEQ ID NO: 444), orf03318 (SEQ ID NO: 538), orf03320 (SEQ ID NO: 539), orf03322 (SEQ ID NO: 540), orf03323 (SEQ ID NO: 541), orf03324 (SEQ ID NO: 542), orf03325 (SEQ ID NO: 543), orf03326 (SEQ ID NO: 544), orf073327 (SEQ ID NO: 545), orf03562 (SEQ ID NO: 581), orf03660 (SEQ ID NO: 593), and immunogenic fragments thereof.

### 6. Sequences Identified from INV200

>orf00004
   LKGVDDFLFIFEEGFKQGGKARADRDYSGVSSLRNSSKVYLEFLY (SEQ ID NO: 598)
>orf00005
   LCSALKNSYDIELIKVLSNKAHLYLPIETVTPQTVSTS (SEQ ID NO: 599)
>orf00006 
MRVAETSIVKKNHQIPCIINQKIAQKLIKKTSMTDIDHQLSISTSTVIRKINDFHFEHDF
SRLPEIMS (SEQ ID NO: 600)
>orf00010
   MFKSNLSLSQSLPHKDFFFFKRIIHLFSLFLLIDFIIIS (SEQ ID NO: 601)
>orf00015 
VEEVEVAEVKNARVSLTGEKTKPMKLAEVTSINVNRTKTEMEEFNRVLGGGVVPGSLVLI
GGDPGIGKSTLLLQVSTQLSQVGTVLYISGEESAQQIKLRAERLGDIDSEFYLYAETNMQ
SVRAEVERIQPDFLIIDSIQTIMSPEISGVQGSVSQVREVTAELMQLAKTNNIAIFIVGH
VTKEGTLAGPRMLEHMVDTVLYFEGERHHTFRILRAVKNRFGSTNEIGIFEMQSGGLVEV
LNPSQVFLEERLDGATGSSIVVTMEGTRPILAEVQALVTPTMFGNAKRTTTGLDFNRASL
IMAVLEKRAGLLLQNQDAYLKSAGGVKLDEPAIDLAVAVAIASSYKDKPTNPQECFVGEL
GLTGEIRRVNRIEQRINEAAKLGFTKIYVPKNSLTGITLPKEIQVIGVTTIQEVLKKVFA
(SEQ ID NO: 602)
 
>orf00018
MGVSIFLALFYMIPALYFLFRIGKKWELPKKVLILSLLGGMFLSGWLSSFANTYIHDFMN
CTPKVRQKKSNFWGVLL (SEQ ID NO: 603)
>orf00019 
MKLSYEDKVQIYELRKQGQSFKQLSKRFGVDVSGLKYMVKLIDRYGIEIVKKGKNRHYSS
KLKQEMMDKALLEGCSQRSISLDYALPNQGMLSFWPAQYKKNGYTIVEKTRGRPAKMGRK
RKKTWEEMTELERLQEENERLRTEVAYLKKLKELEERDEALERERQRQLEKWFQEDFD
(SEQ ID NO: 604)
>orf00020 
MVSGGFRLDFLLETARLARSTYYYQLKQLDGVDKDEIKTEIQGIDNEHKGNYGYRRIHL
ELRNRGFVVNHKKVQRLMRILGLTARIRRKRKYSSYQGEIGKKAENLIQRQFEASRPMEK
CYTDVTEFAIPNSTQKLYLSPVLDGFNSEIIAYHLSTSPNLEQVKSMLEQAFTEKYYENT
ILHSDQGWQYQHDSYHRFLESKGIQASMSRKGNSPDNSMMESFFGILKSEMFYGYEKNFR
SLENLEQAIVDYIDYYNNKRIKVKLKGLSSVQYRTKSFG (SEQ ID NO: 605)
>orf00024
   VNIATLQNGHILGWQIQHIANKLTSNFWIAKDFLSYQVIGWADIARMTYSHISSLFIISQF (SEQ ID NO: 606)
>orf00026 
VSITFSLTNFFKILINLTAQVSPQVIDEKILMMDLNLNNYLSTVIQLRQDVYTGIKILHR
VRHGE (SEQ ID NO: 607)
>orf00027
   MSRYSYSLDSRKIVFEISCFKEKKASLTLFFHLFESSIMKLATQPSYSSFYSELK (SEQ ID NO: 608)
>orf00033 
MKIKEQTRKLAAGCSKHCFEVVDRTDEVSSKHRFEVADRTDEVSSKHRFEVADRTDEVSS
KHRFEVADRTDEVSSKHRFEVADRTDEVSSKHRFEVADRTDEVSSKHRFEVADRTDEVSN
IYLRQGDVDVVEEIFEEY (SEQ ID NO: 609)
>orf00034 
MKPGAEGWKDERSQDIEEKDNGDGLGYFLFLSMDNRRCRCNGRTPTDRRTYSNQGSQFCI
QGKEALEEVGNNQGY (SEQ ID NO: 610)
>orf00035
   LPNCEDLRDIETKTKQDNGILEQFLGTKGQSDIIQLFIRREKGM (SEQ ID NO: 611)
>orf00044
   LSLLDLRGSLCLRIYLHEPLITTVSQDFTSLSDISHF (SEQ ID NO: 612)
>orf00047
   MDFIKSFIIGLVVGIFGPYMDDLIRIKI<FLKSSEKI<TEKSVEK (SEQ ID NO: 613)
>orf00093 
MTYEYKSHIYLAETVLNVKDLASQTTFYQQVIGLEILSQTETESILGLGGKVLVQLIQAQ
ESGEVREHXXXFHSLIN (SEQ ID NO: 614)
>orf00103 
MVSNLVFIGNCNFHNTVIFHLLNRLNQGPLQILSQNHDKGRRLSWIFKSRLGQLNASKNW
MGRKEQAMALAIAADLQDQLLFKKAD (SEQ ID NO: 615)
>orf00113 
MKFNPNQRYTRWSIRRLSVGVASVVVASGFFVLVGQPSSVRADGLNPTPGQVLPEETSGT
KEGDLSEKPGDTVLTQAKPEGVTGNTNSLPTPTERTEVSEETSPSSLDTLFEKDEEAQKN
PELTDVLKETVDTADVDGTQASPAETTPEQVKGGVKENTKDSIDVPAAYLEKAEGKGPFT
AGVNQVIPYELFAGDGMLTRLLLKASDNAPWSDNGTAKNPALPPLEGLTKGKYFYEVDLN
GNTVGKQGQALIDQLRANGTQTYKATVKVYGNKDGKADLTNLVATKNVDININGLVAKET
VEKAVKDNVKDSIDVPAAYLEKAKGEGPFTAGVNHVIPYELFAGDGMLTRLLLKASDKAP
WSDNGEAKNPALSPLGENVKTKGQYFYQVALDGNVAGKEKQALIDQFRANGTQTYSATVN
VYGNKDGKPDLDNIVATKKVTININGLISKETVQKAVADNVKDSIDVPAAYLEKAKGEGP
FTAGVNHVIPYELFAGDGMLTRLLLKASDKAPWSDNGDAKNPALSPLGENVKTKGQYFYQ
LALDGNVAGKEKQALIDQFRANGTQTYSATVNVYGNKDGKPDLDNIVATKKVTININGLI
SKETVQKAVADNVKDSIDVPAAYLEKAKGEGPFTAGVNHVIPYELFAGDGMLTRLLLKAS
DKAPWSDNGDAKNPALSPLGENVKTKGQYFYQLALDGNVAGKEKQALIDQFRANGTQTYS
ATVNVYGNKDGKPDLDNIVATKKVTININGLISKETVQKAVADNVKDSIDVPAAYLEKAK
GEGPFTAGVNHVIPYELFAGDGMLTRLLLKASDKAPWSDNGEAKNPALSPLGENVKTKGQ
YFYQVALDGNVAGKEKQALIDQFRANGTQTYSATVNVYGNKDGKPDLDNIVATKKVTIKI
NVKETSDTANGSLSPSNSGSGVTPMNHNHATGTTDSMPADTMTSSTNTMAGENMAASANK
MSDTMMSEDKAMLPNTGETQTSMASIGFLGLALAGLLGGLGLKNKKEEN
(SEQ ID NO: 616)
>orf00118 
MSLQIKLKKLAKELSKLLKDSNLETVDKDVLENSQKELQKAVLFLADEKGSEHTEAEVID
NLKEVIAKLKANA (SEQ ID NO: 617)
>orf00129
   VGRFFGSSQTSDEFFFSFDSSIVKELSEIVHGFDTVSFR (SEQ ID NO: 618)
>orf00140 
MKIKEQTRKLAAGCSKHCFEVADRTDEVSSKHCFKVVDGTDEVSSKHCFEVVDRTDEVSS
KHCFEVVDRTDEVSNHIRQGDVDVV (SEQ ID NO: 619)
>orf00146 
MNDDDSRCIHIERDGKTIEFGYLNISSTDRNTSHADGLVGIFNSNFSGVRVRGIAVFLNG
PDNLDTTLVGNFQTIWNFRIICIHS (SEQ ID NO: 620)
>orf00147
   LEFNFCRSIIKNGRDNLFNTNSTSGMATRWANHNWSDDIKDRLKTK (SEQ ID NO: 621)
>orf00152
   MSCNCAFYRSQFFDVNSVSNYHSHQKELRFPNSILFTYFVKVA (SEQ ID NO: 622)
>orf00156 
MSKEKVILAYSGGLDTSVAITWLKKDYDVVAVCMDVGEGKDLDFIHDKALKVGAVESYVI
DVKDEFATDYVLVALQSHAYYEQKYPLVSALSRPLISKKLVEIAHQIGATTIAHGCTGKG
NDQVEYQIAVAKKANEAKK (SEQ ID NO: 623)
>orf00157 
MRYDFGKVYKEIRESKGLTQEEVCGGVLSRTSLSKIESGKTTPKYENMEFLLRQINMSFE
EFEYICQLYQPSQRTEIMQTYLNMRSIIGTSDLVNLFQKCQDYLKTHHDLPIEEIRDMLE
VVIYIRQHGAGELSDHAEQVVKKLWRKIEKQDTWYESDLKILNTILFSFPIEYLHLITGK
ILQRLEVYKNYQHLYDLRIAILLNLSTLYLYNQDKNMCKQICYTLLEDAKNKKSYDRLAI
CYVRIGICTDDSKLIQKGFSLLELTEETSMLSHLKKEVEIYYQAKER
(SEQ ID NO: 624)
>orf00158 
MKIREIIGTDMYGTTVSGIVSGLNKLNFTVKAVRVALEDLTPKLTFPAILQVKNDLGQNH
FVVLHSIKEKINGTRITK (SEQ ID NO: 625)
>orf00159 
MELVLPNNYVVIDEEEMMYLDGGAIYIPRWAITGAITGAAYAALAAAGGGLQLVLASYG
LRSALVAGIVKGLGVLGIHIGNAFANTVIRSIASAGIGAGADWIFTNIIDGWDGRRDNQL
RIG (SEQ ID NO: 626)
>orf00161
   MATITNALNIAATVAEVFSLGGAIAYGLDIVDGKFDGYLWA (SEQ ID NO: 627)
>orf00162 
MKDDQKYLLAGLYSLLVAIFYFPLIESKGIFVSILMAVLLLYLIYFIATVIHIVIIKFIR
KKSFKYLVLYPFTYDGSWRFQPINLLYFPEMVRDVIPINLVQEYCQGQPYGLLKKMLKRI
RLSREIALLLATIIVYFFTHRILPLSVFTFMFSYILLFVQSYLGSNTAWIGNRRLIIDDE
FEKILLSKSYIKEISSARYSEYLTCEYKNPTPIILIAIFENLLDSYLLQNQSEVDLDIFY
KVLPLLYKEKYTMGFNYFVSLNYLLYKVGFLGIIYDNEALRDLSKQYLNKNISELQDGSF
EGGIQDAVASKQIVVINEFIACLNSKCMPSQYDRFFYKDRPYIFSRKSSIKG
(SEQ ID NO: 628)
>orf00163 
MKNKRYFFDTILIILLLISTIFCVSPVFIKLDILGTPSHAILTFVLAIPLFYILSQCLHT
LLLLVSSIFCKLRPIYFYFIFVIIIGARKYYRILFHQLMGFSPGIAVFYKESQTTKNLFK
FYYFLYFTTLISYYFFFTFVYDKSLLLPLIPFSIIIALVQKLYRIENQQLFLLKSKVLTI
LESKRDCEFNLQDYHEIWKLQSKSELPCVALSYISLIKPYLSESVREQIDLLEVKRFKKI
NHPISLYGMLDVIKLNLYLRHYNEKNKYESMLNKILEVRPDFVLIEQNIDDSLNSSQPLS
LSLAISEIQLLLEVYIGIKHVSIRR (SEQ ID NO: 629)
>orf00164 
MIRKPIIFLLMLPIWGLWIELHLLVSNLQLNLEIPFDFVVSTSLTFFVLILSKIVLDILY
ALKDLYKKEALITIFPFIFIGRKKVNVRFSPYFSFHRKSLSPDDLRSRIIWSFILEIAII
LVFILKIPFAIIMLTTIFFWTIMDINHLVFNKTEFLFNQNKWQKEDSFESDLTKTLKDKI
QKSELSYSDLMSLQLYDAMNQSTFLTDSELFEDILKKIEDSHNTLLCTGLVELLLYEISI
SNNNNWQEKVDKIRIQLIRINQLDFFYYTSWLRQNFDFCMNREYHKMKSRKLLLSNKKIV
(SEQ ID NO: 630)
>orf00165 
MELVLPNNYVVIDEEEMMYFDGGAYLSKRACQGICAALAMSSGTFIALAGAAVLTKKLIN
YIKVGGLGGWLIGAAAGKIAYYIGYGVLNRGCDINGNPYPWDGFISATVR
(SEQ ID NO: 631)
>orf00166 
MSNVDKIRKIHIIVCWVYIFLSFRAIINDTEYFLLIFLAFIYSIVSLPLYSVKNKIVSIC
LAINSILLMSFPILINKFFPESFLTYTVLISVFITELIIFHLIGKDFDIKLTNEYKKISQ
FRSKVSQSPWIKYLEISSFILTIFPSILYGTVDNHVLTLIFLIKICVDTTIKFLFIRLFD
TSTLMKRRIFFLFALDVIAYLFLGYLLVIQKAGYLFSVLLLFSNFSVPFIKEKEYELFKN
SK (SEQ ID NO: 632)
>orf00167
   LFNEIKKTSSLIGNVFIGMKEDDAMFKKRIEKGKSSVFIFLE (SEQ ID NO: 633)
>orf00168 
MNKKKMILTSLASVAILGAGFVTSQPTFVRAEEAPVASQSKAEKDYDTAKRDAENAKKAL
EEAKRAQKKYEDDQKKTEEKAKEEKQASEAEQKANLQYQLKLREYIQKTGDRSKIQKEME
EAEKKHKNAKAEFDKVRGKVIPSAEELKETRRKAEEAKAKEAELTKKVEEAEKKVTEAKQ
KLDAERAKEVALQAKIAELENQVHRLETELKEIDESDSEDYVKEGLRVPLQSELDVKQAK
LSKLEELSDKIDELDAEIAKLEKDVEDFKNSDGEYSALYLEAAEKDLVAKKAELEKTEAD
LKKAVNEPEKPAEEPENPAPAPKPAPAPQPEKPAPAPAPKPEKSADQQAEEDYARRSEEE
YNRLTQQQPPKAEKPAPAPVPKPEQPAPAPKTGWKQENGMWYFYNTDGSMATGWLQNNGS
WYYLNSNGAMATGWLQYNGSWYYLNANGAMATGWAKVNGSWYYLNANGAMATGWLQYNGS
WYYLNASGAMATGWAKVNGSWYYLNANGSMATGWLQYNGSWYYLNANGAMATGWAKVNGS
WYYLNANGSMATGWVKDGDTWYYLEASGAMKASQWFKVSDKWYYVNGLGALAVNTTVDGY
EVNANGEWV (SEQ ID NO: 634)
>orf00190
   LKKRMNRWQFLLNQSKEMVGILLLKMKEQELIEFVVNL (SEQ ID NO: 635)
>orf00191
   LIKVIKRKAFGFRNFNNFKKRILMTLNIKKESTNFVLSRL (SEQ ID NO: 636)
>orf00195 
MTYNEKRLTNSLERVHMEQLKNTTDLLGLEDKNIKILSVLKYQTHLVVQAKLDSPAPPCP
HCQGKMIKYDFQKASKIPLLDCQGLPTVLHLKKRRFQCKNCLKVVVSQTSIVKKNCQISN
MVRQKIAQLLLEKQSMTEIAHRLAVSTSTVIRKLREFKFETDWTKLPKVMSWDEYSFKKS
KMSFIAQDFESKSILAILDGRTHAVIRNHFQRYQREVRELVEVITMDMYSPYYRLAKQLF
PKAKIVLDRFHIV (SEQ ID NO: 637)
>orf00196 
MGYSLKKSRTYCEQDPEKVNRFLKELNHLSYLTPIYIYETGVETYFYLEYDRALSRQLVS
LEEDIII (SEQ ID NO: 638)
>or00201 
MRFYCEAYEVITEEKKVYFNDIELEVKYSSVEELFIICEKLLDKKKVSFFYVDEKPLRYL
LFDYIFLLVLAKKNIPILDGVSNKQVDPTLLHHFSLEIEKNFIDFCYKNMDLILKTQSIS
LCHREELIIVDVSDDSKFGVFYKRFRTLVDKNNGKYVCVYNFSRVSDILQNWKNYCNRKF
SVTFTESQFELFKLLYNQKNFKTISLLFGKKIVAGGIIYYSDLTNIEYFCIFWWDSMFGK
DSIGKYVYVEEISRCHFLDRNYSFCYGLQDYKSKLIKYFLE
(SEQ ID NO: 639)
>orf00202 
METRNLISYSLTDIFETDKIRIELLGEIYYKNIKLELHEFAGLYKIYGISLIKNITGMFL
IIIFDTKTKELKIFQDITTSYFNLYYTVYGGVFYYSTSLKKVMKLSHVPVTLNNKKIQEF
MRNGFILDSNTLVTEINKLEYFSYISVNNTLRICGIDYNDSNNFTKEQVLKNWDSMLRES
ILRVYSEAGEANITLSSGFDSNYILYTLANYTNSSINAFCIGGEKGINEIPEVTKIAKFY
GINLLVDTVMSQDLEYFPDIVWRLEGSLFECGVILQYYLGRLLFQKGNTSILCGESADEI
MTFKYHSVNYNQFCNDKQKSVYFSYSDYPFYVTNSIVLKKNSLLLHSFGIHPRYPYKMSE
IVEMSKKISDLNDKKEFHKKNCELRFQDSVLDNINSVPGTTHLFSCLNIQTLVKIILYIF
RYNSCMKIFNFRDKELIFFNKIVNGLIQNIEENLEDDIERILKYLYICLFNEIFIIKNKV
NFFDDVEFNQTLSEFLDKL (SEQ ID NO: 640)
>orf00204 
MKFFCENNLNITFFDTFSEIKNNIDYYIIALPTDYDEKIGSFNTYEIEQTVSKILRVKPN
GKIILKSTVPIGFSNKLKRLFDTKNIIFVPEFLREGCSIYDNLYPSRIVVGDETVEGRKI
AELFLSISTHSTANIKNVMLVSPTEAEAIKLFSNTFLALRVAFFNELDSFAERRSLNAEV
VIKGVCLDPRIGNFYNNPSFGFGGYCLPKDTKQLKKEFIEINAPVIEAIDISNTNRKQFI
VKQILERKPKIVGIYKLGMKYNSDNYKESAILSIINELLIVGIKILVYEPNLNVSIDNVI
FEKNFELFTKQSDLIVANRWDRGLEAYKDKVYTRGIWIRD
(SEQ ID NO: 641)
>orf00206
   LEEESFIMENTEFSLELDVTEVATEQDYVSSGVTSTGCCKN (SEQ ID NO: 642)
>orf00207 
MFKIKDNYIYRQCVNDSILIEKLNENNLEIFFDSKIFQEMLMVANPRFFNELTKEKIYQN
STFRNYAKRSLTRATPFGLFSSVGVGSFSKVSYPQQIRENYSKKVSVSGEWISSLCMMLE
NEDSVLLQLHLQWNQKVLELSDKYQLNNINYWGVSEQSRDILIKKTALLEFIKKLTYKSE
VSVLDLVQEIQTKSPNLETQKIIDYLRNLIISEFLFTNLRKVVINHNCLDNLIYILSSIN
EQTKLTTDLLQLKSCIEKYSKSELGEGILQYAEICEKMSHIFNEEKQRYLKVDLVNSYDS
LLPKDLKKTLEDFVNFISRINLGKDYRNKELISYTEKFVEKYGEYVEVPIKQLLDSKLGL
GIPKQNLEPYSILSSVAEQTFLSYLSKEIFKAVKNNKKEIDISNIPPELLYPNLDRFAVN
QFELYCEMKNFGEQPVISIVPNTGSDMIGKSIGRFASYFLNSNIELDSRVDNVELIEFPS
DNKNLNVMSSHHGHSKKLLLSYEDDFDIDSLELDFLVVGVERVNEHYKLYFRDLRTDLIV
NFVTTSMLNHKSIGVFSHLARFLLTVSLEWQDNPFSLFRVIENLDFLPYIPRIKYKNIIL
SEEKWILSDVDKKDMSTISQWKKFFDVPSLLYFHKDDERLLIDLKNSLDVQWILKQNVDK
LHFTRFDKIDGKNCEFIFGFENPRNSVYPHSVSEKTVRRIENDFYKDYVKTFSSDWIYFK
LYGINSSTMPELRENLLIFTDELLAEKLVSDFHFVNYNDGGDGSIRLRFKIMNEDDFEKL
RYRIIHWIDFLLNHYFCKDVSFNLYEREVERYGGIGFLTVCERIFSIDSYLVLKLFSKKV
LKVDDYLSVLHSIFIYIRLLGISPKQLLKLMKDTFTQNIYRKSFKKVFPNNAKVIKEFKQ
YFEDQSKFDIFNEVFKSFSPIEKHFEYKNDMIHSLLHMHMNRIGIFSLNEKEYLYFVRYI
LEVLNNYEKYN (SEQ ID NO: 643)
>orf00208 
MRNIIKKYDEIINKVDSLVLDNNIIDLLQRSCYTENRSYLSEYPSIIIYLSYRLANCDDN
EHSKLLYNRVNYYLHELLKSIKLNSRNNISMCYGFSGYVYALKLLPKRSKEYSKLLETLE
TILVSLTRDRLSEIKKSNKVKEEYIDVIQGVSSVGKYFLSKDKLTSNQELLLKGVLNYLA
GVINNKPTIYPEYMPNEKLKRKFPNGYINLGVAHGILGPLYVLALGFKKFNMPEYLISLK
KGLSYYEKTFQTNKIGKIIGWNGRVSAEVESEKFEYNLSWCYGSLGMARVLYNISKIIDI
PKLQELATDVFHSSIYYLNSSEILNNAICHGRSGIMLLFNLMYLDTGESQFKAISDNLFK
EIVNKATDSEYIFVERDIYFRGVNYDEVIEYIDFCLLNGVSGIVLALMAQRTGNASPLAE
MFFMQ (SEQ ID NO: 644)
>orf00209 
MKKILNNKLYMKVLVSDLISNFGDTLYFIALMTYVTEIKSSNLAISIVNISETIPILFTI
FFGIIADRTLNKVGMIIKTLWIRTILYLLVAVVMNFKESILVVILASIVNLISDTLGQFE
NGLFYPISNRIVKKSDREETMAFRQTATSTMNIVNQSLGAFLITFLSFFHLALINSLTFA
ISLLITLAIKSQINNFYIDKTPSTKVSKVDFKATFSDIISNLKLSLKHLFSLTNMKTVLL
VIPILNGSLAIIIPLAVVNLSKSSALTIISSATTISVLGISTVSGGILGGTLILISKKFK
NLSIENLLKMNLMTILLSFIAFYYQNIYFIVLTLFLSSVFVSALNPKIGAIIFNNLDETK
LATIFGGMVTYFQLGDVVSRLLFSTLVIYLSYTYIAVIYMILVLIVAIYTFRRVQTTS
(SEQ ID NO: 645)
>of00213 
MKIKEQTRKLAAGCSKHCFEVVDRTDEVSSKHCFEVVDRTDEVSNHTYGKVKLTWFEESF
116
 EEYK (SEQ ID NO: 646)
>orf00217
   LKSSILSKMGDFSVRYCNLVGTVLFGVVLIAILRLVF (SEQ ID NO: 647)
>orf00246 
MGLDVGSKTVGVAISDPLGFTAQGLEIIQINEEQGQFGFDRVKELVDTYKVERFVVGLPK
NMNNTSGPRVEASQAYGAKLEEFFGLPVDYQDERLTTVAAERMLIEQADISRNKRKXXIP
FIN (SEQ ID NO: 648)
>orf00270
   LNTSYSFGKKDQFALEHCFCIKLSIFARAVTLFVSCIN (SEQ ID NO: 649)
>orf00291 
MLIGEGYRTFPVLIYTQFISEVGGNSAFAIMAIIIALAIFLIQKHIANRYSFSMNLLHPI
EPKKTTKGKMAAIYATVYGIIFISVLPQIYLIYTSFLKTSGMVFVKGYSPNSYKVAFNRM
GSAIFNTIRIPLIALVLVVLFATFISYLAVRKRNLFTNLIDSLSMVPYIVPGTVLGIAFI
SSFNTGLFGSGFLMITGTAFILIMSLSVRRLPYTIRSSVASLQQIAPSIEEAAESLGSSR
LNIFAKITTPMMLSGIISGAILSWVTMISKLSTSILLYNVKTRTMTVAIYTEILRGNYGV
AAALSTILTVLTVGSLLLFMKISKSNSITL (SEQ ID NO: 650)
>orf00292
   LIIIASMSAPFVGAYSWVLLLGRNEVITKFLTNALYLPAIDIY (SEQ ID NO: 651)
>orf00293
   MERKKLNIWTVSSFFLFLTYPIFLVYPIVTVLKQALIHEGQFSLANFVTFFSKAY
   (SEQ ID NO: 652)
>orf00295 
LLSTTEFIGLSIRILSNLHEFKILVGLLNQFFFWNLLLHKTKSNVVSDSQMWENSVVLEN
HPDIAFAGFHIIDFCIIEVKFSTFDTVETCNHTKKGRFPTS
(SEQ ID NO: 653)
>orf00314 
MITIKKQEIVKLEDVLHLYQAVGWTNYTHQPEMLEQALSHSLVIYLALDGDAVVGLIRLV
GDGFSSVLVQDLIVLPIYQRQGIGSALMKEALEDYKDAYQVQLVTEETERTLGFYRSMGF
EILSTYNCIGMTWMNRKK (SEQ ID NO: 654)
>orf00325 
MKIKEQTRKLAAGCSKQCFEVVDRTNEVSNHTYGKATLTWFEEIFEEYNTNLEYKQPICS
QEKA (SEQ ID NO: 655)
>orf00359
   MVDNIPKRVNDVIRQAGNNAKTSRPHVGIGKSHISVSFLFPYHTANRIKNQEKVIF (SEQ ID NO: 656)
>orf00375 
LFDLLDHGLDTVLVCHVTDISMGLDANFTISFNPFIDQILIDIVKDNSSAGFSVGFGNSK
SNSIRSAGDESNFSF (SEQ ID NO: 657)
>orf00387 
MKSLARLLIIHVFISIFLFFALTSGAISHTVLLLLLFLPALNKGLEKIQSKRIPVLNAA
LFFLLISFPQLLTNPVQWKFSIFLVVTIISSLAYFYNFYQVVKEVDQKQLI (SEQ ID NO: 658)
>orf00390 
LEAAGEIETEFQGWIVLVVFNHIDSLSRDTDILGEFELGNTQFLAKFFHTIHLISFLIYV
VYI (SEQ ID NO: 659)
>orf00403 
MEGVNHVDIIKVSCCSFISQVNWMMKGKIPNREGFKFSVARFDAIDLVVVHIGHTRCQFS
RTGSRSGYDNQVATGFDVVVFAHAFWGNDVIHIRRISFDWIMKIRINSVFLKLVAEGICS
GLASVLCNDNGTNKNP (SEQ ID NO: 660)
>orf00404
   MFNVASINGNHNLNLLFQFLQELDFVVRFITRKDTSSVEIF (SEQ ID NO: 661)
>orf00409 
MIDIHSHIVFDVDDGPKSREESKALLIESYRQGVRTIVSTSHRRKGMFETPEEKIAENFL
QVREIAKEVADDLVIAYGAEIYYTLDALEKLEKKEIPTLNDSRYALIEFSMHTSYRQIHT
GLSNILMLGITPVIAHIERYDALENNEKRVRELIDMGCYTQINSYHVSKPKFFGEKYKFM
KKRARYFLERDLVHVVASDMHNLDSRPPYMQQAYDIIAKKYGAKKAKELFVDNPRKIIMD
QLI(SEQ ID NO: 662)
>orf00410 
MKEQNTLEIDVLQLSRALWKRKLVILLVAIITSSVAFAYSTFVIKPEFTSTTRIYVVNRD
QGEKSGLTNQDLQAGSYLVKDYREIILSQDVLEEVVSDLKLDLTPKGLANKIKVTVPVDT
RIVSVSVNDRVPEEASRIANSLREVAAQKIISITRVSDVTTLEEARPAISPSSPNIKRNT
LIGFLAGVSGTSVIVFLLEFLNTRVKRPEDIENTLQMTLLGVVPNLSKLK
(SEQ ID NO: 663)
>orf00413 
MDKKGLEIFLAVLQSIIVILLVYFLSFVRETELERSSMVILYLLHFFVFYFSSYGNKFFK
RGYLVEFNSTIRYIFFFAIAISVLNFFIAERFSISRRGMVYFLTLEGISLYLLNFLVKKY
WKHVFFNPKNSKKILLLTVTENIEKVLDKLLESDELSWKLVAVSVLDKSDFQHDKIPVIE
KEKIIEFATHEVVDEVFVDLPGESYDIGEIISKFETMGIDVTVNLNAFNKNLGRNKQIHE
IVGLNVVTFSTNFYKTSHVISKRILDICGATIGLILFAIASLVLVPLIRKDGGPAIFAQT
RIGKNGRHFTFYKFRSMRIDAEAIKEQLMDQNTMRGGMFKMDNDPRVTKIGRFIRKTSLD
ELPQFWNVFIGDMSLVGTRPPTVDEYDQYTPEQKRRLSFKPGITGLWQVSGRSKITDFDD
VVKLDVAYIDNWTIWKDIEILLKTVKVVFMRNGAK (SEQ ID NO: 664)
>orf00414 
VTFDKEDARSILENEIFYPCYYPTNRNLKNLIKNTILAFKILRKERPDIIVSSGAAVAVP
FFYLGKIFGAKTVYIEVFDRIDAPTMTGKLVYPVTDRFIVQWEEMKKVYPKAINLGGIF
(SEQ ID NO: 665)
>orf00415 
MIFVTVGTHEQQFNRLIKEVDRLKGEGFIQDDVFIQTGYSNYVPKFCKWEKVISYEKMNQ
LIKESDIIITHGGPATFMAVIAKGKNPIIVPRLKKFGEHVNDHQMQFVKITKEIYNLIVI
DDISDLHLILHNFKDKHFETYLNNERFNVRFNVEISNLFKGNKINEN
(SEQ ID NO: 666)
>orf00416 
MKIRIEPQYFLYKYLWFIILLPKQFMQLILFFLIALTLLPTYIKEKQVFKIDTPSFCMVL
WTIIYSISIIFNSLIDGLAVQVIFSDLSKAFNWLIAVFFYNYYLKMPINIDRIKRYMYYN
FTILVVFVGLFYIQRGSNVILFGRSLLDWDGFTLATSYGVRYTGFLEYATLNGQLILFLL
PLIRLFRFRFFTQTIIFAFLLEVLVLSKSRIAIVAMLIYIAFAVVNEINSNNKWLIGIFC
PIIPFMLFYNFEKIKQIFFQMFSSRSGSNATRFRVYEESLKAINGMEMLLGAGVRIPSTV
DILLGSHSMYISFIYRTGVLGSIIITVMFYYLFSKFLKCDSSERLRSIGYILALSVFWLF
EELDPHYWCLILFFSTISIFINNRKEEIVG (SEQ ID NO: 667)
>orf00417 
MIEVSIIIPIYNAEKTIKNCVDSALKQNLESLEVILVNDGSNDSTSKILEQYGDNPQVMI
FHQVNMGVSAARNVGLSYASGEYVFFLDSDDILDEGMLSKMYQFAKSNKIDLLSCWHKEP
STTQYGGNDNSSASFIARTKEEIGNHFVDIFPRSACAKLFLRRRIEENNIAFSTEMSLGE
DMSFVCQYLMVSRSIAVIDGLYYTIQNVNPQSLSKRYVSNIENSLLMQNQLWDQLLEVYP
KIEENYYKQHMDFRFYLASLYVNNLFKFDSPYSSKEKWDNIAQQLKKYRPFLDEKVSKEK
KPKNMNEMVIFYLLKSKIPALIYSFYSFKEWWKKKRLKN
(SEQ ID NO: 668)
>orf00418 
MEDLVSIVVPVYNVEKYLKKSIESILNQTYDNLEVLLVDDGSTDSSGEICDSFIKVDSRI
RVFHKENGGLSDARNFGIEHMKGQYVSFIDGDDYISKDYVWKLYHSLKNNNSEVSICSFS
LVDETGEKIKDELLDSGEVSLSGQQILEKALTADGYRYVVAWNKLYRSTLFEKLKFKKGM
LYEDEFLNYPLFWDCKRVSIVEEPLYLYVQRKGSIIQSNMTLEKIKMKDKMHTSRIEFYA
EKKNSFLHQRSCQQYCNWIVTITVSHYNVLNVAFLKYLQHQFRRIVKYTQNDDKLIIQN
 ILGYINIRLAAYVKSKVM (SEQ ID NO: 669) ILGYINIRLAAYVKSKVM (SEQ ID NO: 669)
>orf00419 
MFPIYIISNQNIAFQQEIDIAYRKMKRQFSHISLTESEQKNDMNISNKVWICWFQGEERP
PELIRTCIQSMRTHFLGREIIVLTEENISDYIDIPDYITDKYKKGSISRAHYSDILRVEL
LCRYGGLWVDVTVLNTGGDFSNLELPLFVYKSLDLSRKDSQAIVASSWLISSYSNHPILL
YARKLLWEYWRRKNSLCNYFLFHIFFTIATELYPIEWSAVLTFNNHSPHMFNFELNNQFS
EKRWEQLKQISVFHKLNHHIDYSIGVNNFYKFIVFSKVEKNE
(SEQ ID NO: 670)
>orf00420 
MSNKISKNLAYNIGYQLIGIAFPLITSPYLSRILGAENLGIHSFTISVALYFMMFMLLGI
ANYGNRTIATVKREGKEILSKTFWNIYYVQLLMSVLVTIAYLIYLYFWVSSYKFIAILQL
FLLLSNAVDITWLFYGLEDFKQIVFRNTLVKLLGLFLIFSFVHESSDLWKYTLINGGVTL
VGQLLLWGQLKGRLSWVKIQKKDLLSHIKPILVLFIPVLAISIFSNMDKYMLGLMVGVKQ
VGFYDNANRIIDIPKALIAALEAVMLPRTSYLLAEGQEEKSNYYIEVTILYAMMISSVLI
FGIISVSDIFSLVFWGEEFLESGRLIAAMAPVFVFSVPGNIIRTQYLIPRAKDKDYVLSL
IIGALVNILLNCFLIKPFGAMGATISTVLAEFVLYGVQFWTVRRDLDFKKYLKNGFIFYL
FGMIMYLAIIAVKAHLQYNIINLVLLIVLGGIVYTGFCCFYILISRNVHFEILREKIKRK
IGYENIL (SEQ ID NO: 671)
>orf00422 
MFVADIMISDYSSAPIDFLLLNRVVFLYLPDFKEYQSDKNPFFEVFKVSKTKGIALDPFD
EIIGRFQFGVRIV (SEQ ID NO: 672)
>orf00428
   MGFSMKLIHOLNTHTTHSTAKMLYNVKAIKNDFSIRE (SEQ ID NO: 674)
>orf00431 
MEQLHFITKLLDIKDTNTQIIDVVNRDSHKEIIAKLDYDAPSCPECGSQMKKYDFQKPSK
IPYLETTGMPTRILLRKRRFKCYHCSKMMVAETPLVKKNHQIPRIINQKIAQKLIEKISM
TDIAHQLSISTSTVIRKLNDFHFECNFRNLPKIMSWDVETVRGVTVSIGRWR
(SEQ ID NO: 675)
>orf00444 
LQIAQESSQDTDGINPPVVEEAMVFDRNDCLNQICGNIISLGIDAAFRTQVSNELIFIVV
DFTRSCCN (SEQ ID NO: 677)
>orf00446
   MLNLMWMKIFHRNRTFLFCFLDFKVDVISIINARIVRR (SEQ ID NO: 678)
>orf00447
   MYNSQALRQIVVVGSIDHLFKRHSSICEIFGLRKRCLSFL (SEQ ID NO: 679)
>orf00472 
MSLADLLEELEAAKDSKKARSMEAYMRHQFSFLGIAVPERNKLYKNIFQKRKKQRLSIGI
LQTLAGKRSLENTNMWLLTI (SEQ ID NO: 680)
>orf00473
   MEKILLHNLNQTEFFINKAIGWTLRDYSKTNPTMVTCFIEKNKERMAELSIKEASKYL (SEQ ID NO: 681)
>orf00977
   LSTCWNGKFCHICVALFHCFRAFKLALNEILCLLTNVSFIFVSVAF (SEQ ID NO: 682)
>orf00487
   LLGSFFSWTTKELMGIIFFNNFPTVHKNNMMGYISSKTYLIKLIKNSI (SEQ ID NO: 683)
>orf00509
   LKNVFSVGCHFFQFFVRFFMFGKFDHFNLVELVQTDQATRITTGRTSLRTE (SEQ ID NO: 684)
>orf00535
   LIDIKHFFLCLPLSKKMIIDIIVNKNPDRFCMIEKVKKTMAENR (SEQ ID NO: 685)
>orf00539
   VNIDSSEFYISHITDGIFDSFLDSNRYLRNFYSVLKVEIDICCEFFVHVFKINATAE (SEQ ID NO: 686)
>orf00540
   VNTLYLCSSDSNDFFKYTWGDNDFAKLFFNSHRMTSF (SEQ ID NO: 687)
>orf00550
   VKEEKKAIVLGADNAYMDKVETTIKSLCVHHYNLI<FYVFNDDLFREWFQLMEKRLETLNS EIVNV (SEQ ID NO: 688)
>orf00551
   VSNEIKIIALKLSIFWGHNHFRLTGNWKIFYLCLKSGLA (SEQ ID NO: 689)
>orf00552 
MKRIQLNMNETKKYLVIKAIAQGKKTKKRACVELNLSERQINRLLLAYQQKGKEAFRHGH
GNRNRKPKHAIPDEIKERVLKKYLSYETYKPNVLHFCELLAEEEGIKLSDTTVRKILYKK
NILSPKSHRKTKKRVRKQAKLNLNQPLDNPILPTAKDFLEDPKKVHPSRPRKKFAGELIQ
MDASPHAWFGPETTNLHLAIDDASGNILGAYFDKQETLNAYYHVLEQILANHGIPLQMKT
DKRTVFTYQASNSKKMEDDTYTQFGYACHQLGILLETTSIPQAKGRVERLNQTLQSRLPI
ELERNNIHTLEDANTFLLSYIQTFNEQFGNKTKLSVFEEAPNPSERNLILARLAERVVDS
GHHIRFQNRYYMPVEQGKEVYFIRKTKALVLKAFDGDIYLNIADKIYHTKELLDHELYSK
NFEQEPEQKKKDASISLHKPIRGNSHLSNNTFIKIKRIMKSLLVRSFILLNYKYNLFFAK
WEAFP (SEQ ID NO: 690)
>orf00556
   LVEQLTFNQWVTGSSPVRVIYAGLAELADAPDLGSGA (SEQ ID NO: 691)
>orf00557
   MGEEEMRNKMIIAVSLVVAGVMTYLMFSGLDEDFYHFP (SEQ ID NO: 692)
>orf00567
   MNTIERTRRLVKGCATHCFEVVDRTDEVSSKHVFEVVDETNEVSSKHVFEVVDETDEVSN HTYGKAT (SEQ ID NO: 693)
>orf00581
   MLSNDFIQLRKDDIKTTSVLYFPIRLFSLETMNMSSQYF (SEQ ID NO: 694)
>orf00582 
LTCYPNPQKRLEAGFDKLIEIKRLTASKIQDILSVAPRSIGTTSPAREFEIIENIKHYKR
LIDKAKKCVNDLMAEFNSVITTVTGIENRLGAVILAEIRNIHAFDNPAQLQAFAGLDSSI
YQSGQIDLAGRMVKRGSPHLR (SEQ ID NO: 695)
>orf00595 
MKRIQLNMNETKKYLVIKAIAQGKKTKKRACVELNLSERQINRLLLAYQQKGKEAFRHGN
RNRKPKHAIPDEIKERILKKYLSYETYKPNVLHFCELLAEEEGIKLSDTTVRKILYKKNI
LSPKSHRKTKKRVRKQAKLNLNQPLDNPILPTAKDFLEDPKKVHPSRPRKKFSGELIQMD
ASPHAWFGPETTNLHLAIDDASGNILGAYFDKQETLNAYYHVLEQILANHGIPLQMKTDK
RTVFTYQASNSKKMEDDTYTQFGYACHQLGILLETTSIPQAKGRVERLNQTLQSRLPIEL
ERNNIHTLEDANTFLLSYIQTFNEQFGNKTKLSVFEEAPNPSERNLILARLAERVVDSGH
HIRFQNRYYMPVEQGKEVYFIRKTKALVLKAFDGDIYLNIADKIYHTKELLDHELYSKNF
EQEPEQKKERRKYIPPQTHPWKLTSFKQYLHKNKKDYEEFTSEEIHSPQLQV
(SEQ ID NO: 696)
>orf00601
   MDTKSSCLITTGRNDSPSTCLPRVASNNDRFSSEFRIIPDFHCSKKGIHVNMDDFS (SEQ ID NO: 697)
>orf00604 
MMSIREQDLKDIGAIIKYKNFHSPFDTFKYLKDMGFDTIDLSVLLEGFSYAYGMDWLEKF
FKENQDKLREFY (SEQ ID NO: 698)
>orf00610 
MIPLYRTDNDITKFFTKIRNGHLAKTAGGLDDKFHEANASTSKAFDRQGVGEVNDIRDSA
GSQELRINDKRKTENILFLEIRVRIFRVPHPNDSFFSSHFLG
(SEQ ID NO: 699)
>orf00611 
VLSQGDKDITILDAGLLKNGKIGPVTKDTNDIKATDNMIENSFVLLNQQNIMLFCNQGAT
EGKTNFSPSDKDNFHNKTYFFMM (SEQ ID NO: 700)
>orf00616 
MKIKEQTRKLAAGCSKQCFEIVDRTDEVSSKHGFEVVDETDEVSNHTYGKAKLTWFEEIF
EEYKMMGKAGQLVFFDVYRLVRQVS (SEQ ID NO: 701)
>orf00645 
LVEIVRGGSPRPIKDYLTSEVDGINWIKIGDTEKGEKYINNVKEKIKKSGLNKTRFVKKG
TFLLTNSMSFGRPYILNVDGAIHDGWLAISNYENSLNKDYLFYILSSNVVYSQFLSLISG
AVVKNLNSDKVASILIPLPPLAEQQRIIEAIESALEKVDEYAESYNRLEQLDKEFPDKLK
KSILQYAMQGKLVEQDPNDESVEVLLEKIRAEKQKLFEEGKIKKKDLDISIVSQGDDNSY
YEEVPCEIPESWEWVRLNDITSYIQRGKSPKYSNIPIYPVIAQKCNQWSGFSIDLARFID
PETVHSYQKERLLRDGDLMWNSTGLGTLGRLAIYHENKNPYVWAVADSHVTVIRVLSGVI
NCHFIYNFLSSPIVQSVIEEKASGSTKQKELLTKTIKEYLIPLPPLPEQSRIVDKIEQFF
AHIDALI (SEQ ID NO: 702)
>orf00657 
MTPEQLKASILQRAMEGKLVPQNPNDEPASELLKRIKAEKEKLISEGKIKRDKKETEIFR
GDDGKHYGKFADGSTQEIDVPYDIPDTWEWVRFSTLVEIVRGGSPRPIKDYLTSEVDGIN
WIKIGDTEKGEKYINNVKEKIKKSGLNKTRFVKKGTFLLTNSMSFGRPYILNVDGAIHDG
WLAISNYENSLNKDYLFYILSSNVVYSQFLSLISGAVVKNLNSDKVASILIPLPPLAEQQ
RIIEAIESALEKVDEYAESYNRLEQLDKEFPDKLKKSILQYAMQGKLVEQDPNDESVEVL
LEKIRAEKQKLFEEGKIKKKDLDISIVSQGDDNSYYGNIPMNWVVIKIKDIFSINTGLSY
KKGDLSINKGVRIIRGGNIKPLEFSLLDNDYYIDTQFISSEQVYLKHNQLITPVSTSIEH
IGKFARIDKDYDGVVAGGFIFQLTPFESSEIISKFLLFNLSSPLFYKQLKAITKLSGQAL
YNIPKTTLSELLIPLAPFEEQELITQKVEKLFEKVNQLWK
(SEQ ID NO: 703)
>orf00669
   MCKANSRNDIFILQDSFCFEIFSRKKFKIVKEVLPNSTCKFRVVQ (SEQ ID NO: 704)
>orf00673
   VDRTDEVSSKHCFEVVDRTDEVSNHTHDKPTLTWFEEIFEEYHSPFHN (SEQ ID NO: 705)
>orf00674
   LDIJIHIVLDSLNAVSGIQDFICDGLAI FCIJQITSGCSSCK (SEQ ID NO: 706)
>orf00683
   MGPLLMHLCQQLVWLAKYLKRAGSDMMFLQEFLIJRRFNPSLLGKIIL (SEQ ID NO: 707)
>orf00684
   LVAKGQGHKLRVSRHKDNQGIGVLFPNLSSHFQPLHLLISNLNIQKE (SEQ ID NO: 708)
>0ref00692 
MTSYKRTFVPQIDARDCGVAALASIAKFYGSDFSLAHLRELAKTNKEGTTALGIVKAADE
MGFETRPVQADKTLFDMSDVPYPFIVHVNKEGKLQHYYVVYQTKKDYLIIGDPDPSVKIT
KMSKERFFSEWTGVAIFLAPKPSYQPHKDKKNGLLSFLPLIFKQKSLIAYIVLSSLLVTI
 
INIGGSYYLQGILDEYIPNQMKSTLGIISVGLVITYILQQVMSFSRDYLLTVLSQRLSID
VILSYIRHIFELPMSFFATRRTGEIISRFTDANSIIDALASTILSLFLDVSILILVGGVL
LAQNPNLFLLSLLSIPIYMFIIFSFMKPFEKMNHDVMQSNSMVSSAIIEDINGIETIKSL
TSEENRYQNIDSEFVDYLEKSFKLSKYSILQTSLKQGTKLVLNILILWFGAQLVMSSKIS
IGQLITFNTLFSYFTTPMENIINLQTKLQSAKVANNRLNEVYLVESEFQAPENPVHSHFL
MGDIEFDDLSYKYGFGRDTLTDINLTIKQGDKVSLVGVSGSGKTTLAKMIVNFFEPYKGH
ISINHQDIKNIDKKVLRRHINYLPQQAYIFNGSILENLTLGGNHMISQEDILKACELAEI
RQDIERMPMGYQTQLSDGAGLSGGQKQRIALARALLTKAPVLILDEATSGLDVLTEKKVI
DNLISLTDKTILFVAHRLSIAERTNRVIVLDQGKIIEVGSHQELMQAQGFYHHLFNK
(SEQ ID NO: 709)
>orf00699 
LRIYLHEPLITTVSQDFSSLSDISATHFEQLHIVAIVHSDIQRNNSPLTCDNRLSLHSVK
FLFTRIIG (SEQ ID NO: 710)
>orf00723
   MGLIKTLAKIYGNYFLTVQGVKVMKTIKKDDNAVVGLGKLFIADKLMDTARWLIKPEDKK (SEQ ID NO: 711)
>orf00724
   MKFFWGLLAILFIKPIIGIVKFFWMIISFAVQLLFYKILDWFFLI (SEQ ID NO: 712)
>orf00725 
MKIKEQTRKLAADCSKQCFEVVDRTDEVSSKHRFEVVDRTDEVSNHTYSKVKLTWFEEIF
EEYKMILLLLILYHMERD (SEQ ID NO: 713)
>orf00733
   MHSQTFQFLLMTDKTSLLHRKHRSFIRNIHSKFLILFDLLCGILSRNDSNHNPIS (SEQ ID NO: 714)
>orf00736 
MARTELPDKIETERLVLRVRTVADAEDIFDYASLPEVAYPAGFPPVKTLEDEIYYLEYIF
PERNQKENLPAGYGIVVKGTDKIVGSVDFNHRHEDDVLEIGYTLHPDYWGRGYVPEAARA
LIDLAFKDLGLHKIELTCFGYNLQSKRVAEKLDFTLEARIRDRKDAQGNCCDDLRYALLK
SEWEVI (SEQ ID NO: 715)
>or00741
   MGIKIVAIDLFNGAGGTTSGLKKSGIDVQVAVEIDSVAVKTYKLNNPEVSVIDME (SEQ ID NO: 716)
>orf00746
   LLRKQEGEYLRAENAILKKLRELRLKEEKEKEERQKLFKN (SEQ ID NO: 717)
>orf00768
   LKHLFCHFNPLWIDEIIRLAYKDQDTKDVKSKVKIGN (SEQ ID NO: 718)
>orf00792 
LCCNRHIANLDLEFISYYLGQVGFDTRISTGLGIFVTKIGNVLFDTDNQFASFLNVCDTS
ISLDWFGSSKAEKANQ (SEQ ID NO: 719)
>orf00817 
MKTKEQTRKLASGCSKHCFEVVDGTDVVSSKHCFEVVDRTDEVSNHTHGKATLTWFEEIF
EEY (SEQ ID NO: 720)
>orf00819
   MDFFFMNEVKEQVLFRDNHSEHIFWIEGVSDFMIKVNTALW (SEQ ID NO: 721)
>orf00839 
MEELVTLDCLFIDGTKIEANANKYSFVWKKTTEKFSAKLQEQIQVYFQEEITPLLIKYAM
FDKKQKRGYKQSAKNLANWHYNDKEDSYIHPDGWCYRFHHIKYQKTQTDFQQEIKVYYAD
EPESAPQKGLYMNERYQNLKAKECQALLSPQDRQIFAQRKIDVEPVFGQIKACLGYKRCN
 LRGKRQVRIDMGLVLMANNLLKHSEMK (SEQ ID NO: 722)
>orf00840 
MHIHYNTNQTTLPLEISSFLPQDHLVFTIEKVVNTLEERHFYAFYHAFGRPSYHPKMLVS
TLLFAYSQGIFSGRKIEKWKS (SEQ ID NO: 723)
>orf00843 
LRLWVIFVIMKVIKSYNTLNDYYRKLFGEKTFKVPIDAGFDCPNRDGTVAHGGCTFCTVS
GSGDTIVAPDPPIREQFYKEIDFMHRKWPDVQKYLVYFQNFTNTHEKVEVIRERYEQAIN
EPGVVGINIGTRPDCLPDETIEYLAELSECMHVTFELGLQTTYEATSDLINRAHSYEL
(SEQ ID NO: 724)
>orf00845 
VETVKRLRKYPKIEIVSHLINGLPGETHEMMVENVRRCVTDNDIQGIKLHLLHLMTNTRM
QRDYHEGRLQLMSQDEYVRVICDQLEIIPKHIVIHRITGDAPRDMLLGPMWSLKKWEVLN
SIEMEMRRRGSVQG (SEQ ID NO: 725)
>orf00853
   MKIKEQTRKLAAGCSKHCFEVVDKTDEVSHIHTVRRR (SEQ ID NO: 726)
>orf00859
   VQVCVFTNFCFFHCFSSLANCRLFNLRGICLPCISYQ (SEQ ID NO: 727)
>orf00868 
VFKKDRFSIRKIKGVVGSVFLGSLLMAPSVVDAATYHYVNKEIISQEAKDLIQTGKPDRN
EVVYGLVYQKDQLPQTGTEASVLTAFGLLTVGSLLLIYKRKKIASVFLVGAMGLVVLPSA
EAVDPVATLALASREGVVEMDGYRYVGYLSGDILKTLGLDTVLEETSAKPGEVTVVEVET
PQSTTNQEQARTENQVVETEEAPKEEAPKTEESPKEEPKSEVKPTDDTLPKVEEGKEDSA
EPAPVEEVGGEVESKSEEKVAVKPESQPSDKPAEESKVEQAGEPVAPREDEKAPVEPEKQ
PEAPEEEKAVEETPKQEDTQPEVVETKDEAANQPVEEPKVETPAVEKQTEPTEEPKVEQV
GEPVEPREDEKAPVSPEKQPEAPEEEKTAEETPKQEDKIKGIGTKEPVDKSELNNQIDKA
SSVSPTDYSTASYNALGPVLETAKGVYASEPVKQPEVNSETKAEKVAANTDAKQSEVNSE
TASLKTAISGLNTDKVELENQLKIAQGKTETDFSMESWTVLSTAKNKAQEVKDNGTATQE
QINEAEKSLKTALADLSVDKTALGSAIDTATKKNKENYTNQTWAELETVLTAAKSVNTNE
SKQSEVNEAVEKLTATIEKLVELSEKPRLTLSIEKRDIDRKVTVTYTLENPANTQIKSIT
ATLKKGEEVVKDFVLTEENLKTNHLTALFEKLDYYKEYTLSTDMVYNRGNDDETESISEE
LIQLNLKKLELKDIQTVSLMKFENGQESQVTHLSDKPTDLSKLYLKVTSSTSKDAVLAVS
SIEEEIVENKKIFKIHADTPELVVRKKDGSLSKGFDYYMERVIPHDGDIYYDFKDLISAM
TSNPTGTFILGRDISSRNVKPDGNGKSYIKGEFKGKLLGTNDNVRHSIFDLEYPLFDTIK
SGVVKDIDFKHVNMVFPDSNQGDNVATIARVIKDKTKIENVNVEGYLEGRDHVAGLVNNL
EGNSEIENISFTGKIKSKGGNSITAGIAGRNILSRVKRAYVNANIEVLGSTNSSMLVAVN
GTTLNASGGWGAWGRLTESVAKGTLEIKRSGQAGGVTATVWPYGAIDKVVSYAKVTKGKE
LFGSDGDLNNNWFMQKINNIFGVQGISSGDSGNDSKFKRISEEEAKQKVASYNITAPNLM
SDSSLLVDRLNESWKNTDQFESIQDYQSQNQLIYQNLTKFTPYYNKEFIVHEGNALTPEQ
EILKTKKIKSIVGLKGTEFVVDGSDIDTIMLHFEDGSQKRYKVTSTGKFSITNLPEYQVE
DLNVVYTSEHIVHPLDSSLINNLVEELKKVELYTESTYQVLGIDKDNANKLNRTKRLFLD
ESLDAVKTQLPTFVKTMFENEWLHINGESSGAVAALRQKIMDNKTAILLALTYINRYYDV
KFSDYNIKKLMLFKPTFHGEKIDLLDRLIRLGSSGENRLKGSENAETFKQLFASETKQKD
LVTYLDYNRSLLTNYQTTGEWFKETTKDYIQFEERPSLVEEIKDAKYRVYDNLTAPYYQG
YILPLLTLKNTHLAILSNYSTMTFVSREKRPNWKNEDFDKWVKYVATAHRNHVDTWYKIL
PDNIKGKMVKENVTAVWEGLSIPGSEWVDQNAVDRKGRDYAPAREFFNLVGGPMGGWYAY
HGYGAHAGGRNRVNYEVFDVLSEYGISVFTHELTHVNDTWIYLGGYGRRENMGPEAYAQG
LFQSPVPGQPGWGALGLNMAFERKNDGDLIYNASPTQFENRKELDSYMKNYNDTLMMVDY
LEGDAVISKGKEAITKWFKKVEPKVVSQTAQYDTVRQLTAEEKEKLSVSSVDDLVDQGLM
SDRAVGNNTYNPADFETSYIAIDYMTGIYGGGKNSVGSPGALMFKHNTFRMWGYYGFEEG
VLGYASNKFKQASRDEGHAGLSDNFIISKISKGEFLTMEAFKKGYFKKVVEELKTKGIRP
VTINQKTYSTFEELQEGFKQAVERDLKKNQLDERETRNFKFQVFRQLLQQTDSFKTSIFR
(SEQ ID NO: 728)
>orf00883 
VGNRIFIAFLQKLGLLDNLTGIREKLHPITGQGNTLGIADKDFNAHFIFQISHCIGETGL
SDKELLGCLIHRASFDDFDNIM (SEQ ID NO: 729)
>orf00892 
MKTKKHRLLALALISSFTLLGAASAAVQYPDGGVWTYGEGSGGGWAFSNYYHGKKYHYSS
LVSRWNSHSDKGEASAGKTSYAWIWTKWGEQVAFYCDYD (SEQ ID NO: 730)
>orf00903 
MSMIEVSHLSKSFGDKIALNDISFTVKEGQIFGFLGPSGSGKTTTINILTGQLLADKGQS
IILGQKSQNLTSGELKRIGLVSDTSGFYEKMSLYNNLLFYSKFYNISKLRVDNLLKRVGL
YDSCKMVAGKLSTGMRQRMLLARALINKPAVLFLDEPTSGLDPTTSRTIHELILELKTAG
TTIFLTTHDMNEATLLCDYVALLNKGKLVEQGAPSELIQRYNKDKKIKVTDYNGNQITFD
FTSLEQVSQADLENIFSIHSCEPTLEDIFITLTGGKLNA (SEQ ID NO: 731)
>orf00911 
LISNKVDITLANFTVTDERKKQVDFALPYMKVSLGVVSPKTGLITDVKQLEGKTLIVTKG
TTAETYFEKNHPEIKLQKYDQYSDSYQALLDGRGDAFSTDNTEVLAWALENKGFEVGITS
LGDPDTIAAAVQKGNQELLDFINKDIEKLGKENFFHKAYEKTLHPTYGDAAKADDLVVEG
GH (SEQ ID NO: 732)
>orf00912 
MKLFKPLLTVLALAFALIFITACSSGGNAGSSSGKTTAKARTIDEIKKSGELRIAVFGDK
KPFGYVDNDGSYQGYATILN (SEQ ID NO: 733)
>orf00946
   MTGKKGFLFLNCHICMVTTTTCFLKERVESELLIFFTFC (SEQ ID NO: 734)
>orf00948 
MDTPDENGYVADDYRITYLEAHIKAMRDAIYQDGVDLLGYTTWSCIDPVSAGTGEMNKRY
GFIYVDRDNVGNGTLKRSKKKSFYWYMSFIAMV (SEQ ID NO: 735)
>orf00953
   LSCQIAFCLIDRLDYPIMFSKVCQENHFQVFTPFSKKLKNFLKNA (SEQ ID NO: 736
>orf00966
   MFLGMIGNISIILQFFGITIIVKIDNQARAIDFFKHDKSSF (SEQ ID NO: 737)
>orf00968
   MFSLNFFDDNVFLSIKIAHKGCFQLLDMTNPNFFNKFFLAQASDQLLHFLSWNIEL (SEQ ID NO: 738)
>orf00978 
MTEPDFWNDNIAAQKMSQELNELKNTYNTFHKMEELQDEVEILLDFLAEDESVHDELVAQ
LAELDKIMTSYEMTLLLSEPYDHNNAILEIHPGSGGTEAQDWGDMLLRMYTRYGNAKGFK
VEVLDYQAGDEAGIKSVTLSFEGPNAYGLLKSEMGVHRLVRISPFDSAKRRHTSFTSVEV
MPELDDTIEVEIREDDIKMDTFRSGGAGGQNVNKVSTGVRLTYIPTGIVVQSTVDRTQYG
NRDRAMKMLQAKLYQMEQEKKAAEVDSLKGEKKEITWGSQIRSYVFTPYTMVKDHRTSFE
VAQVDKVMDGDLDGFIDAYLKWRIS (SEQ ID NO: 739)
>orf01011 
MQVIKRNGEIAEFNPDKIYQAILKAAQTVYVLTDDLRQNLAQVTKKVVLDLQEAKVERAT
ISMIQSMVEHRLLGAGYITIAEHYISYRLQRDLERSGYGDHIAVHLHFEQIR
(SEQ ID NO: 740)
>orf01015 
MKIKEQTRKLAAGCSKHCFEVVDETDEVSSKHCFEVVDETDEVSNHTYGKAKLMRFEEIF
EEY (SEQ ID NO: 741)
>orf01068 
MKVINQTLLEKVIIERSRSSHKGDYGRLLLLGGTYPYGGAIIMAALAAVKSGAGLVTVGT
DRENIPALHSHLPEPMAFSLQDQQLLKEQLEKAEVVLLGPGLRDDASGENLVKQVFVNLS
QNQILIVDGGALTILARTSLSFPSSQLILAPHQKEWEKLSGITIEKQKEDATASVLTSFP
QGTILVEKGPATRIWEVGQSDYYQLQVGGPYQATGGMGDTLAGMIAGFVGQFRQASLYER
VAVATHLHSAIAQELSQENYVVLPTEISRYLPKIMKIICQQERGSKDKLV
(SEQ ID NO: 742)
>orf01077 
VLDSKEELKESENDAPKLETPLREEPRLAPQTLPEASEVLENKREESKVEIIEPAQADDI
RKVVGELAKDISITKLYMTGHSLGCYLAQIAAVEAYQKYPDFYNHVLRKVTTFSAPKVIT
SRTVWDAKNGF (SEQ ID NO: 743)
>orf01091
   LSYSILICLCNSTINESLRAFYCWQKFITFNQVTGNARGKGTTCTSIGPDN (SEQ ID NO: 744)
>orf01094
   MGRKPRTRPEERTELERLQAENEYLRAENAILKKLRELRLKEEKEKEERQKLFKN (SEQ ID NO: 745)
>orf01096
   LSTCWNGKFCHICVALFHCFRAFKLALNEILCLLTNVSFIFVSVAF (SEQ ID NO: 746)
>orf01109 
VVLSTSAILVACGKTDKEADAPTTFSYVYAVDPASLGYSIATRTSRTDVIGNVIDGLMEN
DKYGNVAPSQKDYDLNSTGWAPSYQDPASYLNIMDPKSGSAMKHLGITKGKDKDVVAKPG
LDKYKKLLEDAVSEITDLEKRYEKYAKAQAWSTDSSLLMPTASSGGFPVVSNVVPFSKPY
SQVGIKGEPYIFKGMKLQKDIVTTKEYNEVFKKWQKEKLESNSKYQKELEKYIK
(SEQ ID NO: 747).
>orf01113
   LNFDFFIFLAHFIPLFTFSILQENPKTSKKKLYIRLL (SEQ ID NO: 748)
>orf01119
   MGFSMKLIHDLNTHTTHSTAKMLYNVKAIKNDFSIRE (SEQ ID NO: 749)
>orf01134 
LIRIIRNIYRSGEGNTSVFQSFIDQINSNQFCYGSNFDRLRCILLIENFTSICLNSNRMF
SGNGKILSNSSRSTP (SEQ ID NO: 750)
>orf01137 
MNATDIKNTYLKYIKENAVFNDVTDTHTEVITPFIDPLGEAIGFSIKSNGKHLTVTDDGY
TIWNLSINNIDVTKKGRRQDIFNSLLHFNGFDLHDGAIERTTGKEHLGQVIHDMTQLLMN
VYDFIQLTPNNIKSQFLDDVKSYFMKNEHYTVFPAFSIAGKSRLEHRFNFVFMSKGISKI
ARVHNNITKQQVDTILASWLDTSEYRRKEYGDTEQLYIIVSDEGYNNIKDDHQIALQEYG
INILNFSDKEQLEIQLGK (SEQ ID NO: 751)
>orf01138 
MSKVVKVTGAEVVISHNEEYLKVNPSELNFVPKLGDEVEVHKVDGEIIVIKVKDKKDDKI
NINIVNENNAMQNQSQVVHTQEIATGVHYVNKWVYVILALFLGGLGIHHFYAGYNGKGFL
FLILSLTGIPAIIALFQGIIALFKKPDVYGRIAV (SEQ ID NO: 752)
>orf01149 
MKIKEQTRKLAAGCSKHCFEVVDRTDEVSSKHGFEVVDETDEVSSKHGFEVVDETDEVSN
RTTVRRR (SEQ ID NO: 753)
>orf01156
   LQbIDKNHKLFDNYTCQKEKDVLQCKQVI<RKEERSYDVGTRIYTIYYFLLF (SEQ ID NO: 754)
>orf01158
   VDRTDEVSSKHGFEVVDETDEVSNHTYGKVKLTWFEEIFEEY (SEQ ID NO: 755)
>orf01159 
LFFKDEKQALYTKPKTKSSSFRASKVSNQTIVATTRTDCQVIALNLCDKLENGVVVVVQT
THHIGIDDVIYSKIFQHLTHSIKMSLAFFIKKVQDRRRILYCHLVFFFLRVQDTKRIFLQ
ATLAILRQGLLERCQIVNQGLAVGCTALRISKSVEVQFDTLNTDFLQKMGCHSDCFHIGS
WIARAKTLNTNLVELAQAPCLWTLITEHRSHIVELAWLLHFWGEEFIFHIGTDNGRSSFW
TEGNMTVTLVIKIVHFLGYDIRCISDRATDNLVMLKNRRAHFCIVIALENLTGKALNVLP
 LSRFSR (SEQ ID NO: 756) LSRFSR (SEQ ID NO: 756)
>orf01164 
MEQIGKVFRQLRESRNISLRQATGGQFSPSMLSRFETGQSELSVEKFLFALENISASVEE
ILFLARGFQYDTDSELRKEITDVLEPKNIAPLEDLYRREYQKHAHSHNKQKHILNAIMIK
SYMKSIDERVELTAEEGKVLHDYLFSTEIWGIYELNLFSVSSPFLSVSLFTRYVREMVRK
SDFLMEMSGNRNLFHTILLNGFLASIECEEFTNAYYFKRVIEEHFYKENETYFRIVYLWA
EGLLDSKQGRVKEGQKKMEDAVCIFEMLGCNKSAEYYRNTTEC (SEQ ID NO: 757)
>orf01165
   LIPYFLHFIIFFRKFIKNLPNCQNYEKIEDIYHVEGLL (SEQ ID NO: 758)
>orf01167
   MKIKGQTRKLAAGCSKHCFEVMDRTDEVSSKCYCFEVVDRTDEVSNHTYGKATLT (SEQ ID NO: 759)
>orf01170 
VVPFSDTFKDRNQVDIFTIKISRCNSSTIGENSWDIHISNSNHRSRHVLVTATDSDEGIH
VVTTHSRLDGVRDDVTRC (SEQ ID NO: 760)
>orf01190 
MKKVKLGEVLSLKKGKKATVLAEQTTLSQRYIQIDDLRNNNNLKFTESLNMTEALPDDIL
IAWDGANAGTVGYGLSGAVGSTITVLKKNERYKEKIISDYLGVFLESKSQYLRDHSTGAT
IPHLNKNILLDLQLELLGIEEQENIICILNTIKRLITKRKFQLDELNLLVKSRFNEMFEE
YPDSVFLDTYIKELRAGKSLAGEENNKNKVLKTGAVSYDYFNSSEVKNLPIDYIPLDEHK
VEIGDVIISRMNTSELVGAAGYVWAINSDNIYLPDRLWKVILNDRVNPVFLWKLITNEKT
KLKIKRISSGTSGSMKNISKSQLLQIRVPFPPLALQNEFADFVALVDKSQLAIQKSLEEL
ETLKKSLMQEYFG (SEQ ID NO: 761)
>orf01199 
MKIKEQTRKLAAGCSKHCFEVVDKTDEVSSKHGFEVVDETDEVSNHTYGKATLTRIEEIF
EEYKSS (SEQ ID NO: 762)
>orf01226
   MRLSIQLIHDLNTHTTHSTAKMLYNVKAIKNDFSIRE (SEQ ID NO: 763)
>orf01237
   LVYAPFSFNILLDYITFDFKILLFSVFLAINRFHNDFIQFLL (SEQ ID NO: 764)
>orf01240 
LGTKIGISKNTIGNYEKRVKSTKKNTIFDLAKVFSSLIDALFPPVQKDSPSDIQSIYDQR
APPRQGKVLTYA (SEQ ID NO: 765)
>orf01246 
LFVFILIFLKSSIYIGIFWFIDFGKAVDFQGWKVLFEFFMVVIDQFSFGCNPGVVFILPG
IALGQQSIDTRICDTVDNAESEQKLTIGMTGVVIDKACKLDCLALKFIWIVVDSLHDFHI
VFISDLNTILG (SEQ ID NO: 766)
>orf01247
   MTVGFDLLHPDIQLNNCQDKGKHHGDIGQIGIVHVDVLV (SEQ ID NO: 777)
>or01249 
MEGVAKGRIGRKKNNGIDNRCCHKKRNGRVTWNLFFQKTIDDGDDSTFTRREKYTDKGPK
KDSPPTISREKMINLVRCDINFNQP (SEQ ID NO: 778)
>orf01262 
VVIGVASATTNIWIIFLSGFTAILAGAFSMAGGEYVSVSTPKDTEEAAVSREKLLLDQDR
ELAKKSLYAAYIQNGEFKTSAQLLTNKIFLKNPLKALVEEKYGIEYEEFTNPWHAAISSF
VAFFLRSLPPMLSVTIFPSDYRIPATVLIVGVALLLTGYTSARLGKAPTKTAMIRNLAIG
LLTMGVTFLLGQLFSI (SEQ ID NO: 779)
>orf01277 MILMTKNINLTNEELELIQGGADPYGKEPNGYYPWKMEPVLTLLVHGFCPRDTDDLGYIG GGNHLCKGSAARF (SEQ ID NO: 780)
>orf01282
   LQVGQANEIGDPGAHFTQWNLLDDIGFDQLIQPNQKQYNDCHYCSFFHDFLF (SEQ ID NO: 781)
>orf01301 
LLHICIGETFDCIPYCMLTFFLSKSIGLTILLHKVKTVVFIDDQSNDKTCKICIHISFFR
IKLSQQCQLSFSVYF (SEQ ID NO: 782)
>orf01309 
MTTIFLTRTSCSNCGKQSTFERFDRVYAAKTPEIISAILDWDFFKFTCHNCNHKVLIDYP
TVVVDEEQKTIIQYCADGNVDVLSMQICSLISEGVNLSEYRIRVVSDIESFVEKVQIVSV
GYDDRAIELMKYMNSPLEDGDIQFNYEHMVFTKVGHENYQFMFINNQIAVASLDFSQEQY
EYYLADVEDLETNTYYIDSRWAESFFRTSLA (SEQ ID NO: 783)
>orf01310 
MVRRNSKITRQQKKIRDAFVSERTVEIIPAKREFTDVKTKKLRVAAYCRVSTFDESQSGS
FELQKQTYTERINSNPDWIMAGIYADQGASGTSIKRREQFQQMLHDCRCGKIDLIIVKSV
SRFARNQLDFISIYRELKALSPPVGIYIEDINLNTLDTNSEFILGIMAIVAQGESEQKSA
SITWSVIERFKRGIPMIPTHNLLGYTKDQYGRVVIDETEAKIVRLIYDSYIEGMTASEIA
STLMTNHIPTVTGLERWTSLAVYNILRNEKYKGEIIMQKTYTVDCFSHKTRKNNGEKPKY
RLKNGIPSIIPESRWDLVQELLKQPRRKSKSTSEIFVPKLYIKKLKSGKLRDFVVLDPSW
KSEDIHEVFK (SEQ ID NO: 784)
>orf01311 
MTVNKNFVTFSKGIVQALEYPAHVLVAFNKDTKVMGIQVCRAKTRGAFSFSKPVGEQKGI
VQVGHKTLKETLLTIMSEWKSDKRYRVEGIHIPEDKAFVFELKDFDELSDFRKNDNR
(SEQ ID NO: 785)
>orf01313 
MEKYNNWKLKFYTIWAGQAVSLITSAILQMAIIFYLTEKTGSAMVLSMASLLGFLPYAVF
GPAIGVLVDRHDRKKIMIGADLIIAAAGSVLTIVAFYMELPVWMVMIVLFIRSIGTAFHT
PALNAVTPLLVPEEQLTKCAGYSQSLQSISYIVSPAVAALLYSVWELNAIIAIDVLGAVI
ASITVAIVRIPKLGDRVQSLDPNFIREMQEGMAVLRQNKGLFALLLVGTLYMFVYMPINA
LFPLISMDYFNGTPVHISITEISFASGMLIGGLLLGLFGNYQKRILLITASIFMMGISLT
ISGLLPQSGFFIFVVCCAIMGLSVPFYSGVQTALFQEKIKPEYLGRVFSLTGSIMSLAMP
IGLILSALFADRIGVNHWFLLSGTLIICIAIVCPMINEIRKLDLK
(SEQ ID NO: 786)
>orf01315 
MELILKAKDISVEFKGHDVLDINELEVYDYDRIGLVGANGAGKSTLFKVLLGELIPPGCK
MNHLGELAYIPQLDEVTLQEEKDFALVGKLGVEQLNIQTMSGGEETRLKIAQALSAQVHG
ILADEPTSHLDREGIDFLIGQLKYFTGALLVISHDRYFLDEIVDKIWELKDGKITEYWGN
YSDYLRQKEEERKRQAAEYEQFIAERARLERAAEEKRKQARKIEQKAKGSSKKKSTEGGG
RLAHQKSIGSKEKKMHNAAKSLENRIAALGKVEAPEGIRRIRFRQSKALELHNPYPIVGA
EINKVFGDKALFENASFQIPLGAKVALTGGNGTGKTTLIQMILNHEEGISISPKAKIGYF
AQNGYKYNSNQNVMEFMQKDCDYNISEIRSVLASMGFKQNDIGKSLSVLSGGEIIKLLLA
KMLMGRYNILIMDEPSNFLDIPSLEALEILMKEYTGTIVFITHDKRLLENVADVVYEIRD
KKIKLKH (SEQ ID NO: 787)
>orf01316 
MNQLEFQRNHLQMDYYSESYQDFERDFYRYSNMNIPLTFLTDDILKTMATSRKNYFVLNK
EKSRDNRDHFFIFEVRTLEENPLIYHYTYKKTTTYLAEK (SEQ ID NO: 788)
>orf01317 
MQKWMGFFLSEHTSALTDDANKVTYMSDLSLEKKLLLLSQVYAGQLNTRIHVVKKNNQVS
YTGTIPSLTKDFILIKTTTGHINLKLKDIVSIELVEEVLYESA (SEQ ID NO: 789)
>orf01318 
MKKSINAQKKIDPANLPKTMVGHVLELFRKKYTSGAVRQIGVSYGGFVDENFTLLSLFDD
VEQIEKENRLQTAIDVVREQFGFLAIQKGTVLTEGSRNIERSKLIGGHSAGGLEGLK
(SEQ ID NO: 790)
>orf01343
   MNXXFISTKDKHTLIQVSAVRFRDGREIDAYDSYVHTSVPLKSFINEFDRGLQLRP (SEQ ID NO: 791)
>orf01363
   MIAMRSYITLTCNLNNNLFCLNSFFLTNLVWSQIFSLLSVFITVYI (SEQ ID NO: 792)
>orf01364 
MVFDANRIISEDSEGFVIPHGDHNHYIKVQTKGYEAALKNKIPSLQSNYQPGTFDEKAVL
AKVDQLLADSRSIYKDRLS (SEQ ID NO: 793)
>orf01390 
MARLEPAKIAKIVLGILLYIIDLIKSSFVLPIPKAAKKSLILISFVPSFNDKNIVIRRPR
QITKIMPRFICFLFRIFACIS (SEQ ID NO: 794)
>orf01396 
MASKRLSIEEQIEKKEESIKQLQNQKRQLKKKLNEQERKARNKRLIEKGAVFESIFEESI
DLTKDEFYKLIKTLNDEEIRLNIMEILEERIDDNVEKSSKDEIT (SEQ ID NO: 795)
>orf01397 
MADSFHFSVNIISRGKGKSAVASAAYISGEKIKNEWDGVTHDYTRKEKILVKNIILPDHI
PKEFNDRSTLWNKVEMAEKNSNAQLARQFIIGLPKELSLSENKNLVERYIKENLTSQGMI
VDYAIHDESQDKNGNIHCHIMTIMRPINEKGEFLAKSKKEYILDEKGEKVLNKNGKPKTR
KVELTTWNDTGNVEKWRENFSDLCNKYLERAGAEKRVDHRVLKDKIQIIYRQSI
(SEQ ID NO: 796)
>orf01398 
MERKGIETDKGNYNREIRKYNQLVKTIKEEIKTLKGWIGNLLDNLSTAYEKFKDIERDKV
IDNPKLFNLTNYLLTYSEIQKEKSKYLKGYAKTNKEKYDFKKLTSAYSYLRKNNIETIGQ
LQTKIETLKSNSYRLNKKAKTIHKEMEDVEKKILYYEIYKAKKEVYEEYQKKNIFTKEAF
YNKHKKDIDQYKVVSGKLKKLLSDKEKLSPKKWNEEKILLMSNLEEINKEKDKIKDEYQE
INHIKYSVDFVNKELGIDLSIEIDKLIKQGEKPSVIAQIKKFQDQVNKDNEYREMMKNKK
MDQER (SEQ ID NO: 797)
>orf01407 
MELSAIYHRPESEYAYLYKDKKLHIRIRTKKGDIESINLHYGDPFIFMEEFYQDTKEMVK
ITSGTLFDHWQVEVSVDFARIQYLFELRDTEGQNILYGDKGCVENSLENLHAIGNGFKLP
YLHEIDACKVPDWVSNTVWYQIFPERFANGNALLNPEGTLDWDSSVTPKSDDFFGGDLQG
IIDHMDYLQDLGITGLYLCPIFESTSNHKYNTTDYFEIDRHFGDKETFRELVDQAHHRGM
KVMLDAVFNHIGSQSLQWKNVVKNGEQSAYKDWFHIQQFPVTTEKLVNKRDLPYHVFGFE
DYMPKLNTANPEVKNYLLKVATYWIEEFNIDAWRLDVANEIDHQFWKDFRKAVLAKNPDL
YILGEVWHTSQHWLNGDEFHAVMNYPLSDSIKDYFLRGIKKTDQFIDEIN
(SEQ ID NO: 798)
>orf01408 
MFNLLDSHDTERILWTANEDVQLVKSALAFFFLQKGTPCIYYGTELALTGGPDPDCRRCM
PWERVSSDNDMLNFMKRLIKIRKYASVIISHGKYSLQEIKSDLVALEWKYEGRILKVIFN
QSTEDYLLEKEAVALASNCQELENQLVISPDGFVIF (SEQ ID NO: 799)
>of01414 
MSEQYRDIRKEVNLTADELKQIEKMMEVDNYRHFSPFVRDKILMTDDKQLAAKEWFSLWQ
SQKFEQISRDVHLVLIIARENHQVTQEHVSILLTCVQELIAEVNQVQSLSRGFREKYMR
(SEQ ID NO: 800)
>orf01415 
MVYRYRTNLKKVFLTDSELHQLNERIAKSNCQNFSVYARKVLLNPNMSFVTINTDTYDQL
VFELRRIGNNINQIARAINQSRLISQEQLQELSKGVGELIKEVDKEFQVEVKRLKEFHGS
H (SEQ ID NO: 801)
>orf01417 
MVVTKHFATHGKKYRRRLIKYILNPDKTDNLKLVSDFGMSNYLDFPSYEEMVEMYNVNFT
NNDKLYEYRNDRQEKHQQNIHAHHLIQSFSPEDNLTPEEINRIGYETIMELTGGRFRFIV
 
ATHTDKDHIHNHILINAIDCNSDKKLIWNYALERNLRMISDRISKMAGAKIIEKRFSYRD
YQKYRATSHKFELKQRLYFLMQQSKSFDDFLEKAEQLHVHIDFSQKHSRFMMTDRAMTKP
IRGRQLSKRDLYDEDFFRMHFTKQEIASRLEFLLNCVNSLEGLLTKSKELNLTIDLKQKN
VIFILEENGKQFSLSHKKISDEKLYDVNFFQDYFKNKEVGVSEGIENLQAQYRAFQEERD
KEKVSTEEIEEAFETFKEKRDAVHEFEVKLTEHQIEKLVDEGIYIKVSFGINQSGLIFIP
NYQLDIMEEENQKKYKVYIRETTSYFVYNKEHSDKNQYIKGRTLIRQLTNDSRVIPYRRP
TVERLQEKISEISLLIELTETDKKYQDIKDNLVSEIAELDIKLTQTNEKIATLNKMAEVL
INSKSEGSGSQKLARHEFSKLNMTESTTLEQVNEELLKLQQEFGNVLDEYEKTIRKLGQL
FKVFDECINKEIMNEI (SEQ ID NO: 802)
>orf01419 
MVCLIIDVSPYSTLCDIVVPKTHFLRQLMELCDFSFIYDELEKNYQPDFGCRSYSLLIMM
FKYLLLKDIYKLSDVDVVERSFSGMTFKYFLGLAPVIEPSSLTKFRKLRNKDERLLDLLI
AKSVQIAIELGLIKSNILIVDATHTKVHYNHKKPQEVLRERSKALRKTIYQYSEYIKAEF
PSKPQEDTLVAELRYTQEVISVLEKHDELTGIPAISQNSITLKKL (SEQ ID NO: 803)
>orf01420 
LESSVKEEARIGHKSADSSFYGYKEHFAMTDERIITACVVTSGEKSDGPVLEELYHKSKD
NGVTIEAIVGDRAYSGKDNMQFTKKERVH (SEQ ID NO: 804)
>orf01421
   MSVFKFRIFGFYLVAMFGLFFKIGRFLKPLLENMFIALKGYQISLRLSPFFITAHF (SEQ ID NO: 805)
>orf01425 
MQEHYTPKGKHLTIDNRRLIERWKNENKSNREIAGLLGKAPQTIHNEVKRGTTLQQVRKG
LYKKVYSADYAQTVYQFNRKRSVKKLILTKEIREKILHYHKQKFSPEMMVNKKQVKVGIS
TIYYWFHNGHLGLTKADMLYPRKRKGVKKQASPNFKPAGKSIEERPDVINLRLENGHYEI
DTVLLTKIKNYCLLVLTDRRSRHQIIRLIPNKTAESVNQALTLLLGEHRILSITADNGSE
FKRLSEVFPEEHIYYAHAYSSWERGSNENHNRLIRRWLPKGTKKTTPKEVAFIENWINNY
PKKCLDYKSPSEFLLGG (SEQ ID NO: 806)
>orf01426 
MLHPIFIIRRSWDGIFHLSEWKRNEEFDFFNKMDVPYLSMSSRIEVTQAINFHKKHSISL
YAIISWCVMSAINSIPELLMDTDGKIVWQYNQRGCSFTTLTSEDKLNFSSFTMGDNLIEF
VSAFNINKQKAEEGQKPNIDKNNIAYLSCVPWIDFLHVSTPMNLSKIDTVPRITWGKVIQ
ENQRYFCTVNLQINHGMGDGLHVSNFFVLLQRFVNKINEYFQKK
(SEQ ID NO: 807)
>orf01428 
MSIFIGGAWPYANGSLHIGHAAALLPGDILARYYRQKGEEVLYVSGSDCNGTPISIRAKK
ENKSVKEIADFYHKEFKETFEKLGFTYDLYSRTDSPLHHEIVQELFLQLYEKKFLYTKKI
KQLYCTFDNQFLPDRFVEGKCPNCGTHSRGDQCDNCSAILDPIDLVDKRCSICSNEPEVR
ETEHFYYVFSEFQNLLETYLNDAEETVRWRKNAINLTKRYLREGLPDRAVTRDLPNGIPV
PIDGFRDKKIYVWFEAVAGYYTASVDWAQKLQNNITDFWNNRTKSYYVHGKDNIPFHTII
WPAILSGLEIEPLPEYIISSEYLTLENKKISTSNNWAIWLNDIIKKYDADSIRYFLTINA
PEMKDANFSWREFIYSHNSELLGSYGNFINRTLKFIEKYFESEIPTKYLEGEILYNLKEL
YTTVGNLVESGHMKQALEEIFEYIRSANKFYDDMKPWALRESDIEKCKEVLATCVIIILN
LGQMLNPFIPFSGKKIEDMFKTKLNTWNYISNLPNKLSDVSMLFDRIDLKKIDEEVLELQ
QTSSR (SEQ ID NO: 808)
>orf01429 
LNNLTLLKEYNFRDLGNHLTQTGQKIKPKTLFRSSKLFGISKIDVDLLQSYGITKVIDFR
SANEIKKAPDPDIKNIKNIVIPIFYNDDSELTEFPIEFFNKSDAGFQHMIKTYDQMINQK
QSKLGYKKFFKLLLSHPKDESLLFHCSMGKDRTGIASLFLLYILGVDMNDIFHDYLLSNK
YLINVRKENIEYVNNHSGNVILMHNLLSLSSAKEEYINRVLNVLDKEYGGILRYINTELG
ISSQEIEELKDRYLF (SEQ ID NO: 809)
>orf01431 
MDFLNEVLDLKEFIQDPVRTLSLGQRMRADIAASLLHNPKVLFLDEPTIGLDVSVKDNIR
RAITQINQEEETTILLTTHDLGDIEQLCDRIFMIDKGREIFDGTVNQLKKTFGKMKTLSF
ELHPGQDYIVSHFEGLSDIYVTRQELSLDIQYDSSQYQTADIIQQTLSDFTIRDLKMTDA
NIEDIIRRFYRKEL (SEQ ID NO: 810)
>orf01432 
MTKLWKRYKPFVSAGIQELITYRVNFFLYRIGDVMGAFVAFYLWKAVFDSSHQSLIQGFT
LSDMTLYIIMSFVTNLLTKSDSSFMIGWEVKDGSIIMRLLRPVHFAMSYLFTEIGSRWLV
FVSVGLPFVILIAGLKLLSGESFLQIVLITTVYLLSLILAFLINFFSIFALVFQLLCLKT
YGDQIF (SEQ ID NO: 811)
>orf01433 
MKKYQRMHLIFIRQYLKQIMEYKADFLVGVVGVFLTQGLNMLFLNILFQHIPLLDGWSFH
QVAFIYGFSLIPKGIDHLFFDNLWALGQHLIRKGEFDKYLTRPISPLFHILVETFQIDAL
GELLVGVLLLLMTITSLTWTWAKVFLFLISIPFATLIYTSLKIVTASIAFWTKQSGAIIY
IFYMFNDFAKYPIAIYHSFLRWLISFIIPFAFTAYYPASYFLKDKDGLFNIGGLILISLI
FFTLSLKLWNKGLDAYESAGS (SEQ ID NO: 812)
>orf01434
   MIELAEPLPEYEILLSIPGIAETTATSIIGELETFVAFSLPTKSMPLSVLTSDTMNLAIS (SEQ ID NO: 813)
>orf01435 
MLGWKDGHEVPILFPCRSREKVLYFWKGNLKHLVQAILSPNDVFCQILIESTEVTQILID
FFLNISWFAVKDEL (SEQ ID NO: 814)
>orf01437
   MMRTVFRMDVSKASSEVAILVNGEKVHGYTMPNDAIGFSRLLEDLK (SEQ ID NO: 815)
>orf01438 
MKKNNVEIIKADSLVRRRGDNVERHLKRVAAYCRVSSDSEDQKNSYDSQVRHYKEYISQR
SDWELADIYADEGISGTQVGKRQDFQRLINDCANGEIDYIVTKAIARFARNTLDTLKYVR
MLKDMQIGVYFEEENIDTLTMDGELLLTILSSVAQQEVENTSAHVKKGLKMKMQRGELVG
FQGCLGYDYDVETKQISINKKEAKIVRYIFERYLEGIGGKVIARELDELGYKSPRGLEHW
NDTTVLGIIKNEKYKGDILMGKTFTVDPISKRRLSNFGEEDKYYIKDNHEPIISKEDFEK
AQEIRLRRAGNKKTAANVNGKRERYSKMYAFSSMLECGFCGSILSRRSWHCRSDYRKVVW
HCVTSIKKGKKFCKHSKGLEEIAIEGAFLEAYRQVYHSNENLMTDLLETIESELNDNSLN
KELKRITNKLRILLKKEENLVNLRLEGKVSDSIYNEKYNEISSEKEFLAEEKVNIETTLK
SEIDVKKRLTEFKHLLSSQKMLTEFDRAVFESIVEKIIVGGVNSNGEIDPAMLTIIFKTG
EIQNKDGKQFKSKRKNAKLETDKLCPQNSDEDKKLYSQGTDNTRGVCSVAGSILASQ
(SEQ ID NO: 816)
>orf01439 
MGGNPPMKKYSIVDKIVLSTKIKRIIIFTVFRENWEPYMKKYTEVFQSQFPNLNIDYLLL
DTEQIDLDSYLDADIIIIGGNTEKYIATYVNQEFKSYIDHMLNKEAKIIGFSAGALLLG
EKVYVSPNDNSDHQIKIKNGLGLFSQFLISVHYDSWNDKANKDRAEELVNVPIIPLNDHS
CLVLDKLGNIIEKID (SEQ ID NO: 817)
>orf01440 
MDDEASKQLSDSRFKILVGVQRTTFEEMLAVLKTAYQRKRAKGGRKTKLSLDDLLMVTIQ
YMRE (SEQ ID NO: 818)
>orf01457
   MGFSMKLIHDLDTHTTHSTAKMLYNVKAIKNDFSIRE (SEQ ID NO: 819)
>orf01492
   LILNEYEKRIFHEKTHNIECFDTCYYAFFIIFAPFLAFVIDKHCSSSLFLER (SEQ ID NO: 820)
>orf01520 
MSQVKGLCVLDVDGTLILEEVIDLLGREAGHEAEISQITSRAMRGELVFESSLRKRVSLL
EGLPILVFDNVFNSIHLSLNVPEFISILQKNGILVGLVPGGFTPIVGEISKIPWYCLFHC
QPA (SEQ ID NO: 821)
>or01521
   MLKSAELGIAFCSKEMLKKEIPHHVDKRDFLEVLPLIDCLE (SEQ ID NO: 822)
>orf01527
   MGFSMKLIHDLNTHTTHSTAKMLYNVKAIKNDFSIRE (SEQ ID NO: 823)
>orf01537 
MDKLIIFIEKGKPFFEKLSRNIYLRAIKDGFISSMPAVLFSSIFILIAAVPNIFGFKWSD
EQLAFILKPYNYSMGILALLVAGTTAKSLTDSVNTRSMEKTNQINYMSTFLAAVVGLLIL
AADPIEGGFANGLLGTRGLLTAFLAAFITVNIYKVCIKNNVTIRLPEEVPPNIAQVFKDV
IPFALSVLSIYGLDLIVRNIFGTNVAESVGKILAPLFSATDGYIGLAIVFGAYAFFWFVG
IHGPSVVEPLIVAISYANIEANVQLVQAGMHADKILNPVTQTFVVTMGGTGATLVVPFMF
MWLCKSKRNRIVGRASVVPTFFGVNEPILFGAPIVLNPIFFIPFVTAPIINVWIMKFFVD
VLQMNSFSIILPWTTPAPIGIVMGTALAPLSFVLAITLIIIDTLIYYPFVKVYDHQILEE
ERKGNSSSELKEKVAANFNTVKADAILEKAGVDAAQNTITEETNVLVLCAGGGTSGLLAN
ALNKAAAEYNVPVKAAAGGYGAHREMLPEFDLVILAPQVASNFEDMKAETDKLGIKLAKT
EGAQYIKLTRDGKGALAFVQEQFD (SEQ ID NO: 824)
>orf01552
   LFKTRSNSSALGSSYISNRNIFSYFTNQFNNTFCNVFGM (SEQ ID NO: 825)
>orf01557
   MALTQRQFVELFQETINVITLTCLTVSVAVVACVSICSS (SEQ ID NO: 826)
>orf01558
   MLTMFLFLPIDFFFCTDIIRMSCILKVNIVFSIYLNHITTLDFTDNILVL (SEQ ID NO: 827)
>orf01560
   LVCYFDDDLFGIDSFTLANLIRSQILRFLRRLFSIYIGNTIISLTVLA (SEQ ID NO: 828)
>orf01570
   MGFSMKLIHDLNTHTTHSTAKMLYNVKAIKNDFSIRE (SEQ ID NO: 829)
>orf01585 
MEKYFGEKQERFSFRKLSVGLVSATISSLFFMSVLASSSVDAQETAGVHYKYVADSELSS
EEKKQLVYDIPTYVENDDETYYLVYKLNSQNQLAELPNTGSKNERQALVAGASLAALGIL
IFAVSKKKVKNKTVLHLVLVAGIGNGVLVSVHALENHLLLNYNTDYELTSGEKLPLPKEI
SGYTYIGYIKEGKTTSDFEVSNQEKSAATPTKQQKVDYNVTPNFVDHPSTVQAIQEQTPV
SSTKPTEVQVVEKPFSTKLINPRKEEKQSSDSQEQLAEHKNLETKKEEKISPKEKTGVNT
LNPQDEVLSGQLNKPELLYREETIETKIDFQEETQENPDLAEGTVRVKQEGKLGKKVEIV
RIFSVNKEEVSREIVSTSTTAPSPRIVEKGTKKTQVIKEQPETGVEHKDVQSGAIVEPAI
QPELPEAVVSDKGVPEVQPALSEAVVTDKGEPAVQPELSEAVVTDKGEPAVQPELSEAVV
TDKGEPAVQPELPEAVVSDKGEPAVQPELPEAVVTDKGETEVQPESPDTVVSDKGEPKQV
APLPEYTGPQASAIVEPEQVAPLPEYTGVQAGSIVEPEKVEAPKEYTGKIEQPSAEDTKP
ENEASSTNGESERPKDKIKEEKQVDKKLELRNVSNVELYTVENNKYRHITAVDGALDSSL
KYFMKVKSENFKDIMLPVTKIESTTKNNKEVYKIVAHAENLIQHENNVISNDYTYYLPKT
QQSETGVYTSFKNLVDAMNSDPNGTFHLGATMDAREVELPDDQESYVKNEFYGKLIGENN
GKYYAIYNLKKPLFKTLNTATIQNLSIKEANVSSKEDAATISKEAKYNTLIDNVHSDGII
AGERGIGGLVSKVDNSRISNSSFTGRITNTYDTTAGYEIGGLVGKLSGSLASIEKSIASI
DIASNAKSGDQIVGGIAGVVEKSATIKYSYVEGNVNNVRHFGKVGGVAGNLWDRDSQDVS
KSGKLSYVLSDVNVTNGNAIAGYNFNGIKTIETYSNKNNKVVNVVQEDDEVVTKDSDVQR
GTVLDADKVKEKKVELVSKHSTKVEDFDFTSRYNTNYNEVTGYQQSREQVYKNIEKLLPF
YNRETIVKYGNLVEDNSDLFTKKLLSVVPMKNNEVITDINKNKQEINKLLLHFEGNKSRV
LNIAYKNDFSKVAEYDIANTKLMYTPNTMLHDYNNIVKTILNDLKSVQYSSADVRKVLDI
SGNIKLTELYLGEQFEKTKANIEDSLSKLLTADAAIVENNNKVIDNYVIEKIKNNKEALL
LGLTYLERWYNFNYGETNAKDLIMYHLDFFGKSNSSALDNVIELGKSGFNNLLAKNNVIT
YNVLLAKNYGTESLFKALEGYRKVFLPTISNNEWFKKQTKAYIVEEKSTIEEGREKQGKE
GTKYSIGVYDRLTNPSWKYQSMVLPLLTLPEEKTVFMIANISTIGFGAYDRYRSSEYPKG
EKLNKFVEDNAKEAAKRFRDHYDYWYKILDNDNKEKLYRSILVYDAFKFGTDKDKDKVTH
QATFETDHPAIKYFFGPAGNNVVHNGHGAYATGDAFYYMAYRMLDKDGAVTYTHEMTHNS
DREIYLGGYGRRSGLGPEFYAKGLLQAPDHPYDPTITINSVLKYEDSENSTRLQVADPTQ
RFNSAEDLHNYMHNMFDVIYMLEYLEGKAVANLETNQKYELLRKIENKFDLDQDGNNVYA
TNVVRRLTMDEVNKLNSFDSLIENDIITSRGYKDQEYKRNGYYTIDLFSPIYSALSGEKG
 
TPGDLMGRRIAFELLAAKGYKEGMVPYISNQYEKDAKAAGSKINSYGKEVGLVTDELVLE
KVFNGQYKTWTQFKKDMYKEREKQFSKLNRVNFINPNNPLSRQRNVSVTDIGVLERMIVE
AVRDDAQDDVAKFYPETNSRVLKLKKAIYKAYLDQTNDFRSSIFENKK
(SEQ ID NO: 830)
>orf01588 
LSLLKKDKFSIRKIKGIVGSVFLGSLLFAPSVVGASTYHYLDYSSLTQTERDQLKQGRPD
ESKESYALDYEKDALPNTGSSQSIMTALGLLAIGSLIVIITKDNRNKKIATFLIVGATGL
VTLSTASALNLNANIHESGRDGVLQISGYRYVGYLELDDKTVSSVSPASTVSPVEQPKVV
TEKGEPEVQPALPEAVVTDKGEPEVQPTLPEAVVTDKGEPEVHEKPDYTQPIGANLVEPE
VHEKLAYTESVGTTGMDENGNLIEPPVSDIPEYTESVGTTGVDENGNLIEPPVSDIPEYT
ESVGTTGVDENGNLIEPPVNDIPEYTEPISTVSEVASEREELPSLHTDIRTETIPKTTIE
ESDPSKFIGDDSVKEVGEDGERQIVTSYEELHGKKISEPVETVTILKEMKPKILVKGTKE
NPKEKTVPVLTLTKVTEDAMNRSANLNYELDNKDNAEISSIIAEIKDGDTVVKKVDLSKE
KLTDAVQNLDLFKDYKIATTMIYDRGQGSETSKLDEKTLRLELKKVEIKNISSTNLVKVN
DDGTEIPSDFMSEKPSDEDVKKMYLKITSRDNKVTRLAVDKIELVTEKEKELYKITASAQ
DLIQHVDPSKTRNEYIHYIEKPVPKVNNVYYNFNELVRDMQEHPNDEFKLGADLNATNVS
AFGKSYVTKDFKGKLLSDGDNHYTIHNLSRPLFGNVIGGTIKNINLGNVDINMPWANQVA
AVANIIKGGTTIENVKVKGNIVGKDWVSGFIDKIDNQGTLRNVAFIGNVTSVGDGGQFLT
GIVGENWKGLVERAYVNANLIGKKAKAAGIAYWTQNEGNNNTVRQEGAIKKSIAKGTIQV
TEAIESGGVVGSMKHHGSVEDSVSMMKVPNGEIFYGSSDIDYDDGYWTGDNVRRNYVVIG
VSDGHSSYQRSKDKNRIRPISEEEAKSKIEATGITADKYEINEPVVNRLNRLTRREDEYK
STQDYKVDRDLAYRNIEKLQPFYNKEWIVNQGNKLAEDSNLAKKEVLSVTGMKDGQFVTD
LSDIDHVMIHYADKTKEIKAVHQKESKVAQVREYSIDGLDDIVYTPNMVDKNRDQLIKDI
KDRLATVELISPEVRALMDKRDTSRDPNANSDERKNGYIRDLYFEESFSETKANLDKLVK
SLIENADHQLNSDEAAMKALVKKVDENKAKIVMALTYLNRYYDIKYGDMTIKNLMMFKPD
FYGKSVDLLDFLIRIGSSERNIKGDRTLDAYRDMIGGTIGKSELHGFLDYNMRLFTNDTD
LNDWFIHAAKNVYIVEPKTTNPDFVNKRHRAFDGLNNGVHNRMILPLLTLKNAHMFLIST
YNTMAYSSFEKYGKYTEAEREAFKDKIKEVAHAQQTYLDFWSRLALPSVRDQLLKSQNRV
PTPVWDNQNYHNVEGVNRMGYDKNNKPIAPIRELYGPTWRYHTTNWYMGAMASIFQDPNN
NDQVYFMGTNMISPFGISAFTHETTHVNDRMLYFGGHRHRQGTDVEAYAQGMLQTPDKSG
NGEYGALGLNMAYHRENDGDQWYNYDPDKLKTREDIDRYMRNYNDALMMLDHLEADAVIP
KLHGNISRWFKKMDRQYRKNGELHQFDKVRELTEDEKKKIVINNIDDLVNNNLMTKHGAP
SDRTYNPEDFDSAYVNINMMTGIYGGNTSQGAPGAASFKHNTFRMWGYFGYENGFISYAS
SKYQGEADKTNKKLLGDDFIIKKVSKDKFNNLEEWKKQYFKDVKSKAEKGFTAIEIDGRQ
ITNYAQLKTLFAEAVQKDIDGMSDPKIKDHFKNTVDLKSKVFKALLKNTDGFFNKLFKED
I (SEQ ID NO: 831)
>orf01603 
VLGGRANSVTSCTTNSHWNLTFTTKHVTCFSSLVDDIVHGNNREVHEGHIDDWTKSCHGC
SCCCSRDGSFRNRTVTDTFWTKFFKHSNRSTEVSSEDTDVFSHQEHIFIATHFLRHSKDN
GVTEGHCFCFHFISFSLVCVNIFKG (SEQ ID NO: 832)
>orf01604 
MDMFYIGHFLDIRRDTVTVVNAIENDWQVPDRSHVHCFVENTFIGRTISKEADNDFTGIL
HLLTEGCTDSDPHTTTYDTIGTKVPSIKVSDMHRSTFPFTGSSVFTKDFSHHSVEVNPFS
NSLPVSTVV (SEQ ID NO: 833)
>orf01606 
MNXXDFIGHCDKIKRNIFEKSHKVFSGLFGLHPKDFLNLIFSNQIPLPFSECNPLTNYNH
LFSLIISDKRDIVIHWI (SEQ ID NO: 834)
>orf01622 
LIEIQVFSSLQVCCLNLCHLKFQHFDTCLVFLLVFLDFQNLLAHFPIGIKTRLIGFFQVP
KSGITKFIQHLDMQLGTH (SEQ ID NO: 835)
>orf01623
   MVMLTMNIYKMLPNSSQNRQINHLTIYTADTTTILQDFPTDDNFIT (SEQ ID NO: 836)
>orf01624
   MTDINICRRTSSQHHIHGINDNRLPCTRFTSQDSHPLFKIEGNSLNNGKVFYRNFK (SEQ ID NO: 837)
>orf01634
   LFVIRNPSSQTLFQTQLQLVQALQITVIQALRLSKDNRLTAFFQSLLFLR (SEQ ID NO: 838)
>orf01636 
MPHTRDNWQTRFKNSSYHNFFVKGPEILNRTTSTTNNEQIQIVPLISTRNISSNFLRSPF
TLNLGRIKKDVNTWESPADGRDNISNNGSTTAGYYPNSLRKLGQSLLEAFLKQAFFCQFF
LKLFKLNRKRPNPIRLNFFNDDGVATTWFIDLYTPNHIDLHSFFQVKP
(SEQ ID NO: 839)
>orf01637
   VTLADFVADRRAATPTAAAELATPVTKVGCISSFAKSGKTDGI4GSPKCSI (SEQ ID NO: 840)
>orf01640 
LAIIRNRTCSLKLINDHLTFWTLRFLTSTRILIELATINLNCRIHRGNLSNRPSQASNRF
INKLFIQGRQNRGFCDHFPTSILSRRGIAQSDFPLIDLTLVLHKLDHACRLANRNRQNTH
HIRIQGSTMTNFLGSQNLTQFKNRIMRGHSCFLF (SEQ ID NO: 841)
>orf01642 
MEDDLNYENLMDDVTEAIKKFNLVIFIGAGVSIAQGYPNWNNYIEHLIKYWQGQVLSVSG
EKRLGREHHVVFDLISKSSISNKRKVDLVNYELKKVFGEDFEKRRLDFEKGYFKNLLPYS
IVNQTVESLASLNAIFITSNYDYEIENHIKRLKNAVVTINDLNEFTKNKNGKLQFGDVLH
IHGTPDCDVKYFVSSSADYSKTYLKNRENFENLVTWFKETKPTVLFIGAGLEEDEILSLL
CKDSKNYALMKSENTGNQRVDEHYRGVVEGFFSSENHTQIIWYGDEFEKLPLFVKKLVAD
INEKLGTHDFYNQWNNLLNPSINQEEYNKNLDSISNDFKYLSSVLDKVIENDNNQLDQLM
LNALLRSETLTVIKKNFVLVFWKFIVKNIEKLSDNEWDVIYKIIYEGSQNYFIDDVFFVY
NYAIDNKISSFTNNNKLNELREIISKDGYIVNSNFNKDKTLLGYWLVSAFEQQNRDLYIK
EDSEVEVNLNYECVNKLMSILNNPEFLSYNYYSIEHQLKEYDVVKFLYELVKSKKLFIEE
EKFLESDSEDLISTILIQKLLVQLDNEINLDLEFIKRLIDKIDFSNIHFGEELNTFIKEH
RSIIREKNIEIPKKPYRNWISSLEGGFVSQFSYLTQENLVEYDESRVLEILVNAEKEQRG
SSFLEEKTINETENFFITVLKESNEISKKVSDLLKNHIDDLYPKYKRLYVKIISFPEIEE
NLRKIVREKYLKRFNKESFDSNDRKFFEYHIKQQNTDIDIFEKLLSINVNELSTPKGDNK
QLDILHFINSEMGSYFQCLISLFINHSSYRDVIIQIINSVTDTDYREFAQGILLNEYNPN
RINVTYNTFLGFAYYHSTITIEAADVFTDVVRDILNKKIEDNQILNKVYLVALERVDPTI
ESFSLSKNNYSQMINIIFTGDYEFRYSKEWLGALFKFDSSANYLVTIFYLLYNENLKKNR
FALFIEELSDYLTTYNQKLSLRGMNYKLNHEELNNFDLLKKMFLKLMETDKIENDIFYLD
GIKSILPLLSLDDRRNVLQHIQKQNNCPPPEIEELQRIIVN
(SEQ ID NO: 842)
>orf01645 
MFMSNLCQFFQVWNINQGVTQGFNQDKLGIVFDSCFYFLQIINIDKGCCDTITRKEFFQK
IEGSTVNSRSSHYMVTSMGKRQNRISHCSHT (SEQ ID NO: 843)
>orf01646 
LINVFSHGVDIAIHSATKFIGGHGTTIGGIIVDSGRFDWMASGKFPQFVDEGSSCHNLSY
TRDVGAVAFIIAVRVQLLRDTGAALSPFNAFLLLQRLETLSLRVERHVQNAETIVDFLVN
HPKVEKVNYPKLADSPYYALAEKYLPKSVGSIFTFHVKGGEEEARKVIDNLEIFSDLANA
ADAKSLVVHPATTTHGQLSEKDLEAAGVTPN (SEQ ID NO: 844)
>orf01647
   MTCDFKFETLQLHAGQVVAPATKSRAVPIYQTTFFVFDDT (SEQ ID NO: 845)
>orf01651 
MAWLLVGNVGVRQVLEHLNAELKKVMQLSGTQNIENVKPFNSVTSIKPTLPNDPPDLKFI
DKKNAPPKCGVFLCYGKKFVLKNKKNEIRIGRIVQDSQIDF
(SEQ ID NO: 846)
>orf01656 
LKKKWFFADYYDTTIILLALISVILVLLGFAEMIDLDNPPYSIIDLVIWGVFVIDYSWRF
FITKRKWRFILENVFDLLAILPLNAIFTVFRLGRIFRLARLTKLLKLTRLLRIIGLTGKL
ERKISRFLRTNGLIYILYVNIFIVLVGSSILSVVEEKSFSDSLWWALVTVTTVGYGDIVP
 VSLFGKTNYRAKEY (SEQ ID NO: 847)
>orf01666 
VVDFKQTRQDPHDITIYSWLRQVKSNTGNGSCCVRSNPFQAGNSFIGIWKLATKVSHNLL
GCSLHIANSRIITQALPSFQ (SEQ ID NO: 848)
>orf01671 
MAERTVVQVHNAFPEDTTLINSQLIPLVQVVVNQGRKGIVGSCNSMHISSKVEVDVFHWQ
NLCIPTTSSTTLDPHDWTKRRFADSNHGFLANLVQGIRKTNGKRRLSFTCRCWVDGSNQD
QFTDWIALNCTNFIKAEFSLVLSVQLQIVVRNTKFLYNINNWLQLNTLCDFNICFHSKFL
(SEQ ID NO: 849)
>orf01684 
MLFIIGHLNFPTAGSFIDSTLHRLGNRVCIHDDMAFTVTSSTSNSLDESTFVAKETFLVS
IENSYEAHFRNVNSFTEQVNSDQDIKDTQAQVTDNLRPFQGLDIRVHVLDLDTHFLEVVG
QILCHFLGQSCDKGTLIFFNAGIDFTQEVINLSHSRTDFHLWIQESRWTNDLLNHCLGLF
IFIVTRCR (SEQ ID NO: 850)
>orf01685 
MNVTLKLLPTERTIVQSRRQTETIINQHFFTRTVSIVHALDLPYGHMTLVNHNQEIIWEE
VEKRIRRLSFAPSIHVARIIFNPIGIAHLTQHFDIILCPLFQTLGFKQFTFLFKDS
(SEQ ID NO: 851)
>orf01686 
MIHFSQHLTCQSLNFTNTVNFVSKKFYSKGMFISGSWENLYHIPTNAKSSALEINIITFK
LNIDQVIQEFITRNL (SEQ ID NO: 852)
>orf01687 
VAKLVNLVIDRTILLNIGIARRDIGLWLVIIIVGYEILNCIFREKFLKLPIELTSQSFIV
GNNQSWFIDFRNDLTHSIGLPCSSRPHQNLSFFSPLNVIHQLLDSLGLIS
(SEQ ID NO: 853)
>orf01705 
MNITQTDFLAVNLVFAISTTIDMAFHPDFLTCILDKSVMIIQSHNYRSIIERFATFCSSK
DDIRHLAPTETLDTRLPQSPTQTFCNIGLSRSIGSNNCRHTLVKNDLGLISKRLEPLNFD
FL (SEQ ID NO: 854)
>orf01707 
MSFIVCNHLKFACFNLRNHDLIDKFLDLGHILIQKKGTKKGFKGITKNGVTIATTRFFFP
FTQLDKLVKLAITRKTS (SEQ ID NO: 855)
>orf01719 
LFTCFSKLDNKTASTTYISHKFFTAIPVCFEFFKGFWFPRKDTTKKNIFIPMFLVECFNF
WVELR (SEQ ID NO: 856)
>orf01726 
MTKKIVALAGDGIGPEIMEAGLEVLEALAKKTGFVYEIDRRPFGGAGIDAAGHPLPDETL
KACREADAILLAAIGSPQYDGAVVRPEQGLLALRKELNLYANIRPVKIFESLKHLSPLKS
ERIAGVDFVIVRELTGGIYFGDHILEERKARDINDYSYEEVERIIRKAFEIARNRRKIVT
SIDKQNVLATSKLWRKVAEEVAQDFPDVTLEHQLVDSAAMLMITNPAKFDVIVTENLFGD
ILSDESSVLSGTLGVMPSASHSENGPSLYEPIHGSVPDIAGQGIANPISMILSVSMMLRD
SFGGYEDAERIKRAVETSLAAGILTRDIGGQASTKEMTEAIIARL
(SEQ ID NO: 857)
>orf01727 
VVRNTASHLTCILFLNKGISVYIGNSRSLKHIKIKPCCIKDGFCISVFNSDQNPILGIDS
ICYRIDSVGHQTNRLVKELIDSIKDCFNGTLPCRIKFDFLTIHIG
(SEQ ID NO: 858)
>orf01729
   VEAFWIFNHGSSYQSSNICICDFLLLIGQCLELSKEWFDILFCKI (SEQ ID NO: 859)
>orf01732 
LANIESHCNFFQSSISSSLPNTIDSPFNTSCTILDSSKAICHCHSEVIMTVRRIDDLTIR
LDILNQVFEDGTIFL (SEQ ID NO: 860)
>or01741 
MQEHYTPKGKHLTIDNRRLIERWKNENKSNREIAGLLGKAPQTIHNEVKRGTTLQQVRKG
LYKKVYSADYAQTVYQFNRKRSVKKLILTKEIREKILHYHKQKFSPEMMVNKKQVKVGIS
TIYYWFHNGHLGLTKADMLYPRKRKGVKKQASPNFKPAGKSIEERPDVINLRLENGHYEI
DTVLLTKIKNYCLLVLTDRRSRHQIIRLIPNKTAESVNQALTLLLGEHRILSITADNGSE
FKRLSEVFPEEHIYYAHAYSSWERGSNENHNRLIRRWLPKGTKKTTPKEVAFIENWINNY
PKKCLDYKSPSEFLLGG (SEQ ID NO: 861)
>of01752 
VHAHTDKLCNGCNRIFNSIISHHTIFRERNKLSHKAIKSTRQEMGPCHVVFIEFFITLHR
RLIGNHDNFLTNLVGSGRVRNDGST (SEQ ID NO: 862)
>orf01753
   VNHCHWKLFIQNLGITFSLIVTLIRMTDSHVVGTDKDMIFLVNSLFLIFDIDKLRLS (SEQ ID NO: 863)
>orf01755 
VGNNDILWSKRTISINGFNDFLNTCIAVSTTLCNDDTFLIKRKIFIYKIFCMRNPVSMNT
NYNFFNTWLQDKFFNCMNQNRSIT (SEQ ID NO: 864)
>orf01765 
LVAPVASSTRFFKNNDSLTSWNNGFIIITINTIISYQRISKGQDLSIIRLVCNGFLVAGH
PCIKDDFACYINICSEGLAFKNCAIF (SEQ ID NO: 865)
>orf01767
   VVCYFYITIDWSWVHEDCCFFQTIVTFLSQAMLGMVVFF (SEQ ID D10: 866)
>orf01768
   MAFVLHTEKHHDINLINDFINGYKLSIVCKLLTSPFLRSSEKEFRSQAFQNLHIGFGNA (SEQ ID NO: 867)
>orf01769 
VVQVTCNSNFKTLKVAKFLINGHQIKQALARVLARTISTIDDGSRNRWTSNQFSIVVDLW
MANHTDIHS (SEQ ID NO: 868)
>orf01770
   MCPCRILKEEIGNNRMVFIGKLGSIFKLNSSLDQFHYLIDSEVFHGHHMVQCLLIF (SEQ ID NO: 869)
>orf01776
   MSINCKGWNPKSTHDNIGCLATNTCQTLQFFTCLRDLTIKIV (SEQ ID NO: 870)
>orf01790
   MPDCTLTNFLDKVLYNRQGNVGLEQGQANFFGCLLDIRFRDFSFFT (SEQ ID NO: 871)
>orf01793 
VQFHLIIFQNLFCSLDIVIDSLTTDTELLGNFSKAVIISVVELDIIHLLICQKRRIKFKE
RIHTIGFFDFHNFYYTKN (SEQ ID NO: 872)
>orf01796 
MKFNHYFFLFLIIEKQVAIISFFMHFHIIKLVNHFQLLIKLNCISHPNLHIRPSFLSLVL
LFYQKEQDFAIMVI (SEQ ID NO: 873)
>orf01799 
LANNRKTETLGVSYLSTFIDKHELLQSYFESNDKTPVWDGEIHVLKSPSEKKDEILGKVP
VQIKTTRQKKDVLKSFSLDTRDLELYKPNGGVVLFVVWLNEDNGLRDIYYKSLPPLSIKN
LLKKSKLKNKSTNRKKLSIEIFKLDEKKMYPMLVDFINNSQKQYSFINVEGISVEDIPDD
 
KTLKFYFYGQEKEEIFNYQEEHDLFIYYLDPITGIEIPLENTIKIVETEEETDLIIKIGD
YVFQDVKRHRFPDGSVQLHFGESFTMSFDIKKKQFKFNYTRPDLLSKAIKCTQVFQELGK
IGYFTLNGNKIELDERSIKDISSLDLEADIKGLLKISNFMKKMGIQKDVDLSCFDKQSQR
NLNILYSGLVLKKKVALNYNESKLLHLNIANIHIITLYSFLSDKNGTMIDIFTETPWCRE
GETEDEDYLDISIFEVFEPNDWLKIDNCKIDSVIASYQRLVDNKLKYEGADRTILKIVIA
ADMAEDKTKRELLLNWAQCLSDWNLKYSKNCEMAIINDLQIKSRVRKLNSKETETLTNIL
VNSNDNYELCFGSSVLLKSKPQADLFWNKLDNETKERYKDFPIYTLYMKLS
(SEQ ID NO: 874)
>orf01800 
MKVSKKITLFSLSFAGFVLLTLPQAGKAFELKEDWAFKGGIRYENGKVSKINNGYEVNIK
VLDLPSTSAIEWTVRLNGEKQNTNFLAEERTVSKTEDKGRFLHFYIPYGYRGDIVVEAKS
GNEVKTWSTKVVDDVYSDSAKSGYFILDGEQILESSWDSVNESYIATLPTVTSGKTVVAW
REKGTLNLIKPGRIARQYNSSGSYVELSPIFETASWLKSNQNWYYQKQGQLVQNSWIKDQ
GSWYFMDDEGVMFNQTWLHQGGSWYAFKSSGAMISADWLYDNGSWYYLKDSGSMVTGWLK
NGGSWYYLNKSGSMATGWIKDSGTWYYLKNSGSMATGWVKDSGSWYYLKNSGSMATGWVK
DNGKWYYLASSGNMLRNTRTPDGYYVDGSGAWK (SEQ ID NO: 875)
>orf01801 
MKKILLSTVALLSLVASLLANNPVSAQESSSQATYSKSSGSWIKSGNRWWYKHSDGSYTT
NGWEKINGTWYYFDSEGWMKTGWIKEYGKWYYLDDSGAMKTGWCLVSGSWYYLNSSGVMQ
TGLQTINGKQYYLAAGGAMQTGWHNIGDDTYFFANSGENQNINRRALVLGETSTRAVPIA
DVNAMEKVFNNQNFSEVVRFPDRTKSEIIAKMQELFESSSEGDVNYLYFTCHGGRDGRIY
IGSDGLAFSGWELASVLKQYKGKFVVMLDCCHAGTIISKDNTGEGNEGASTEYFDLDEFV
SGFSNMDGNEKSGEMIDSKFLVLCSSRGAEYSSGGSLSLATKYWSLGSGWNPLQNSQAYL
AADQNNNRRITLNELYTYSREQVLKQNSNQHIEVYPDNSQFVLFKK
(SEQ ID NO: 876)
>orf01802 
MENFGAVLKDIRISKNFRLKDLSCNEISESTISRFENGITKLSINHFYILLNRLGISFSE
FEELVHCYYSKKECLFEELEHAVNSSDIFLLQELVDKIELKQKQEKSLCNYHIKLIAEQQ
INRLANLPYNSSKCNELIKYLLSVDTWMEYELKLFYNSVFFMNTRTISLLYRIVIKKTRY
FLKTNTGTHRIIPLYLFNLKLLLKNNLLGSAQFFIDDLENLLTRQGYYFEKNYLLFLKGI
YLIKTNQIELGKKECFKAMRIFKEYNDSDTINELNQKFKLDLTI
(SEQ ID NO: 877)
>orf01803
   MSSIYSSAKKDFLYWNVLIFIMELPNDVKVQFYELRKKVQSFNQLSKRFGMDVSG (SEQ ID NO: 878)
>orf01810 
LSFLILSPAGAQESLSFFFVKITDASKTVKNGGQTETQKLVTKMASDFERVENKDSEVGK
IVKEKLALSGDITEAKLTEISSALLAFEKEQNPVDLDAEKEKLVNRLSPRFETLEQAIAS
KDLEKVREAFKKMNSTWTINESVVRDNSTAHYGRVETAISFLPSSMETEPTDESGT
(SEQ ID NO: 879)
>of01812 
MQKNIYFVVLDLHTTDRDKIIQLFKDWTDYSAKLVEGELVKKDGQNALFPPSDTGETVGL
NPHRLTLTFGVSASFLKRMNLENKRPRLFRDLPLFPKEQLREKYTGGDIVIHACADDEQI
AFHAIRNLIRKGRNAVPLRWSQSGFAAIGDRMETPWNLFGFKDGTANPTKEQDFDRVIWA
DSKDWMENGSYMAVRRIQMFLETWDRTSLEEQENTFGRYKESGAPFGKKNEFDEVDLSLL
PDDSHVCLAKEVDKPLLRRSYSYSDGIDEKTGQFDTGLLFISFQKDPDNFVKVQTNLGAT
DKMNEYITHIGSGLFTCFGGVEKGGYIGQKLLEG (SEQ ID NO: 880)
>orf01815
   MTGKKGFLFLNCHICMVTTTTCFLKERVESELLIFFYISLNRCLITV (SEQ ID NO: 881)
>orf01818 
MSLRNKIEQHIKELEGGKFQKLGDAYLSRKYNFNIVSLGSQEGTDKTTKGIPDSYAVENG
KYVYIMYGTHKSVISKLEGDIQSVKKKILEENIAEDKVGRLICCHTSSNITIKQKEDLEK
MAEPYHLELIGINEIANDLTKIDFQYLAKEYLSISESTEQVWSINDFIRIHDESKTNAPI
SNDYIGDVSEIINTIKSSEKRIFLISAKPGTGKTRLAIEICSLLDRNKYNIICVKSNNQD
 
IYQDVKRNLNLHKENIVFIDDVNTTQNYISTLGLLNTTSNIRFILTVRDYAKKDVINNIK
VYGYNNIEPELIKDDNFKELLNQFSRNDFTNQEIEHIKTISKSNPRIAVIAAKLSSSQDL
TNFNDEIDILKDYYEEILNKNNIIYAEQKTLFILSYLKKIRLESLEENQEFNKLLKITDI
TNTDFKSAVEKLHERELCNIYNDKIVKIADQSLDDYIVIKFLINKKISILEILHELYPVN
DQRVVQILNQCSNFIRKESDLEGVSDAVKSYYYNESNFESDELKEKFLIQFGVLLPLEAI
SHVKNKIDNIESQVYTKTNFINQKDKKGSIEDSVLNIVFVTTRTKYCSQILQLLLKYFDK
NPNKISEVYSILEANYGLVTEREYIDYTLAENTISELANLDLTKSYNQELIVTILKQFLK
IEIERTEAHEEKFTFGRYKVPDSEKLKQYHRSILKLLANLYNIGSCETRFYIEKMLYDYR
RKILTYSESHRNTIFGDLRNIRKLFFNDIKNLSMIGEKIVYALHKAEVKENLPIVFDDYI
ISDRQKIYNNLTNPNHAWFYDASEIKLQQIANSYSNVWLKIFNFANQFKHSLFMNDNNIE
LVLFNMFLLSKNDKKIKFLNYMFKSNYHFVNMNPISFLENIEESSMQSVIVSSPESEKYE
WQLAYLTQLENVKNEDLQTLKSILEANSLPCYFTILNFERLILKDPSLKELLIQKAGNTN
FVISDFIREEEVPKLINLIGVKELKFWYLINLENCQNHSYNLFQKLGEKDVDFSVEVLKK
IDELRIGHSNLGYMVLHSISEFRDKKEIYKKFIRFAINRPYYYYNNMIDDIIKNDSQIIL
EILEETNNEQSAIRLVNLGVEFLENNNQKLILFNLLRAKGFGKKSFQEIHFTPYSHFYTG
SHVPVLELEKELLERIKKIFETGIDYINLLLYLNKLIDCKRKAIERELEKEF
(SEQ ID NO: 882)
>orf01822 
MNESLDDITHKQFTSNLTTKADNVSVQLFFSIKGCCHITNQGRTYTWNFIYSVVDTNTSS
TDTYLKISLAASYSFPYFFTKDWVESPCMVICTKVNDFISF (SEQ ID NO: 883)
>orf01824 
MAISQMKRISLLFSKSSLDDVLKTIQELESVQFRDLKVQDNWSEALEKDEVVFPTIQISH
TSNSNHGVIEGNDALIYLMNQQQYLEATVEKLQEYLPKENTFKLVRQPPITTSYKELEKL
VKLMLPRVFLKK (SEQ ID NO: 884)
>orf01826
   MNRACIIQPSLVEIQIWLLNHVCKCSDFLSHRMRSLLDRKLDLISLLIKLFPKKNWKN (SEQ ID NO: 885)
>orf01828
   LNGGEFLETEFGHSVLAIQSVVWFSFFCLKSNASSLAHGI (SEQ ID NO: 886)
>orf01829
   LLAGILELENWGKTTELRPTLLSGPVQNKIEALKRAKI (SEQ ID NO: 887)
>orf01834 
MRSQNHNCRPSTRKVGCIGPIFFGHLLNHRKFSYQVLTITLMEEVSLDCLPSGHHVSCQQ
GSNRYIGDRTCSNSFLIRQFFRQDTTAVAST (SEQ ID NO: 888)
>orf01861 
LRQNRCYNCFHDHSCSWKSSRITSLHGCLVRFVGFDIHTHKRFIKSRNGFHDPTNNDGLP
ISHTTFKTT (SEQ ID NO: 889)
>orf01862 
LAAFTITSLKAKTKFHPFKGIDRDNSLSQSCIQFSIPLDIGTKTNWNASDDCLHNPTDGI
TTTFDLVNIVLDFLFSFLVDNRNFRLGSSLLNFSDCQIFRNIYFLTTKDHDMVGNLHIQL
SQEAFGYCTNCHPHGGFTS (SEQ ID NO: 890)
>orf01863 
MTWARMSNFPLAFKAVFNVLRRHDVQPFLVVLIDDIHSNRRPCRLPVANARSKDNLVTLN
LHTTTTTVATLTASKVLIDILSCQWKSSWNSLNNSC (SEQ ID NO: 891)
>orf01865 
MHDLAITGSRFDGMANSVAKIEVKTNTIVQLIFNHHLALHLTRMFNQGLCMFQNTLNRTI
QSRQESPQFWILNQAILDNFTHPFNQLSFSEGFKNKWINQNPIWLGKGPHHIFSKWCVNA
CLSTDRRINLSCQTSRNLNKVNTPHIGRGYKASQVPNNATTKSNDSIATSQTLLD
(SEQ ID NO: 892)
>orf01867
   MVICHNDYLLRLPEFSQPLTSLGHTTFFNLNIIRMMRNIDSDFHRRVSLSLLVFFC (SEQ ID NO: 893)
>orf01872
   LIEGHLVFADKPAQALVLLRKVGSPKKVSFLTLHLYFLILKIDILKITGF (SEQ ID NO: 894)
>orf01882 
MSTTTKFNRVVTDSHDTNFLAVFLTKEGHSSHFFSSVNIRFHCLNFKSFPDFFVDLLFNR
TQFFSSYRLGSG (SEQ ID NO: 895)
>orf01887 
VTWIHSHFNPAVVRIFIVWIVGHVKFFSREIKPFRACQKLISPSDSFVTEVIPDREVPQH
FKHGMVTRSLPYVFDVVGTDSLLGIGNTWIFRDNGPVKVFLKRLLPQS
(SEQ ID NO: 896)
>orf01906
   MNGHFLLLFCLFNIFFHLVNIELSKQVLTVLDWETLVQYXIPFIDI (SEQ ID NO: 897)
>orf01911
   MRLRDLRRVDFPDPDGPIKAVISLGWKDRETLFKAFFLL (SEQ ID NO: 898)
>orf01914
   LKNHSNVFTHFINVDFWTVDINSTIENLPSYFSNINSIIHAIETA (SEQ ID NO: 899)
>orf01915
   LHINPLNGFIFTIVNMDILSRKGYFFFRKGKDMLLIPVIC (SEQ ID NO: 900)
>orf01920 
LSPFQHCHSSGSIFFNLHFLNRNILQSFDNPFLGLIRENKIEKFCSQLIGLPQCIHMLIR
PQGPIIATYIFWT (SEQ ID NO: 901)
>orf01921 
MNXXNSRCNHPTWSNFLDILEVDFLGNIVGQKIRSHDLKNPVQVFTVIDMTIHIQVVKTN
MVILADRLFQGFILRSTDKFFIKIRLVRSHNLRFNNMDFSTVAVHENKGRHHVDELLPRF
IINSKATVAKKSIVAQGFRFDGNFFRKTRQTNHLNIIFCDNPDQIIVFQNGLITNSQFNR
LHP(SEQ ID NO: 902)
>orf01930 
MWYFYNTDGSMATGWVQVNGSWYYLNSNGSMKVNQWFQVGGKWYYVNTSGELAVNTSIDG
YRVNDNGEWVR (SEQ ID NO: 903)
>orf01931
   LTFIKSWAIEIFCFDWDWFLDKNLGLGSFFNNSCLRVFFLT (SEQ ID NO: 904)
>orf01932 
LLLSCRKVIVCFIFSSKWNKNFFNLAFSWNFDNCIRGFFSINSNLFGNITSLWINIVGPC
RSYIAILSINCNRIFTTVFCFIFFITNSRT (SEQ ID NO: 905)
>orf01949
   MRFIVGRFTSFSLGIEFSPTSKLDDLLFKIAFLMILATWIKARKTKGAT (SEQ ID NO: 906)
>orf01961 
LVNCEPLEAYRQLEEAELVGCWAHVRRKFFEATPKQADKSSLGAKGLAYRDQLFALERDW
EALPADERLQKRPAPNGRLLCLVPPSVSFSRFKTRKGN
(SEQ ID NO: 907)
>orf01963 
LKRNKIWKKTLTYPVEREEITYKRKKAKGKRQAILAQFDSEEVHHRLENCICPDCQGELK
EIGASLQRQELVFILAQLKRVNHIQHAYKCQTCSKNNPSDKIVKAPIPKAPLAHSLGSAS
IIAHTIHQKFILKVPNYR (SEQ ID NO: 908)
>orf01964 LKIIQQQSATIDSLTNELALLREQVAYLTQKLYGKSSEKSVCPSGQLSLFEEEQNMEEDS DLPS (SEQ ID NO: 909)
>orf01972 
LICQTIKYWHKFHLHIGRCKLLIGLIPILNFFIRADIDCLLVLLSLIDRQNGKQFNLCQW
IIASNGLNDSFEIIESLIHRNILSDIICPNQKKNFIYCSTI
(SEQ ID NO: 910)
>orf01978 
MSFSCSDSCFSILLLDGDIHENTTFSPLSILFISHRFNSLIGNEVPHLIDNELLISIFFH
RFRWFNNVRMPSKDNIRSPIDHLVIKSFLFFSWFQSILNTHLKHDNGDICFLLCPFNFSL
HLIFV (SEQ ID NO: 911)
>orf01981 
VVEQIPVGHNSGSFFLFLLLRLLLSPLLRNSISFLTSQGIPWKLSNNKTKPIDKSTASKS
IATNPLLLHLR (SEQ ID NO: 912)
>orf01988 
MLKLIIYQFQYSKRQWLGTIPLLFVSSLIVGTSLFGIASSIKTANINASQLFQMLIIFGG
TTLFFLISNNIRLLIDIFKKDYQLWTILGASRTQLSLLVSGQFYLMAVIVSSIGTILSFI
MADSYYKFLQNLLGRDELPDLVITANIQSILLSIFIVPTIVGIGAYFYSSRILKISSILK
PKKKKRKVTVTGFVNISVRLFLWLLCIGSIVSAGFIRNKEIIEKQSSIILFLLIIHILII
QSLSPSIQMFLIKFLMRIFPTENYVINTGFWNLLSNPSYLKSIQTSMSMGVTLISGFILY
TQNMYSFMNTANGVNEARASFIAYMSAPIILIITSSISLTILSSNKDIEDIKQLKTLGVS
RLQLFKIRIGEAIIHSVLILLVSVIFNLIILILVSIIGQFLGRSLVDISGFWQPSLIVIS
LLVIFYSITKGFYLFQDR (SEQ ID NO: 913)
>orf01989 
MVNNVAVKVSNLSKEFLLGQDKTVSILKDISLSVNYGEFVSILGVSGSGKSTLLSCLSSL
SEPTSGEVVINGVNPYTLKEGKLAKFRRQDIAIIFQNYNLVPALPVLENVTLPLRLSGKS
VDSNKVKKMLDSLNFKAELSSLVATLSGGEQQKVAITRAIIADSKIIFADEPTGALDSVS
RKLIFETLRNLASQGKCVLMVTHDIELASKTDRALILKDGKISRQIIKPSADELYQALES
SKD (SEQ ID NO: 914)
>orf01994
   LALVRKFIDYFFGVLVPFPDLYVFKSCFKFAGSFTDFDTFDWWLDWCRSCSENRFFV (SEQ ID NO: 915)
>orf02006
   MPTILLLKKFYERLITNFFRLKFLFCKEILATNIFNHPLFEPDIRVITIKII (SEQ ID NO: 916)
>orf02009
   MXXGAFGQGELLLQQSRNSSITEIVSDSWAGAGRRILPLPKSVTPLVSS (SEQ ID NO: 917)
>orf02013
   MLLLISLTQLIIFLFFERFNLLLKTFLLVDLKSNKSA (SEQ ID NO: 918)
>orf02022
   MAGKKGFLFLNCHICMVTTTTCFLKERVESELLIFFYISPNRCLITVYSVLNL (SEQ ID NO: 919)
>orf02029 
VIPRYVTKHQGWDHNPHTITNSDDDPATLVTFRTFKFNVGNCTIPKNDQNGSSQKFSGIL
QCPCEIHLLDSP (SEQ ID NO: 920)
 >orf02034
   LLVRKFNIQTFFIQVFILNDFGYTVNGLIVYRLLLTSSILSFNDYSIGSFRTVIVI (SEQ ID NO: 921)
>orf02040
   MRLSIQLIHDLNTHTTHSTAKMLYNVKAIKNDFSIRE (SEQ ID NO: 922)
>orf02048

MTAQLPSDALQMALWRRKRPRNVIVHTDRGGQYCSADYQAQLKRHNLRGSMSAKGCCYDN
ACVESFFHSLKVECIHGEHFISREIMRATVFNYIECDYNRWRRHSWCGGLSPEQFENKNL
A (SEQ ID NO: 923)

>orf02093
   MEIVLVSFSISFQHFIIAYCLDFSSAGFRNSQNFSNFC (SEQ ID NO: 924)
>orf02105 
LTEKIQEHELIKTNQAEKSVQDVLDNCIERVQNNSLKSDRVTSFETPFALLFIFATIAVM
LTYGGYRVSAGYISVGTLVSFLIYLFQLLNPISNIANFVTVYSRSKGSSVALENLLAVPK
EKFEGGKSVSGRGLNFNHVYFGYDENRPVLKDITCSIFKGQKIAFVGPSGSGKSTIVRLL
EQFYKPLSGDILMEQSSIYDFNLKEWRSKIAWVSQNNAVLSGSIRDNLCLGLNRLVTDDE
LMKVLDLVSLGDEIRSMKEGLDTEVGERGRFLSGGQSQRLQIARTYLKDAEILIFDESTA
NLDADSEYAIISILYSALKEKTVVIIAHRLSTVKDVDCIFFLEERKITGSGTHKELLENH
ERYARFVQEQMIE (SEQ ID NO: 925)
>orf02106 
MKLKLLRVDTKVIMGSFLLVLSSLLALLLPLILKGLIDGSSIENIGSKVFQSFLIFIGQA
LFSSIGYYLFSQSGEKKIAKIRKKVIEGLIYAEKSFFDKSQSGELTSAIVNDTSVIREFL
ITTFPNIILSLVMVLGSIVVLFSLDWNLSLLLFITLPCMMFIILPLSNISEKYSRRLQEE
I (SEQ ID NO: 926)
>orf02115 
MXXALIPRVSIGSVGLLLLTENYVKGDLKAASRLVQDSLTLLFMFLLPATVGVVMVGEPL
YTVFYGKPDSLALGLFVFAVLQSIILGLYMVLSPMLQAMFRNRKAVLYFIYGSIAKLVLQ
LPTIALFHSYGPLISTTIALIIPNVLMYRDICKVTGVKRKVILKRTILISLLTLVKVSVN
RNHPVAVRIFLPTKWTFVELPLCSSCRCHGGWTLYGYESAYLFIR
(SEQ ID NO: 927)
>orf02125
   MRERVRLSGSLFTSLKTREHIKSTMELFHKYVFFLIQEIKIKMINFLKIGDLPTL (SEQ ID NO: 928)
>orf02134
   MANHLYIVPIQVNHKSSIVNRMLTSITRNPIVSPTCLYASLITSLNFFLIFC (SEQ ID NO: 929)
>orf02137
   VIVFLSRNKDGNAFCHLDLISIANPVWGWDDDFITWIDHSHKEGIERIFGSRSDCHLI (SEQ ID NO: 930)
>or02140
   LSNQFYFSLQTKPILKVKQFLLFQSQMTRVSEILQFSNKL (SEQ ID NO: 931)
>orf02141 
MSKKVLFIVGSVRQGSFNHQMALEAEKTLAGKAEVSYLDYSDLPLFSQDLEVPTHPAVAA
AREAVLVEDAIWIFSHSLQLLYPRYSEKLA (SEQ ID NO: 932)
>or02144
   MEDKEMGFYLMVASMLLGLLALKIGFSQFKEKKDKFLSILTSLAGTALVLVAVWLGWPK (SEQ ID NO: 933)
>orf02166
   MLDSDIGCSRKNLLGLFWIRRRRNIHIVDRAMEKGISNRAPNKISLKACFFNFF (SEQ ID NO: 934)
>or02193 
LLHPFTRNITCDRHILTLLGNLVNFIHIDNATLCTFDVKVSNLQEFEEYIFHILTHITSL
RQSCRIRNSKRYIQALSQGLGKESFP (SEQ ID NO: 935)
>orf02194
   VEIDAFVVVINRHCQGTLGTILTNYIVVQDMEEFNWFWHLRQVCQDFLNQFFSNDFLS (SEQ ID NO: 936)
>or02198 
MEXXXTELAGRGFLVWHPKMDEYMEALDGHLDEISERLITLGGSPFSTLTEFLQNSEIEE
EAGEYRNVEESLERVLAIYRYLITLFQKALDVTDEEGDDVTNDIFVGAKAELEKTVWMLA
AELGQAPGL (SEQ ID NO: 937)
>orf02199 
LITRLLHRVHVLVDDFNPSICITWSSLALIYIFPVLNIIIDRVRQVHIVFLYKSHGLFSV
ILSIGLIFSIGIEIDTIRNSQNG (SEQ ID NO: 938)
>0ref02200 
MXXTGSLSANFAGSTTASSSSEQNQSSNKTQTSAEVQTNAAAHWDGDYYVKDDGSKAQSE
WIFDNYYKAWFYINSDGRYSQNEWHGNYYLKSGGYMAQNEWIYDSNYKSWFYLKSDGAYA
HQEWQLIGNKWYYFKKWGYMAKSQWQGSYFLNGQGAMMQNEWLYDPAYSAYFYLKSDGTY
ANQEWQKVGGKWYYLKKWGYMARNEWQGNYYLTGSGAMATDEVIMDGARYIFAASGELKE
KKDLNVGWVHRDGKRYFFNNREEQVGTEHAKKIIDISEHNGRINDWKKGIDEKRVDGVIC
RLGYSGKEDKEWRIH (SEQ ID NO: 939)
>orf02202 
MHKNFVVVVTNFFTAVQFIQFNKEGTTCHNTTKFFNHLDSCLNSSTCRQKVIYNKNTLTW
LNGIRVHSQGIDTVLFFIVSRNNFAWQFTWLTNRRKTNSQLKGNWTTHDKSTSFRSHDHV
DFLVSSILNDFTNSVAISISISHQRTNITEGNAFLWIIFNCCNVIF
(SEQ ID NO: 940)
>orf02209 
MNRCNSRQAIWKIISTLNRENTHIMLNRQVCFCFVNHISPLNVVIWENLSLEELLYAICI
CFITHKIAKQTSLTIDNAGIAMNNIR (SEQ ID NO: 941)
>or02214
   LDSRFFCTDFFKGRQAKGCSFSCTSLSLTDNILAFKGQRNSLFLDRTSFYKTSFFNFC (SEQ ID NO: 942)
>orf02225
   MGRKPRTRPEERTELERLQAENEYLRAENAILKKLRELRLKEEKEKEERQKLFKN (SEQ ID NO: 943)
>orf02246 
MLSKEEYIEEIGLIEKQNYVEVELYPLVADIINPTLKNSLSKRYVFGRRKSNMGQIYYGL
SNFPDIVILDKNYQNKARKSIEIEEWKKLRGCVEIKSLKHDLITEEKIKSTISNSFEHIT
GEMGQLIGDLLWYKKVIYTNGIEWRFLSLDDKEEIDNTIVQVVNKRIETEEAGNSFDWWK
NIKDLSFNYTDIYLSKDCIQEWDEFVKKVKEIEW (SEQ ID NO: 944)
>orf02248
   LEVCIHHHHQISCRILQACIKGCFFAKISRERNIMDCRILLPIGL (SEQ ID NO: 945)
>orf02255
   VDRTDEVSSKHCFEVVDRTDEVSNHTHGKATLTWFELDFRRV (SEQ ID NO: 946)
>orf02263 
MTPIKDKVRRVKTPMMVNPDTDLTISSVQQDYFSLALIGFSLLTGDFLSFSKGDQKTGLS
AFIKICHLIKIARLDNKITKQQEYWLYDLLMMSQGEKIQKIKQLKQVTSDILLNTPDFSS
YFEKYNFKEEAENIKSYLLAKSMDKSGRLFPSNEFGEFVSPVSFQHGFGGVLFFMNKYYV
EEDENTVKEWLTKLENYEAANFLHGYSLLFGKAGFLFGILDRYEKTKERYLIDISKRLVD
HLMRVYDNISNLDFALGKSGILLSLMKYCTIFDDKKLANFIKNNINDAYSLLESEDNGDI
YSNNFAHGRSGAAYVLKAYTDIFGDSRYQNHLQKFSDGISELLEEKLSSFSKLDNLGLSW
CDGVSGLILYLCLIDKERYSEIIYKSQLEMVQQYEAMGTSFCHGLSSLLQTTIYNKNQKV
EQFIKKILLTRSYRNNDRLLQFQGEDGINSYFDFGVNLGIYWTLLGYTFPFELSKGD
(SEQ ID NO: 947)
>orf02264 MHIFLKNRAFRQLTVNEWISSFGDTIFYLAFINYVSSYAFAPLAIFLISLSETIPQVLQL 
FTGVIADFQKNRISKYISILFIKVLLYSGVTLLLTSTDFSLFSVFFICSMNLISDTIGFL
AGYMLTPIYIRLINDDMTEAMGFRQSTSSIVRLIGNLSGGVFLGLFSISTLAFVNVLTFL
FAFLGSLLIRNRLKKEEEKIEVPPYVGMSSFFQHLKESMKLLMTMEDVMVLLWILSISQA
VLMMVEPVSAILLIHHPFMGLSTGQSLAILIMISLLHVILGGLLSGFLSKKISIRLNIYW
SLLMESLIVIDFLRGSFLLILLGSAGDAFSAGVLSPRLQAMIFGIIPEELMGSVQSSINV
INLLIPAVLSLALVFLATSAGLEVVAFALIILLLIAAYLVHQMKNLPNQEEV
(SEQ ID NO: 948)
>orf02266 
MTVPDRLPARMRMDGRSIFQQIKNYFDTENKEYFKHPNTYDGISMHLEPNILTSMEHFDL
TGFHCECKDFQNQGVCKHWVAMDLYFRSLPQAIQERIGKASHKPSFASQLIPTLPSEELQ
DELVEEKATAPSLALHGQVEIRNHSLAWTLKLQVEQAPRAYVIKDIAHFIFLIFQKEDYF
VSQKIGTIRLSLNQFNQASQNLLLYIKKYFIDRNEHSYFNFSYGINPRDYGRYLETPVSY
LNDLVPLFQALDVFQYVTSKAEYPLIFLDDSPFIPEEEIFKVVKSNNHYEIINTAYFGFI
IQEKLWIRHNHFHIIKEEHRYFLDKLATWIYHYQENSPLIFSKENKAELMQVCNIISNYV
PISIPDELQIHDFIPTFAFSKTRNEIALNMVWSFGEKQVHSKQDLLTLPYTYQASKARKI
YHQLLSAGFKEEFHSLSKIKIVDFFLKELPRFRTLGQVQLDESLEKLLVEDPAVIDIFDD
ESFLSVQFDFSMISEDEVEKAIQALWNQESHYQTKQGKVLVFDDESLKVAQSLQDLRAKF
SDGKIKMHKSRAFSLSETFKDNEHVNFSRDFKKMAYDLTHPEEFDIKPYEVKAKLRSYQK
EGVKWLSMLDHYHFGGILADDMGLGKTLQTITLLEANLKPDQKALILAPASLLYNWKEEF
RKFVPHKQVEVAYGSKTERIKQIEKSATITITSYPSFRSDLEHYQKQSYDYLILDEAQMI
KNSQTKTAQALREFDVKTCYALSGTPIENRLEEIWSIFQIVLPGLLPSKKEFSKLSPQLV
AKLIQPFVLRRKKDEVLTELPELSEHLYSNELSSSQKTLYLAQLRRMQEMVSGASAYEIK
RHKIEILAGLTRLRQICNTPALFLEDYKGDSGKMDSLFELLDTIREKGSRPLIFSQFTSM
LDLIEQELEKKEMSHFKITGQTPSDKRQEMVNLFNQGEKDCFLISLKAGGTGLNLTGADT
VILCDLWWNPAVEMQAIGRSHRLGQTKQVDVYRLITLGTIEEKIQELQESKKELFNTVLE
GQESRSNLSVDDIKEILGVE (SEQ ID NO: 949)
>orf02283 
MMSMVDPIDQTFIVNLKIGKSQVFSQLQFSCHIVVYPSEVHIYQAFVIKLQNHILGPQVL
P (SEQ ID NO: 950)
>orf02284 
LPNRTRIDNQLPTSPVTKQLLVNMSINSNITGRMSHQAVKLLLFASMNQLSPPVLIRQMM
ANSHRQIPKLTMNLKRLIVEHFNFF (SEQ ID NO: 951)
>orf02285
   LIQQVQNPSTPCPWHENISQKPVFIHSYLPSICQNSLQGGGISMNI (SEQ ID NO: 952)
>orf02308 
MIDKVVRNLLLTFLFCKMTKIINFLTTILVKKKMCYNVSKLREKKKGAMMWVLGFILFI
IFFYSNNSKKIKKLRE (SEQ ID NO: 953)
>orf02309
   VDRTDEVSSKHGFEVVDETDEVSNHTYGKVLTWFEEIFEEY (SEQ ID NO: 954)
>orf02314 
MIAEFIDGLQKFHFLQNALITAIVVGIVAGAVGCFIILRGMSLMGDAISHAVLPGVALSF
ILGLDFFIGAIVFGLLAAIIITYIKGNSIIKSDTAIGITFSSFLALGIILIGVAKSSTDL
FHILFGNILAVQDTDMFITMGVGAAILLLIWIFFKQLLITSFDELLAKAMGMPVNFYHYL
LMVLLTLVSVTAMQSVGTILIVAMLITPAATAYLYANSLKSMIFLSSTFGATASVLGLFI
GYSFNVAAGSSIVLTAASFFLISFFIAPKQRYLKLKNKHLLK
(SEQ ID NO: 955)
>orf02336
   MYEEPEVAPVHPTGPTPATETVDSAPGFEAPQESVTIL (SEQ ID NO: 956)
>orf02363 
MGNNGQFTFGYRHDFFQNQLAIFNALVDTFTRRTIDIKTLNTFINEVLNQGTRTLWTYFS
LLIITCVEGWNDTFVFFQI (SEQ ID NO: 957)
>orf02368
   LHEWIPSIDEGKDCKGCKPWFHNREGYTPEGTNLTTTVDFS (SEQ ID NO: 958)
>orf02369
   LFHEEDTEWPSNQRQDNCPESIVDSHEVDDTYQWYKDNLFWKRHSSDKDSIC (SEQ ID NO: 959)
>orf02393 
MSYFRNRDIDIERISMNRSVQERKCRYSIRKLSVGAVSMIVGAVVFGTSPVLAQEGASEQ
PLANETQLSGESSTLTDTEKSQPSSETELSGNKQEQERKDKQEEKIPRDYYARDLENVET
VIEKEDVETNASNGQRVDLSSELDKLKKLENATVHMEFKPDAKAPAFYNLFSVSSATKKD
EYFTMAVYNNTATLEGRGSDGQQFYGNYNDAPLKVKPGQWNSVTFTVEKPTAELPKGRVR
LYVNGVLSRTSLKSGNFIKDMPDVTHVQIGATKRANNTVWGSNLQIRNLTVYNRALTPEE
VQKRSQLFKRSDLEKKLPEGAVLTEKTDIFESGRNGKPNKDGIKSYRIPALLKTDKGTLI
AGADERRLHSSDWGDIGMVIRRSEDNGKTWGDKVVISNLRDNPEAKDPAAPSPLNIDMVL
VQDPTTKRIFSIYDMFPEGRAVFGMPKTPEKAYEKIGDKTYQILYKQGESGHYTVRENGE
VYNAQNQKTDYRVVVNPTEPGYRDKGNLYKGQELIGNIYFAHSTKNPFRVANTSYLWMSY
SDDDGKTWSAPRDITPGLRKDWMKFLGTGPGTGIVLRNGPHKGRILIPVYTTNNVSHLNG
SQSSRVIYSDDHGKTWHAGEAVNDNRQVDGQKIHSSTMNNERAQNTESTVVQLNNGDVKL
FMRGLTGDLQVATSKDGGVTWEKDIKRYPQVKDVYVQMSAIHTMHEGKEYIILSNAGGPK
RENGMVHLARVEENGELTWLKHNPIQKGEFAYNSLQELGNGEYGILYEHTEKGQNAYTLS
FRKFNWDFLSKDLISPTEAKVKRTREMGKGEMGKGVIGLEFDSEVLVNKAPTLQLANGKT
ATFLTQYDSKTLLFAVDKEDIGQEIIGIAKGSIESMHNLPVNLAGARVPGGVNGSKAAVH
EVPEFTGGVNGTEPAVHEIAEYKGSDSLVTLTTKEDYTYKAPLAQQALPETGNKESDLLA
SLGLTAFFLGLFTLGKKREQ (SEQ ID NO: 960)
>orf02395 
VADRTDEVSSKHRFEVADRTDEVSSKHRFEVADRTDEVSSKHRFEVADRTDEVSSKHRFE
VADRTDEVSNIYTARRS (SEQ ID NO: 961)
>orf02399 
MKIKEQTRKLTAGCSKHCFEVVDETDEVSSKHCFEVADRTDEVSSKHCFEVADRTDEVSN
IYTVRRR (SEQ ID NO: 962)
>orf02407
   MSCNCAFYRSQFFDVNSVSNYHSHQKELRFPNSILFTYFVKVA (SEQ ID NO: 963)
>orf02428 
VGLIKLTSYVFVCISNSFLTRHDKNDNICFFHGNFCLVLDLFHERSIDIINSSCINHAKR
TIEPLTRCINTVTCHSFDIFYNGDSLTSDPIK (SEQ ID NO: 964)
>orf02430 
LSSKSCIDRTNQETFHTLGLEGVGMKSGSLFCSVQISDKEKENSRLANGFLRYQFIQGIF
LLLTSYHNHRVGLEILPR (SEQ ID NO: 965)
>orf02448 
MKSKEQTRKLAVGCSKYSFEVADKTDEVSSKHCFEVVDRTDEVSNYIYGKAKLTWFEEIF
EEY (SEQ ID NO: 966)
>orf02450
   LSNSFFL1KFSSSKTSGKKR1VSDN1FIRNKFICHFKKE (SEQ ID NO: 967)
>orf02459 
MDYSKVAAEVIEAVGKDNLVAAAHCATRLRLVLKDEAKVNQAALDNNADVKGTFSTNGQY
QIIIGPGDVNFVYAEIIKKTGLKEVSTDDLKEIANKDKKFNPLMDLIKLLSDIFVPIIPA
LVAGGLLMALRNFLTSPDLFGPQSIEDMYPAIKGFSAMIQLMSAAPFMFLPVLVGISAAK
RFGANQFLGAAIGMIMTTPDLGGKEAFWDILGFHVTQTNYAYQVIPVLVAVWLLANLEKF
FHKKLPSAVDFTFTPLLSVMITGFLTFTVIGPVMLVVSDAITNAIVWLYNTTGAFGMGLF
GGTYSLIVMTGLHQSFPAIETQLLSAYNNNGTGFGDYIFVVASMANVAQGAATLAVYFLT
KNAKTKGLSSSAAVSAFLGITEPALFGVNLKYKFPFFCALAGSAIGAFVAGLTHVIAVSL
GAAGFIGFLSIKAGSIPMYIIAEIMSFVAAFAFTYFYGKTKAASVFADEAATATAETVTE
PTVEAPVVEETDTLQNETLVTPIVGDVVALADVNDPVFSSGAMGQGIAVKPSQGVVYAPA
DAEVSIAFPTGHAFGLKTRNGAEVLIHVGIDTVSMNGEGFEAKVAQGDKVKAGDVLGTFD
SNKIAAAGLDDTTMVIVTNTADYASVAPVATGSVAKGDAVIEVKI
 (SEQ ID NO: 968) (SEQ ID NO: 968)
>orf02466 
MTESYTWVEADRATLSRYRHGQGHLTDQFFSFKVQRPAAKTLIASISTGKGMGPSFDGTP
VITSGNQNRINTIKNSFIMSSSSVRISLRKLTSQRNFLRNLSSLILLAAQVAKGDATACS
HQRISRVVGQDSHETLSLTEFF (SEQ ID NO: 969)
 >orf02467
   MNINNEKVWFAFYLLDMQITRPTPTFNDRRIGLIGKLQELRFLAGNLLLR (SEQ ID NO: 970)
>orf02468 
LIKGYLPNHLALMDLCSKTTCTLDDFAGIAGRRNHRGFFCHIGNGVFLTVDKYLRNQRIR
QRKSSHHILTQLVCHSHTHLFILLQTSLSLRTKERLSF (SEQ ID NO: 971)
>orf02474
   LIKLTGRNFSDILIKCLVKCFTNLLSNQLMLLPSTLKL (SEQ ID NO: 972)
>orf02479 
MIESENHCSASHSNRDYQSQHDNQGRTCQCFIIVPCHKKGSCSVGEITWNQRCQNGQDKD
HSRCLIKNT (SEQ ID NO: 973)
>orf02486
   MIARQLMVFFSTNQADTRITNMSIDSLIINNSKDFQSSSHASVSFILTKLVLLIFNF (SEQ ID NO: 974)
>orf02487
   MGEPFTHFIDCIDLGINPSYTQVCDRHFTSDIPCTMTSHPIS (SEQ ID NO: 975)
>orf02494 
LSSDSHFIGIKAFVILILGKSNSIVLRIVGLYQDLTCFFSPTCSTCHLSQELEGSLRRTE
IRQIQGRIRI (SEQ ID NO: 976)
>orf02495
   MAVHSLGIHMQGQRNIAVGTSIHRPTLPTHOKARITTAIEHENHLLFFNQTVLOSL (SEQ ID NO: 977)
>or02496
   MVTGIAVLLISHFMLFINNHDTQIFQRSKDSRSGTNNNLGIATLHLAPFIILFTIG (SEQ ID NO: 978)
>orf02497
   VKNGYLVPKTCYKTLGHLRSQGNLRYQQNSCLALIQGTLDNLQVNLGLPTSCNPLK (SEQ ID NO: 979)
>orf02498
   MVNLIPRLGLDLLLIDCLIFQTKQAFSSQTHHFSLLGKV (SEQ ID NO: 980)
>orf02499
   LGLQTKNNPLNQAIPLTKRHMNPHPNFQHSLKFLRNPVTIGLVRLHQGHIYDNLS (SEQ ID NO: 981)
>orf02502 
LGNHFCTICSTTYQAILQFIQIWWCQEDKDSIWNLFLDLKSTLNFNFKENIDSLVQGFID
IGQRSSIVVADIFCVFQHLSLTNQLFKFFTSTEEIVNTVHFSRTLCACRHRYRILKLVFR
TLKNLSSNRSFSNP (SEQ ID NO: 982)
>orf02527
   VGCSYICHELVTNHDHFLFVIVEFLHSTVNTKCEGLQGPVNVINPKFLNCSLNAFFGVI (SEQ ID NO: 983)
>orf02523
   LLHLWRSIRVVPSMGGIIQIDQNSLDSLRLQAWDCQIIDCFHSKIWYIIFNRHSGSFC (SEQ ID NO: 984)
>orf02530 
MIGTFAAALVAVLASFIVPIEITLNSANTEIAPPDGIGQVLSNLLLKLVDSPVNALLTAN
YIGILSWAVIFGIAMREASKNSKELLKTIADVTSKIVEWIINLTPFGILGLVFKTISDKG
VGSLANYGILLVLLVTTMLFVAPMVNPLIAFFFMRRNPYPLVWNCLRVSGVTAFFTRSSA
ANIPVNMKLCHDLGLNPDTYSVSIPLGSTINMAGVAITINLLTLVTVNTLGIPVDFATAF
VLSVVAAISACGASGIAGGSLLLIPVACSLFGISNDIAIQIVGVGFVIGVIQDSCETALN
SSTDVLFTAVAEYAATRKK (SEQ ID NO: 985)
>orf02537 
MWNKNRQLRKVKKILNQINRRKEEMALLTDEELAAKTQEFKRRLTAGETLDDILVEAFAV
VREADKRILGMFPYDVQVMGGIVIHQGNVAEMNTGEGKTLTATLPIYLNALSGQGVILVT
TNSYLAKRDAEEMGKVYEFLGLTIRLPFADDEEEKITPKEKKEIYSADIVYTTNSGLGFD
YLIDNLASSEEQKYMPEFNFVLVDEIDSVLLDSAQTPLVISGSPRVQSNFYGIIDTLMTT
LVDGEDYIFKEEKKEVWLTNKGAKIAEKFLGIDNLYAEENNVLARHLVFALRAHTLFKRD
KDYIIRKGEKDQELVLLDQGTGRLMEMTKLQGGLHQAIEAKEHVKLSPETRAMASITYQS
LFKMFNKISGMTGTGKVAEKEFIETYNMSVVRIPTNRPRQRIDYPDNLYITLPEKVYASL
EYIKQYHAKGNPLLVFVGSVEMSQLYSSLLFREGIAHNVLNANNAAREAQIISESGQMGA
VTVATSMAGRGTDIKLGKGVAELGGLIVIGTERMESQRIDLQIRGRSGRQGDPGMSKFFV
SLEDDVIKKFGPSWVHKKYKDYQVQDMTQPEVLKGRKYRKLVEKAQHASDSAGRSARRQT
LEYAESMNIQRDIVYKERNRLIDGSRDLEDVVVDIIERYTEEVAADHYASRELLFHFIVT
NISFHVKEVPDYIDVTDKTAVRSFMKQVIDKELSEKKELLNQHDLYEQFLRLSLLKAIDD
NWVEQVDYLQQLSMAIGGQSASQKNPIVEYYQEAYAGFEAMKEQIHADMVRNLLMGLVEV
TPKGEIVTHFP (SEQ ID NO: 986)
>orf02538 
VSNKLHILQIGNRNWSHYYEIPENIEWHFFWPGSTTAIKKVMKMEGIRTFSGVVIENPDY
LPDLLPLINILTPYTIFYSDICASYSPLVEEFLKKTCAQVTDFSNPRELLRILSKALFKG
QYGDKLTPIDMVVNPYFAGSIRYNGYENLELVGSYGEDFRPLISWKYNIRASEWNPIELW
LEYEKDLSCDIRIVVRNIQDGSTADFIKERIFTTDDMEAAILLDDDFSSFISVSLEAKGN
GRLKIGALHQRLTRYQFGKFVLGGNIIRSKNREEINYFFYPGDFKPPLNVYFSGYRRAEG
FEGFGMMKSLGSPFLLFQDPRIDGGAFYLGDDDFENAVRRVIQHHLDLLGFSNKELILSG
ISMGTYGALYYSSDFEPKAVIVSKPLTNLGLIAERGRLEAPGLFPTAFDILRHHSQGKAD
IDSINILNARFWERFRGADFNQTIFGLSYMKEEDYDPIAYDSLVDSLYSTGARIMVKGTS
GRHNDDNDSTILWFVNFYKMVLEQEFGRKY (SEQ ID NO: 987)
>orf02539 
MYYFIPFLESMNQSWQVDIVPWYQTTHRLEFDDVLHQIRIFKREGIKSKIVLLPYHPHMR
YLLHRQDLLEVEAFSVFDAIQDIENEEIYPLQLKDLAWDEDCDFIYTPFLIAVKQKGELH
AHIEFGTEGFISYITYFKDNQVDFICYFDDRGFLSSLVKYQDNQAVSRYYYNSNAEWQIK
EYLQGIHTKVEVNPRFSHRFRKSTYQSMDEVVWEFFEKFLTAEYKEGESFVLAAQTKYQN
QLLKHLPEHADKILTFFIERNQEDDLNLHHQAVKQAKILISDRQDFLERLKQHYPQFTYK
MHHLPSFDTRLKLGVSQRVKESKLYVQLDLNTPLNSEALYEVLNFVSQNPLTEIVFATFN
AEGYQIEALQKHLFTLISERLNFRDLLKESIISGAENKLEENKEENYRFQIVNLNDEIGL
IRELEYTRLIVDLNPIANIYTQIAGISAGIPQINLSESEYVTHLQNGYILSDLSEFSKAG
HYFLDTLEHWNQALIHSIDKIRQNTGNQFVQKWERWLEEAKSEQ (SEQ ID NO: 988)
>orf02540 
LKKKLKSVVVKRVMWTICFIFVYILGSRLTLPFVNVNDTSFLGGNAAFLAFSTAMTGGNL
RSLSLFSVGLSPWMSAMILWQMFSMSKKLGLGNLPLEIQERRKMILTFIISFIQTLAITL
NLPIQEGVNHDLVLILNILLLISGTFFLVWLSDLNSLLGVGGSVVILMSSMIVSVPENIV
RSIIDLHVNLLFIISLLLISIAFLYIAVRVQKARYRILVNKIMIHNRFKRYSYFDIMLNP
AGGMPFMYAISLVSIPQYLLMLLHIFVPKTRWVDNWIAEFTIGRPVWVYTYIIVLFLLGI
AFAFVNMNGEQIADKMKKSGEYIYDIYPGEDTALYINRLVLRFAVIGSIYILLMAGIPML
IILYEPRYMQLSMLPGLFLMFNGMIFNVKEEINALTLNESYRPLVERK
(SEQ ID NO: 989)
>orf02541 
MKINITNIYGMSGQSTALIAQNETVKIAKKLDFHELSFYFYNIYSDSEGELNSRLDGVLA
KLGYGDIVVYQSPTWNGREYDQAFIRKCKILNTRIITFIHDVPPLMFPSNYYLMSEYIEM
YNQSDLVVVPSEKMKERLIQEGLTVQKIIIQGMWDHVHNYPLKQPSFQKKLSFAGSVERF
GHLSNWSYSTPLDIFSESNYENSNPRVSFKGWKTDPELLFALSEGGFGLVWGTNENPADE
 
EDYYRLNISHKVSTYLAAGIPVVVPSYLSNASFIKEKGLGYVVDSLEEANRLVEETTVEV
YQQMVENVSKVSYALKEGYFTKKLLTDAIMQLLDI (SEQ ID NO: 990)
>orf02543 
MKAIVLAGDKNYLTPILTTIKSILYYNQNVKIYILHQDIPSDWLQELKIQVEKLGSVVEG
IYIGDAIDSEWKTQAHISPIAYARYLISRLITEDRVVYLDSDIIVNGDLSPLFELSLGDY
SLAAVRDVDGNGFNSGMLVIDCQKWREKDVTSMLFDKTVEYMSYLDHTDTDGFNGDQTIF
NLVFQNHWLELDKRFNFQVGHDIIAFYSHWDSHFELDEEPLIIHYTTYRKPWTTLMGYCY
RDLWWSFHDVTFDQISDHYQGRFAVKRVYDFHDINLFTFTDSQDLLYIDELAQSLLDIAF
HIGAYTDMGDILLALDKYPNVYLYPSMVGAVIDEMIEKSDAYLDIHKGSSMDFIVNRYTS
AGRPVLTFDVTNKNQLEEIVVPSQSPLEMIKVIKKLKSDKMETKAIVFGANYQYADKVLT
TIKSICCHNRGLRFYLINSDFPTEWFYNLNRKLTKLDCEIVNARVNSSHISQYKTNIHYA
VFLRYFISDFVEEAKVLYLDCDLVVTRDLSPLFDLELGDYPLAAVKDLGGQIYFGEHIFN
SGVMLINNRLWKQEEVRKQLIEMTNELHDKVAQSDQSILNLLFKDRWLALDFKYNCITLH
THFSDYRPESGTYPPIIHYLTERKPWGLYECSIYRDVWWYYNAQDWSDMSQVTPSLTKDQ
VSQYTGVQYSALVYTFSSDLRNMGYLIENLPDVKFYVAAPVMVADSITDLLAYPNVSVLS
DIAGQPPLIDSLVEGCDFLLDINADIEVDGIIERFRQAGKPVFAFESVVHGEQGQFLYDQ
AHPEEMVLAIEAYCQNGELPVKKFQSYPKVLDIQQSLDYILEHHTSVIRYGDGEMDIMMG
HGIPYQDYDETLAEQLRSMIQLESSPELLVCLSDVFEGLERYNSESVNFWKKHLEHYKDA
YQQYCTASFYGSTFISRPYMDLKDKTASVAHFEKLKQLWDERDILIVEGENSRSGVGNDL
FDNAQSVERIICPSRNAYSKVQVIQEAVEKYADGKLVFLMLGPTAKVLAYHLSQKGIQAI
DLGHVDSEYEWFKMGATSKVKFSHKHTAEHNFDQEIQFVEDEIYNKQVVVRI
(SEQ ID NO: 991)
>orf02545 
MTDKASKKAIVLGADSNYMDKVETTIKSVCSHNRDIRFYIFNSDFPTEWFQLMNKRLSVL
NSEIINIKITDDTISHFHLPTPHLSSATYLRYFIPNFVFEKKVLYLDSDIVVTSSLTALF
DIDLDGYPLGVVPDIPTTDEEFNSGVLLIDTNRWREEDIYRQLFELTIAHHEHVYGDQGI
FNILFKDRWKRLDITYNLQVGVDAHRYYMGDYDWYELFEGVPCIIHYTTENKPWKHFRFN
RFRDVWWFYYGLNWNDILLRTHVLKETFLELISPISKHVSIFTNTGDIESIGYLLEKLPD
VQFHIVAPTYFSPNVIELQRYSNCYIYPCADPKMKQDIIDKTDICLDINYGPAMDQMLQE
MVRRGKTIYSFDCTNHFFNGESTVFTVDEIDELIRSVKELKM
(SEQ ID NO: 992)
>orf02546 
LKDLVSVVIPVYNVENYLEECIQSVLNQTYTNLDIVLVNDGSTDASAEICARFAEIDGRV
RVFHTENRGAALSKNFGVTQALGEYVLFVDSDDIAEKRMVETLYRQVEETGADIVIGNYF
LYDENDGQYKLYVLERDFCIEELSAQELIDRQAGKWHLNASAFIMPVFKLFKKDLLLQVP
LLMVGALMMKQRFIDCS (SEQ ID NO: 993)
>orf02550 
MRGGVDTTQVMTETVEDKVSHSITGLDILKGIAAVGAVISGTVATQTKVFTNESAVLEKT
VEKTDALATNGTVVLGTISTSNSASSTSLSASESASTSASESASTSASTSASTSASESAS
TSASTSISASSTVVGSQTAAATEATAKKVEEDRKKPASDYVASVTNVNLQSYAKRRKRSV
DSIEQLLASIKNAAVFSGNTIVNGAPAINASLNIAKSETKVYTGTGKDSFYNIPIYYQLT
VINDGSKLTFTYTVTYVNPKTKTLGNISKKMSNGYSIYNTGTSIQTMLTLGSGLGKPSGV
KNSITDKNGKQVRPYNTSTMTMWRSGYTWANGAQMNGFFAKKGYGLTSSWTVPITGTDTS
FTFTPYAAKTDRIGTNYFNGTRKVVESSTTSQSLSQSKSLSVSASQSASASASTSASASA
STSTSASTLASTSASTSASTSASTSASTSASTSASTSASTSASTSASTSASTSASTSAST
SASTSASTSASTSASTSASTSASTSASTSASTSASTSASTSASTSASTSASTSASTSAST
SASTSASTSASTSASTSASTSASTSASTSASTSASTSASTSASTSASTSASTSASTSAST
SASTSASTSASTSASTSASTSASTSASTSASTSASTSASTSASTSASTSASTSASTSAST
SASTSASTSASTSASTSASTSASTSASTSASTSTSTSASTSASTSASTSASTSASTSAST
SASTSASTSASTSASTSASTSASTSASTSASTSASTSTSTSASTSASTSASTSASTSAST
SASTSASTSASTSASTSASTSASTSASTSASTSASTSASTSASTSASTSASTSASTSAST
SASTSASTSASTSASTSASTSASTSASTSASTSASASASTSASTSASASASTSASTSASA
SASTSASTSASTSASTSASTSASTSASTSASASASTSASTSASTSASTSASTSASTSAST
SASTSASTSASTSASTSASTSASTSASTSASTSASTSASTSASTSASASTSASASASTSA
SASESASTSASASASTSASVSTSESTSTSGSTNSRPQETITNSRQELPNTGEKQSVKSGL
LGLILGLTGLGLVAKRRKRDDED (SEQ ID NO: 994)
>orf02551 LKAFVEAHPDAFLREIAARFDCALPSVWAVLKQIKVILKKTTSFKEQKPEKVSEFLDILD 
NLKDLPVLYIDETGINRYLYRPYAGAPRGEKVYDKISGRRFERTNEVEQKLNGSFLIRYI
DSQIRE (SEQ ID NO: 995)
>orf02552
   MAYSTDFKQGALDSIKEGHRHVEAAKVFDVGVRTLFTWEKKDVNKGT (SEQ ID NO: 996)
>orf02559 
MKIKEQTRKLAAGCSKQCFEVVDETDEVSLKHCFEVADRTDEVSNHTYDKVKLTWFEEIF
EEYHTKKPCSSR (SEQ ID NO: 997)
>or02561 
MKKSDVLDLIKYHYEGRETEFRNQSIAIARNFNKHGDTQIAQYIMGLMSQSDRFMPQIEN
PSEYLTPAKLDIGPLPLPLSIMNDLKGIINAVNHHIGINKFLFVGSPGTGKTESVKQVAR
LIGKELLVVDFSHLVDSKLGQTVKNLATLFNEINNLPFKQNYIILFDEIDSIVLDRVNQN
DLREMGRVTSAFLKELDRLSPEIVLIATTNLFENLDKAVTRRFDAIIDFDRYTDEDKVEV
ATIILNELLKQFKNVARDLKLFKKIINSANVIPNPGDLRNSIRTSLAFSDPSDPHDYQKR
LLRSLHNGRNLSISKLSKLGFTVREIEILTGISKSSVSRELSED
(SEQ ID NO: 998)
>orf02597
   MRFIVGIFISFSPEIEFLSSLKFLTDFVEICLLWQVMTP (SEQ ID NO: 999)
>orf02598 
VVEIIYFLIIIIASGLGSISGMGGGIIIKPLMDSFGYHSVSDIAFYSSFSVFIMAIISTT
KRFSQSKEIKWRLIFTVSFSSVLGGFLGHLIFQVLLSQLSVRLVSIVQMILLFVMLLVSF
VLTDFKKTYQFDKIGFYMICGLLLGLISSFLGIGGGPLNVSLLMVFFSISIKEATMYSLA
IIFFSQLSHLATIVVVTGLNQYHLAPVPVIFLASICGGVLGTVVSKVLPENWVRYCFKGM
LFFVMGMTLYNLFHIL (SEQ ID NO: 1000)
>orf02599 
MMGTNSEEGFLDDFEGPQVAVSVKDFSIADTPVTNQEFAQFVKETGYKTLAERQEWSFVF
ILFVPEAEREGYPHPAGAPWWLQVSNACWKHPYGENSNLVGLEDHPVVYVALEDALAFCN
WSGMSLPTEAQWEYAARGGRQSEYPWGDTLLEGGYYHANTWQGRFPYENTALDGFIGTAP
VYEFLPNDFGLYQMIGNVWEWCRNPRYTLLASFNEDDYELPKYGIQDEEYAIRGGSFLCH
CSYCNRYRVAARNGCISTSTSSHLGFRCLKE (SEQ ID NO: 1001)
>orf02602 
MVQTKQPNIILIVVDQMRADALSLNSKDKLVSTPTLDMMASVGYNFENAYSPVPSCVPAR
AALLTGLDQDKSGRVGYQDEVPWNFTNTLPKVFKDMGYQTECIGKMHVFPSRQRLGFDHV
LLHDGYLHVDRKYDKAYGSQFDYASDYLAFLKGKVGYDVDLIDDGMDCNSWEARPWDKDE
KLHPTNWVVSESISFLQRRDPTVPFFLKMSFEKPHAPLNPPKYYFDIYMERLPQFLDLHI
GNWEVLEKQIPSIYALRGKLKEDDQRRMVAAYFGLITHIDHQISRFLTALKEFRHDKDTI
IWFVSDHGDQLGEHYLFRKGYPYQGSIHIPSFIYDPAGLIAGNRGTIKQLVKIQDIFPSL
VDLAGGTTTDELDGRSVKNLLFGQYEGWRTEFHGEHALGKDSSQYILTDQWKFIWFPVLN
HYQLFDMKKDPHEMNDLYPSEKYQPIVRQMKKKLVDFLRYREEGFVVDEELVPVELSKIT
PTLTKTGDSQS (SEQ ID NO: 1002)
>orf02604 
MNTMLDKMQEKLSPIAMKVENQKFLVALRDSFVGTMPVIMTGSIALLLNAFLVDLPQQFH
LESITKTFQWLVDINNLVFKGSIPIVSLLFIYCLGVNIAKIYKVDTVSAGLVSLASFVIS
IGSTVTKSFPLANVGDVKLDQILQGIDNLAFDGKNLMVTIGNVIPGNHINARGYFTAMMI
GFLASIIFCKVMKKNWVIKLPDSVPPAIAKPFTSIIPGFMAMYIVAILTYVFHLLSNDLL
IDWVYKVLQTPLLGLSQSFFAVILMIFLNKLFWFFGLHGGNVLAPIMEGLFGVAMLANLD
AFQKGEPIPYIWTSGSFGAFVWFGGLGLVLAILIFSRNSHYRKVAKLGLAPVLFNIGEPV
NYGLPVVLNPLLFIPFVLSPVFMATVAYWATSWGLVSPVTQNVTWVMPPILYGFFSTAFD
WRAIILSVVCLIISVLTYFPFVKMADKTELS (SEQ ID NO: 1003)
>orf02605 
MNESNLESVMGLIMYGGEAKSNAMEAIQAAKKGDFSKANRRLADANAALLQAHKAQTEML
TREAQGEKTSISLLMVHAQDHLMTSLTFVDLAKEVVEVYERFEKN
(SEQ ID NO: 1004)
>orf02606 
MAKVTIMLACAAGMSTSLLVTKMQKAAEDKGLDAEIFAVPAPEAEEIVATKEVNVLLLGP
QVRYLLGDFQEKLKDRQIPVAVIPMTDYGMMNGSKVLDLAESLLD
(SEQ ID NO: 1005)
>orf02607 
MKRLISANPSEILQMNAEELKQSILASEGRVVLSENVVTRETFVGDITNSEIARAFGADM
ILLNCVDVFEPKIYALDSSGDDVIHRLHQLVACPIGVNLEPIDPSAKMLEETQEIVAGRV
ASVETLNRIEELGFDFVCLTGNPGTGVSNREIIKAVQTAKENFSGLIIAGKMHGAGVNEP
VAELSVAEQLLEAGADVILVPAVGTVPAFHDQELREVVDLVHSKGGLVLSAIGTSQETSD
TDTIKEIALRNKICGVDIQHIGDAGYGGLATVDNIYALSKAIRGVRHTVSRLARSVNR
(SEQ ID NO: 1006)
>orf02608 
MEKLLQEKLLPVAARLGNNKALVSIRDGITLTIPLLLIGSLLMVIASFPIPGWEKYLGDI
GVADYLWKGVDSSFGLLGLVASFGIAYFMARQYKVDGIPAGIVSLSSFITVTPFIRGEAG
AGMPTAFMASKGLFVAMILGLINGYIYQWFINHNIQIKMPDGVPPAVSKSFSAIIPGAVT
IVGWLIVYATLDKLSLPNLHEIAQVALGGPLGLLGNNVIGLLILIFLNSSFWFVGLHGGN
VVNAVMKPLWLANLDANKVAYQTGETLPNIFTSVFMDNFVFIGGGGATIGLVLALGYLAH
KKKASKQLKTLAPITVIPGLFNINEPAMFGVPIVLNILLLVPFILAPMFNLLVAWGAMAS
GLVPLTYTDPGWTMPPVISGLLATGSISGSLLQIVLIVLDVLLYLPFVIAIEKRFKLLED
(SEQ ID NO: 1007)
>orf02609 
MTLSKKQLQLRAKILETVYTLGPISRIEIATKTGITPATTSSITNDLIKENILLELGEDE
HDTSVGRKKILLDIQAKRFYYIGCELSEKHFTFALGDNLGNILKEEKEIVTKQLIQEKGN
QLINQTLKQFLNNCSDYEIEAIGIALPGRYLDDYKITTNNPLWQHIDLEMIQSHFDKPLF
FSNNVNCMAIGKRLFSRQQNDPNFAYFHFARGMHCSYIYDGNIYGKGNLMIGEIGHTVVS
SEGEECSCGRKGCLQTFAGESWLIKKSKILYHQSPYSLLPSLVKNADDIDIQVILTAYQL
GDTGIITLIHQALLYLSQTILNISMMIDSQKIYLHSPLLTNQHIIQKLYSEMNYKPKLLY
NRLPEVIIEPYNDFTAAHSAIALCLYHTILHS (SEQ ID NO: 1008)
>orf02628 
MPFKENLICQHRNHHCSVFFISLGLLHNIHIEIDISQTRASFLDLSDYLQAVLMILQKFC
QAIGLAQRLDLLQLHLLHLTRLLL (SEQ ID NO: 1009)
>orf02636
   MYLLLLVVKDHIALIDKEMHVWRPNCILRDLTNFFIKRNHIVTHKTNGSTTKR (SEQ ID NO: 1010)
>orf02637
   VLTLMNHFIKEIQGISINHLTILIKDISIFKLNLKNRIIG (SEQ ID NO: 1011)
>or02641 
MKIKEQTRKLAAGCSKPCFEVVDRTDEVSSKYCFEVVDRTDEVSSKHCFEVADRTDEVSN
HTYGKATLTRFEEIFEEYKGVPR (SEQ ID NO: 1012)
>orf02655
   VCQRMDARTCKTTIIAVHNVLTALQQTMIAVQLYQTK (SEQ ID N0: 1013)
>orf02656
   LHLGKSILSLPVKGKDLEFLVHLFVINHWIGSPSRTSTFCRCKVLNGME (SEQ ID NO: 1014)
>orf02657
   LEQTVIIANDIPCELYWDNHLSFLSDSLLIVQVIVHLKRICLDIHHDRGCSHVRNDTT (SEQ ID NO: 1015)
>orf02673 
MLEEGTKDQLAELTYPFGRGVNLSFGIKDVPKLYQKVMEANYPIYRLLTKRKFRVSDPYI
YPHKFAVLDPDGYFLRFSE (SEQ ID NO: 1016)
>orf02689 
MIACRHDICKSQKGLEHPFCIIRRLTRDFNQRPVCIVEANIFCLKITPQIITNMIVARTV
KSSKTGITLTTSMCKRDNHKITWFHRRNGFPSFFNNPNRFVSTIFMSSFRFWITVPP
(SEQ ID NO: 1017)
>orf02705 
MNMNKDQIAILNGADNLNLTLWITLKEICKEGCKSFFPVRNTCRMLDIGIPYRLGLSLSN
SSVLNGMDV (SEQ ID NO: 1018)
>orf02725 
MHKLRIFVNQLCRRFGIILGPFLVLGFQVLTQELELAIFFDLREEVLLQVIPQVCHFCYL
RKEFTTLNQHELTSHDHVLTRHFQTHGLQG (SEQ ID NO: 1019)
>orf02732 
MQVTIEADSGLFLLSPVVQLLKVEIDVKQVTMSLDNLGRTKLSHQTFWVAGVEVHVPFNN
ADALPKWRIRT (SEQ ID NO: 1020)
>orf02734
   MKNGIDFAHIAITDFFHNQAIAMGIAHYNDGLLCHDGNTSKSFLTAKAR (SEQ ID NO: 1021)
>orf02756 
MSIVKSHSFSISLGIFNSFWNNIHTSECFYFLCKGKSNRSNSTISVNQMVFFINIQRFYC
FAIEDFCLLRI (SEQ ID NO: 1022)
>orf02759
   LNTLLPPDNLCLFTIYLTGFSCICINSYCHNFWEIFNQLFYQLS (SEQ ID NO: 1023)
>orf02778 
MYNKVIMIGRLTSTPELHKTNNDKSVARATIAVNRRYKDQNGEREADFVNMVLWGRLAET
LASYATKGSLISVDGELRTRRFEKNGQMNYVTEVLVTGFQLLESRAQRAMRENNAGLKGQ
IWXXIHSLIN(SEQ ID NO: 1024)
>orf02782 
MQFTRTTHHPKTLFTTKFAWENEIPFWHHGTRKSHNGFQPNTRIRCSCNDLHYLVTCDCN
LADVEVVTVWMSYHLDNFTNNKLRFLIINNFFCKTFRL (SEQ ID NO: 1025)
>orf02784 
LTALCNFKQARNLKNVPSYCFPIFFEENTGYLAFAFHIQFGISAIFHDDHKDITDMFFVI
RKNHCFLFLLFQSC (SEQ ID NO: 1026)
>orf02788
   MKIKDQTRKLAAGCSKHCFEVVDRTDEVSSKHCFEVVDEADVV (SEQ ID NO: 1027)
>orf02789 
MYYSVDDVVSNAFKKRMILDSFFAFNCSGTMKVSTWVYDKGEWYYVSSSGSMIANDWVKD
NGK (SEQ ID NO: 1028)
>orf02793
   LTWILTIKIARKDSLQLFELEANLISFLLVMSVDLAPFCFKEENF (SEQ ID NO: 1029)
>orf02803
   MEDIDEDELLIFEKVLGQLQANIKGIGGENKEISQKN (SEQ ID NO: 1030)
>orf02804 
LHLRTCFVRQTNKLSPLINRTRLQFHQTILHYTLNQITSNRLGNIEFLLDIFNQDQVLVF
LAIIQKTHNLTLRPTHKFNAATFGFLLHHQVNLMTKTLKD (SEQ ID NO: 1031)
>orf02821 
MLEIWKYRPFVSEFWNDFKNNHDKQFVDPISLYLTLKDDDDPRIEEESEALENMILQYLG
EDDAS (SEQ ID NO: 1032)
>orf02823 
MQDLLFHFYSYRLNLTFFFFELLICLLNSEFDLSKFIFVYFDEYFHEDSLKMNLHQFSFS
F (SEQ ID NO: 1033)
>orf02829
   MAFNQFNRCIGLSIPTAPNVPGTIINRSYLHDATVPNNVREKT (SEQ ID NO: 1034)
>orf02845
   LTDFHDFKFIFFENLFKSRQLYLQSQNSVLSNLWLAT (SEQ ID NO: 1035)
>orf02850 
MKKLFILISNLLASLFFVWVFTIWTDTYVSHYYPNVVVHDSSPETTFQHVATRLEKLAEE
TDSFIAIQHQDPNSEGTTVFSYTTFGDGKLPDGLQEKNLEDAQSSSVETNYFVFDGHLDI
HLLREELSQLGLTNMHLTIPSKLSTLMAIFSNGFQLISLLIFILTFVALTLISQISQLRS
SGIRLISGEKRWSIFLRPVGEDLKAIAVGFSLAGVLAILMQKILSLPTQSLMTIGAGLLS
YNLILLSISLFFAQLFAVGIKKIHLMQIIKGQVPVRGIISLILIGQLLAIIIVTLGIGSS
LKYSQAWQQHRIGQEIWSQERQLITLSISREGTSPGFDEQAQRKLRTWYQLMDLAVSEQK
AFLSRHQLIDRTLQNGMASSKNLITSTEWHDYNPNGNVLIVTPQYLERQNIPVDTTIEQK
MNHLNVGEFVLLLPEHLRSEEEHYKSVFEDDLTSRMSSQDERQQMTATVGYLESGQDRFV
YNTTPISYQQFLKDPIIIVITPQSTGPQSILFWIDAVQNYVLFNQLSDAQELIQRQGIEN
WVSEMQTGYHNYITLLDNIQRERWVMLAGAVLGIATSILLFNTMNRLYFEEFRRAIFIKR
IAGLRFLEIHRTYLFAQLGVFLLGFVASVFLQVEIGVAFLVLLLFTGLSLLQLHVQMQKE
NKMSMLVLKGG (SEQ ID NO: 1036)
>orfO28S9 
VLKWCILRINHHISRKVDNFLEGTRAHIKGQAHTAWNPLEVPDVRYRSFQFDMSHTLTTN
FRTRYFNPTAVTNNSSVTNAFVLTTSTFPVFCRTKDHFIKESFTFWFQGTIINCFRFFDF
SIRP (SEQ ID NO: 1037)
>orf02869
   MPWKELCHKLAPKVFKVIRIYSRENKKSPSHWAFCSFET (SEQ ID NO: 1038)
>orf02877
   VDSLFLSLGEESNQEINLQESFSSTDCNPTLISPETTVAQGLCQDIIYRPFT (SEQ ID NO: 1039)
>orf02880 
VNPKSLGSFFLQDSKGFKELVLGHAKLSLPRIVHNVCPQFKNASRIITTRDDFWNACYSL
QMFNIFKGIQVNGRTQFTCIGVFLVWRVVGREHNLRTQKVQFMAHQKLYITRAVHTTTFF
LENFQNSWSWSSLNCKIFLKALVPRKSLVDGSCLLTNPLLIIQVKGSRELGNNRF
(SEQ ID NO: 1040)
>orf02884 
MDNLCFHNAWTDWASILKQAVVTEDDMTKQNDFFLGIIDAEFHNCLGNFAINESDMSKKI
TSHCVLCLVWPRQLDDLS (SEQ ID NO: 1041)
>orf02885 
MQHNPRIEQALIELRINFANSVCQTHHGRRMIGQARFKGMVVGLGSWIGVEFLIILGVEI
SDNPLPDRIFNFENHLRHVVTNFLDINW (SEQ ID NO: 1042)
>orf02886
   LIDLRGIVIDIFSASFHVDNLTCGKGLNVMRLGIPELPINLATIILEGKG (SEQ ID NO: 1043)
>orf02899
   MDALVLQKNQETIQQIAVKIRFLDGHDYYSLIDIDNRRTNQTVFPFVNF (SEQ ID NO: 1044)
>orf02900
   MAFFTEIPTRACLINLAITLHIVETCQGFNDLSLHLRVLAL (SEQ ID NO: 1045)
>orf02904
   MLLPLFFNTSKIKQIAMHSDLNQKEMIGHIFHDEDIF (SEQ ID NO: 1046)
>orf02909 
MKQTVKKLALVASIAATLGGGVSVASAAVQYPEGGVWTYGSGNGGAYSNYYHPSKYHSST
VVSRKTGSSDKGYAGAGGTSRAWIRTSWGEKVAFYYNV (SEQ ID NO: 1047)
>orf02919 
MNQENLFLLQEIKDKLLIIIDTVHIAVDFWEDIEPRLGFNGRQTWNILNGIIDEISLLVD
SSTRKKQFID (SEQ ID NO: 1048)
>orf02920
   LLGQNVRAKAHIGQHIEPFDIALNMSLRARQDHPTHTETCYAVGF (SEQ ID NO: 1049)
>orf02921
   MSVHYHAVIDFIRKDNQIVLTGNLHNLQQEFLRIKGSSWVIWIDKDDCLGIGSDF (SEQ ID NO: 1050)
>orf02923 
VTYNRIRQTSHPLCNQKCQQQGDAYNPDSLVNKNSFDITILITNCLHDTYFLGTLHDIDV
NNDTNHNRCYHNSL (SEQ ID NO: 1051)
>orf02924
   LIRQHETVAVLHVIFIIDYTYNLRLKLSNLTSFGSTFYNAG (SEQ ID NO: 1052)
>orf02954
   LHTSFRSSVGHSHTWHQDIVRPILFSRFNDSIVILWQNCPTFN (SEQ ID NO: 1053)
>orf02968 
LRLAKLVPSLKIALKSFSLFTRKFFFKTQLHLLNYYYYTIFFKKANHSKVLDNFIRKRSW
KNNFPNSLY (SEQ ID NO: 1054)
>orf02973 
LKAEQQAIKNIQFLEQDLPKNPLEKEFDCLAVSRVLHHMPDLDADLSLFHQHLKEDGKLI
IADFTKTEANHHGFDLAELENKLIEHGFSSVHSQILYSAEDLFQGNHSEFFLTVSQKSLA
(SEQ ID NO: 1055)
>orf02974 
MKHDFNHKAETFDSPKNIFLANLVCQAVEKQIDILSDKVILDFGGGTGLLALPLAKQAKS
VTLVDISEKMLE (SEQ ID NO: 1056)
>orf02978 
MVDLQSFFTRKYLNLNSVDAYLILPRLQGHLSYPQDFFLLQDFCFLLPIFLNLSQKEGRN
AGKDS (SEQ ID NO: 1057)
>orf02991 
VTENPAPFVFTVSINSFFTVVAFTTGTDARNQDLVTFFEVGNSFPNFFNNPNPFVAKNGT
TLASRNIPFDNMEVCSTNSGFYNTHNSICWLANNWFVYINKRSKSWFNIRXXIPFIN
(SEQ ID NO: 1058)
>orf02993 
MRIRNSPFDHILQTIFEFEDRTCQVTCRFEACSSICNDNWEFSQHIISVFQSPSCHTVCD
KSDVFCSFLFDKNFASLWIYVVTITDQLCIGMWQLVHGSNHTQFTVSQPTHSIVSMHPNT
RSSIDCFFGFIKSRV (SEQ ID NO: 1059)
>orf02994 
MSKSNRHTFARNCTNKVFHPITFWCKGNFIKQAICRFLPRMKLLNTRVSHISWILCPLKS
FCEIWTFIINPTNLSTCCFFIMVSKIFSDCKQLLISGC (SEQ ID NO: 1060)
>orf02996 
MQCTFNVVVHHIYTCISMNMSIHKSWGNAITCIVNHLSPFRNLLYMFPKLAVHKFQVTTS
TNSVWVEKLIRFNIVRHNVNLLKRLILQFIMSITL (SEQ ID NO: 1061)
>orf03011
   MTLHQTFRFQNLEMPCQSSLINFQTLLNRHLVTRRMLQQKQ (SEQ ID NO: 1062)
>orf03023
   LLSSFQDAVKFFAVVFFRKVQPSQEVAPNASSFTDQFMGG (SEQ ID NO: 1063)
>orf03025
   MWTTFNDFSIFKDNNLICIENGFQAVGNDETSSTCYNHLHGMLNLAFRHRIYV (SEQ ID NO: 1064)
>orf03031 
MAEFNSVITTVTGIGDRLGAVILAEIRNIHAFDNPAQLQAFAGLDSSIYQSDQIDLAGRM
VKRSSPHLR (SEQ ID NO: 1065)
>orf03041 
MQQYVDIKKQYPDAFLLFRMGDFYELFYEDAVNAAQILEISLTSRNKNADNPIPMAGVPY
HSAQQYIDVLIEQGYKVAIAEQMEDPKQAVGVVKREVVQVITPGTVVDSSKPDSQNNFLV
SIDREGNQFGLAYMDLVTGDFYVTGLLDFTLVCGEIRNLKAREVVLGYDLSEEEEQILSR
QMNLVLSYEKESFEDLHLLDLRLATVEQTASSKLLQYVHRTQMRELNHLKPVIRYEIKDF
LQMDYATKASLDLVENARSGKKQGSLFWLLDETKTAMGMRLLRSWIHRPLIDKERIVQRQ
EVVQVFLDHFFERSDLTDSLKGVYDIERLASRVSFGKTNPKDLLQLATTLSSVPRIRAIL
EGMEQPTLAYLIAQLDAIPELESLISAAIAPEAPHVITDGGIIRTGFDETLDKYRCVLRE
GTSWIAEIEAKERENSGISTLKIDYNKKDGYYFHVTNSQLGNVPAHFFRKATLKNSERFG
TEELARIEGDMLEAREKSANLEYEIFMRIREEVGKYIQRLQALAQGIATVDVLQSLAVVA
ETQHLIRPEFGDDSQIDIRKGRHAVVEKVMGAQTYIPNTIQMAEDTSIQLVTGPNMSGKS
TYMRQLAMTAVMAQLGSYVPAESAHLPIFDAIFTRIGAADDLVSGQSTFMVEMMEANNAI
SHATKNSLILFDELGRGTATYDGMALAQSIIEYIHEHIGAKTLFATHYHELTSLESSLQH
LVNVHVATLEQDGQVTFLHKIEPGPADKSYGIHVAKIAGLPADLLARADKILTQLENQGT
ESPPPMRQTSAVTEQISLFDRAEEHPILAELAKLDVYNMTPMQVMNVLVELKQKL
(SEQ ID NO: 1066)
>orf03051 
LTNLSSVDSEELFQFYRERGNAENFIKERKAGFFGDKTDSSTMIKNEVRMMMGCLAYNLY
LFLKQLAGDEVKALTIKRFRRLFLHIAGKYVSTARRHILKFSSLYAYSKQFQALFDTICQ
INLILPVPYRARGQGKTCLTE (SEQ ID NO: 1067)
>orf03061 
LFDDRQAINICPPTNGSLRLTSLQVDQNPCPPSTNLNKILARSQFLNHIQQISLSLELLQ
ANLWNLV (SEQ ID NO: 1068)
>orf03092 
MKIKVQTRKLAAGCSKHCFEVVDRTDEVSSKHGFEVADRTDEVSSKHGFEVADRTDEVSN
IYTVRRR (SEQ ID NO: 1069)
>orf03093 
MDFFNYLLWMICHNHGLHTLLLSKDCVCHTARDKDGNHRIKSVFPTKGQTCYQHDSSIYQ
ERNTTDILTRFLTNSQADDVRPTTGDIVSKSKTNPQTHNNTPKKGIDNGILCQGCHRDKL
DKEGTHRYRDKGKDGELMANLIPS (SEQ ID NO: 1070)
>orf03104 
MIKQIKAHLNKSIQSIIGQKVEFVKQDEQAFTRKRRLSLETMIRTILGMGGKSLSKELLD
ARLTVSNSALVQRRYQIKPEAFYALFKEFTAPIPLNTDFPIFAADGSDICIPRNPMDTET
SIQTQTDVKSYNLIHINALYDLTTGVYRDVSIQDKHAQHERLALIQMMEASPFRESSCYH
G (SEQ ID NO: 1071)
>orf03108 
MAHFQEKGWLYIIRIRDGKQSMPSSFNLPNTECFDQKVSLKLSRKQTNQLKKLYRDFPND
YHFIPHNSIFDFLPETSRKQDPVTLYELPFRMVRLKVEEGKYETLVTNTDYSVQELKNLY
ASRWGIETSFRDLKYSIGLVNFHAKKKEGILQEIFARFTNFNFCRWVTSQVAIDSSHKKQ
RYKVCFSDAAYACRLFFNGSLSSHQLKNYLKKQLSIIRPNRKYSRKIKAQSVVDFICRVT
(SEQ ID NO: 1072)
>orf03110 
MCELDILHDSLYQFCPELHLKRLNSLTLACHALLDCKTLTLTELGRNLPTKARTKHNIKR
IDRLLGNRHLHKERLAVYRWHASFICSGNTMPIVLVDWSDIREQKRLMVLRASVALHGRS
VTLYEKAFPLSEQCSKKAHDQFLADLASILPSNTTPLIVSDAGFKVPWYKSVEKLGWYWV
SRVREKVQYAP (SEQ ID NO: 1073)
>orf03139
   LSIQVETLELRVIFKEIKEIVKQFHQLHTMAFKRQVPLTIPVTM (SEQ ID NO: 1074)
>orf03154 
MGALGYYEGFVPYVSNQYKNQAEEEGKPLSDKYIFEKILGKTYAAFKKDQINERVEKLGK
LKPITINYNGKSEVIDSKEKLQELMNKAVKDEVAQIS (SEQ ID NO: 1075)
>orf03155 
MMGDGMKEFQFERKQRFSLRKYAIGACSVLLGTSLFFAGMGAQPVQDTETSSALISSHYL
DEQDLSEKLKSELQWFELENKLLNLWEH (SEQ ID NO: 1076)
>orf03159 
VNITKTSIIKAHTTKEDGIDHTFTRFNIMSIFYSTRKIFLDKLNSTNRQFLGYIISTRCY
QSFNSVSQSIHTSSSSQAFRFGKHEFRVINRDKSKAILVNHYHLNLAFFISNHIVNSDFC
RSSCRCIDSHNWQAFFSRLMKPFIILWFSTICSHDRNTTSCILWRTPAKTDDKVTAMFLQ
SSYPICDIFTSRVWLYIAKDDIFDSFCIQWF (SEQ ID NO: 1077)
>orf03175 
MSMDNCIDIITSLILNQMHIPFARWQAFSLYNISINIHHYNIGFFDFKEINTRRGNCHQL
FFTIENAEIPTCSFRQICFY (SEQ ID NO: 1078)
>orf03182
   MNIAIRIILNFFRVMGNHQNSLAMMMGAVVHEFVKFIFTSCIHPRCRLV (SEQ ID NO: 1079)
>orf03183
   MLLIMSIQTTEPAFSRIATRLDKFIDRTWKTSIKTGNLLRKIGYSQFLTLRICL (SEQ ID NO: 1080)
>orf03184 
LQNSKTSLDERRLSRSIFPSQGNKFPTINTIIDMFKNRLLIIIEGQILYRNISHYLISPT
KAVKNR (SEQ ID NO: 1081)
>orf03197 
MSNSFVKLLVSQLFANLADIFFRVTIIANIYIISKSVIATSLVPILIGISSFVASLLVPL
VTKRLALNRVLSLSQFGKTILLAILVGMFTVMQSVAPLVTYLFVVAISILDGFAAPVSYA
IVPRYATDLGKANSALSMTGEAVQLIGWGLGGLLFATIGLLPTTFIILVLYIISSFLMLF
LPNAEVEVLESETNLEILLKGWKLVARNPRLRLFVSANLLEIFSNTIWVSSIILVFVTEL
LNKTESYWGYSNTAYSIGIIISGLIAFRLSEKFLAAKWESILFPLVAMAIVTLTILYFPN
AQMFLLFSALVGMLSQLKEVPESVFLQETVEENHLVNVYSVLEVISTLAFSVFVLLMSYI
TESFGISISFWLSAICLMIEAILIYIRRDYFK (SEQ ID NO: 1082)
>orf03198 
MSKLLDKILSRENMLEAYNQVKSNKGSAGIDGMTIEEMDNYLRQNWRLTKELIKQRKYKP
QPVLKVEIPKPDGGIRQLGIPTVMDRMIQQAIVQVMSPICEPHFSDTSYDFRPNRSCEKA
IMKLLEYLNDGYEWIVDIDLEKFFDTVPQDRLMSLVHNIIEDGDTESLIRKYLHSGVIIN
GQRYKTLVGTPQGGNLSPLLSNIMLNELDKELEKRGLRFVRYADDCVITVGSEAASKRVM
YSVSRFIEKRLGLKVNMTKRVEISRFWVLEIIRWLEKPSTSR (SEQ ID NO: 1083)
>orf03202 
MFLRCATFKLADSRLNIFTCFFFGEIRFNSRNQVVKAFITDGTVISTIIVRGTVPCNQWT
KTCPAAFDIINGDVGFWKAVVDNAK (SEQ ID NO: 1084)
>orf03203
   MLQITCVVCISCTKVSLVFTWENKDHTTVTQTCVKVNWL (SEQ ID NO: 1085)
>orf03204 
LRSLIRQITYFITPRTCCINNQTGLDFKHLVCQEITSYNTCNLATFVKEEAFCLHVVGNE
GTVLVGTFDVFNHETRIVVTEVKIHSTSYQAFLLQVWLAFQDLILAQNLVRSWCVAHTC
(SEQ ID NO: 1086)
>orf03205
   LHFNQTSLKTASCRLQGYTSSCDSSTDNQEVQGAFLHFFN (SEQ ID NO: 1087)
>or03216
   LKIDHTQLSPSNLLNTFVTPFIFYLKHSINLTNAEIICFSFYFHADFLVHYPENQ (SEQ ID NO: 1088)
>orf03224 
LKKVQHTQNVDFNKKLSRIKTKYLYGLKEKSEAELTLKTKETKEELTAAFEQFKKDTLKS
GKKVAEAEKKAKAQKEEDRRNYPTNTYKTIELEIAEAEVGVAKAELELEFAQAQVQIPQD
TEKINAAKSKVEAAKSNVKKLEKIKSDIEKTYLYKLDNSTKETPKSRVRRNSPQVGDSRE
LKETIDKAKETLSTYMVTRLTKLDPSVFWFADLLMDAKKVVEEYKTKLEDASDKKSVEDL
RKEAEGKIESLIVTHQNREKENQPAPQPGGQAGGSMVVPPVTQTPPSTSQSPGQKATEAE
KKKLQDLIRQFQEALNKLDDETKTVPDGAKLTGEAGKAYNETRTYAKEVVDKSKKLLSQT
AVTMDELAMQLTKLNDAMSKLKEAKAKLVPEVKPQPENPEPKPQPEGEKPSVPDINQEKE
KAKLAIATYMSKILDDIKKHHLKKEKHHQIVALIKDLDKLKKQALSEIDNVNTKVEIENT
VHKVFADMDTVVTKFQKGLIQNTPQVPEAPKSPEVPKVSDTPKAPDTPQVPEAPKSPEVP
KVPEAPKAPDTPQVPEAPKSPEVPKVPDTPKAPDTPQVPEAPKSPEVPKVPDTPKAPDTP
QVPEAPKAPDTPQIPEAPAPETPKTGWKQENGMWYFYNTDGSMATGWLEYNGSWYYLNAN
GAMATGWLEYNGSWYYLNTNGAMETGWLEYNGSWYYLNTNGAMETGWLEYNGSWYYLNTN
GAMETGWLEYNGSWYYLNTNGAMETGWLEYNGSWYYLNTNGAMETGWLEYNGSWYYLNTN
GAMETGWLEYNGSWYYLNANGSMATGWLKDGDTWYYLEASGAMKESQWFKVSDKWYYVNG
SGALAVNTTVGGYRVNANGKWVN (SEQ ID NO: 1089)
>orf03230
   MDREILKFFQDLLSILSHNDMITLFCQKCCNSFSNHFLVICN (SEQ ID NO: 1090)
>orf03232
   MFITLRRICLRACVVEKEQSYLKFLFFQKRPVSFLHVKSVLAGI (SEQ ID NO: 1091)
>orf03233
   MVKTTNRLEAIGFSFILFENLFKPRQLYLQPQTSVLSNLRLAA (SEQ ID NO: 1092)
>orf03239
   MTRKLNPSYTNVASATTLTFNQVASTFRKACLOHVVNLTRNNLKGICQLTPLQLHOTRLI (SEQ ID NO: 1093)
>orf03270 
MRTFFLYSSAFKKHSSPSPINDGLYHLLLQSLYNILELIHDIFQSLKGFILKSTFTNLFP
HLFNGVHLWCVWRNKCKANISRNL (SEQ ID NO: 1094)
>orf03277
   LVSVFYSLLQVDNVDSVTFSKDVLSHLRIPATSLVTKVYTSLKKLFH (SEQ ID NO: 1095)
>orf03286
   LIVWILKNHTDLTTYIPNIFLSQTLAINYNLSRFCFQ (SEQ ID NO: 1096)
>orf03287
   MPYNRKPFSTFHVKRNILHIVVVLIFFITKRKIFYINY (SEQ ID NO: 1097)
>orf03291 
MFKKMSNSSRILFYISVNFCDKRIYRTKLYSDTPVNLFKFLFRQKSNCQSIGQTSSINLF
FYSWIVFFFKNNLCHSIPSIK (SEQ ID NO: 1098)
>orf03304 
LADGSGKLAEGGTKLTSGLEDLQTGLASLGQGLGNASDQLKSVSTESKNAEILSNPLNLS
KTDNDQVPVNGIAIAPYMISVALFFAAISTNMIFAKLPSGRHPESRWAWLKS
(SEQ ID NO: 1099)
>orf03310
   MKNTVKLEQFVALKEKDLQKIKGGEMRLSKFFRDFILQRKK (SEQ ID NO: 1100)
>orf03330
   LVEQLTFNQWVTGSSPVRVIYAGLAELADAPDLGSGA (SEQ ID NO: 1101)
>orf03344
   MKIKEQTRKLAAGCSKHCFEVVDETDEVSNHTYGKVKLTWFEEIFE (SEQ ID NO: 1102)
>orf03352 
LIDVLFINSFIGRICFYCYRRIHATCLFLQLFSIVILNVAHTLKHSIFIVITFISRCRNF
IIVRILLENQFSRNQGIDNRVGQSRY (SEQ ID NO: 1103)
>orf03353
   MVNVNQVSIEVKNTFKNWNFTSSIELTTFSKFSQSPTMT (SEQ ID NO: 1104)
>orf03364
   MGFSMKLIHDLDMHTTHSTAKMLYNVKAIKNDFSIRE (SEQ ID NO: 1105)
>orf03368
   LKSVGSRVEDKRYQETIVATFSDILKRSREMQDQNIKXXFIH (SEQ ID NO: 1106)
>orf03372
   MLTIEPTKAPLVNVCDTANTARIDDEPYSPGDFXYHSIH (SEQ ID NO: 1107)
>orf03373 
MRWNIGCHPNRDTSCSINQKVWKTRWQDQGFPFIGIIVINEINCIFVDITKHFQSNLAHT
CLGITLSGSTISIHGTKIPMTIYKHVTVAPPLSHTDHGFINRGIPVWVIFTHDIPCNTSR
FFMGFVWGHTQFIHSVKNATVNRF (SEQ ID NO: 1108)
>orf03380
   MTDFNTFLQLSEGWSLFRSDFLLCIKHFLNSFSSSKGQLKASPTRCNLDNRLVVLL (SEQ ID NO: 1109)
>orf03390 
MGGNPPMKKYSIVDKIVLSTKIKRIIIFTVFRENWEPYMKKYTEVFQSQFPNLNIDYLLL
DTEQIDLDSYLDADIIIGGGNTEKYIATYVNQEFKNYIDHMLNKGAKVIGFSAGALLLG
EKVYVSPNDNSDHQIKIKDGLGLFSQFLISVYYDSWNDKANKDRAEELVNVPIIPLNDHS
CLVLDKLGNIIEKID (SEQ ID NO: 1110)
>orf03393 
MYGGEAKSNAMEAIQAAKKGDFSKANRRLADANAALLQAHKAQTEMLTREAQGEKTSISL
LMVHAQDHLMTSLTFVDLAKEVVEVYERFEKN (SEQ ID NO: 1111)
>orf03396
   MLARSKNCFMKSLSIFLLIFYFFDSYQISKKRRSLIGL (SEQ ID NO: 1112)
>orf03399 
VTAHRIFGTSSIHSKLIGLAMLGITAMKIICHKLNRNHINIFRRLGIQGKTEFLLIHLIR
QVKMNHLSQGMNPTICPTSTVNSNGLPFI (SEQ ID NO: 1113)
>orf03402
   MLKNGIISWKDFKSFFCQGCQTSHCYKPMQAVQGIGSQIS (SEQ ID NO: 1114)
>orf03403
   MRFLADQDRIQHHRYSWALFDKVQGLLSHADSREKTNLNSPKFHITQAI (SEQ ID NO: 1115)
>orf03405 
MSYGRPYILNVDGAIHDGWLAISNYENSLNKDYLFYILSSNVVYSQFLSLISGAVVKNLN
SDKVASILIPLPPLAEQQRIIEAIESALEKVDEYAESYNRLEQLDKEFPDKLKKSILQYA
MQGKLVEQDPNDESVEVLLEKIRAEKQKLFEEGKIKKKDLDISIVSQGDDNSYYEEVPCE
IPESWEWVRLNDITSYIQRGKSPKYSNIPIYPVIAQKCNQWSGFSIDLARFIDPETVHSY
QKERLLRDGDLMWNSTGLGTLGRLAIYHENKNPYVWAVADSHVTVIRVLSGVINCHFIYN
 
FLSSPIVQSVIEEKASGSTKQKELLTKTIKEYLIPLPPLPEQSRIVDRIEQFFAHIDALI
(SEQ ID NO: 1116)
>orf03424 
LAQISILHFDFLSIDKHSHTVFNTLRKSLQTTLALSATSKQCFEQLAASFLVCSLIFIEY
KV (SEQ ID NO: 1117)
>orf03430
   MGFKVSHFKIPSSHLSINVLRTVENFTEIGQGLLHISP (SEQ ID NO: 1118)
>orf03431 
VGFFDFGLTNSCRQVRQFTQTVQDFLVCCHQGIVKEGQGYAGICFKFHPSLGNIGKFVIA
IVRRLRHKSIVANMAHLNVDLFQFRKGLLEILKSVKIALVITAKLVDVFASFLDCTQEIL
TVLV (SEQ ID NO: 1119)
>orf03439 
MNITYIVGNGLDLQYGLKTRYRDFYEFQNKVYISRTENEEKYSNFIYESLFSDKVNDYEN
WSDFELSIGKLTKDNDLISSSIEIKEKFIDDFSEVVDDLREYLRIQQEKNLEKGNAIDFI
STLDDMRTSLPVINQPAIDKKYNENPHQDDIVNIVTLNYTHVIDKLYNGSAKSFRNQLRA
NLYNFYIEPPIHAHGTVDVCTVLGVSDEIQISNSFDEEQKESLIKNLVLKNYRENMDVKN
SDIIKNSDIIILYGVSLGETDGYIWNQIAEQSIRSSVPVIIYHYVPHFDAGNPTRVKRLY
RNVEDKFIQNSGIDLELEKKLRDNLIVVIGKTIFNLMER
(SEQ ID NO: 1120)
>orf03440 
VGAKFNDEKTKHIVTHYISRDALNKTITVLSKIIEVFEEHFDRAITCEMFSDSSTFASIN
FSEYGISKSKFQQYLRDSCFIENFGVEHTTVSDIQNSIVTFYDVHTDIFRLLNKLNIDIS
EANIMNQTTVLLDEKNIELLLSKAPYLVSMIVEDFSKLSVDDFSLDNNDLKINLPSPMNE
PVVGVIDTLFDKRVYFNEWVEYHDFVSPDISKDSQDYKHGTAVTSLIVDGANLNPNLDDG
CGNFRVRHFGVSLQSGFNSFTIIKQIKEIVSQNADIKVWNLSLGSNDEIRENFISAEGAL
LDEIQFENDVIFIIAGTNASVINGKRKRIGAPADSLNSIIVNSVDFNNQSVSYSREGIVL
SFFVKPDVSYYGGGNGDFINVCEPLGLGRVAGTSFAAPFIARKMAYLIHIMGLSREEAKA
LLIDAAIPWNDKKTFTDLSLIGNGIVPIKMDDILSTPDDEIKFIVSDISRAYDTYNYDFP
VPISSESYPYVAKATMCYFPNCSRKQGVDYTNTEMQLTFGRLKSDGIKSINKDNQHAEDT
PGYVRENAARNIFRKWDNVKHIGESFTSRKRAKAILNPSNPQWGMSIKTIERLKSGDGQG
VRFGVVVTLKELNGVNRIEDFIQQAELRGWLVNRLQVEAQVDLFNSLNEEIEFE
(SEQ ID NO: 1121)
>orf03442 
MFVADIMISDYSSAPIDFLLLNRVVFLYLPDFKEYQSDKNPFFEVFKVSKTKGIALDPFD
EIIGRFQFGVRIV (SEQ ID NO: 1122)
>orf03450
   MGFSMKLIHDLNTHTTHSTAKMLYNVKAIKNDFSIRE (SEQ ID NO: 1124)
>orf03453 
MEQLHFITKLLDIKDTNTQIIDVVNRDSHKEIIAKLDYDAPSCPECGSQMKKYDFQKPSK
IPYLETTGMPTRILLRKRRFKCYHCSKMMVAETPLVKKNHQIPRIINQKIAQKLIEKISM
TDIAHQLSISTSTVIRKLNDFHFECNFRNLPKIMSWDVETVRGVTVSIGRWR
(SEQ ID NO: 1125)
>orf03462 
LDPWDGNSQKPRFQGLWKFIQRDSRKWRERRFYGPTFGKHLTNKKVFDKVFELFTRPGNI
IIIFINFCGFTSEIRNRGKFFGLIEDNLKQVHPIFQTVFKTFLKDKEKIINALQLHYSNA
KPEATNNLIKLIKRNAFGFRNFENFKKRIFIALNIKKERTKFVLSRA
(SEQ ID NO: 1126)
>oref03466 
MVLYFWKVFQRVPNKLWKNMGENFQVRSLQDKIIQNLTNKGFSYFDAKMPIDEWDSQVDE
ETTQELISRDLISNILSMPESMKDTN (SEQ ID NO: 1127)
>orf03469 
MSKSHSFSISLGISNSFWNNIHTSECWYFLAEGKSNRSNSTISVNQMVFFINIQRFYCFA
156
 IEDFCLLRI (SEQ ID NO: 1128)
>orf03470
   LNTLLPPDNLCLFTIYLTGFSCICIDISYCHNFWEIFNQLFYQLS (SEQ ID NO: 1129)
>orf03475 
MKLLSIAISSYNAAAYLHYCVESLVIGGEQVGILIINDGSQDQTQEIAECLASKYPNIVR
AIYQENKCHGGAVNRGLAEASGRYFKVVDSDDWVDPRAYLKILETLQELESKGQEVDVFV
TNFVYEKEGQSRKKSMSYDSVLPVRQIFGWDQVGNFSKGQYTMMHSLIYRTDLLRASQF
(SEQ ID NO: 1130)
>orf03476 
MYYLPVDFYRYLIGREDQSVNEQVMIKCIDQQLKVNRLLIDQLDLSQVSHPKMREYLLNH
IEITTVISSTLLNRSGTAEHLAKKRQLWTYIQQKNPEVFQAIRKTMLSRLTKHSVLPDRK
LSNVVYQITKSVYGFN (SEQ ID NO: 1131)
>orf03484

MKRIQLNMNETKKYLVIKAIAQGKKTKKRACVELNLSERQINRLLLAYQQKGKEAFRHGH
GNRNRKPKHAIPDEIKERVLKKYLSYETYKPNVLHFCELLAEEEGIKLSDTTVRKILYKK
NILSPKSHRKTKKRVRKQAKLNLNQPLDNPILPTAKDFLEDPKKVHPSRPRKKFAGELIQ
MDASPHAWFGPETTNLHLAIDDASGNILGAYFDKQETLNAYYHVLEQILANHGIPLQMKT
DKRTVFTYQALQL (SEQ ID NO: 1132)

[0402] In some embodiments, preferred INV200 antigens are selected from the polypeptides orf00159 (SEQ ID NO: 626), orf00162 (SEQ ID NO: 628), orf00163 (SEQ ID NO: 629), orf00164 (SEQ ID NO: 630), orf00165 (SEQ ID NO: 631), orf00166 (SEQ ID NO: 632), orf00201 (SEQ ID NO: 639), orf00209 (SEQ ID NO: 645), orf01109 (SEQ ID NO: 747), orf01137 (SEQ ID NO: 751), orf01138 (SEQ ID NO: 752), orf01309 (SEQ ID NO: 783), orf01313 (SEQ ID NO: 786), orf01315 (SEQ ID NO: 787), orf01431 (SEQ ID NO: 810), orf01433 (SEQ ID NO: 812), orf01434 (SEQ ID NO: 813), orf01537 (SEQ ID NO: 824), orf01588 (SEQ ID NO: 831), orf01642 (SEQ ID NO: 842), orf01656 (SEQ ID NO: 847), orf01800 (SEQ ID NO: 875), orf01801 (SEQ ID NO: 876), orf01810 (SEQ ID NO: 879), orf01812 (SEQ ID NO: 880), orfD1818 (SEQ ID NO: 882), orf01988 (SEQ ID NO: 913), orf01989 (SEQ ID NO: 914), orf02105 (SEQ ID NO: 925), orf02106 (SEQ ID NO: 926), orf02263 (SEQ ID NO: 947), orf02264 (SEQ ID NO: 948), orf02459 (SEQ ID NO: 968), orf02538 (SEQ ID NO: 987), orf02539 (SEQ ID NO: 988), orf02541 (SEQ ID NO: 990), orf02545 (SEQ ID NO: 992), orf02604 (SEQ ID NO: 1003), orf02608 (SEQ ID NO: 1007), orf02609 (SEQ ID NO: 1008), orf02850 (SEQ ID NO: 1036), orf03197 (SEQ ID NO: 1082), orf03439 (SEQ ID NO: 1120), orf03448 (SEQ ID NO: 1123), and imnunogenic fragments thereof.

### 7. Sequences Identified from 23F

>orf00010 
LPVTFDFLIEGSTKGNIDKLDTATDCHNWFILTKRFLQECQFKFVTDQIVIIAFDGLFLS
IKLRMNILASCQNEFVNHFHIITYN (SEQ ID NO: 1133)
>orf00017 
MSLITHKRFISCNENIKHYKRLIDKAKKCVNDLMAEFNSVITTVTGIENRLGAVILAEIR
NIHAFDNPAQLQAFAGLDSSIYQSGQIDLAGRMVKRGSPHLR (SEQ ID NO: 1134)

157
>orf00027 
MQQYVDIKKQYPDAFLLFRMGDFYELFYEDAVNAAQILEISLTSRNKNADNPIPMAGVPY
HSAQQYIDVLIEQGYKVAIAEQMEDPKQAVGVVKREVVQVITPGTVVDSSKPDSQNNFLV
SIDREGNQFGLAYMDLVTGDFYVTGLLDFTLVCGEIRNLKAREVVLGYDLSEEEEQILSR
QMNLVLSYEKESFEDLHLLDLRLATVEQTASSKLLQYVHRTQMRELNHLKPVIRYEIKDF
LQMDYATKASLDLVENARSGKKQGSLFWLLDETKTAMGMRLLRSWIHRPLIDKERIVQRQ
EVVQVFLDHFFERSDLTDSLKGVYDIERLASRVSFGKTNPKDLLQLATTLSSVPRIRAIL
EGMEQPTLAYLIAQLDAIPELESLISAAIAPEAPHVITDGGIIRTGFDETLDKYRCVLRE
GTSWIAEIEAKERENSGISTLKIDYNKKDGYYFHVTNSQLGNVPAHFFRKATLKNSERFG
TEELARIEGDMLEAREKSANLEYEIFMRIREEVGKYIQRLQALAQGIATVDVLQSLAVVA
ETQHLIRPEFGDDSQIDIRKGRHAVVEKVMGAQTYIPNTIQMAEDTSIQLVTGPNMSGKS
TYMRQLAMTAVMAQLGSYVPAESAHLPIFDAIFTRIGAADDLVSGQSTFMVEMMEANNAI
SHATKNSLILFDELGRGTATYDGMALAQSIIEYIHEHIGAKTLFATHYHELTSLESSLQH
LVNVHVATLEQDGQVTFLHKIEPGPADKSYGIHVAKIAGLPADLLARADKILTQLENQGT
ESPPPMRQTSAVTEQISLFDRAEEHPILAELAKLDVYNMTPMQVMNVLVELKQKL
(SEQ ID NO: 1135)
>orf00033
   MMRRKYKSIALKKELANDSGKKKFHAMKAQAIVTSQGRIVSIAMI (SEQ ID NO: 1136)
>orf00042 
LTNLSSVDSEELFQFYRERGNAENFIKERKAGFFGDKTDSSTMIKNEVRMMMGCLAYNLY
LFLKQLAGDEVKALTIKRFRRLFLHIAGKYVSTARRHILKFSSLYAYSKQFQALFDTICQ
INLILPVPYRARGQGKTA (SEQ ID NO: 1137)
>orf00051 
LFDDRQAINICPPTNGSLRLTSLQVDQNPCPPSTNLNKILARSQFLNHIQQISLSLELLQ
ANLWNLV (SEQ ID NO: 1138)
>orf00055
   LSVHFCSSHRCLLVRYNDTYSTKKGLKFETFLSVFRYDFLGM (SEQ ID NO: 1139)
>orf00086 
VDRTDEVSSKHGFEVVDETDEVSSKHGFEVADRTDEVSSKHGFEVADRTDEVSSKHGFEV
ADRTDEVSSKHGFEVADRTDEVSSKHGFEVADRTDEVSSKHGFEVADRTDEVSSKHGFEV
ADRTDEVSSKHGFEVADRTDEVSSKHGFEVADRTDEVSNIYTAR
(SEQ ID NO: 1140)
>orf00088 
MDFFNYLLWMICHNHGLHTLLLSKDCVCHTARDKDGNHRIKSVFPTKGQTCYQHDSSIYQ
ERNTTDILTRFLANSQADDIRPTTGDIVSKSKTNPQTHNNTPKKGIDNGILRQGCHRDKL
DKEGTHRYRDKGKDGELMANLIPS (SEQ ID NO: 1141)
>orf00096 
MKIKEQTRKLAAGCSKHCFEVVDETDKVSSKHGFEVVDETDEVSSKHGFEVVDETDEVSN
HTYGKATLTWFEEIFEEY (SEQ ID NO: 1142)
>orf00103
   LQNDKNHKLFDNYTCQKEKDVLRCKQVKRKEERSYDVGTRIYTIYDFLLF (SEQ ID NO: 1143)
>orf00105 
MKIKEQTRKLAAGCSKHCFEVMDRTDEVSSKHGFEVVDETDEVSNHTYGEVKLTWFEEIF
EEY (SEQ ID NO: 1144)
>orf00106 
LFFKDEKQALYTKPKTKSSSFRASKVSNQTIVATTRTDCQVIALNLCDKLENGVVVVVQT
THHIGIDDVIYSKIFQHLTHSIKMSLAFFIKKVQDRRRILYCHLVFFFLRVQDTKRIFLQ
ATLAILRQGLLERCQIVNQGLAVGCTALRISKSVEVQFDTLNTDFLQKMGCHSDCFHIGS
WIARAKTLNTNLVELAQAPCLWTLITEHRSHVVELAWLLHFWGEEFIFHIGTDNGRSSFW
 
TEGNMAVTLVIEIVHFLGYDIGRISDRAADNLVMLKNGRAHFCVVVALENFTGKALNVLP
FGRFSR (SEQ ID NO: 1145)
>orf00114 
MEQIGKVFRQLRESRNISLRQATGGQFSPSMLSRFETGQSELSVEKFLFALENISASVEE
ILFLARGFQYDTDSELRKEITDVLEPKNVAPLEDLYRREYQKHAHSHNKQKHILNAIMIK
SYMKSMDERVELTAEEGKVLHDYLFSTEIWGIYELNLFSVSSPFLSVSLFTRYVREMVRK
SDFLMEMSGNRNLFYTILLNGFLASIECEEFTNAYYFKRVIEEHFYKENETYFRIVYLWA
EGLLDSKQGRVKEGQKKMEDAVRIFEMLGCNKSAEYYRNTTEC
(SEQ ID NO: 1146)
>orf00118 
MQEHYTPKGKHLTIDNRRLIERWKNENKSNREIAGLLGKAPQTIHTEVKRGTTLQQVRKG
LYKKVYSADYAQTVYQFNRKRSVKKLILTKEIREKILHYHKQKFSPEMMVNKKQVKVGIS
TIYYWFHNGHLGLTKADMLYPRKRKGVKKQASPNFKPAGKSIEERPDVINLRLENGHYEI
DTVLLTKIKNYCLLVLTDRRSRHQIIRLIPNKTAESVNQALTLLLGEHHILSITADNGSE
FKRLSEVFPEEHIYYAHAYSSWERGSNENHNRLIRRWLPKGTKKTTPKEVAFIENWINNY
PKKCLDYKSPSEFLLGG (SEQ ID NO: 1147)
>orf00121
   MKIKGQTRKLAAGCSKHCFEVVDRTDEVSDIHTYGKATLT (SEQ ID NO: 1148)
>orf00124 
VVPFSDTFKDRNQVDIFTIKISRCNSSTIGENSWDIHISNSNHRSRHVLVTATDSDEGIH
VVTTHSRLDGVRDDVTRC (SEQ ID NO: 1149)
>orf00139
   MDLKFEGVDLEYKKAKNNLPESFWETYSAFANTNGGKIILGIDEKNIDTYQRVNRLPAKL (SEQ ID NO: 1150)
>orf00156
   LSIQVETLELRVIFKEIKEIVKQFHQLHTMAFKRQVPLTVPVTM (SEQ ID NO: 1151)
>orf00171
   VQKLKKAIYKAHLKDSDDFRPETSTPNLFESCLKLCPCFLSS (SEQ ID NO: 1152)
>orf00172 
MGALGYYEGFVPYVSNQYKNQAEEEGKPLSDKYIFEKILGKTYAAFKKDQINERVEKLGK
LKPITINYNGKSEVIDSKEKLQELMNKAVKDEVAQI (SEQ ID NO: 1153)
>orf00173 
MMGDGMKEFQFERKQRFSLRKYAIGACSVLLGTSLFFAGMGDQPVQDTETSSALISSHYL
DEQDLSEKLKSELQWFELENKLLNLWEH (SEQ ID NO: 1154)
>orf00177 
VNIAKTSIIKAHTTKEDGIDHTFTRFNIMSIFYSTRKIFLDKLNSTNRQFLGYIISTRCY
QSFNSVSQSIHTSSSSQAFRFGKHEFRVINRDKSKAILVNHYHLNLAFFISNHIVNGNFC
(SEQ ID NO: 1155)
>orf00178 
MKPFIILWSSTICSHDRNTTSCILWRTPAKTDDKVTAMFLQSSYPICDIFTSRVWLYIAK
DDIFDSFCIQWF (SEQ ID NO: 1156)
>orf00194 
MHIPFARWQAFSLYNISINIHHYNIGFFDFKEINTRRGNCHQLFFTIENTEIPTCSFRQI
CFY (SEQ ID NO: 1157)
>orf00205
   MNIAIRIILNFFRVMGNHQNSLAMMMGAVVHEFVKFIFTSCIHPRCRLV (SEQ ID NO: 1158)
>orf00206
   MLLIMSIQTTEPAFSRIATRLDKFIDRTWKTSIKTGNLLRKIGYSQFLTLRICL (SEQ ID NO: 1159)
>orf00207 
LQNSKTSLDERRLSRSIFPSQGNKFPTINTIIDMFKNRLLIIIEGQILYRNISHYLISPT
KAVKNR (SEQ ID NO: 1160)
>orf00220 
MSNSFVKLLVSQLFANLADIFFRVTIIANIYIISKSVIATSLVPILIGISSFVASLLVPL
VTKRLALNRVLSLSQFGKTILLAILVGMFTVMQSVAPLVTYLFVVAISILDGFAAPVSYA
IVPRYATDLGKANSALSMTGEAVQLIGWGLGGLLFATIGLLPTTFIILVLYIISSFLMLF
LPNAEVEVLESETNLEILLKGWKLVARNPRLRLFVSANLLEIFSNTIWVSSIILVFVTEL
LNKTESYWGYSNTAYSIGIIISGLIAFRLSEKFLAAKWESILFPLVAMAIVTLTILYFPN
AQMFLLFSALVGMLSQLKEVPESVFLQETVEENHLVNVYSVLEVISTLAFSVFVLLMSYI
TESFGISISFWLSAICLMIEAILIYIRRDYFK (SEQ ID NO: 1161)
>orf00221 
MSLVHNIIEDGDTESLIRKYLHSGVIINGQRYKTLVGTPQGGNLSPLLSNIMLNELDKEL
EKRGLRFVRYADDCVITVGSEAASKRVMYSVSRFIEKRLGLKVNMTKTKITRPRELKYLG
FGFWKSSDGWKSRPHQDSVRRFKLKLKKLTHRKWSIDLTRRIEQLNLSIRGWISYFSLGN
MKV (SEQ ID NO: 1162)
>orf00222 
MSKLLDKILSRENMLEAYNQVKSNKGSAGIDGMTIEEMDNYLRQNWRLTKELIKQRKYKP
QPVLKVEIPKPDGGIRQLGIPTVMDRMIQQAIVQVMSPICEPHFSDTSYGFRPNRSCEKA
IMKLLEYLNDGYEWIVD (SEQ ID NO: 1163)
>orf00229
   LHFNQTSLKTASCRLQGYTSSCDSSTDNQEVQGAFLHFFN (SEQ ID NO: 1164)
>orf00247
   MFCLTFICLIRRSGYLGSYLLLCRMNHTSHKKTGNSYTSYSNTKFTN (SEQ ID NO: 1165)
>orf00248 
LPSEIKAKLDAAFEQFKKDTLPTEPGKKVAEAEKKVEEAKKKAEDQKEKDLRNYPTNTYK
TLELDIAESDVEVKKAELELVKEEAKESRDEKKINQAKAKVENKKAEATRLKNIKTDREK
AEEAKRRADAKLQEANVATSEQDKSKRRAKREVLGELATPDKKENDAKSSDSSVGEETLT
SPSLKPEKKVAEAEKKVEEAKKKAEDQKEEDRRNYPTNTYKTLELEIAESDVEVKKAELE
LVKEEAKESRDEKKINQAKAKVENKKAEATRLKNIKTDREKAEEAKRRADAKLQEANVAT
SEQDKSKRRAKREVLGELATPDKKENDAKSSDSSVGEETLTSPSLKPEKKVAEAEKKVEE
AKKKAEDQKEEDRRNYPTNTYKTLELEIAESDVEVKKAELELVKEEAKESRNEEKIKQVK
AKVESKKAEATRLENIKTDRKKAEEEEAKRRAAEEDKVKEKPAEQPQPAPAPQPEKPTEE
PENPAPAPAPKPENPAEKPKAEKPADQQAEEDYARRSEEEYNRLTQQQPPKAEKPAQPST
PKTGWKQENGMWYFYNTDGSMATGWLQNNGSWYYLNSNGAMATGWLQNNGSWYYLNANGS
MATGWLQNNGSWYYLNANGSMATGWLQYNGSWYYLNANGDMATGWLQNNGSWYYLNANGD
MATGWLQNNGSWYYLNANGDMATGWLQYNGSWYYLNANGDMETGWVKDGDTWYYLEASGA
MKASQWFKVSDKWYYVNGSGALAVNTTVDGYGVNANGEWVN
(SEQ ID NO: 1166)
>orf00254
   MDREILKFFQDLLSILSHNDMITLFCQKCCDISFSNHFLVICN (SEQ ID NO: 1167)
>orf00261
   MTRKLNPSYTNVASATTLTFNQVASTFRKACLDHVVNLTRNNLKGICQLTPLQLHDTRLI (SEQ ID NO: 1168)
>orf00300
   LVSVFYSLLQVDNVDSVTFSKDVLSHLRIPATSLVTKVYTSLKKLFH (SEQ ID NO: 1169)
>orf00309
   LIVWILKNHTDLTTYIPNIFLSQTLAINYNLSGFCFQ (SEQ ID NO: 1170)
>orf00310
   MPYNRKPFSTFHVKRNILHIVVVLIFFIAKRKIFYINY (SEQ ID NO: 1171)
>orf00314 
MFKKMSNSSRILFYISVNFCDKRIYRTKLYSDTPVNLFKFLFRQKSNCQSVGQTSSINLF
FYSWIVFFFKNNLCHSIPSIK (SEQ ID NO: 1172)
>orf00327 
LADGSRKLAEGGTKLTSGLEDLQTGLASLGQGLGNASDQLKSVSTESKNAEILSNPLNLS
KTDNDQVPVNGIAIAPYMISVALFLQQYQQI (SEQ ID NO: 1173)
>orf00356
   MEMSFIAQDFDKLNIITVLESRTQAIIRNPMNTRLSSATGSSFNKIVRN (SEQ ID NO: 1174)
>orf00358 
MELAETSIVKKNHQIPCIINQKIAQKLIEKTSMTDIDHQLSISTSTVIRKINNFHFEHDF
SRLPEIMS (SEQ ID NO: 1175)
>orf00364 
MNYIDTNEMLFVETPRKVITSDELRKKNTKYLDQKEFKLFIQNLKDEALCDYRITKYIRI
AKVLFLTGMRYGELAALNYKEDIDFSKKTIHIKHTYDFRQKERTTPKTIKSDRVITAPQK
VLDIIKEQIIENATNGFDTDFIFINTLGEPITNARVICALKRHGQKIGIEKNITTHTFRH
SHISLLAELGIPLTAIMDRVGHSDSKTTLEIYSHVTQKMVSDISSKLDKIKF
(SEQ ID NO: 1176)
>orf00365 
MWMEELPNGKYKFFERYKDPYTEKLKKVSVTMEKKTPQARNQAAILLQEKIKQKLGEKQH
SVSNITFEKLYEEFEENWKHGVKNSTVYASKNVKKEILKQIEGDYLVRNLIDVYYKK
(SEQ ID NO: 1177)
>orf00367 
MEIDKVKADLKQVGKRVADLSQSITNEEQTKNAFIMPFFQALGYDIFNPLEFVPEFTADV
GIKKGEKVDYAIILDGEPQILIECKSITENLTKHDSQLFRYFVTTKSKFGILTNGREYKF
FTDLDEPNKMDTTPFLTIDVTDIKENQFTEIIKFHKENFDIDNIVSSASELKYLNNLKAF
LTENITTPSDSFLRYLTSEIYEGRVTQNILTTFSPIIVKGFNQFITERVNEKLSAALNTS
VETKVTTDIPKVEAEAEEIVEVTDEIITTPAELEVYTVVKMLARDVVSPERVFYRDNRSY
FNVLVDDNIKKWVLRYRSNSKKSTIEIRDKGIFPVSTPLEVANYANEILEVIKKFS
(SEQ ID NO: 1178)
>orf00368 
MTLAKLCEEYQVELCLFDGSNWHNSGFYNPDTNVLAIDHNLTPEQQIQVALHELGHKDHT
RSEHQNARLRCENEADRNMIHHLVKDALENLDDPTEFDYLKFMSYYNLKTMTNEIMVKEE
YLALVN (SEQ ID NO: 1179)
>orf00369 
MYRLDIDKKALKQLKKLDTPTRKQILSWLAKNIENTTNPRQHGKALKANLAGYWRYRVEN
YRIICDIQDDKLVVLAVEIAHRRDVYK (SEQ ID NO: 1180)
>orf00370 
MTITINFTEKNSYITDYLNKHGIDTTTMDFDDFMALMEDIEDARAADQAYMEYLADPATY
TMDEVLDELGLTREDIA (SEQ ID NO: 1181)
>orf00371 
MFETFEKIKELAKKRGKALGQVEEDLGYGRNTLYKIKNSTPNAERIAEIANYFNVSTDYL
LGRTDNPAIAGSDEFAQVNGQIIDLRKAAANTMLFDGKPLNEDDIDFITSVLSAHFKSKG
ER (SEQ ID NO: 1182)
>orf00372
   MVSILKNLEQEKDHLEKVIkVVSAGGKFLRLFYQISHARLVRI (SEQ ID NO: 1183)
>orf00373 MPDIANGRERVIAFLKEKGIKKATLAVAYGFKRQEVTNILSGTTKGPRANSFILQVIEDY GIE (SEQ ID NO: 1184)
>orf00374 
MRPKRYPYSGQKESTFVKADPELVEKLLRNTSFLECLQKKPINFQIDSEEFKRLSYEAIH
DTSQVTQ (SEQ ID NO: 1185)
>orf00377
   LKNREEEWQGIIARXIAILLIIAPFYFLIIVKNGVLSKIKTVTEITAYQL (SEQ ID NO: 1186)
>orf00378
   MREVIQELLDSSMSTSAISQGAGVPWTTVSDLRKGKTSMDKMALLTAEKLYEFATTDKQ (SEQ ID NO: 1187)
>orf00382 
VEEVEVAEVKNARVSLTGEKTKPMKLAEVTSINVNRTKTEMEEFNRVLGGGVVPGSLVLI
GGDPGIGKSTLLLQVSTQLSQVGTVLYVSGEESAQQIKLRAERLGDIDSEFYLYAETNMQ
SVRAEVERIQPDFLIIDSIQTIMSPEISGVQGSVSQVREVTAELMQLAKTNNIAIFIVGH
VTKEGTLAGPRMLEHMVDTVLYFEGERHHTFRILRAVKNRFGSTNEIGIFEMQSGGLVEV
LNPSQVFLEERLDGATGSSIVVTMEGTRPILAEVQALVTPTMFGNAKRTTTGLDFNRASL
IMAVLEKRAGLLLQNQDAYLKSAGGVKLDEPAIDLAVAVAIASSYKDKPTNPQECFVGEL
GLTGEIRRVNRIEQRINEAAKLGFTKIYVPQNSLTGITLPKEIQVIGVTTIQEVLKKVFA
(SEQ ID NO: 1188)
>orf00389
   VNIATLQNGHILGWQIQHIANKLTSNFWIAKDFLSYQVIGWANARMTYSHISSLFIISQF (SEQ ID NO: 1189)
>orf00391 
VSITFSLTNFFKILINLTAQVSPQVIDEKILMMDLNLNNYLSTVIQLRQDVYTGIKILHR
VRHGE (SEQ ID NO: 1190)
>orf00392
   MSRYSYSLDSRKIVFEISCFKEKKASLTLFFHLFESSIMKLATQPSFSSFYSELK (SEQ ID NO: 1191)
>orf00396 
MKIKEQTRKLAAGCSKHCFEVVDRTDEVSSKHRFEVADRTDEVSSKHRFEVADRTDEVSN
IYLRQGDVDVV (SEQ ID NO: 1192)
>orf00408
   LSLLDLRGSLCLRIYLHEPLITTVSQDFTSLSDISHF (SEQ ID NO: 1193)
>orf00411
   MDFKSFIIGLVVGIFGPYMDDLIRKKFLKSSEKKTEKSVKK (SEQ ID NO: 1194)
>orf00434 
MFEKIKGINIKSGIFEDETKLELFEGNFEGTNPVQNDRASLLFGRNGSGKSTIARGINQL
KNGEIGTDRVSFIDKNNNNIVLSDTERKSIFVFNEHYVDQKVKIAQEGLDTIVILGEQVD
IDEELDRLRTQLSESQIESQDYYAEYEEYLDEKNEKSPDFWKKEMTDSLKGVGNWAERDR
EIKGNRAASPVHNNTFQNFVDLQPILDKNELEVEFNNKKARYFSIRDSAVTINNELSLPD
INFDSNELSTLLSEKIEEPELNSRDKYLLTLLSDSTKGERHLREVKDFFEDEHQKKCPFC
TQSVSEDVKVELTNGITKLLSRAVEEHQSALRGKKIDEINQDFSGYEQIDPILIQSYQNS
INALNAKFNEINSIIDKKIDNPYNIVELPNISFSQELSQAEHDIEKINQAIIKHNSEISG
IQKLKVDLLQINNELAFYEIQDAYKKFQEKTNKKAICENNYNNSSKRVKDYEKQISDLED
KKLNIDIAVDEINKSLNYIFFSKNRLAIQNQNGKYYLLSRGKSVVPSRVSVGERNALALC
YFFTEIIQQRELADAYSHEYFIVIDDPISSFDMENKVGITSYLKYCLTRFFKGNSNTRVL
LMTHDKQTIYDFDIFLKEIMESCKEEEGGQKSKYKKLELVSGKLQEFKTSTHDYTELLEI
VFGYALGNSTPTSESFVGNAMRKILEAYGSFNYKKGIAELTTDPLIVEKIDKEYRTYFEN
LMYRLVLNGESHFKDPVKTLSIDFFDTISDEERKKTARDLLVLLYLLDDLHVLKHLEGVS
NAENRLEQWKCEILE (SEQ ID NO: 1195)
>orf00458 
MIEGDRDCADIVTQLTAVKSSVERVIEMIITENLTECINQPLDDSEAQ[?][?]L[?][?][?]R[?][?]
KRK (SEQ ID NO: 1196)
>orf00460 
METSISMADFYGKYQNENLELIDVREAHEFQAGHAPGAKNLPLSTLEQGYKELKPDHEYY
VICQGGVRSASTCQFLSSQGLTVTNVEGGMNAWPGQVE (SEQ ID NO: 1197)
>orf00462
   LGGKSCLLEDRLCDIAAQTTVAADDVGLFFVQFISFLLDTLSVFDTIVQN (SEQ ID NO: 1198)
>orf00466
   MKIKDQTRKLAAGCSKHCFEVVDRTDEVSSKHCFEVADRTDEVSNIYTARRR (SEQ ID NO: 1199)
>orf00467 
MKLLSIAISSYNAAAYLHYCVESLVIGGEQVGILIINDGSQDQTQEIAECLASKYPNIVR
AIYQENKGHGGAVNRGLAEASGRYFKVVDSDDWVDPRAYLKILETLQEFESKGQEVDVFV
TNFVYEKEGQSCKKSMSYDSVLPVRQIFGWDQVGNFSKGQYIMMHSLIYRTDLLRASQF
(SEQ ID NO: 1200)
>orf00468 
MYYLPVDFYRYLIGREDQSVNEQVMIKCIDQQLKVNRLLVDQLDLSQVSHPKMREYLLNH
IEITTVISSTLLNRSGTAEHLAKKRQLWTYIQQKNPEVFQAIRKTMLSRLTKHSVLPDRK
LSNVVYQITKSVYGFN (SEQ ID NO: 1201)
>orf00476 
MSLQIKLKKLAKELSKLLKDSNLETVDKDVLENSQKELQKAVLFLADEKGSEHTEAEVID
NLKEVIAKLKANA (SEQ ID NO: 1202)
>orf00483
   MKIKEQTRKLAAGCSKHCFEVVDKTDEVSYIYLRQGEADAV (SEQ ID NO: 1203)
>orf00503 
MKIKEQTRKLAAGSSKHCFKVVDGTDEVSSKHCFKVVDGTDEVSSKHCFEVVDRTDEVSN
HIRQGDVDVV (SEQ ID NO: 1204)
>orf00509 
MNDDDSRCIHIERDGKTIEFGYLNISSTDRNTSHADGLVGIFNSNFSGVRVRGIAVFLNG
PDNLDTTLVGNFQTIWNFRIICIHS (SEQ ID NO: 1205)
>orf00510
   LEFNFCRSIIKNGRDNLPNTNSTSGMATRWANHNNSDDIKDRLKTK (SEQ ID NO: 1206)
>orf00515 
MSNVDKIRKIHIIVCWMYIFLSFRAIINDTEYFLLIFLAFIYSIVSLPLYSVKNKIVSIC
LVINSILLMSFPILINKFFPESFLTYIVLISVFITELIIFHLIGKDFDTKLTNEYKKISQ
FRSKVSQSPWIKYLEISSFILTIFPSILYGTVDNHVLTLIFLIKICVDTTIKFLFIRLFD
TSTLMKRRIFFLFALDVIAYLFLGYLLVIQKAGYLFSVLLLFSNFSVPFIKEKEYELFKN
SK (SEQ ID NO: 1207)
>orf00516 
MNKKKMILTSLASVAILGAGFVASSPTVVRAEDAPQVVEKSSLEKKYEEAKTKADTAKKD
YETAKKKAEDAQKKYDEDQKKTEEKAKKEKEAAKKVDDASLAVQKAYVEYRKVQESRSNY
RNRSDYNKKLAEAQVKIDEANKKLTAANNEFKTVRAVVVPEPNALAETKKKAEEAKAEK
(SEQ ID NO: 1208)
>orf00518 
LEQEVATAQHQVDNLKKLLAGVDPDDTEAIEAKLKKGEAELNAKQAELAKKQTGLEKLLD
SLDPEGKTQDELDKEAAEAELNKKVESLQNKVADLEKEISNLEILLGGADSEDDTAALQN
KLAAKKAELAKKQTELEKLLDSLDPEGKTQDELDKEAAEAELDKKADELQNKVADLEKEI
SNLEILLGGADPEDDTAALQNKLATTKAELEKTQKELDAALNELGPDGDEEETPAPAPQP
EQPAPAPAPKPEQPAPAPKPEKSADQQAEEDYARRSEEEYNRLTQQQPPKAEKPAPAPAP
 
KPEQPAPAPKTGWKQENGMWYFYNTDGSMATGWLQNNGSWYYLNSNGAMATGWAKVNGSW
YYLNANGSMATGWVKDGDTWYYLEASGAMKASQWFKVSDKWYYVNSNGAMATGWLQYNGS
WYYLNANGAMATGWAKVNGSWYYLNANGSMATGWVKDGDTWYYLEASGAMKASQWFKVSD
KWYYVNGLGALAVNTTVDGYEVNANGEWV (SEQ ID NO: 1209)
>orf00519
   LTISFKKQFLSSSLSSLTKRVIMNTAQATFNREAHTTFNRE (SEQ ID NO: 1210)
>orf00525
   MKIKEQTRKLAVGCSKHCFEVVDRTDEVSSKHRFEVVDRTDEVSNIYTARRS (SEQ ID NO: 1211)
>orf00539
   LKKRMNRWQFLLNQSKEMVGILLLKMKEQELIEFVVNL (SEQ ID NO: 1212)
>orf00540
   LIKVIKRKAFGFRNFNNFKKRILMTLNIKKESTNFVLSRL (SEQ ID NO: 1213)
>orf00544 
MTYNEKRLTNSLERVHMEQLKNTTDLLGLKDKNIKILSVLKYQTHLVVQAKLDSPAPPCP
HCQGKMIKYDFQKASKIPLLDCQGLPTVLHLKKRRFQCKNCLKVVVSQTSIVKKNCQISN
MVRQKIAQLLLEKQSMTEIAHRLAVSTSTVIRKLREFKFETDWTKLPKVMSWDEYSFKKS
KMSFIAQDFESKSILAILDGRTHAVIRNHFQRYQREVRELVEVITMDMYSPYYRLAKQLF
PKAKIVLDRFHIVQHLSRAMNRVRIQIMNQFDRKSLEYRALKRFWNPRFFVSRLGLNQST
GLIYYTRIASSSVRNDSISPRFECT (SEQ ID NO: 1214)
>orf00545 
MGYSLKKSCTYCEQDPEKVNRFLKELNHLSYLTPIYIYETGVETYFYLEYDRALSRQLVS
LEEDIII (SEQ ID NO: 1215)
>orf00552 
MNIAVIGLGHVGLAYALLFASKYKVVAYDIDSVKINNLKKGILPSKNEELMKFFCENNLN
ITFFDTFSEIKNNIDYYIIALPTDYDEKIGSFNTYEIEQTVSKILRVKPNGKIILKSTVP
FGFSNKLKRLFDTKNIIFVPEFLREGCSIYDNLYPSRIVVGDETVEGRKIAELFLSISTH
STANIKNVMLVSPTEAEAIKLFSNTFLALRVAFFNELDSFAERRSLNAEVVIKGVCLDPR
IGNFYNNLSFGFGGYCLPKDTKQLKKEFIEINAPVIEAIDISNTNRKQFIVKQILERKPK
IVGIYKLGMKYNSDNYKESAILSIINELLIVGIKILVYEPNLNVSIDNVIFEKNFELFTK
QSDLIVANRWDRGLEAYKDKVYTRGIWIRD (SEQ ID NO: 1216)
>orf00554 
MLNLQFAETMELTEAELEIVYGGEFGNNAVIPAGAWGGFGTPWSITNFWKKNFNDRPDFD
SDRRRY (SEQ ID NO: 1217)
>orf00599 
MGLDVGSKTVGVAISDPLGFTAQGLEIIQINEEQGQFGFDRVKELVDTYKVERFVVGLPK
NMNNTSGPRVEASQAYGAKLEEFFGLPVDYQDERLTTVAAERMLIEQADISRNKRKKVID
KLAAQLILQNYLDRKF (SEQ ID NO: 1218)
>orf00635
   LNPSYSFGKKDQFALEHCFCIKLSIFARAVTLFVSCIN (SEQ ID NO: 1219)
>orf00656 
MITGTAFILIMSLSARKLPYTIRSSVASLQQIAPSIEEAAESLGSSRLNIFAKITTPMML
SDIISGAILSWVTLISELSTSILLYNVKTRTMTVAIYTEVLRGNYGVAAALSTILTVLTV
GSLLLFMKISKSNSITL (SEQ ID NO: 1220)
>orf00657 
MLIGEGYRTFPVLIYTQFISEVGGNSAFAIMAIIIALAIFLIQKHIANRYSFSMNLLHPI
EPKKTTKGKMAAIYATVYGIIFISVLPQIYLIYTSFLKTSGMVFVKGYSPNSYKLAFNRM
GSAIFNTIRIPLIALVLVVLFTTFISYLAVRKRNLFTNLIDSLSMVPYIVPGTVLGIAFI
SLVYLEVDFL (SEQ ID NO: 1221)
>orf00658 
MECKKLNIWTASSFFLFLTYLVFLVYPIVTVLKQALIHEGQFSLANFVTFFSKAYYSETL
VNSFRVSITATVTSLVVGTLLAYLFSMYDFKGKKFLQILIIIASMSAPFVGAYSWILLLG
RNEVITKFLTNALYLPAIDIYGFKGIVLVFTLQLFPLVFLYVAGTMNSIDNSLLEAAESM
GSFGFKPIVTVVLPLLVPTLLAAPCLYL (SEQ ID NO: 1222)
>orf00660 
LLSTTEFIGLSIRILSNLHEFKILVGLLNQFFFWNLLLHKTKSNVVSDSQMWENSVVLEN
QPDIAFAGFHIIDFCIIEVKFSIFDTVETCNHTKKGRFPTS (SEQ ID NO: 1223)
>orf00679 
MITIKKQEIVKLEDVLHLYQAVGWTNYTHQPEMLEQALSHSLVIYLALDGDAVVGLIRLV
GDGFSSVLVQDLIVLPIYQRQGIGSALMKEALEDYKDAYQVQLVTEETERTLGFYRSMGF
EILSTYNCIGMTWMNRKK (SEQ ID NO: 1224)
>orf00710
   VLKIRYHKQFKKDFKLAMKRGLNAELLEEVLKIWFKKKNFLLDIVIIN (SEQ ID NO: 1225)
>orf00714 
MLGSMFVGLLVGFLAGTLTNRGEHMGCFGKMFLGWIGAFIGHLLFGTWGPIIAGTAIIPA
VLGSMIVLAIFWRRGS (SEQ ID NO: 1226)
>orf00741
   MIDDIPKRVNDVIGQAGNNAKTSRPHVGIGKSHISVPFLFPYHTANRIKNQEKVIF (SEQ ID NO: 1227)
>orf00755 
VAIDKIAGITSEKDSRAHQIFRISPTCSRCFCNDELVKWVARTIFLQLTKRCCLRSGNIT
RSNSVTLDIGSTVFRRNVAGQHFQAPFSSSISANCFTSQFAHHRTNIDNLSMPFLYHRRN
NCL (SEQ ID NO: 1228)
>orf00756 
LFDLLDHGLDTVLVCHVTDISMGFDANFTISFNPFIDQILIDIVKDNSSAGFSVGFGNSK
SNSIRSAGDESNFSF (SEQ ID NO: 1229)
>orf00768 
MKSLARLLIIHVFISIFLFFALISGAVSHTVLLLLLFLPALNKGLEKIQSKRIPVLNAA
LFFLLISFPQLLTNPVQWKFSIFLVVTIISSLAYFYNFYQVVKEVDQKQLI
(SEQ ID NO: 1230)
>orf00769

LEAASEIETEFQSWIVLVVFNHIDGLSRDTDILGELELGNAQFLAKFFHTIHLVSFLICV
VYI (SEQ ID NO: 1231)

>orf00774 
MKWTKRVIRYATKNRKSPAENRRRVGKSLSLLSVFVFAIFLVNFAVIIGTGTRFGTDLAK
EAKKVHQTTRTVPAKRGTIYDRNGVPIAEDATSYNVYAVIDENYKSATGKILYVEKTQFN
KVAEVFHKYLDMEESYVREQLSQPNLKQVSFGSKGNGITYANMMSIKKELETAEVKGIDF
TTSPNRSYPNGQFASSFIGLAQLHENEDGSKSLLGTSGMESSLNSILAGTDGIITYEKDR
VGNIVPGTELVSQQTVDGKDVYTTLSSPLQSFMETQMDAFLEKVKGKYMTATLVSAKTGE
ILATTQRPTFNADTKEGITEDFVWRDILYQSNYEPGSAMKVMTLASSIDNNTFPSGEYFN
SSEFKIADATTRDWDVNDGLTTGGMMTFLQGFAHSSNVGMSLLEQKMGDATWLDYLKRFK
FGVPTRFGLTDEYAGQLPADNIVSIAQSSFGQGISVTQTQMLRAFTAIANDGVMLEPKFI
SAIYDTNNQSVRKSQKEIVGNPVSKEAASTTRNHMILVGTDPLYGTMYNHYTGKPIITVP
GQNVAVKSGTAQIADEKNGGYLVGSTNYIFSVVTMNPAENPDFILYVTVQQPEHYSGIQL
GEFATPILERASAMKESLNLQSPAKNLDKVTTESSYAMPSIKDISPGELAEALRRNIVQP
IVVGTGTKIKETSVEEGTNLAPNQQVLLLSDKVEEIPDMYGWKKETAETFAKWLDIELEF
EGSGSVVQKQDVRTNTAIKNIKKIKLTLGD (SEQ ID NO: 1232)
>orf00776
   MVDRTDEVSSKHGFEVVDKEKLMWFEEVFEECKKILVS (SEQ ID NO: 1233)
>orf00783 
MEGVNHVDIIKVSCCSFISQVNWMMKGKIPNREGFKFSVARFDAIDLVVVHIGHTRCQFS
RTGSRSGYDNQVATGFDVVVFAHAFWGNDVIHIRRISFDWIMKIRINSVFLKLVAEGICS
GLASVLCNDNGTNKNP (SEQ ID NO: 1234)
>orf00784
   MFNVASINGNHNLNLLFQFLQELDFVVRFITRKDTSSVEIF (SEQ ID NO: 1235)
>orf00790 
LTNQDLQAGTYLVKDYREIILSQDALEKVATNLKLDMPAKTLASKVQVAVPADTRIVSIS
VKDKQPEEASRIANSLREVAAEKIVAVTRVSDVTTLEEARPATTPSSPNVRRNSLFGFLG
GAVVTVIAVLLIELLDTRVKRPEDVEDVLKIPLLGLVPDFDKIK
(SEQ ID NO: 1236)
>orf00791 
MPTLEISQAKLDSVKKAEEYYNALCTNLQLSGDGLKVFSITSVKIGEGKSTTSANIAWAF
ARAGYKTLLIDGDIRNSVMLGVFKARNKITGLTEFLSGTTDLSQGLCDTNIENLFVIQAG
SVSPNPTALLQSKNFTTMLETLRKYFDYIIVDTAPVGVVIDAAIITRNCDASILVTEAGE
INRRDIQKAKEQLEHTGKPFLGIVLNKFDTSVDKYGSYGNYGNYGKNKK
(SEQ ID NO: 1237)
>orf00792 
MNEKILRSSLAIIQSFLVILLTYLLSAVRETEIVSTTAIALYILHYFVFYISDYGQDFFK
RRYLIELVQTLKYILFFALAIGISNFFLEDRFSISRRGMIYFLTLHALLVYVLNLFIKWY
WKRAYPNFKGSKKILLLTATSRVEKVLDRLIESNEVVGKLVAVSVLDKPDFQHDCLKVVA
EGEIVNFATHEVVDEVFINLPGEKYNIGELVSQFETMGIDVIVNLNAFDRSLARNKQIRE
MAGLNVVTFSTTFYKTSHVIAKRIIDIVGALVGLILCGLVSIVLVPLIRKDGGSAIFAQT
RIGKNGRQFTFYKFRSMCVDAEAKKRELMEQNTMQGGMFKVDDDPRITKIGCFIRKTSLD
ELPQFYNVLKGDMSLVGTRPPTVDEYEHYTPEQKRRLSFKPGITGLWQVSGRSEIKNFDE
VVKLDVAYIDGWTIWKDIEILLKTVKVVFMRDGAK (SEQ ID NO: 1238)
>orf00793 
MKKSVYIIGSKGIPAKYGGFETFVEKLTAFQQDKAIQYYVACMRENSAKSGTTEDVFEHN
GAICYNVDVPNIGPARAIAYDIAAINRAIEIAKENKDEDPIFYILACRIGPFIHGIKKKI
QEIGGTLLVNPDGHEWLRAKWSAPVRRYWKISEGLMVKHADLLVCDSKNIEKYIQEDYKQ
YQPKTTYIAYGTDTTRSVLKSSDEKVRSWFKEKNVSENEYYLVVGRFVPENNYESMIRGF
LASNSKKDFVLITNVEQNKFYNQLLAKTGFDKDPRVKFVGTVYEQELLKYIRENAFAYFH
GHEVGGTNPSLLEALASTKLNLLLDVGFNREVAEDGAIYWKKDNLHEIIETSEQKTQKEI
DEKDILSIKQVTERFSWELIVNEYEKLFLCEK (SEQ ID NO: 1239)
>orf00794 
VTIKINNLFFVCLSFFGIVLSSSQVIVNLGLSSIIQYISYFMLMCVFLTLIKNTLNVFA
NRIIYFLIISFLFIIGINLQNLPLSRKIYLSFSMLIISSLSTLPIKLINNLSDLRRISYY
LLHSIFLSVFLGLVFKISLVTVAVEGIGFSYGFNGGLTHKNFYAITILVSYILLYVSRKY
DAKHQIDSFVLWLDLFLLLISNTRTVYIILVVFWIIINRNFINNIKKEHRLVVTATTIVI
SLLALTFFFKHIINNSESYSHRVLGVVNFFKYYESDRFHLFFGDAELAFGNTTKGYGHNI
RSVLGWDGTVEMPLLSVMIKNGYVGLVGYIIVLFKFISSIISVKNSTKKNIGLSIFIPLL
LSATVENYIVNISFVFMPVCFCILCSIKNIKLVNNRK (SEQ ID NO: 1240)
>orf00796 
MEKLVSIILPVYNVEQYIKNCLESIQQQTYSNLEVIIVNDGSTDKSVEYCEQICKIDSRF
SITHKENGGLSDARNVGIDKSKGDYLIFVDSDDFVSQDMVSYLVSCMENNEADIAICDPV
HYYSDRQNNDLNIFSPASNVKVYETTEALCEMFYQKSFLVSAWAKIFKRELFDDIRFPVG
KLFEDSAIMYLLFEKCETIAYSDAELYAYVHRDNSITTKKFSDRDLDILEITNTIINHYG
DNLRVYTAAVSYKVSACFRILLNSPSGEKYKKVQKECLSYILQNWRNILFNNNVRLKNKL
ALISITIFNPFVKFIYSKVNRWE (SEQ ID NO: 1241)
>orf00797 
MNKYEERYQENLSKNDFYKLINKSYLSDKELQVQQVKAGIVLPPKAFETKLSNKLGLQKS
LHGKGGVVDSNGNYIELSAQKAVGMRNRVYGPYKINYDNLPIRNEKVIYLNYFIKQWGHF
LLDVVGRLWYPLLQDNDTKLVYTCYAGTETKIEGNYLEFLKLLGIDQSRLIMINCPTQFS
EVIIPESSILPGGYYTKEYKQLFSSVVENIKLDKYDVNAKMIYCSRSKLGIAKSKEFGED
GIEGIFKQNGYTSVYMETMSLEEQIKTLLSAKTIVLTSGSLAHNLLFVNKDIDVFILNKT
YRVNLHQFLINEISDATVRFVDIYRSPLPILYGYGPFLMDLTKPLANFLDDNEFVYEKGT
 
VLSKKDYFKYYLKWLWSYRFFLFRLNGIKEGNSEFEKSFKIIRRYYKTGR
(SEQ ID NO: 1242)
>orf00798 
MSKYKELAKNTGIFALANFSSKILIFLLVPIYTRVLTTTEYGFYDLVYTTIQLFVPILTL
NISEAVMRFLMKDGVSKKSVFSIAVLDIFIGSIAFALLLLVNNLFSLSDLISQYSIYIFV
IFVFYTLNNFLIQFSKGIDKIGVTAISGVISTAVMLAMNVILLVVFDWGLLGFFIANVCG
YVIPCIYIVSRLRLWELFEIKIDKKLQWEMVYYALPLVLNILSWWVNNTSDRYIVTAIVG
IQASAIISVAYKIPQILSTISAIFIQSWQISAIKIQEDKSDTTFVSNMLLYYNALLLIIA
SGIILFVKPISNILFGISFYSAWELVPFLIISSLFNAISGCIGAIMGAKMDTHNIAKSAL
VGMIANIILNIVLTFLMGPQGITISTLIASFLIFYMRKDSVKEINSETYRAIYLSWILLV
VEACLLIYMDFIIGALIAMVINLFLLKDVIKPLYLKIFKRN
(SEQ ID NO: 1243)
>orf00799 
MIVLQYFKILARFVFMFLISAVLLPFKIKPNKIVFINFNGKGYGDNPKSICEYLRTTYPD
LDLVWLARDNEGFPDGVRVVKYGTFQAFYEQASSKVWVYNVRAFARILKKRGQIYIQTWH
GASSFKLIEKQADLPINYVLEAKYDARVTDIMISDSRKQTEEFQKYFWYSGEIFEVGMPR
NDALFHYKEDYDKLNNIRKELSIHSDDYVILYAPTFRDDGDASYLDINFERLLQCVEHGI
KKKCKFLIRLHPNHSHLCNNISFNKNIINATFYSDMQELTLLADVLVTDYSSSIFDFMLL
NKPYVRYVNDLEKYAELRGVSDTYYELPDSIIKTAEELYDLLPKKIENFDYDSIKKYRNE
ILCPIFNGTASENVGGRIIQEL (SEQ ID NO: 1244)
>orf00800 
LKNNDLKIGSGAIHQISATLSQNSISGKILYCADPVVDDLYGSIVRSQIEEIGRVKEESC
NYNTIAYAMNIAERAIATDIDCIVGMGGGRVLDVCKYASFISKRPYLSIPTTAANDGIAS
PVAVLKRQDDRPKSLGAAIPSMTLIDIDVIASGPIQNIKAGIGDTISNYTALKDWELAVE
RGKDEMHGFAYLMSQNSLDALMKTKYNSITPDFIEVLVNSLVLSGIAMDFAGSSRPVSGS
EHLFSHALDYYGSTRNLHGIQVALGTVAVLKLIENSVDTVVDYLQRFEVHINPKLLGIDE
ELFIYCMQHATKMRSNRYTYLHEVDLSTDRLKQIYKELISEL
(SEQ ID NO: 1245)
>orf00801 
MKALILAAGLGTRLAPITNEVPKSLVPVNGKPILMKQIENLYQNNITDITIIAGYKSSVL
TDAVTEKYPEINIIDNVDFKTTNNMYSAYLGKAAMGDSDFLMMNADVFYDASVIKSLLLH
KAPNAIVTDLGIYIEESMKVVEKNGRLVEISKQISPEETLGASIDVYKFSYEAGARFFEK
CKEFIEDKRELQMWSEVALNAILSEVEFVACPLEGRWLEIDNHEDLVAAEKLFA
(SEQ ID NO: 1246)
>orf00802 
MKLTNRVDYFGADISELQNKKLFLFDMDGTIYEEDRLFEGTLELLDYIHNIGGEYIFITN
NSSKSVVDYVEKVNRLGIKAERDNFFTSAQATIVYIKENYPKSKVYCQGTKSLIKELSDA
GIDVTEQVSADIDVVLVGFDTELTSDKIRNTCEILSTKDVPFIATNPDIRCPVSFGFIPD
CGSICDMISKSVDRKPVYIGKPEPTMVDIVRKKLNYSLFETVVIGDRLYTDIMTGINAGV
TSVCVLTGEATVNDIQQDSIKPTYTFKNVKEMWKGIV (SEQ ID NO: 1247)
>orf00804 
MKGIILAGGSGTRLYPLTRAASKQLMPVYDKPMIYYPLSTLMLAGIRDILIISTPQDLPR
FKELLQDGSEFGIKLSYAEQPSPDGLAQAFIIGEEFIGDDSVALILGDNIYHGPGLSTML
QKAAKKEKGATVFGYHVKDPERFGVVEFDENMNAISIEEKPEYPRSNYAVTGLYFYDNDV
VEIAKSIKPSPRGELEITDVNKAYLDRGDLSVELMGRGFAWLDTGTHESLLEASQYIETV
QRMQNVQVANLEEIAYRMGYISREDVLALAQSLKKNEYGQYLLRLIGEA
(SEQ ID NO: 1248)
>orf00806 
MTDNFFGKTLAARKVEAIPCMLEFDIPVHGDNRGWFKENFQKEKMLPLGFPESFFAEGKL
QNNVSFSRKNVLRGLHAEPWDKYISVADGGKVLGSWVDLREGETFGNTYQTVIDASKGIF
VPRGVANGFQVLSDTVSYSYLVNDYWALELKPKYAFVNYADPSLGIEWENIAEAEVSEAD
KNHPLLKDVKPLKKEDL (SEQ ID NO: 1249)
>orf00810 
MTEYKNIIVTGGAGFIGSNFVHYVYENFPDVHVTVLDKLTYAGNRANIEEILGNRVELVV
GDIADAELVDKLAAQADAIVHYAAESHNDNSLNDPSPFIHTNFIGTYTLLEAARKYDIRF
 
HHVSTDEVYGDLPLREDLPGHGEGPGEKFTAETKYNPSSPYSSTKAASDLIVKAWVRSFG
VKATISNCSNNYGPYQHIEKFIPRQITNILSGIKPKLYGEGKNVRDWIHTNDHSSGVWTI
LTKGQIGETYLIGADGEKNNKEVLELILKEMGQAADAYDHVTDRAGHDLRYAIDASKLRD
ELGWKPEFTNFEAGLKATIKWYTDNQEWWKAEKEAVEANYAKTQEIITV
(SEQ ID NO: 1250)
>orf00813 
MILITGANGQLGTELRYLLDERNEEYVAVDVAEMDITNEEMVEKVFEEVKPTLVYHCAAY
TAVDAAEDEGKELDFAINVTGTKNVARASEKHGATLVYISTDYVFDGKKPVGQEWEVDDR
PDPQTEYGRTKRMGEELVEKHVSNFYIIRTAWVFGNYGKNFVFTMQNLAKTHKTLTVVND
QYGRPTWTRTLAEFMTYLAENRKEFGYYHLSNDATEDTTWYDFAVEILKDTDVEVKPVDS
SQFPAKAKRPLNSTMSLAKAKATGFVIPTWQDALQEFYKQEVR
(SEQ ID NO: 1251)
>orf00814 
LVNCEPLEAYRQLEEAELVGCWAHVRRKFFEATPKQADKSSLGAKGLAYCNQLFSLERDW
EALPADERLQKRQEELQPLMEDFFAWCRRQSVLSGSKLGRAIEYSLKYKETFKTILKDGH
LVLSNNLAERAIKSLVMGRSKRVQWTLLA (SEQ ID NO: 1252)
>orf00823 
MNKGLFEKRCKYSIRKFSLGVASVMIGAAFFGTSPVLADSVQSGSTANLPADLATALATA
KENDGRDFEAPKVGEDQGSPEVTDGPKTEEELLALEKEKPAEEKPKEDKHAAAKPETLKT
VTPEWQTVEKKEQQGTVTIREEKGVRYNQLSSTAQNDNAGKPALFEKKGLTVDANGNATV
DLTFKEDSEKGKSRFGVFLKFKDTNNNVFVGYDKDGWFWEYKSPTTSTWYRGSRVAAPET
GSTNRLSITLKSDGQLNASNNDVNLFDTVTLPAAVNDHLKNEKKILLKAGSYDDERTVVS
VKTDNQEGVKTEDTPAEKETGPEVDDSKVTYDTIQSKVLKAVIDQAFPRVKEYSLNGHTL
PGQVQQFNQVFINNHRITPEVTYKKINETTAEYLMKLRDDAHLINAEMTVRLQVVDNQLH
FDVTKIVNHNQVTPGQKIDDERKLLSSISFLGNALVSVSSDQTGAKFDGATMSNNTHVSG
DDHIDVTNPMKDLAKGYMYGFVSTDKLAAGVWSNSQNSYGGGSNDWTRLTAYKETVGNAN
YVGIHSSEWQWEKAYKGIVFPEYTKELPSAKVVITEDANADKNVDWQDGAIAYRSIMNNP
QGWEKVKDITAYRIAMNFGSQAQNPFLMTLDGIKKINLHTDGLGQGVLLKGYGSEGHDSG
HLNYADIGKRIGGVEDFKTLIEKAKKYGAHLGIHVNASETYPESKYFNEKILRKNPDGSY
SYGWNWLDQGINIDAAYDLAHGRLARWEDLKKKLGDGLDFIYVDVWGNGQSGDNGAWATH
VLAKEINKQGWRFAIEWGHGGEYDSTFHHWAADLTYGGYTNKGINSAITRFIRNHQKDAW
VGDYRSYGGAANYPLLGGYSMKDFEGWQGRSDYNGYVTNLFAHDVMTKYFQHFTVSKWEN
GTPVTMTDNGSTYKWTPEMRVELVDADNNKVVVTRKSNDVNSPQYRERTVTLNGRVIQDG
SAYLTPWNWDANGKKLSTDKEKMYYFNTQAGATTWTLPSDWAKSKVYLYKLTDQGKTEEQ
ELTVKDGKITLDLLANQPYVLYRSKQTNPEMSWSEGMHIYDQGFNSGTLKHWTISGDASK
AEIVKSQGANDMLRIQGNKEKVSLTQKLTGLKPNTKYAVYVGVDNRSNAKASITVNTGEK
EVTTYTNKSLALNYVKAYAHNTRRNNATVDDTSYFQNMYAFFTTGSDVSNVTLTLSREAG
DEATYFDEIRTFENNSSMYGDKHDTGKGTFKQDFENVAQGIFPFVVGGVEGVEDNRTHLS
EKHDPYTQRGWNGKKVDDVIEGNWSLKTNGLVSRRNLVYQTIPQNFRFEAGKTYRVTFEY
EAGSDNTYAFVVGKGEFQSGRRGTQASNLEMHELPNTWTDSKKAKKATFLVTGAETGDTW
VGIYSTGNASNTRGDSGGNANFRGYNDFMMDNLQIEEITLTGKMLTENALKNYLPTVAMT
NYTKESMDALKEAVFNLSQADDDISVEEARAEIAKIEALKNALVQKKTALVADDFESLDA
PAQPDEGLENAFDGNVSSLWHTSWNGGDVGKPATMVLKEATEITGLRYIPRGSGSNGNLR
DVKLVVTDESGKEHTFAATDWPDNNKPKDIDFGKTIKAKKIVLTGTKTYGDGGDKYQSAA
ELIFTRPQVAETPLDLSGYEAALAKAQKLTDKDNQEEVASVQASMKYATDNHLLTERMVE
YFADYLNQLKDSATKSDAPTVEKPEFKLSSLASEQGKTPDYKQEIDRPETPEQILPATGE
SQSDTALFLAGVSLALSALFVVKTKKD (SEQ ID NO: 1253)
>orf00824 
LQIAQESSQDTDGINPPVVEEAMVFDRNDCLNQICGNIISLGIDAAFRTQVSNELIFIVV
DFTRSCCN (SEQ ID NO: 1254)
>orf00826
   MLNLMWMKIFHRNRTFLFCFLGFKVDVISIINARIVRR (SEQ ID NO: 1255)
>orf00827
   VYNSQALRQIVVVGSIDHLFKRHSSICEIFGLFKRWLSFL (SEQ ID NO: 1256)
>orf00830 MTSIIFSAKDIFEQEFGREVRGYSKVEVDEFLDDVIKDYETYATLVKSLRQEIADLKEEL 
TRKPQVSSAPSPSHPDPIDVAASSSMTNFDILKRLNRLEKEVFGKQILDNTDL
(SEQ ID NO: 1257)
>orf00854
   LISIKHFFWLPLSKKMIIDIIVNKNPDRFCMIEKVKKTMAENR (SEQ ID NO: 1258)
>orf00858
   VNIDSSEFYISHITDGIFDSFLDSNRYLRNFYSVLKVEIDICCEFFVHVFKINATAE (SEQ ID NO: 1259)
>orf00859
   VNPLYLCSSDSNDFFKYTWGDNDFAKLFFNSHRMTSF (SEQ ID NO: 1260)
>orf00887 
LAQISILHFDFLSIDKHSHTVFNTLRKSLQTTLALSATSKQCFEQLAASFLVCSLIFIEY
KV(SEQ ID NO: 1261)
>orf00897 
MLYVGIDIAKNKHDVTALNVPGKTVLKPLTFSNNKAGFELLDLSLRQLNQDCLIALKLLS
DPNREQFQHDNRQVDLKILARHIHRLKKKQSDWKVQYTRCLDIIFPELDKIVGKHSEYTY
QLLTRYPNPQKRIEAGFDKLIEIKRLTASKIQDILSVAPRSIETTSPAREFEIIEIIKHY
KRLIDKAETCVNDLMAEFNSVITTVTGIGGRLGAVILAEIRNIHAFDNPAQLQAFAGLDS
SIYQSGQIDLAGRMIKRGSPHLRWALIQAAKACARFSPAFKAYLKTKLEQGKHYNVAIIH
LAKKLIRTLFYILKKSCHLTNKK (SEQ ID NO: 1262)
>orf00900
   MDTKSSCLITTGRNDSPSTCLPRVASNNDRFSSEFRIIPDFHCSKKGIHVNMDDFS (SEQ ID NO: 1263)
>orf00903 
MMSIREQDLKDIGAIIKYKNFHSPFDTFKYLKDMGFDTIDLSVLLEGFSYAYGMDWLEKF
FKENQDKLREFY (SEQ ID NO: 1264)
>orf00909 
MIPLYRTDNDITKFFTKIRNGHLAKTAGGLDDKFHEANASTSKAFDRQGVGEVNDIRDSA
GSQELRINDKRKTENILFLEIRVRIFRVPHPNDSFFSSHFLG
(SEQ ID NO: 1265)
>orf00910 
VLSQGDKDITILDAGLLKNGKIGPVTKDTNDIKATDNMIENSFVLLNQQNIMLFCNQGAT
EGKTNFSPSDKDNFHNKTYFFMM (SEQ ID NO: 1266)
>orf00915 
MKIKEQTRKLAAGCSKQCFEIVDRTDEVSSKHGFEVVDETDEVSNHTYGKAKLTWFEEIF
EEYKMMGKAGQLVFFDVYRLVRQVS (SEQ ID NO: 1267)
>orf00942 
LVEIVRGGSPRPIKDYLTSEVDGINWIKIGDTEKGEKYINNVKEKIKKSGLNKTRFVKKG
TFLLTNSMSFGRPYILNVDGAIHDGWLAISNYENSLNKDYLFYILSSNVVYSQFLSLISG
AVVKNLNSDKVASILIPLPPLSEQQRIIEAIESALEKVDEYAESYNRLEQLDKEFPDKLK
KSILQYAMQGKLVEQDPNDESVEVLLEKIRAEKQKLFEEGKIKKKDLDISIVSQGDDNSY
YGNIPMNWVVIKIKDIFSMNTGLSYKKGDLSINNKGVRIIRGGNIKPLEFSLLDNDYYID
TQFISSEQVYLKHNQLITPVSTSLEHIGKFARIDKDYDGVVAGGFIFQLTPFESSEIISK
FLLFNLSSPLFYKQLKAITKLSGQALYNIPKTTLSELLIPLAPFEEQELITQKVEKLFEK
VNQLWK (SEQ ID NO: 1268)
>orf00963 
VDRTDEVSSKHCFEVVDTTDEVSSKHCFEVVDRTDEVSNHTHDKPTLTWFEEIFEEYHSP
FHN (SEQ ID NO: 1269)
>orf00964
   LDNIHIVLDSLNAVSGIQDFICDGLAIFCDQITSGCSSCK (SEQ ID NO: 1270)
>orf00979 
MKSTLGIISVGLVITYILQQVMSFSRDYLLTVLSQRLSIDVILSYIRHIFELPMSFFATR
RTGEIISRFTDANSIIDALASTILSLFLDVSILILVGGVLLAQNPNLFLLSLISIPIYMF
IIFSFMKPFEKMNHDVMQSNSMVSSAIIEDINGIETIKSLTSEENRYQNIDSEFVDYLEK
SFKLSKYSILQTSLKQGTKLVLNILILWFGAQLVMSSKISIGQLITFNTLFSYFTTPMEN
IINLQTKLQSAKVANNRLNEVYLVESEFQVQENPVHSHFLMGDIEFDDLSYKYGFGRDTL
TDINLTIKQGDKVSLVGVSGSGKTTLAKMIVNFFEPYKGHISINHQDIKNIDKKVLRRHI
NYLPQQAYIFNGSILENLTLGGNHMISQEDILRACELAEIRQDIERMPMGYQTQLSDGAG
LSGGQKQRIALARALLTKAPVLILDEATSGLDVLTEKKVIDNLMSLTDKTILFVAHRLSI
AERTNRVIVLDQGKIIEVGSHQELMQAQGFYHHLFNK
(SEQ ID NO: 1271)
>orf00981 
MTSYKRTFVPQIDARDCGVAALASIAKFYGSDFSLAHLRELAKTNKEGTTALGIVKAADE
MGFETRPVQADKTLFDMSDVPYPFIVHVNKEGKLQHYYVVYQTKKDYLIIGDPDPSVKIT
KMSKERFFYEWTGVAIFLATKPSYQPHKDKKNGLLSKLPSSDFQTKISHCLHCSLKLIGH
YYQYRWFLLSPRNLG (SEQ ID NO: 1272)
>orf00984 
MDTKMMSQFSVMDTEMLACVEGGGCNWGDFAKAGVGGAAVVAALGCAAGGVKYGKILGPW
GAAIGGIGGAVVCGYLAYTATS (SEQ ID NO: 1273)
>orf00988
   MKKKILIIFVLYLIMSIFLYPLRESIWYNLFYTIAYMIAVMIYFSLIKKKEKK (SEQ ID NO: 1274)
>orf01008
   LNCKGNDHPKEFHNPNNRFDKKNSKKTKKNFILSPLA (SEQ ID NO: 1275)
>orf01009 
MKIKEQTRKLAAGCSKQCFEVVDRTDEVSSKHRFEVVDRTDEVSSKHRFEVVDRTDEVSS
KHRFEVVDRTDEVSNIYTAR (SEQ ID NO: 1276)
>orf01017
   MHSQTFQFLLMTDKTSLLHRKHRSFIRNIHSKFLILFDLLCGILSRNDSNHNPIS (SEQ ID NO: 1277)
>orf01021 
MSDVKEEVSSLSEKQLRQIDVEYAELNDSDIIERLAYLEINNNEKRIVISDIEPTKEIMS
VSDQIFEIQKNFQKIKNMFELFISDVSDFLSIKNKLESKELEIEEADVNRFMIHLLSSGK
LFVDFNENQIKQKYSKDSEEFDCIHGFASYQYDTNFAYRFCHSLRNYSQHTDLPINEVKA
VSPDDETVIIDFYIDLDYLLNSNFKWKKLKGELIKLNQETSKIDAIALVKEYFNALTELY
GNYNKLFLKLNHNTLVDIKSKLESLKLKHSRYYISKISKYDLKYNPGNYTMSPLAAFAEI
EEIYIELSKIGLVKIVNKSN (SEQ ID NO: 1278)
>orf01025 
MSKHPHYELLNLIGYGLAKFDKLFIKEFQCFSKSEFYRYVVSLGIAETTGVVKNRMDLFD
PYFDNNRKGWWQKAEVYRFRKDLIDMMFGNEDVHSYAEIVKMLLASEGKKTGITIVEKPI
VRTKFKRLQETGMEAENYFILHFDKEEKFQGGQLTDARLYGDGYDFQVDVQEYSYLAEVK
GIRKSKGRVRLTAKEFEKVKEFQSDFILSLVTNLDDIPKLVLIDNPLKHFEFKKNIIKNE
IIEYRSVEDLY (SEQ ID NO: 1279)
>orf01027
   MFIAEFTAILLNEFPVALDSLVFMGFSMKLIHDLDTHTTHSTAKMLYNMKAIKNDFSIRE (SEQ ID NO: 1280)
>orf01049
   LKHLFCHFNPLWIDEIIRLAYKDQDTKDVKSKVNIGN (SEQ ID NO: 1281)
>orf01077 
LCCNRHIANLDLEFISYHLGQVGFDTRISTGLGIFVTKIGNVLFDTDNQFASFLNVCDTC
ISLDWFGSSKAEKANQ (SEQ ID NO: 1282)
>orf01095
   MKEIAFDAFYQLYQNDQLSLVDVREVDEFAALHLECAHNLPLSQLADSYD (SEQ ID NO: 1283)
>orf01098 
MCLICQRIELIKAGQNPYFVKELETGYVVIGDYQYFKGYTLFLAKDHVTELHHMETSVKL
RFLEEMSLVQEAVAKAFEAEKMNIELLGNGDAHAHWHLFPRRAGDMKSHGLNGRGPVWWV
PWEEMAAEDCQVQSPELEEMIKILSHELEKYLA (SEQ ID NO: 1284)
>orf01099 
MKKRYVILSGLLALTLAACSQEKTKVEENTQKTEQSSQPEGTVGSKSQASSQKKAEVSNK
GSYYSIQGKYDEIILANKRYPLSKDYNPGENPTAKAELLKLIAAMQAEGYPISDQYSGFR
SYETQAKLYQDYVNQDGKEAADRYSARPGYSEHQTGLAFDLIGTDGDLVTEEKAAQWLLD
HAADYGFVVRYLKGKEKETGYMAEEWHLRYVGKEAKEIAASGLSLEEYYGFEGGDYVD
(SEQ ID NO: 1285)
>orf01104
   MKTI<EQTRKLAAGCSKHCFEVVDRTDEVSNHTHGI<ATLTWFEEIFKEY (SEQ ID NO: 1286)
>orf01105
   MDFFFMNEVKEQVLFRDNHSEHIFWIEGVSDFMIKVNTALW (SEQ ID NO: 1287)
>orf01109
   VCFLGFQTILANPSKPQRQLPFLIFILDFFNYKHHKFLS (SEQ ID NO: 1288)
>orf01124 
MEELVTLDCLFIDGTKIEANANKYSFVWKKTTEKFSAKLQEQIQVYFQEEITPLLIKYAM
FDKEQKRGYKESAKNLANWHYNDKEDSYTHPDGWYYRFHHTKHQKTQTDFQQEIKVYYAD
EPESAPQKGLYMNERYQNLKAKECQALLSPQGRQIFAQRKIDVEPVFGQIKASLGYKRCN
LRGK (SEQ ID NO: 1289)
>orf01126 
MHIHYNTNQTTLPLEISSFLPQDHLVFTIEKVVNTLEDCHFHAFYHAFDRLSYHLKMLVS
TLLFAYSQGIFSGRKIEKWKS (SEQ ID NO: 1290)
>orf01129 
LRLWVIFVIMKVIKSYDTLNDYYRKLFGEKTFKVPIDAGFDCPNRDGTVAHGGCTFCTVS
GSGDAIVAPDDPPIREQFYKEIDFIHRKWPDVQKYLVYFQNFTNTHEKVEVIRERYEQAIN
EPGVVGINIGTRPDCLPDETIEYLAELSECMHVTVELGLQTTYEATSDLINRAHSYEL
(SEQ ID NO: 1291)
>orf01131 
VETVKRLRKYPKIEIVSHLINGLPGETHEMMVENVRRCVTDNDIQGIKLHLLHLMTNTRM
QRDYHEGRLQLMSQDEYVRVICDQLEIIPKHIVIHRITGDAPRDMLIGPMWSLNKWEVLN
SIEMEMRRRGSVQGCKAVKQEFENEKTT (SEQ ID NO: 1292)
>orf01143
   VQVCVFTNFCFFHCFSSLANCRLFNLRGICLPCISYQ (SEQ ID NO: 1293)
>orf01152 
VFKKDRFSIRKIKGVVGSVFLGSLLMAPSVVDAATYHYVNKEIISQEAKDLIQTGKPDRN
EVVYGLVYQKDQLPQTGTEASVLTAFGYLSGDILKTLGLDTVLEETSAKPGEVTVVEVET
PQSTTNQEQARTENQVVETEEAPKEEAPKTEESPKEEPKSEIKPTDDTLPKVEEGKEDSA
EPAPVEEVGGEVESKPEEKVAVKPESQPSDKPTEEPKVEQVGEPVEPSEDEQAPTAPVEP
EKQPEAPEEEKAVEETPKPEDKIKGIGTKEPVDKSELNNQIDKASSVSPTDYSTASYNDL
GPVLETAKGVYASEPVKQPEVNSETNKLKTAIDALNVDKTELNNTIADAKTKVKEHYSDR
SWQNLQTGVTEAEKVAANTDAKQSEVNSETASLKTAISRLNTDKVELENQLKIAQGKTET
DFSMESWTVLSTAKNKAQEVKDNGTATQEQINEAEKSLKTALADLSVDKTALGSAIDTAT
KKNKENYTNQTWAELETALTAAKSVNTNESKQSDVNEAAEKLTATMEKLVELSEKPRLTL
SIEKRDIDRKATVTYTLENPANTQIKSITATLKKGEEVVKDFVLTEENLKTNHLTALFEK
LDYYKEYTLSTDMVYNRGNDDETESISEELIQLNLKKLELKDIQTVSLMKFENGQESQVT
HLSDKPTDLSKLYLKVTSSTSKDAVLAVSSIEEEIIENKKIFKIHADTPELVVRKKDGSL
 
SKGFDYYMERVIPHDGDIYYDFKDLISAMTSNPTGTFILGRDISSRNVKPDGNGKSYIKG
EFKGKLLGTNDNVRHSIFDLEYPLFDTIKSGVVKDIDFKHVNMVFPDSNQGDNVATIARV
IKDKTKIENVNVEGYLEGRDHVAGLVNNLEGNSEIENVSFTGKIKSKGGNSITAGIAGRN
ILSRVKRAYVNADIEVHRSSNSSMLVAVNGINADASGGWGTWGRLTESVAKGTLETKQGG
QAGGASSTVWPYGAIDNVVSYAKVTKGKELFGSDGDLNYDWFMKKISNIFGVQGISSGDS
GSDSKFTRISEEEANQKVASYNITAPNLMSDSSLLVDRLNESWKNTDQFESIQDYQAQNQ
LIYQNLTKFTPYYNKEFIVHEGNALTPEQEILKTKKIKSIVGLKGTEFVVDGSDIDTIML
HFEDGSQKRYKVTSTGKFSITNLPEYQVEDLNVVYTSEHIVHPLDSSLINNLVEELKKVE
LYTESTYQVLGIDKDNANKLNRTKRLFLDESLDAVKTQLPTFVKTMFENEWLHINGESSG
AVAALRQKIMDNKTAILLALTYINRYYDVKFSDYNIKKLMLFKPTFHGEKIDLLDRLIRL
GSSGENRLKGSENAETFKQLFASETKQKDLVTYLDYNRSLLTNYQTTGEWFKETTKDYIQ
FEERPSLVEEIKDAKYRVYDNLTAPYYQGYILPLLTLKNTHLAILSNYSTMTFVSREKRP
NWKNEDFDKWVKYVATAHRNHVDTWYKILPDNIKGKMVKENVTAVWEGLSIPGSEWVDQN
AVDRKGRDYAPAREFFNLVGGPMGGWYAYHGYGAHAGGRNRVNYEVFDVLSEYGISVFTH
ELTHVNDTWIYLGGYGRRENMGPEAYAQGLFQSPVPGQPGWGALGLNMAFERKNDGDLIY
NASPTQFENRKELDSYMKNYNDTLMMVDYLEGDAVISKGKEAITKWFKKVEPKVVSQTAQ
YDTVRQLTAEEKEKLSVSSVDDLVDQGLMSDRAVGNNTYNPADFETSYIAIDYMTGIYGG
GKNSVGSPGALMFKHNTFRMWGYYGFEEGVLGYASNKFKQASRDEGHAGLSDNFIISKIS
KGEFLTMEAFKKGYFKKVVEELKTKGIRPVTINQKTYSTFEELQEGFKQAVERDLKKNQL
DERETRNFKFQVFRQLLQQTDSFKTSIFR (SEQ ID NO: 1294)
>orf01156 
MKSKIVLGASLAIATLSLVSLVEIEGLSPFLIENVSANTHSANKVINHKVSIYLENADEG
KGLTVNFSTDSVSPNLFDEFEKKSGITITTMLVNAKTGEVVEKRLTPSVFLRSNDLTSGT
ISSFIFSEYPDGEYKYVVSKGDFIDPKTQFKHQYRGESPVFRIRNRKYVELGTTDKKLDE
RRDNSVYKDGVVEHKVNLSLTSYQGGNGVTAIFSTDSVNSNLLNSFGEKAKKVLIRSKLI
NVKTGEVIDETFSPKVSLTSKILKSGSTAVFYFIDLTDGEYKYVAYESQQYTDPQTTLTH
QYRGESPIFSIKDGKFSGLVSASKPDENPKPTPKPDEKPKPSAPQQEKTKPTVQSGWVGS
SYYQNGKKVTSKWIFDKKYNSYFYLDASGNYVQNAWVGNYYLKSGGYMAKSEWIYDKNYG
SYYYLTSEGSYARNTWSGNYYLKSNGKMAKSEWVYDSNYKSYYYLTSEGSYARNTWVGNY
YLKSNGKMAVNERTPDGYRVDGSGKWVK (SEQ ID NO: 1295)
>orf01157
   MSACTVCAEKGRTPDLSIVDNVPIVENAKAHENNFFYSSDITYYPIF (SEQ ID NO: 1296)
>orf01158 
MAKYYIILPKDAEIYKTWRGTVNIPIIDATKTTPELSYFKEDHRNYIANENKSGANYIEW
KGTVEEFKEAIKKLTDKKSTTATPKKDEKPTPKPDEKPKPTPTVQSGWVGSSYYQDGKKV
ISKWIFDKKYNSYFYLDASGNYVQNAWVGNYYLKSGGYMAKGEWVYDATYQAWYYLTSDG
SYAYSTWQGNYYLKSDGKMAVNEWVDGGRYYVGADGVWKEGQASTASSSNDSNSEYSAAL
GKAKSYNSLFHMSKKNVCIDN (SEQ ID NO: 1297)
>orf01179
   VSRWDGHSDKGEAPAGKTSYAWIWTKWGEQVAFYCDYD (SEQ ID NO: 1298)
>orf01193 
MKSKKGGELRIAVFGDKKPFGYVDNDGSYQGYDIELGNQLAKDLGVKVKYISVDAANRAE
YLISNKVDITLANFTVTDERKKQVDFALPYMKVSLGVVSPKTGLITDVKQLEGKTLIVTK
GTTAETYFEKNHPEIKLQKYDQYSDSYQALLDGRGDAFSTDNTEVLAWALENKGFEVGIT
SLGDPDTIAAAVQKGNQELLDFINKDIEKLGKENFFHKAYEKTLHPTYGDAAKADDLVVE
GGH (SEQ ID NO: 1299)
>of01194
   MKLFKPLLTVLALAFALIFITACSSGGNAGSSSGKTTAKARTIDEIKKRR (SEQ ID NO: 1300)
>orf01231 
MYQDEAGFGRISKLGSCWAPIGVGPHVSHYIREFHYCYGAVDAHTGESFFLIAGGCNTE
WMNAFLEELSQAYPDDYLLLVMDNAIWHKSSTLKIPTNIGFTFIPPYTPEMNPLNKCGKR
FVNVDLRIRPFELWKMS (SEQ ID NO: 1301)
>orf01233
   MVTATTCFLKERVEFELLIFFYISPNRCLITVYSVLNL (SEQ ID NO: 1302)
>orf01234 
MDTPDENGYVADDYRITYLEAHIKAMRDAIYKDGVDLLGYTTWGCIDSVSAGTGEMNKRY
GFIYVDRDNVGNGTLKCSKKKSFYWYMSFIAMV (SEQ ID NO: 1303)
>orf01255
   MFLGMIGNISIILQFFGITIIVKIDNQARAIDFFKHDKSSF (SEQ ID NO: 1304)
>orf01257
   MFSLNFFDDSVFLSIKIAHKGCFQLLDMTNPNFFNKFFLAQASDQLLHFLSWNIEL (SEQ ID NO: 1305)
>or01266 
MTEPDFWNDNIAAQKTSQELNELKNTYNTFHKMEELQDEVEILLDFLAEDESVHDELVAQ
LAELDKIMTSYEMTLLLSEPYDHNNAILEIHPGSGGTEAQDWGDMLLRMYTRYGNAKGFK
VEVLDYQAGDEAGIKSVTLSFEGPNAYGLLKSEMGVHRLVRISPFDSAKRRHTSFTSVEV
MPELDDTIEVEIREDDIKMDTFRSGGAGGQNVNKVSTGVRLTHIPTGIVVQSTVDRTQYG
NRDRAMKMLQAKLYQMEQDKKAAEVDSLKGEKKEITWGSQIRSYVFTPYTMVKDHRTSFE
VAQVDKVMDGDLDGFIDAYLKWRIS (SEQ ID NO: 1306)
>orf01267
   MLQSNQVQNFHHSSFDITAIFPDYFHSVSNIFIDSFLW (SEQ ID NO: 1307)
>orf01299 
MQVIKRNGEIAEFNPDKIYQAILKAAQTVYVLTDDLRQNLAQVTKKVVLDLQEAKVERAT
ISMIQSMVEHRLLGAGYITIAEHYISYRLQRDLERSGYGDHIAVHLHFEQIR
(SEQ ID NO: 1308)
>orf01305 
MKLKLCIIGFFFCLIATIGLVTISDTEIPIPLPIDGAFSIQGKSNLSNNEIYEMVRDLSK
TEKVTIYKPIVQSSGQLKYVNFDDVNNEQLKSAPIVGMYYTLGKMDVDSLKPLTMTGLQT
VYMAYPWYIGGILQFTGTLRILLMGSIYLTLLVVLFVVRTRQIKEGVIRRSLGLPIYDLR
REYGISLIFELIMMALLMISYSSFLGNGFFTYSSKLFFSLLLTNFILFQIIDLITFVLFW
LTIQIEKPIEIIKNKAKNKLIFVVWLAIISIIILVSGVFLQETKSSQSSINIQIQNLVPW
DTVKDWRRIEFLGIESNSTKNREVNDSDGQYLQIVAALKNLDFLYIERSSAYVPDFMKTS
HVIENFSKQLENDGITNPEINKELIYINQTGANLQNKVNGTNYHLLDNKIATIYIPEKWK
ENQKSIENTVVAEQFIGTNYTREQLAVQIIPDGEKIFYFNEDADNNLKMKDILPLANVAD
SKDNIVVVLDTDKMMENNKFSLASNILYKSLFSPEAVKKINEMTVLLNFSMNPVDVYQIV
KLKIQSLEHQILLSQILQKIIYSIVFILIYQYVQLFITLKQNEYVKKIILGLSKTYIAIS
SLKYFMMTITMVILFTFLMTGQIELLYIGAASLLVLMLSIIMSFRKLSESYTKILKGDES
(SEQ ID NO: 1309)
>orf01306 
MTIDLLNVSKSFGSKKIFTDLNLIFESGKSYALIGGSGSGKSTLLNIIGRLEKIDSGNVL
VDKQDIWKIKERTFFKNTVGYVFQNYSLIDNKTVYDNLSLITKDKKTITDVLEKVGLSSD
YLHQKIYELSGGQAQRVAIARMLMKPRKIILADEPTGALDGEIGKEIIRLLLNETAEDKY
VIIATHDPAVYNEVDVIIDMKDIGYKV (SEQ ID NO: 1310)
>orf01307 
MKKKIYIALIFVTGVLAIFFFGKQMITKENINKPTVELTIYTLSSSDTEKWNKVRQVETE
EAIYFITVKEVSSSEEVFSNIIANGAATGFGVREEEVKKFNNGLGDTIEDSKHNKLIEIE
FFTFSDDGAGFVVANFDYGKEELNSQKKDIKELYKKIYESFKEKNK
(SEQ ID NO: 1311)
>orf01317
   MKIKEQTRKLAAGCSKHSFEVVDETDEVSSKHSFEVVDETDEVSNHT (SEQ ID NO: 1312)
>orf01324 
MELFKTWKKNMVLYGLKSQIGTVYRNNDRTTSFYDVGNFLYLAGELDSRFWEDFVRKYGL
DYKIIISENTNWQDFLHRKVGLNSFTRYSFKDKANFQVEFLNNLVTHLEEGYNIVPIDNH
IYNCFSTEEWSQDLQGDFESYQDFVLKGGFGFVILKNNELIAGISSGLVYRKAVEVEVAT
 
RPNEQGNGFAKKLGAAMILESLNRDMFPLWDAHNEASKKVAEFLGYELSEPYEAFELEEI
LI (SEQ ID NO: 1313)
>orf01369 
MKVIDQALLEKVIIERSRTSHKGDYGRLLFLGGTYPYGGAIIMAALAAVKSGAGLVTVGT
DRENIPALHSHLPEAMAFSLQDQQLLKEQLEKAEVVLLGPGLRDDAFGEDLVKQVFAGLR
QNQILIVDGGALTILARTSLSFSSSQLILTPHQKEWEKLSGITIEKQKEDATASALTSFP
QGTILVEKGSATRIWQAGQSDYYQLQVGGPYQATGGMGDTLAGMIAGFAGQFKQASLYER
VAVATHLHSAIAQELAQEQYVVLPTEISNCLPKVMKRYV (SEQ ID NO: 1314)
>orf01376 
VLDSKEELKESENDAPKLETPLREEPRLAPQTLPEASEVLENKREESKVEITEPAQADDI
RKVVGELAKDISITKLYMTGHSLGGYLAQIAAVEDYQKYPDFYNHVLRKVTTFSAPKVIT
SRTVWDAKNGF (SEQ ID NO: 1315)
>orf01404
   MGRKPKKRPEERTELEHLQAENEYLRAENAILKKLRELRLKEEKEKEERQKLFKN (SEQ ID NO: 1316)
>orf01417 
VVLSTSAILVACGKTDKEADAPTTFSYVYAVDPASLGYSIATRTSRTDVIGNVIDGLMEN
DKYGNVAPSQKDYDLNSTGWAPSYQDPASYLNIMDPKSGSAMKHLGITKGKDKDVVAKPG
LDKYKKLLEDAVSETTDLEKRYEKYAKAQAWSTDSSLLMPTASSGGSPVVSNVVPFSKPY
SQVGIKGEPYIFKGMKLQKDIVTTKEYNEVFKKWQKEKLESNSKYQKELEKYIK
(SEQ ID NO: 1317)
>orf01421
   LNFDFFIFLAHFIPLFTFSILQENPKTSKKKLYIRLL (SEQ ID NO: 1318)
>orf01428
   MRLSMKLIHDLDMHTTHSTAKMLYNMKAIKNDFSIRE (SEQ ID NO: 1319)
>orf01442 
LIRIIRNIYRSGEGNTSVFQSFIDQINSNQFCYGSNFDRLRCILLIENFTSICLNSNRMF
SGNGKILSNSSRSTP (SEQ ID NO: 1320)
>orf01453 
MSNYRRTSKPKTEHIKKGFTVFQKTITTIGSILGLITAGITIMNALDNNNKNTKKEPTTS
QTTTFVKEIQKESPQENTTPNKENNTSQEKTQQEETPKSSVKEEKKEDQKTATQDSTTPA
TSKPATENEKQPNTPTSENNTQ (SEQ ID NO: 1321)
>orf01457 
MNQSYFYLKMKEHKLKVPYTGKERRVRILLPKDYEKDTDRSYPVVYFHDGQNVFNSKESF
IGHSWKIIPAIKRNPDISRMIVVAIDNDGMGRMNEYAAWKFQESPIPEQQFGGKGVEYAE
FVMEVVKPFIKHKTGWFDGMMTTGCSMGAYHALNFFLQHPDVFTKVIALSGVYDARFFVG
DYYNDDAIYQNSPVDYIWNQNDGWFIDRYRQAEIVLCTGLGAWEQDGLPSFYKLKEAFDQ
KQIPAWFAEWGHDVAHDWEWWRKQMPYFLGNLYL (SEQ ID NO: 1322)
>orf01466 
MSSIHTKNSSLKSKSRFNEMFGDPLNNNKKFAVKTGQQCFKFSSGKFLDKHDRVFEGYPA
YGGNGIAWKSRKYLIDNPTIIIGRVGAYCGNVRTTHGKVWISDNAIYIKEFKNSDFNLVF
LLELMKVIDFSKFADFSGQPKITQKPLENQKYILPPLALQNEFADFVALVDKSQFACEIA
IKVWRNSLKFSII (SEQ ID NO: 1323)
>orf01476
   LVYAPFSFNILLDYITFDFKILLFSVFLAINRFHNDFIQFLL (SEQ ID NO: 1324)
>orf01479
   MSEYSGLSFFEVALAEFLDIVSAVYLEDADGIIVNLWGILDK (SEQ ID NO: 1325)
>orf01490 
MEGVAKGRIGRKKNNGIDNRCCHKKRNGRVTWNLFFQKTIDDGDDSTFTRREKYTDKGPK
KDSPPTISREKMINLVRCDINFNQP (SEQ ID NO: 1326)
>of01493 
VDKTDEVSSKHCFEVVDRTDEVSSKHCFEVVDRTDEVSSKHCFEVVDRTDEVSNRTTVRR
S (SEQ ID NO: 1327)
>orf01495
   MTEFMSDNFPKNLHTQFLINLGIKIQMPIFGEKSPTCRT (SEQ ID NO: 1328)
>orf01503 
VVIGVASATTNIWIIFLSGFTAILAGAFSMAGGEYVSVSTPKDTEEAAVSREKLLLDQDR
ELAKKSLYAAYIQNGEFKTSAQLLTNKIFLKNPLKALVEEKYGIEYEEFTNPWHAAISSF
VAFFLRSLPPMLSVTIFPSDYRIPATVLIVGVALLLTGYTSARLGKAPTKTAMIRNLAIG
LLTMGVTFLLEQLFSI (SEQ ID NO: 1329)
>orf01535 
MSFKNNWIDKEGRVFIYFTVEEIMKRRNISKPTAIKTLDELDIKKGIGLIERVRLGLGKP
NIIYVKDFMSIFQVKENDLQKSKNLTSEVKDFNLRSKENELQEVKNLDSNYIENNKSKYS
KREYSFGENGLGTFQNVFLAAEDI (SEQ ID NO: 1330)
>orf01543 
LLHIRVCKTFDRIPYCMLALFLSKSIGLTILLHKVKTVIFIDDQSNDKTCKICIHISFFR
IKLSQQCQLSFSVYF (SEQ ID NO: 1331)
>orf01547
   MTQEDALIVISHIKVLSIVPNRCLKPLDKTFSLYNWIFLSQKYLLQANFLKISRVQLQ (SEQ ID NO: 1332)
>or01552 
VTTHDEPVYEKHGVLHYAVANIPGAVARTSTIALTNVTLPYIEALAGKGFAQAISEDEGL
RQGVTTYQGYLTSLPVAQGLNRDYTDINDLV (SEQ ID NO: 1333)
>orf01553 
VFFIDGFIVRCHTVSCFDNATLVNSNVNDTEPGRICLTISSVTNSGAFAPGMRMAPITTS
ASLTLASMLNELDIRV (SEQ ID NO: 1334)
>orf01555
   LSTKTKGDAGSMCTDDTITDDSYFSFGTPGTPPGRTPEPPACLVRK (SEQ ID NO: 1335)
>orf01556 
MLIGIPKEIKNNENRVALTPAGVHSLVSRGHRVLIETNAGLGSGFTDADYQKQGAEIVAT
AGEAWAAELVVKVKEPLNSEYGYLRDDLLLFTYLHMAAAPELADAMLAAKTTGIAYETVR
DNQGQLPLLVPMSEVAGRMAV (SEQ ID NO: 1336)
>orf01576
   MASRNVLSMEPKFLLAGHFKGQFLILKIVSSDIDDGFAIAC (SEQ ID NO: 1337)
>orf01577 
LSSADKTCLNQFFTDFSDFFQSSLVEDGFYTFQIENSGFGFFNQISQVFDSFFEFLIPFK
IALGILVGSQSLIKRNHDRLVGIVVV (SEQ ID NO: 1338)
>orf01578 
VSVFLKFIFNTTNQFTGLLFDAVALSLILIVGVQQIRKICKRLSHLICKRNWTEGSLSQA
WLGFLIEKISESGKFFTNQYPFQFICSIASQTLKEALKIFCC
(SEQ ID NO: 1339)
>orf01579 
MVSNLVFIGNCNFHNTVIFHLLNRLNQGPLQILSQNHDKGRRLSWIFKSRLGQLNASKNW
MGRKEQAMALAIAADLQDQLLFKRLIDFLDATIH (SEQ ID NO: 1340)
>orf01599
   LLYNPVEKTRVHIKKGIGKLQYLFTRLFYLIFVSTDYISYGSSSEG (SEQ ID NO: 1341)
>orf01630
   MRSYITLICNLNNNLFCLNSFFLTNLVWSQIFSLLSVFITVYI (SEQ ID NO: 1342)
>orf01631 
LLEITLKSPYQFAHILFQSTIVPHGGHYHFIPESDLSAGELAATYVFNPNDIVRDTGDAY
IVRHGDHYHYIPKSSLNNPPSHSNTEEVGSSSSSVLSNPSLHVHHEEEDGHGFDANRIIS
EDSEGFVIPHGDHNHYIKVQTKGYEAALKNKIPSLQSNYQPGTFDEKAVLAKVDQLLADS
RSIYKDRLS (SEQ ID NO: 1343)
>orf01664 
MARLEPAKIAKIVLGILLYIIDLIKSSFVLPIPKAAKKSLILISFVPSFNDKNIVIRRPR
QITKIMPRFICFLFRIFACIS (SEQ ID NO: 1344)
>orf01680 
MELSAIYHGPESEYAYLYKDKKLHIRIRTKKGDIESINLHYGDPFIFMEEFYQDTKEMVK
ITSGTLFDHWQVEVSVDFARIQYLFELRDTEGQNILYGDKGCVENSLENLHAIGNGFKLP
YLHEIDACKVPDWVSNTVWYQIFPERFANGNALLNPEGTLDWDSSVTPKSDDFFGGDLQG
IIDHMDYLQDLGITGLYLCPIFESTSNHKYNTTDYFEIDRHFGDKETFRELVDQAHHRGM
KVMLDAVFNHIASQSLQWKNVVKNGEQSAYKDWFHIQQFPVTTEKLVNKRDLPYHVFGFE
DYMPKLNTANPEVKNYLLKVATYWIEEFNIDAWRLDVANEIDHQFWKDFRKAVLAKNPDL
YILGEVWHTSQPWLNGDEFHAVMNYPLSDSIKDYFLRGIKKTDQFIDEINGESMYYKQQI
SEVMFNLLDSHDTERILWTANEDVQLVKSALAFLFLQKGTPCIYYGTELALTGGPDPDCR
RCMPWERVSSDNDMLNFMKRLIKIRKYASVIISHGKYSLQEINSDLVALEWKYEGRILKA
IFNQSTEDYLLEKEAVALASNCQELDNQLVISPDGFMIF (SEQ ID NO: 1345)
>orf01688 
MGQEIKLIRKQFRITRQEEKQIKEMMREQKVDSFSEFLRQNLLKKNYQDRIFESWFSLWQ
SQKFEQISRDVYEVLVVARENHQVTQEHVSILLTCVQELIAEVNQVQPLSREFREKYMG
(SEQ ID NO: 1346)
>orf01689 
MVYRYRTNLKKVFLTDPELHQLNERIAKSNCQNFSVYARKVLLNPNMSFVTINTDTYDQL
VFELRRIGNNINQIARAINQSHLISQDQLQELSKGVGELIKEVDKEFQV
(SEQ ID NO: 1347)
>orf01690 
MVVTKHFATHGKKYRRRLIKYILNPDKTDNLKLVSDFGMSNYLDFPSHTEMVEMYNVNFT
NNDKLYESRNDRQEKHQQTIHAHHLIQSFSPEDNLTPEEINRIGYETMMELTGGRFKFIV
ATHTDKDHVHNHILINAIDRNSDKKLIWNYALERNLRMISDRISKMAGAKIIEKRYSYRG
YKKYRESSHKFELKQRLYFLMQQSK (SEQ ID NO: 1348)
>orf01691 
MMTDRAMTKPIRGRQLSKRDLYDEEFFRTHFAKQEIESRLEFLLNRVNSLEELITKAKEL
NLTIDLKQKNVTFILKENNQKISLGHQKISDKKLYDVKFFQDYFKNKEVIASEGLENLQE
QYHAFQEERDKDKVSTEEIEEAFKTFKKPLRHLRKNEIPFVNLKWNLQRTK
(SEQ ID NO: 1359)
>orf01692 
MEEENHKKYKVYIRETSSYFVYNKENMDNNCFIKGRTLIRQLSNDSQKLPYRRPTLKSLQ
EKISEINLMIELSNTNKQYQEIKDELVLEIAEIDMKLEETQEKIATLNKMAEVFINLKSE
DEIGRKLAKYDFDQMNMTESIMLDRLNTDILKLQQELGNEINKYEEIARRLDLFVKIINT
NKFTVLKFHENALLE (SEQ ID NO: 1350)
>orf01693 
MKSKLGITLRKVRKGKQISLCSVADEHLSKSQISRFERGESEISCIRLINILDKLHITLD
EFLVLHNDDYTSSESFANLVQYIRKQYSSQSINNIACLLSDTSDYTLNSFEKTMVKSILH
TMDSNIIPSDEELLHLTDYLFKIEKWGYYEIILLGNCVRTINYNSYFLLTKEMLNNYIYS
SLNKTNKQIVSQLAINCFILSIDKEEFSNCSYLISKIKTLLDNELNFYEQTVFLYATGYY
EFKRQLSSGIETMKQAIQVLDILGEDKLKLHYTSHFDKLVNNK
(SEQ ID NO: 1351)
>orf01694 MSLSYYYEINPSTDILKCIEELLYKEDKCFNNILKNWKDIRRNHNDSFPNFWCYGAPGIL 
LARKEIFDKTNIGNNDLSIIKNVLTNVEKIRELNLCHGSVGTISCLDAILKDEENLLIKE
SIDFYFDNVVSQVIKPELSTDLNTMNTFSFMLGVSGVVYEISRKQDDRLLNVLLLELRGH
DD (SEQ ID NO: 1352)
>orf01695 
MMTRKVPNIEQMSQIECGLCCCLSILHFYKSKETLLDLRRDIEKGRDGYSIGDLKQLLNK
RNFDTGSYQVKDVNKISELPLPLIAFWDNQHYVVIYKVKKNKVYIMDPSKGYINYEFKEF
SKHFSNIVLLSFPNENYQSLKSQFPSPWIRVFSSFSKVKGRLILTLLFSIISYLIIILSVP
VMTSKFINSALGNTFSFQTSFLILFSLLCLYLISILARSMGILFSNIFFSRDIESFTFKH
LLKLPYSFFELRAKGDILYRISSLSGFRELFTNQVVGGVVDIGTILSVVIYMFLSSKTLS
IIALILSLINFLFLFSTRKIMYDTVNRELQEQSLIYSVETEALNTISSIKISGLEDEIYE
NWSKYLKNVLTKYKKRSIVHILYNSATNVFQLFAPIIILIFGLDNVLNGKILLGEVVAFQ
TMASILFSSEISIFNAYTQYILAAGYLNRVNDIWLENEENVENGLKKCSLEGRIDIKDLS
FSYSKDSAPVIENLNLTIEPGQRIALVGQSGSGKSTLSKILSGLYKIDTGKILFDGVNIN
QIDKKILSQNLGVVPQDSFLLNRSILDNITLKNEVTSQKIEEVCKAVQIYDEIMAMPMKF
NTIISEMGSNISGGQRQRIALARALINNPSIVILDEATSALDTINEERITKYIQSQGCTQ
IIIAHRLSTIKDADIIFVMKGGKIVESGNHKYLMDLGGEYYSLYTKRK
(SEQ ID NO: 1353)
>orf01696 
MAIVEIINLTKSFKDIEVIHNTSFYLNKGKVYGFVGPNGAGKTTIIKMILGILKPDSGKI
TIFNQTVEQNSENILSRIGLVLGPSFYGHLDAYKNLKLIANMKGLSLDTERLNEYLSMVG
LKDVKKKKVKNFSMGMKQRLSIAASLLGSPEILIWDEPINGLDPQGVIEIRSLIRFLQEK
KGITFLISSHILSELDKVISDIIIINYGKVEFFGSCHYLLQKYNCRNLEEAYLACLAGGE
YD (SEQ ID NO: 1354)
>orf01697 
MIKLEFLKQKKSILWFVLIFPIILNVLLYIDLTFRYRGYLLVHQNELALSNWQLIFKEQT
IFYFSELFYLVLSLIIYEVFAVEFKNDAWLTVISLPFRNKYTINSKLLITVVYTFTFWLS
DYISLYVIGKAIDNSLEIGLIFFLKTFTIQLISSLMIMLLYFLTLVLIRKISGIIPIGII
MMILTISIYYNDYNFKIYLPFTYLSHAFRVTESQFYMILLSNIIIIVLFYILIRKLNERS
FEMKL(SEQ ID NO: 1355)
>orf01698 
MKLLKNELIKSKIFLFIIVDICIQILVILAIKTYILDISALYSELDYNKYWYILHTLIYM
LMIFPIQILYQNLREALIEDNNNGWNIMVINTNNLVKIIYIKVTINIVRCFICYFVYTIF
SLIQLGGMGTDMLLTNIIFPNIMSFLLFLPIAIFMQICCIRFDSILAKALPNILLILIVL
ITFQSDWNIFIPATYYYTEIQSTTNLGIKLLVCIWIMGFEFFLLPKLIKLKEQNLV
(SEQ ID NO: 1356)
>orf01725 
MRFSAFKIFSNSVCKRIITKGLGFRALLLYTISKVKLREDILVSQSIVPVEIPQYCRFDS
KKRNGILFNVRIANLKFTFFRLDFLRNKIWYSSSMNDEASKQLTDARFKRLVGVQRTTFE
EMLAVLKTAYQLKHAKGGRKPKLSLEDLLMATLQYVREYRTYEEIAAVFGIHESNLIRRS
(SEQ ID NO: 1357)
>orf01753 
MHTKSRTIKSLITQFTAILLYELPLALDSLVFMGFSMKLIHDLNTHTTHSTAKMLHNVKA
IKNDFSIRE (SEQ ID NO: 1358)
>orf01776
   MIKIYFTKFSENHNPFCKIFEIIFTNLIFQSILNKNKKNPLRQGEANVV (SEQ ID NO: 1359)
>orf01783 
MSQVKGLCVLDVDGTLILEEVIDLLGREAGHEAEISQITSRAMRGELVFESSLRKRVSLL
EGLPILVFDNVFNSIHLSLNVPEFISILQKNGILVGLVSGGFTPIVGEISKIPWYCLFHC
QPA (SEQ ID NO: 1360)
>orf01784
   MLKSAELGIAFCSKEMLKKEIPHHVDKRDFLEVLPLIDCLE (SEQ ID NO: 1361)
>orf01789
   MFGNWFFKAFVCSLERLAQDRTMNWFSCIGNKNTVAFVPILIGCFA (SEQ ID NO: 1362)
>orf01804 
MEKYFGEKQERFSFRKLSVGLVSATISSLFFMSVLASSSVDAQETAGVHYKYVADSELSS
EEKKQLVYDIPTYVENDDETYYLVYKLNSQNQLAELPNTGSKNERQALVAGASLAALGIL
IFAVSKKKVKNKTVLHLVLVAGMGNGVLVSVHALENHLLLNYNTDYELTSGEKLPIPKEI
SGYTYIGYIKEGKTTSDFEVSNQEKSAATPTKQQKVDYNVTPNFVDHPSTVQAIQEQTPV
SSTKPTEVQVVEKPFSTELINPRKEEKQSSDSQEQLAEHKNLETKKEEKISPKEKTGVNT
LNPQDEVLSGQLNKPELLYRDETIETKIDFQEEIQENPDLAEGTVRVKQEGALGKKVEIV
RIFSVNNEEVSREIISTSTTAPVSRIVEKGTKKAQVIKEQAETGAEHKEVQSGAIVEPAI
QPELPAAVLTDKGESAVQPELPEAVVSDKGVPEVQPALPEAVMTDKGDPEQVEPLPEYTG
VQAGAIVEPEKVEPEYAGVQAGAIVEPEQVAPLPEYTGVQAGAIVEPEKVEAPKEYTGVQ
AGAIVEPEKVEAPKEYTGVQAGAIVEPEKVEPSKEYTGVQAGAIVEPEQIAPLPEYTGVQ
AGAIVEPEKVEALKEYTGKIEQPSAEDTKPNNENTNTPEEMSIQKKSSALINMNFITDSS
KVTGVGSATFIAPNVLLTVAHNFINNSTDNTTGEFRGDKSKNVYEWVTPDGQKGTFTANN
IHFYNKKDYPKGFIYDLAVIKLPETTGREHVELVKNYSKVNLNDKLNVHGYPAGKYTHLK
DATVEMEQEYANNTYGVQYQGGNPGMSGGGIFNANGEVIGVHQNGAQNRSGGLILSPTQL
AWIKSIIAGNEIPPVYDELYRHKDEKKDDAKDEKEVIKKLELRNISSVELYSKDGNKYRH
VTSLASLPSNAENYFMKVKSENFKDVMLPVTSITNDTKDNRDVYKIVASANSLIQHENNN
VLENYTYYLPKTQQSETGVYTSFKNLVDAMNSNPNGTFRLGATMDAREVELPDGQESYVN
NVFHGILVGTNNEKYYAIYNLKKPLFGELNGATVEKLSLKDVNISAKDDTATLAKEANNN
THIDNVHADGAIAGERSIGGLVSQVNNSTISNSSYTGRITNTYKTVASYQIGGLVGKLSG
PRGLIDKSFASIDLSSNATQGDQSIGGIVGAVENSALISNSYAEGNLNNVQRFANVGGVV
GNLWDPVGGLEKSGRLSNVLSDVNVTNGNAIAGYNFNGIKANGTYSNKNNKVVNVVQEDD
EILTKDSTVQRGEVLEDAQIKEKKATFVSKNTIKTEDFNFSSRYVTDYKNLENADSSKEK
VYKNIEKLLPFYNRETIVKYGNLVETSSNLYNKELLSVVPMKDKEVISDINKNKSSINKL
LLYYADNTSETLNVNYQTDFSNVAEYRIGGTNLIYTPNTLLRNYQNILDEVLPALNSVEY
KSEAIRKVLDVSKDVSLTELYLEEQFNTTKTNLKDSLTKLLTADAAIAENNNKVIDNYVI
EKIKNNKEALLLGLTYLERWYDFKYRDTKAKDLVMYHLDFFGKSNSSALDNVIELGKSGY
NNLLAKNNVITYNVLLAKNYKTNNLFDALEKYRKAFVPDKTNNEWFKEQTKAYIVEEKST
IKEVSDKQSIAGSPYSIGVYDRLTSPSWKYPSMVLPLLTLPEKSVFIIANISTIGFGAYD
RYRSKEHPAGTDLNDYVEKKAKEAAVRFRDHYDYWYRILDDKNKEKLYRSVLVYDAFRFG
TDEKEDKDTYQATFETNHPAIKHFFGPAGNNVVHNSNGAYATGDAFYYMAYRMLDKDGAV
TYTHEMTHNSDREIYLGGYGRRNGLGPEFYAKGLLQAPDHPNDPTVTINSILKYDQSEES
TRLQVADPTQRFGSVDDLNKYMHNMFDVIYMLEILEGKAVAKLDTNQKYDLLRKIENEYK
PDPDGNSVYATNVVRRLKPEELTKLTTFNSLIEHDIITRRGYVDEATYKRNGYYTINLFS
PIYSALSSKIGTPGDLMGRRIAFELLAAKGYKDGMVPYISNQYEKEAKAQGKVITSYGKQ
IGLVTDEIVLSKVFNNQYNSWIDFKKDMYKEREDKFGKLNKVSFIDPNGSWARQQKVTID
NINRLEKMIEDAVKFDAEDEVAKLYPETNSRVLKLKKAIFKAYLDQTGDFRSSIFENKK
(SEQ ID NO: 1362)
>orf01807 
MTALGLLAIGSLIVIITKDNRNKKIATFLIVGATGLVTLSTASALNLNANIHESGRDGVL
QISGYRYVGYLELDDKTVSSVSPASTVSPVEQPKVVTEKGEPEVHEKPDYTQPIGANLVE
PEVHEKLAYTEPVGTTGVDENGNLIEPPVNDIPEYTEPVGTTGVDENGNLIEPPVSDIPE
YTEPISTVSEVASEREELPSLHTDIRTETIPKTTIEESDPSKFIGDDSVRQVGEDGERQI
VTSYEELHGKKISDPVETVTILKEMKPKILVKGTKEKPKEKTAPVLTLDRTNTNVLNRSA
TLSYHLVNTDGVTINKITATIKDGNEIVKTVDLTSEQLDKQVEDLKFYKDYKIETTMTYD
RGKGEETATLEEKPLRLDLKKVEIKNIASTNLVKVNDDGTETPSDFMTEKPSDEDVKKMY
LKITSRDNKVTRLAVDKIEEVTEEGKKLYKITAEAQDLIQHTDPTKVRNKYVHYIEKPVP
KVDDVYYNFKELVDAMNADKNGTFKIGADLNATNVPTPNKQYVPGTFKGHLSSVDGKQYT
IHNIARPLFDRVENGSVKNINLGNVDINMPWADGIAPVANMVKNATVEDVKVTGNVVANN
NIAGIVNKIDSGGQLTNVAFIGNLTGVGDKGQYMAGIAGEIWRGNLAKAYVEADIVANRA
RIGGLVAKTDNGNDSMGIGKYGSIRKSVTKGTIKTKVLFETGGFINSNLPFGKLEDNISM
MRVENGEEFFGSSDLDYDGGYFTNGWLERNFVVKGVSSGKHSYKRSRDKIKEISQDEANK
RIANFGLTADKYEINEPVVNRLNRLTRREDEYKSTQDYKSERDLAYRNIEKLQPFYNKEW
IVNQGNKLAEDSNLAKKEVLSVTGMKDGQFVTDLSDIDKIMVHYADGTKEEMDVTKNTDS
KVQQVREYSVSGLGDVVYTPNMVVKNRDKLIADVKSQLSSVELISQEVRDLMSRRDKPAE
NTDERKNGYIKDLYLEESFAEVKQNLDKLVKSLVENEDHQLNGDEAAIKSLLKKVETNKA
KIMMALTYLNRYYDIKYGDISIKNIMMFKPDFYGKTPSVIDRLINIGSSEKNLKGDRTQD
AYREIIAGNTGKSNLRNFLEYNMRLFTEDKDINDWFIHSAKNVYVSEPKTTNTELKDKRH
RVFDGLDNGVHGRMILPLLTLKDAHMFLISTYNTMAYSSFEKYGKHTEEARNEFKTKIDE
 
VAHAQQTYLDFWSRLALPNVRDRLLKSQNMVPTPVWDNQTYNGSPVGRRGFDSKGNPIAP
IRELYGPTWRHHDRDWRMGAMASIFPNPNNDDKVLFMVTDMISPFGISAFTHETTHVNDR
MLYFGGHKHRQGTDVEAYAQGMLQTPDSSTTNGEYGALGINMAYHRPNDGNQWYNPDPDK
LKTRDDIDRYMRNYNEAMMLLDHVEADAVLPKIKGDNSKWFKKIDKEMRSKIQYNDLLGP
NQWDSIRDLKDEEKVMTLSSVNDLVDNNFMTKHGNPGNGRYRPEDFTPNSAYVNVNMMAG
IYGGNTSQGAPGSLSFKHNAFRMWGYYGYENGFISYVSNKYKAEADKNNHGLLSDKLIIN
KVSKGNFNTLEEWKRHWYGEVLAKAKKGFEAIDIDGVHISNYDELRPLFDKAVEEDLKKP
DDFSHTVALKSKVFKALLKNTDGFFNKLFKEDI (SEQ ID NO: 1364)
>orf01818 
VFHKSLNNCKRKKVCYSSLLPSCFHDWLKNLLTKSQHFSHINFIVEGEGWRSQVRFNHAL
GNNLTHWCHWNTLDFTIWCYVIRDFFHFFNLSRRFDAIVFDIFRKQGQNILLHDFTTMTG
SLDFLPSNVMFEGNSFCKWRNANHVCVFISFHVFFVDTTVCT
(SEQ ID NO: 1365)
>or01822 
VLGGRANSVTSCTTNSHWNLTFTTKHVTCFSSLVDDIVHGNNREVHEGHIDDWTKSCHGC
SCCCSRDGSFRNRTVTDTFWTKFFKHSNRSTEVSSEDTDIFSHQEHIFIATHFLRHSKDN
GVTEGHCFCFHFISFSLVCVNIFKG (SEQ ID NO: 1366)
>orf01823 
MDMFYIGHFLDIRRDTVTVVNAIENDWQVPDRSHVHCFVENTFIGRTISKEANNDFTGIL
HLLTEGCTDSDPHTTTYDTIGTKVPSIKVSDMHRSTFPFTGSSVFTKDFSHHSVEVNPFS
NSLPVSTVV (SEQ ID NO: 1367)
>orf01841 
MISVWHCNTSSCSTCDLRWVENKAIRFHMALTQRQFVELFQETINVITLTCLTVSVAVVA
CVSICSSWIAYRRYPVCS (SEQ ID NO: 1368)
>orf01842 
MISMRNDISITSILYDIRSIKDITIICSIASLRTCQGNSSIVSWSPSFTILTMFLFLSID
FLFCTDVIRVGSILKVNIVFSIYLDNISTLDLINNILIF (SEQ ID NO: 1369)
>orf01843
   LVCYLDDDLLSIDSFTLANLIRSQILRFLRRLFSIYIGNTIIFLNRSSLIQSQLVRTNT (SEQ ID NO: 1370)
>orf01859 
MDKLIIFIEKGKPFFEKLSRNIYLRAIKDGFISSMPAVLFSSIFILIAAVPNIFGFKWSD
EQLAFILKPYNYSMGILALLVAGTTAKSLTDSVNTRSMEKTNQINYMSTFLAAVVGLLIL
AADPIEGGFANGLLGTRGLLTAFLAAFITVNIYKVCIKNNVTIRLPEEVPPNIAQVFKDV
IPFALSVLSIYGLDLIVRNIFGTNVAESVGKILAPLFSATDGYIGLAIVFGAYAFFWFVG
IHGPSVVEPLIVAISYANIEANVQLVQAGMHADKILNPVTQTFVVTMGGTGATLVVPFMF
MWLCKSKRNRIVGRASVVPTFFGVNEPILFGAPIVLNPIFFIPFVTAPIINVWIMKFFVD
VLQMNSFSIILPWTTPAPIGIVMGTALAPLSFVLAITLIIIDTLIYYPFVKVYDHQILEE
ERKGNSSSELKEKVAANFNTAKADAILEKAGVDAAQNTITEETNVLVLCAGGGTSGLLAN
ALNKAAAEYNVPVKAAAGGYGAHREMLPEFNLVILAPQVASNFEDMKAETDKLGIKLAKT
EGAQYIKLTRDGKGALAFVQEQFD (SEQ ID NO: 1371)
>orf01861
   MIFSNQIPLLLSECNPLTNYNHLFSLIISDKRDIVIHWI (SEQ ID NO: 1372)
>orf01868 
MDGFIVTVKIIGHLLVVVFSAIPFFKEFCKEVCVCLLSIVTFKVFNFRNQFLVFFRWFVF
SMNESFDDITHKQFTSNLTTKADNVSVQLFFSIKGCCHITNQGRTNTWNFIYSVVDTNTS
TTDTYPKISLAASYSFPYFFTKDWVVSPCMVICTKVNDFISF (SEQ ID NO: 1373)
>orf01871 
MLDFQDRSPWLEGQKEIDLSYDLFSTDAVTLDELQSRTIALRSLKHDKGLKVHFAEFPNL
IIWSTLNKGPFITFEPWSGLSTFLEEGDHLEDKKNVCLLEANQVEELGFEIEVL
(SEQ ID NO: 1374)
>orf01872 
MKLFKMSCRNIGQAGKILADSGYQGLMKIYPQAQTPRKSSKLKPLTAEDKACNHALSKGE
ARLRTSLPK (SEQ ID NO: 1375)
>orf01874
   MRRKYKSIALKKELANDSGKKKCHAMKAQAIVTSQGRIVSLDIAVNYLL (SEQ ID NO: 1376)
>orf01878
   MKIKEQTRKLAAGYSKHNFEVVDETDEVSNHTYSKATLTWFEEIFEEYKN (SEQ ID NO: 1377)
>orf01886 
LIESQVFSSLQVCCLNLCHLKFQHFDTCLVFLLVFLDFQNLLAHFPIGIKTRLIGFFQVP
KSGITKFIQHLDMQLGTH (SEQ ID NO: 1378)
>orf01887
   MVMLTMNIYKMLPNSSQNRQINHLTIYTADTTTILQDFPTDDNFIT (SEQ ID NO: 1379)
>orf01888
   MTNNICRRTSSQHHIHGINDNRLPCTRFTSQDSHPLFKIEGNSLNNGKVFYRNFK (SEQ ID NO: 1380)
>orf01899 
MPHTRDNWQTRFKNSSYHNFFVKGPEILNRTTSTTNNEQIQIVPLISTRNISSNFLRSPF
TLNLGRIKKDVNTWESPADGRDNISNNGSTTAGYYPNSLRKLG
(SEQ ID NO: 1381)
>orf01900 
LLEAFLKQAFFCQFFLKLFKLNRKRPNPIRLSFFNDDGVATTWFIDLYTPNHIDLHSFFQ
VKP (SEQ ID NO: 1382)
>orf01911 
MFMSNLCQFFQVWNINQGVTQGFNQDKLGIVFDSCFYFLQIINIDKGCCDTITRKGFFQK
IEGSTVNSRSSHYMVTSMGKRQNRISHCSHT (SEQ ID NO: 1383)
>orf01912 
LINVFSHGVDIAIHSATKFIGGHGTTIGGIIVDSGRFDWMASGKFPQFVDEGSSCHNLSY
TRDVGAVAFIIAVRVQLLRDTGAALSPFNAFLLLQRLETLSLRVERHVQNAETIVDFLVN
HPKVEKVNYPKLADSPYYALAEKYLPKGVGSIFTFHVKGDEEEARKVIDNLEIFSDLANA
ADAKSLVVHPATITHGQLSEKDLEAAGVTPNQIHLSIGLENVEDLIEDLRLALEKI
(SEQ ID NO: 1384)
>orf01913
   MTRDFKFETLQLHAGQVVTPATKSRAVPIYQTTSFVFDDT (SEQ ID NO: 1385)
>orf01917 
MSQKNNKKKNKRKNLLTNILAGFLILLSLALIFNTQIRNIFIVWNTNKYQVSQVSKEKLE
ENQDTEGNFDFDSVKAISSEAVLTSQWDAQKLPVIGGIAIPELEMNLPIFKGLDNVNLFY
GAGTMKREQVMGEGNYSLASHHIFGVDNANKMLFSPLDNAKNGMKIYLTDKNKVYTYEIR
EVKRVTPDRVDEVDDRDGVNEITLVTCEDLAATERIIVKGDLKETKDYSQTSDEILTAFN
QPYKQFY (SEQ ID NO: 1386)
>orf01924 
MRWNIGCHPNRDTSCSINQKVWKTRWQDQGFPFIGIIVINEINCIFVDITKHFQSNLAHT
CLGITLSGSTISIHGTKIPMTIYKHVTVAPPLSHTDHGFINRGIPVWVIFTHDIPCNTSR
FFMGFVWGHTQFIHSVENATVNRF (SEQ ID NO: 1387)
>or01928 
LKKKWFFVDYYDTTIILLALISVILVLLGFAEMIDLDNPPYSIIDLVIWGVFVIDYSWRF
FITKRKWRFILENIFDLLAILPLNAIFTVFRLGRIFRLAKLTKLLKLTRLLRIIGLTGKL
ERKISRFLRTNGLIYILYVNIFIVLVGSSILSVVEEKSFSDSLWWALVTVTTVGYGDIVP
ASIFGKWLAVLLMLVGIGTIGMLTSALTNFFVKDNPDEQIKLDKLQDELSSQRILLEKQS
 KKIEELHKMIQDLIEKT (SEQ ID NO: 1388)
>orf01938 
VVDFKQTRQDPHDITIYSWLRQVKSNTGNGSCCVRSNPFQAGNSFIGIWKLATKVSHNLL
GCSLHIANSRIIAQALPSFQ (SEQ ID NO: 1389)
>orf01943 
MAERTVVQVHNAFPEDTTLINSQLIPLVQVVVNQGRKGIVGSCNSMHISSKVEVDVFHWQ
NLCIPTTSSTTLDPHDWTKRRFADSNHGFLANLVQGIRKTNGKRRLSFTCRCWVDGSNQD
QFTDWIALNCTNFIKAEFSLVLSVQLQIVVRNTKFLYNINNWLQLNTLCDFNICFHSKFL
(SEQ ID NO: 1390)
>orf01950 
LRILDSQPCFFVDFTNDRLRKSLIIFYMTSRKGITRPAIVFRGAILHHHALSFEVFNQTN
IG (SEQ ID NO: 1391)
>orf01957 
MLFIIGHLNFPTAGSFIDSTLHRLGNRVCIHDDMAFTVTSSTSNSLDESTFVAKETFLVS
IENSYEAHFRNVNSFTEQVNSDQDIKDTQAQVTDNLRPFQGLDIRVHVLDLDTHFLEVVG
QILCHFLGQSCDKGTLIFFNAGIDFTQEVINLSHSRTDFHLWIQESRWTNDLLNHCLGLF
IFIVTRCR (SEQ ID NO: 1392)
>orf01958 
MNVTLKLLPTERTIVQSRRQTETIINQHFFTRTVSIVHALDLPYGHMTLVNHNQEIIWEE
VEKRIRRLSFAPSIHVARIIFNPIGIAHLTQHFDIILCPLFQTLGFKQFTFLFKDS
(SEQ ID NO: 1393)
>orf01959 
MIHFSQHLTCQSLNFTNTVNFVSKKFYSKGMFISGSWENLYHIPTNAKSSALEINIITFK
LNIDQVIQEFITRNL (SEQ ID NO: 1394)
>orf01960 
VAKLVNLVIDRTILLNIGIARRDIGLWLVIIIVGYEILNCIFREKFLKLPIELTSQSFIV
GNNQSWFIDFRNDLTHSIGLPCSSRPHQNLSFFSPLNVIHQLLDSLGLIS
(SEQ ID NO: 1395)
>orf01979 
MNITKTNFLAVNFVFTIPTTIDMAFYSDFLTCILDKSIMIIQSHNYRSIIKRFTTFCSSK
DDIRHLAPTETLDTRLSQGPSQTFCNIRLSRSIGSNDCRHTLVKDDLGLISKRLESLNFD
FL (SEQ ID NO: 1396)
>orf01981 
MGFIVCNHLKFACFNLRNHDLIDKFLDLGHILVQKKGTKKGFKGITKNGITIAPTRFFFP
LTQLDKLVKLAITRKASQTLLTDNHSTEF (SEQ ID NO: 1397)
>orf01989 
MRITDNQHKIAKEDFVAEYPKLSQALLDRTLDNLSREDNIFIFPNDLTHTPDLDKDQKIF
ETVNQKIKTGNVIGFLGYGQERLTISSRFSDESNDHFLHYLLNKVLHINLTSLDVALSRE
ERLYQLLMYLFPKYLQAAIRKGLYKEYHRFSHNDSHVKGVIDVRNHLKKNLPFTGNIAYT
TREFTYDNPLMQLVRHTIECIKNQKSIGQGVLDNLSTSRENVSEIVRVTPSYKLADRAKI
IRMNKIKLIRHAYFREYRKLQELCLVILSREKHGLGPQAQRVHGILFDVAWLWEEYVYTL
LPKGFVHPRNKDKTDGISVFSVGKRKVYPDFYDRERKIVLDAKYKKLELTEKGINREDLF
QLISYSYILKAEKAGLVFPSKDKVIDNEIGNLAGYGLFESLRMPHSIVHFVK
(SEQ ID NO: 1398)
>orf01995 
LDEDILLGCILPWKPEAFEKLKAYGNGREELMTDVRGTSCFVIKFGKAGEQLAAKLWEEG
KMVYASSASMTKRLKLAMSKV (SEQ ID NO: 1399)
>orf02000 
MAKKIVALVGDGIGPEIMEAGLEVLEALAEKTGFDYEIDRRPFGGADIDAAGPPLPDETL
KASREADAILLAAIGSPQYDGAAVRPEQGLMALRKELNLYANIRPVKIFDSLKYLSPLKP
ERISGVDFVVVRELTGEIYFGDHILEERKARDINDYSYEEVERIIRKAFEIARNRRKIVT
 
SIDKQNVLATSKLWRKVAEEVAQDFPDVTLEHQLVDSAAMLMITNPAKFDVIVTENLFGD
ILSDESSVLSGTLEVMPSASHSENGPSLYEPIHGSAPDIAGQGIANPTSMILSVAMMLRD
SFGRYEDAERIKHAVETSLAAGILTRDIGGQASTKEMTEAIIARL
(SEQ ID NO: 1400)
>orf02004 
LANIESHCNFFQSSIFSSLPNTIDSPFNTSCTILDSSKAICHCHSEVIMTVRRTDDLTIR
LDILNQVFEDGTIFL (SEQ ID NO: 1401)
>or02011
   MTAIWEIATSVEFTKTTKFNDHWTATHFTVKSSWFILNLDFFHFFFSLGNFF (SEQ ID NO: 1402)
>orf02016 
MPRNRFSFTVRVTREKNFISFFSFFFQVIDKRAFSSDINILRFIIIFNIDGHTGFLQITD
MPDTG (SEQ ID NO: 1403)
>orf02020
   MKIKAQTRKLATGCSKHCFEVVDKTDEVSSKYCFEVADGS (SEQ ID NO: 1404)
>orf02029 
VHAHTDKLCNGCNRIFNSIISHHTIFRERNKLSHKAIKSTRQEMGPCHVVFIEFFITLHR
RLIGNHDNFLTNLVGSGRVRNDGST (SEQ ID NO: 1405)
>orf02030
   VNHCHWKLFIQNLGITFSLIVTLIRMTDSHVVGTDKDMILLVNSLFLIFDIDKLRLS (SEQ ID NO: 1406)
>orf02032 
VGNNDILWSKRTISINGFNDFLNTCIAVSTTLCNDDTFLIKRKIFIYKIFCMRNPVSMNT
NYNFFNTWLQDKFFNCMNQNRSIT (SEQ ID NO: 1407)
>orf02042 
LVAPVASSTRFFKNNDSLTSWNNGFIIITINTIISYQRISKGQDLSIIRLVCNGFLVAGH
PCIKDDFACYINICSEGLAFKNCAIF (SEQ ID NO: 1408)
>orf02044
   VVCYFYITIDMSWVHEDCCFFQTIVTFLSQAMLGMVVFF (SEQ ID NO: 1409)
>orf02045
   MAFVLHTEKHHDINLINDFINGYKLSIVCKLLTSPFLRSSEKEFSSQAFQNLHIGFGNA (SEQ ID NO: 1410)
>orf02046 
VIQVTCNSNFKTLKVAKFLINGHQIKQALARVLARTISTIDDGSRNRWTSNQFSIVVDLW
MANHTDIHS (SEQ ID NO: 1411)
>orf02047
   MCPCRILKEEIGNNRMVFIGKLGSIFKLMSSLDQFHYLIDSEVFHGHHMVQCLLIF (SEQ ID NO: 1412)
>orf02059 
MQEHYTPKGKHLTIDNRRLIERWKNENKSNREIAGLLGKAPQTIHTEVKRGTTLQQVRKG
LYKKVYSADYAQTVYQFNRKRSVKKLILTKEIREKILHYHKQKFSPEMMVNKKQVKVGIS
TIYYWFHNGHLGLTKADMLYPRKRKGVKKQASPNFKPAGKSIEERPDVINLRLENGHYEI
DTVLLTKIKNYCLLVLTDRRSRHQIIRLIPNKTAESVNQALTLLLGEHHILSITADNGSE
FKRLSEVFPEEHIYYAHAYSSWERGSNENHNRLIRRWLPKGTKKTTPKEVAFIENWINNY
PKKCLDYKSPSEFLLGG (SEQ ID NO: 1413)
>orf02076 
VQFHLIIFQNLFCSLDIVIDSLTTDTELLGNFSKAVIISVVELDIIHLLICQKRRIKFKE
RIHTIGFFDFHNFYYTKN (SEQ ID NO: 1414)
>orf02079 
MKFNHYFFLFLIIEKQVAIISFFMHFHIIKLVNHFQLLIKLNCISHPNLHIRPSFLSLVL
LFYQKEQDFAIMVI (SEQ ID NO: 1415)
>orf02085 
MKKEQFYPLGIFLAAMLGGLVRYLVSTWLPASPDFPWGTLFVNYLGIFCLIYLVKGYLVY
KGTSKGLILALGTGFCGGLTTFSSLMLDTVKLLDTGRYPSLKPELAFEYRWRPAFSLLFG
EEEMVIVYLAIACGLGALVRYFFSRYNQASKLPLGTLIANLLGCFLIGVFYNHVESKEVY
AILATGFCGGLTTFSTLNDELQRLLSDKKVFYSYLTLTYIGGLVAIFLGILL
(SEQ ID NO: 1416)
>orf02097 
LNRSILDNITLKHEVTSQKIEEVCKAVQIYDEIMAMPMKFNTIISEMGSNISGGQRQRIA
LARALINNPSIVILDEATSALDTINEERITKYIQSQGCTQIIVAHRLSTIKDADVIFVMK
GGKIVESGNHKYLITLGGEYYSLYTKRK (SEQ ID NO: 1417)
>orf02100
   MSLLETAKRHQLNSEKYLSYLLECLPNEETLVNKEVLEAYLPWTKVVQEKCK (SEQ ID NO: 1418)
>or02101
   LKRPPKQADKSSLGAKGLAYCDQLFSLERDWEALPADERLQKRQEHLQPLMEDFFA (SEQ ID NO: 1419)
>orf02102 
VISIEMRTFFLYSSAFKKHSSPSPINDGLYHLLLQSLYNILELIHDIFQSLKGFILKSTF
TNLFPHLFNGVHLWCVWRNKCKANISRNL (SEQ ID NO: 1420)
>orf02129 
METKKIKNLKGQIIVSCQALEGEPLYTPNGGVMPLLAKAAFQAGAKGIRANSVRDISEIK
EEVDLPIIGIIKRDYDGFEPFISATMKEIDELVSEGVDILALDCTNRSRPGYDNITDFIH
DIKVKYPNQLLMADISTFEEGKVAAESGVDFVGTTLSGYTPYSPKKDNPDFELVERLVKE
LDVPVIAEGRISTPEQARKMLDLGAYAVVVGGAITRPKEIAQRFINVIK
(SEQ ID NO: 1421)
>orf02134 
MTKDILELESQKMSSDTFIDEIKNNYLSIVESTRKLIDGRQIELAIKLIREANQILMIGV
GSSGNAAREFESSLLRIGIISKTVIDTHFQLMHTALLKDNDLIIAFSLSGSTKEVEETLL
NAKRKNVKIISITNYSSRNIAKLSDCVLLTSKKESYLEGGSLMAKASQLFIIDVICTRLS
LINYEDTICKKEEIASLLSNKVE (SEQ ID NO: 1422)
>orf02135 
MQIKFIDKVSNLIMLNLLYVASVVTVIAIGSGESALIATLIKIVRHEESYPYRDFANSFF
KDYWKNLGAALISNLPILILLFSLFFLPYIPLPIYIISILRHIGVIYIILHLIATTFLIP
LIGRYNNTLKNSLHNSIMLAYKHFFIAVLIRIIEIIPVLLFFILQNQLLVWITLMIFILP
SITKYANAFLYNFIFSKYEKLN (SEQ ID NO: 1423)
>orf02136 
MVSGGFRLDFLLETARLARSTYYYQLKQLDGVDKDKEIKTEIQGIDNEHKGNYGYRRIHL
ELRNRGFVVNHKKVQRLMRILGLTARIRRKRKYSSYQGEIGKKAENLIQRQFEASRPMEK
CYTDVTEFAIPNSTQKLYLSPVLDGFNSEIIAYHLSTSPNLEQVKSMLEQAFTEKYYENT
ILHSDQGWQYQHDSYHRFLESKEIQASMSRKGNSPDNGMMESFFGILKSEMFYGYEKNFR
SLENLEQAIVDYIDYYNNKRIKVKLKGLSSVQYRTKSFG (SEQ ID NO: 1424)
>orf02137 
MKLSYEDKVQIYELRKQGQSFKQLSKRFGVDVSGLKYMVKLIDRYGIEIVKKGKNRHYSS
KLKQEMMDKALLEGCSQRSISLDYALPNQGMLSFWPAQYKKNGYTIVEKTRGRPAKMGRK
RKKTWEEMTELERLQEENERLRTEVAYLKKLKELEERDEALERERQRQLEKWFQEDFD
(SEQ ID NO: 1425)
>orf02152 
MVISKTKKYKGVYKDSKGKIYFQIELGVDPITGKRIQKKGRKNQQGLPFNSFKEAYEEIL
RLKHEFVNSTINNSFLTFREFMEEIYLKYYQQKVQFVTYQTALPHHQLFIKQFGSKKLSD
100
 
ISTIDCERFRLAIIDKYSSNYAKNMWSRFKACLGYAERLGYIDRVPFKGLDNPRGKHPDT
KFWTFDEFKKIINSFDISEYEGLHNYMTIWLYFMTGLRVSEGIALKWEDIDFERKWIHVH
STIEKDKNGVWYAKQQTKTVAGNRKIDLDDFTITILKKWREVQIKNDDKDYVISRFGAPL
CKSTISRIIKRHAKITGVPEITGKGLRHSHASYLINVLHKDTLYVSYRLGHADKSTTLNT
YSHWYYSGDSTISEEITNSLDNLGLSIYLPNSCQS (SEQ ID NO: 1426)
>orf02153 
METVNYKDLVAIGFPEHTSRNIIRQAKKIAVKKFEEARKNDKNAVQLGCSPFDNKRLGIA
PKNIVENLIGISFSDIEGEKNGYIKDKEI (SEQ ID NO: 1427)
>orf02154 
MLKRIRDLREDDDLTQEYIAKIVLNCTRSSYSKMEAGSRLISINDLIKLADFYKVSLDYL
VGRVDNKEDHYSKK (SEQ ID NO: 1428)
>or02155 
MVITKHFAIHGKNYRSKLVKYILNPSKTKNLALVSDFGMRNYLDFPSYKELVKMYNDNFL
SNDGLYEFRHDRQEVNQRRIYSHHIIQSFSPDDHLTPEQINRIGYETVKELTGGRFRFIV
ATHVDKGHIHNHIILNSIDQNSDKKFLWNYKSERNLRMVSDRLSKIAGAKIIENRYSHRQ
YEVYRKTNYKYEIKQRVYFLIENSKNFEDFRKKAKALHLIIDFRHKHVTFFMTDSNMKQV
VRDDKLNRKQPYNETYFKQKFVQREIINILEFLLPKMKNMNELIQQAEFFDLKIIPKEKH
VLFEFNGIKLSEQELGKMNQYSVSYFQDYFNNKNETFVLDNNNLIELYNKEKLIKEKELP
TEEVVWKSYQDFKRNRDAVHELEVELNLNQIEAVVDDGIYIKVQFGIRQEGLIFVPNIQI
NMEEEKVKVFLRETSSYYVYHKDSADKNRFMKGKTLIRQFTLQHEPQHMYRRIPLSKIKE
KIEQLDFLISAENSPNDFEDITNDFIAQISYLENMIEQVQNKIDDLTNLEEVLLNNTTNS
SSNLENSIQGKSSVDTIEKDLYIYKGKIETLKEQHGEAINLFEMFNKTIKKYKKKQNMKS
IEENEIHLE (SEQ ID NO: 1429)
>orf02156 
MKRDIRSIRKQFRLTETEEKQILDLMREKGEDNFSDFLRKSLLLSDGQKQMEKWFNLWKK
QKLEQISRDVHEILIIAKINHQVTQEHVSILLTCIQELIKEVEKTSPLSENFRNKYMR
(SEQ ID NO: 1430)
>orf02157 
MEYVEAVNQFIERHYKEKDIGHIEIDFWGNKNHPSLYIYKRSKKIEYDYFFFDSIDYYE
EPDFLEFKYIVHLENITYIFWQED (SEQ ID NO: 1431)
>orf02162 
MTTLDFKTLFKEEYDKLNKQQKKAVDTVEGPVMVIAGPGTGKTQILSRRVANILTNYHTS
SEEIVCLTYTEAGASEMLDRLEKLIGEEGRKVRVSTIHAFCSELILRNSEIFGGQPKIIS
TAAKYEILKEIMDEYVIEGNPLYKNSGKRYSAKDQLLELFYKMKRENLNKEDFEKEIDEY
FKMIDLSIPGDDLYSKFKYARNSKSKDKKVGDYKDKAINELKENTQKLLAGVEIIEKYSS
DISNHNYFDFDDMILWTIEKLEENEGFQRSVSDTIRYLFVDEFQDTSVVQNKLVDLLVKG
KDNPNIFVVGDDDQSIYRFQGVSANNIRDFDKKYKPTKIVLDENYRSSQAIIDASRQLIS
HNPREEKLLIAAGANKDYDYQLPILKSYENAKAEMFGVLTEIKELIDSGVSPNEIGVIYG
RNSYGEEFAKILRDKGIFVQMKENKDLFSEPFFKKIVAILKYLCKPSRDVRELRKIVYFD
FFEVVLSQIVMIRNLKKDEKISIPTIAEIDQKLEIIRKKVNQSSKYLSPMYVLSDVLKSL
SIDEYIMKSKEKYHLVSVLNELYKLMLMECHIHPKLTVKGFLNQLSALEEMGISLPIEDI
SGSPSNCVQLMTAHGSKGLEFDHVFIMKCNDGKKKSEAWPGGENNSGRFSYPPSLNGKDE
NESQLKEEENRRLFYVAMTRAKKVLHLSYANDSTKTHLINEFEEFIDEVDVTESFEDCQS
VDKVVMPKFSNNVINEIFDELSLSVSTLNSFLKCPLSFYFNKGLKLPSETNEAMVFGSII
HEVLEKIYISVDGSQSSELTAKTVLSLEEALKLFETVFEEKSYQLTSNKIKKDDYARGKK
IIENLYKKSGYLKDGVVAVEVPIQGIRLGDILNTTVDLSEVSNIEINGKIDKIECDGNIV
CLVDYKTGNFENAKKKLVAPSEKEPLGGDYWRQAVFYYILFKNAGIDISDKEILVKYVLV
ENSTNEDGFSETEDIRITQKEVDIVLNQIKESIMKIKQGDFNCGCVLKKDRDNYPCDYC
LQVSANTTPKFDNTEALEVATYQQTRGNYKSLSVSKLNRYLRCPKSIYFEDVLQLSQAAG
LSAGAKEKSTKITINHAPTGPVFGTAIHETMEKIYKEDLQLEDAIEFYDSSLYSHQEEII
DTMSVEELKEYGHNLLTNLFEHFIPNSLKGEHVSLEKELRVKLGDNYSINGIIDKLEFDN
DLIRVVDYKTGSAQRGVEELEVGHDYWRQAVFYNLLLENSSEIDTTDKRIETQYIFLDDN
STESGYSIHTIQVTKEDLDLVTSQIQNFWSHMNTADFTGSCGKNDCDYCRLAEFVDFELL
KETIESGKESNLVN (SEQ ID NO: 1432)
>or02163
   MSGRLTRQNYYLLGKLIDEFHAVKAAMRVIETKRNDFNI (SEQ ID NO: 1433)
>orf02164 
MKPQERLLTIFFRLQAGERLSKAQLSDEYEIDYRTVQRYMSTLKNFLQEQRISNTEIKFD
TSDNTYRLIAKTTFNKKDILVISKILLENRALNKSELYSLLEDLLSLLSSEEQKEIDAII
GSERFNYKSLTNDKDRIDTIWILSEAIRREQMLEIEYKAPLKDIKSHIIFPVSLYYDAHY
FYLVAYHLKHENYTTYRVDRMESLSESHVKKPEISYGRKYRDGEVRNQKVDAFEGRKIDV
TLIYKGNTEIVLDQFPEREILSENHDEIKVKIKTQDTPGLKRWILGQGDAVTLLSPSKLI
EEIQESLENTLRNYKK (SEQ ID NO: 1434)
>orf02165 
MSVQKTKNTLNEPLKTLLDEYHDKVGKINNSSELFDIYSPWNNFNIEKMIESFNKALQSN
SNNFSWLDIEEDLPKSTDVDIKYGLPNHIKGNIDEATLFLCLVNPNIDEVKTEKKDVGIH
TYYKKAREMESGDDSLNILNDKGKLRIDPKVYIKEHILDVRETSSILYNELQIVKQTRSY
KDTYYLGHYLPHFIKEFLNKKGSFKNVIHNLTDEWDELEKMSKKIANLEAFPFRSQNPNY
TYKSNKRATNFTNLLIESDSKVNLLSARVIIWRIVKHLESSQHKPAFILRRFNTFWLPTI
SKVLEQDLNFTKEEINQIINALDEEYFFTVRKKDYNGQSGYFGRNFCKNNERISNSSFKH
LVQETLGEYVKK (SEQ ID NO: 1435)
>orf02166 
MSGSFSDSPTHDDKFSIENYINGLSNFIIECETPLTVAIQGDWGTGKTSIMYQVEKRLNP
EKQDKKIQTIFFNTWQYSQFDMGNNLAVALITDLISELNVEDSKKKQFFKKAKGALSKGL
EYVNLDFGILNGEKLTEKFQDLIIGFGERTDDIKHLKENLQDIINDAIKENKSDRIVIFI
DDLDRLVPEKAIELLEVLKLFLDCEHCVFVLAIDYNVVVRGAKSKYGKDLDDEKGKAFFE
KIIQVPFTVPVANYDLQNFIESSLKKLDFCFDKNNKERNQLETITQLIRYSIGNNPRSIN
RLFNSVSLLMYINNGDKVDHDEKLMILAMVCFQLRFEEAYNYLLTAYNNSPEDSDDIESY
LIDLLENSFELLDDEVYYNSLVSLLGKFTFKDKKDRDDFTNFYRTLKELLGYNEQGLTME
QFNKLIEKMTFSNAVSIGNTDTITADKKKQNHAPNEDVQFVIRKLFNTLVGDENYFDLKK
PELFGKETREKREAPLSEEFISIPNEFDRIRLTRGKGQGLNIYSSHNKSNFIYISGDTHG
RMLNDGMAIVVNNNLVEKIKDNILASDLRSEELYHEFEMNFRDNLNKLLSKASKILNN
(SEQ ID NO: 1436)
>orf02167 
MEFIRAANQFIENYYPREDLDRIESIEIGIRDSENYSRYFLEIQKQSEEFECDFFNFDNI
DYYVVDDSVHFKQIINLENSSYVFWKDY (SEQ ID NO: 1437)
>orf02168 
MNKPIAAIFDIDGTIFRDSLLLKHMEKCVSYDVFPNSVNSEIKFHKNAWENRELDYDDYL
YIAATLYTKYIADKDILDIDFVAKKVIEKESKKLYRYTRDRIKWHKEQGHQIIFISGSPD
FLVSKMAEKLGADIWYASNYLQLDSKYTGEVIPMWDSTSKLQVLKKLFIDFEKSYAYGDT
TGDFTMLQSVGFPTAINPNKKLLDKITMEKLDCKIIIERKDVIYKLDEVTHGIY
(SEQ ID NO: 1438)
>orf02169 
MTNAKEFALTAHKNQTRKGKITPYSFHLFLVNNILETLTEDPHIIATGWLHDTVEDTDVS
LEDIKQEFNDEIYSYMSLESEDKSIKDWQTRKELQLAKFREAAEDESLRKVLLVTFSDKL
ANLMELYQDYLIIGGLLWDRFNSKDPKKQRWYFNEFYKIFKDNQDLFSKNKDILNNYKEI
LKLLFYNN (SEQ ID NO: 1439)
>orf02170 
LKAIVKNPKRLFELLRLYFVPKGRKVVHVPAYAYKEDENEKIYLHNNELHLSKKMFEFL
VNQGLDLVECLPEE (SEQ ID NO: 1440)
>orf02172 
MANSSEAHGRVYIKASNLKTIEDFLLIQEERNKYVYYPTDIIDSQSNISDIVSSRTTQEN
GYYICNMWFTAEGRWCFENNIDDFFDCTLFQDTDDVLIRQMKEYVCSQDIQIKFEYVDAE
ASQNFVKEQEATITYNSKTKDISIDVKTIKDLPYTAENLIVYGYYECDEIVSVQFLLDYY
DDYLRGNEFYLKHKDGIVPILERQQ (SEQ ID NO: 1441)
>orf02173 
MAESALINLINFSKENEELTNLVSGHASKREKATISKDGLIQARSIENFIDNYALSDFDF
STIKEKCVFIKINNSFQADDTTEDIYHNVRGVWNISESRKKDLEYALALYRGVCVGVYKI
QGWKKAYEHSSEYPFPTRKEGGKIETSEETIVKYSNIEDLKKDYPELYKRSFSNSEFPQK
SLDKWRNRSFFYGNWDGSDVPQHLAQCLNKRIINIPKFTKSVKEFKSIDNQASVIYNDLK
 (SEQ ID NO: 1442) (SEQ ID NO: 1442)
>orf02174 
MDLVEDCNTFLSFVADKTLEKQKLYKANSCKNRFCPVCAWRKARKDALGLSLMMQYIKQQ
EKKEFIFLTLTTPNVMSDELENEIKRYNNSFRKLIKRKKVGSVIKGYVRKLEITYNKKRD
DYNPHFHVLIAVNKSYFTDKRYYISQQEWLDLWRDVTGISEITQVQVQKIRQNNNKELYE
MAKYSGKDSDYLINQKVFDAFYKSLKGKQVLVYSGLFKEAKKKLKNGDLDYLKEIDPTEY
IYQIFYIWKQKEYLASELYDLTEQEKREINHKMIDEIEEEQ (SEQ ID NO: 1443)
>orf02175 
MNFNKIDLDNWKRKEIFNHYLNQQTTFSITTEIDISVLYRNIKQEGYKFYPAFIFLVTRV
INSNTAFRTGYNSDGELGYWDKLEPLYTIFDGVSKTFSGIWTPVKNDFKEFYDLYLSDVE
KYNGSGKLFPKTPIPENAFSLSIIPWTSFTGFNLNINNNSNYLLPIITAGKFINKGNSIY
LPLSLQVHHSVCDGYHAGLFMNSIQELSDRPNDWLL (SEQ ID NO: 1444)
>or02176 
MNYQKLNDITGATKNEKDKYYVYGLYEEGKQLPFYIGKGEGTRLISHIDEALTEINQEEN
IQISKKIQIIRKHKGKIIFVII (SEQ ID NO: 1445)
>orf02177 
MTTTKNPWNQLSNVDINGEQAILATEDVELIKKYTSTKHYKNLKDDIYRLQLGFCPQQFV
GDIQNADIIVLSKNPGYTPEFKTLYDHDKNYQKTLLNNLQLKGNLYFHAFDLDTNEFGYW
AKKFKVWFDDVDNLQDLKEKLPWFSKHVALAEYFPYYSTKYDDKLNDFISKEGYFPTQKF
LFNLIRERVLDDNDPVIIIITRSYNKWYDAIPQLKEYKNCYETSNPSNPSLKPENLLKVK
RYSAKKEVEKVLEDSLKKLEHK (SEQ ID NO: 1446)
>orf02181 
MFTKLFKKNQDNSDVFKKLIHRLSDMSIQDLKKIDRLLDIIFTPDQGSEQLKTEATYREE
TLDDTLKEAKNQLHMEQLEKNLERFRKNSQ (SEQ ID NO: 1447)
>or02183 
MTKDWNFNQPLESKSENQEDPDKIAALFGNHQGGNEVNYEAAFQKRKQAPVTESNSSSKP
KVTEVRTGKETDITTSYQQHLKRLIADNNSDIQSSQKKIEELHTLIDTKNKDNKKLQSIY
DAISELH (SEQ ID NO: 1448)
>or02135 
MTIIERLEEKVTRQESKVARETEKLAAYKEQLETAMFATFKRRQSISHMSFEEALDHAFG
KERQFDDSEFRKDEMSE (SEQ ID NO: 1449)
>or02187 
MEIEECKKISILDVANRLGISFKQVSSSVYEHPEHDSFRIFSTTNTFKWFSRDIQGDVID
FVRLVKGISFKEALAFLSEEPFQKEAVQEKRERPFYYPLKRIEDSNCSLARYYLTECRGI
SEEIIQKMIQQGLMSQASWKTNETVEPVIVFKSFDHRHKLQAASLQGIYKNHSLPRERLK
TILKGSHGHVGISFDIGKPNRLVFCESFIDLMSYYELHQQNLFDVRLVSMEGLKRSVVAY
QTLRLIAEENQKLEFLDTVIPSKLLPLINTIRDTTSYFDNHFDLLTLAVDCDDAGKDFSD
KLSRSGFPVFLDLPDNESGKEKRDWNDILREKKSDLQLMIENAKETLRNQPVRQTSQCLE
L (SEQ ID NO: 1450)
>orf02189 
MLNKVKTKALISVGAVAATSFILMVGYTIGQHSTAKQSRKEIELAATKLVEDKQAEDKAS
ILSSDTVKEFLTQYYTKEKLGENNTRIQPYMTESAYSQELSSQNDAMNQVYKDYILDYHF
EKADIFVNQTTNQAIAMVSYNVTYVSDLKNANQSKTNQTETRTVKLSYSKLPGKLLVNQV
QVWKSGLDDLDKVTPKTLEESSSIPSLPNTTTK (SEQ ID NO: 1451)
>orf02190 
LKKVKFIQQLSETSCGLACMAMILDYYGHEANLYELCCDFENSRDGLSIREIKDIASYFG
LDSKATEILNIKKFLGNKFVEPYIALTQNAHYIVVEKHNEHSVVFIDPERGRITEDISNF
ANNISGIVIFFSPNRKFTKKKKHSNFFKILKIGKIDIKRLCYISLISILVQSLTLLLPLL
TRFIIDNVISKGEMYRLGMLFSIFSLITFYALFSFIRTKLIITVEKRYIFTLKDKIVGKI
FTLPMKFFDSRSSGEIVTRINNLDSLEKIISSGISSLLIDLSTIIIAFIAMAMISLYFTL
IITCFAIFLFIVLYFLLKKLEEKNSNFISSKELTQGYLMEIFSNLLFLKVSAKGDVSYTK
WKEMFSNELKFDVERENYLNIFQTFISIYRLVPNLLILILGGLEVQQHMMSLGSLMSFLS
LVSLLLSPITLIVQNCFQFQFCFTILDRVFDIIYTPEEKNQFSISKLPPFEMMTFRNLSF
 
AYSSACKVIYDISLTIKKGERIAIVGRTGCGKTTLIKLLLRLYDVEKNTILYNGNDINLF
DLNSYRKSFGVVLQNDVMFNDTVISNIDLTHSHSMEDVISAASLAELDIEINNMPMGYYT
SIGDNGNNLSGGQRQRLAIARAILQNPEIIIFDEGTGQLDTITEKKIMDNLKHKGITQIF
ITHRLSNAQEYDNIIVMDNGKIIDSGKHDELCGKEGMYKRLFETSYN
(SEQ ID NO: 1452)
>orf02191 
MNRKLNITDFKDASYFSERKISSHDLGDGLKSRKRKEYWDNFIGEGSNEFWKYLQEKNDF
SKHDLDIFLEEHTYDVKDIDSFSHMDEFIEFFFKRNDYTLPEFYIVDSEGKRKPIFSNFV
IPFLKFAAYKLEDNLSSQNLKVTRDVLNSLLIALFQQILNISYRTLILELQVLKEQNMLK
GETGEKRFKYFSEIYLSDHFWDILKEYPVMFRLIIENIQNWVTNNVEFLTNLKEDKALLQ
EHFSINGELTKIESGVSDFHNHGKSVYLLWFGTNKLVYKPRDLILDVKFQNLLSWYNLKF
NKNLYVTNILNRGNYGWVEYIEHLPCTYESDFIQFYTHLGYLLFLLFAMRGNDIHFENII
AKGNRPVLVDIETLFHNTTEYRKEYETADKLIFSLLEKSVKRVGILPNIVWGKDGNSGVD
ISGLSSSAGEMIPIERASIMHSMTDEMKIGYEQSALQSKDNQPFIQSGKDVDLNSYKNYV
SAGFKEAYEIISKDPSSIEEFLVEIEKFNNAYSRQIMRPTQFYSNLIQTSYHPSFLRSGL
DREMLFSKVWKIVFEDKKVQRIASSEFESLLLGDIPLFQTKISDRFLCSELTKYQNFFNI
SGMELAIQQIKDFCQKDMEFQLNLIETTLNYDPSYNLVQDSFVPLKPSVKVIRNLNQKEI
QETKSRIVPLTEKIADYLSKISYSGTSGDICWVDMNILGEKTNDWNMVPIGCDLYNGISG
IMLFYLFLYLETKKKDYLIYLKKCYRSLKYYLDSRKQFATHSQVLFGGFSGETPIIYVLL
LLKTRMPEHFNSDELDVYIYDIIDDLKKGYRYDENFDVLVGSAGAIIHLLNVFEVTRDEE
LLILAHDLFSHLEKNSTKITVEGQDGRAWKGTMASNPLAGFAHGVSGIVWALSKLSRYFP
EDKKLKTIIKQGIIFENSMFDTEKSNWSDYRETESGIKYKDIVENIPVSWCHGAPGILIS
RLELYKNNTLNVEFRRTMKSDMDVAIDTTIKYGFGKSHCLCHGDLGNLNILFYVAKKMSS
EHLYNVVYSYLNTILDDLESENWKCGLPYKNSPSLMSGIAGIGLGLLTLNNLSIPSVINL
EIW (SEQ ID NO: 1453)
>orf02192 
MLMLEFTKLRRRKILYMIPFVAILLFLLEFMIGHQIYQGHSYGSVNGWYVENGFFFLNYY
FLLPFASMILVDLIRIEQVSKTISNLRLIPVDLKQLLQAKFMLALLINLLVSEFTFLAML
VLELMDGDFAFSSLAMLSWGVVYGAIAFAYTLSAGIIVLFLGKYRKELILALPLAFLLSF
AGLFALTTVVGRYYLANLLLIIMEQFTALTVSVYAIWLVTLVACLLYLLADKRMMNIIFA
YK (SEQ ID NO: 1454)
>orf02194 
LLDLVKIEFLKQRHQKLNFVVYGVVSLYLALICYYVNDARGLFDSFPFVYKFSLSYLNFL
ILPLYCVSYTIQAFGLEYRYRIMNNLKLASANLMKTFWAKILYIEINALCIMLFTYISVS
LFALLSRFSSTVSLSLLLRFFYLCMSSGILIPMGVFPLVALVMIKVRGKEIVGNLVGVMY
VLVSFFLARTSPNISPVTSANSLIWEGNREGVVLQQPAILSIVVLGLSLLVLSFLSIKSW
LRKVDE (SEQ ID NO: 1455)
>orf02195 
MRNPVIQTFNLSKSYDGKIVLDRIDFTLRQGEIYGLLGRNGTGKTTFIKAILGLTAMDSG
EVNILSEKLLGEFSKDLLSQIGVVLDSASFYPNLTGPENLSIFARLRGISLKQVEQALQV
VGLDGENKKLFKQYSLGMKQRLAIANAIMHQPKILILDEPTNGLDPIGILEMRRYLKELS
TNHGISILISSHIISELEKLVDRVGILHDAHLMAEKTMKELIDGADKRKIHLIVSDAPQA
KEVLCRINLQEQISILSDIELELQGESPTFDIAVVSNSLKDNGIVLKEYSYKNNESLEDY
FKRITGGEGIA (SEQ ID NO: 1456)
>or02196 
MTILSDKLKAKRKEKGFSQKTLSEGICEQSQISKIERGNYMPAADLLYKLANRLQVPLDY
FFDEQIEMTSNITPFKKLAEKLLEDRNYEDLEYLLNLEKEKSQYLSTEDEFYLLWIQSII
LFYLHSSKDEAIASLENALPKLSVSSSVYLKLLNTLSNFYFSVGRDAEYEENNSLLISLY
QEKDLNHQEYLFGYIRVKHNFAYYLHSKGKELEAVQEALETIDFCKQKETSYQLAPLLTI
VANAGKDFLKHDEILDYYLQARDICKIYEHKLMMAKIDHFLKDKDR
(SEQ ID NO: 1457)
>orf02197 
MNDLLLIPVIFLAVGGILILLWRLFLIASGLFLIGFVSFLIFVEVYGIYLLFTETELYTA
DLAQNGLFGFTTFFIIFNLVLLALACWAGYKWKRGY (SEQ ID NO: 1458)
>or02199 MEDTYYQLEEALVQGFQTPEEYQAYKELKEHYEEVTGDYSFSKRELTSQLEIALQNHRGV DFEEYEKKDYLELVQKLEEFDSSLATHYRQLID (SEQ ID NO: 1459)
>orf02200 
MVRRWVLSLQRNGRIIRQGNENKEVDIYHYITKGSFDNYLWATQENKLRYIKQIMTSKEP
IRAAEDIDEQTMTASDFKALATGNPYLKYKMELENDLTLLENQRRAFQRSKDHYRHTISY
CEENMPILEKRLSKYEGDIQQSEISKDQAFSMRVGKQSFDQRAEAGESLHRLIRHNQADS
KEFRTLASYRGFDIKMLSLPTNQPLPETFSVKIVGENQYSVSLDLYSPLGTIQRLQHTID
HIKEDQVKTQNLLDELKDKWNTAKVEIEKNFPKEEDYQTKKAEYDVLAPLIETETDLDTI
DQALRQFHEKGKEKQEQLSFELD (SEQ ID NO: 1460)
>orf02202 
MRNPQNVLNNLTKHSKDKNYQFERLYRLLYNKEMYLVAYQTIYANPGHLTPGVDELTIDG
MSIARIDQLIDSLKDESYQPHPSRRTYIPKKNGKLRPLGIPSFDDKLLQQVIKMILEAIY
EGQFEPSSHGFRPNKSCHTALTQIQKTYTGTKWFIEGDIKSFFDNINHDVMIHILRERIT
DERFLRLIRKFLNAGYVEDWKFYKTYSGTPQGGIISPILANIYLDKFDKYMTDYVKNFCQ
GKYRKRTPEYRQNEIALGKARRALECVSTENQRQEVIQRIRQLEKERVLIPHSDPMDSSF
KRLTYTRYADDFICGVIGSKEDAHRIKADIKDYLEAVLKLELSVEKTLITNARDKAKFLG
YHLYIRQSNLAKRDSAGRLVRNYTGRLVLEVSIETIRDRLLSYGAMKMTYHRGYEVWKPT
ARYFMKDCDDLEILERYNAEIRGFYNYYCIANNSSILHRFKYIMEYSMYKTYATKYRTTK
SHIIRKYKKDGQFSVQYIGRKGDTMTRYLYNGGFKRQKKSFLENDNLPNTAKYFSRTNLI
DRLKASRCEYCQATDSSLEIHHVRKLKDLKGKTFWERLMISRQRKTIALCKDCHKKLHHG
KLD (SEQ ID NO: 1461)
>orf02208 
MEVMKLLAMFRGTIPKDREKMDLFLRYQAQHFDEKWQDLVESFLAEEGKIEEIPHVYSFH
QDIISFLEASSENNDQDLESYTRNFGQAGLSKLSQLSNFEKNLVLEVATYNLSTRFYIQS
EKEKLEPLSELVCLQNQDVNLVNVYRVANNLSDRISRDIEEFLLMVDSKELTKEVLEIHF
EEKEGDVLAYLGSELMATLDTVTDLVHHEENYTQLPLTQKLKIITHFDDVKARSEKSNQV
EEVLSPSSDIEQETEETNSFSNVDKIVEEALREYPIGSQVSYKGQVFQLVSIENAQLNDL
IRLELFNDSNQLFEENPILYLNSLEEIEQVLSLVELEKEDSEIEIDSSSESQEIDLFSYL
EEEKENEKDKETETLIVGIEETDVPVQDFVFPDDLEDFYPKTNREKIETNIAAIELVKRL
EKEGRQANPEEQELLAKYVGWGGLANEFFDELNPKYETERLTLKSLVSKSEYSTMKQSSL
TAYYTDPMIIRQIWQKLLDDGFEGGRILDPSMGTGNFFAAMPRSIRDKSELYGVELDSVT
GAIAKKLHPNTHIEVRGFEEVPYQNNSFDLVLTNVPFGNFRIADKNYDKPYMIHDYFVKH
SLDLVRDGGQVSIISSIGTMDKRTDNVLQEIKTNTHFLGGVRLPDTAFKSIAGTRVTTDI
LFFQKDQAKNLNEEELVFSGSIPFEEDKRVWINPYFDGKYNTQVLGEYEVRNFNGGTLNV
KGVSETLSTDIMKALENVEAPKQIDNFLKAPVFIQEEVDNSLPSRIREDLALYSFGYERN
QIYYRDTHGIRKSSKVDEISYYVDEKGDFKAWDSSLSEHKIDRFVQLHLTDEEALDVYKS
EEASKRGKYKGLFKKTVFYESPLSDKDISRIKGMVDLRETYQSLIEIQRHQDYSRTDFQV
LLSKLNHNYDRFVSQFGYLNASVNRNLFDSDDKYSLLASLEDEYIDSKDQKVKYKKSLAF
EKALVRPERVIARVSTALDALNSSLSDGRGVDLDYMVSIYPEHSQAAILDELGDQILIDP
ESYLRGERKYLSKNQFLSGDILNKIEVVQLLVEENNQEYDWNHALDLLESVRPPRIHLAD
IEFKIGSRWIPQSVYGKFAFECFTNREFELSSPDVEQVIEANPVDGQVHLRTSFAYRYPS
AKDSSLGVSGSRYDTGRKIFENLLNSNQPTITMTVTEGEKKKTITDLEKTSVLRAKEQHL
QELFQDFVSRYPEVQQVIEESYNRLYNRTVSREYDGSHLVIDGLAQNISLRPHQENAIQR
IVEEKRALLAHEVGSGKTLTMLGAGFKLKELGMVHKPLYVVPSSLSAQFGQEIMKFFPTK
KVFVTTKKDFVKARRKQFVSRIITGDYDAIVIGDSQFEKIPVSKERQMNYIEDKLNELRE
IKTHSENKYTVKEAEQSISGLEKQLEELQRFNRDSFIDFENLGIDFLFVDEAHHFKNIRP
ITGLGNVAGITNTTSKKNVDMEMKVRQIQEEHDFKNIVFATGTPVSNSISELYTMMNYIQ
PDILKRYQVDYFDSWVGAFGEIQNSMELAPTGDKYQPKKRFKKFVNLPELMKIYKETADI
QTQDMLDLPVPEAHIIPIESELTENQKLYLEELVMRSDMVKCGTVDPSQDNMLKITGEAR
KLAIDMRLLDSSYSLADNHKLLQVVDNVERIYREGMENKATQMIFSDIGTPKKKDNGFDV
YSEIKALLVDRGIPSKEIAFVHDANSDEKKNSLSRKVNAGEVRILLASTEKGGTGLNVQS
KMKAVHHLDVPWRPSDIQRASVKAV (SEQ ID NO: 1462)
>orf02209 
MEGIYQRDSDQDGLTDAQELALGTNPLSADSDGDGRSDLVEVEEGTNPLEKDLQDIDQTS
ITEPSSVFMEMKQKISDMMESHYKEFIQALISIETGIENEQDLEDLYTYYMRTDSISLLS
SDLETSPQKVEMEIEL (SEQ ID NO: 1463)
>orf02210 
VADTRTKSDSNLGRKGQAVADTRINRLTRWLTDSVNHLFCEENMANSRDYRNPNYTEKIK
LQRFFTQLQIAASFFKEHFVGKIMYYETEIESVELHFSPTNFMHLCGVDYRKGAGSFFDD
 
CLNRHVIIDELKIKKDGTTMQKLQVLGSIEELLGKHVHLTGSGRYLYLEFDYALRTRKQI
LALTLKETSRKIVPQSLLDLKRKTVFPKGQKVISIYSKHLQTSELFYYLKD
(SEQ ID NO: 1464)
>orf02217 
MKDKREIIRARKAFRRSLKDEKKFLKKGKKEVKKQKKDSAVLDEKAWKKEIKKKLEEMRE
ASKARVKQANEDYNHILQNSPPSLLNRKELRDRRLPHARKRLKIAKKQYREAKVEAKEER
KESRKERKTNQKFLYGQESKAKSNFFFQGKSLEELKVKKEVKTAKENLKSTKQAYKSKKV
SRKAKTFLYVLGREGGELASENEDLDGYRTLQETIRKGKRYSRLSYNLGKASVKTGQATC
RFTKKRLTNTKERYHHFKDGKGWKLAKDKPSSFKNRFRKLKKQGLTSVRNIYQKLKAAFS
FFTFAAGNPVTWIVGGIVFLLLLIMSFFLGFSSASLIQQDEFELTKAYTHLTWEDAEHTR
TNDKGITYYTKVDDVMGYMNFKFHDYELHKPVHLFSSETYKDYLSTLWHDLNDGEDLKSM
QDLYETPKYKLSKDDQEEMKELKEEGIYASMQELDNPFEGKSNEDSLTMTYRYGYYDLDG
KPTLQEYILLEAKAHQTIVAPMDGVVSLDGDNVILTNGKGENESRLTLYSIHNGRAIEGT
RVLTGDIIGETPDDTGLKVSYQKYKNKKEKLVYVNPQFYFPKVIQLQTTILPAIGQFGGD
EFERAKHIYEFLKSQGASPQAIAAILGNWSVESSINPKRAEGDYLSPPVGATDSSWDNET
WLAIGGPAIYSGAYPNILHRGLGLGQWTDTADGSTRHTALLNYAHSKNKKWYDLDLQLDF
MLHGDSPYYQSWLKDFFGNTGSAANLAQLFLTYWEGNSGDKLLERQTRATEWYYQIEKGF
SQTNGGQAKSDPQSLEGVRGDLYDHSVPGGGDGMAYAYGQCTWGVAARMNQLGLKLKGRN
GEKISIINTMGNGQDWVATASSLGGETGSTPKAGAIVSFVGGTHGTPAIYGHVAFVEKVY
DDGSFLVSETNYGGNPNYTFRKISQADSAISFAYTTK
(SEQ ID NO: 1465)
>orf02219 
MTYKKEEVKGKKEEVLPSTANTISYQALYQNGLMQVKEDYFSQSYLLGDVNYQTVGLEDK
GAIIEKYSDLINSLDDQTNFQLTIFNKRLNLEKFRQSVLYEEKEDGYDTYRKELNRMMNQ
NLDSGENNFSAVKLISFGRKDSNPKQAYRSLSQIGEYFKSGFSEIDARFESLAGEERVNL
LADMLRGEHHLPFSYCDLTRSGQTTRHFIAPNLLDFKNKNYLQINDRLLQIVYVRDYGME
LGDQFIRDLMQGDLELIVSLHAQSSTKADAMKKLRTKKTLMESQKIGEQQKLARTGIYLE
KVGHVLESNIDEAEELLKTMTETGDKLFQTVFLIGVFGQDEEELKQALDTIQQVAGSNDL
MIDKLPYMQEAAFNCLLPFGCDFLEGVSRSLLTSNIAVNSPWTSVDLQDRSGKYYGINQI
SSNIITIDRSLLNTPSGLILGTSGAGKGMATKHEIITTKIKESGENTEIIIVDPEAEYSV
IGRAFGGEMIDIAPDSQTYLNVLDLSEENMDEDPVKVKSEFLLSFIGKLLDRKMDGREKS
IIDRVTRLTYQSFKEPSLEEWVFVLSQQPEEEAQNLALDMELYVEGSLDIFSHKTNIQTG
SNFLIYNVKKLGDELKQIALMVVFDQIWNRVVRNQKLGKKTWIYFDEMQLLLLDKYASDF
FFKLWSRVRKYGASPTGITQNVETLLLDPNGRRIIANSEFMILLKQAKNDREELVQLLGL
SKELEKYLVNPEKGAGLIKAGSVVVPFKNKIPKGTQLFDIMSTDPDKMASN
(SEQ ID NO: 1466)
>orf02220 
MNTRVFKDISKYQHRAWLGFTTRQIIFVLPAFIVTIIVLGLNLFFWQFGDWFVYGFVFAF
TIPLMLFGVYKPNDLYFEHYLKYRLHFELTVPLRTITGKKGHEHEKKIKYIKETKSFNDL
(SEO ID NO: 1467)
>orf02221 
MNLSLVSPFVYLASEKISAENLFEGFSVDLQSTVDLIKSLSSYNPTVWTYMSSITKSVMQ
PLGVAILSVVLILEFSKMAKKIANSGGAMTFEALAPMLISYIMVAVVITNTTVIVEAIIG
IASHAIEQVASIVAHGGAKYDTISGLKGSGFIGRMIVGFFALLIWLVRIVSAAMVNLLVS
IRFIQLYLMIPFAPLTIPTFLSDEWKSIGIGYLKNIMVYAVQGVLIFLIVSLVPLFESAG
KIAVSNGAGVLQSLAIMFGSLVQAILLIIALVGSQRTARSILGM
(SEQ ID NO: 1468)
>orf02223 
MITHFKGFVYGVDASAMFAQAMSLLQKGLIAVGAFLVVVGIVNLATNIKDGGPGVRNAIL
EIVGGVMVGAAGAFVTQISI (SEQ ID NO: 1469)
>of02224 
MMYSGKKFLLFSLLGILLGYLFHRLTLLYDSYTGNSLDKWTHLLMEGQDEVLQSPWNVSF
TGKSSAFFLLGFVMMLLVYLYLETGKKQYREGIEYGSAHFGTLKEKKLFYGKEFSHDTIL
AQDVRLTLLDKKPPQYDRNKNIAVIGGSGSGKTFRFVKPNLIQMNSSNIVVDPKDHLAEK
TGKLFLEHGYQVKVLDLVNMKNSDGFNPFRYIETENDLNRMLTVYFNNTKGSGSRSDPFW
DEASMTLVRALASYLVDFYNPPKTREQLIEESRLSQKEHQNLLKRQKKEVEERKKRGRYP
SFAEISKLIKHLSKGENQEKSVLEILFENYAKKYGTENFTMRNWADFQNYKDKTLDSVIA
 
VTTAKFALFNIQSVMDLTKRDTLDMKTWGKEKSMVYLVIPDNDSTFRFLSALFFSTVFQT
LTRQADIDFKGQLPLHVRVYLDEFANIGEIPDFAEQTSTVRSRNMSLVPILQNIAQLQGL
YKEKEAWKTILGNCDSLVYLGGNDEDTFKFMSGLLGKQTIDVRNTSRSFGQTGSGSLSHQ
KIARDLMTPDEVGNMKRHECLVRIANMPVFKSKKYNSTKHPNWKYLANQETDERWWDYQI
NPLNQSQENHLEGLRIRDLTFESSLK (SEQ ID NO: 1470)
>orf02225 
MSSEQQERMAVQYAERSLLFTVKSLLKILEWSRRQALAQDSAYKIGVQKLEELLQSPYSI
DTINLKKDFLDKPIDIEKFKAFLEKEEIPLAIAWQGDSLHFYTKDRSILDNHLDQLLEKM
VNDPEKLADFTMDKSLDDAIDEAKSQITFRQEGAVKQKEMVR
(SEQ ID NO: 1471)
>orf02226 
MKVVNLYDLKQMGNKGGCTIQLIHHFPFGMLGHLKKDYIEFKRVGIVDGKAVEVTLREP
YSRDLLQVVKSIKQRQKLIAYRYKEGKLLFVKEEASDVL (SEQ ID NO: 1472)
>orf02227 
MFSNANSFKAKIKNISKDKGIPAQQVQQHYLIEQVLKLISTSSYRDSFIVKGGYLIGQMI
GLDKRTTMDLDVTLKGTEMSRENLIHIFEEILCSKTDGFSFSVDKLEPIRQDDEYGGFSL
KLNATFDTLKEVVFIDITTGDKITPREITYSMTSIFTNESIKIWTYNLETVLAEKLETII
SRGLASTRPRDRYDLFTLYKLRKEEINLEVLKNALENTAEKRKSKDTIYNWEEQVRGIEI
SDYQKELWIRYQRQFKYAKDISFDNSVQVIREIMQQIF (SEQ ID NO: 1473)
>orf02228

MVDKREKLMNSFNQYGFLTFKQVIDENLHYKTLLKMVAEGKIDAEEKGLYRLPDIYLDEW
FVLQYRFPKGIFSLETALWLHGLSLTIPFNMTMSFPYGTNTKNIKEADICPIILRSHYSE
GIIEIERLPGQFIKVYEVERVLVECLRPVHQVDLQIIAPAFKKYFQQNKIHLHKLFYYAQ
LFKVTDKLQSYTEVLS (SEQ ID NO: 1474)

>orf02229
   MRCLFFYPILKGSELMKTKNQESKGRSPLFKTIKHSFSQ (SEQ ID NO: 1475)
>orf02230 
MELKFVIPNMEKTFGNLEFAGEDKVVQRRINGRLTVLSRSYNLYSDVQRADDIVVVLPAE
AGEKHFGFEERVKLVNPRITAEGYKIGTRGFTNYLLHADDMIKE (SEQ ID NO: 1476)
>orf02231 
MRLANGIVLDKDTTFGELKFSALRREVRIQNEDGSVSDEIKERTYDLKSKGQGRMIQVSI
PASVPLKEFDYNARVELINPIADTVATATYQGADVDWYIKADDIVLTKDSSSFKAQPQAK
KEPTQDK (SEQ ID NO: 1477)
>orf02233 
MKQRGKRIRPSGKDLVFHFTIASLLPVFLLVVGLFHVKTIQQINWQDFNLSQADKIDIPY
LIISFSVAILICLLVAFVFKRVRYDTVKQLYHRQKLAKMILENKWYESEQVKTEGFFKDS
AGRTKEKITYFPKMYYRLKNGLIQIRVEITLGKYQDQLLHLEKKLESGLYCELTDKELKD
SYVEYTLLYDTIASRISIDEVEAKDGKLRLMKNVWWEYDKLPHMLIAGGTGGGKTYFILT
LIEALLHTDSKLYILDPKNADLADLGSVMANVYYRKEDLLSCIETFYEEMMKRSEEMKQM
KNYKTGKNYAYLGLPAHFLIFDEYVAFMEMLGTKENTAVMNKLKQIVMLGRQAGFFLILA
CQRPDAKYLGDGIRDQFNFRVALGRMSEMGYGMMFGSDVQKDFFLKRIKGRGYVDVGTSV
ISEFYTPLVPKGYDFLEEIKKLSNSRQSTQATCEAEVAGVD (SEQ ID NO: 1478)
>orf02234 
LAYGLSQNRLAVATGITRQYLSDIETGKVKPSEDLQQSLWEALERFNPDAPLEMLFDYVR
IRFPTTDVQQVVENILQLKLSYFLHEDYGFYSYSEHYALGDIFVLCSHELDKGVLVELKG
RGCRQFESYLLAQQRSWYEFFMDVLVAGGVMKRLDLAINDKTGILNIPVLTEKCQQEECI
SVFRSFKSYRSGELVRKEEKECMGNTLYIGSLQSEVYFCIYEKDYEQYKKNDIPIEDAEV
KNRFEIRLKNERAYYAVRDLLVYDNPEHTAFKIINRYIRFVDKDDSKPRSDWKLNEEWAW
FIGNNRERLKLTTKPEPYSFQRTLNWLSHQVAPTLKVAIKLDEINQTQVVKDILDHAKLT
DRHKQILKQQSVKEQDVITTKK (SEQ ID NO: 1479)
>or02235 
MNFGQNLYNWFLSNAQSLVLLAIVVIGLYLGFKREFSKLIGFLIIAIIAVGLVFNAAGVK
DILLELFNRIIGA (SEQ ID NO: 1480)
>orf02236
   MNGVFLIFIIQADFLFDFLKVNGKGKPRTDQFALIVFA (SEQ ID NO: 1481)
>orf02237
   MYDVARYYIEETGALGEVPASLQNYIDYQAYGRDLDLSGTFISTNHGIFEIVY (SEQ ID NO: 1482)
>orf02239 
MYLIGYAIKFTPNCCNGFLWLVSAKRYFFSCIGQLWSQCISNDRLQKSIRLFTIKSFCRH
KPCESHRNPKVFEKCSLYHGKRGQVAKYHHCKE (SEQ ID NO: 1483)
>orf02242 
MAYPIKYIENNLVWNKDGECYAYYELVPYNYSFLSPEQKIQVHDSFRQLIAQNRDGKIHA
LQISTESSIRSAQERSKNEVTGKLKAVAYDKIDQQTDALISMIGENQVNYRFFIGFKLLL
NDQEFSMKSLTVEAKNALSDFVYDVNHKLMGDFVSMSNDEILRFQKMEKLLENKISRRFK
IRRLDKDDFGYLIEHLYGQTGTAYEEYEYHLSKKKLDNETLIKYYDLIKPTRCLVEEKQR
YLKIQQEDETVYVAYFTINSIVGELDFPSSEIFYYQQQFTFPIDTSMNVEIVANRKALS
TVRNKKKELKDLDNHAWQSDNETSSNVAEALESVNELETNLDQSKESMYKLSYVVRVSAN
DLDELKRRCNEVKDFYDDLSVKLVRPFGDMLGLHEEFLPASKRYMNDYIQYVTSDFLAGL
GFGATQMLGENEGIYVGYSLDTGRNVYLKPALASQGVKGSVTNALASAFVGSLGGGKSFA
NNLIVYYAVLYGAQAVIVDPKAERGRWKETLPEISHEINIVTLTSDEKNKGLLDPYVIMK
NPKDSESLAIDILTFLTGISSRDGERFPILRKAIRAVTNSEVRGLMKVIEELRVENTPLS
TSIADHIESFTDYDFAHLLFSNGYVEQSISLEKQLNIIQVADLVLPDKETSFEEYTTMEL
LSVAMLIVISTFALDFIHTDRSIFKIVDLDEAWSFLQVAQGKTLSMKLVRAGRAMNAGVY
FVTQNTDDLLDEKLKNNLGLKFAFRSTDLNEIKKTLAFFGVDPEDENNQKRLRDLENGQC
LISDLYGRVGVIQFHPVFEELLHAFDTRPPVRKEV (SEQ ID NO: 1484)
>orf02244 
VKPSIVNRIKSNWTLKRLGKVAMTVAFTLVIAIFLLAMLGTVVQAAGLVDDTVNVANEYS
RYPLENYQLDFYVDNSWGWLPWNWSDGIGKQVMYGLYAITNFIWTISLYVSNATGYLVQE
AYSLDFISATADSIGKNMQTLAGVSANGFSTEGFYVGFLLLLILVLGVYVAYTGLIKRET
TKAIHAIMNFVLVFILSASFIAYAPDYIKKINDFSSDISNASLSLGTKIVMPHSDSQGKD
SVDLIRDSLFSIQVQQPWLLLQYNSSDIESIGIDRVESLLSTSPDSNNGEDREKIVAEEI
EDRSNTNLTITKTINRLGTVFFLFVFNIGISIFVFLLTGIMIFSQVLFIIYAMFLPVSFI
LSMIPSFDGMSKRAITKLFNTILTRAGITLIITTAFSISTMLYTLSAGYPFFLIAFLQIV
TFAGIYFKLGDLMSMFSLQSNDSQSVGSRVMRKPRMLMHAHMHRLQRKLGRSMTTLGAGS
AIVTGKKGQSGSGSSARTQADHSRPDGKEKSTLGKRIGQTIGTVADTKDRMVDTASGLKE
QVKDLPTNARYAVYQGKSKVKENVRDLTSSISQTKADRASGRKEQQEQRRKTIAKRRSEM
KQVKQKKQPASSVHERPTTRQEQYHDEQTSKQSNIQTSYKESQQAKQERPAVKSDFSSPK
VERQGNTVQEKTVQKPATSTTTADRTSQRPITKERPSTVQRVPLQNTRTTNQNRHH
(SEQ ID NO: 1485)
>orf02246 
MKLKTLVIGGSGLFLMVFSLLLFVAILFSDEQDSGISNIHYGGVNVSAEVLAHKPMVEKY
AKEYGVEEYVNILLAIIQVESGGTAEDVMQSSESLGLPPNSLSTEESIKQGVKYFSELLA
SSERLSVDLESVIQSYNYGGGFLGYVANRGNKYTFELAQSFSKEYSGGEKVSYPNPIAIP
INGGWRYNYGNMFYVQLVTQYLVTTEFDDDTVQAIMDEALKYEGWRYVYGGASPTTSFDC
SGLTQWTYGKAGINLPRTAQQQYDVTQHIPLSEAQAGDLVFFHSTYNAGSYITHVGIYLG
NNRMFHAGDPIGYADLTSPYWQQHLVGAGRIKQ (SEQ ID NO: 1486)
>orf02247 
MMKFRKNQNKEKQIPKEKKPRVYKVNPHKKVVIALWVLLGLSFSFAIFKHFTAIDTHTIH
ETTIIEKEYVDTHHVENFVENFAKVYYSWEQSDKSIDNRMESLKGYLTDELQALNVDTVR
KDIPVSSSVRGFQIWTVEPTGDNEFNVTYSVDQLITEGENTKTVHSAYIVSVYVDGSGNM
VLVKNPTITNIPKKSSYKPKAIESEGTVDSITTNEINEFLTTFFKLYPTATASELSYYVN
DGILKPIGKEYIFQELVNPIHNRKDNQVTVSLTVEYIDQQTKATQVSQFDLVLEKNGSNW
KIIE (SEQ ID NO: 1487)
>orf02249 
MKIINIGVLAHVDAGKTTLTESLLYNSGAITELGSVDKGTTRTDNTLLERQRGITIQTGI
TSFQWENTKVNIIDTPGHMDFLAEVYRSLSVLDGAILLISAKDGVQAQTRILFHALRKMG
IPTIFFINKIDQNGIDLSTVYQDIKEKLSAEIVIKQKVELYPNMCVTNFTESEQWDTVIE
 
GNDDLLEKYMSGKSLEALELEQEESIRFQNCSLFPLYHGSAKSNIGIDNLIEVITNKFYS
STHRGQSELCGKVFKIEYSEKRQRLAYIRLYSGVLHLRDSVRISEKEKIKITEMYTSING
ELCKIDKAYSGEIVILQNEFLKLNSVLGDTKLLPQRERIENPLPLLQTTVEPSKPQQREM
LLDALLEISDSDPLLRYYVDSATHEIILSFLGKVQMEVTCALLQEKYHVEIEIKEPTVIY
MERPLKKAEYTIHIEVPPNPFWASIGLSVAQLPLGSGVQYESSVSLGYLNQSFQNAVMEG
IRYGCEQGLYGWNVTDCKICFKYGLYYSPVSTPADFRMLAPIVLEQVLKKAGTELLEPYL
SFKIYAPQEYLSRAYNDAPKYCANIVDTQLKNNEVILSGEIPARCIQEYRSDLTFFTNGR
SVCLTELKGYHVTTGEPVCQPRRPNSRIDKVRYMFNKIT (SEQ ID NO: 1488)
>orf02250 
MKPSSFQTTIENQFDYICKRAMEDERKNYMLYLSRIAKREVSFSDVGDYLVSQFATTDNY
STDFQIFTLNGLSVGVENDLLSEALRELPDKKREILLLFYFMDMSDSEIADLLKLNRSTV
YRHRTSGLALIKKFMEEFEE (SEQ ID NO: 1489)
>or02251
   LATLDCVQCIYNFFKLFSFNLNTIAIHNQPICIFIFLCQAS (SEQ ID NO: 1490)
>orf02252 
MSEKRRDNKGRILKTGESQRKDGRYLYKYIDSFGEPQFVYSWKLVATDRVPAGKRDCISL
REKIAELQKDIHDGIDVVGKKMTLCQLYAKQNAQRPKVRKNTETGRKYLMDILKKDKLGV
RSIDSIKPSDAKEWAIRMSENGYAYQTINNYKRSLKASFYIAIQDDCVRKNPFDFQLKAV
LDDDTVPKTVLTEEQEEKLLAFAKADKTYSKNYDEILILLKTGLRISEFGGLTLPDLDFE
NRLVNIDHQLLRDTEIGYYIETPKTKSGERQVPMVEEAYQAFKRVLANRKNDKRVEIDGY
SDFLFLNRKNYPKVQVITTA (SEQ ID NO: 1491)
>orf02253
   MGHANIAMTLNYYAHATFDSAMAEMKRLNKEKQQERLVA (SEQ ID NO: 1492)
>orf02254 
MKRIIPVYIFQQVNVLLVSLYLLKLLCISELTILQILYCASLISFLWMYGQRKQVVKVNM
KTRMKWLGIGFVSLLIINLCFSLIHAQGTTNQANLIGLQHQVPWFSFLLLLINASMVEEF
LYREILWNLVRKLDIRVALTSILFVLAHHPGTILAWCLYVSLGMFLGLVRYKSDLWGSMG
LHLVWNLSVYVLFFL (SEQ ID NO: 1493)
>orf02259 
MKRITANQYQTSERYYKLPKILFEDEKYMDMKLEVKVAYSILKDRLELSLSRGWIDEEGA
VYLVFSNSKLMKLLGCSKSKLLSIKKILKEYDLIDEVQQSSSEKGRLANKIYLGELSSTP
VASSNRPSVKKKIGQVENETAPVSHSAPSETEVSETKYSETDSLFSEDEEERYTQPILKR
KVEKVTKYDQDYIWGLVQDQFRREGFSETASEIAMTDFERIYQYALDNVRFVRRAEVLAE
FVFNGLYSVWNNRVRKGGG (SEQ ID NO: 1494)
>orf02260
   MTKELQSSRYIVISFLVREMGIDIVEAISLMAELEKSGLVRLESSGDLILKELGGAL (SEQ ID NO: 1495)
>orf02261
   MIVILLSFFLQKIKKGEQYSTVLQNIFIKKKNPAKLIFGRVFGRKLN (SEQ ID NO: 1496)
>orf02276 
LQVWYNLQSDFEQEITLIMWNPFANLVFNQPLISFFADLNLKILGYSYTDRVKTWPDIGT
GCRYNNLHLILLAP (SEQ ID NO: 1497)
>orf02314
   MVICHNDYLLWLPEFSQPLTSLSQTTFFNLNIIRMMRNIDSDFHRRISFSLLVFFC (SEQ ID NO: 1498)
>orf02318
   LIEGHLVFADKPAQALVLLRKVGSPKKVSFLTLHLYFLILKIDILKITGF (SEQ ID NO: 1499)
>orf02324 MFLHHLLQIKGGLGIQTSQGFVQNPNIRSRQECPNDKDFLTHSVRKSFNNLIAVFSKIKN VQ (SEQ ID NO: 1500)
>orf02326 
MTNQAHNLSIFNLQIEITQGLFITIQLTNILKFNHGTTPIFICIIHRYTSTLIQKNQYFI
LSS (SEQ ID NO: 1501)
>orf02348
   MAVTKSQVFSRQGFDFSILGQDLTRLQDVSNLATIGTRIHKDSTANASWNTTSKLKAS (SEQ ID NO: 1502)
>orf02349 
MTEGNASCFNQVSPSFCFNGIAINRNVIELVTQDDKSTNPTITNDDIACIAKNHPRDIFL
VGKFHNASQLKTISWKDQIISLSTYFCITIAMQGFLKTDINSF (SEQ ID NO: 1503)
>orf02361
   MRLRDLRRVDFPDPDGPIKAVISLGWKDRETLFKAFFLL (SEQ ID NO: 1504)
>orf02364
   LKNHSNVFTHFINVDFWTVDINSTIENLPSYFSNINGIIHAIETA (SEQ ID NO: 1505)
>orf02365
   LHINPLNGFIFTIVNMDILSRKGYFFFRKGKDMLLIPVIC (SEQ ID NO: 1506)
>orf02387 
MTIHIQVVKTNMVILADRFFQGLILRSTDKFFIKIRLVRSHNLRFNNMDFSTVAVHENKG
RHHVDELLLRFIINSKATVAKKSIVAQGFRFDGNFFRKTRQTNHLNIIFYDNPDQIIFFQ
NGLITNSQFNRLHP (SEQ ID NO: 1507)
>orf02404 
MVKRRIRRGTREPEKVVVPEQSSIPSYPVSVTSNQGTDVAVEPAKAVAPTTGWKQENGMW
YFYNTDGSMATGWVQVNGSWYYLNSNGSMKVNQWFQVGGKWYYVNTSGELAVNTSIDGYR
VNDNGEWVR (SEQ ID NO: 1508)
>orf02420
   MRFIVGRFTSFSLGIEFSPTSKLDDLLFKIAFLMILATNIKARKTKEAT (SEQ ID NO: 1509)
>orf02424
   MANDNKSHYLIYRVLGISFEEGENIDLYQNKGRFLYKYAGSFLEEAAVLSFNEKFGTENT (SEQ ID NO: 1510)
>orf02433
   LHYRTTPTLIMVVQRDCLILSFPRQKGPIVGQMAIIL (SEQ ID NO: 1511)
>orf02435 
VFALLDNSTFLRKSLHLRKMEMFPVETEEITYKRKKSKGKRQAILAQFDSEEVHHQVEES
ICPDCQDDLKEIGASLQRQELVFIPAQLKRVDHIQHAYKCQTCSKNNPSDKIVKAPIPKA
PL (SEQ ID NO: 1512)
>orf02451 
LVCQTIKYWHKFHLHIGRCKLLIGLIPVLNFFIRADIDCLLVLLSLIDRQNGKQFNLCQW
IIASNGLNDSFEIIESLIHRNILSDIICPNQKKNFIYCSTI (SEQ ID NO: 1513)
>orf02459 
VGHNSGSFFLFLLLRLLLSPLLRNSISFLTSQGIPWKLSNNKTKPIDKPTASKSIATNPL
LLHLR (SEQ ID NO: 1514)
>orf02480 
LDFLNHLWVAHAGNSSSCTNISRNSFQGHHSCRTSSFCDTSLFRILHIHNNTTLEHLCQV
FI (SEQ ID NO: 1515)
>orf02505
   MLLLISLTQLIIFLFFERFNLLLIKTFLLVDLKSNKSA (SEQ ID NO: 1516)
>orf02517
   MGFSMKLIHDLNTHTTHSTAKMLHNVKAIKNDFSIRE (SEQ ID NO: 1517)
>orf02524 
MQEHYTPKGKHLTIDNRRLIERWKNENKSNREIAGLLGKAPQTIHTEVKRGTTLQQVRKG
LYKKVYSADYAQTVYQFNRKRSVKKLILTKEIREKILHYHKQKFSPEMMVNKKQVKVGIS
TIYYWFHNGHLGLTKADMLYPRKRKGVKKQASPNFKPAGKSIEERPDVINLRLENGHYEI
DTVLLTKIKNYCLLVLTDRRSRHQIIRLIPNKTAESVNQALTLLLGEHRILSITADNGSE
FKRLSEVFPEEHIYYAHAYSSWERGSNENHNRLIRRWLPKGTKKTTPKEVAFIENWINNY
PKKCLDYKSPSEFLLGG (SEQ ID NO: 1518)
>orf02527
   VVPRYVTKHQGWDHNSHTITNSDDDPATLVTFRTFKFNVGNCTIPKNDQNGSSQKFSGIL (SEQ ID NO: 1519)
>orf02534
   MAYSTDFKQRALDYIKEGHSHVEAAKFFGVGVRTLFTWEKKDVNKDT (SEQ ID NO: 1520)
>orf02585
   MAVQANWSFDITHDSSFFFSNQKRGLNFSQMCFKDRRRNGFFDRKIFKFKFNNPIQIF (SEQ ID NO: 1521)
>orf02595
   MEIVLVSFSISFQHFIIAYCLDFSSAGFRNSQNFSNFC (SEQ ID NO: 1522)
>orf02608 
MKLKLLRVDTKVIMGSFLLVLSSLLALLLPLILKGLIDGSSIENIGSKVFQSFLIFIGQA
LFSSIGYYLFSQSGEKKIAKIRKKVIEGLIYAEKSFFDKSQSGELTSAIVNDMSVIREFL
ITTFPNIILSLVMVLGSIVVLFSLDWNLSLLLFITLPCMMFIILPLSNISEKYSRRLQEE
IGFLTGQLTEKIQEHELIKTNQAEKSVQDVLDNCIERVQNNSLKSDRVTSFETPFALLFI
FATIVVMLTYGGYRVSAGYISVGTLVSFLIYLFQLLNPISNIANFVTIYSSSKGSSVALE
NLLAVPKEKFEGGKSVSGQGLNFNHVYFGYDENRPVLKDITCSIFKGQKIAFVGPSGSGK
STIVRLLERFYKPLSGDILMEQSSIYDFNLKEWRSKIAWVSQNNAVLSGSIRDNLCLGLN
RLVTDDELMKVLDLVSLGDEIRSMKEGLDTEVGERGRLLSGGRSQRLQIARAYLKDAEIL
IFDEATANLDADSEYAIISSLYSVLKEKTVVIIAHRLSTVKDVDCIFFLEEGKITGSGTH
KELLENHERYARFVQEQMIE (SEQ ID NO: 1523)
>orf02621 
LIRYLDQYEDVILREIKAQFPDVAVDKLMEEYIKAGLILRENKRYYLNFPTLESLDSLEL
DQEIFVREASPVYQALLEQSFETELRNQINAAILVEKTDFARIKMTLSNYFYKVKQQYPL
TEKQQELYDILGDVNPEYALKYMTAFLLKFLKKDQLMQKCRDIFVDSLVVLGYIVQNEDG
KYELAIDFDKERLTFYLA (SEQ ID NO: 1524)
>orf02622
   MIGLKEVCRFLTDNTSLSTSMINHPIQINGNMAIVTCGSLDGLSHV (SEQ ID NO: 1525)
>orf02633
   MRERVRLSGSLFTSLKTREHIKSTMELFHKYVFFLIQEIKIKMINFLKIGDLPTL (SEQ ID NO: 1526)
>orf02643 
MIDHFEIKVKDLQISEGFYRSFLAPLDYKLAFKTSSLISFLSPNSPHPGGDFWLTQGTQD
PVHFAFLAENKEEVQACYEAGLEAGGRDNGAPGYRSEHPIYYAAFMIDLDGNNIEVVCHK
E (SEQ ID NO: 1527)
>orf02645
   VIVFLSRNKDGNAFCHLDLISIANPVWGWDDDFITWIDHSHKEGIERIFGSRSDCHLI (SEQ ID NO: 1528)
>orf02648
   LSNQFYFSLQTKPILKVKQFLLFQSQMIRVSEILQFSNKL (SEQ ID NO: 1529)
>orf02652 
MKHSHKKSFDWYSMQQRYSIRKYYFGAVSVLLGTALVLGAAASVQTVQAEENKQETTNSI
SVGRGEAATKPAEVSASNKEKTYAAPTVANPVETTPVKTGEVTKPAEKVEEAKDKKEEVT
HQDAIDKSKLLTALSRAKKLESKLYTEASAANLQTSIQAGQSLLGKADASEAELSAAESS
IQSSIIGLELRSNSNKGTVSETPVAKKANIVEAKEETKPAVTTTERSAVDSAILPISTAA
KVETTSAPASTNEILKPSLSLSDARQNPAIRKEDVDRGYSGFRTAGSGFRAAGSGFRAAG
PENKPILNPNNTIAFSDISQGLHSFRGIGHSRGGREIHYDVTTVRRGNRLNFTIKYSGPG
EFVNNNFILDKGDGFGNPSNATITSSNPRVREQSKSISQGANYVSHSGYSMTSATSTNTE
QTIRFSLPIINPNGDLSVRLKPVTFNVDQGGGAATSNDPYSNSNYYHRANPLLLDANPY
GGTNNKTVSEDIDFQTVYLPTSKLPEGQTRLVREGEKGQRQITYKVHRFGNETLLGLPIS
NRVTKEAKPRIMQIGVAKELIDTVKPRVDQNKVGDTNNLTFYLDNDGNGVYTEGVDELVQ
KIAIKDGAKGEKGDQGERGLTGAQGTKGEKGDQGERGLTGAQGAKGEKGEQGFQGRDGEQ
GPKGEDGKTPTVKVTDGQDGTHTITINDGKGGITTTVVRDGFDGASPLVSTHRNEADKTT
TVIFYYDLNDNNQFDEGDTKLKEVVIADGKQGPKGDKGDNGKDGFTPEVTVTDNNNGTHT
ITITQPDNRPSLTTIVKNGEDGKTPKVKAERDDAKKQTTLTFYIDKDGDGSYTAGKDELV
QTTVVKDGQDGAAGASGRDGKEVLNGKADPTTEGKDGDTFVNTQTGDVFVKKGNTWEPAG
NIKGPKGDKGADGAKGEKGDRGERGLTGAQGTKGEKGDQGERGLTGAQGTKGEKGDRGER
GLTGAQGQAGRDAVTPTVTVKDNKNDGTHTITINDGRGNVASTVVRDGFDGASPLVATQR
NEADKTTTVIFYYDQNGNNELDASDKKLKEVIIADGAKGEQGLQGRDGQDGAQGQAGRDG
KDVLNGKANPEVNQGKDGDKYVNTETGDVFVKNNGNWDKEGNIKGPKGDKGERGEDGKTP
EVTVTPGKDGHSTDITFTVPGKDPVTFTVKDGKNGKDGRAPKIKVEDITSPSRIRRDTDA
AATPTRNGIRVTVYDDVNDNGVYDEGVDKVLNSKDIYNGIDGRDGSAPTITTKDNGDGTH
TITVQNPDSSESTTVVKDGKDGKTANITTTENPDGSHTITVTNPDGSTKETVVKNGKDGK
TPKVEVTDNNDGTHTVKVTDGDGNVTNAIIKDGKDGKAATATTTENPDGSHTVTITNPDG
TKNEFVVKNGRDGVDGRTPTASVRDNGDGSHTIVITNPEGVTTETTVRDGKSPKVTITDE
QNGTHKISVLNGDGTTTETIIKDGKSPVATVRDNQDGTYTIRVENGNGTVSETTVRDGKS
PTAKVVDNGDGTHTITVVNSDGTTTTTTVRDGREPKLEVIDNNDGSHTIKVTGADGKGTT
TTIFDGKSPKANIVDNGDGTHTLTIVDSDGREYKSIIKDGKDGKDSVSPTVTVKNNNDGT
HVVTITNPDGSKTEMVIKDGKDGKCGCQDKPVTPSNDKPVPPTPNVPTPEVPVKPVPAQP
TPNVPTPEVPVQPTPAVPTPEVPVKPVPAVPEQPVVPTPAQPATPVNANPVAPTTGKENR
GDKLPETGSQSDYISVLLGSGILLSLYVGRRKED (SEQ ID NO: 1530)
>orf02654 
MRNLLSTKVQRQLRLMETLIQNRNWMKLHELAEKLGCTERILKSDLNELRIAFPSINIQS
SVNGIMIDLEVNTSVEDIYQYFLANSQSFQLLEYMFFNEGLPIYRTIENLYFSSANLYRL
GRNITKVLSSQFQIELSFTPSEIRGNEIDIRYFFAQYFSERYYFLDWPFPDLPEEDLTEF
ADFFYKITNYPMRFSIYRMYKLMIAISIHRVKNGHFIDLPNHFYKEYYPLLKSIPNFQET
LAYFSKHFGLEMTPDTIAQIFISFLQNDIFLDPQEFFNSLEDNSQARYSYQLLSQILERL
SKQYKITFTNHDELIWHLHNTAFFERQEIFSTPILFEQKALTIKKFEVYFPDFMGSARQE
LAQYRQAIGQHDHPEQLEHLMYTILTHAENLSTQLLENRPPIKVLIISNFDHAISLTFVD
MLSYYCNNRFTFDIWDELKTSPEILNQTDYDIIVSNFYISGITKKFICRNHLSIMNLVNH
LNTLSNEIHLSNTL (SEQ ID NO: 1531)
>orf02655
   MIFKIGLFYLGQFVSLDMTVHKPIKKLQGMVVLSSLPFQSLDILTFFRSLLS (SEQ ID NO: 1532)
>orf02657
   LAGFYLMVASMLLGLLALKIGFSQFKEMKDKFLSILTSLAGLALVLVAVWLGWPK (SEQ ID NO: 1533)
>orf02677
   MLDSDIGCSRKNLLGLFWIRRRRNIHIVDRAMEKGISNRAPNKISLKACFFNFF (SEQ ID NO: 1534)
>orf02696
   MAFNQFNRCITLSIPTAPNIPTSVVHRTYLHDATVPNNVREKT (SEQ ID NO: 1535)
>orf02698 
MQQITEIIIAFATSFLTVAVGGIVKAVKDYLLRKGGEKAVIIAEILAKNAVHAVEQVASE
TGYKGEEKLEQARAKVRAELTKYNISMTDKDLDTFVESAVKQMNDAWKGR
 (SEQ ID NO: 1536) (SEQ ID NO: 1536)
>orf02699 
MKIEFFNFLRSVIQTEDGLVLYALALIVSMEIIDFVTGTIAAIINPDIEYKSKIGINGLL
RKISGVLLLMILIPASVLLPEKTGFAFLYSICLGYIAFTFQSLIENYRKLKGNVTLFQPI
VKVFQRLLEKDDDTKKGE (SEQ ID NO: 1537)
>orf02700 
MLKVTKTRQLVTEFFAQDGDQQKLVKTTVINTDNKAVSTISETLHDPDLYANNRISMRKH
EQELREMRYKIEDAILAELEADSEHKE (SEQ ID NO: 1538)
>orf02702 
MTKFINSSGSLHLNIYIEQVSQDIANNSSRVSWKATVDRDGAYRTYTYGNISNLSVWLNG
SSVHSSHPNFDTSGQEFTLASGEVTIPHSGDGTKTFAVWASFDPNNGVHGNITVSANYTL
SSIPRSSSVSDNALSGNRRLGSPHTLTIDRKSSSFTHQVWYRVFGSNWIDLGKNHATGVS
FVPNIDLARYNTKAKSGTMDICVRTYNGTTQVGNDVYSNGWYFEIPESVKPTFSGITLTD
MNTVARQLLSGNNFLQIISDIQVNFNNPSGAYGSTITGYRAEIVNKNQVTTVNGGRLGMM
NFNGSATIRASVVDSRGRQSDTRDITINVIEYFAPAFSFTAFRTRETPNIIQVVRNAKIA
PITLSGSQKNVMTLSFKVARLGSTTFTADHGRASGIWTTQHTLNNSAANMAGNYVATKSF
VVIGTLSDKFTSTEFTATVATESVVMSYDKDGRVGIGKVAEQGGAGSLDVLGDIYARNKP
IQQYQLTDNNGCGKLIKQDFNTMKETGTWWINGSSQNNPFSGTWGMLEVFRPNPGSHERI
QRFTTSTGYMAVRENGYDNNWRPWRYLVQQSKSTNNSDYVALLKSESTPTPWQNAILQNG
WNHHRDYGGVQFSKTFDGVVCFKGTCKGGKIARESIILTLPEHFRPSTTLFKTALNNDYG
SAVIGIYPNGNVVVKSNVDATWLNFDNVFFKI (SEQ ID NO: 1539)
>orf02709 
MLLTIHDANLQKVAFIDNEKQGTLNYYDDTWTRSLATGSSTFEFTVFKKAVKSDLPLAKA
YHHLNEHAFVSFKYKGKSFVFNIIIVEENEQTIKCYCENLNLELINELANPYKSNKAMTF
KEYCEAMDLLNYTHLSIGINEISDYKRTLEWEGQETKLARLLSLAKRFDAEIEFDTQLNA
DSTIKKFSVNVYHENDDNHQGVGRVRNDVIVKYGKNIHSITRKVDKTGIFNTIRPTGKMP
TVEEELSGDKGSKSETVKNADGSTTKTTISTASDGTKSKTIVHTKVTKLADKTRITTTTT
TRSDGSIEQTVTTSKKGGASTSETKVLKKPNPKEKTNTTEDVLTIEGLDEWEVKNEKGIV
EFYQRGQALYAPISMQLYPSTFTHSTGELDQWTRKDFHFETDEPNELRRLGYLKLKKYCY
PAITYEVDGFVDADIGDTVKVHDDGFAPLLMIQARVTDQKISFTNPVRNKTIFDNFKALE
NKLSADIQSAFERLFEAAKPYTIKLSTDNGVIFKNQIGQSLVTPTLYKGGKPVVVGVTWR
WALDGEVTTGMTYLVRGSNVTDTVTLTVAAYIGNKEVAVDEISLVNVADGKLGTPGTPGR
DGRTPYVHTAWANNATGTDGFSLDSSINKLYIGIYTDFEPNDSTDPKKYKWAKVKGEKGE
KGDKGEPGQRGLDGLQGARGEQGLPGRNGADGRTQYTHIAYSNSADGTKDFSVSASDRAY
IGMYVDFNRADSNTPSDYNWTLVKGSDGANGVAGKAGTDGRTPYLHIAYATSNNGSQGFS
TTDSTNKTYIGTYTDYTQADSTDYRVYKWTLIKGADGTGISNVTNYYLATTVSTGITRTS
AGWTTTPQPITSDKRYLWNYRVELYTNGTSKTTEPTVIGVHGEKGERGLQGLQGLQGARG
EQGIPGPRGADGRTQYTHMAYADNATGGGFSQTNTDKAFVGVYIDFNPTDSRNPADYRWT
RWKGRDGANGVAGRAGADGRTPYLHIAYATSNNGSQGFSTTDSTNKTYIGTYTDYTQADS
TDPKKYKWAKVKGDKGEKGDKGERGLQGLQGLQGARGEQGIPGPRGADGRTQYTHMAYAD
NATGGGFSQTNTDKAFVGVYIDFNPTDSRNPADYRWTRWKGRDGANGVAGRAGADGRTPY
LHIAYATSNNGSQGFSTTDSTNKTYIGTYTDYTQADSTDPKKYKWAKVKGDKGEKGDKGE
RGLQGLQGLQGARGEQGIPGPRGADGRTQYTHMAYADNATGGGFSQTNTDKAFVGVYIDF
NPTDSRNPADYRWTRWKGRDGANGVAGRAGADGRTPYVHFAYSENADGSGLTMTDNGQRY
FGHYSDYEKPDSSDKTKYKWADRWAKVDGGYVNIYALSKNRSIGKSYHVSEFNMDVLSGN
ITLKAIGSDPYIGAVSSHPGIFIKQQGMKIPVIQGRSICITITNPLFRKNYISFFNSLGK
TVKTYKHYNTNKFLISSADLVGVEFIALRYGAGSSNIQIGTVLETKVKVEYGTVHSDWSP
APEDIESNINSKADQGLTQEQLNALNEKSQILEAEMKAKASMEAFSELEKAYNAFVKSNA
DSRKKSESDLVEAGRRIDLLTTQFGGLAELKTFIDTYMKSTNEGLIIGKNDASSTIKVSS
DRISMFSAGKEVMYISQGVINIDNGIFTASIQIGRFRTEQYHLNKDVNVIRYIGG
(SEQ ID NO: 1540)
>orf02711 
MTKIMTFNGVDMSKFFRITDIIRPIGNKRSVSTDNAPLLGVNIQQVKIGEKEHIIKFDIK
TTNAIEMEQLKHDLAGILNVLEPVKITYGDEPDKYYMGLPVDEITPENLTRWFQRSELKI
IIPDGVAHSTTLKNFDIDTNETSAPDRIVFNLTNTGTEPAYPIIRIKHNSENGYIGVVNN
RAAFELGNREEADTEKYRDSETLIDYRGTNILKGFQNGTKGVAVTNDNKERLVGTLSTTS
MWGRNHIELSNRGTVEKNRNNAQSLTWAIPVDSSGEVGSLNDYLLWRQVFMAAVANQYGF
IKVTVSDTDGNFLYGVETYKRYQTLDCEYSFFTTDGKGGYKFIKWWYFTGTGAQVGKLDP
 
FSAEKGWSELKRNDDRVQVFFDGSHYDFIIPEIKDKKSAKIHITLGALRDWPLVSHMYVD
EFMYRKDFVTKSRDIPNRYPIGSNVVINSEDDSVYIDGISKVSEVVDGSHWPAIPPGKSQ
LELYFSRFVKKKPTVTIEFEERWI (SEQ ID NO: 1541)
>orf02714 
MADGKVTIVVDVDGNKVKVLNDELDKAAQKGDRGSDSLKKFAIGGAAFKLASKAVDLLTD
SLGGAIQRFDTLESYPRVMQAMGHSTEDVTRSTKKLAAGIEGLPTTLNEVVGTAQRLTSI
TGDINKSTDLTLALNNAFLASGSSSADASRGLQQFSQMLSAGKVDMQSWKTLQETMPYAL
QKTADSFGFAGQSAQNDFYSALKEGRITFNQFSKKLVELNGGVGGFAELAKSNSKGIQTS
FGNLKNAVVKGVANTIKALDDLTKAATGKTIAENFDALKVIINAAFGVIVNVIKASTPVF
QTLFSILGTGASVISSLTPVIISLVSALVAMRAANEAITATKNLINSWQTFKTTATGAIQ
IINLMTAAQATCGSVTKAQLVANLANNGALTASNLLYGVLTGSISLQTAATIAATAATTA
FKAALTALTGPIGLVVAGVGLAVGALVGLWQWLTAESEETKRLKSEQEELVKSTDQLTDS
VKQSAKERQKNLESVKGNTESYQKLADEIVQLSQKTNKTAADKKNLKKKIDALNASVSGL
NLVYDKNTDSLSHNNDQIKARISAMEAESTWETSQKNLLDIEQKRAEIGEQLKQIAEQRK
KWNEESNVSDSVRKERLQELNDKETELKNTQTELQTEYEKTSQVQQAASEAMAAAAENGS
NRQVISYEGMSKAQQKAVDDMRSKYNELLETTTNMFDQIQMKSAISVDEMIANLQKNQEA
VNNWATNLNTLAERGVNEGILAKLQAMGPQGGLYVQELVNASDEKLATLNEVFTQGGESA
MNGLTAGMDTGALGITDKIKGIVQSQVSSLQEEIAAADFPEKGKNIPEGVGDGIKAGAEI
ASEASKNMANDIKESFTSEMDINSPSRVFNEYGGFITTGLAEGVDKGTNQPVSSVTNLAN
QIKKPFDSLQSDFTYIGEMAMSGLNAGLWSGSGSVMATANSIAERVKATIKSALDIHSPS
RAMRDEVGRFIPQGIAVGIEADAGVVEKSMLRLKESMMIDTRPEIALGLNKKLGAQVTVK
QSSKQTIAEKIKVTMDKSSELLEKALDVAETAVRRPNEMYLNDGTLVARTGDKFAKYQSE
QLRRDNRMKGVLS (SEQ ID NO: 1542)
>orf02715 
MSMKLNDALITNFSIADKEYDIDLSFNKVLDVFEILKEDEMTRLEQAQLIVHLLTGQELY
DIKEVVDCWIYIKEHFLGIEKETVQYDLLGNPMPKAKGEEEQEKLIDFEQDAEYIYASFL
QAYGINLLKVQNELTWTEFKALLNALPDNTIMQQIIEIRAWKPEYGGDKNKMRKLQAKYS
LGKEGEDNG (SEQ ID NO: 1543)
>orf02717 
MTDIQIELKRTGFPVKIGEVELWFDTSQESLMCFYDMEEELKRRLVQYELDVVSANINNK
IERDGVTKEVVAGAIELEKKQLEIQYDLIFGDGTFDKLYSIYPDYNALNNALEQTAIMLH
DKLEEVAEQHKTVVKERASHYLNKGKVTPIKNNKKQKKNKKK (SEQ ID NO: 1544)
>orf02718 
MTRQKNALRGHFVAPYNGGTEPSTEDTWLELAKWISDVSDDTDEKTDDQAYYDGDGVEET
TVVSVKGAYTFEGTYDPDDKAQALIAGMKYKTGDDRKLWHKVVSSDRKKQWVGAATATEI
KAGSGAASDYEAFGCKLSYNSTPKETGIG (SEQ ID NO: 1545)
>orf02719 
MRENDFQNVLLKHIKTLNLPVEPRFDYFEDDKDDLVINQIPGGKVDREYMDGTQEVSLPF
EIAVKAKKNSVANDTIWLVTSELAKIDLVLPSDNNSYEYMGMEVSRPAMKGKDEQGYYYY
TIEIVAKIVIERNKQ (SEQ ID NO: 1546)
>orf02720 
MNIAIKVDLQKAKQKLSNESMTRGKIAVASKILLDNEQYIPLRGGELRASGRIVGQGDAV
VYGTVYARAQFYGSNGIVTFRRYTTPGTGKRWDQVATSKHAEEWARAFVKGMGL
(SEQ ID NO: 1547)
>orf02721 
MTYLTQEEFDELDFDEVTDFEKLAKRAKIAIDLYTNGIYQKDIDFEKEIAYRKSAVKLAM
AFQIAYLDASGIMSADDKQLANSVSIGRTSISYSTSQSTLAGQRFNLSMDAENALRQAGF
SLVVGVAYDR (SEQ ID NO: 1548)
>orf02722 
MALYKATKNLFFEQLNMDVIVDDIIELDEDYAKEVNKKLKNAFPDVKNVLELVDKNGTLE
PEDAPSVDDASQATVED (SEQ ID NO: 1549)
>orf02723 
MPSNQNNAVRRYEKQYAGILETVFGVRAAFSNALAPIQILDGVQENSKAFSVKTNNTPVV
IGEYKTGENDGGFGDNSGAQSRFGGVTEVKYENTDVNYDYTLTIHEGLDRYTVNNDLNAA
107
 
VADRLKLQSEAQTRTVNKRIGKYLSDNATKTEALADFTDDKVKALFNKLSAFYTNNEVTA
PITVYLRSEFYNAIVDMASVTSAKGATISLDENGLPKYKGFTLEETPAQYFETGVIAIFS
PNGIIIPFVGISTARVIEAENFDGVKLQAAAKGGTYTLDDNKKAIYKVTGTIV
(SEQ ID NO: 1550)
>orf02724 
MAFTTEELLNLGLTEEQAKSVFALRGKELNEDKSALETIKQERDSLKSQLQKAEEQVEHL
KSLENISAEQKDAIDKLQAEYDKYKNEAAAELAQTKKVSAISLALKDTNAFNPDKLMKFI
DVDAIQLDDNGKPQIDEVINGLKESDPYLFKAEESKPSPNILPQGNPAGEGTSDVDPFQA
IIDGYGK (SEQ ID NO: 1551)
>orf02727 
MKKKRKQITFNDQQFPLQMQGVGDIYEKLQIDIFDRMIKRLKERGSIDLMRNPYIWQLEK
LNDMHMLNEQNLKLISERTGIAERLLRDVIENEGLKVYKDTKQQLEEDLNKIPEGEISNG
VTDSLEAYSRQAVSDLNLINTTLPKSLQVAYKSIVEETVAQVVAGTKTSDVALHDTIMKW
QKNAFTGFVDKGGRHWKADSYARAIIKSTTYKVYNEMRTRPAEELGVDTFYYSMKAMARP
ACSPLQGQIVTKGTGREIDGITIYSLLDYGYGTAAGCLGIHCGHYLTPFIVGVHELPNLP
DYLKNLTPEQAEENARIEAGQRGLERLIKTHKERLHYAHTLQDDKMIQAERLKVRGYQTK
IRNLINQHDFLTRDYRREKLYIS (SEQ ID NO: 1552)
>orf02728 
MSLFQKVKDFFSRGRYYMQTSNLNSILEHPKIAVTQEEYDRIKRNLVYYQSKWDDVQYKN
TDGDIKSRPMNHLPIARTASKKIASLVYNEQATITTKNEILQKFLDDMLTNDRFNKNFER
YLESCLALGGLAMRPYIDGDKVRVAFIQAPVFFPLESNTQDVSSAAILTKTIKSEGRKNV
YYTLVEFHEWVTADGQETGSTNDKKYYRITNELYRSDVNDVLGQRVNLSELDKYKNLEPV
TVFENLSRPLFTYLKTPGMNNKDINSPLGLSIFDNAKTTIDFINRSYDEFMWEVRMGQRR
VIVPEHLTQRQYQRPDGTIDFRPRFDVEQNVYMQIGGSSMDAGGITDLTSPIRANDYILA
ISEGLKLFEMQIGVSSGMFTFDGQGMKTATEIVSENSDTYQMRSSIVALVEQSIKELCVS
MCELGKAVGVYSGEIPELDDISVNLDDGVFTDRHAELDYWAKMVAAGFSTKKRAIGKTLN
ISGVEAEKELNAINSELLPMNDAELAIYGMHDQNEEKADDKG
(SEQ ID NO: 1553)
>orf02730 
MTFNVQKNINPHFKSVWISSLPYNVLKGGRNSFKSSVIVLKLAYMMIRYIIAGEAANIVV
IRKVANTIRDSVFNKVWWALNLFGIAEQFTKTVSPFKIVHKTTGSTFYFYGQDDFQKLKS
NDIGNIIAVWYEEAAEFNDQEDFDQSNVTFMRQKHPRAKFVQFFWSYNPPRNPYSWINEW
FESIKTNKNYLAHSSTYLDDELGFVTEQMLEDIERIKENDYDYYRYLYLGEAVGLGNNVY
NMSMFHAIDALPSDDKLIGISFALDGGHQQSATACCAFGITAKGKVILLDTWYYSPAGQV
VKKAPSQLSKEIYAYMRSVIEKYRVQALQYTIDSAEGALRNQMFLDFGLKWHPVAKLRKV
TMIDSFQSLLAQGRFYYLNTENNKIFIEEHKMYRWDEKTIKSDNPSVIKEDDHTCDTTQY
FVLDNAKLLGLRVGNV (SEQ ID NO: 1554)
>orf02731 
LPRDGTKNLKPVTERTKDEARAISSKGGKASGIARRKKADLKKAFETLLSLDVTDSKIKK
QLEEMGMAGNNEALLAFATFQQAVKGNQKATENIIKLTNTKDKYDIQEQKERIKALKYEN
RERAEAEKGSSETIEIVDAWAEDVRGATDDL (SEQ ID NO: 1555)
>orf02732 
VAQKLTKLKDIFKHVSSIDLGKEILFEDLELYNKETETSKQYQSIEEAENDLYLMEKVNK
INFTLGGGRGANFEKGKDGKYPGFRGAGGARDSGSSKALHPASLNNQGRFSSVEGAIQGF
IKKHGGSRTEYSTAVDSQGFAHNYVHGGKNSVQILPISGGFTAIHNHPNGSNFSSTDLHS
FAALKGMNTLVATNSSKAYRITKGANFDAKGFDKAVSKSRFTTKDYNKGADLWLKKNAKK
YGYTYSYE (SEQ ID NO: 1556)
>orf02735 
MLQIEYVDIKSIKPYHKNARHNDGEATEKVAASIKAFGFQQPILVDDNNIIITGHTRLKA
ALSLGIDTIPIAHAVNLTDEQIKAYRLADNRVAEYSTWDSELLNIELSQFETIDMAQFGF
ELSVTGFNFGNEEEQQEETENEEEDAEDFHRDTTINQYNLFNYDDTRVEGFYNMPKIEGV
DHIPKDFQGFNYVLNKPDYSSCVHFFLDDYQFERIWQRPDFYIEKLLEFDSALAPDFSLY
LDMPIAMQVWNIYRSRLIGQIMQDYGLTVIPTVSWASEESFDFCFDGLPKNSTLAISTIG
VKQNKEQFEVWKNGVTEMIKRLTPKRIVVYGGKVEYDYKDIEVVYFENATTERMKESGTK
TN (SEQ ID NO: 1557)
>orf02736
   MNVIGACQKILFYSPTQAYVLLNAWFNDYFRATYTELLENAILDK (SEQ ID NO: 1558)
>orf02738 
MNPEIIDNINKPSHYQGANGLEAIDVVHNFVGSLSGASAFFWGNAIKYMLRFQKKNGLED
LKKARKNLDWLIEEMDKEGR (SEQ ID NO: 1559)
>orf02739 
MFFQDSEIEEFELNDTLRNDYITAYPDEIELMQSTGLKDKNGKEIFEGDIVRTTRFLGRA
DEIGGFYEYEKDYVGVVKVLEGSWVIDTGSVAVRLWSEIDESEVLGNIYENLEFLEVNE
(SEQ ID NO: 1560)
>orf02740
   MEDEQNILETQLILGKQVLEIVLDLLKDDSKIGVVLPLNINDREFTITVEKEVTDRD (SEQ ID NO: 1561)
>orf02741 
MTQTLEEGMKNQSKCIKIPMEIRPFDVGYRIVNKHGQALALKNGASIFALPSLAEKAIKK
EFGKNDPDFDIEKHFVEEVAIVNLSKFHSYFEEVE (SEQ ID NO: 1562)
>orf02742 
MNIKALIKKYEELWNEHSPFYEPVPYTSMVELFLKELKQLDEPEKVKVPRFVAEWIEEAR
KACKDVVELFEFDFTNDEVRKWFMQERPFDLVARAWLDGYEVEEEKRYLVTLKNRQPLVK
SQSGSTLYFSQDITARNYKGTQKELEDANFGWVFDCEGIEIEEVE
(SEQ ID NO: 1563)
>orf02743 
MMEELKQKVNEVYNWTVEDGKPQPPKQDLPQAVKERVDYFWEMAEDGMTFMGAMECIFAD
EKPTDYDLGATKDWLPKSKEFDDWIGYAPSMAQVVIAVYLIYRGN
(SEQ ID NO: 1564)
>orf02744 
VIEVNIKFDNFEAHGFYQDDTKLGKIRDAIISQMNNGHVVVLGEDRGILLNPKVIKSVQF
KVVEDNQI (SEQ ID NO: 1565)
>orf02745
   LYPTVKAIIDGHTDAGIWTDDNHKVIKKLSFVYGGLSEEKGHYRLEFDIEEV (SEQ ID NO: 1566)
>orf02746 
MTTENLKSALEYAVELNEHGLEILTAADGTEYYDANKFNLKELDPKRYPKTLELSTLTSL
VDYLKTDLNNLKNQRLIVAVEKNDEVCVWSENDEIEHRTLLVDVKARIPELSFGRFLSLE
QFNIMLQSNFIDDNDRGTLLEFASALKIENGAEIEDNGVSQVATVKTGVASLAKGKAPNP
VTLRPYRTFSEVEQPASLFVFRIDKQANMALFEADGKRWVADAVGNIASYLKEQLADQKH
ITVLA (SEQ ID NO: 1567)
>orf02747 
MDKKLIGLDLTHIADGGLQEKLDKELEKVFDNILDLNTDAKAKRKVTITLTMSANEERTV
VDTTMEVKSKFAPQNGVATTILIGRDFDTGQVHANELKSTVPGQMYFDENGEILTDIGQP
VAEIEQQAETKSDIIDFNKKKVGN (SEQ ID NO: 1568)
>orf02749 
MESAGHECIGFCEIDKFARASYKAIHDTKGEIELHDITTVSDDTIRGIGHVDVICGGFPC
QAFSIAGNRRGFEDTRGTLFFEIARFASILKPRYLFLENVKGLLNHDRGNTFEVILSALD
ELGYDVEWQVLNSKNFGVPQNRERVFIIGHLRGGSGRKVFPLSGDGAAITCEQPKINKVG
NTRKKGKSQSGDVVSIDSLAPTLCSTTTQKDPLKVLIENEIKQFGVLQPNYNQSGVVYEI
DGISPTIRAYQGGNLEPKIRVKEATKQGYQEAEIGDSVNLSHPNSKTRRGRVGKQIANTL
LTGESQGVVEPDFRIRKLTPRECWRLQGFPDWAFDKAQEVNSNSQLYKQAGNSVTVNVIS
AIAQGLGGN (SEQ ID NO: 1569)
>orf02751 MINNVVLVGRLTRDAELRYTQSNIAVATFTLAVNRPFKNEAGEREADFINCVIWRQLAEN 
LANWAKKGSLIGVTGVIQTRSYDNQQGQRVYVTEVVASNFQLLESRNSQQNNQGHQDHHG
GYQQQGYSNQGSSFQNGNSYGQQGSFVEGNTTNLVPDFTRDNNPFGRPTNPLDISDDDLP
F (SEQ ID NO: 1570)
>orf02752
   MRCFYVSGKIADLDLGSEINAENSFMAAIEFVKRYTDLLKFGSNEIKVSEVEEVQNDK (SEQ ID NO: 1571)
>orf02754 
MVKDVTNSLTEIKVDFQPAVINVDREAIEAQVAAAIAQYSGREVTVDNYKEVYEERTRFN
KLIGGLDTQRKDFNRQINEPAKDFDKWVKEKVIKPIEAVTDAMSAGLNAIDEHERLMRVD
VVRATFEDKCMVAGIEKSTFADKYDEYSLKKYFKTGKYELKKTTLDEMDGLVLSEFDALE
EYKANKQAIQEQAQEYDLPADSYIRHLEDGKSLVDILKMMKTDRDAEIARKEQKEIQEKA
KAERLEEIAQSAKKNANANIKAYDAETGEILEQGTITPEPQNNAREVAKFEPSEPLVKLV
RLELHGGLEQWENTQEYFEDNFIGFETLED (SEQ ID NO: 1572)
>orf02756 
MADLTFAELQRKMQIEKQTKQGVKYPFRTAEDINNKFKSLDSGWSVSFPEDDIIQKGDKL
YYKAVAVVKRESDGTIEKAIGWAREEDVPIFHTQKGDVKQMQDPQWTGAVGSYARKYALQ
GLFAIGGEDVDEYPVEESQEQGQNNQQQKPNNQQAQGQNQVRYIDNTQYQEINDLINDIA
KIKGMPFDTLANYVLSEKLKGLQDFHRVQVGDYEVLKNYLTEQLAKAKAKAKRGN
(SEQ ID NO: 1573)
>orf02757 
MPNWAEGTLKLRGRRENVASALKEMLLGNKGATLEEEYDGTLLIFKNEYDYFYINGTRRA
FISSKDIEIWLDDDFVIIELEDFEQAWAALADNYTEISSKFDVDIKIFTFEMGMEFTQEI
EISKGEIIKNIVNENFTNYSWDVPFSRLGG (SEQ ID NO: 1574)
>orf02758 
MKKIATAMNVSVSDLFTQDTPIKKNRHSTPVNPKIYKEFIDNVNQYQRLTGATYEKISNI
IGKSNSYIYDVIDKQRKSTLSIKSNASLTKGSMILRQEIEKIESGKNRIPSKLTYQTIDR
DSEVVFRFNGLIKSVNELSDKELQIIVSIFDALKIPAKISKIEIRETNVFGGGK
(SEQ ID NO: 1575)
>orf02760
   MKKLPSQQKYLRNDGQLVTIKGFDAYLQYRGSQSWKKEMAKTVKMTR (SEQ ID NO: 1576)
>orf02761 
MPDITNGREKVNDFLKDKGIKKTSLAIAYGFKRQEVTNILSGTTKGPRANSFILQVIEDY
GIE (SEQ ID NO: 1577)
>orf02762 
MFETFEKIKSLAKKQGISLNTLEDRVGLGKNYIYSLKNKKTPSAEHISKIADYFNVSTDY
LLGRTDNPTIANKKEQFFFEGKEVDVEELASTAMRFNGKPLTEEDKKAIQNIIEIYLRKQ
(SEQ ID NO: 1578)
>orf02763 
MTEKEFSQNLGIDIEIFEDGLFPDEAFYIPALKTMFLSDAISDEKRVQVALHEIGHRNHA
PDTYQLFREKCELEANRNMIHHLMKAELDIAEDATTFNYLVFMEKYNLKTIADEIMVKEE
YLALLN (SEQ ID NO: 1579)
>orf02769 
MNIIAIIIIVIFVGGVIGAVIDNQKKSPEQRERELETFRANQEKKKQEKKQNIITCPNCK
SKDVTFLQQDKKAFSVGKAVGGAVLTGGVGALAGFAGKKGNKQWHCQNCGNFFETK
(SEQ ID NO: 1580)
>orf02765 
MWMEELSNGKYKFFERYKDPYTEKLKKVSVTMEKKTPQARNQAAILLQEKINKKLSTKQV
ESITFEEIYNLFYKSWAQTVKESTKHNCKSVDKKMKEVIPSDTILANLDRRFLQEAIEKI
IESNGYITAKKVRHRLRGIFNYAVQYSYIENNEVDYTTIPQKPKTLEELEKKRNNFLTMQ
EIKALVDVLNRREYHQKYADMVLVLTLTGMRYGELTALQLKNIDFENNKIEITGNFDSVN
KIKTLPKTTNSIRTIKVSESVIEAIQRQIVRLSERFQPLSSDDYIFCFEKWNQPTTIACF
 
IQILKKYGKQAKIEKNLSSHIFRHSHISFLAESGLPIKSIMDRVGHSNAKMTLEIYSHTT
EDMEDKLVNKLDTIF (SEQ ID NO: 1581)
>orf02777 
LHPFTRNITCDRYILALFSNLVNFIHVDNPTFCTLNVKVSSLQEFEEDIFHILTYITSLR
QSCRIRNRKRYIQALSQGLSKESFP (SEQ ID NO: 1582)
>orf02778
   MIINRHCQGTLGTILTDYIVVQDMEEFDWFWYLRQVCQDFLNQFFSNNFLS (SEQ ID NO: 1583)
>orf02786 
LKTKIGLASICLLGLATSHVAANETEVAKTSQDTTTASSSSEQNQSSNKTQTSAEVQTNA
AAHWDGDYYVKDDGSKAQSEWIFDNYYKAWFYINSDGRYSQNEWHGNYYLKSGGYMAQNE
WIYDSNYKSWFYLKSDGAYAHQEWQLIGNKWYYFKKWGYMAKSQWQGSYFLNGQGAMMQN
EWLYDPAYSAYFYLKSDGTYANQEWQKVGGKWYYFKKWGYMARNEWQGNYYLTGSGAMAT
DEVIMDGARYIFAASGELKEKKDLNVGWVHRDGKRYFFNNREEQVGTEHAKKIIDISEHN
GRINDWKKVIDENEVDGVIVRLGYSGKEDKELAHNIKELNRLGIPYGVYLYTYAENETDA
ENDAKQTIELIKKYNMNLSYPIYYDVENWEYVNKSKRAPSDTDTWVKIINKYMDTMKQAG
YQNVYVYSYRSLLQTRLKHPDILKHVNWVAAYTNALEWENPYYSGEKGWQYTSSEYMKGI
QGRVDVSVWY (SEQ ID NO: 1584)
>orf02791 
MHKNFVVVVTDFFTAVQFIQFNKEGTTCHNTTKFFNHLDSCLNSSTCRQKVIYNKNTLTW
LNGIRVHSQGIDTVLFFIVSRNNFAWQFTWLTNRRKTNSQLKGNWTTHDKSTSFRSHDHV
DFLVSSILNDFTNSVAISISISHQRTNITEGNAFLWIIFNCCNVIF
(SEQ ID NO: 1585)
>orf02795
   MEIKEQTRKLAVSYSKYSFEVADKTDEVSNHTYGKATLTWFEEIFEEYKEHHNIDV (SEQ ID NO: 1586)
>orf02801
   LHKTLENIGEFEEDNLYYSSMTKAETRISFPIFSLILHYI (SEQ ID NO: 1587)
>orf02803
   MLNRQVCFCFVNHISPLNVVIWENLSLEELLYAICICFITHKIAKQTSLTIDNAGIAMNN IR (SEQ ID NO: 1588)
>orf02808
   LNSRFFYTDFFKGRQAKGCSFSCTSLSLTDNILAFKGQRNSLFLDRTSFYKTSFFNFC (SEQ ID NO: 1589)
>orf02821
   MRFLADQDRIQHHRYSMALFDKVQGLLSHTDSREKTNLNSPKFHIT (SEQ ID NO: 1590)
>orf02822
   MLKNGIISMKDFKSFFCQGCQTSHCYKPMQAVQGIGSQIS (SEQ ID NO: 1591)
>orf02825 
VTAHRIFGTSSIHNKLIGLAMFGITAMKIICHKLNRNHINIFRRLGIQGKTEFLLIHLIR
QVKMNDLSQGMNPTICPTSTVNSNGLPFI (SEQ ID NO: 1592)
>orf02829
   MLARSKNCFMKSLSIFLLIFYFFDSYQISKKRRSLIGL (SEQ ID NO: 1593)
>orf02840
   LEVCIHHHHQISCRILQACIKGCFFAKISRERNIMDCRILLPIGL (SEQ ID NO: 1594)
>orf02847
   VDRTDEVSSKHCFEVVDRTDEVSNHTHGKATLTWFELDFRRV (SEQ ID NO: 1595)
>orf02893 
MIAEFIDGLQKFHFLQNALITAIVVGIVAGAVGCFIILRGMSLMGDAISHAVLPGVALSF
ILGLDFFIGAIVFGLLAAIIITYIKGNSIIKSDTAIGITFSSFLALGIILIGVAKSSTDL
FHILFGNILAVQDTDMFITMGVGAAILLLIWIFFKQLLITSFDELLAKAMGMPVNFYHYL
LMVLLTLVSVTAMQSVGTILIVAMLITPAATAYLYANSLKSMIFLSSTFGATASVLGLFI
GYSFNVAAGSSIVLTAASFFLISFFIAPKQRYLKLKNKHLLK (SEQ ID NO: 1596)
>orf02913
   MYEEPEVAPVHPTGPTPATETVDSAPGFEAPQESVTIL (SEQ ID NO: 1597)
>orf02945 
MGNNGQFTFGYRHDFFQNQLAIFNALVDTFTRRTIDIKTLNTFINEVLNQGTRTLWTYFS
LLIITCVEGWNDTFVFFQI (SEQ ID NO: 1598)
>orf02948
   LTKIFGWILRIAVLAADVYGNFANNIAVAWDAHDKIPNNGRINF (SEQ ID NO: 1599)
>orf02974
   LSTRNKYCKNLIIFESTFNILDIVKKDLKLNSKLEKDLKY (SEQ ID NO: 1600)
>orf02976 
MSYFRNRDIDIERISMNRSVQERKCRYSIRKLSVGAVSMIVGAVVFGTSPVLAQEGASEQ
PLANETQLSGESSTLTDTEKSQPSSETELSGNKQEQERKDKQEEKIPRDYYARDLENVET
VIEKEDVETNASNGQRVDLSSELDKLKKLENATVHMEFKPDAKAPAFYNLFSVSSATKKD
EYFTMAVYNNTATLEGRGSDGKQFYGNYNDAPLKVKPGQWNS[?]TFTVEKPTPELPKGRVR
LYVNGVLSRTSLKSGNFIKDMPDVTHVQIGATKRANNTVWGSNLQIRNLTVYNRALTPEE
VQKRSQLFKRSDLEKKLPEGAVLTEKTDIFESGRNGKPNKDGIKSYRIPALLKTDKGTLI
AGADERRLHSSDWGDIGMVIRRSEDNGKTWGDRVTITNLRDNPKAFDPSIGSPVNIDMVL
VQDPETKRIFSIYDMFPEGKGIFGMSSQKEEAYKKIDGKTYQILYREGEKGAYTIRENGT
VYTPDGKATDYRVVVDPVKPAYSDKGDLYKGNQLLGNIYFTTNKTSPFRIAKDSYLWMSY
SDDDGKTWSAPQDITPMVKADWMKFLGVGPGTGIVLRNGPHKGRILIPVYTTNNVSHLNG
SQSSRVIYSDDHGKTWHAGEAVNDNRQVDGQKIHSSTMNNKRAQNTESTVVQLNNGDVKL
FMRGLTGDLQVATSKDGGVTWEKDIKRYPQVKDVYVQMSAIHTMHNGKEYIILSNAGGPN
RENGMVHLARVEENGELTWLKHNPIQKGEFAYNSLQELGNGEYGILYEHTEKGQNAYTLS
FRKFNWEFLSKDLISPTEAKVKRTREMGKGEMGKGVIGLEFDSEVLVNKAPTLQLANGKT
ATFLTQYDSKTLLFAVDKEDIGQEIIGIAKGSIESMHNLPVNLAGARVPGGVNGSKAAVH
EVPEFTGGVNGTEPAVHEIAEYKGSDSLVTLTTKEDYTYKAPLAQQALPETGNKESDLLA
SLGLTAFFLGLFTLGKKREQ (SEQ ID NO: 1601)
>orf02978 
VDKTDKVSSKHRFEVADRTDEVSSKHRFEVADRTDEVSSKHRFEVADRTDEVSSKHRFEV
ADRTDEVSSKHRFEVADRTDEVSSKHRFEVADRTDEVSNIYTARRS
(SEQ ID NO: 1602)
>orf02989
   MSCNCAFYRSQFFDVNSVSNYHSHQKELRFPNSILFTYFVKVT (SEQ ID NO: 1603)
>orf03007 
MTVKHRIVNSNGEARSGESLTTCGTRSTHGNWATVGYVYAADMARQNWWDLSAAISGSWS
PN (SEQ ID NO: 1604)
>orf03009 
MRWDYGQIFKEIRKSKGLTQQDVCGQVIHRTTLTNIEHGKVIPSFENMVFLLEQIDMSLA
EFKYICNEYHPSKRRDIIVESQNPSTFQDTRKMVELTEKCQKYLKTHHDVPIQNIYRHTK
IVTELRTKGFKNNHVLKDLYEEIWDYLEPMDTWYISDLKLLGTILFFFPSENLPLLIDRI
MKTIEKYKYFRETKAFLSSFLANLSTVYFQHHLFKECETITLQLLVLAEELKIYDILGFS
QVRLGILQHNSDLIDKGITLLRLTKEEALVKILEKEINDFSNL
(SEQ ID NO: 1605)
>orf03014 
VGLIKLTSYVFVCISNSFLTRHDKNDNICFFHGNFCLVLDLFHERSIDIINSSCINHAKR
TIEPLTRCINTVTCHSFDIFYNGDSLTSDPIK (SEQ ID NO: 1606)
>orf03016 
LSSKSCIDRTNQETFHTLGLEGVGMKSGSLFCSVQISDKEKENSRLANGFLRYQFIQGIF
LLLTSYHNHRVGLEILPR (SEQ ID NO: 1607)
>orf03049 
MTESYTWVEADRATLSRYRHGQGHLTDQFFSFKVQRPAAKTLIASISTGKGMGPSFDGTP
VITSGNQNRINTIKNSFIMSSSSVRISLRKLTSQRNFLRNLSSLILLAAQVAKGDATACS
HQRISRVVGQDSHETLSLTEFF (SEQ ID NO: 1608)
>orf03050
   MNINNEKVWFAFYLLDMQITRPTPTFNDRRIGLIGKLQELRFLAGNLLLR (SEQ ID NO: 1609)
>orf03051 
LIKGYLPNHLALMDLCSKTTCTLDDFAGIAGRRNHRGFFCHIGNGVFLTVDKYLRNQRIR
QRKSSHHILTQLVCHSHTHLFILLQTSLSLRTKERLSF (SEQ ID NO: 1610)
>orf03057
   LIKLTDRNFSDILIKCLIKCFTNLLSNQLMLLPSTLKL (SEQ ID NO: 1611)
>orf03060
   LFKGGVTISRTPLSSEDTVMIDATEVQINCPKKTISE (SEQ ID NO: 1612)
>orf03062 
MIQSENHCSASHSNRDYQSQHDNQGRTCQCFIIVPCHKKGSCSVGEITWNQRCQNGQDKD
HSRCLIKNT (SEQ ID NO: 1613)
>orf03069
   MIARQLMVFFSTNQADTRITNMSIDSLIINNSKDFQSSSHASVSFILTKLVNLLIFNF (SEQ ID NO: 1614)
>orf03070
   MGEPFTHFIDCIDLGINPSYTQVCDRHFTSDIPCTMTSHPIS (SEQ ID NO: 1615)
>orf03077 
LSSDSHFIGIKAFVILILGKSNSIVLRIVGLYQDLTCFFSPTCSTCHLSQELEGSLRRTE
IRQIQGRIRI (SEQ ID NO: 1616)
>orf03078
   MAVHSLGIHMQGQRNIAVGTSIHRPTLPTHDKARITTAIEHENHLLFFNQTVLDSL (SEQ ID NO: 1617)
>orf03079
   MVTGIAVLLISHFMLFINNHDTQIFQRSKDSRSGTNNNLGIATLHLAPFIILFTIG (SEQ ID NO: 1618)
>orf03080
   VKNGYLVPKTCYKTLGHLRSQGNLRYQQNSCLALIQGTLDNLQVMLGLPTSCNPLK (SEQ ID NO: 1619)
>orf03081
   MVMLIPRLGLDLLLIDCLIFQTKQAFSSQTHHFSLLGKV (SEQ ID NO: 1620)
>orf03082
   LGLQTKMNPLNQAIPLTKRHMNPHPNFQHSLKFLRNPVTIGLVRLHQGHIYDNLS (SEQ ID NO: 1621)
>orf03085 
LGNHFCTICSTTYQAILQFIQIWWCQEDKDSIWNLFLDLKSTLNFNFKENIDSLVQGFID
IGQRSSIVVADIFCVFQHLSLTNQLFKFFTSTEEIVNTVHFSRTLCACRHRYRILKLVFR
TLKNLSSNRSFSNP (SEQ ID NO: 1622)
>orf03092 
MKFKNYLFDLYPYFPKGFSLDNREDPDVCSKALYDDLCKMFFDDDSKEKLKITSVCNKCQ
NYGNRDYYTLFIDKDKYLLSSDYIGASIYWAQEAGLNDRIILDHLSISRTIGGHILFPRG
GKLETVNQARGGEKGYYDRFDLTLYAIKEWFVENKNTKIGYAIENYHEWFELFSGDDNCK
NGFENFVEFFKLEGFIYEQNKIIDLIKSDLENNQVVFLDKEDILIASTEEEYIRYMKNLN
IIILERTKKILL (SEQ ID NO: 1623)
>orf03093
   MELSIQLIHDLNTHTTHSTAKMLHNVKAIKNDFSIRE (SEQ ID NO: 1624)
>orf03096 
LKAKLFSQVIVRAKHIHSNNFDATSDSSIVAKKVISNDSLFSSLKNSDDIEIVKILRNEA
HLSLCKSILIPRHNLRKTRKIMFKVKIKEQTRKLAAGCT (SEQ ID NO: 1625)
>orf03113
   VGCSYICHELVANHDHFLFVIVEFLHGTVNTKCEGLQGPVNVINPKFLNCSLNAFFGVI (SEQ ID NO: 1626)
>orf03114
   LLHLWRSIRVVPSNEGIIQIDQNSLDSLRLQAWDCQIIDCFHSKIWYIIFNRHSGSFS (SEQ ID NO: 1627)
>orf03118 
VKGLLLATKLCRTNSHTDNLTRYSNRSICQNDLISHIQLTFKEDEKAIDDIRQKALGSHT
NRYPSNTSSSQQTRNWQT (SEQ ID NO: 1628)
>orf03120 
LWGILGTLPNLSGIGLLGDLFVGGLKAVAPILVFALVANALSQHQKGQDSNMKTVVFLY
IL (SEQ ID NO: 1629)
>orf03121 
MIGTFAAALVAVLASFIVPIEITLNSANTEIAPPDGIGQVLSNLLLKLVDSPVNALLTAN
YIGILSWAVIFGIAMREASKNSKELLKTIADVTSKIVEWIINLTPFGILGLVFKTISDKG
VGSLANYGILLVLLVTTMLFVAPVVNPLIAFFFMRRNPYPLVWNCLRVSGVTAFFTRSSA
ANIPVNMKLCHDLGLNPDTYSVSIPLGSTINMAGVAITINLLTLVTVNTLGIPVDFATAF
VLSVVAAISACGASGIAGGSLLLIPVACSLFGISNDIAIQIVGVGFVIGVIQDSCETALN
SSTDVLFTAVAEYAATRKK (SEQ ID NO: 1630)
>orf03124 
MKIKEQTRKLAAGCSKHGFEVVDRTDEVSSKHRFEVVDRTDEVSSKHRFEVVDRTDEVSS
KHCFEVVDRTDEVSSKHCFEVVDRTDEVSSKHGFEVVDRTDEVSSKHGFEVVDRTDEVSS
KHSFEVVDRTDEVSSKHGFEVVDRTDEVSSKHGFEVVDRTDEVSSKHGFEVVDRTDEVSS
KHSFEVVDRTDEVSNIYTAR (SEQ ID NO: 1631)
>orf03145 
MKDLISVIVPVYNVEPFISSCLDSLSKQIYQNFEVLLVNDGSTDNSGAICREYADRDSRF
HYFEKENAGVADARNFGIERSKGDYITFVDSDDWVTEEYLSILIETLKEQHSEIVVSTYS
TYNESDGLFYIHVFDSDYYVKNYNSKLLMEELPLLERYDMSFLTSWGILFKRELFQEVQF
PFGRVCEYIGTNYKLFMQVEKVTYINKVLYWYRVGKEGLSNSYSPKMMRDDCDFRLERIA
VLALKGYDVSKYLDQMKFYLKYRHDIAIQRELKENVETRHLEMLDYLLNGNKYN
(SEQ ID NO: 1632)
>orf03148 
LNVRGGAYITFVDSDDWLEHDALDRLYGALKKENADISIGRYNSYDETRYVYMTYVTDPD
DSLEVIEGKAIMDREGVEEVRNGNWTVAVLKLFKRELLQDLPFPIGKIAEDTYWTWKVLL
RASRIVYLNRCVYWYRVGLSDTLSNTWSEKRMYDEIGAREEKIAILASSDYDLTNHILIY
KNRLQRVIAKLEEQNMQFTEIYRRMMEKLSLLP (SEQ ID NO: 1633)
>orf03167 
VLAKAEAEALVDADSDADVLADTEAEALVDAEAEALVEADAEALVLAEAEALVDAEADAL
VEAEAEALVDADADALVDADSEALVDADSDALVLAEAEALVDADSEALVLADSDALVLAE
AEALVDAEADALVDAEADALVLAEAEALVDADSEALVDAETEALVDAEAEALVDAEADAL
VDADSDALVDADSDAEVLAEADALVDAETEALVEADSDAEVLAEADALVLAEAEALVDAE
 
ADALVDAETEALVEADSDAEVLAEADALVEADSEAEVLAEAEALVDADSDAEVLAEADAL
VDADSEALVDAEAEALVDAEADALVLAEAEALVDAEAEALVDAEAEALVLAEADALVDAE
ADALVDAEAEALVDAEAEALVDADSDALVDADSDAEVLAEADALVDADSEALVDAEAEAL
VDAEADALVLAEADVLALVDADSEADVLAEADALVDAEADALVDAEAEADVDADSDAEVL
AEADALVEAEALVLAEAEALVDAETDALVDAEAEALVDAEADALVLAEADVLALVDADSE
ADVLAEADALVDAEADALVDAEADALVDAEADALVLAEADALVLAEADALVLAEADALVL
AEADALVDADSEADVLAEADALVDAEADALVDAEAEALVLAEAEALVLAEADALVDAEAD
ALVLAEADALVDAEAEALVLAEAEALVLAEADALVDAEADALVLADSDALVDAEADALVD
AEADALVLAEADALVDADSEALVDAEADALVDAEAEALVLAEAEALVLAEAEALVDAEAD
ALVDADSDALVDAEAEALVLAEALVLAEADALVDAEADALVLAEADALVDAEADALVLAE
AEADVDADSEADVLAEAEALVDAEAEALVLAEAEALVNAEADVLAEADALVDADSEALVL
AEADALVLAEAEALVDAEAEALVDAEADALVLAEADALVLAEAEADVDADSDALVLAEAE
ALVDAETEALVDAEAEALVLAEAEALVLAEAEALVDAEADALVLAEADALVDADSEALVD
AETEALVDAEAEALVLAEAEALVLAEADALVDAEADALVDAEAEALVDAEADALVLAEAD
ALVDAEAEALVDAEADALVLAEAEALVDADSDALVDAEADALVDAEAEALVDADSDADVL
ADTEAEALVDAEADALVLVDADVLALVDADVLADVLALVDADVLAEAEALVLAEAEALVD
AEAEALVDADSDAEVLAEADALVLAEADALVDAEAEALVDAEAEALVDAEAEALVDADSD
ADVLAEADALVDAEADALVLAEADALVLAEADALVDADSEALVDAEAEALVDAEAEALVD
AEAEALVDAEAEALVDAEAEADVDAEAEALVDADAEALVLAEADALVDADSDADVLAEAE
ALVDAEADALIDADSEADVLAEAEALVDAEADALVDAEAEALVLADAEALVDAEADALVD
AEADALVDAEADALVDAEAEALVDAEADALVLAEAEALVDAEADALVDADSDAEVLVLAE
AEALVDAEADALVDADSDAEILAEADALVDAEAEALVLADSDALVNAEADVLAEADALVD
ADSEALVLAEADALVLAEAEALVDAEAEALVDAEADALVLAEADALVLAEAEADVDADSD
ALVLAEAEALVDAETEALVDAEAEALVLAEAEALVLAEAEALVDAEADALVLAEADALVD
ADSEALVDAETEALVDAEAEALVLAEADALVDAEAEALVDADSDADVLADTEAEALVDAE
ADALVLVDADVLALVDADVLADVLALVDADVLAEAEALVLAEAEALVDAEAEALVDADSD
AEVLAEADALVLAEADALVDAEAEALVDAEAEALVDAEAEALVDADSDADVLAEADALVD
AEADALVLAEADALVLAEADALVDADSEALVDAEAEALVDAEAEALVDAEAEALVDAEAE
ALVDAEAEADVDAEAEALVDADAEALVLAEADALVDADSDADVLAEAEALVDAEADALID
ADSEADVLAEAEALVDAEADALVDAEAEALVLADAEALVDAEADALVDAEADALVDAEAD
ALVDAEAEALVDAEADALVLAEAEALVDAEADALVDADSDAEVLVLAEAEALVDAEADAL
VDADSDAEILAEADALVDAEAEALVLADSDALVNAEADVLAEADALVDADSEALVLAEAD
ALVLAEAEALVDAEAEALVDAEADALVLAEADALVLAEAEADVDADSDALVLAEAEALVD
AEADALVLAEADALVDADSEALVDAETEALVDAEAEALVLAEAEALVLAEADALVDAEAD
ALVDAEAEALVDAEADALVLAEADALVDAEAEALVDAEADALVLAEAEALVDADSDALVD
AEADALVDAEAEALVDADSDADVLADTEAEALVDAEADALVLVDADVLALVDADVLADVL
ALVDADVLAEAEALVLAEAEALVDAEAEALVDADSDAEVLAEADALVLAEADALVDAEAE
ALVDAEAEALVDAEAEALVDADSDADVLAEADALVDAEADALVLAEADALVLAEADALVD
ADSEALVDAEAEALVDAEAEALVDAEAEALVDAEAEADVDAEAEALVDADAE
ALVLAEADALVDADSDADVLAEAEALVDAEADALIDADSEADVLAEAEALVDAEADALVD
AEAEALVLADAEALVDAEADALVDAEADALVDAEADALVDAEAEALVDAEADALVLAEAE
ALVDAEADALVDADSDAEVLVLAEAEALVDAEADALVDADSDAEILAEADALVDAEAEAL
VLADSDALVDADSEALVDAETEALVDAEADALVLAEAEALVDAEAEALVLAEAEALVLAE
ADALVDAEAEALVDAEADALVLAEAEALVDADSDALVDAEADALVDAEAEALVDADSDAD
VLADTEAEALVDAEADALVLVDADVLALVDADVLADVLALVDADVLAEAEALVLAEAEAL
VDAEAEALVDADSDAEVLAEADALVLAEADALVDAEAEALVDAEAEALVDAEAEALVDAD
SDADVLAEADALVDAEADALVLAEADALVLAEADALVDADSEALVDAEAEALVDAEAEAL
VDAEAEALVDAEAEALVDAEAEADVDAEAEALVDADAEALVLAEADALVDADSDADVLAE
AEALVDAEADALIDADSEADVLAEAEALVDAEADALVDAEAEALVLADAEALVDAEADAL
VDAEADALVDAEADALVDAEAEALVDAEADALVLAEAEALVDAEADALVDADSDAEVLVL
AEAEALVDAEADALVDADSDAEILAEADALVDAEAEALVLADSDALVDADSEALVDAETE
ALVDAEADALVLAEAEALVDAEADALVDAEAEALVDADSDAEILAEADALVDAEAEALVD
ADSDAEILAEAEALVLAEVDALVEADSDADVLALVDADVLALVDADVLADVLVLVDADVL
AEAEALVLAEADALVDAEAEALVDADSDAEVLAEADADALVDAEAEALVLADAEALVDAE
AEALVDAEADALVDAEADALVDAEAEALVLADAEALVDAEAEALVLAEADALVDADSDAL
VEADSDAEVLAEAEALVDAEADALVDAEAEALVLAEAEADVDADSEADVLAEADALVDAE
AEALVLAEADALVDAEADALVDAEADALVDAEAEALVLAEAEALVLAEADALVDAEADAL
VLADSDALVDAEADALVDAEADALVLAEADALVDADSEALVDAEADVLVDAEAEALVLAE
AEALVLAEAEALVDAEADALVDADSDALVDAEAEALVLAEALVLAEADALVDAEADALVL
AEAEALVDADSDAEVLAEAEALVDAEADALVDAEADALVLAEAEALVDAEADALVLADSD
ALVDAEAEALVDAEADALVLAETDALVDADSDAEVLAEAEALVDADVLADVLALVDADVL
ADVLALVDADVLADVLALVDADVLADVLALVDADVLAEVDALVDADVLADVLAEADALVD
ADSDAEAL (SEQ ID NO: 1634)
>orf03175 
VFEVVDKTDEVSSKHCFEVADRTDEVSLKHCFEVADRTDEVSLKHCFEVADRTDEVSNHT
YDKVKLTWFEEIFEEYHTKKPCSSR (SEQ ID NO: 1635)
>orf03178 
MYQDLLRKIAEEKPNYNQEEIQWLLDHLGDPSPEIRDDLVFTSFARGIQEELFTQEQFHF
IAEGVSSDGGLDKEIDKIGLPTLERSFRALIYATLLSDDANQQSIFYQRLKAEIRNVLLN
QGLHYLSKEKDTTGFSSQYGWVHSFAHGADLLKEVVCHPDFPKNRVHEVFDILGQLFKRM
SIRFTDDEDWRLARVIYEPILQGKLEQEQVASWIKTVDFPIEEREDFSKFSNFRSCLVEV
YVQLDQRNSLQDELKEAIQSFQY (SEQ ID NO: 1636)
>orf03181
   MGFKVSHFKIPSSHLSINVLRTIENFTEIGQGLLHISP (SEQ ID NO: 1637)
>orf03182 
VGFFDFGLTNSCRQVRQFTQTVQDFLVCYHQGIVKEGQGYAGICFKFHPSLGNIGKFVIA
IVRRLRHKSIVANMAHLNVDLFQFRKGLLEILKSVKIALVITAKLVDVFTSFLDCTQEIL
TVLV (SEQ ID NO: 1638)
>orf03190 
MNITYIVGNGLDLQYGLKTRYRDFYEFQNKVYISRTENEEKYSNFIYESLFSDKVNDYEN
WSDFELSIGKLTKDNDLISSSIEIKEKFIDDFSEVVDDLREYLRIQQEKNLEKGNAIDFI
STLDDMRTSLPVINQPAIDKKYNENPHQDDIVNIVTLNYTHVIDKLYNGSAKSFRNQLRA
NLYNFYIEPPIHAHGTVDVCTVLGVSDEIQISNSLEEQKESLIKSLVLKNYRENMDVKNS
DIIKNSDIIILYGVSLGETDRYIWSQIAERSISGSVPVIIYHYVPHFDPGNPIRAKRLYR
NVEDKFIQNSGIDLELEKKLRDNLIVVIGKTIFDLIER (SEQ ID NO: 1639)
>orf03191 
MNTLLTLRGKSFTQKSRNNGMGPITIPKKTIITLEHLKYLHFSLEETKTYWEKNNIIDGI
LISIYYNRIVAKSNRINGYFNVGGGNPFPNDTIVGAKFNDEKTKHIVTHYISRDALNKTI
TVLSKIIEVFEEHFDRAITCEMFSDSSTFASINFSEYGISKSKFQQYLRDSCFIENFGVE
HTTVSDIQNSIVTFYDVHTDIFRLLNKLNIDISEANIMNQTTVLLDEKNIELLLSKAPYL
VSMIVEDFSKLSVDDFSLDNNDLKINLPSPMNEPVVGVIDTLFDKRVYFNEWVEYHDFVS
PDISKDSQDYKHGTAVTSLIVDGANLNPNLDDGCGNFRVRHFGVSLQSGFNSFTIIKQIK
EIVSQNADIKVWNLSLGSNDEIRENFISAEGALLDEIQFENDVIFIIAGTNASVINGKRK
RIGAPADSLNSIIVNSVDFNNQSVSYSREGIVLSFFVKPDVSYYGGGNGDFINVCEPLGL
GRVAGTSFAAPFIARKMAYLIHIMGLSREEAKALLIDAAIPWNDKKTFTDLSLIGNGIVP
IKMDDILSTPDDEIKFIVSDISRAYDTYNYDFPVPISSESYPYVAKATMCYFPNCSRKQG
VDYTNTEMQLTFGRLKSDGIKSINKDNQHAEDTPGYVRENAARNIFRKWDNVKHIGESFT
SRKRAKAILNPSNPQWGMSIKTIERLKSGDGQGVRFGVVVTLKELNGVNRIEDFIQQAEL
RGWLVNRLQVEAQVDLFNSLNEEIEFE (SEQ ID NO: 1640)
>orf03192 
MKKSDVLDLIKYHYEGRETEFRNQSIAIARNFNKHGDTQIAQYIMGLMSQSDRFMPQIEN
PSEYLTPAKLDIGPLPLPLSIMNDLKGIINAVNHHIGINKFLFVGSPGTGKTESVKQVAR
LIGKELLVVDFSHLVDSKLGQTVKNLATLFNEINNLPFKQNYIILFDEIDSIVLDRVNQN
DLREMGRVTSAFLKELDRLSPEIVLIATTNLFENLDKAVTRRFDAIIDFDRYTDEDKVEV
ATIILNELLKQFKNVARDLKLFKKIINSANVIPNPGDLRNSIRTSLAFSDPSDPHDYQKR
LLRSLHNGRNLSISKLSKLGFTVREIEILTGISKSSVSRELSED
(SEQ ID NO: 1641)
>orf03200
   MINSQVFEIRIFNSYYKDAIYYFKNINYSIFHIFSTHY (SEQ ID NO: 1642)
>orf03205
   VGHRFDPCRGHLNTTGKALEPRFFCLNKIFFKFFRKLA (SEQ ID NO: 1643)
>orf03206
   MGWKGTPPCLHPSNQDTTILIVQQCLRRIEVLAMINFLN (SEQ ID NO: 1644)
>orf03207
   VFGSYYRVIASIFFKEFWITEISSNQLIWQVCSSYNWILGNLFKVNPVI (SEQ ID NO: 1645)
>orf03208
   VLPSHQVLTFSMSPVHRSPNTIIMIELIKEMVFSTKINKSIWIIDPTNLS (SEQ ID NO: 1646)
>orf03219
   LLRFAIHSNLLFLKRFIFSIKDSAWRCLFISFFETFHFCFKNKSFYLNSFYH (SEQ ID NO: 1647)
>orf03230 
LIVSLKTKSRKAKDMAESIQGWLAQFLVNLFKSITFDCGKEFSKWKDISNHHDSESFFAN
LECPRQRCLNEHSNRLLRCHDLPKQTDFNEVSQEF (SEQ ID NO: 1648)
>orf03231 
VVEIIYFLIIIIASGLGSISGMGGGIIIKPLMDSFGYHSVSDIAFYSSFSVFIMAIISTT
KRFSQSKEIKWRLIFTVSFSSVLGGFLGHLIFQVLLSQLSVRLVSIVQMILLFVMLLVSF
VLTDFKKTYQFDKIGFYMICGLLLGLISSFLGIGGGPLNVSLLMVFFSISIKEATMYSLA
IIFFSQLSHLATIVVVTGLNQYHLAPVPVIFLASICGGVLGTVVSKVLPENWVRYCFKGM
LFFVMGMTLYNLFHIL (SEQ ID NO: 1649)
>orf03232 
MMGTNSEEGFLDDFEGPQVAVSVKDFSIADTPVTNQEFAQFVKETGYKTLAERQEWSFVF
ILFVPEAEREGYPHPAGAPWWLQVSNACWKHPYGENSNLVGLEDHPVVHVALEDALAFCN
WSGMSLPTEAQWEYAARGGRQSEYPWGDTLLEGGYYHANTWQGRFPYENTALDGFIGTAP
VYEFLPNDFGLYQMIGNVWEWCRNPRYTLLASFNEDDYELPKYGIQDEEYAIRGGSFLCH
CSYCNRYRVAARNGCISTSTSSHLGFRCLKE (SEQ ID NO: 165)
>orf03235 
MVQTKQPNIILIVVDQMRADALSLNSKDKLVSTPTLDMMASVGYNFENAYSPVPSCVPAR
AALLTGLDQDKSGRVGYQDEVPWNFTNTLPKVFKDMGYQTECIGKMHVFPSRQRLGFDHV
LLHDGYLHVDRKYDKAYGSQFDYASDYLAFLKGKVGYDVDLIDDGMDCNSWEARPWDKDE
KLHPTNWVVSESISFLQRRDPTVPFFLKMSFEKPHAPLNPPKYYFDIYMERLPQFLDLHI
GNWEVLEKQIPSIYALRGKLKEDDQRRMVAAYFGLITHIDHQISRFLTALKEFRHDKDTI
IWFVSDHGDQLGEHYLFRKGYPYQGSIHIPSFIYDPAGLIAGNRGTIKQLVKIQDIFPSL
VDLAGGTTTDELDGRSVKNLLFGQYEGWRTEFHGEHALGKDSSQYILTDQWKFIWFPVLN
HYQLFDMKKDPHEMNDLYPSEKYQPIVRQMKKKLVDFLRYREEGFVVDEELVPVELSKIT
PTLTKTGDSQS (SEQ ID NO: 1651)
>orf03237 
MNTMLDKMQEKLSPIAMKVGNQKFLVALRDSFVGTMPVIMTGSIALLLNAFLVDLPQQFH
LESITKTFQWLVDINNLVFKGSIPIVSLLFIYCLGVNIAKIYKVDTVSAGLVSLASFVIS
IGSTVTKSFPLANVGDVKLDQILQGIDNLAFDGKNLMVTIGNVIPGNHINARGYFTAMMI
GFLASIIFCKVMKKNWVIKLPDSVPPAIAKPFTSIIPGFMAMYIVAILTYVFHLLSNDLL
IDWVYKVLQTPLLGLSQSFFAVILMIFLNKLFWFFGLHGGNVLAPIMEGLFGVAMLANLD
AFQKGEPIPYIWTSGSFGAFVWFGGLGLVLAILIFSRNSHYRKVAKLGLAPVLFNIGEPV
NYGLPVVLNPLLFIPFVLSPVFMATVAYWATSWGLVSPVTQNVTWVMPPILYGFFSTAFD
WRAIILSVVCLIISVLTYFPFVKMADKTELS (SEQ ID NO: 1652)
>orf03238 
MNESNLESAMGLIMYGGEAKSNAMEAIQAAKKGDFSKANRRLADANAALLQAHKAQTEML
TREAQGEETSISLLMVHAQDHLMTSLTFVDLAKEVVEVYERFEKN
(SEQ ID NO: 1653)
>orf03239 
MAKVTIMLACAAGMSTSLLVTKMQKAAEDKGLDAEIFAVPAPEAEEIVATKEVNVLLLGP
QVRYLLGDFQEKLKDRQIPVAVIPMTDYGMMNGSKVLDLAESLLD
(SEQ ID NO: 1654)
>orf03240 
MKRLISANPSEILQMNAEELKQSILASEGRVVLSENVVTRETFVGDITNSEIARAFGADM
ILLNCVDVFEPKIYALDSSGDDVIHRLHQLVACPIGVNLEPIDPSAKMLEETQEIVAGRV
ASVETLKRIEELGFDFVCLTGNPGTGVSNREIIKAVQTAKENFSGLIIAGKMHGAGVNEP
 
VAELSVAEQLLEAGADVILVPAVGTVPAFHDQELREVVDLVHSKGGLVLSAIGTSQETSD
TDTIKEIALRNKICGVDIQHIGDAGYGGLATVDNIYALSKAIRGVRHTVSRLARSVNR
(SEQ ID NO: 1655)
>orf03241 
MEKLLQEKLLPVAARLGNNKALVSIRDGITLTIPLLLIGSLLMVIASFPIPGWEQYLGDI
GVADYLWKGVDSSFGLLGLVASFGIAYFMARQYKVDGIPAGIVSLSSFITVTPFIRGEAG
AGMPTAFMASKGLFVAMILGLINGYIYQWFINHNIQIKMPDGVPPAVSKSFSAIIPGAVT
IVGWLIVYATLDKLSLPNLHEIAQVALGGPLGLLGNNVIGLLILIFLNSSFWFVGLHGGN
VVNAVMKPLWLANLDANKVAYQTGETLPNIFTSVFMDNFVFIGGGGATIGLVLALGYLAH
KKKASKQLKTLAPITVIPGLFNINEPAMFGVPIVLNILLLVPFILAPMFNLLVAWGAMAS
GLVPLTYTDPGWTMPPVISGLLATGSISGSLLQIVLIVLDVLLYLPFVIAIEKRFKLLED
(SEQ ID NO: 1656)
>orf03242 
MTLSKKQLQLRAKILETVYTLGPISRIEIATKTGITPATTSSITNDLIKENILLELGEDE
HDTSVGRKKILLDIQAKRFYYIGCELSEKHFTFALGDNLGNILKEEKEIVTKQLIQEKGN
QLINQTLKQFLNNCSDYEIEAIGIALPGRYLDDYKITTNNPLWQHIDLEMIQSHFDKPLF
FSNNVNCMAIGKRLFSRQQNDPNFAYFHFARGMHCSYIYDGNIYGKGNLMIGEIGHTVVS
SEGEECSCGRKGCLQTFAGESWLIKKSKILYHQSPYSLLPSLVKNADDIDIQVILTAYQL
GDTGIITLIHQALLYLSQTILNISMMIDSQKIYLHSPLLTNQHIIQKLYSEMNYKPKLLY
NRLPEVIIEPYNDFTAAHSAIALCLYHTILHS (SEQ ID NO: 1657)
>orf03243 
MTIRFEEKVSTENAQFVCQWSNSLGKVFQEQWIGPRIPFPLTIQVFQDLEGILSIFEGQE
FVGLIQKIRLEDSNLHIGRFFINPQKQGQGLGSQALRKFVSLAFENRDIDSISLNVFEAN
QRAQNLYQKEGFEIV (SEQ ID NO: 1658)
>or03261 
MPFKENLICQHRNHHCSVFFISLGLLHNIHIEIDISQTRASFLDLSDYLQAVLMILQKFC
QAIGLAQRLDLLQLHLLHLTRLLL (SEQ ID NO: 1659)
>orf03271
   MYLLLLVVKDHIALIDKEMHVWRPNCILRDLTNFFIKRNHIVTHKTNGSTTKR (SEQ ID NO: 1660)
>orf03272
   VLTLMNHFIKEIQGIPINHLTILIENSIFKLNLKNWIIG (SEQ ID NO: 1661)
>orf03274
   VDRTDEVSSKHCFEVADRTDEVSNHTYDKATLTRFEEFFEEYKGVPR (SEQ ID D10: 1662)
>orf03293
   VCQRMDARTCKTTIIAVHNVLTALQQTWIAVQLYQTK (SEQ ID NO: 1663)
>orf03294
   LHLGKSILSLPVKGKDLEFLVHLFVINHMIGFPSRTSTFCRCKVLNSME (SEQ ID NO: 1664)
>orf03295
   LEQTVIIANNPCELYWDNHLSFLSDSLLKQVIVHLKRICLDIHHDRGCSHVRNDTT (SEQ ID NO: 1665)
>orf03297 
LTDDGVLILVVDAGWRGNSCLQEQGCHHFRAILLCITWHFRSCTDKGHLTFKDIDQLRQF
VQTDTSDEISNLGNTAIVSRSHQTSFFIRIRHHGTELFNLEPTVVLGHTLLLVNHWPLAI
QLDPNAQDEKDGRS (SEQ ID NO: 1666)
>orf03298
   MLKMRKMGEVRTSKTKAKTQSKQRLKISEPFLLETSW (SEQ ID NO: 1667)
>orf03313 
MLEEGTKDQLAELTYPFGRGVNLSFGIKDVPKLYQKVMEANYPIYRLLTKRKFRVSDPYI
YPHKFAVLDPDGYFLRFSE (SEQ ID NO: 1668)
>orf03316 
MDQNLFNYNDEDIDSVIEYSHKLLNRKFSDVMEEYNRSLYKSYDDYNDRVVSEVQDKAIS
MKSKGQYGNYIEKYFYGYQPNSDSEADFEKIGVELKVTPFKINKNGTLSAKERLVLTILN
YMEENLEDFYSTHLWKKCAKILLLFYNGLIPNQTMKDYVIEKIFLYEWFEEDMAVILEDY
QKITDKIKNGKAHELSESDGNYLSTCTKGAGKGKDLRQQPFSHELAKQRAWELKSSYMTY
LINHKIFNQSDQESVLANFRGEKKSFTEVIAEKILSYKGFSEQELYDRFEVNSKAKGKNS
TLIRKILGLTGDLDKTKEFQKANMNLRVIRVDKNNLPKEDSPFKTYCFKELAATDSWESS
HVYNEIYNKRFLFVIFKEIEPKLFVLDSIKFWGFQDRQLEEIQRVWQETRQIISDGVKLT
QNGNKVSTNFPQSKINKILFTKLHATNTYYEIDKGKFVGKGSLSDTDELPDGRRITKHSF
WMPKKFIKEILDGNWD(SEQ ID NO: 1669)
>orf03317 
MKVLELFAGVGGFRIGLENADKQLFKTKWANQWEPSRKSQDAFEVYDYHFPNSKNINISI
SDITDEQFSKMDADMIVGGFPCQDYSVARSKKNEKGIEGKKGVLFWEIIRATEIIKPKYL
ILENVDRLLKAPSKQRGRDFAIMLTAFNNLGYSVEWRVINAAEYGRSQRRRRVFFFVYRN
DTVFAQKIDNLYEKNEEIFEDNRYDDYIFNQGLFAKQFPIKPIAVKNRHVFYELPNDIVE
VSDTFTGTVWNTGIMRRGKYYSIDTEPNYNGNPITLGEILQDESEVPEKYFLTDQSKLEK
FQYLRGPKKIERTSSDGHQYIYSEGGMSPYDDLNLPGRTMLTSEGTVNRSTHLLFVNNKY
RLITPIEAERLQDFPDDWTAKKKLSDDSIVEVSDKMRMFFMGNALVTEIVKEIAKFIKEI
D (SEQ ID NO: 1670)
>orf03318
   MDTFSFNGQYIVEFSCLKVVDRGLECHPIKSQRDNHQTTDLVT (SEQ ID NO: 1671)
>orf03320
   MGIAIVVERRVHYFGRHHNVTISHFFNFVIFKGRYSVKMKVFHRFLLIFQTTL (SEQ ID NO: 1672)
>orf03333 
MIACRHDICKSQKGLEHPFCIIRRLTRDFNQRPVCIVEANIFCLKITPQIITNMIVARTV
KSSKTGITLTTSMCKRDNHKITWFHRRNGFPSFFNNPNRFVSTIFMSSFRFWITVPP
(SEQ ID NO: 1673)
>orf03341
   MLRQFRLGFFDVRMTECHLKWKERENFHDFLKFYCKDS (SEQ ID NO: 1674)
>orf03350 
MNMNKDQIAILNGADNLNLTLWMTLKEICKEGCKSFFPVRNTCRMLDIGIPYRLGLSLSN
SSVLNGMDV (SEQ ID NO: 1675)
>orf03370 
MHKLRIFVNQLCRRFGIILGPFLVLGFQVLTQELELAIFFDLREEVLLQVIPQVCHFCYL
RKEFTTLNQHELTSHDHVLTRHFQTHGLQG (SEQ ID NO: 1676)
>orf03382
   MLHMNLFFQPFFTNLCKTLATGCCVKTVMEWSSIATTIDFKIIE (SEQ ID NO: 1677)
>orf03383
   LDNRAKEWIMSTAQNQAIHLSNQGTQGFIDHLLGNTG (SEQ ID NO: 1678)
>orf03385 
LSLDFFPDDRSRSVTSNDNHFDILGQEKVDQLPSIFTNLLSRTGAIGRPRRISNIDDFFM
GKLAHELAHNGQAPDTRIQKTNWSIIHTVFFLVFFLIDRSL (SEQ ID NO: 1679)
>orf03394
   VSYGSHIFFASNCLKQIFGFLFKFSHLILLILVRASLI (SEQ ID NO: 1680)
>orf03397 MTSLLTLENIHKTFEAGTVNENHVLKGLDLEVEEGDFISVIGGNGAGKSTLMNILAGNLS 
VDEGDLLLAGKSIKNLSVRKRAKDIARVFQDPKMGTASRLTIEENMAIALRRGQKRGLGW
GVKEKDRIQFQEALKELNIGLENRLKVDTQYLSGGQRQALTLVMTDLMKPKLLLLDEHTA
ALDPKTSQMVMDLTQKIVEHHQWTTLMITHDMNHAIEYGNRLIMLYQGKIVVDVKGEEKK
HLTVEDLMHLFQKNSGQSLVSDELVLG (SEQ ID NO: 1681)
>orf03398 
MNFVLSSLSEGLLWSIVAIGDYLTFRILDIADMTAEGAFPLGAAVVVSQIQAGANPWLAT
LLALLAGMVAGLVSGMLHTKMKIPALLTGIVTLTGLYSINIKIMGSVPNLSLGDSATVFK
QLASLGLTNEGAVFSLSLVCFLLVCLVLTLLMKTEIGLVLRSTGDNIPMSEANGVNVDTM
KIVGYMISNGLIALCGSLFAQNDGFSDVTSGTGTIVVGLSSVIIAEVLIHDLTIGGRLLS
IGIGAIVYRLIIILNIYEIPNLDQNLVRLFNAILLALVLFAPELQKRLKIRGLKLRNE
(SEQ ID NO: 1682)
>orf03399 
MAEVDMVFVPTDNIILSTMETVKQVSIKHKVPVFGGSTEMIAVGGLYNYGTNYEELGRQT
ARMLIRVLKGEEPENIAVELPEKLELHTNQEMADALGIDISKLEGKE
(SEQ ID NO: 1683)
>orf03400 
VDELAKQGYVEGENIEIDLQNAQGEQRNLKTISQQLAESSDVVLAIARPSAQSLANTTQT
TPVIFSAVTDPVSAKLVESREHPGGNVTGTSDQSSDAISTQINLIKKVLLKAKTIGILYT
QSEPNSVV (SEQ ID NO: 1684)
>orf03401
   MQTDQRSQEEPHYQEGASDFRTTFIMKLLLRKDKTKNRLDTI (SEQ ID NO: 1685)
>orf03402
   MLPILSPFSSPVNNISEFFKIFRKFFQEAFKVFQISPTKKVL (SEQ ID NO: 1686)
>orf03412
   MINVNQVSIEVKNTFKNWNFTSSIELTTFSKFSQSPTMT (SEQ ID NO: 1687)
>orf03414 
LIDVLFINSFIGRICFYCYRRIHATCLFLQLFSIVILNVAHTLKHSIFIVITFISRCRNF
IIVRILLENQFSRNQGIDNRVGQSRY (SEQ ID NO: 1688)
>orf03423
   MKIKEQTRKLAAGCSKHCFEVVDETDEVSNHTYGKVKLTWFEEIFEEYKKSSWNL (SEQ ID NO: 1689)
>orf03442
   LVEQLTFNQWVTGSSPVRVIYAGLAELADAPDLGSGA (SEQ ID NO: 1690)
>orf03443 
MSIVKSHSFSISLGIFNSFWNNIHTSECFNFLCKGKSNRSNSTISVNQMVFFINIQRFYC
FAIEDFCLLRI (SEQ ID NO: 1691)
>orf03444
   LNTLLPPDNLCLFTIYLTGFSCICINSYCHNFWEIFNQLFYQLS (SEQ ID NO: 1692)
>or03451
   MGFSMKLIHDLNTHTTHSTAKMLHNVKAIKNDFSIRE (SEQ ID NO: 1693)
>orf03460 
MYNKVILIGRLTSTPELHKTNNDKSVARATIAVNRRYKDQNGEREVDFVNMVLWGRLAET
LASYATKGSLISVDGELRTRRFEKNGQMNYVTEVLVTGFQLLESRAQRAMRENNAGQDLA
DLVLEEEELPF (SEQ ID NO: 1694)
>orf03464 
MQFTRTAHHTKTLFTTKFTWENEIPFWHHSSRKRDNGFQPHTRIGSSCNDLYSLITCDCN
LADVEVVTIWMGYHLNNFTDNKLRFLIINNFFCKTFRLQLLVQTSDLLICQKDLTALCSF
K (SEQ ID NO: 1695)
>orf03469
   MILDSFFAFNCSGTMKVSTWVYDKGEWYYVSSSGSMIANDWVKDNGK (SEQ ID NO: 1696)
>orf03476 
MCTSINKKKKAVKPSFDLFFCFIFCKLTIVQVSVEATLRHQFLIVALLDDISIFHDQDQV
CISDG (SEQ ID NO: 1697)
>orf03484 
LHLRTCFVRQTNKLSPLINRTRLQFHQTILHYTLNQITSNRLGNIEFLIDIFNQDQVLVF
LAIIQKMHNLTLRPTHKFNAATFGFLLHHQVNLMTKTLKD (SEQ ID NO: 1698)
>orf03499 
MLEIWKYRPFVSEFWNDFKNNHDKQFVDSISLYLTLKDDDDPRIEEESEALENMILQYLG
EDDAS (SEQ ID NO: 1699)
>orf03507
   MAFNQFNRCIGLSIPTAPNVPGTIINRSYLHDATVPNNVREKT (SEQ ID NO: 1700)
>orf03523
   LTDFHDFKFIFFENLFKSRQLYLQSQNTVLSNLWLAT (SEQ ID NO: 1701)
>orf03533 
MKIMKKKYWTLAILFFCLFNNSVTAQEIPKNLDGNITHTQTSESFSESDEKQVDYSNKNQ
EEVDQNKFRIQIDKTELFVTTDKHLEKNCCKLELEPQINNDIVNSESNNLLGEDNLDNKI
KENVSHLDNRGGNIEHDKDNLESSIVRKYEWDIDKVTGGGESYKLYSKSNSKVSIAILDS
GVDLQNTGLLKNLSNHSKNYVPNKGYLGKEEGEEGIISDIQDRLGHGTAVVAQIVGDDNI
NGVNPHVNINVYRIFGKSSASPDWIVKAIFDAVDDGNDIINLSTGQYLMIDGEYEDGTND
FETFLKYKKAIDYANQKGVIIVAALGNDSLNVSNQSDLLKLISSRKKVRKPGLVVDVPSY
FSSTISVGGIDRLGNLSDFSNKGDSDAIYAPAGSTLSLSELGLNNFINAEKYKEDWIFSA
TLGGYTYLYGNSFAAPKVSGAIAMIIDKYKLKDQPYNYMFVKKILEETLPVKNGIKVLNI
PNVLRYDLNMLQLEYKNEQSWDSFIDNVNLIELEERIQTTIGIKQINTHNIITIAREGYS
QNYLPNTSENTYNSLQVSLVGVLLLFISMVNILWAKKSK (SEQ ID NO: 1702)
>orf03543 
VLKWCILRINHHISRKVDNFLEGTRAHIKGQAHTAWNPLEVPDVRYRSFQFDMSHTLTTN
FRTRYFNPTAVTNNSSVTNAFVLTTSTFPVFCRTKDHFIKESFTFWFQGTIIDCFRFFDF
SIRP (SEQ ID NO: 1703)
>orf03553
   MPWKELCHKLAPKVFKVIRIYSRENKKSPSHWAFCSFET (SEQ ID NO: 1704)
>orf03559
   VSVLFFCSYFSLSLEKGWFSSLISCKFMNQFLPFCWRQDSPNILTLAQDSITYH (SEQ ID NO: 1705)
>orf03564
   VTDENTRKVRLLVAFFSIVIGYILSSFFISLYHLWQEALRGLL (SEQ ID NO: 1706)
>orf03566
   LHVELIDSHKFNIGRTTCSLLSTTNICKRCQPSINHMS (SEQ ID NO: 1707)
>orf03567
   MLNTNRNELIGTSFLIFCVIYFKDLANIFRTTWNLYIIRQGHYKCQESHNQGRNDV (SEQ ID NO: 1708)
>orf03570 
VNKPILSDIDCHLTNSINLFLPDTQTGNLFWKFNGLIRLAHNHNIFRKKLSLSHFFNICY
DLFLGIGRV (SEQ ID NO: 1709)
>orf03571
   LKFSNFLGHLDIFSHDGLSLTVSLHQNSTGHATRYCFDR (SEQ ID NO: 1710)
>orf03584
   VDSLFLSLGEESNQEINLQESFSSTDCNPTLISPETTVAQGLCQDIIYRPFT (SEQ ID NO: 1711)
>orf03586 
VNPKSLGSFFLQDSKGFKELVLGHAKLSLPRIVHNVCPQFKNASRIITTRDDFWNACYSL
QMFNIFKGIQVNGRTQFTCIGVFLVWRVVGREHNLRTQKVQFMAHQKLYITRAVHTTTFF
LENFQNSWSWSSLNCKIFLKALVPRKSLVDGSCLLTNPLLIIQVKGSRELGNNRF
(SEQ ID NO: 1712)
>orf03590 
MDNLCLHNTWTDWTSIFKQAVVTEDDMTKQNDFFLGIIDAEFHNCLGNFAINESDMSKKI
TSHCVLCLVWPRQLDDLSQVMQHNPRIEQALIELRINFANSVCQTHHGRRMIGQARFKGM
VVGLGSWIGVEFLIILGVEISDNPLPDRIFNFENHLRHVVTNFLDINW
(SEQ ID NO: 768)
>orf03591
   LIDLRGIVINFSASFHVDNLTCGKGLNVMRLGIPELPINLATIILEGKG (SEQ ID NO: 1713)
>orf03604
   MDALVLQKNQETIQQIAVKIRFLDGHDYYSLIDIDNRRTNQTVFPFVNFEDIAF (SEQ ID NO: 1714)
>orf03605
   MAFFTEIPTRACLINLAITLHIVETCQGFNDLSLHLRVLAL (SEQ ID NO: 1715)
>orf03609
   MLLPLPFNTSKIKQIAMHSDLNQKEMIGHIFHDEDIF (SEQ ID NO: 1716)
>orf03614 
MKQTVKKLALVASIAATLGGGVSVASAAVQYPEGGVWTYGSGNGGAYSNYYHPSKYHSST
VVSRKTGSSDKGYAGAGGTSRAWIRTSWGEKVAFYYNV (SEQ ID NO: 1717)
>orf03643
   LVEQLTFNQWVTGSSPVRVIYAGLAELADAPDLGSGA (SEQ ID NO: 1718)
>orf03675
   MKELLNKAFFNKNKASLSKEVLLELQGKRLPVNLFLSKSLFQASL (SEQ ID NO: 1719)
>orf03690 
MMTKIKLTIDIIMPCRHITNIWIYKEEGRVNLFFYSQIFFDAIDERIIHNFNSKYHLSFF
SPVTGFSQIFDKTLACLG (SEQ ID NO: 1720)
>orf03695
   LHTSFRSSVGHSHTWHQDIVRPILFSRFNDSIVILWQNCPTFN (SEQ ID NO: 1721)
>orf03713 
MLEQARLKVEQQAIKNIQFLEQDLPKNPLEKEFDCLAVSRVLHHMPDLDAALSLFHQHLK
EDGKLIIADFTRTEANHHGFDLAELENKLIEHGFSSVHSQILYSAEDLFQGNHSEFFLIV
AQKSLA (SEQ ID NO: 1722)
>orf03714
   MKHDFNHKAETFDSPKNIFLANLVCQAAEKQIDLLSDKEILDFGGGTGLLALPLTPSQAG (SEQ ID NO: 1723)
>orf03716 
VWKKKKVKAGVLLYAVTIAAIFSLLLQFYLNRQIAHYQDYALNKEKLVAFAMAKRTKDKA
EQESGEQVFNLGQVSYQNKKTSLVTTVRTSKSQYEFLFPSVKIKEEKRDKKEEVATDSSE
KAEKKNQKRSLKRKRIPSQFNYNALNPE (SEQ ID NO: 1724)
>orf03718 
MVDLQSFFTRKYLNLNSVDAYLILPRLQGHLSYPQDFFLLQDFCFLLPIFLNLSQKEGRN
AGKDS (SEQ ID NO: 1725)
>orf03733 
MRIRNSPFDHILQTIFEFEDRTCQVTCRFEACSSICNDNWEFSQHIISVFQSPSCHTVCD
KSDVFCSFLFDKNFASLWIYVVTITDQLCIGMWQLVHGSNHTQFTVSQPTHSIVGMHPNT
RSSIDCFFGFIKSRV (SEQ ID NO: 1726)
>orf03734 
MSKSNRHTFARNCTNKVFHPITFWCKGNFIKQAICRFLPRMKLLNTRVSHISWILCPLKS
FCEIWTFIINPTNLSTCCFFIMVSKIFSDCKQLLISGC (SEQ ID NO: 1727)
>orf03736 
MQCTFNVVVHHIYTCISMNMSIHKTWGNAITCIVNHLSPFRNLLYMFPKLAVHKFQVTTS
TNSVWVEKLIRFNIVRHNVNLLKRLILQFIMSITL (SEQ ID NO: 1728)
rf03750 
MKKRMLLASTVALSFAPVLATQAEEVLWTARSVEQIQNDLTKTDNKTSYTVQYGDTLSTI
AEALGVDVTVLANLNKITNMDLIFPETVLTTTVNEAEEVTEVEIQTPQADSSEEVTTATA
DLTTNQVTVDDQTVQVADLSQPIAEVTKTVIASEEVAPSTGTSVPEEQTTETTRPVEEAT
PQETTPAEKQETQASPQAASAVEVTTTSSEAKEVASSNGATAAVSTYQPEETKIISTTYE
APAAPDYAGLAVAKSENAGLQPQTAAFKEEIANLFGITSFSGYRPGDSGDHGKGLAIDFM
VPERSELGDKIAEYAIQNMASRGISYIIWKQRFYAPFDSKYGPANTWNPMPDRGSVTENH
YDHVHVSMNG (SEQ ID NO: 1729)
>orf03763 
VLAEADALVDAEAEALVDAEADALVLAEAEALVDADSDALVDAEADALVDAEAEALVDAD
SDADVLADTEAEALVDAEADALVLVDADVLALVDADVLADVLALVDADVLAEAEALVLAE
AEALVDAEAEALVDADSDAEVLAEADALVLAEADALVDAEAEALVDAEAEALVDAXXHSF
IN (SEQ ID NO: 1730)
>orf03764 
VRRLQHRQVLQLQRQPVRRLQRQPVRQPQQAPVLRLQQVLAPQPQHQQVLRSQRQPVPLN
PHQPVHRLQQVLAPQLQHQRVLQLSMNQCVGIRINQCIGFSKY (SEQ ID NO: 1731)
>orf03766 
VRRNPHQPVHRLQQVLVHQLQHQRVLRLQQAPVRLNPHQRLPQPQQVPVRQLQQVLVHQL
PHQQVLQLQRQPAPQPQQVPVRQLQQAQAPLSQRQPVRQLQXXXFHSLIN
(SEQ ID NO: 1732)
>orf03772
   VTDENTRKVRLLVAFFSIVIGYILSSFFISLYHLWQEALRGLL (SEQ ID NO: 1733)
>orf03774 
MNXXALVDAEAEADVDAEAEALVDADAEALVLAEADALVDADSDADVLAEAEALVDAEAD
ALIDADSEADVLAEAEALVDAEADALVDAEAEALVLADAEALVDAEADALVDAEADALVD
AEADALVDAEAEALVDAEADALVLAEAEALVDAEADALVDADSDAEVLVLAEAEALVDAE
ADALVDADSDAEILAEADALVDAEAEALVLADSDALVNAEADVLAEADALVDADSEALVL
AEADALVLAEAEALVDAEAEGTGMRKLIH (SEQ ID NO: 1734)
>orf03777 
VLALVDADVLADVLALVDADVLAEAEALVLAEAEALVDAEAEALVDADSDAEVLAEADAL
VLAEADALVDAEAEALVDAEAEALVDAEAEALVDADSDADVLAEADALVDAEADALVLAE
ADALVLAEADALVDADSEALVDAEAEALVDAEAEALVDAEAEALVDAEAEALVDAEAEAD
VDAEAEALVDADAEALVLAEADALVDADSNADVLAEAEALVDAEXXIPFIN
(SEQ ID NO: 1735)
>orf03794
   MKIKEQTRKLAVGGLKQCFEVVDRTDEVSSKYCFEVADGS (SEQ ID NO: 1736)
>orf03804
   LLGSFFSWTTKELMGIIFFNNFPTVHKNNMIGYISSKTYLIKLIKNSI (SEQ ID NO: 1737)
>orf03818
   MEKILLHNLNQTEFFINKAIGWTLRDYSKTNPTWVTCFIEKNKERMAELSIKEASKYL (SEQ ID NO: 1738)
>orf03819 
MSLADLLEELEAAKDSKKARSMEAYMRHQFSFLGIAVPERNKLYKNIFQKRKKQRLSIGI
LQTLAGKRILENTNMWLLTI (SEQ ID NO: 1739)
>orf03829
   MTTGWFQVNGRWYYAYSSGALAVNTTVDGYSVNYNGEWAQ (SEQ ID NO: 1740)
>orf03851 
MAFTTEELLNLGLTEEQAKSVFALRGKELNEDKSALETIKQERDSLKSQLQKAEEQVEHL
KSLENISAEQKDAIDKLQAEYDKYKNEAAAELAQTKKVSAISLALKDTNAFNPDKLMKFI
DVDAIQIDDNGKPQIDEVINGIKESDPHLFQAEESKPSPNIFPLR
(SEQ ID NO: 1741)
>orf03853

MGSSGEMRTRPAEELGVDTFYYSMKAMARPACSPLQGQIVTKGTGREIDGITIYSLLDYG
YGTAAGCLGIHCGHYLTPFIVGVHELPNLPDYLKNLTPEQAEENARIEAGQRGLERLIKT
HKERLHYAHTLQDDKMIQAERLKVRGYQTKIRNLINQHDFLTRDYRREKLYIS
(SEQ ID NO: 1742)

In some embodiments, preferred 23F antigens are selected from the polypeptides orf01155 (SEQ ID NO: 1297), orf01305 (SEQ ID NO: 1309), orf01307 (SEQ ID NO: 1311), orf01631 (SEQ ID NO: 1343), orf01804 (SEQ ID NO: 1362), orf01807 (SEQ ID NO: 1364), orf02164 (SEQ ID NO: 1434), orf02189 (SEQ ID NO: 1451), orf02194 (SEQ ID NO: 1455), orf02219 (SEQ ID NO: 1466), orf02221 (SEQ ID NO: 1467), orf02224 (SEQ ID NO: 1470), orf02228 (SEQ ID NO: 1474), orf02242 (SEQ ID NO: 1484), orf02244 (SEQ ID NO: 1485), orf02246 (SEQ ID NO: 1486), orf02247 (SEQ ID NO: 1487), orf02652 (SEQ ID NO: 1491), and immunogenic fragments thereof.

### Example 3: Pilus adhesion

The pilus 2 mediates adhesion to alveolar epithelial cells A459. Dual-label staining shows that a pilus 2-positive strain adheres to the surface of the A549 cells and that the pilus (visualized using labeled anti-01287 antibody) is contacting the cells.

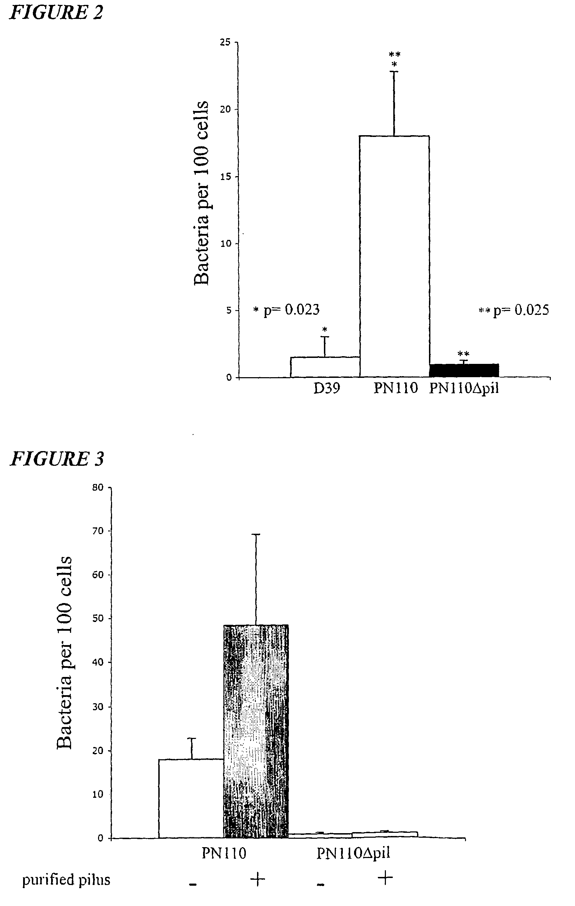

Furthermore, isogenic knockout mutants of the pilus are significantly impaired in host cell interaction. Figure 2 shows that a pilus-negative strain (D39) fails to bind to A549 cells, in contrast to a pilus-positive strain (PN110). Knockout of the pilus in PN110 removes binding.

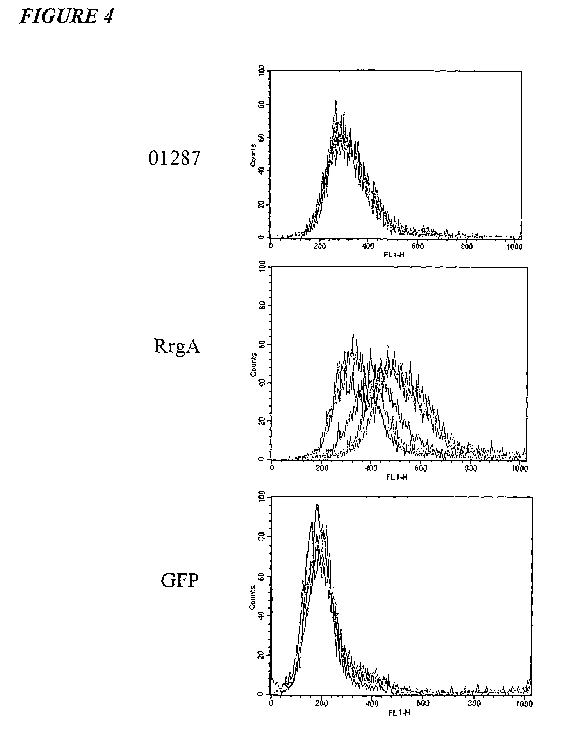

Incubation of A459 cells grown on glass coverslips with 01287 purified protein shows low level binding by confocal microscopy inspection. This observation was confirmed by incubating the protein with cells in suspension and quantifying the level of adherence by FACS analysis (Figure 4). Pneumococcal pilus 1 RrgA subunit was used as positive control comparison, and green fluorescent protein (GFP) as a negative control.

Purified pilus was imaged by confocal microscopy. It was shown to adhere to A459 cells grown on glass coverslips. Furthermore, purified pilus seems to increase adherence to the respiratory cells when added to strains expressing the pilus type 2. This effect is probably due to the interaction of the purified pilus with both bacteria and A549 cells. Purified pilus does not increase adherence of isogenic knockout mutants of the pilus 2 to the respiratory cells (Figure 3).

### Example 4: Other Sequences From INV104B

An exemplary nucleic acid sequence for LepA Peptidase (orf 01289) is hereby provided:

ATGCTGCTTAAAAAGAAACATAAGAAACCAGTAACACAAGTCAATCGGG
ATAAGTCTCCGCCGAGTGTCTGGGGAGATATCCTTTACTTAGTCAGTAA
ACTTCTGATGGTTGGATTTGTACTAGCCATCCTTTACTTTTTCGTCTTT
GGATTATTAAGATACAATGACGATGGCATGAAGCCCGCCTTAAAAGATG
GCGACTTGGTCGTCTATTATAGGTTGGATAAACGCTATTCGATTGGTGA
TTTGCTAGTCTATAGTTATAAAGGTAAGGAAAGAGTGGCGCGTGTCATA
GCAACCGAAGGAAGTACAATCGATATAAACGAAAATGGTCTCATCATCA
ACGGTTCTCCTCAACAAGAGCAAGATATCTACAAAGAAACGCTGCTCTA
TAAGGAAGGGGCAACCTTCCCGATGAAAGTCCCAGCAGGACAACTTTTT
GTCCTCGGGGACAATCGAACAACGGCTGTAGACAGTCGTGCTTTTGGAA
CCATCCCTATACAGGATACTCAAGGCAAAGTTGTAACAGTCATTAGAAG
ACGAGGCTTT (SEQ ID NO:1743)

An exemplary amino acid sequence for LepA Peptidase is hereby provided:

MLLKKKHKKPVTQVNRDKSPPSVWGDILYLVSKLLMVGFVLAILYFFVF
GLLRYNDDGMKPALKDGDLVVYYRLDKRYSIGDLLVYSYKGKERVARVI
ATEGSTIDINENGLIINGSPQQEQDIYKETLLYKEGATFPMKVPAGQLF
VLGDNRTTAVDSRAFGTIPIQDTQGKVVTVIRRRGF (SEQ ID
NO:673)

An exemplary nucleic acid sequence for sort-1 (orf01285) is hereby provided:

ATGATGAAAACCAAGCGTGAGAAACCAAAAAAGAGTCTGTCTAGGCGTC
TCGTTCTTGCTGTGGATGGGGTGATCAATCACTTGCTGCTCATTTTTGC
AGCTTTGATCTTTCTCTTTGGTTTCTACGCCCTTTGGGATTCCAACCAA
GTCTACTCCTTAGCTTCGTCAAGTGAGTACGAAGCTTATCGACCTGTCA
CGACGCAACAGGATGAGCTGGCCAGTTTTTCAGGCTTCAGCAAACTCCA
AGAACTCAATCCCGAAGTCCTCGGTTGGATCAATGTCTATGGCACCAAT
ATCGACTATCCCTTAGTCCAAGCCAAGGACAATGAAAAGTATCTCAACA
AGGACTCCAAAGGTGAGTTTGCAGCGACAGGCGCTATCTTTCTCGATGC
ACGAAATAATCCTAAGTTCGAAGACTTTAATACCATTATCTACGGGCAC
CACGTAGAAAATGGGGTCATGTTTGGTGATGTGGCTAAGTTTGCTGATC
AGGAATTTTTTGACCAGCATCGTTACGGTAGTATATACTACAATGGTGT
GGAAAAAGGGCTCGAGATCTTTGAGATGTTGGAGGTTGATGCCTATGAC
TTTAACATCTATGATCCAGGAATACAGGGTGAGGACCGCCAGCAGGCCT
ATCTAGACCACCTGCTCTCAGTCGCCATGCACAAGCGGGATATCTCACT

CTCACCGAGTGATCGTATCATCCTACTCAGTACCTGTTTTCTCGATGTG
ACCAATGGTCGTCATATCGTAGTCGCAAAGATTACAGACACCGTCCCTA
AAAATACTTTCCATACAAAAAAATCAAAACCATTTCCATACAGTGTCTT
TGATGACTCGTCTCTTGGACGTTTCCTCTCATCAATCCCACTATGGATT
TGGTACCTTATCTTGTTTGTATTGTTCTTGCTCTTGATTTCTTACTCC
TTGTCCTCTACTTGATCCTACGTCGTAGAAGAGAGAGTAAAAAAAATGC
AAGAAGCAGACCCTTTTACTGACTAAGGGTGAATAGAAA (SEQ ID
NO:1744)

An exemplary amino acid sequence for sort-1 is hereby provided:

MMKTKREKPKKSLSRRLVLAVDGVINHLLLIFAALIFLFGFYALWDSNQ
VYSLASSSEYEAYRPVTTQQDELASFSGFSKLQELNPEVLGWINVYGTN
IDYPLVQAKDNEKYLNKDSKGEFAATGAIFLDARNNPKFEDFNTIIYGH
HVENGVMFGDVAKFADQEFFDQHRYGSIYYNGVEKGLEIFEMLEVDAYD
FNIYDPGIQGEDRQQAYLDHLLSVAMHKRDISLSPSDRIILLSTCFLDV
TNGRHIVVAKITDTVPKNTFHTKKSKPFPYSVFDDSSLGRFLSSIPLWI
WYLILFVLFLLLIFLLLVLYLILRRRRESKKNARSRPFY (SEQ ID
NO:676)

An exemplary nucleic acid sequence for sort-2 (orf01282) is hereby provided:

ATGACGGTTCAAAAAGAGCGCGATTTAAAAACGTATTTCTGGTATTCT
TCTGTGTTTTTGTAGCTCTTTTTAGTTGGCAGAGAGTAGTAGAAGCAAG
TGACTATGATCACTATAATCCTATTGAAAAGGATGCTTCGAGCACAGGT
TTTGAAACCCTACAGCACTTGAACAAAGATGTTTGCGGTTGGATTAGCC
TTGATGGGACCAAGGTAGACTATCCGCTTCTACAAAGTCAGGATAATGT
CAAATACCTTGACCGCAATGCCTTTGGCGATTATACGATAATGGGATCA
ATTTTTCTCGACTATCGCTTTAATCCCAACTTTACTGATTTTAATACGA
TCATCTACGGACACTCTATGGCTTCAGGGGCTATGTTCGGTGAGATTAA
GAAATTTGCTGATAAGGAATTCTTCGACCAGCATCGCTACGGTTCTATC
TACTACAATGGTCGAGAACGTGGTCTTGAAATTTTTGGATTTAGAAG
TGGATGCCTATGACACGGAGATTTATCGAACCTTGAGTTCCAAGGATGA
GGAACACCAGGCTTACTATCAATATCTGCTAAGTAAAGCCAAGTACAAG
CGAGATGTTTCCTTAACA (SEQ ID NO:1745)

An exemplary amino acid sequence for sort-2 is hereby provided:

MTVQKRARFKNVFLVFFCVFVALFSWQRVVEASDYDHYNPIEKDASSTG
FETLQHLNKDVCGWISLDGTKVDYPLLQSQDNVKYLDRNAFGDYTIMGS
IFLDYRFNPNFTDFNTIIYGHSMASGAMFGEIKKFADKEFFDQHRYGSI
YYNGRERGLEIFGILEVDAYDTEIYRTLSSKDEEHQAYYQYLLSKAKYK
RDVSLT (SEQ ID NO:1123)

A number of embodiments of the inventive methods and compositions have been described.

**Table 1: Pilus and Pilus II Screening Results**

| **Strain Identity & Pilus Presence** | | | | **Patient Information** | | | | **Strain Source Information** | | |
|---|---|---|---|---|---|---|---|---|---|---|
| **Strain** | **Serotype** | **Pilus** | **Pilus II** | **Year** | **Age (yrs)** | **Sex** | **Diagnosis** | **Source** | **Country** | **Reference** |
| 19FIJ | 19F | No | No | | | | | Iceland | | Same ST of NCTC11906 |
| England14-9 | 14 | No | No | | | | | PMEN | England | PN93/872/B (ATCC 700676) |
| PN137 | 14 | No | No | 1999 | 51 | F | meningitis | ISS | Italy - Arezzo | Same ST of England14-9 /mefA |
| PN099 | 14 | No | No | 1998 | 9 mos. | M | meningitis | ISS | Italy - Reggio Emilia | erm |
| Spain14-5 | 14 | No | No | 1990 | | | | PMEN | Spain | MS22 (ATCC 700902) |
| CSR14-10 | 14 | No | No | 1987 | | | | PMEN | Slovakia | 87-029055 (ATCC 700677) |
| Tennessee23F-4 | 23F | No | No | 1991 | 1 | F | bacteraemia | PMEN | USA | CS111 (ATCC 51916) |
| South Africa19A-13 | 19A | No | No | | | | | PMEN | South Africa | 51702 (ATCC 700904) |
| 67A | 8 | No | No | | | | Meningitis | Brazil | Brazil | |
| G54 | 19F | No | No | | | | | Lab strains | Italy | Complete - Glaxo Wellcome |
| South Africa19A-7 | 19A | No | No | 1989 | | | | PMEN | South Africa | 17619 (ATCC 700674) |
| Spain23F-1 | 23F | No | No | 1984 | 67 | | pneumonia | PMEN | Spain | SP264 (ATCC 700669) - Finishing - Sanger |
| Spain6B-2 | 6B | Yes^{a} | No | 1988 | | | other | PMEN | Spain | GM17 (ATCC 700670) |
| 1889 | 18C | No | No | 1996 | | | sepsis | ISS | Italy - Bergamo | (ST100 generally associated to serotypes 33F) |
| AP141 | 14 | No | No | 2002 | 38 | | sepsis | ISS | Italy - Bergamo | |
| AP173 | 14 | No | No | 2003 | 3 | | sepsis | ISS | Italy - Novara | |
| 6BIJ | 6B | Yes^{a} | No | | | | | Iceland | | |
| PT051 | 14 | Yes^{b} | No | 2001 | 49 | | sepsis | ISS 1 | Italy - Torino | Same ST of Spain9V-3 / erm |
| Spain9V-3 | 9V | Yes^{c} | No | 1993 | | | | PMEN | France | TL7/1993(ATCC 700671) |
| Poland23F-16 | 23F | Yes^{a} | No | 1999 | 13 | F | LRTI | PMEN | Poland | 178 (ATCC BAA-343) |
| PB011 | 68 | Yes^{d} | No | 2001 | 11 mos | | meningitis | ISS | Italy - Bari | |
| PB001 | 3 | No | No | 2001 | | | meningitis | ISS | Italy - Bari | erm |
| PT131 | 3 | No | No | 2002 | 34 | | pneumonia | ISS | Italy - Novara | |
| 70A | 3 | No | no | | | | Meningitis | Brazil | Brazil | |
| OXC141 | 3 | No (by BLAST) | no | | | | | Sanger | | Finishing - Sanger |
| South Africa6B-8 | 6B | No | No | 1990 | | | | PMEN | South Africa | 50803 (ATCC 700675) |
| AP/PT108 | 7F | No | Yes | 2003 | 3 | | sepsis | ISS | Italy - Cuneo | erm |
| 32/14 | 7F | No | Yes | 1999 | <5 | | carriage | ISS | Italy - Roma | |
| PN195 | 7F | No | Yes | 2002 | 44 | | meningitis | ISS | Italy - Napoli | |
| 86A | 7F | No | Yes | | | | Meningitis | Brazil | Brazil | |
| 16117 | 18C | No | No | | 10 mos. | | Carriage | Brazil | Brazil | |
| 6054 | 18C | No | No | No | 6 | | Carriage | Brazil | Brazil | |
| 19135 | 18C | No | No | | 10 | | Carriage | Brazil | Brazil | |
| TIGR4 | 4 | Yesc | No | | 30 | M | meningitis | Lab strains | Norway-Kongsvinger | JNR.7/87 (ATCC BAA-334) - Complete - TIGR |
| INV104B | 1 | No (by BLAST) | Yes | | | | | Sanger | | Finishing - Sanger |
| PNS28 | 14 | No | No | 2001 | | | sepsis | ISS | Italy - Perugia | Same ST of Clone 32 |
| Taiwan19F-14 | 19F | Yesc | Yes | 1997 | | | meningitis | PMEN | Taiwan | TW31 (ATCC 700905) |
| Taiwan23F-15 (**) | 23F | Yes^{e} | No | 1997 | | | bacteraemia | PMEN | Taiwan | TW17 (ATCC 700906) |
| Hungary19A-6 | 19A | Yes^{a} | No | 1989 | | | | PMEN | Hungary | HUN663 (ATCC 700673) |
| Finland6B-12 | 6B | Yes^{a} | No | 1987 | | | | PMEN | Finland | 43362 Fi10 (ATCC 700903) ' |
| SP307 | 5 | No | No | 2000 | 3 | | sepsis | ISS | Italy - Bergamo | |
| PT075 | 5 | No | No | 2001 | 61 | | sepsis | ISS | Italy - Torino | |
| 96A | 5 | No | no | | | | Meningitis | Brazil | Brazil | |
| PN57 | 1 | No | No | 1997 | | | meningitis | ISS | Italy - Roma | Same ST of INV1871 |
| P1031 | 1 | No | No | - 2002 | 45 | F | Meningitis | Ghana | Ghana | |
| P1074 | 1 | No | No | 2003 | 70 | M | Meningitis | Ghana | Ghana | |
| PN110 | 1 | No | Yes | 1998 | 5 | | meningitis | ISS | Italy - Lecco | |
| PB013 | 1 | No | Yes | 2002 | | | sepsis | ISS | Italy - Bari | erm |
| SPPD | 1 | No | Yes | 2005 | | | bilateral pneumonia + sepsis | Padova | Italy-North | (isolated from from BE; patient's sera available) |
| D39 | 2 | No | No | 1916 | | | pneumonia | Lab strains | Caucasian | NCTC 7466 |
| R6 | cps- | No | No | 1930 | | | | Lab strains | USA | (ATCC BAA-255) -Complete - Eli Lilly |
| P1054 | 1 | No | No | 2001 | 32 | F | Meningitis | Ghana | Ghana | |
| 1IJ | 1 | No | No | | | | | Iceland | | |
| 2010 | 34 | No | No | | 3 | | Carriage | Brazil | Brazil | |
| 30A | 3 | Yes?^{b} | no | | | | Meningitis | Brazil | Brazil | |
| 3IJ | 3 | No | No | | | | | Iceland | | |
| PN95 | 3 | No | No | 1998 | 65 | | meningitis | ISS | Italy - Milano | |
| PNS32 | 14 | No | No | 2001 | 32 | | sepsis | ISS | Italy - Napoli | Same ST of Sweden15A-25 / erm |
| 27/13 | 14 | No | No | 1999 | <5 | | carriage | ISS | Italy - Roma | |
| 6AIJ | 6A | No | No | | | | | Iceland | | |
| 117 | 4 | Yes^{b} | no | | | | Meningitis | Brazil | Brazil | |
| JJA | 14 | No | no | | | | Meningitis | Brazil | Brazil | |
| 279A | 14 | No | no | | | | Meningitis | Brazil | Brazil | |
| P1040 | 14 | No | No | 2002 | 0 | M | meningitis | Ghana | Ghana | |
| P1059 | 6A | No | No | 2002 | 47 | F | meningitis | Ghana | Ghana | |
| P1075 | 14 | No | No | 2003 | 8 | M | meningitis | Ghana | Ghana | |
| P1076 | 14 | No | No | 2003 | 0 | F | meningitis | Ghana | Ghana | |
| P1077 | 14 | No | No | 2003 | 54 | M | meningitis | Ghana | Ghana | |
| P1083 | 38 | Yes^{f} | No | 2003 | 58 | M | meningitis | Ghana | Ghana | |
| P1086 | 4 | No | No | 2002 | | | meningitis | Ghana | Ghana | |
| P1095 | 4 | No | No | 2004 | | | | Ghana | Ghana | |
| P1101 | 38 | No | No | 2004 | | | | Ghana | Ghana | |
| P1104 | 4 | No | No | 2004 | | | | Ghana | Ghana | |
| PJ1466 | 7F | no | Yes | | | | | Sweden | Sweden | |
| PJ176 | 14 | no | No | | | | | Sweden | Sweden | |
| PJ1354 | 1 | no | Yes | | | | | Sweden | Sweden | |
| I101 | 3 | no | No | | | | | Sweden | Sweden | |
| P1022 | 3 | No | No | 2001 | 1 | M | meningitis | Ghana | Ghana | |
| P1068 | 10F | No | No | 2003 | 42 | M | meningitis | Ghana | Ghana | |
| RP1554 | 9V | yes^{c} | No | | 68 | | Sweden | Sweden | Sweden | |
| RP3718 | 9V | yes^{b} | No | | 4 | | | Sweden | Sweden | |
| AP207 | 9V | Yes | No | 2003 | 71 | | meningitis | ISS | Italy | |
| PT052 | 9V | Yes | No | 2001 | 74 | | meningitis | ISS | Italy | |
| PN131 | 24F | Yes | No | 1998 | 72 | M | meningitis | ISS | Italy | |
| AP062 | 9V | Yes | No | 2003 | 91 | | | ISS | Italy | |
| AP233 | 6B | Yes | No | 2003 | 82 | | | ISS | Italy | |
| PT134 | 6B | Yes | No | 2003 | 44 | | | ISS | Italy | |
| PN6 | 6B | Yes | No | 1996 | | | meningitis | ISS | Italy | |
| PN20 | 6B | Yes | No | 1997 | 1 | M | meningitis | ISS | Italy | |
| PN68 | 6B | Yes | No | 1997 | 65 | F | meningitis | ISS | Italy | |
| PN126 | 6B | Yes | No | 1998 | 3 | M | meningitis | ISS | Italy | |
| SP95 | 6B | Yes | No | 1999 | 4 | F | meningitis | ISS | Italy | |
| AP174 | 6B | Yes | No | 2003 | 18 | | ISS | ISS | Italy | |
| PN217 | 6B | No | No | 2003 | | | meningitis | ISS | Italy | |
| 1404 | 6B | Yes | No | 1999 | | F | carriage | ISS | Italy | |
| Pn102 | 6B | Yes | No | 1998 | 2 | F | meningitis | ISS | Italy | |
| PB018 | 6B | Yes | No | 2001 | | | | ISS | Italy | |
| PN218 | 19F | No | No | 2003 | 58 | | meningitis | ISS | Italy | |
| AP235 | 23F | No | No | 2003 | 63 | | | ISS | Italy | |
| PGX1416 | 19F | Yes | Yes | | | | | ISS | | proviene dalla GSK |
| SME15 | 35B | Yes^{f} | No | | | | | Sweden | Sweden | |
| PJ1423 | 4 | Yes | No | | | | | Sweden | Sweden | |

| | | | | | | | | | | |
|---|---|---|---|---|---|---|---|---|---|---|
| Pilus Type: ^{a}6B/c; ^{b}T4; ^{c}T4/c; ^{d}6B; ^{e}23F/c; ^{f}23F | | | | | | | | | | |

## Claims

1. An isolated pilus encoded by the *Streptococcus pneumoniae* pilus II island (INV104B).

2. The pilus of claim 1, wherein the pilus comprises a sortase or a LPXTG cell wall anchored protein.

3. The pilus of claim 1, wherein the pilus has been separated from cells by enzymatic digestion or mechanical shearing, optionally wherein the mechanical shearing comprises ultrasonication.

4. The pilus of claim 1, wherein the pilus is substantially free of bacterial cells.

5. An immunogenic composition comprising one or more pili of claim 1.

6. A method of producing the pilus of claim 1, the method comprising subjecting a bacterial cell that produces the pilus to enzymatic digestion or mechanical shearing and isolating the pilus from the cell.

7. The method of claim 6, wherein:
the mechanical shearing involves ultrasonication;
the enzymatic digestion is performed using a lytic enzyme, preferably mutanolysin;
the isolating comprises one or more density gradient centrifugations;
the isolating comprises reducing polydispersity, optionally by separating components by size;
or any of the above methods further comprising degrading nucleic acids with a nuclease.

8. Pili encoded by the *Streptococcus pneumoniae* pilus II island (INV104B) for use in a method of inducing an immune response against *Streptococcus pneumoniae* in a subject.

9. The Pili of claim 8, wherein the pili are isolated.

## Patentansprüche

1. Isolierter Pilus, codiert von der *Streptococcus pneumoniae*-Pilus-II-Insel (INV104B).

2. Pilus nach Anspruch 1, wobei der Pilus eine Sortase oder ein LPXTG-zellwandverankertes Protein umfasst.

3. Pilus nach Anspruch 1, wobei der Pilus durch enzymatische Verdauung oder mechanisches Scheren von Zellen getrennt wurde, wobei gegebenenfalls das mechanische Scheren Ultraschallbehandlung umfasst.

4. Pilus nach Anspruch 1, wobei der Pilus weitgehend frei von Bakterienzellen ist.

5. Immunogene Zusammensetzung, umfassend einen oder mehrere Pili nach Anspruch 1.

6. Verfahren zur Herstellung des Pilus nach Anspruch 1, wobei man eine den Pilus produzierende Bakterienzelle enzymatischer Verdauung oder mechanischem Scheren unterzieht und den Pilus von der Zelle isoliert.

7. Verfahren nach Anspruch 6, wobei:
das mechanische Scheren Ultraschallbehandlung beinhaltet;
die enzymatische Verdauung unter Verwendung eines lytischen Enzyms, vorzugsweise Mutanolysin, erfolgt;
das Isolieren eine oder mehrere Dichtegradientenzentrifugationen umfasst;
das Isolieren das Reduzieren der Polydispersität umfasst, gegebenenfalls durch Trennen von Komponenten nach Größe;
oder eines der obigen Verfahren ferner den Abbau von Nukleinsäuren mit einer Nuklease umfasst.

8. Pili, codiert von der *Streptococcus pneumoniae-*Pilus-II-Insel (INV104B), zur Verwendung in einem Verfahren zum Induzieren einer Immunantwort gegen *Streptococcus pneumoniae* bei einem Patienten.

9. Pili nach Anspruch 8, wobei die Pili isoliert sind.

## Revendications

1. Pilus isolé codé par l'îlot de pilus II du *Streptococcus pneumoniae* (INV104B).

2. Pilus selon la revendication 1, dans lequel le pilus comprend une sortase ou une protéine ancrée sur la paroi cellulaire LPXTG.

3. Pilus selon la revendication 1, dans lequel le pilus a été séparé de cellules par une digestion enzymatique ou un cisaillement mécanique, optionnellement dans lequel le cisaillement mécanique comprend une application d'ultrasons.

4. Pilus selon la revendication 1, dans lequel le pilus est sensiblement exempt de cellules bactériennes.

5. Composition immunogène contenant un pilus ou plusieurs pili selon la revendication 1.

6. Procédé de production du pilus selon la revendication 1, le procédé comprenant les étapes consistant à soumettre une cellule bactérienne qui produit le pilus à une digestion enzymatique ou à un cisaillement mécanique, et à isoler le pilus de la cellule.

7. Procédé selon la revendication 6, dans lequel:
le cisaillement mécanique implique une application d'ultrasons;
la digestion enzymatique est exécutée en utilisant un enzyme lytique, de préférence la mutanolysine;
l'isolation comprend une ou plusieurs centrifugation(s) en gradient de densité;
l'isolation comprend la réduction de la polydispersité, optionnellement en séparant les composants en fonction de leur taille; ou
l'un quelconque des procédés ci-dessus comprenant en outre la dégradation des acides nucléiques avec une nucléase.

8. Pili codés par l'îlot de pilus II du *Streptococcus pneumoniae* (INV104B) à utiliser dans un procédé pour induire une réponse immunitaire contre le *Streptococcus pneumoniae* chez un sujet.

9. Pili selon la revendication 8, dans lesquels les pili sont isolés.
